# EUROPEAN PATENT APPLICATION

(11) **EP 1 944 043 A1**
(43) Date of publication of application: **16.07.2008**
(21) Application number: 08003966.2
(22) Date of filing: 20.11.2002
(51) Int. Cl.: A61K 47/42, C07H 21/00, C07K 14/075, C12N 7/00, C12N 15/33, C12N 15/861

(54) **Simian adenovirus nucleic acid and amino acid sequences, vectors containing same, and methods of use**

(30) Priority: 21.11.2001 US 331951 P; 22.03.2002 US 366798 P
(62) Divisional of application: 02803963.4
(71) Applicant: The Trustees of the University of Pennsylvania, Philadelphia PA (US)
(72) Inventor: Wilson, James, M., Gladwyne, Pennsylvania 19035 (US); Gao, Guangping, Rosemont, Pennsylvania 19010 (US); Roy, Soumitra, Wayne, Pennsylvania 19087 (US)
(74) Representative: Manaton, Ross Timothy

(57) **Abstract**

A recombinant vector comprises simian adenovirus sequences and a heterologous gene under the control of regulatory sequences. A cell line which expresses simian adenovirus gene(s) is also disclosed. Methods of using the vectors and cell lines are provided.

SEQUENCE LISTING

<110>  The Trustees of the University of Pennsylvania
       Wilson, James M.
       Gao, Guangping
       Roy, Soumitra

<120>  Simian Adenovirus Nucleic Acid and Amino Acid Sequences, Vectors Containing
Same, and Methods of Use

<130>  UPN-02677PCT

<150>  US 60/331,951
<151>  2001-11-21

<150>  US 60/366,798
<151>  2002-03-22

<150>  PCT/US02/33645
<151>  2002-11-20

<150>  EP 02803963.4
<151>  2002-11-20

<160>  39

<170>  PatentIn version 3.1

<210>  1
<211>  36462
<212>  DNA
<213>  chimpanzee adenovirus serotype Pan5

<220>

<221>  CDS

<222>  (13898)..(15490)

<223>  L2 Penton


<220>

<221>  CDS

<222>  (18315)..(21116)

<223>  L3 Hexon


<220>

<221>  CDS

<222>  (32035)..(33372)

<223>  L5 Fiber


<400>  1
catcatcaat aatatacctc aaacttttgg tgcgcgttaa tatgcaaatg aggtatttga      60

atttggggat gcggggcggt gattggctgc gggagcggcg accgttaggg gcggggcggg     120

tgacgttttg atgacgtggc cgtgaggcgg agccggtttg caagttctcg tgggaaaagt     180

gacgtcaaac gaggtgtggt ttgaacacgg aaatactcaa ttttcccgcg ctctctgaca     240

ggaaatgagg tgtttctggg cggatgcaag tgaaaacggg ccattttcgc gcgaaaactg     300

aatgaggaag tgaaaatctg agtaattccg cgtttatggc agggaggagt atttgccgag     360

ggccgagtag actttgaccg attacgtggg ggtttcgatt accgtatttt tcacctaaat     420

ttccgcgtac ggtgtcaaag tccggtgttt ttacgtaggt gtcagctgat cgccagggta     480

tttaaacctg cgctctctag tcaagaggcc actcttgagt gccagcgagt agagttttct     540

cctccgcgcc gcgagtcaga tctacacttt gaaagatgag gcacctgaga gacctgcccg     600

gtaatgtttt cctggctact gggaacgaga ttctggaact ggtggtggac gccatgatgg     660

gtgacgaccc tccggagccc cctaccccat ttgaagcgcc ttcgctgtac gatttgtatg     720

atctggaggt ggatgtgccc gagaacgacc ccaacgagga ggcggtgaat gatttgttta     780

gcgatgccgc gctgctggct gccgagcagg ctaatacgga ctctggctca gacagcgatt     840

cctctctcca taccccgaga cccggcagag gtgagaaaaa gatccccgag cttaaagggg     900

aagagctcga cctgcgctgc tatgaggaat gcttgcctcc gagcgatgat gaggaggacg 960
aggaggcgat tcgagctgca gcgaaccagg gagtgaaaac agcgagcgag ggctttagcc 1020
tggactgtcc tactctgccc ggacacggct gtaagtcttg tgaatttcat cgcatgaata 1080
ctggagataa gaatgtgatg tgtgccctgt gctatatgag agcttacaac cattgtgttt 1140
acagtaagtg tgattaactt tagctgggga ggcagagggt gactgggtgc tgactggttt 1200
atttatgtat atgtttttta tgtgtaggtc ccgtctctga cgtagatgag acccccacta 1260
cagagtgcat ttcatcaccc ccagaaattg gcgaggaacc gcccgaagat attattcata 1320
gaccagttgc agtgagagtc accgggcgta gagcagctgt ggagagtttg gatgacttgc 1380
tacagggtgg ggatgaacct ttggacttgt gtacccggaa acgccccagg cactaagtgc 1440
cacacatgtg tgtttactta aggtgatgtc agtatttata gggtgtggag tgcaataaaa 1500
tccgtgttga ctttaagtgc gtggtttatg actcaggggt ggggactgtg ggtatataag 1560
caggtgcaga cctgtgtggt cagttcagag caggactcat ggagatctgg acagtcttgg 1620
aagactttca ccagactaga cagctgctag agaactcatc ggagggagtc tcttacctgt 1680
ggagattctg cttcggtggg cctctagcta agctagtcta tagggccaag caggattata 1740
aggatcaatt tgaggatatt ttgagagagt gtcctggtat tttgactctc ctcaacttgg 1800
gccatcagtc tcactttaac cagagtattc tgagagccct tgacttttct actcctggca 1860
gaactaccgc cgcggtagcc ttttttgcct ttatccttga caaatggagt caagaaaccc 1920
atttcagcag ggattaccgt ctggactgct tagcagtagc tttgtggaga acatggaggt 1980
gccagcgcct gaatgcaatc tccggctact tgccagtaca gccggtagac acgctgagga 2040
tcctgagtct ccagtcaccc caggaacacc aacgccgcca gcagccgcag caggagcagc 2100
agcaagagga ggaccgagaa gagaacctga gagccggtct ggaccctccg gtggcggagg 2160
aggaggagta gctgacttgt ttcccgagct gcgccgggtg ctgactaggt cttccagtgg 2220
acgggagagg gggattaagc gggagaggca tgaggagact agccacagaa ctgaactgac 2280
tgtcagtctg atgagtcgca ggcgcccaga atcggtgtgg tggcatgagg tgcagtcgca 2340
ggggatagat gaggtctcag tgatgcatga gaaatattcc ctagaacaag tcaagacttg 2400
ttggttggag cccgaggatg attgggaggt agccatcagg aattatgcca agctggctct 2460
gaggccagac aagaagtaca agattaccaa actgattaat atcagaaatt cctgctacat 2520
ttcagggaat ggggccgagg tggagatcag tacccaggag agggtggcct tcagatgctg 2580
catgatgaat atgtacccgg gggtggtggg catggaggga gtcaccttta tgaacgcgag 2640
gttcaggggt gatgggtata atggggtggt ctttatggcc aacaccaagc tgacagtgca 2700
cggatgctcc ttctttggct tcaataacat gtgcattgag gcctggggca gtgtttcagt 2760
gaggggatgc agtttttcag ccaactggat gggggtcgtg ggcagaacca agagcatggt 2820

gtcagtgaag aaatgcctgt tcgagaggtg ccacctgggg gtgatgagcg agggcgaagc 2880
caaagtcaaa cactgcgcct ctaccgagac gggctgcttt gtactgatca agggcaatgc 2940
caaagtcaag cataatatga tctgtggggc ctcggatgag cgcggctacc agatgctgac 3000
ctgcgccggt gggaacagcc atatgctagc caccgtgcat gtggcctcgc acccccgcaa 3060
gacatggccc gagttcgagc acaacgtcat gacccgctgc aatgtgcacc tggggtcccg 3120
ccgaggcatg ttcatgccct accagtgcaa catgcaattt gtgaaggtgc tgctggagcc 3180
cgatgccatg tccagagtga gcctgacggg ggtgtttgac atgaatgtgg agctgtggaa 3240
aattctgaga tatgatgaat ccaagaccag gtgccgggcc tgcgaatgcg gaggcaagca 3300
cgccaggctt cagcccgtgt gtgtggaggt gacggaggac ctgcgacccg atcatttggt 3360
gttgtcctgc aacgggacgg agttcggctc cagcggggaa gaatctgact agagtgagta 3420
gtgtttggga ctgggtggga gcctgcatga tgggcagaat gactaaaatc tgtgtttttc 3480
tgcgcagcag catgagcgga agcgcctcct ttgagggagg ggtattcagc ccttatctga 3540
cggggcgtct cccctcctgg gcgggagtgc gtcagaatgt gatgggatcc acggtggacg 3600
gccggcccgt gcagcccgcg aactcttcaa ccctgaccta cgcgaccctg agctcctcgt 3660
ccgtggacgc agctgccgcc gcagctgctg cttccgccgc cagcgccgtg cgcggaatgg 3720
ccctgggcgc cggctactac agctctctgg tggccaactc gagttccacc aataatcccg 3780
ccagcctgaa cgaggagaag ctgctgctgc tgatggccca gctcgaggcc ctgacccagc 3840
gcctgggcga gctgacccag caggtggctc agctgcaggc ggagacgcgg gccgcggttg 3900
ccacggtgaa aaccaaataa aaaatgaatc aataaataaa cggagacggt tgttgatttt 3960
aacacagagt cttgaatctt tatttgattt ttcgcgcgcg gtaggccctg gaccaccggt 4020
ctcgatcatt gagcacccgg tggatctttt ccaggacccg gtagaggtgg gcttggatgt 4080
tgaggtacat gggcatgagc ccgtcccggg ggtggaggta gctccattgc agggcctcgt 4140
gctcgggggt ggtgttgtaa atcacccagt catagcaggg gcgcagggcg tggtgctgca 4200
cgatgtcctt gaggaggaga ctgatggcca cgggcagccc cttggtgtag gtgttgacga 4260
acctgttgag ctgggaggga tgcatgcggg gggagatgag atgcatcttg gcctggatct 4320
tgagattggc gatgttcccg cccagatccc gccgggggtt catgttgtgc aggaccacca 4380
gcacggtgta tccggtgcac ttggggaatt tgtcatgcaa cttggaaggg aaggcgtgaa 4440
agaatttgga gacgcccttg tgaccgccca ggttttccat gcactcatcc atgatgatgg 4500
cgatgggccc gtgggcggcg gcttgggcaa agacgtttcg ggggtcggac acatcgtagt 4560
tgtggtcctg ggtgagctcg tcataggcca ttttaatgaa tttggggcgg agggtgcccg 4620
actgggggac gaaggtgccc tcgatcccgg gggcgtagtt gccctcgcag atctgcatct 4680

cccaggcctt gagctcggag ggggggatca tgtccacctg cggggcgatg aaaaaaacgg 4740
tttccggggc gggggagatg agctgggccg aaagcaggtt ccggagcagc tgggacttgc 4800
cgcagccggt ggggccgtag atgaccccga tgaccggctg caggtggtag ttgagggaga 4860
gacagctgcc gtcctcgcgg aggagggggg ccacctcgtt catcatctcg cgcacatgca 4920
tgttctcgcg cacgagttcc gccaggaggc gctcgccccc aagcgagagg agctcttgca 4980
gcgaggcgaa gtttttcagc ggcttgagcc cgtcggccat gggcattttg gagagggtct 5040
gttgcaagag ttccagacgg tcccagagct cggtgatgtg ctctagggca tctcgatcca 5100
gcagacctcc tcgtttcgcg ggttggggcg actgcgggag tagggcacca ggcgatgggc 5160
gtccagcgag gccagggtcc ggtccttcca ggggcgcagg gtccgcgtca gcgtggtctc 5220
cgtcacggtg aaggggtgcg cgccgggctg ggcgcttgcg agggtgcgct tcaggctcat 5280
ccggctggtc gagaaccgct cccggtcggc gccctgcgcg tcggccaggt agcaattgag 5340
catgagttcg tagttgagcg cctcggccgc gtggcccttg gcgcggagct tacctttgga 5400
agtgtgtccg cagacgggac agaggaggga cttgagggcg tagagcttgg gggcgaggaa 5460
gacggactcg ggggcgtagg cgtccgcgcc gcagctggcg cagacggtct cgcactccac 5520
gagccaggtg aggtctggcc ggtcggggtc aaaaacgagg tttcctccgt gctttttgat 5580
gcgtttctta cctctggtct ccatgagctc gtgtccccgc tgggtgacaa agaggctgtc 5640
cgtgtccccg tagaccgact ttatgggccg gtcctcgagc ggggtgccgc ggtcctcgtc 5700
gtagaggaac cccgcccact ccgagacgaa ggcccgggtc caggccagca cgaaggaggc 5760
cacgtgggag gggtagcggt cgttgtccac cagcgggtcc accttctcca gggtatgcaa 5820
gcacatgtcc ccctcgtcca catccaggaa ggtgattggc ttgtaagtgt aggccacgtg 5880
accgggggtc ccggccgggg gggtataaaa gggggcgggc ccctgctcgt cctcactgtc 5940
ttccggatcg ctgtccagga gcgccagctg ttggggtagg tattccctct cgaaggcggg 6000
catgacctcg gcactcaggt tgtcagtttc tagaaacgag gaggatttga tattgacggt 6060
gccgttggag acgcctttca tgagcccctc gtccatctgg tcagaaaaga cgatcttttt 6120
gttgtcgagc ttggtggcga aggagccgta gagggcgttg gagagcagct tggcgatgga 6180
gcgcatggtc tggttctttt ccttgtcggc gcgctccttg gcggcgatgt tgagctgcac 6240
gtactcgcgc gccacgcact tccattcggg gaagacggtg gtgagcttgt cgggcacgat 6300
tctgacccgc cagccgcggt tgtgcagggt gatgaggtcc acgctggtgg ccacctcgcc 6360
gcgcaggggc tcgttggtcc agcagaggcg cccgcccttg cgcgagcaga aggggggcag 6420
cgggtccagc atgagctcgt cgggggggtc ggcgtccacg gtgaagatgc cgggcaggag 6480
ctcggggtcg aagtagctga tgcaggtgcc cagatcgtcc agcgccgctt gccagtcgcg 6540
cacggccagc gcgcgctcgt aggggctgag gggcgtgccc cagggcatgg ggtgcgtgag 6600

cgcggaggcg tacatgccgc agatgtcgta gacgtagagg ggctcctcga ggacgccgat 6660
gtaggtgggg tagcagcgcc ccccgcggat gctggcgcgc acgtagtcgt acagctcgtg 6720
cgagggcgcg aggagcccgg tgccgaggtt ggagcgctgc ggcttttcgg cgcggtagac 6780
gatctggcgg aagatggcgt gggagttgga ggagatggtg ggcctctgga agatgttgaa 6840
gtgggcgtgg ggcagtccga ccgagtccct gatgaagtgg gcgtaggagt cctgcagctt 6900
ggcgacgagc tcggcggtga cgaggacgtc cagggcgcag tagtcgaggg tctcttggat 6960
gatgtcgtac ttgagctggc ccttctgctt ccacagctcg cggttgagaa ggaactcttc 7020
gcggtccttc cagtactctt cgagggggaa cccgtcctga tcggcacggt aagagcccac 7080
catgtagaac tggttgacgg ccttgtaggc gcagcagccc ttctccacgg ggagggcgta 7140
agcttgcgcg gccttgcgca gggaggtgtg ggtgagggcg aaggtgtcgc gcaccatgac 7200
cttgaggaac tggtgcttga agtcgaggtc gtcgcagccg ccctgctccc agagctggaa 7260
gtccgtgcgc ttcttgtagg cggggttggg caaagcgaaa gtaacatcgt tgaagaggat 7320
cttgcccgcg cggggcatga agttgcgagt gatgcggaaa ggctggggca cctcggcccg 7380
gttgttgatg acctgggcgg cgaggacgat ctcgtcgaag ccgttgatgt tgtgcccgac 7440
gatgtagagt tccacgaatc gcgggcggcc cttgacgtgg ggcagcttct tgagctcgtc 7500
gtaggtgagc tcggcggggt cgctgaggcc gtgctgctcg agggcccagt cggcgaggtg 7560
ggggttggcg ccgaggaagg aagtccagag atccacggcc agggcggtct gcaagcggtc 7620
ccggtactga cggaactgct ggcccacggc cattttttcg ggggtgacgc agtagaaggt 7680
gcgggggtcg ccgtgccagc ggtcccactt gagctggagg gcgaggtcgt gggcgagctc 7740
gacgagcggc gggtccccgg agagtttcat gaccagcatg aaggggacga gctgcttgcc 7800
gaaggacccc atccaggtgt aggtttccac gtcgtaggtg aggaagagcc tttcggtgcg 7860
aggatgcgag ccgatgggga agaactggat ctcctgccac cagttggagg aatggctgtt 7920
gatgtgatgg aagtagaaat gccgacggcg cgccgagcac tcgtgcttgt gtttatacaa 7980
gcgtccgcag tgctcgcaac gctgcacggg atgcacgtgc tgcacgagct gtacctgggt 8040
tcctttgacg aggaatttca gtgggcagtg gagcgctggc ggctgcatct ggtgctgtac 8100
tacgtcctgg ccatcggcgt ggccatcgtc tgcctcgatg gtggtcatgc tgacgaggcc 8160
gcgcgggagg caggtccaga cctcggctcg gacgggtcgg agagcgagga cgagggcgcg 8220
caggccggag ctgtccaggg tcctgagacg ctgcggagtc aggtcagtgg gcagcggcgg 8280
cgcgcggttg acttgcagga gcttttccag ggcgcgcggg aggtccagat ggtacttgat 8340
ctccacggcg ccgttggtgg cgacgtccac ggcttgcagg gtcccgtgcc cctggggcgc 8400
caccaccgtg ccccgtttct tcttgggtgc tggcggcggc ggctccatgc ttagaagcgg 8460

cggcgaggac gcgcgccggg cggcaggggc ggctcggggc ccggaggcag gggcggcagg 8520
ggcacgtcgg cgccgcgcgc gggcaggttc tggtactgcg cccggagaag actggcgtga 8580
gcgacgacgc gacggttgac gtcctggatc tgacgcctct gggtgaaggc cacgggaccc 8640
gtgagtttga acctgaaaga gagttcgaca gaatcaatct cggtatcgtt gacggcggcc 8700
tgccgcagga tctcttgcac gtcgcccgag ttgtcctggt aggcgatctc ggtcatgaac 8760
tgctcgatct cctcctcctg aaggtctccg cgaccggcgc gctcgacggt ggccgcgagg 8820
tcgttggaga tgcggcccat gagctgcgag aaggcgttca tgccggcctc gttccagacg 8880
cggctgtaga ccacggctcc gtcggggtcg cgcgcgcgca tgaccacctg ggcgaggttg 8940
agctcgacgt ggcgcgtgaa gaccgcgtag ttgcagaggc gctggtagag gtagttgagc 9000
gtggtggcga tgtgctcggt gacgaagaag tacatgatcc agcggcggag cggcatctcg 9060
ctgacgtcgc ccagggcttc caagcgctcc atggcctcgt agaagtccac ggcgaagttg 9120
aaaaactggg agttgcgcgc cgagacggtc aactcctcct ccagaagacg gatgagctcg 9180
gcgatggtgg cgcgcacctc gcgctcgaag gccccggggg gctcctcttc ttccatctcc 9240
tcctcctctt ccatctcctc cactaacatc tcttctactt cctcctcagg aggcggcggc 9300
gggggagggg ccctgcgtcg ccggcggcgc acgggcagac ggtcgatgaa gcgctcgatg 9360
gtctccccgc gccggcgacg catggtctcg gtgacggcgc gcccgtcctc gcggggccgc 9420
agcgtgaaga cgccgccgcg catctccagg tggccgccgg gggggtctcc gttgggcagg 9480
gagagggcgc tgacgatgca tcttatcaat tggcccgtag ggactccgcg caaggacctg 9540
agcgtctcga gatccacggg atccgaaaac cgctgaacga aggcttcgag ccagtcgcag 9600
tcgcaaggta ggctgagccc ggtttcttgt tcttcgggta tttggtcggg aggcgggcgg 9660
gcgatgctgc tggtgatgaa gttgaagtag gcggtcctga gacggcggat ggtggcgagg 9720
agcaccaggt ccttgggccc ggcttgctgg atgcgcagac ggtcggccat gccccaggcg 9780
tggtcctgac acctggcgag gtccttgtag tagtcctgca tgagccgctc cacgggcacc 9840
tcctcctcgc ccgcgcggcc gtgcatgcgc gtgagcccga acccgcgctg cggctggacg 9900
agcgccaggt cggcgacgac gcgctcggcg aggatggcct gctggatctg ggtgagggtg 9960
gtctggaagt cgtcgaagtc gacgaagcgg tggtaggctc cggtgttgat ggtgtaggag 10020
cagttggcca tgacggacca gttgacggtc tggtggccgg ggcgcacgag ctcgtggtac 10080
ttgaggcgcg agtaggcgcg cgtgtcgaag atgtagtcgt tgcaggtgcg cacgaggtac 10140
tggtatccga cgaggaagtg cggcggcggc tggcggtaga gcggccatcg ctcggtggcg 10200
ggggcgccgg gcgcgaggtc ctcgagcatg aggcggtggt agccgtagat gtacctggac 10260
atccaggtga tgccggcggc ggtggtggag gcgcgcggga actcgcggac gcggttccag 10320
atgttgcgca gcggcaggaa gtagttcatg gtggccgcgg tctggcccgt gaggcgcgcg 10380

cagtcgtgga tgctctagac atacgggcaa aaacgaaagc ggtcagcggc tcgactccgt 10440
ggcctggagg ctaagcgaac gggttgggct gcgcgtgtac cccggttcga gtccctgctc 10500
gaatcaggct ggagccgcag ctaacgtggt actggcactc ccgtctcgac ccaagcctgc 10560
taacgaaacc tccaggatac ggaggcgggt cgttttggcc attttcgtca ggccggaaat 10620
gaaactagta agcgcggaaa gcggccgtcc gcgatggctc gctgccgtag tctggagaaa 10680
gaatcgccag ggttgcgttg cggtgtgccc cggttcgagc ctcagcgctc ggcgccggcc 10740
ggattccgcg gctaacgtgg gcgtggctgc cccgtcgttt ccaagacccc ttagccagcc 10800
gacttctcca gttacggagc gagcccctct ttttcttgtg tttttgccag atgcatcccg 10860
tactgcggca gatgcgcccc caccctccac cacaaccgcc cctaccgcag cagcagcaac 10920
agccggcgct tctgcccccg ccccagcagc agcagccagc cactaccgcg gcggccgccg 10980
tgagcggagc cggcgttcag tatgacctgg ccttggaaga gggcgagggg ctggcgcggc 11040
tgggggcgtc gtcgccggag cggcacccgc gcgtgcagat gaaaagggac gctcgcgagg 11100
cctacgtgcc caagcagaac ctgttcagag acaggagcgg cgaggagccc gaggagatgc 11160
gcgcctcccg cttccacgcg gggcgggagc tgcggcgcgg cctggaccga aagcgggtgc 11220
tgagggacga ggatttcgag gcggacgagc tgacggggat cagccccgcg cgcgcgcacg 11280
tggccgcggc caacctggtc acggcgtacg agcagaccgt gaaggaggag agcaacttcc 11340
aaaaatcctt caacaaccac gtgcgcacgc tgatcgcgcg cgaggaggtg accctgggcc 11400
tgatgcacct gtgggacctg ctggaggcca tcgtgcagaa ccccacgagc aagccgctga 11460
cggcgcagct gtttctggtg gtgcagcaca gtcgggacaa cgagacgttc agggaggcgc 11520
tgctgaatat caccgagccc gagggccgct ggctcctgga cctggtgaac attctgcaga 11580
gcatcgtggt gcaggagcgc gggctgccgc tgtccgagaa gctggcggcc atcaacttct 11640
cggtgctgag cctgggcaag tactacgcta ggaagatcta caagaccccg tacgtgccca 11700
tagacaagga ggtgaagatc gacgggtttt acatgcgcat gaccctgaaa gtgctgaccc 11760
tgagcgacga tctgggggtg taccgcaacg acaggatgca ccgcgcggtg agcgccagcc 11820
gccggcgcga gctgagcgac caggagctga tgcacagcct gcagcgggcc ctgaccgggg 11880
ccgggaccga gggggagagc tactttgaca tgggcgcgga cctgcgctgg cagcctagcc 11940
gccgggcctt ggaagctgcc ggcggttccc cctacgtgga ggaggtggac gatgaggagg 12000
aggagggcga gtacctggaa gactgatggc gcgaccgtat ttttgctaga tgcagcaaca 12060
gccaccgccg cctcctgatc ccgcgatgcg ggcggcgctg cagagccagc cgtccggcat 12120
taactcctcg gacgattgga cccaggccat gcaacgcatc atggcgctga cgacccgcaa 12180
tcccgaagcc tttagacagc agcctcaggc caaccgactc tcggccatcc tggaggccgt 12240

ggtgccctcg cgctcgaacc ccacgcacga gaaggtgctg gccatcgtga acgcgctggt 12300

ggagaacaag gccatccgcg gcgacgaggc cgggctggtg tacaacgcgc tgctggagcg 12360

cgtggcccgc tacaacagca ccaacgtgca gacgaacctg gaccgcatgg tgaccgacgt 12420

gcgcgaggcg gtgtcgcagc gcgagcggtt ccaccgcgag tcgaacctgg gctccatggt 12480

ggcgctgaac gccttcctga gcacgcagcc cgccaacgtg ccccggggcc aggaggacta 12540

caccaacttc atcagcgcgc tgcggctgat ggtggccgag gtgccccaga gcgaggtgta 12600

ccagtcgggg ccggactact tcttccagac cagtcgccag ggcttgcaga ccgtgaacct 12660

gagccaggct ttcaagaact tgcagggact gtggggcgtg caggccccgg tcggggaccg 12720

cgcgacggtg tcgagcctgc tgacgccgaa ctcgcgcctg ctgctgctgc tggtggcgcc 12780

cttcacggac agcggcagcg tgagccgcga ctcgtacctg ggctacctgc ttaacctgta 12840

ccgcgaggcc atcgggcagg cgcacgtgga cgagcagacc taccaggaga tcacccacgt 12900

gagccgcgcg ctgggccagg aggacccggg caacctggag gccaccctga acttcctgct 12960

gaccaaccgg tcgcagaaga tcccgcccca gtacgcgctg agcaccgagg aggagcgcat 13020

cctgcgctac gtgcagcaga gcgtggggct gttcctgatg caggaggggg ccacgcccag 13080

cgccgcgctc gacatgaccg cgcgcaacat ggagcccagc atgtacgccc gcaaccgccc 13140

gttcatcaat aagctgatgg actacttgca tcgggcggcc gccatgaact cggactactt 13200

taccaacgcc atcttgaacc cgcactggct cccgccgccc gggttctaca cgggcgagta 13260

cgacatgccc gaccccaacg acgggttcct gtgggacgac gtggacagca gcgtgttctc 13320

gccgcgcccc accaccacca ccgtgtggaa gaaagagggc ggggaccggc ggccgtcctc 13380

ggcgctgtcc ggtcgcgcgg gtgctgccgc ggcggtgccc gaggccgcca gccccttccc 13440

gagcctgccc ttttcgctga acagcgtgcg cagcagcgag ctgggtcggc tgacgcggcc 13500

gcgcctgctg ggcgaggagg agtacctgaa cgactccttg cttcggcccg agcgcgagaa 13560

gaacttcccc aataacggga tagagagcct ggtggacaag atgagccgct ggaagacgta 13620

cgcgcacgag cacagggacg agccccgagc tagcagcagc accggcgcca cccgtagacg 13680

ccagcggcac gacaggcagc ggggtctggt gtgggacgat gaggattccg ccgacgacag 13740

cagcgtgttg gacttgggtg ggagtggtgg tggtaacccg ttcgctcacc tgcgcccccg 13800

tatcgggcgc ctgatgtaag aatctgaaaa aataaaagac ggtactcacc aaggccatgg 13860

cgaccagcgt gcgttcttct ctgttgtttg tagtagt atg atg agg cgc gtg tac 13915
Met Met Arg Arg Val Tyr
1               5

ccg gag ggt cct cct ccc tcg tac gag agc gtg atg cag cag gcg gtg 13963
Pro Glu Gly Pro Pro Pro Ser Tyr Glu Ser Val Met Gln Gln Ala Val
            10                  15                  20

gcg gcg gcg atg cag ccc ccg ctg gag gcg cct tac gtg ccc ccg cgg 14011

Ala Ala Ala Met Gln Pro Pro Leu Glu Ala Pro Tyr Val Pro Pro Arg
        25                  30                  35

tac ctg gcg cct acg gag ggg cgg aac agc att cgt tac tcg gag ctg      14059
Tyr Leu Ala Pro Thr Glu Gly Arg Asn Ser Ile Arg Tyr Ser Glu Leu
    40                  45                  50

gca ccc ttg tac gat acc acc cgg ttg tac ctg gtg gac aac aag tcg      14107
Ala Pro Leu Tyr Asp Thr Thr Arg Leu Tyr Leu Val Asp Asn Lys Ser
55                  60                  65                  70

gcg gac atc gcc tcg ctg aac tac cag aac gac cac agc aac ttc ctg      14155
Ala Asp Ile Ala Ser Leu Asn Tyr Gln Asn Asp His Ser Asn Phe Leu
                75                  80                  85

acc acc gtg gtg cag aac aac gat ttc acc ccc acg gag gcc agc acc      14203
Thr Thr Val Val Gln Asn Asn Asp Phe Thr Pro Thr Glu Ala Ser Thr
            90                  95                  100

cag acc atc aac ttt gac gag cgc tcg cgg tgg ggc ggc cag ctg aaa      14251
Gln Thr Ile Asn Phe Asp Glu Arg Ser Arg Trp Gly Gly Gln Leu Lys
        105                 110                 115

acc atc atg cac acc aac atg ccc aac gtg aac gag ttc atg tac agc      14299
Thr Ile Met His Thr Asn Met Pro Asn Val Asn Glu Phe Met Tyr Ser
    120                 125                 130

aac aag ttc aag gcg cgg gtg atg gtc tcg cgc aag acc ccc aac ggg      14347
Asn Lys Phe Lys Ala Arg Val Met Val Ser Arg Lys Thr Pro Asn Gly
135                 140                 145                 150

gtc aca gta aca gat ggt agt cag gac gag ctg acc tac gag tgg gtg      14395
Val Thr Val Thr Asp Gly Ser Gln Asp Glu Leu Thr Tyr Glu Trp Val
                155                 160                 165

gag ttt gag ctg ccc gag ggc aac ttc tcg gtg acc atg acc atc gat      14443
Glu Phe Glu Leu Pro Glu Gly Asn Phe Ser Val Thr Met Thr Ile Asp
            170                 175                 180

ctg atg aac aac gcc atc atc gac aac tac ttg gcg gtg ggg cgg cag      14491
Leu Met Asn Asn Ala Ile Ile Asp Asn Tyr Leu Ala Val Gly Arg Gln
        185                 190                 195

aac ggg gtg ctg gag agc gac atc ggc gtg aag ttc gac acg cgc aac      14539
Asn Gly Val Leu Glu Ser Asp Ile Gly Val Lys Phe Asp Thr Arg Asn
    200                 205                 210

ttc cgg ctg ggc tgg gac ccc gtg acc gag ctg gtg atg ccg ggc gtg      14587
Phe Arg Leu Gly Trp Asp Pro Val Thr Glu Leu Val Met Pro Gly Val
215                 220                 225                 230

tac acc aac gag gcc ttc cac ccc gac atc gtc ctg ctg ccc ggc tgc      14635
Tyr Thr Asn Glu Ala Phe His Pro Asp Ile Val Leu Leu Pro Gly Cys
                235                 240                 245

ggc gtg gac ttc acc gag agc cgc ctc agc aac ctg ctg ggc atc cgc      14683
Gly Val Asp Phe Thr Glu Ser Arg Leu Ser Asn Leu Leu Gly Ile Arg
            250                 255                 260

aag cgg cag ccc ttc cag gag ggc ttc cag atc ctg tac gag gac ctg      14731
Lys Arg Gln Pro Phe Gln Glu Gly Phe Gln Ile Leu Tyr Glu Asp Leu
        265                 270                 275

gag ggg ggc aac atc ccc gcg ctg ctg gac gtg gac gcc tac gag aaa     14779
Glu Gly Gly Asn Ile Pro Ala Leu Leu Asp Val Asp Ala Tyr Glu Lys
    280                 285                 290

agc aag gag gat agc gcc gcc gcg gcg acc gca gcc gtg gcc acc gcc     14827
Ser Lys Glu Asp Ser Ala Ala Ala Ala Thr Ala Ala Val Ala Thr Ala
295                 300                 305                 310

tct acc gag gtg cgg ggc gat aat ttt gct agc gcc gcg aca ctg gca     14875
Ser Thr Glu Val Arg Gly Asp Asn Phe Ala Ser Ala Ala Thr Leu Ala
                315                 320                 325

gcg gcc gag gcg gct gaa acc gaa agt aag ata gtg atc cag ccg gtg     14923
Ala Ala Glu Ala Ala Glu Thr Glu Ser Lys Ile Val Ile Gln Pro Val
            330                 335                 340

gag aag gac agc aag gag agg agc tac aac gtg ctc gcg gac aag aaa     14971
Glu Lys Asp Ser Lys Glu Arg Ser Tyr Asn Val Leu Ala Asp Lys Lys
        345                 350                 355

aac acc gcc tac cgc agc tgg tac ctg gcc tac aac tac ggc gac ccc     15019
Asn Thr Ala Tyr Arg Ser Trp Tyr Leu Ala Tyr Asn Tyr Gly Asp Pro
    360                 365                 370

gag aag ggc gtg cgc tcc tgg acg ctg ctc acc acc tcg gac gtc acc     15067
Glu Lys Gly Val Arg Ser Trp Thr Leu Leu Thr Thr Ser Asp Val Thr
375                 380                 385                 390

tgc ggc gtg gag caa gtc tac tgg tcg ctg ccc gac atg atg caa gac     15115
Cys Gly Val Glu Gln Val Tyr Trp Ser Leu Pro Asp Met Met Gln Asp
                395                 400                 405

ccg gtc acc ttc cgc tcc acg cgt caa gtt agc aac tac ccg gtg gtg     15163
Pro Val Thr Phe Arg Ser Thr Arg Gln Val Ser Asn Tyr Pro Val Val
            410                 415                 420

ggc gcc gag ctc ctg ccc gtc tac tcc aag agc ttc ttc aac gag cag     15211
Gly Ala Glu Leu Leu Pro Val Tyr Ser Lys Ser Phe Phe Asn Glu Gln
        425                 430                 435

gcc gtc tac tcg cag cag ctg cgc gcc ttc acc tcg ctc acg cac gtc     15259
Ala Val Tyr Ser Gln Gln Leu Arg Ala Phe Thr Ser Leu Thr His Val
    440                 445                 450

ttc aac cgc ttc ccc gag aac cag atc ctc gtt cgc ccg ccc gcg ccc     15307
Phe Asn Arg Phe Pro Glu Asn Gln Ile Leu Val Arg Pro Pro Ala Pro
455                 460                 465                 470

acc att acc acc gtc agt gaa aac gtt cct gct ctc aca gat cac ggg     15355
Thr Ile Thr Thr Val Ser Glu Asn Val Pro Ala Leu Thr Asp His Gly
                475                 480                 485

acc ctg ccg ctg cgc agc agt atc cgg gga gtc cag cgc gtg acc gtc     15403
Thr Leu Pro Leu Arg Ser Ser Ile Arg Gly Val Gln Arg Val Thr Val
            490                 495                 500

act gac gcc aga cgc cgc acc tgc ccc tac gtc tac aag gcc ctg ggc     15451
Thr Asp Ala Arg Arg Arg Thr Cys Pro Tyr Val Tyr Lys Ala Leu Gly
        505                 510                 515

gta gtc gcg ccg cgc gtc ctc tcg agc cgc acc ttc taa aaaatgtcca     15500
Val Val Ala Pro Arg Val Leu Ser Ser Arg Thr Phe
    520                 525                 530

ttctcatctc gcccagtaat aacaccggtt ggggcctgcg cgcgcccagc aagatgtacg 15560
gaggcgctcg ccaacgctcc acgcaacacc ccgtgcgcgt gcgcgggcac ttccgcgctc 15620
cctggggcgc cctcaagggc cgcgtgcgct cgcgcaccac cgtcgacgac gtgatcgacc 15680
aggtggtggc cgacgcgcgc aactacacgc ccgccgccgc gcccgtctcc accgtggacg 15740
ccgtcatcga cagcgtggtg gccgacgcgc gccggtacgc ccgcgccaag agccggcggc 15800
ggcgcatcgc ccggcggcac cggagcaccc ccgccatgcg cgcggcgcga gccttgctgc 15860
gcagggccag gcgcacggga cgcagggcca tgctcagggc ggccagacgc gcggcctccg 15920
gcagcagcag cgccggcagg acccgcagac gcgcggccac ggcggcggcg gcggccatcg 15980
ccagcatgtc ccgcccgcgg cgcggcaacg tgtactgggt gcgcgacgcc gccaccggtg 16040
tgcgcgtgcc cgtgcgcacc cgcccccctc gcacttgaag atgctgactt cgcgatgttg 16100
atgtgtccca gcggcgagga ggatgtccaa gcgcaaattc aaggaagaga tgctccaggt 16160
catcgcgcct gagatctacg gcccggcggc ggtgaaggag gaaagaaagc cccgcaaact 16220
gaagcgggtc aaaaaggaca aaaaggagga ggaagatgtg gacggactgg tggagtttgt 16280
gcgcgagttc gccccccggc ggcgcgtgca gtggcgcggg cggaaagtga aaccggtgct 16340
gcgacccggc accacggtgg tcttcacgcc cggcgagcgt tccggctccg cctccaagcg 16400
ctcctacgac gaggtgtacg gggacgagga catcctcgag caggcggccg aacgtctggg 16460
cgagtttgct tacggcaagc gcagccgccc cgcgcccttg aaagaggagg cggtgtccat 16520
cccgctggac cacggcaacc ccacgccgag cctgaagccg gtgaccctgc agcaggtgct 16580
gcctggtgcg gcgccgcgcc ggggcttcaa gcgcgagggc ggcgaggatc tgtacccgac 16640
catgcagctg atggtgccca agcgccagaa gctggaggac gtgctggagc acatgaaggt 16700
ggaccccgag gtgcagcccg aggtcaaggt gcggcccatc aagcaggtgg ccccgggcct 16760
gggcgtgcag accgtggaca tcaagatccc cacggagccc atggaaacgc agaccgagcc 16820
cgtgaagccc agcaccagca ccatggaggt gcagacggat ccctggatgc cggcaccggc 16880
ttccaccacc cgccgaagac gcaagtacgg cgcggccagc ctgctgatgc ccaactacgc 16940
gctgcatcct tccatcatcc ccacgccggg ctaccgcggc acgcgcttct accgcggcta 17000
caccagcagc cgccgccgca agaccaccac ccgccgccgc cgtcgtcgca cccgccgcag 17060
cagcaccgcg acttccgccg ccgccctggt gcggagagtg taccgcagcg ggcgcgagcc 17120
tctgaccctg ccgcgcgcgc gctaccaccc gagcatcgcc atttaactac cgcctcctac 17180
ttgcagatat ggccctcaca tgccgcctcc gcgtccccat tacgggctac cgaggaagaa 17240
agccgcgccg tagaaggctg acggggaacg ggctgcgtcg ccatcaccac cggcggcggc 17300
gcgccatcag caagcggttg gggggaggct tcctgcccgc gctgatgccc atcatcgccg 17360

cggcgatcgg ggcgatcccc ggcatagctt ccgtggcggt gcaggcctct cagcgccact 17420

gagacacagc ttggaaaatt tgtaataaaa aatggactga cgctcctggt cctgtgatgt 17480

gtgtttttag atggaagaca tcaatttttc gtccctggca ccgcgacacg gcacgcggcc 17540

gtttatgggc acctggagcg acatcggcaa cagccaactg aacgggggcg ccttcaattg 17600

gagcagtctc tggagcgggc ttaagaattt cgggtccacg ctcaaaacct atggcaacaa 17660

ggcgtggaac agcagcacag ggcaggcgct gagggaaaag ctgaaagagc agaacttcca 17720

gcagaaggtg gtcgatggcc tggcctcggg catcaacggg gtggtggacc tggccaacca 17780

ggccgtgcag aaacagatca acagccgcct ggacgcggtc ccgcccgcgg ggtccgtgga 17840

gatgccccag gtggaggagg agctgcctcc cctggacaag cgcggcgaca agcgaccgcg 17900

tcccgacgcg gaggagacgc tgctgacgca cacggacgag ccgcccccgt acgaggaggc 17960

ggtgaaactg ggtctgccca ccacgcggcc cgtggcgcct ctggccaccg gggtgctgaa 18020

acccagcagc agcagcagcc agcccgcgac cctggacttg cctccgcctg cttcccgccc 18080

ctccacagtg gctaagcccc tgccgccggt ggccgtcgcg tcgcgcgccc cccgaggccg 18140

cccccaggcg aactggcaga gcactctgaa cagcatcgtg ggtctgggag tgcagagtgt 18200

gaagcgccgc cgctgctatt aaaagacact gtagcgctta acttgcttgt ctgtgtgtat 18260

atgtatgtcc gccgaccaga aggaggagga agaggcgcgt cgccgagttg caag atg 18317
Met

gcc acc cca tcg atg ctg ccc cag tgg gcg tac atg cac atc gcc gga 18365
Ala Thr Pro Ser Met Leu Pro Gln Trp Ala Tyr Met His Ile Ala Gly
            535                 540                 545

cag gac gct tcg gag tac ctg agt ccg ggt ctg gtg cag ttc gcc cgc 18413
Gln Asp Ala Ser Glu Tyr Leu Ser Pro Gly Leu Val Gln Phe Ala Arg
        550                 555                 560

gcc aca gac acc tac ttc agt ctg ggg aac aag ttt agg aac ccc acg 18461
Ala Thr Asp Thr Tyr Phe Ser Leu Gly Asn Lys Phe Arg Asn Pro Thr
    565                 570                 575

gtg gcg ccc acg cac gat gtg acc acc gac cgc agc cag cgg ctg acg 18509
Val Ala Pro Thr His Asp Val Thr Thr Asp Arg Ser Gln Arg Leu Thr
580                 585                 590                 595

ctg cgc ttc gtg ccc gtg gac cgc gag gac aac acc tac tcg tac aaa 18557
Leu Arg Phe Val Pro Val Asp Arg Glu Asp Asn Thr Tyr Ser Tyr Lys
                600                 605                 610

gtg cgc tac acg ctg gcc gtg ggc gac aac cgc gtg ctg gac atg gcc 18605
Val Arg Tyr Thr Leu Ala Val Gly Asp Asn Arg Val Leu Asp Met Ala
            615                 620                 625

agc acc tac ttt gac atc cgc ggc gtg ctg gat cgg ggc cct agc ttc 18653
Ser Thr Tyr Phe Asp Ile Arg Gly Val Leu Asp Arg Gly Pro Ser Phe
        630                 635                 640

aaa ccc tac tcc ggc acc gct tac aac agc ctg gct ccc aag gga gcg 18701

Lys Pro Tyr Ser Gly Thr Ala Tyr Asn Ser Leu Ala Pro Lys Gly Ala
    645                 650                 655

ccc aac act tgc cag tgg aca tat aaa gct gat ggt gat act ggt aca 18749
Pro Asn Thr Cys Gln Trp Thr Tyr Lys Ala Asp Gly Asp Thr Gly Thr
660                 665                 670                 675

gaa aaa acc tat aca tat gga aat gcg cct gtg caa ggc att agt att 18797
Glu Lys Thr Tyr Thr Tyr Gly Asn Ala Pro Val Gln Gly Ile Ser Ile
                680                 685                 690

aca aaa gat ggt att caa ctt gga act gac act gat gat cag ccc att 18845
Thr Lys Asp Gly Ile Gln Leu Gly Thr Asp Thr Asp Asp Gln Pro Ile
            695                 700                 705

tat gca gat aaa act tat caa cca gag cct caa gtg ggt gat gct gaa 18893
Tyr Ala Asp Lys Thr Tyr Gln Pro Glu Pro Gln Val Gly Asp Ala Glu
        710                 715                 720

tgg cat gac atc act ggt act gat gaa aaa tat gga ggc aga gct ctc 18941
Trp His Asp Ile Thr Gly Thr Asp Glu Lys Tyr Gly Gly Arg Ala Leu
    725                 730                 735

aag cct gac acc aaa atg aag ccc tgc tat ggt tct ttt gcc aag cct 18989
Lys Pro Asp Thr Lys Met Lys Pro Cys Tyr Gly Ser Phe Ala Lys Pro
740                 745                 750                 755

acc aat aaa gaa gga ggt cag gca aat gtg aaa acc gaa aca ggc ggt 19037
Thr Asn Lys Glu Gly Gly Gln Ala Asn Val Lys Thr Glu Thr Gly Gly
                760                 765                 770

acc aaa gaa tat gac att gac atg gca ttc ttc gat aat cga agt gca 19085
Thr Lys Glu Tyr Asp Ile Asp Met Ala Phe Phe Asp Asn Arg Ser Ala
            775                 780                 785

gct gcg gct ggc ctg gcc cca gaa att gtt ttg tat act gag aat gtg 19133
Ala Ala Ala Gly Leu Ala Pro Glu Ile Val Leu Tyr Thr Glu Asn Val
        790                 795                 800

gat ctg gaa act cca gat act cat att gta tac aag gcg ggc aca gat 19181
Asp Leu Glu Thr Pro Asp Thr His Ile Val Tyr Lys Ala Gly Thr Asp
    805                 810                 815

gac agc agc tct tct atc aat ttg ggt cag cag tcc atg ccc aac aga 19229
Asp Ser Ser Ser Ser Ile Asn Leu Gly Gln Gln Ser Met Pro Asn Arg
820                 825                 830                 835

ccc aac tac att ggc ttt aga gac aac ttt atc ggg ctc atg tac tac 19277
Pro Asn Tyr Ile Gly Phe Arg Asp Asn Phe Ile Gly Leu Met Tyr Tyr
                840                 845                 850

aac agc act ggc aac atg ggc gtg ctg gct ggt cag gcc tcc cag ctg 19325
Asn Ser Thr Gly Asn Met Gly Val Leu Ala Gly Gln Ala Ser Gln Leu
            855                 860                 865

aat gct gtg gtg gac ttg cag gac aga aac act gaa ctg tcc tac cag 19373
Asn Ala Val Val Asp Leu Gln Asp Arg Asn Thr Glu Leu Ser Tyr Gln
        870                 875                 880

ctc ttg ctt gac tct ctg ggc gac aga acc agg tat ttc agt atg tgg 19421
Leu Leu Leu Asp Ser Leu Gly Asp Arg Thr Arg Tyr Phe Ser Met Trp
    885                 890                 895

aat cag gcg gtg gac agc tat gac ccc gat gtg cgc att att gaa aat     19469
Asn Gln Ala Val Asp Ser Tyr Asp Pro Asp Val Arg Ile Ile Glu Asn
900                 905                 910                 915

cac ggt gtg gag gat gaa ctc cct aac tat tgc ttc ccc ctg gat gct     19517
His Gly Val Glu Asp Glu Leu Pro Asn Tyr Cys Phe Pro Leu Asp Ala
                920                 925                 930

gtg ggt aga act gat act tac cag gga att aag gcc aat ggt gct gat     19565
Val Gly Arg Thr Asp Thr Tyr Gln Gly Ile Lys Ala Asn Gly Ala Asp
            935                 940                 945

caa acc acc tgg acc aaa gat gat act gtt aat gat gct aat gaa ttg     19613
Gln Thr Thr Trp Thr Lys Asp Asp Thr Val Asn Asp Ala Asn Glu Leu
        950                 955                 960

ggc aag ggc aat cct ttc gcc atg gag atc aac atc cag gcc aac ctg     19661
Gly Lys Gly Asn Pro Phe Ala Met Glu Ile Asn Ile Gln Ala Asn Leu
    965                 970                 975

tgg cgg aac ttc ctc tac gcg aac gtg gcg ctg tac ctg ccc gac tcc     19709
Trp Arg Asn Phe Leu Tyr Ala Asn Val Ala Leu Tyr Leu Pro Asp Ser
980                 985                 990                 995

tac aag tac acg ccg  gcc aac atc acg ctg  ccg acc aac acc aac     19754
Tyr Lys Tyr Thr Pro  Ala Asn Ile Thr Leu  Pro Thr Asn Thr Asn
                1000                 1005                 1010

acc tac gat tac atg  aac ggc cgc gtg gtg  gcg ccc tcg ctg gtg     19799
Thr Tyr Asp Tyr Met  Asn Gly Arg Val Val  Ala Pro Ser Leu Val
                1015                 1020                 1025

gac gcc tac atc aac  atc ggg gcg cgc tgg  tcg ctg gac ccc atg     19844
Asp Ala Tyr Ile Asn  Ile Gly Ala Arg Trp  Ser Leu Asp Pro Met
                1030                 1035                 1040

gac aac gtc aac ccc  ttc aac cac cac cgc  aac gcg ggc ctg cgc     19889
Asp Asn Val Asn Pro  Phe Asn His His Arg  Asn Ala Gly Leu Arg
                1045                 1050                 1055

tac cgc tcc atg ctc  ctg ggc aac ggg cgc  tac gtg ccc ttc cac     19934
Tyr Arg Ser Met Leu  Leu Gly Asn Gly Arg  Tyr Val Pro Phe His
                1060                 1065                 1070

atc cag gtg ccc caa  aag ttc ttc gcc atc  aag agc ctc ctg ctc     19979
Ile Gln Val Pro Gln  Lys Phe Phe Ala Ile  Lys Ser Leu Leu Leu
                1075                 1080                 1085

ctg ccc ggg tcc tac  acc tac gag tgg aac  ttc cgc aag gac gtc     20024
Leu Pro Gly Ser Tyr  Thr Tyr Glu Trp Asn  Phe Arg Lys Asp Val
                1090                 1095                 1100

aac atg atc ctg cag  agc tcc ctc ggc aac  gac ctg cgc acg gac     20069
Asn Met Ile Leu Gln  Ser Ser Leu Gly Asn  Asp Leu Arg Thr Asp
                1105                 1110                 1115

ggg gcc tcc atc gcc  ttc acc agc atc aac  ctc tac gcc acc ttc     20114
Gly Ala Ser Ile Ala  Phe Thr Ser Ile Asn  Leu Tyr Ala Thr Phe
                1120                 1125                 1130

ttc ccc atg gcg cac  aac acc gcc tcc acg  ctc gag gcc atg ctg     20159
Phe Pro Met Ala His  Asn Thr Ala Ser Thr  Leu Glu Ala Met Leu
                1135                 1140                 1145

cgc aac gac acc aac gac cag tcc ttc aac gac tac ctc tcg gcg 20204
Arg Asn Asp Thr Asn Asp Gln Ser Phe Asn Asp Tyr Leu Ser Ala
                1150                1155                1160

gcc aac atg ctc tac ccc atc ccg gcc aac gcc acc aac gtg ccc 20249
Ala Asn Met Leu Tyr Pro Ile Pro Ala Asn Ala Thr Asn Val Pro
                1165                1170                1175

atc tcc atc ccc tcg cgc aac tgg gcc gcc ttc cgc gga tgg tcc 20294
Ile Ser Ile Pro Ser Arg Asn Trp Ala Ala Phe Arg Gly Trp Ser
                1180                1185                1190

ttc acg cgc ctc aag acc cgc gag acg ccc tcg ctc ggc tcc ggg 20339
Phe Thr Arg Leu Lys Thr Arg Glu Thr Pro Ser Leu Gly Ser Gly
                1195                1200                1205

ttc gac ccc tac ttc gtc tac tcg ggc tcc atc ccc tac ctc gac 20384
Phe Asp Pro Tyr Phe Val Tyr Ser Gly Ser Ile Pro Tyr Leu Asp
                1210                1215                1220

ggc acc ttc tac ctc aac cac acc ttc aag aag gtc tcc atc acc 20429
Gly Thr Phe Tyr Leu Asn His Thr Phe Lys Lys Val Ser Ile Thr
                1225                1230                1235

ttc gac tcc tcc gtc agc tgg ccc ggc aac gac cgc ctc ctg acg 20474
Phe Asp Ser Ser Val Ser Trp Pro Gly Asn Asp Arg Leu Leu Thr
                1240                1245                1250

ccc aac gag ttc gaa atc aag cgc acc gtc gac gga gag ggg tac 20519
Pro Asn Glu Phe Glu Ile Lys Arg Thr Val Asp Gly Glu Gly Tyr
                1255                1260                1265

aac gtg gcc cag tgc aac atg acc aag gac tgg ttc ctg gtc cag 20564
Asn Val Ala Gln Cys Asn Met Thr Lys Asp Trp Phe Leu Val Gln
                1270                1275                1280

atg ctg gcc cac tac aac atc ggc tac cag ggc ttc tac gtg ccc 20609
Met Leu Ala His Tyr Asn Ile Gly Tyr Gln Gly Phe Tyr Val Pro
                1285                1290                1295

gag ggc tac aag gac cgc atg tac tcc ttc ttc cgc aac ttc cag 20654
Glu Gly Tyr Lys Asp Arg Met Tyr Ser Phe Phe Arg Asn Phe Gln
                1300                1305                1310

ccc atg agc cgc cag gtc gtg gac gag gtc aac tac aag gac tac 20699
Pro Met Ser Arg Gln Val Val Asp Glu Val Asn Tyr Lys Asp Tyr
                1315                1320                1325

cag gcc gtc acc ctg gcc tac cag cac aac aac tcg ggc ttc gtc 20744
Gln Ala Val Thr Leu Ala Tyr Gln His Asn Asn Ser Gly Phe Val
                1330                1335                1340

ggc tac ctc gcg ccc acc atg cgc cag gga cag ccc tac ccc gcc 20789
Gly Tyr Leu Ala Pro Thr Met Arg Gln Gly Gln Pro Tyr Pro Ala
                1345                1350                1355

aac tac ccc tac ccg ctc atc ggc aag agc gcc gtc gcc agc gtc 20834
Asn Tyr Pro Tyr Pro Leu Ile Gly Lys Ser Ala Val Ala Ser Val
                1360                1365                1370

acc cag aaa aag ttc ctc tgc gac cgg gtc atg tgg cgc atc ccc 20879
Thr Gln Lys Lys Phe Leu Cys Asp Arg Val Met Trp Arg Ile Pro

1375                1380                1385

ttc tcc agc aac ttc atg tcc atg ggc gcg ctc acc gac ctc ggc      20924
Phe Ser Ser Asn Phe Met Ser Met Gly Ala Leu Thr Asp Leu Gly
                1390                1395                1400

cag aac atg ctc tac gcc aac tcc gcc cac gcg cta gac atg aat      20969
Gln Asn Met Leu Tyr Ala Asn Ser Ala His Ala Leu Asp Met Asn
                1405                1410                1415

ttc gaa gtc gac ccc atg gat gag tcc acc ctt ctc tat gtt gtc      21014
Phe Glu Val Asp Pro Met Asp Glu Ser Thr Leu Leu Tyr Val Val
                1420                1425                1430

ttc gaa gtc ttc gac gtc gtc cga gtg cac cag ccc cac cgc ggc      21059
Phe Glu Val Phe Asp Val Val Arg Val His Gln Pro His Arg Gly
                1435                1440                1445

gtc atc gag gcc gtc tac ctg cgc acg ccc ttc tcg gcc ggc aac      21104
Val Ile Glu Ala Val Tyr Leu Arg Thr Pro Phe Ser Ala Gly Asn
                1450                1455                1460

gcc acc acc taa gccccgctct tgcttcttgc aagatgacgg cctgtgcggg      21156
Ala Thr Thr

ctccggcgag caggagctca gggccatcct ccgcgacctg ggctgcgggc cctgcttcct   21216

gggcaccttc gacaagcgct tcccgggatt catggccccg cacaagctgg cctgcgccat   21276

cgtcaacacg gccggccgcg agaccggggg cgagcactgg ctggccttcg cctggaaccc   21336

gcgctcccac acctgctacc tcttcgaccc cttcgggttc tcggacgagc gcctcaagca   21396

gatctaccag ttcgagtacg agggcctgct gcgccgcagc gccctggcca ccgaggaccg   21456

ctgcgtcacc ctggaaaagt ccacccagac cgtgcagggt ccgcgctcgg ccgcctgcgg   21516

gctcttctgc tgcatgttcc tgcacgcctt cgtgcactgg cccgaccgcc ccatggacaa   21576

gaaccccacc atgaacttgc tgacgggggt gcccaacggc atgctccagt cgccccaggt   21636

ggaacccacc ctgcgccgca accaggaggc gctctaccgc ttcctcaacg cccactccgc   21696

ctactttcgc tcccaccgcg cgcgcatcga gaaggccacc gccttcgacc gcatgaatca   21756

agacatgtaa accgtgtgtg tatgtgaatg ctttattcat aataaacagc acatgtttat   21816

gccacctttt ctgaggctct gactttattt agaaatcgaa ggggttctgc cggctctcgg   21876

cgtgccccgc gggcagggat acgttgcgga actggtactt gggcagccac ttgaactcgg   21936

ggatcagcag cttcggcacg gggaggtcgg ggaacgagtc gctccacagc ttgcgcgtga   21996

gttgcagggc gcccagcagg tcgggcgcgg agatcttgaa atcgcagttg ggacccgcgt   22056

tctgcgcgcg ggagttgcgg tacacggggt tgcagcactg gaacaccatc agggccgggt   22116

gcttcacgct cgccagcacc gtcgcgtcgg tgatgccctc cacgtccaga tcctcggcgt   22176

tggccatccc gaagggggtc atcttgcagg tctgccgccc catgctgggc acgcagccgg   22236

gcttgtggtt gcaatcgcag tgcaggggga tcagcatcat ctgggcctgc tcggagctca   22296

tgcccgggta catggccttc atgaaagcct ccagctggcg gaaggcctgc tgcgccttgc 22356
cgccctcggt gaagaagacc ccgcaggact tgctagagaa ctggttggtg gcgcagccgg 22416
cgtcgtgcac gcagcagcgc gcgtcgttgt tggccagctg caccacgctg cgcccccagc 22476
ggttctgggt gatcttggcc cggtcggggt tctccttcag cgcgcgctgc ccgttctcgc 22536
tcgccacatc catctcgatc gtgtgctcct tctggatcat cacggtcccg tgcaggcatc 22596
gcagcttgcc ctcggcctcg gtgcacccgt gcagccacag cgcgcagccg gtgcactccc 22656
agttcttgtg ggcgatctgg gagtgcgagt gcacgaagcc ctgcaggaag cggcccatca 22716
tcgtggtcag ggtcttgttg ctggtgaagg tcagcgggat gccgcggtgc tcctcgttca 22776
catacaggtg gcagatgcgg cggtacacct cgccctgctc gggcatcagc tggaaggcgg 22836
acttcaggtc gctctccacg cggtaccggt ccatcagcag cgtcatgact tccatgccct 22896
tctcccaggc cgagacgatc ggcaggctca gggggttctt caccgccgtt gtcatcttag 22956
tcgccgccgc tgaggtcagg gggtcgttct cgtccagggt ctcaaacact cgcttgccgt 23016
ccttctcggt gatgcgcacg gggggaaagc tgaagcccac ggccgccagc tcctcctcgg 23076
cctgcctttc gtcctcgctg tcctggctga tgtcttgcaa aggcacatgc ttggtcttgc 23136
ggggtttctt tttgggcggc agaggcggcg gcggagacgt gctgggcgag cgcgagttct 23196
cgctcaccac gactatttct tcttcttggc cgtcgtccga gaccacgcgg cggtaggcat 23256
gcctcttctg gggcagaggc ggaggcgacg ggctctcgcg gttcggcggg cggctggcag 23316
agccccttcc gcgttcgggg gtgcgctcct ggcggcgctg ctctgactga cttcctccgc 23376
ggccggccat tgtgttctcc tagggagcaa caagcatgga gactcagcca tcgtcgccaa 23436
catcgccatc tgcccccgcc gccgccgacg agaaccagca gcagaatgaa agcttaaccg 23496
ccccgccgcc cagccccacc tccgacgccg ccgcggcccc agacatgcaa gagatggagg 23556
aatccatcga gattgacctg ggctacgtga cgcccgcgga gcacgaggag gagctggcag 23616
cgcgcttttc agccccggaa gagaaccacc aagagcagcc agagcaggaa gcagagagcg 23676
agcagcagca ggctgggctc gagcatggcg actacctgag cggggcagag gacgtgctca 23736
tcaagcatct ggcccgccaa tgcatcatcg tcaaggacgc gctgctcgac cgcgccgagg 23796
tgcccctcag cgtggcggag ctcagccgcg cctacgagcg caacctcttc tcgccgcgcg 23856
tgccccccaa gcgccagccc aacggcacct gcgagcccaa cccgcgcctc aacttctacc 23916
cggtcttcgc ggtgcccgag gccctggcca cctaccacct ctttttcaag aaccaaagga 23976
tccccgtctc ctgccgcgcc aaccgcaccc gcgccgacgc cctgctcaac ctgggtcccg 24036
gcgcccgcct acctgatatc gcctccttgg aagaggttcc caagatcttc gagggtctgg 24096
gcagcgacga gactcgggcc gcgaacgctc tgcaaggaag cggagaggag catgagcacc 24156

acagcgccct ggtggagttg gaaggcgaca acgcgcgcct ggcggtgctc aagcgcacgg 24216
tcgagctgac ccacttcgcc tacccggcgc tcaacctgcc ccccaaggtc atgagcgccg 24276
tcatggacca ggtgctcatc aagcgcgcct cgcccctctc ggatgaggac atgcaggacc 24336
ccgagagctc ggacgagggc aagcccgtgg tcagcgacga gcagctggcg cgctggctgg 24396
gagcgagtag caccccccag agcttggaag agcggcgcaa gctcatgatg gccgtggtcc 24456
tggtgaccgt ggagctggag tgtctgcgcc gcttcttcgc cgacgcagag accctgcgca 24516
aggtcgagga gaacctgcac tacctcttca ggcacgggtt tgtgcgccag gcctgcaaga 24576
tctccaacgt ggagctgacc aacctggtct cctacatggg catcctgcac gagaaccgcc 24636
tggggcagaa cgtgctgcac accaccctgc gcggggaggc ccgccgcgac tacatccgcg 24696
actgcgtcta cctgtacctc tgccacacct ggcagacggg catgggcgtg tggcagcagt 24756
gcctggagga gcagaacctg aaagagctct gcaagctcct gcagaagaac ctgaaggccc 24816
tgtggaccgg gttcgacgag cgcaccaccg cctcggacct ggccgacctc atcttccccg 24876
agcgcctgcg gctgacgctg cgcaacggac tgcccgactt tatgagtcaa agcatgttgc 24936
aaaactttcg ctctttcatc ctcgaacgct ccgggatcct gcccgccacc tgctccgcgc 24996
tgccctcgga cttcgtgccg ctgaccttcc gcgagtgccc cccgccgctc tggagccact 25056
gctacctgct gcgcctggcc aactacctgg cctaccactc ggacgtgatc gaggacgtca 25116
gcggcgaggg tctgctcgag tgccactgcc gctgcaacct ctgcacgccg caccgctccc 25176
tggcctgcaa cccccagctg ctgagcgaga cccagatcat cggcaccttc gagttgcaag 25236
gccccggcga gggcaagggg ggtctgaaac tcaccccggg gctgtggacc tcggcctact 25296
tgcgcaagtt cgtgcccgag gactaccatc ccttcgagat caggttctac gaggaccaat 25356
cccagccgcc caaggccgaa ctgtcggcct gcgtcatcac ccagggggcc atcctggccc 25416
aattgcaagc catccagaaa tcccgccaag aatttctgct gaaaaagggc cacggggtct 25476
acctggaccc ccagaccgga gaggagctca accccagctt cccccaggat gccccgagga 25536
agcagcaaga agctgaaagt ggagctgccg ccgccggagg atttggagga agactgggag 25596
agcagtcagg cagaggagga ggagatggaa gactgggaca gcactcaggc agaggaggac 25656
agcctgcaag acagtctgga agacgaggtg gaggaggagg cagaggaaga agcagccgcc 25716
gccagaccgt cgtcctcggc ggagaaagca agcagcacgg ataccatctc cgctccgggt 25776
cggggtcgcg gcgaccgggc ccacagtagg tgggacgaga ccgggcgctt cccgaacccc 25836
accacccaga ccggtaagaa ggagcggcag ggatacaagt cctggcgggg gcacaaaaac 25896
gccatcgtct cctgcttgca agcctgcggg ggcaacatct ccttcacccg ccgctacctg 25956
ctcttccacc gcggggtgaa cttcccccgc aacatcttgc attactaccg tcacctccac 26016
agcccctact actgtttcca agaagaggca gaaacccagc agcagcagaa aaccagcggc 26076

agcagcagct agaaaatcca cagcggcggc aggtggactg aggatcgcag cgaacgagcc 26136
ggcgcagacc cgggagctga ggaaccggat ctttcccacc ctctatgcca tcttccagca 26196
gagtcggggg caggagcagg aactgaaagt caagaaccgt tctctgcgct cgctcacccg 26256
cagttgtctg tatcacaaga gcgaagacca acttcagcgc actctcgagg acgccgaggc 26316
tctcttcaac aagtactgcg cgctcactct taaagagtag cccgcgcccg cccacacacg 26376
gaaaaaggcg ggaattacgt caccacctgc gcccttcgcc cgaccatcat catgagcaaa 26436
gagattccca cgccttacat gtggagctac cagccccaga tgggcctggc cgccggcgcc 26496
gcccaggact actccacccg catgaactgg ctcagcgccg ggcccgcgat gatctcacgg 26556
gtgaatgaca tccgcgcccg ccgaaaccag atactcctag aacagtcagc gatcaccgcc 26616
acgccccgcc atcaccttaa tccgcgtaat tggcccgccg ccctggtgta ccaggaaatt 26676
ccccagccca cgaccgtact acttccgcga gacgcccagg ccgaagtcca gctgactaac 26736
tcaggtgtcc agctggccgg cggcgccgcc ctgtgtcgtc accgccccgc tcagggtata 26796
aagcggctgg tgatccgagg cagaggcaca cagctcaacg acgaggtggt gagctcttcg 26856
ctgggtctgc gacctgacgg agtcttccaa ctcgccggat cggggagatc ttccttcacg 26916
cctcgtcagg ccgtcctgac tttggagagt tcgtcctcgc agccccgctc gggtggcatc 26976
ggcactctcc agttcgtgga ggagttcact ccctcggtct acttcaaccc cttctccggc 27036
tcccccggcc actacccgga cgagttcatc ccgaacttcg acgccatcag cgagtcggtg 27096
gacggctacg attgaatgtc ccatggtggc gcagctgacc tagctcggct tcgacacctg 27156
gaccactgcc gccgcttccg ctgcttcgct cgggatctcg ccgagtttgc ctactttgag 27216
ctgcccgagg agcaccctca gggcccggcc cacggagtgc ggatcatcgt cgaagggggc 27276
ctcgactccc acctgcttcg gatcttcagc cagcgaccga tcctggtcga gcgcgagcaa 27336
ggacagaccc ttctgaccct gtactgcatc tgcaaccacc ccggcctgca tgaaagtctt 27396
tgttgtctgc tgtgtactga gtataataaa agctgagatc agcgactact ccggactcga 27456
ttgtggtgtt cctgctatca accggtccct gttcttcacc gggaacgaga ccgagctcca 27516
gcttcagtgt aagccccaca agaagtacct cacctggctg ttccagggct ccccgatcgc 27576
cgttgtcaac cactgcgaca acgacggagt cctgctgagc ggccccgcca accttacttt 27636
ttccacccgc agaagcaagc tccagctctt ccaacccttc ctccccggga cctatcagtg 27696
cgtctcggga ccctgccatc acaccttcca cctgatcccg aataccacag cgccgctccc 27756
cgctactaac aaccaaacta cccaccatcg ccaccgtcgc gacctttctg aatctaacac 27816
taccacccac accggaggtg agctccgagg tcgaccaacc tctgggattt actacggccc 27876
ctgggaggtg gtggggttaa tagcgctagg cctagttgtg ggtgggcttt tggctctctg 27936

ctacctatac ctcccttgct gttcgtactt agtggtgctg tgttgctggt ttaagaaatg 27996
gggaagatca ccctagtgag ctgcggtgcg ctggtggcgg tggtggtgtt ttcgattgtg 28056
ggactgggcg gcgcggctgt agtgaaggag aaggccgatc cctgcttgca tttcaatccc 28116
gacaattgcc agctgagttt tcagcccgat ggcaatcggt gcgcggtgct gatcaagtgc 28176
ggatgggaat gcgagaacgt gagaatcgag tacaataaca agactcggaa caatactctc 28236
gcgtccgtgt ggcagcccgg ggaccccgag tggtacaccg tctctgtccc cggtgctgac 28296
ggctccccgc gcaccgtgaa caatactttc atttttgcgc acatgtgcga cacggtcatg 28356
tggatgagca agcagtacga tatgtggccc cccacgaagg agaacatcgt ggtcttctcc 28416
atcgcttaca gcgcgtgcac ggcgctaatc accgctatcg tgtgcctgag cattcacatg 28476
ctcatcgcta ttcgccccag aaataatgcc gaaaaagaga aacagccata acacgttttt 28536
tcacacacct ttttcagacc atggcctctg ttaaattttt gcttttattt gccagtctca 28596
ttactgttat aagtaatgag aaactcacta tttacattgg cactaaccac actttagacg 28656
gaattccaaa atcctcatgg tattgctatt ttgatcaaga tccagactta actatagaac 28716
tgtgtggtaa caagggaaaa aatacaagca ttcatttaat taactttaat tgcggagaca 28776
atttgaaatt aattaatatc actaaagagt atggaggtat gtattactat gttgcagaaa 28836
ataacaacat gcagttttat gaagttactg taactaatcc caccacacct agaacaacaa 28896
caaccaccac cacaaaaact acacctgtta ccactatgca gctcactacc aataacattt 28956
ttgccatgcg tcaaatggtc aacaatagca ctcaacccac cccacccagt gaggaaattc 29016
ccaaatccat gattggcatt attgttgctg tagtggtgtg catgttgatc atcgccttgt 29076
gcatggtgta ctatgccttc tgctacagaa agcacagact gaacgacaag ctggaacact 29136
tactaagtgt tgaattttaa ttttttagaa ccatgaagat cctaggcctt ttaatttttt 29196
ctatcattac ctctgctcta tgcaattctg acaatgagga cgttactgtc gttgtcggaa 29256
ccaattatac actgaaaggt ccagcgaagg gtatgctttc gtggtattgc tggtttggaa 29316
ctgacgagca acagacagag ctctgcaatg ctcaaaaagg caaaacctca aattctaaaa 29376
tctctaatta tcaatgcaat ggcactgact tagtactgct caatgtcacg aaagcatatg 29436
ctggcagcta cacctgccct ggagatgata ctgagaacat gattttttac aaagtggaag 29496
tggttgatcc cactactcca cctccaccca ccacaactac tcacaccaca cacacagaac 29556
aaaccacagc agaggaggca gcaaagttag ccttgcaggt ccaagacagt tcatttgttg 29616
gcattacccc tacacctgat cagcggtgtc cggggctgct cgtcagcggc attgtcggtg 29676
tgctttcggg attagcagtc ataatcatct gcatgttcat ttttgcttgc tgctatagaa 29736
ggctttaccg acaaaaatca gacccactgc tgaacctcta tgtttaattt tttccagagc 29796
catgaaggca gttagcactc tagttttttg ttctttgatt ggcactgttt ttagtgttag 29856

ctttttgaaa caaatcaatg ttactgaggg ggaaaatgtg acactggtag gcgtagaggg 29916
tgctcaaaat accacctgga caaaattcca tctagatggg tggaaagaaa tttgcacctg 29976
gaatgtcagt acttatacat gtgaaggagt taatcttacc attgtcaatg tcagccaaat 30036
tcaaaagggt tggattaaag ggcaatctgt tagtgttagc aatagtgggt actataccca 30096
gcatactctt atctatgaca ttatagttat accactgcct acacctagcc cacctagcac 30156
taccacacag acaacccaca ctacacaaac aaccacatac agtacatcaa atcagcctac 30216
caccactaca acagcagagg ttgccagctc gtctggggtc cgagtggcat ttttgatgtt 30276
ggccccatct agcagtccca ctgctagtac caatgagcag actactgaat ttttgtccac 30336
tgtcgagagc cacaccacag ctacctcgag tgccttctct agcaccgcca atctatcctc 30396
gctttcctct acaccaatca gtcccgctac tactcctacc cccgctattc tccccactcc 30456
cctgaagcaa acagacggcg acatgcaatg gcagatcacc ctgctcattg tgatcgggtt 30516
ggtcatcctg gccgtgttgc tctactacat cttctgccgc cgcattccca acgcgcaccg 30576
caagccggcc tacaagccca tcgttgtcgg gcagccggag ccgcttcagg tggaaggggg 30636
tctaaggaat cttctcttct cttttacagt atggtgattg aattatgatt cctagacaaa 30696
tcttgatcac tattcttatc tgcctcctcc aagtctgtgc caccctcgct ctggtggcca 30756
acgccagtcc agactgtatt gggcccttcg cctcctacgt gctctttgcc ttcatcacct 30816
gcatctgctg ctgtagcata gtctgcctgc ttatcacctt cttccagttc attgactgga 30876
tctttgtgcg catcgcctac ctgcgccacc acccccagta ccgcgaccag cgagtggcgc 30936
ggctgctcag gatcctctga taagcatgcg ggctctgcta cttctcgcgc ttctgctgtt 30996
agtgctcccc cgtcccgtcg acccccggac ccccacccag tcccccgagg aggtccgcaa 31056
atgcaaattc caagaaccct ggaaattcct caaatgctac cgccaaaaat cagacatgca 31116
tcccagctgg atcatgatca ttgggatcgt gaacattctg gcctgcaccc tcatctcctt 31176
tgtgatttac ccctgctttg actttggttg gaactcgcca gaggcgctct atctcccgcc 31236
tgaacctgac acaccaccac agcaacctca ggcacacgca ctaccaccac caccacagcc 31296
taggccacaa tacatgccca tattagacta tgaggccgag ccacagcgac ccatgctccc 31356
cgctattagt tacttcaatc taaccggcgg agatgactga cccactggcc aacaacaacg 31416
tcaacgacct tctcctggac atggacggcc gcgcctcgga gcagcgactc gcccaacttc 31476
gcattcgcca gcagcaggag agagccgtca aggagctgca ggacggcata gccatccacc 31536
agtgcaagaa aggcatcttc tgcctggtga aacaggccaa gatctcctac gaggtcaccc 31596
agaccgacca tcgcctctcc tacgagctcc tgcagcagcg ccagaagttc acctgcctgg 31656
tcggagtcaa ccccatcgtc atcacccagc agtcgggcga taccaagggg tgcatccact 31716

gctcctgcga ctcccccgac tgcgtccaca ctctgatcaa gaccctctgc ggcctccgcg 31776

acctcctccc catgaactaa tcaccccctt atccagtgaa ataaagatca tattgatgat 31836

ttgagtttaa taaaaataaa gaatcactta cttgaaatct gataccaggt ctctgtccat 31896

gttttctgcc aacaccactt cactcccctc ttcccagctc tggtactgca ggccccggcg 31956

ggctgcaaac ttcctccaca ccctgaaggg gatgtcaaat tcctcctgtc cctcaatctt 32016

cattttatct tctatcag atg tcc aaa aag cgc gtc cgg gtg gat gat gac 32067
Met Ser Lys Lys Arg Val Arg Val Asp Asp Asp
    1465                1470

ttc gac ccc gtc tac ccc tac gat gca gac aac gca ccg acc gtg 32112
Phe Asp Pro Val Tyr Pro Tyr Asp Ala Asp Asn Ala Pro Thr Val
1475                1480                1485

ccc ttc atc aac ccc ccc ttc gtc tct tca gat gga ttc caa gag 32157
Pro Phe Ile Asn Pro Pro Phe Val Ser Ser Asp Gly Phe Gln Glu
1490                1495                1500

aag ccc ctg ggg gtg ctg tcc ctg cgt ctg gcc gat ccc gtc acc 32202
Lys Pro Leu Gly Val Leu Ser Leu Arg Leu Ala Asp Pro Val Thr
1505                1510                1515

acc aag aac ggg gaa atc acc ctc aag ctg gga gat ggg gtg gac 32247
Thr Lys Asn Gly Glu Ile Thr Leu Lys Leu Gly Asp Gly Val Asp
1520                1525                1530

ctc gac tcc tcg gga aaa ctc atc tcc aac acg gcc acc aag gcc 32292
Leu Asp Ser Ser Gly Lys Leu Ile Ser Asn Thr Ala Thr Lys Ala
1535                1540                1545

gcc gcc cct ctc agt ttt tcc aac aac acc att tcc ctt aac atg 32337
Ala Ala Pro Leu Ser Phe Ser Asn Asn Thr Ile Ser Leu Asn Met
1550                1555                1560

gat acc cct ttt tac aac aac aat gga aag tta ggc atg aaa gtc 32382
Asp Thr Pro Phe Tyr Asn Asn Asn Gly Lys Leu Gly Met Lys Val
1565                1570                1575

act gct cca ctg aag ata cta gac aca gac ttg cta aaa aca ctt 32427
Thr Ala Pro Leu Lys Ile Leu Asp Thr Asp Leu Leu Lys Thr Leu
1580                1585                1590

gtt gta gct tat gga caa ggt tta gga aca aac acc act ggt gcc 32472
Val Val Ala Tyr Gly Gln Gly Leu Gly Thr Asn Thr Thr Gly Ala
1595                1600                1605

ctt gtt gcc caa cta gca tcc cca ctt gct ttt gat agc aat agc 32517
Leu Val Ala Gln Leu Ala Ser Pro Leu Ala Phe Asp Ser Asn Ser
1610                1615                1620

aaa att gcc ctt aat tta ggc aat gga cca ttg aaa gtg gat gca 32562
Lys Ile Ala Leu Asn Leu Gly Asn Gly Pro Leu Lys Val Asp Ala
1625                1630                1635

aat aga ctg aac atc aat tgc aat aga gga ctc tat gtt act acc 32607
Asn Arg Leu Asn Ile Asn Cys Asn Arg Gly Leu Tyr Val Thr Thr
1640                1645                1650

aca aaa gat gca ctg gaa gcc aat ata agt tgg gct aat gct atg 32652

Thr Lys Asp Ala Leu Glu Ala Asn Ile Ser Trp Ala Asn Ala Met
1655                1660                1665

aca ttt ata gga aat gcc atg ggt gtc aat att gat aca caa aaa 32697
Thr Phe Ile Gly Asn Ala Met Gly Val Asn Ile Asp Thr Gln Lys
1670                1675                1680

ggc ttg caa ttt ggc acc act agt acc gtc gca gat gtt aaa aac 32742
Gly Leu Gln Phe Gly Thr Thr Ser Thr Val Ala Asp Val Lys Asn
1685                1690                1695

gct tac ccc ata caa atc aaa ctt gga gct ggt ctc aca ttt gac 32787
Ala Tyr Pro Ile Gln Ile Lys Leu Gly Ala Gly Leu Thr Phe Asp
1700                1705                1710

agc aca ggt gca att gtt gca tgg aac aaa gat gat gac aag ctt 32832
Ser Thr Gly Ala Ile Val Ala Trp Asn Lys Asp Asp Asp Lys Leu
1715                1720                1725

aca cta tgg acc aca gcc gac ccc tct cca aat tgt cac ata tat 32877
Thr Leu Trp Thr Thr Ala Asp Pro Ser Pro Asn Cys His Ile Tyr
1730                1735                1740

tct gaa aag gat gct aag ctt aca ctt tgc ttg aca aag tgt ggc 32922
Ser Glu Lys Asp Ala Lys Leu Thr Leu Cys Leu Thr Lys Cys Gly
1745                1750                1755

agt cag att ctg ggc act gtt tcc ctc ata gct gtt gat act ggc 32967
Ser Gln Ile Leu Gly Thr Val Ser Leu Ile Ala Val Asp Thr Gly
1760                1765                1770

agt tta aat ccc ata aca gga aca gta acc act gct ctt gtc tca 33012
Ser Leu Asn Pro Ile Thr Gly Thr Val Thr Thr Ala Leu Val Ser
1775                1780                1785

ctt aaa ttc gat gca aat gga gtt ttg caa agc agc tca aca cta 33057
Leu Lys Phe Asp Ala Asn Gly Val Leu Gln Ser Ser Ser Thr Leu
1790                1795                1800

gac tca gac tat tgg aat ttc aga cag gga gat gtt aca cct gct 33102
Asp Ser Asp Tyr Trp Asn Phe Arg Gln Gly Asp Val Thr Pro Ala
1805                1810                1815

gaa gcc tat act aat gct ata ggt ttc atg ccc aat cta aaa gca 33147
Glu Ala Tyr Thr Asn Ala Ile Gly Phe Met Pro Asn Leu Lys Ala
1820                1825                1830

tac cct aaa aac aca agt gga gct gca aaa agt cac att gtt ggg 33192
Tyr Pro Lys Asn Thr Ser Gly Ala Ala Lys Ser His Ile Val Gly
1835                1840                1845

aaa gtg tac cta cat ggg gat aca ggc aaa cca ctg gac ctc att 33237
Lys Val Tyr Leu His Gly Asp Thr Gly Lys Pro Leu Asp Leu Ile
1850                1855                1860

att act ttc aat gaa aca agt gat gaa tct tgc act tac tgt att 33282
Ile Thr Phe Asn Glu Thr Ser Asp Glu Ser Cys Thr Tyr Cys Ile
1865                1870                1875

aac ttt caa tgg cag tgg ggg gct gat caa tat aaa aat gaa aca 33327
Asn Phe Gln Trp Gln Trp Gly Ala Asp Gln Tyr Lys Asn Glu Thr
1880                1885                1890

ctt gcc gtc agt tca ttc acc ttt tcc tat att gct aaa gaa taa 33372
Leu Ala Val Ser Ser Phe Thr Phe Ser Tyr Ile Ala Lys Glu
1895                1900                1905

accccactct gtaccccatc tctgtctatg gaaaaaactc tgaaacacaa aataaaataa 33432

agttcaagtg ttttattgat tcaacagttt tacaggattc gagcagttat ttttcctcca 33492

ccctcccagg acatggaata caccaccctc tccccccgca cagccttgaa catctgaatg 33552

ccattggtga tggacatgct tttggtctcc acgttccaca cagtttcaga gcgagccagt 33612

ctcgggtcgg tcagggagat gaaaccctcc gggcactccc gcatctgcac ctcacagctc 33672

aacagctgag gattgtcctc ggtggtcggg atcacggtta tctggaagaa gcagaagagc 33732

ggcggtggga atcatagtcc gcgaacggga tcggccggtg gtgtcgcatc aggccccgca 33792

gcagtcgctg tcgccgccgc tccgtcaagc tgctgctcag ggggtccggg tccagggact 33852

ccctcagcat gatgcccacg gccctcagca tcagtcgtct ggtgcggcgg gcgcagcagc 33912

gcatgcggat ctcgctcagg tcgctgcagt acgtgcaaca caggaccacc aggttgttca 33972

acagtccata gttcaacacg ctccagccga aactcatcgc gggaaggatg ctacccacgt 34032

ggccgtcgta ccagatcctc aggtaaatca agtggcgccc cctccagaac acgctgccca 34092

tgtacatgat ctccttgggc atgtggcggt tcaccacctc ccggtaccac atcaccctct 34152

ggttgaacat gcagccccgg atgatcctgc ggaaccacag ggccagcacc gccccgcccg 34212

ccatgcagcg aagagacccc gggtcccgac aatggcaatg gaggacccac cgctcgtacc 34272

cgtggatcat ctgggagctg aacaagtcta tgttggcaca gcacaggcat atgctcatgc 34332

atctcttcag cactctcagc tcctcggggg tcaaaaccat atcccagggc acggggaact 34392

cttgcaggac agcgaacccc gcagaacagg gcaatcctcg cacataactt acattgtgca 34452

tggacagggt atcgcaatca ggcagcaccg ggtgatcctc caccagagaa gcgcgggtct 34512

cggtctcctc acagcgtggt aagggggccg gccgatacgg gtgatggcgg gacgcggctg 34572

atcgtgttcg cgaccgtgtt atgatgcagt tgctttcgga cattttcgta cttgctgtag 34632

cagaacctgg tccgggcgct gcacaccgat cgccggcggc ggtcccggcg cttggaacgc 34692

tcggtgttga agttgtaaaa cagccactct ctcagaccgt gcagcagatc tagggcctca 34752

ggagtgatga agatcccatc atgcctgatg gctctaatca catcgaccac cgtggaatgg 34812

gccagaccca gccagatgat gcaattttgt tgggtttcgg tgacggcggg ggagggaaga 34872

acaggaagaa ccatgattaa cttttaatcc aaacggtctc ggagcacttc aaaatgaaga 34932

tcgcggagat ggcacctctc gcccccgctg tgttggtgga aaataacagc caggtcaaag 34992

gtgatacggt tctcgagatg ttccacggtg gcttccagca aagcctccac gcgcacatcc 35052

agaaacaaga caatagcgaa agcgggaggg ttctctaatt cctcaatcat catgttacac 35112

tcctgcacca tccccagata attttcattt ttccagcctt gaatgattcg aactagttcc 35172

tgaggtaaat ccaagccagc catgataaag agctcgcgca gagcgccctc caccggcatt 35232
cttaagcaca ccctcataat tccaagatat tctgctcctg gttcacctgc agcagattga 35292
caagcggaat atcaaaatct ctgccgcgat ccctaagctc ctccctcagc aataactgta 35352
agtactcttt catatcctct ccgaaatttt tagccatagg accaccagga ataagattag 35412
ggcaagccac agtacagata aaccgaagtc ctccccagtg agcattgcca aatgcaagac 35472
tgctataagc atgctggcta gacccggtga tatcttccag ataactggac agaaaatcgc 35532
ccaggcaatt tttaagaaaa tcaacaaaag aaaaatcctc caggtgcacg tttagagcct 35592
cgggaacaac gatggagtaa atgcaagcgg tgcgttccag catggttagt tagctgatct 35652
gtagaaaaaa acaaaaatga acattaaacc atgctagcct ggcgaacagg tgggtaaatc 35712
gttctctcca gcaccaggca ggccacgggg tctccggcac gaccctcgta aaaattgtcg 35772
ctatgattga aaaccatcac agagagacgt tcccggtggc cggcgtgaat gattcgacaa 35832
gatgaataca cccccggaac attggcgtcc gcgagtgaaa aaaagcgccc aaggaagcaa 35892
taaggcacta caatgctcag tctcaagtcc agcaaagcga tgccatgcgg atgaagcaca 35952
aaattctcag gtgcgtacaa aatgtaatta ctcccctcct gcacaggcag caaagccccc 36012
gatccctcca ggtacacata caaagcctca gcgtccatag cttaccgagc agcagcacac 36072
aacaggcgca agagtcagag aaaggctgag ctctaacctg tccacccgct ctctgctcaa 36132
tatatagccc agatctacac tgacgtaaag gccaaagtct aaaaataccc gccaaataat 36192
cacacacgcc cagcacacgc ccagaaaccg gtgacacact caaaaaaata cgcgcacttc 36252
ctcaaacgcc caaactgccg tcatttccgg gttcccacgc tacgtcatca aaattcgact 36312
ttcaaattcc gtcgaccgtt aaaaacgtcg cccgccccgc ccctaacggt cgccgctccc 36372
gcagccaatc accgccccgc atccccaaat tcaaatacct catttgcata ttaacgcgca 36432
ccaaaagttt gaggtatatt attgatgatg 36462

<210> 2
<211> 530
<212> PRT
<213> chimpanzee adenovirus serotype Pan5

<400> 2

Met Met Arg Arg Val Tyr Pro Glu Gly Pro Pro Pro Ser Tyr Glu Ser
1               5                   10                  15

Val Met Gln Gln Ala Val Ala Ala Ala Met Gln Pro Pro Leu Glu Ala

20                  25                  30

Pro Tyr Val Pro Pro Arg Tyr Leu Ala Pro Thr Glu Gly Arg Asn Ser
        35                  40                  45

Ile Arg Tyr Ser Glu Leu Ala Pro Leu Tyr Asp Thr Thr Arg Leu Tyr
    50                  55                  60

Leu Val Asp Asn Lys Ser Ala Asp Ile Ala Ser Leu Asn Tyr Gln Asn
65                  70                  75                  80

Asp His Ser Asn Phe Leu Thr Thr Val Val Gln Asn Asn Asp Phe Thr
                85                  90                  95

Pro Thr Glu Ala Ser Thr Gln Thr Ile Asn Phe Asp Glu Arg Ser Arg
            100                 105                 110

Trp Gly Gly Gln Leu Lys Thr Ile Met His Thr Asn Met Pro Asn Val
        115                 120                 125

Asn Glu Phe Met Tyr Ser Asn Lys Phe Lys Ala Arg Val Met Val Ser
    130                 135                 140

Arg Lys Thr Pro Asn Gly Val Thr Val Thr Asp Gly Ser Gln Asp Glu
145                 150                 155                 160

Leu Thr Tyr Glu Trp Val Glu Phe Glu Leu Pro Glu Gly Asn Phe Ser
                165                 170                 175

Val Thr Met Thr Ile Asp Leu Met Asn Asn Ala Ile Ile Asp Asn Tyr
            180                 185                 190

Leu Ala Val Gly Arg Gln Asn Gly Val Leu Glu Ser Asp Ile Gly Val
        195                 200                 205

Lys Phe Asp Thr Arg Asn Phe Arg Leu Gly Trp Asp Pro Val Thr Glu
    210                 215                 220

Leu Val Met Pro Gly Val Tyr Thr Asn Glu Ala Phe His Pro Asp Ile
225                 230                 235                 240

Val Leu Leu Pro Gly Cys Gly Val Asp Phe Thr Glu Ser Arg Leu Ser
                245                 250                 255

Asn Leu Leu Gly Ile Arg Lys Arg Gln Pro Phe Gln Glu Gly Phe Gln
            260                 265                 270

Ile Leu Tyr Glu Asp Leu Glu Gly Gly Asn Ile Pro Ala Leu Leu Asp
        275                 280                 285

Val Asp Ala Tyr Glu Lys Ser Lys Glu Asp Ser Ala Ala Ala Ala Thr
    290                 295                 300

Ala Ala Val Ala Thr Ala Ser Thr Glu Val Arg Gly Asp Asn Phe Ala
305                 310                 315                 320

Ser Ala Ala Thr Leu Ala Ala Ala Glu Ala Ala Glu Thr Glu Ser Lys
                325                 330                 335

Ile Val Ile Gln Pro Val Glu Lys Asp Ser Lys Glu Arg Ser Tyr Asn
            340                 345                 350

Val Leu Ala Asp Lys Lys Asn Thr Ala Tyr Arg Ser Trp Tyr Leu Ala
        355                 360                 365

Tyr Asn Tyr Gly Asp Pro Glu Lys Gly Val Arg Ser Trp Thr Leu Leu
    370                 375                 380

Thr Thr Ser Asp Val Thr Cys Gly Val Glu Gln Val Tyr Trp Ser Leu
385                 390                 395                 400

Pro Asp Met Met Gln Asp Pro Val Thr Phe Arg Ser Thr Arg Gln Val
                405                 410                 415

Ser Asn Tyr Pro Val Val Gly Ala Glu Leu Leu Pro Val Tyr Ser Lys
            420                 425                 430

Ser Phe Phe Asn Glu Gln Ala Val Tyr Ser Gln Gln Leu Arg Ala Phe
        435                 440                 445

Thr Ser Leu Thr His Val Phe Asn Arg Phe Pro Glu Asn Gln Ile Leu
    450                 455                 460

Val Arg Pro Pro Ala Pro Thr Ile Thr Thr Val Ser Glu Asn Val Pro
465                 470                 475                 480

Ala Leu Thr Asp His Gly Thr Leu Pro Leu Arg Ser Ser Ile Arg Gly
                485                 490                 495

Val Gln Arg Val Thr Val Thr Asp Ala Arg Arg Arg Thr Cys Pro Tyr
            500                 505                 510

Val Tyr Lys Ala Leu Gly Val Val Ala Pro Arg Val Leu Ser Ser Arg
        515                 520                 525

Thr Phe
    530

<210>  3

<211>  933

<212>  PRT

<213>  chimpanzee adenovirus serotype Pan5

<400>  3

Met Ala Thr Pro Ser Met Leu Pro Gln Trp Ala Tyr Met His Ile Ala
1               5                   10                  15

Gly Gln Asp Ala Ser Glu Tyr Leu Ser Pro Gly Leu Val Gln Phe Ala
            20                  25                  30

Arg Ala Thr Asp Thr Tyr Phe Ser Leu Gly Asn Lys Phe Arg Asn Pro
        35                  40                  45

Thr Val Ala Pro Thr His Asp Val Thr Thr Asp Arg Ser Gln Arg Leu
    50                  55                  60

Thr Leu Arg Phe Val Pro Val Asp Arg Glu Asp Asn Thr Tyr Ser Tyr
65                  70                  75                  80

Lys Val Arg Tyr Thr Leu Ala Val Gly Asp Asn Arg Val Leu Asp Met
                85                  90                  95

Ala Ser Thr Tyr Phe Asp Ile Arg Gly Val Leu Asp Arg Gly Pro Ser
            100                 105                 110

Phe Lys Pro Tyr Ser Gly Thr Ala Tyr Asn Ser Leu Ala Pro Lys Gly
        115                 120                 125

Ala Pro Asn Thr Cys Gln Trp Thr Tyr Lys Ala Asp Gly Asp Thr Gly
    130                 135                 140

Thr Glu Lys Thr Tyr Thr Tyr Gly Asn Ala Pro Val Gln Gly Ile Ser
145                 150                 155                 160

Ile Thr Lys Asp Gly Ile Gln Leu Gly Thr Asp Thr Asp Asp Gln Pro
                165                 170                 175

Ile Tyr Ala Asp Lys Thr Tyr Gln Pro Glu Pro Gln Val Gly Asp Ala
            180                 185                 190

Glu Trp His Asp Ile Thr Gly Thr Asp Glu Lys Tyr Gly Gly Arg Ala
        195                 200                 205

Leu Lys Pro Asp Thr Lys Met Lys Pro Cys Tyr Gly Ser Phe Ala Lys
    210                 215                 220

Pro Thr Asn Lys Glu Gly Gly Gln Ala Asn Val Lys Thr Glu Thr Gly
225                 230                 235                 240

Gly Thr Lys Glu Tyr Asp Ile Asp Met Ala Phe Phe Asp Asn Arg Ser
                245                 250                 255

Ala Ala Ala Ala Gly Leu Ala Pro Glu Ile Val Leu Tyr Thr Glu Asn
            260                 265                 270

Val Asp Leu Glu Thr Pro Asp Thr His Ile Val Tyr Lys Ala Gly Thr
        275                 280                 285

Asp Asp Ser Ser Ser Ser Ile Asn Leu Gly Gln Gln Ser Met Pro Asn
    290                 295                 300

Arg Pro Asn Tyr Ile Gly Phe Arg Asp Asn Phe Ile Gly Leu Met Tyr
305                 310                 315                 320

Tyr Asn Ser Thr Gly Asn Met Gly Val Leu Ala Gly Gln Ala Ser Gln
                325                 330                 335

Leu Asn Ala Val Val Asp Leu Gln Asp Arg Asn Thr Glu Leu Ser Tyr
            340                 345                 350

Gln Leu Leu Leu Asp Ser Leu Gly Asp Arg Thr Arg Tyr Phe Ser Met
        355                 360                 365

Trp Asn Gln Ala Val Asp Ser Tyr Asp Pro Asp Val Arg Ile Ile Glu
    370                 375                 380

Asn His Gly Val Glu Asp Glu Leu Pro Asn Tyr Cys Phe Pro Leu Asp
385                 390                 395                 400

Ala Val Gly Arg Thr Asp Thr Tyr Gln Gly Ile Lys Ala Asn Gly Ala
                405                 410                 415

Asp Gln Thr Thr Trp Thr Lys Asp Asp Thr Val Asn Asp Ala Asn Glu
            420                 425                 430

Leu Gly Lys Gly Asn Pro Phe Ala Met Glu Ile Asn Ile Gln Ala Asn

435                     440                     445

Leu Trp Arg Asn Phe Leu Tyr Ala Asn Val Ala Leu Tyr Leu Pro Asp
    450                 455                 460

Ser Tyr Lys Tyr Thr Pro Ala Asn Ile Thr Leu Pro Thr Asn Thr Asn
465                 470                 475                 480

Thr Tyr Asp Tyr Met Asn Gly Arg Val Val Ala Pro Ser Leu Val Asp
                485                 490                 495

Ala Tyr Ile Asn Ile Gly Ala Arg Trp Ser Leu Asp Pro Met Asp Asn
            500                 505                 510

Val Asn Pro Phe Asn His His Arg Asn Ala Gly Leu Arg Tyr Arg Ser
        515                 520                 525

Met Leu Leu Gly Asn Gly Arg Tyr Val Pro Phe His Ile Gln Val Pro
    530                 535                 540

Gln Lys Phe Phe Ala Ile Lys Ser Leu Leu Leu Leu Pro Gly Ser Tyr
545                 550                 555                 560

Thr Tyr Glu Trp Asn Phe Arg Lys Asp Val Asn Met Ile Leu Gln Ser
                565                 570                 575

Ser Leu Gly Asn Asp Leu Arg Thr Asp Gly Ala Ser Ile Ala Phe Thr
            580                 585                 590

Ser Ile Asn Leu Tyr Ala Thr Phe Phe Pro Met Ala His Asn Thr Ala
        595                 600                 605

Ser Thr Leu Glu Ala Met Leu Arg Asn Asp Thr Asn Asp Gln Ser Phe
    610                 615                 620

Asn Asp Tyr Leu Ser Ala Ala Asn Met Leu Tyr Pro Ile Pro Ala Asn
625                 630                 635                 640

Ala Thr Asn Val Pro Ile Ser Ile Pro Ser Arg Asn Trp Ala Ala Phe
                645                 650                 655

Arg Gly Trp Ser Phe Thr Arg Leu Lys Thr Arg Glu Thr Pro Ser Leu
            660                 665                 670

Gly Ser Gly Phe Asp Pro Tyr Phe Val Tyr Ser Gly Ser Ile Pro Tyr
        675                 680                 685

Leu Asp Gly Thr Phe Tyr Leu Asn His Thr Phe Lys Lys Val Ser Ile
    690                 695                 700

Thr Phe Asp Ser Ser Val Ser Trp Pro Gly Asn Asp Arg Leu Leu Thr
705                 710                 715                 720

Pro Asn Glu Phe Glu Ile Lys Arg Thr Val Asp Gly Glu Gly Tyr Asn
                725                 730                 735

Val Ala Gln Cys Asn Met Thr Lys Asp Trp Phe Leu Val Gln Met Leu
            740                 745                 750

Ala His Tyr Asn Ile Gly Tyr Gln Gly Phe Tyr Val Pro Glu Gly Tyr
        755                 760                 765

Lys Asp Arg Met Tyr Ser Phe Phe Arg Asn Phe Gln Pro Met Ser Arg
    770                 775                 780

Gln Val Val Asp Glu Val Asn Tyr Lys Asp Tyr Gln Ala Val Thr Leu
785                 790                 795                 800

Ala Tyr Gln His Asn Asn Ser Gly Phe Val Gly Tyr Leu Ala Pro Thr
                805                 810                 815

Met Arg Gln Gly Gln Pro Tyr Pro Ala Asn Tyr Pro Tyr Pro Leu Ile
            820                 825                 830

Gly Lys Ser Ala Val Ala Ser Val Thr Gln Lys Lys Phe Leu Cys Asp
        835                 840                 845

Arg Val Met Trp Arg Ile Pro Phe Ser Ser Asn Phe Met Ser Met Gly
    850                 855                 860

Ala Leu Thr Asp Leu Gly Gln Asn Met Leu Tyr Ala Asn Ser Ala His
865                 870                 875                 880

Ala Leu Asp Met Asn Phe Glu Val Asp Pro Met Asp Glu Ser Thr Leu
                885                 890                 895

Leu Tyr Val Val Phe Glu Val Phe Asp Val Val Arg Val His Gln Pro
            900                 905                 910

His Arg Gly Val Ile Glu Ala Val Tyr Leu Arg Thr Pro Phe Ser Ala
        915                 920                 925

Gly Asn Ala Thr Thr
    930

<210>  4

<211>  445

<212>  PRT

<213>  chimpanzee adenovirus serotype Pan5


<400>  4

Met Ser Lys Lys Arg Val Arg Val Asp Asp Asp Phe Asp Pro Val Tyr
1               5                   10                  15

Pro Tyr Asp Ala Asp Asn Ala Pro Thr Val Pro Phe Ile Asn Pro Pro
            20                  25                  30

Phe Val Ser Ser Asp Gly Phe Gln Glu Lys Pro Leu Gly Val Leu Ser
        35                  40                  45

Leu Arg Leu Ala Asp Pro Val Thr Thr Lys Asn Gly Glu Ile Thr Leu
    50                  55                  60

Lys Leu Gly Asp Gly Val Asp Leu Asp Ser Ser Gly Lys Leu Ile Ser
65                  70                  75                  80

Asn Thr Ala Thr Lys Ala Ala Ala Pro Leu Ser Phe Ser Asn Asn Thr
                85                  90                  95

Ile Ser Leu Asn Met Asp Thr Pro Phe Tyr Asn Asn Asn Gly Lys Leu
            100                 105                 110

Gly Met Lys Val Thr Ala Pro Leu Lys Ile Leu Asp Thr Asp Leu Leu
        115                 120                 125

Lys Thr Leu Val Val Ala Tyr Gly Gln Gly Leu Gly Thr Asn Thr Thr
    130                 135                 140

Gly Ala Leu Val Ala Gln Leu Ala Ser Pro Leu Ala Phe Asp Ser Asn
145                 150                 155                 160

Ser Lys Ile Ala Leu Asn Leu Gly Asn Gly Pro Leu Lys Val Asp Ala
                165                 170                 175

Asn Arg Leu Asn Ile Asn Cys Asn Arg Gly Leu Tyr Val Thr Thr Thr
            180                 185                 190

Lys Asp Ala Leu Glu Ala Asn Ile Ser Trp Ala Asn Ala Met Thr Phe
        195                 200                 205

Ile Gly Asn Ala Met Gly Val Asn Ile Asp Thr Gln Lys Gly Leu Gln
    210                 215                 220

Phe Gly Thr Thr Ser Thr Val Ala Asp Val Lys Asn Ala Tyr Pro Ile
225                 230                 235                 240

Gln Ile Lys Leu Gly Ala Gly Leu Thr Phe Asp Ser Thr Gly Ala Ile
                245                 250                 255

Val Ala Trp Asn Lys Asp Asp Asp Lys Leu Thr Leu Trp Thr Thr Ala
            260                 265                 270

Asp Pro Ser Pro Asn Cys His Ile Tyr Ser Glu Lys Asp Ala Lys Leu
        275                 280                 285

Thr Leu Cys Leu Thr Lys Cys Gly Ser Gln Ile Leu Gly Thr Val Ser
    290                 295                 300

Leu Ile Ala Val Asp Thr Gly Ser Leu Asn Pro Ile Thr Gly Thr Val
305                 310                 315                 320

Thr Thr Ala Leu Val Ser Leu Lys Phe Asp Ala Asn Gly Val Leu Gln
                325                 330                 335

Ser Ser Ser Thr Leu Asp Ser Asp Tyr Trp Asn Phe Arg Gln Gly Asp
            340                 345                 350

Val Thr Pro Ala Glu Ala Tyr Thr Asn Ala Ile Gly Phe Met Pro Asn
        355                 360                 365

Leu Lys Ala Tyr Pro Lys Asn Thr Ser Gly Ala Ala Lys Ser His Ile
    370                 375                 380

Val Gly Lys Val Tyr Leu His Gly Asp Thr Gly Lys Pro Leu Asp Leu
385                 390                 395                 400

Ile Ile Thr Phe Asn Glu Thr Ser Asp Glu Ser Cys Thr Tyr Cys Ile
                405                 410                 415

Asn Phe Gln Trp Gln Trp Gly Ala Asp Gln Tyr Lys Asn Glu Thr Leu
            420                 425                 430

Ala Val Ser Ser Phe Thr Phe Ser Tyr Ile Ala Lys Glu
        435                 440                 445

<210>  5

<211>  36604

<212>  DNA

<213>  chimpanzee adenovirus serotype Pan6

<220>

<221>  CDS

<222>  (13878)..(15467)

<223>  L2 Penton

<220>

<221>  CDS

<222>  (18284)..(21112)

<223>  L3 Hexon

<220>

<221>  CDS

<222>  (32162)..(33493)

<223>  L5 Fiber

<400>  5
catcatcaat aatatacctc aaacttttgg tgcgcgttaa tatgcaaatg agctgtttga      60

atttggggag ggaggaaggt gattggctgc gggagcggcg accgttaggg gcggggcggg     120

tgacgttttg atgacgtggc tatgaggcgg agccggtttg caagttctcg tgggaaaagt     180

gacgtcaaac gaggtgtggt ttgaacacgg aaatactcaa ttttcccgcg ctctctgaca     240

ggaaatgagg tgtttctggg cggatgcaag tgaaaacggg ccattttcgc gcgaaaactg     300

aatgaggaag tgaaaatctg agtaatttcg cgtttatggc agggaggagt atttgccgag     360

ggccgagtag actttgaccg attacgtggg ggtttcgatt accgtatttt tcacctaaat     420

ttccgcgtac ggtgtcaaag tccggtgttt ttacgtaggc gtcagctgat cgccagggta     480

tttaaacctg cgctctctag tcaagaggcc actcttgagt gccagcgagt agagttttct     540

cctccgcgcc gcgagtcaga tctacacttt gaaagatgag gcacctgaga gacctgcccg     600

gtaatgtttt cctggctact gggaacgaga ttctggaatt ggtggtggac gccatgatgg     660

gtgacgaccc tccagagccc cctaccccat ttgaggcgcc ttcgctgtac gatttgtatg     720

atctggaggt ggatgtgccc gagagcgacc ctaacgagga ggcggtgaat gatttgttta 780
gcgatgccgc gctgctggct gccgagcagg ctaatacgga ctctggctca gacagcgatt 840
cctctctcca taccccgaga cccggcagag gtgagaaaaa gatccccgag cttaaagggg 900
aagagctcga cctgcgctgc tatgaggaat gcttgcctcc gagcgatgat gaggaggacg 960
aggaggcgat tcgagctgcg gtgaaccagg gagtgaaaac tgcgggcgag agctttagcc 1020
tggactgtcc tactctgccc ggacacggct gtaagtcttg tgaatttcat cgcatgaata 1080
ctggagataa gaatgtgatg tgtgccctgt gctatatgag agcttacaac cattgtgttt 1140
acagtaagtg tgattaactt tagttgggaa ggcagagggt gactgggtgc tgactggttt 1200
atttatgtat atgttttttt atgtgtaggt cccgtctctg acgtagatga gacccccact 1260
tcagagtgca tttcatcacc cccagaaatt ggcgaggaac cgcccgaaga tattattcat 1320
agaccagttg cagtgagagt caccgggcgg agagcagctg tggagagttt ggatgacttg 1380
ctacagggtg gggatgaacc tttggacttg tgtacccgga aacgccccag gcactaagtg 1440
ccacacatgt gtgtttactt aaggtgatgt cagtatttat agggtgtgga gtgcaataaa 1500
atccgtgttg actttaagtg cgtgttttat gactcagggg tggggactgt gggtatataa 1560
gcaggtgcag acctgtgtgg tcagttcaga gcaggactca tggagatctg gactgtcttg 1620
gaagactttc accagactag acagttgcta gagaactcat cggagggagt ctcttacctg 1680
tggagattct gcttcggtgg gcctctagct aagctagtct atagggccaa acaggattat 1740
aaggaacaat ttgaggatat tttgagagag tgtcctggta tttttgactc tctcaacttg 1800
ggccatcagt ctcactttaa ccagagtatt ctgagagccc ttgacttttc tactcctggc 1860
agaactaccg ccgcggtagc cttttttgcc tttattcttg acaaatggag tcaagaaacc 1920
catttcagca gggattaccg tctggactgc ttagcagtag ctttgtggag aacatggagg 1980
tgccagcgcc tgaatgcaat ctccggctac ttgccagtac agccggtaga cacgctgagg 2040
atcctgagtc tccagtcacc ccaggaacac caacgccgcc agcagccgca gcaggagcag 2100
cagcaagagg aggaccgaga agagaacccg agagccggtc tggaccctcc ggtggcggag 2160
gaggaggagt agctgacttg tttcccgagc tgcgccgggt gctgactagg tcttccagtg 2220
gacgggagag ggggattaag cgggagaggc atgaggagac tagccacaga actgaactga 2280
ctgtcagtct gatgagccgc aggcgcccag aatcggtgtg gtggcatgag gtgcagtcgc 2340
aggggataga tgaggtctcg gtgatgcatg agaaatattc cctagaacaa gtcaagactt 2400
gttggttgga gcccgaggat gattgggagg tagccatcag gaattatgcc aagctggctc 2460
tgaagccaga caagaagtac aagattacca aactgattaa tatcagaaat tcctgctaca 2520
tttcagggaa tggggccgag gtggagatca gtacccagga gagggtggcc ttcagatgtt 2580

gtatgatgaa tatgtacccg ggggtggtgg gcatggaggg agtcaccttt atgaacacga 2640
ggttcagggg tgatgggtat aatggggtgg tctttatggc caacaccaag ctgacagtgc 2700
acggatgctc cttctttggc ttcaataaca tgtgcatcga ggcctggggc agtgtttcag 2760
tgaggggatg cagcttttca gccaactgga tgggggtcgt gggcagaacc aagagcaagg 2820
tgtcagtgaa gaaatgcctg ttcgagaggt gccacctggg ggtgatgagc gagggcgaag 2880
ccaaagtcaa acactgcgcc tctaccgaga cgggctgctt tgtgctgatc aagggcaatg 2940
cccaagtcaa gcataacatg atctgtgggg cctcggatga gcgcggctac cagatgctga 3000
cctgcgccgg tgggaacagc catatgctgg ccaccgtgca tgtggcctcg cacccccgca 3060
agacatggcc cgagttcgag cacaacgtca tgacccgctg caatgtgcac ctgggctccc 3120
gccgaggcat gttcatgccc taccagtgca acatgcaatt tgtgaaggtg ctgctggagc 3180
ccgatgccat gtccagagtg agcctgacgg gggtgtttga catgaatgtg gagctgtgga 3240
aaattctgag atatgatgaa tccaagacca ggtgccgggc ctgcgaatgc ggaggcaagc 3300
acgccaggct tcagcccgtg tgtgtggagg tgacggagga cctgcgaccc gatcatttgg 3360
tgttgtcctg caacgggacg gagttcggct ccagcgggga agaatctgac tagagtgagt 3420
agtgtttggg gctgggtgtg agcctgcatg aggggcagaa tgactaaaat ctgtggtttt 3480
ctgtgtgttg cagcagcatg agcggaagcg cctcctttga gggaggggta ttcagccctt 3540
atctgacggg gcgtctcccc tcctgggcgg gagtgcgtca gaatgtgatg ggatccacgg 3600
tggacggccg gcccgtgcag cccgcgaact cttcaaccct gacctacgcg accctgagct 3660
cctcgtccgt ggacgcagct gccgccgcag ctgctgcttc cgccgccagc gccgtgcgcg 3720
gaatggccct gggcgccggc tactacagct ctctggtggc caactcgagt tccaccaata 3780
atcccgccag cctgaacgag gagaagctgc tgctgctgat ggcccagctc gaggccctga 3840
cccagcgcct gggcgagctg acccagcagg tggctcagct gcaggcggag acgcgggccg 3900
cggttgccac ggtgaaaacc aaataaaaaa tgaatcaata aataaacgga gacggttgtt 3960
gattttaaca cagagtcttg aatctttatt tgatttttcg cgcgcggtag gccctggacc 4020
accggtctcg atcattgagc acccggtgga tcttttccag gacccggtag aggtgggctt 4080
ggatgttgag gtacatgggc atgagcccgt cccgggggtg gaggtagctc cattgcaggg 4140
cctcgtgctc ggggatggtg ttgtaaatca cccagtcata gcaggggcgc agggcgtggt 4200
gctgcacgat gtccttgagg aggagactga tggccacggg cagccccttg gtgtaggtgt 4260
tgacgaacct gttgagctgg gagggatgca tgcgggggga gatgagatgc atcttggcct 4320
ggatcttgag attggcgatg ttcccgccca gatcccgccg ggggttcatg ttgtgcagga 4380
ccaccagcac ggtgtatccg gtgcacttgg ggaatttgtc atgcaacttg gaagggaagg 4440
cgtgaaagaa tttggagacg cccttgtgac cgcccaggtt ttccatgcac tcatccatga 4500

tgatggcgat gggcccgtgg gcggcggcct gggcaaagac gtttcggggg tcggacacat 4560
cgtagttgtg gtcctgggtg agctcgtcat aggccatttt aatgaatttg gggcggaggg 4620
tgcccgactg ggggacgaag gtgccctcga tcccgggggc gtagttgccc tcgcagatct 4680
gcatctccca ggccttgagc tcggaggggg ggatcatgtc cacctgcggg gcgatgaaaa 4740
aaacggtttc cggggcgggg gagatgagct gggccgaaag caggttccgg agcagctggg 4800
acttgccgca accggtgggg ccgtagatga ccccgatgac cggctgcagg tggtagttga 4860
gggagagaca gctgccgtcc tcgcggagga ggggggccac ctcgttcatc atctcgcgca 4920
catgcatgtt ctcgcgcacg agttccgcca ggaggcgctc gccccccagc gagaggagct 4980
cttgcagcga ggcgaagttt ttcagcggct tgagtccgtc ggccatgggc attttggaga 5040
gggtctgttg caagagttcc agacggtccc agagctcggt gatgtgctct agggcatctc 5100
gatccagcag acctcctcgt ttcgcgggtt ggggcgactg cgggagtagg gcaccaggcg 5160
atgggcgtcc agcgaggcca gggtccggtc cttccagggc cgcagggtcc gcgtcagcgt 5220
ggtctccgtc acggtgaagg ggtgcgcgcc gggctgggcg cttgcgaggg tgcgcttcag 5280
gctcatccgg ctggtcgaga accgctcccg gtcggcgccc tgcgcgtcgg ccaggtagca 5340
attgagcatg agttcgtagt tgagcgcctc ggccgcgtgg cccttggcgc ggagcttacc 5400
tttggaagtg tgtccgcaga cgggacagag gagggacttg agggcgtaga gcttgggggc 5460
gaggaagacg gactcggggg cgtaggcgtc cgcgccgcag ctggcgcaga cggtctcgca 5520
ctccacgagc caggtgaggt cggggcggtt ggggtcaaaa acgaggtttc ctccgtgctt 5580
tttgatgcgt ttcttacctc tggtctccat gagctcgtgt ccccgctggg tgacaaagag 5640
gctgtccgtg tccccgtaga ccgactttat gggccggtcc tcgagcgggg tgccgcggtc 5700
ctcgtcgtag aggaaccccg cccactccga gacgaaggcc cgggtccagg ccagcacgaa 5760
ggaggccacg tgggaggggt agcggtcgtt gtccaccagc gggtccacct tctccagggt 5820
atgcaagcac atgtccccct cgtccacatc caggaaggtg attggcttgt aagtgtaggc 5880
cacgtgaccg ggggtcccgg ccgggggggt ataaaagggg gcgggcccct gctcgtcctc 5940
actgtcttcc ggatcgctgt ccaggagcgc cagctgttgg ggtaggtatt ccctctcgaa 6000
ggcgggcatg acctcggcac tcaggttgtc agtttctaga aacgaggagg atttgatatt 6060
gacggtgccg ttggagacgc ctttcatgag cccctcgtcc atttggtcag aaaagacgat 6120
ctttttgttg tcgagcttgg tggcgaagga gccgtagagg gcgttggaga gcagcttggc 6180
gatggagcgc atggtctggt tcttttcctt gtcggcgcgc tccttggcgg cgatgttgag 6240
ctgcacgtac tcgcgcgcca cgcacttcca ttcggggaag acggtggtga gctcgtcggg 6300
cacgattctg acccgccagc cgcggttgtg cagggtgatg aggtccacgc tggtggccac 6360

ctcgccgcgc aggggctcgt tggtccagca gaggcgcccg cccttgcgcg agcagaaggg 6420
gggcagcggg tccagcatga gctcgtcggg ggggtcggcg tccacggtga agatgccggg 6480
caggagctcg gggtcgaagt agctgatgca ggtgcccaga ttgtccagcg ccgcttgcca 6540
gtcgcgcacg gccagcgcgc gctcgtaggg gctgaggggc gtgccccagg gcatggggtg 6600
cgtgagcgcg gaggcgtaca tgccgcagat gtcgtagacg tagaggggct cctcgaggac 6660
gccgatgtag gtggggtagc agcgcccccc gcggatgctg gcgcgcacgt agtcgtacag 6720
ctcgtgcgag ggcgcgagga gccccgtgcc gaggttggag cgttgcggct tttcggcgcg 6780
gtagacgatc tggcggaaga tggcgtggga gttggaggag atggtgggcc tttggaagat 6840
gttgaagtgg gcgtggggca ggccgaccga gtccctgatg aagtgggcgt aggagtcctg 6900
cagcttggcg acgagctcgg cggtgacgag gacgtccagg gcgcagtagt cgagggtctc 6960
ttggatgatg tcatacttga gctggccctt ctgcttccac agctcgcggt tgagaaggaa 7020
ctcttcgcgg tccttccagt actcttcgag ggggaacccg tcctgatcgg cacggtaaga 7080
gcccaccatg tagaactggt tgacggcctt gtaggcgcag cagcccttct ccacggggag 7140
ggcgtaagct tgcgcggcct tgcgcaggga ggtgtgggtg agggcgaagg tgtcgcgcac 7200
catgaccttg aggaactggt gcttgaagtc gaggtcgtcg cagccgccct gctcccagag 7260
ttggaagtcc gtgcgcttct tgtaggcggg gttaggcaaa gcgaaagtaa catcgttgaa 7320
gaggatcttg cccgcgcggg gcatgaagtt gcgagtgatg cggaaaggct ggggcacctc 7380
ggcccggttg ttgatgacct gggcggcgag gacgatctcg tcgaagccgt tgatgttgtg 7440
cccgacgatg tagagttcca cgaatcgcgg gcggcccttg acgtggggca gcttcttgag 7500
ctcgtcgtag gtgagctcgg cggggtcgct gagcccgtgc tgctcgaggg cccagtcggc 7560
gacgtggggg ttggcgctga ggaaggaagt ccagagatcc acggccaggg cggtctgcaa 7620
gcggtcccgg tactgacgga actgttggcc cacggccatt ttttcggggg tgacgcagta 7680
gaaggtgcgg gggtcgccgt gccagcggtc ccacttgagc tggagggcga ggtcgtgggc 7740
gagctcgacg agcggcgggt ccccggagag tttcatgacc agcatgaagg ggacgagctg 7800
cttgccgaag gaccccatcc aggtgtaggt ttccacatcg taggtgagga agagcctttc 7860
ggtgcgagga tgcgagccga tggggaagaa ctggatctcc tgccaccagt tggaggaatg 7920
gctgttgatg tgatggaagt agaaatgccg acggcgcgcc gagcactcgt gcttgtgttt 7980
atacaagcgt ccgcagtgct cgcaacgctg cacgggatgc acgtgctgca cgagctgtac 8040
ctgggttcct ttggcgagga atttcagtgg gcagtggagc gctggcggct gcatctcgtg 8100
ctgtactacg tcttggccat cggcgtggcc atcgtctgcc tcgatggtgg tcatgctgac 8160
gagcccgcgc gggaggcagg tccagacctc ggctcggacg ggtcggagag cgaggacgag 8220
ggcgcgcagg ccggagctgt ccagggtcct gagacgctgc ggagtcaggt cagtgggcag 8280

cggcggcgcg cggttgactt gcaggagctt ttccagggcg cgcgggaggt ccagatggta 8340
cttgatctcc acggcgccgt tggtggctac gtccacggct tgcagggtgc cgtgcccctg 8400
gggcgccacc accgtgcccc gtttcttctt gggcgctgct tccatgtcgg tcagaagcgg 8460
cggcgaggac gcgcgccggg cggcaggggc ggctcggggc ccggaggcag gggcggcagg 8520
ggcacgtcgg cgccgcgcgc gggcaggttc tggtactgcg cccggagaag actggcgtga 8580
gcgacgacgc gacggttgac gtcctggatc tgacgcctct gggtgaaggc cacgggaccc 8640
gtgagtttga acctgaaaga gagttcgaca gaatcaatct cggtatcgtt gacggcggcc 8700
tgccgcagga tctcttgcac gtcgcccgag ttgtcctggt aggcgatctc ggtcatgaac 8760
tgctcgatct cctcctcctg aaggtctccg cggccggcgc gctcgacggt ggccgcgagg 8820
tcgttggaga tgcggcccat gagctgcgag aaggcgttca tgccggcctc gttccagacg 8880
cggctgtaga ccacggctcc gtcggggtcg cgcgcgcgca tgaccacctg ggcgaggttg 8940
agctcgacgt ggcgcgtgaa gaccgcgtag ttgcagaggc gctggtagag gtagttgagc 9000
gtggtggcga tgtgctcggt gacgaagaag tacatgatcc agcggcggag cggcatctcg 9060
ctgacgtcgc ccagggcttc caagcgttcc atggcctcgt agaagtccac ggcgaagttg 9120
aaaaactggg agttgcgcgc cgagacggtc aactcctcct ccagaagacg gatgagctcg 9180
gcgatggtgg cgcgcacctc gcgctcgaag gccccggggg gctcctcttc catctcctcc 9240
tcttcctcct ccactaacat ctcttctact tcctcctcag gaggcggtgg cgggggaggg 9300
gccctgcgtc gccggcggcg cacgggcaga cggtcgatga agcgctcgat ggtctccccg 9360
cgccggcgac gcatggtctc ggtgacggcg cgcccgtcct cgcggggccg cagcatgaag 9420
acgccgccgc gcatctccag gtggccgccg ggggggtctc cgttgggcag ggagagggcg 9480
ctgacgatgc atcttatcaa ttgacccgta gggactccgc gcaaggacct gagcgtctcg 9540
agatccacgg gatccgaaaa ccgctgaacg aaggcttcga gccagtcgca gtcgcaaggt 9600
aggctgagcc cggtttcttg ttcttcgggt atttggtcgg gaggcgggcg ggcgatgctg 9660
ctggtgatga agttgaagta ggcggtcctg agacggcgga tggtggcgag gagcaccagg 9720
tccttgggcc cggcttgctg gatgcgcaga cggtcggcca tgccccaggc gtggtcctga 9780
cacctggcga ggtccttgta gtagtcctgc atgagccgct ccacgggcac ctcctcctcg 9840
cccgcgcggc cgtgcatgcg cgtgagcccg aacccgcgct gcggctggac gagcgccagg 9900
tcggcgacga cgcgctcggt gaggatggcc tgctggatct gggtgagggt ggtctggaag 9960
tcgtcgaagt cgacgaagcg gtggtaggct ccggtgttga tggtgtagga gcagttggcc 10020
atgacggacc agttgacggt ctggtggccg ggtcgcacga gctcgtggta cttgaggcgc 10080
gagtaggcgc gcgtgtcgaa gatgtagtcg ttgcaggcgc gcacgaggta ctggtatccg 10140

acgaggaagt gcggcggcgg ctggcggtag agcggccatc gctcggtggc gggggcgccg 10200
ggcgcgaggt cctcgagcat gaggcggtgg tagccgtaga tgtacctgga catccaggtg 10260
atgccggcgg cggtggtgga ggcgcgcggg aactcgcgga cgcggttcca gatgttgcgc 10320
agcggcagga agtagttcat ggtggccgcg gtctggcccg tgaggcgcgc gcagtcgtgg 10380
atgctctaga catacgggca aaaacgaaag cggtcagcgg ctcgactccg tggcctggag 10440
gctaagcgaa cgggttgggc tgcgcgtgta ccccggttcg aatctcgaat caggctggag 10500
ccgcagctaa cgtggtactg gcactcccgt ctcgacccaa gcctgctaac gaaacctcca 10560
ggatacggag gcgggtcgtt ttttggcctt ggtcgctggt catgaaaaac tagtaagcgc 10620
ggaaagcggc cgcccgcgat ggctcgctgc cgtagtctgg agaaagaatc gccagggttg 10680
cgttgcggtg tgccccggtt cgagcctcag cgctcggcgc cggccggatt ccgcggctaa 10740
cgtgggcgtg gctgccccgt cgtttccaag accccttagc cagccgactt ctccagttac 10800
ggagcgagcc cctctttttt tttcttgtgt tttgccagat tgcatcccgt actgcggcag 10860
atgcgccccc accctccacc acaaccgccc ctaccgcagc agcagcaaca gccggcgctt 10920
ctgcccccgc cccagcagca gccagccact accgcggcgg ccgccgtgag cggagccggc 10980
gttcagtatg acctggcctt ggaagagggc gaggggctgg cgcggctggg ggcgtcgtcg 11040
ccggagcggc acccgcgcgt gcagatgaaa agggacgctc gcgaggccta cgtgcccaag 11100
cagaacctgt tcagagacag gagcggcgag gagcccgagg agatgcgcgc ctcccgcttc 11160
cacgcggggc gggagctgcg gcgcggcctg gaccgaaagc gggtgctgag ggacgaggat 11220
ttcgaggcgg acgagctgac ggggatcagc cccgcgcgcg cgcacgtggc cgcggccaac 11280
ctggtcacgg cgtacgagca gaccgtgaag gaggagagca acttccaaaa atccttcaac 11340
aaccacgtgc gcacgctgat cgcgcgcgag gaggtgaccc tgggcctgat gcacctgtgg 11400
gacctgctgg aggccatcgt gcagaacccc acgagcaagc cgctgacggc gcagctgttt 11460
ctggtggtgc agcacagtcg ggacaacgag acgttcaggg aggcgctgct gaatatcacc 11520
gagcccgagg gccgctggct cctggacctg gtgaacattt tgcagagcat cgtggtgcag 11580
gagcgcgggc tgccgctgtc cgagaagctg gcggccatca acttctcggt gctgagtctg 11640
ggcaagtact acgctaggaa gatctacaag accccgtacg tgcccataga caaggaggtg 11700
aagatcgacg ggttttacat gcgcatgacc ctgaaagtgc tgaccctgag cgacgatctg 11760
ggggtgtacc gcaacgacag gatgcaccgc gcggtgagcg ccagccgccg gcgcgagctg 11820
agcgaccagg agctgatgca cagcctgcag cgggccctga ccggggccgg gaccgagggg 11880
gagagctact ttgacatggg cgcggacctg cgctggcagc ccagccgccg ggccttggaa 11940
gctgccggcg gttcccccta cgtggaggag gtggacgatg aggaggagga gggcgagtac 12000
ctggaagact gatggcgcga ccgtattttt gctagatgca gcaacagcca ccgccgccgc 12060

ctcctgatcc cgcgatgcgg gcggcgctgc agagccagcc gtccggcatt aactcctcgg 12120
acgattggac ccaggccatg caacgcatca tggcgctgac gacccgcaat cccgaagcct 12180
ttagacagca gcctcaggcc aaccggctct cggccatcct ggaggccgtg gtgccctcgc 12240
gctcgaaccc cacgcacgag aaggtgctgg ccatcgtgaa cgcgctggtg gagaacaagg 12300
ccatccgcgg tgacgaggcc gggctggtgt acaacgcgct gctggagcgc gtggcccgct 12360
acaacagcac caacgtgcag acgaacctgg accgcatggt gaccgacgtg cgcgaggcgg 12420
tgtcgcagcg cgagcggttc caccgcgagt cgaacctggg ctccatggtg gcgctgaacg 12480
ccttcctgag cacgcagccc gccaacgtgc cccggggcca ggaggactac accaacttca 12540
tcagcgcgct gcggctgatg gtggccgagg tgccccagag cgaggtgtac cagtcggggc 12600
cggactactt cttccagacc agtcgccagg gcttgcagac cgtgaacctg agccaggctt 12660
tcaagaactt gcagggactg tggggcgtgc aggccccggt cggggaccgc gcgacggtgt 12720
cgagcctgct gacgccgaac tcgcgcctgc tgctgctgct ggtggcgccc ttcacggaca 12780
gcggcagcgt gagccgcgac tcgtacctgg gctacctgct taacctgtac cgcgaggcca 12840
tcggacaggc gcacgtggac gagcagacct accaggagat cacccacgtg agccgcgcgc 12900
tgggccagga ggacccgggc aacctggagg ccaccctgaa cttcctgctg accaaccggt 12960
cgcagaagat cccgccccag tacgcgctga gcaccgagga ggagcgcatc ctgcgctacg 13020
tgcagcagag cgtggggctg ttcctgatgc aggagggggc cacgcccagc gcggcgctcg 13080
acatgaccgc gcgcaacatg gagcccagca tgtacgcccg caaccgcccg ttcatcaata 13140
agctgatgga ctacttgcat cgggcggccg ccatgaactc ggactacttt accaacgcca 13200
tcttgaaccc gcactggctc ccgccgcccg ggttctacac gggcgagtac gacatgcccg 13260
accccaacga cgggttcctg tgggacgacg tggacagcag cgtgttctcg ccgcgtccag 13320
gaaccaatgc cgtgtggaag aaagagggcg gggaccggcg gccgtcctcg gcgctgtccg 13380
gtcgcgcggg tgctgccgcg gcggtgcccg aggccgccag ccccttcccg agcctgccct 13440
tttcgctgaa cagcgtgcgc agcagcgagc tgggtcggct gacgcgaccg cgcctgctgg 13500
gcgaggagga gtacctgaac gactccttgt tgaggcccga gcgcgagaag aacttcccca 13560
ataacgggat agagagcctg gtggacaaga tgagccgctg gaagacgtac gcgcacgagc 13620
acagggacga gccccgagct agcagcgcag gcacccgtag acgccagcgg cacgacaggc 13680
agcggggact ggtgtgggac gatgaggatt ccgccgacga cagcagcgtg ttggacttgg 13740
gtgggagtgg tggtaacccg ttcgctcacc tgcgcccccg tatcgggcgc ctgatgtaag 13800
aatctgaaaa aataaaagac ggtactcacc aaggccatgg cgaccagcgt gcgttcttct 13860
ctgttgtttg tagtagt atg atg agg cgc gtg tac ccg gag ggt cct cct 13910
Met Met Arg Arg Val Tyr Pro Glu Gly Pro Pro

                    1               5                   10
ccc tcg tac gag agc gtg atg cag cag gcg gtg gcg gcg gcg atg cag     13958
Pro Ser Tyr Glu Ser Val Met Gln Gln Ala Val Ala Ala Ala Met Gln
            15                  20                  25

ccc ccg ctg gag gcg cct tac gtg ccc ccg cgg tac ctg gcg cct acg     14006
Pro Pro Leu Glu Ala Pro Tyr Val Pro Pro Arg Tyr Leu Ala Pro Thr
        30                  35                  40

gag ggg cgg aac agc att cgt tac tcg gag ctg gca ccc ttg tac gat     14054
Glu Gly Arg Asn Ser Ile Arg Tyr Ser Glu Leu Ala Pro Leu Tyr Asp
    45                  50                  55

acc acc cgg ttg tac ctg gtg gac aac aag tcg gca gac atc gcc tcg     14102
Thr Thr Arg Leu Tyr Leu Val Asp Asn Lys Ser Ala Asp Ile Ala Ser
60                  65                  70                  75

ctg aac tac cag aac gac cac agc aac ttc ctg acc acc gtg gtg cag     14150
Leu Asn Tyr Gln Asn Asp His Ser Asn Phe Leu Thr Thr Val Val Gln
                80                  85                  90

aac aac gat ttc acc ccc acg gag gcc agc acc cag acc atc aac ttt     14198
Asn Asn Asp Phe Thr Pro Thr Glu Ala Ser Thr Gln Thr Ile Asn Phe
            95                  100                 105

gac gag cgc tcg cgg tgg ggc ggc cag ctg aaa acc atc atg cac acc     14246
Asp Glu Arg Ser Arg Trp Gly Gly Gln Leu Lys Thr Ile Met His Thr
        110                 115                 120

aac atg ccc aac gtg aac gag ttc atg tac agc aac aag ttc aag gcg     14294
Asn Met Pro Asn Val Asn Glu Phe Met Tyr Ser Asn Lys Phe Lys Ala
    125                 130                 135

cgg gtg atg gtc tcg cgc aag acc ccc aac ggg gtg gat gat gat tat     14342
Arg Val Met Val Ser Arg Lys Thr Pro Asn Gly Val Asp Asp Asp Tyr
140                 145                 150                 155

gat ggt agt cag gac gag ctg acc tac gag tgg gtg gag ttt gag ctg     14390
Asp Gly Ser Gln Asp Glu Leu Thr Tyr Glu Trp Val Glu Phe Glu Leu
                160                 165                 170

ccc gag ggc aac ttc tcg gtg acc atg acc atc gat ctg atg aac aac     14438
Pro Glu Gly Asn Phe Ser Val Thr Met Thr Ile Asp Leu Met Asn Asn
            175                 180                 185

gcc atc atc gac aac tac ttg gcg gtg ggg cgg cag aac ggg gtg ctg     14486
Ala Ile Ile Asp Asn Tyr Leu Ala Val Gly Arg Gln Asn Gly Val Leu
        190                 195                 200

gag agc gac atc ggc gtg aag ttc gac acg cgc aac ttc cgg ctg ggc     14534
Glu Ser Asp Ile Gly Val Lys Phe Asp Thr Arg Asn Phe Arg Leu Gly
    205                 210                 215

tgg gac ccc gtg acc gag ctg gtg atg ccg ggc gtg tac acc aac gag     14582
Trp Asp Pro Val Thr Glu Leu Val Met Pro Gly Val Tyr Thr Asn Glu
220                 225                 230                 235

gcc ttc cac ccc gac atc gtc ctg ctg ccc ggc tgc ggc gtg gac ttc     14630
Ala Phe His Pro Asp Ile Val Leu Leu Pro Gly Cys Gly Val Asp Phe
                240                 245                 250

acc gag agc cgc ctc agc aac ctg ctg ggc atc cgc aag cgg cag ccc     14678

Thr Glu Ser Arg Leu Ser Asn Leu Leu Gly Ile Arg Lys Arg Gln Pro
            255                 260                 265

ttc cag gag ggc ttc cag atc ctg tac gag gac ctg gag ggg ggc aac      14726
Phe Gln Glu Gly Phe Gln Ile Leu Tyr Glu Asp Leu Glu Gly Gly Asn
        270                 275                 280

atc ccc gcg ctc ttg gat gtc gaa gcc tac gag aaa agc aag gag gat      14774
Ile Pro Ala Leu Leu Asp Val Glu Ala Tyr Glu Lys Ser Lys Glu Asp
    285                 290                 295

agc acc gcc gcg gcg acc gca gcc gtg gcc acc gcc tct acc gag gtg      14822
Ser Thr Ala Ala Ala Thr Ala Ala Val Ala Thr Ala Ser Thr Glu Val
300                 305                 310                 315

cgg ggc gat aat ttt gct agc gct gcg gca gcg gcc gag gcg gct gaa      14870
Arg Gly Asp Asn Phe Ala Ser Ala Ala Ala Ala Ala Glu Ala Ala Glu
                320                 325                 330

acc gaa agt aag ata gtc atc cag ccg gtg gag aag gac agc aag gac      14918
Thr Glu Ser Lys Ile Val Ile Gln Pro Val Glu Lys Asp Ser Lys Asp
            335                 340                 345

agg agc tac aac gtg ctc gcg gac aag aaa aac acc gcc tac cgc agc      14966
Arg Ser Tyr Asn Val Leu Ala Asp Lys Lys Asn Thr Ala Tyr Arg Ser
        350                 355                 360

tgg tac ctg gcc tac aac tac ggc gac ccc gag aag ggc gtg cgc tcc      15014
Trp Tyr Leu Ala Tyr Asn Tyr Gly Asp Pro Glu Lys Gly Val Arg Ser
    365                 370                 375

tgg acg ctg ctc acc acc tcg gac gtc acc tgc ggc gtg gag caa gtc      15062
Trp Thr Leu Leu Thr Thr Ser Asp Val Thr Cys Gly Val Glu Gln Val
380                 385                 390                 395

tac tgg tcg ctg ccc gac atg atg caa gac ccg gtc acc ttc cgc tcc      15110
Tyr Trp Ser Leu Pro Asp Met Met Gln Asp Pro Val Thr Phe Arg Ser
                400                 405                 410

acg cgt caa gtt agc aac tac ccg gtg gtg ggc gcc gag ctc ctg ccc      15158
Thr Arg Gln Val Ser Asn Tyr Pro Val Val Gly Ala Glu Leu Leu Pro
            415                 420                 425

gtc tac tcc aag agc ttc ttc aac gag cag gcc gtc tac tcg cag cag      15206
Val Tyr Ser Lys Ser Phe Phe Asn Glu Gln Ala Val Tyr Ser Gln Gln
        430                 435                 440

ctg cgc gcc ttc acc tcg ctc acg cac gtc ttc aac cgc ttc ccc gag      15254
Leu Arg Ala Phe Thr Ser Leu Thr His Val Phe Asn Arg Phe Pro Glu
    445                 450                 455

aac cag atc ctc gtc cgc ccg ccc gcg ccc acc att acc acc gtc agt      15302
Asn Gln Ile Leu Val Arg Pro Pro Ala Pro Thr Ile Thr Thr Val Ser
460                 465                 470                 475

gaa aac gtt cct gct ctc aca gat cac ggg acc ctg ccg ctg cgc agc      15350
Glu Asn Val Pro Ala Leu Thr Asp His Gly Thr Leu Pro Leu Arg Ser
                480                 485                 490

agt atc cgg gga gtc cag cgc gtg acc gtc act gac gcc aga cgc cgc      15398
Ser Ile Arg Gly Val Gln Arg Val Thr Val Thr Asp Ala Arg Arg Arg
            495                 500                 505

acc tgc ccc tac gtc tac aag gcc ctg ggc gta gtc gcg ccg cgc gtc     15446
Thr Cys Pro Tyr Val Tyr Lys Ala Leu Gly Val Val Ala Pro Arg Val
        510                 515                 520

ctc tcg agc cgc acc ttc taa aaaatgtcca ttctcatctc gcccagtaat     15497
Leu Ser Ser Arg Thr Phe
    525

aacaccggtt ggggcctgcg cgcgcccagc aagatgtacg gaggcgctcg ccaacgctcc     15557

acgcaacacc ccgtgcgcgt gcgcgggcac ttccgcgctc cctggggcgc cctcaagggc     15617

cgcgtgcgct cgcgcaccac cgtcgacgac gtgatcgacc aggtggtggc cgacgcgcgc     15677

aactacacgc ccgccgccgc gcccgtctcc accgtggacg ccgtcatcga cagcgtggtg     15737

gccgacgcgc gccggtacgc ccgcaccaag agccggcggc ggcgcatcgc ccggcggcac     15797

cggagcaccc ccgccatgcg cgcggcgcga gccttgctgc gcagggccag gcgcacggga     15857

cgcagggcca tgctcagggc ggccagacgc gcggcctccg gcagcagcag cgccggcagg     15917

acccgcagac gcgcggccac ggcggcggcg gcggccatcg ccagcatgtc ccgcccgcgg     15977

cgcggcaacg tgtactgggt gcgcgacgcc gccaccggtg tgcgcgtgcc cgtgcgcacc     16037

cgcccccctc gcacttgaag atgctgactt cgcgatgttg atgtgtccca gcggcgagga     16097

ggatgtccaa gcgcaaatac aaggaagaga tgctccaggt catcgcgcct gagatctacg     16157

gccccgcggc ggcggtgaag gaggaaagaa agccccgcaa actgaagcgg gtcaaaaagg     16217

acaaaaagga ggaggaagat gacggactgg tggagtttgt gcgcgagttc gccccccggc     16277

ggcgcgtgca gtggcgcggg cggaaagtga aaccggtgct gcggcccggc accacggtgg     16337

tcttcacgcc cggcgagcgt tccggctccg cctccaagcg ctcctacgac gaggtgtacg     16397

gggacgagga catcctcgag caggcggtcg agcgtctggg cgagtttgcg tacggcaagc     16457

gcagccgccc cgcgcccttg aaagaggagg cggtgtccat cccgctggac cacggcaacc     16517

ccacgccgag cctgaagccg gtgaccctgc agcaggtgct accgagcgcg gcgccgcgcc     16577

ggggcttcaa gcgcgagggc ggcgaggatc tgtacccgac catgcagctg atggtgccca     16637

agcgccagaa gctggaggac gtgctggagc acatgaaggt ggaccccgag gtgcagcccg     16697

aggtcaaggt gcggcccatc aagcaggtgg ccccgggcct gggcgtgcag accgtggaca     16757

tcaagatccc cacggagccc atggaaacgc agaccgagcc cgtgaagccc agcaccagca     16817

ccatggaggt gcagacggat ccctggatgc cagcaccagc ttccaccagc actcgccgaa     16877

gacgcaagta cggcgcggcc agcctgctga tgcccaacta cgcgctgcat ccttccatca     16937

tccccacgcc gggctaccgc ggcacgcgct tctaccgcgg ctacaccagc agccgccgcc     16997

gcaagaccac cacccgccgc cgtcgtcgca gccgccgcag cagcaccgcg acttccgcct     17057

tggtgcggag agtgtatcgc agcgggcgcg agcctctgac cctgccgcgc gcgcgctacc     17117

acccgagcat cgccatttaa ctaccgcctc ctacttgcag atatggccct cacatgccgc     17177

ctccgcgtcc ccattacggg ctaccgagga agaaagccgc gccgtagaag gctgacgggg 17237

aacgggctgc gtcgccatca ccaccggcgg cggcgcgcca tcagcaagcg gttgggggga 17297

ggcttcctgc ccgcgctgat ccccatcatc gccgcggcga tcggggcgat ccccggcata 17357

gcttccgtgg cggtgcaggc ctctcagcgc cactgagaca caaaaaagca tggatttgta 17417

ataaaaaaaa aaatggactg acgctcctgg tcctgtgatg tgtgttttta gatggaagac 17477

atcaattttt cgtccctggc accgcgacac ggcacgcggc cgtttatggg cacctggagc 17537

gacatcggca acagccaact gaacgggggc gccttcaatt ggagcagtct ctggagcggg 17597

cttaagaatt tcgggtccac gctcaaaacc tatggcaaca aggcgtggaa cagcagcaca 17657

gggcaggcgc tgagggaaaa gctgaaagaa cagaacttcc agcagaaggt ggttgatggc 17717

ctggcctcag gcatcaacgg ggtggttgac ctggccaacc aggccgtgca gaaacagatc 17777

aacagccgcc tggacgcggt cccgcccgcg gggtccgtgg agatgcccca ggtggaggag 17837

gagctgcctc ccctggacaa gcgcggcgac aagcgaccgc gtcccgacgc ggaggagacg 17897

ctgctgacgc acacggacga gccgcccccg tacgaggagg cggtgaaact gggcctgccc 17957

accacgcggc ccgtggcgcc tctggccacc ggagtgctga aacccagcag cagccagccc 18017

gcgaccctgg acttgcctcc gcctcgcccc tccacagtgg ctaagcccct gccgccggtg 18077

gccgtcgcgt cgcgcgcccc ccgaggccgc ccccaggcga actggcagag cactctgaac 18137

agcatcgtgg gtctgggagt gcagagtgtg aagcgccgcc gctgctatta aaagacactg 18197

tagcgcttaa cttgcttgtc tgtgtgtata tgtatgtccg ccgaccagaa ggaggagtgt 18257

gaagaggcgc gtcgccgagt tgcaag atg gcc acc cca tcg atg ctg ccc cag 18310
Met Ala Thr Pro Ser Met Leu Pro Gln
530                 535

tgg gcg tac atg cac atc gcc gga cag gac gct tcg gag tac ctg agt 18358
Trp Ala Tyr Met His Ile Ala Gly Gln Asp Ala Ser Glu Tyr Leu Ser
    540                 545                 550

ccg ggt ctg gtg cag ttc gcc cgc gcc aca gac acc tac ttc agt ctg 18406
Pro Gly Leu Val Gln Phe Ala Arg Ala Thr Asp Thr Tyr Phe Ser Leu
555                 560                 565                 570

ggg aac aag ttt agg aac ccc acg gtg gcg ccc acg cac gat gtg acc 18454
Gly Asn Lys Phe Arg Asn Pro Thr Val Ala Pro Thr His Asp Val Thr
                575                 580                 585

acc gac cgc agc cag cgg ctg acg ctg cgc ttc gtg ccc gtg gac cgc 18502
Thr Asp Arg Ser Gln Arg Leu Thr Leu Arg Phe Val Pro Val Asp Arg
            590                 595                 600

gag gac aac acc tac tcg tac aaa gtg cgc tac acg ctg gcc gtg ggc 18550
Glu Asp Asn Thr Tyr Ser Tyr Lys Val Arg Tyr Thr Leu Ala Val Gly
        605                 610                 615

gac aac cgc gtg ctg gac atg gcc agc acc tac ttt gac atc cgc ggc 18598
Asp Asn Arg Val Leu Asp Met Ala Ser Thr Tyr Phe Asp Ile Arg Gly

620                    625                    630

gtg ctg gac cgg ggc cct agc ttc aaa ccc tac tct ggc acc gcc tac      18646
Val Leu Asp Arg Gly Pro Ser Phe Lys Pro Tyr Ser Gly Thr Ala Tyr
635                 640                 645                 650

aac agc cta gct ccc aag gga gct ccc aat tcc agc cag tgg gag caa      18694
Asn Ser Leu Ala Pro Lys Gly Ala Pro Asn Ser Ser Gln Trp Glu Gln
                655                 660                 665

gca aaa aca ggc aat ggg gga act atg gaa aca cac aca tat ggt gtg      18742
Ala Lys Thr Gly Asn Gly Gly Thr Met Glu Thr His Thr Tyr Gly Val
            670                 675                 680

gcc cca atg ggc gga gag aat att aca aaa gat ggt ctt caa att gga      18790
Ala Pro Met Gly Gly Glu Asn Ile Thr Lys Asp Gly Leu Gln Ile Gly
        685                 690                 695

act gac gtt aca gcg aat cag aat aaa cca att tat gcc gac aaa aca      18838
Thr Asp Val Thr Ala Asn Gln Asn Lys Pro Ile Tyr Ala Asp Lys Thr
    700                 705                 710

ttt caa cca gaa ccg caa gta gga gaa gaa aat tgg caa gaa act gaa      18886
Phe Gln Pro Glu Pro Gln Val Gly Glu Glu Asn Trp Gln Glu Thr Glu
715                 720                 725                 730

aac ttt tat ggc ggt aga gct ctt aaa aaa gac aca aac atg aaa cct      18934
Asn Phe Tyr Gly Gly Arg Ala Leu Lys Lys Asp Thr Asn Met Lys Pro
                735                 740                 745

tgc tat ggc tcc tat gct aga ccc acc aat gaa aaa gga ggt caa gct      18982
Cys Tyr Gly Ser Tyr Ala Arg Pro Thr Asn Glu Lys Gly Gly Gln Ala
            750                 755                 760

aaa ctt aaa gtt gga gat gat gga gtt cca acc aaa gaa ttc gac ata      19030
Lys Leu Lys Val Gly Asp Asp Gly Val Pro Thr Lys Glu Phe Asp Ile
        765                 770                 775

gac ctg gct ttc ttt gat act ccc ggt ggc acc gtg aac ggt caa gac      19078
Asp Leu Ala Phe Phe Asp Thr Pro Gly Gly Thr Val Asn Gly Gln Asp
    780                 785                 790

gag tat aaa gca gac att gtc atg tat acc gaa aac acg tat ttg gaa      19126
Glu Tyr Lys Ala Asp Ile Val Met Tyr Thr Glu Asn Thr Tyr Leu Glu
795                 800                 805                 810

act cca gac acg cat gtg gta tac aaa cca ggc aag gat gat gca agt      19174
Thr Pro Asp Thr His Val Val Tyr Lys Pro Gly Lys Asp Asp Ala Ser
                815                 820                 825

tct gaa att aac ctg gtt cag cag tct atg ccc aac aga ccc aac tac      19222
Ser Glu Ile Asn Leu Val Gln Gln Ser Met Pro Asn Arg Pro Asn Tyr
            830                 835                 840

att ggg ttc agg gac aac ttt atc ggt ctt atg tac tac aac agc act      19270
Ile Gly Phe Arg Asp Asn Phe Ile Gly Leu Met Tyr Tyr Asn Ser Thr
        845                 850                 855

ggc aat atg ggt gtg ctt gct ggt cag gcc tcc cag ctg aat gct gtg      19318
Gly Asn Met Gly Val Leu Ala Gly Gln Ala Ser Gln Leu Asn Ala Val
    860                 865                 870

gtt gat ttg caa gac aga aac acc gag ctg tcc tac cag ctc ttg ctt      19366

Val Asp Leu Gln Asp Arg Asn Thr Glu Leu Ser Tyr Gln Leu Leu Leu
875                 880                 885                 890

gac tct ttg ggt gac aga acc cgg tat ttc agt atg tgg aac cag gcg     19414
Asp Ser Leu Gly Asp Arg Thr Arg Tyr Phe Ser Met Trp Asn Gln Ala
                895                 900                 905

gtg gac agt tat gac ccc gat gtg cgc atc atc gaa aac cat ggt gtg     19462
Val Asp Ser Tyr Asp Pro Asp Val Arg Ile Ile Glu Asn His Gly Val
            910                 915                 920

gag gat gaa ttg cca aac tat tgc ttc ccc ttg gac ggc tct ggc act     19510
Glu Asp Glu Leu Pro Asn Tyr Cys Phe Pro Leu Asp Gly Ser Gly Thr
        925                 930                 935

aac gcc gca tac caa ggt gtg aaa gta aaa gat ggt caa gat ggt gat     19558
Asn Ala Ala Tyr Gln Gly Val Lys Val Lys Asp Gly Gln Asp Gly Asp
    940                 945                 950

gtt gag agt gaa tgg gaa aat gac gat act gtt gca gct cga aat caa     19606
Val Glu Ser Glu Trp Glu Asn Asp Asp Thr Val Ala Ala Arg Asn Gln
955                 960                 965                 970

tta tgt aaa ggt aac att ttc gcc atg gag att aat ctc cag gct aac     19654
Leu Cys Lys Gly Asn Ile Phe Ala Met Glu Ile Asn Leu Gln Ala Asn
                975                 980                 985

ctg tgg aga agt ttc ctc tac tcg aac gtg gcc ctg tac ctg  ccc gac     19702
Leu Trp Arg Ser Phe Leu Tyr Ser Asn Val Ala Leu Tyr Leu  Pro Asp
            990                 995                 1000

tcc tac aag  tac acg ccg acc aac  gtc acg ctg ccg acc  aac acc     19747
Ser Tyr Lys  Tyr Thr Pro Thr Asn  Val Thr Leu Pro Thr  Asn Thr
        1005                 1010                 1015

aac acc tac  gat tac atg aat ggc  aga gtg aca cct ccc  tcg ctg     19792
Asn Thr Tyr  Asp Tyr Met Asn Gly  Arg Val Thr Pro Pro  Ser Leu
        1020                 1025                 1030

gta gac gcc  tac ctc aac atc ggg  gcg cgc tgg tcg ctg  gac ccc     19837
Val Asp Ala  Tyr Leu Asn Ile Gly  Ala Arg Trp Ser Leu  Asp Pro
        1035                 1040                 1045

atg gac aac  gtc aac ccc ttc aac  cac cac cgc aac gcg  ggc ctg     19882
Met Asp Asn  Val Asn Pro Phe Asn  His His Arg Asn Ala  Gly Leu
        1050                 1055                 1060

cgc tac cgc  tcc atg ctc ctg ggc  aac ggg cgc tac gtg  ccc ttc     19927
Arg Tyr Arg  Ser Met Leu Leu Gly  Asn Gly Arg Tyr Val  Pro Phe
        1065                 1070                 1075

cac atc cag  gtg ccc caa aag ttt  ttc gcc atc aag agc  ctc ctg     19972
His Ile Gln  Val Pro Gln Lys Phe  Phe Ala Ile Lys Ser  Leu Leu
        1080                 1085                 1090

ctc ctg ccc  ggg tcc tac acc tac  gag tgg aac ttc cgc  aag gac     20017
Leu Leu Pro  Gly Ser Tyr Thr Tyr  Glu Trp Asn Phe Arg  Lys Asp
        1095                 1100                 1105

gtc aac atg  atc ctg cag agc tcc  cta ggc aac gac ctg  cgc acg     20062
Val Asn Met  Ile Leu Gln Ser Ser  Leu Gly Asn Asp Leu  Arg Thr
        1110                 1115                 1120

gac ggg gcc tcc atc gcc ttc acc agc atc aac ctc tac gcc acc 20107
Asp Gly Ala Ser Ile Ala Phe Thr Ser Ile Asn Leu Tyr Ala Thr
        1125                1130                1135

ttc ttc ccc atg gcg cac aac acc gcc tcc acg ctc gag gcc atg 20152
Phe Phe Pro Met Ala His Asn Thr Ala Ser Thr Leu Glu Ala Met
        1140                1145                1150

ctg cgc aac gac acc aac gac cag tcc ttc aac gac tac ctc tcg 20197
Leu Arg Asn Asp Thr Asn Asp Gln Ser Phe Asn Asp Tyr Leu Ser
        1155                1160                1165

gcg gcc aac atg ctc tac ccc atc ccg gcc aac gcc acc aac gtg 20242
Ala Ala Asn Met Leu Tyr Pro Ile Pro Ala Asn Ala Thr Asn Val
        1170                1175                1180

ccc atc tcc atc ccc tcg cgc aac tgg gcc gcc ttc cgc gga tgg 20287
Pro Ile Ser Ile Pro Ser Arg Asn Trp Ala Ala Phe Arg Gly Trp
        1185                1190                1195

tcc ttc acg cgc ctg aag acc cgc gag acg ccc tcg ctc ggc tcc 20332
Ser Phe Thr Arg Leu Lys Thr Arg Glu Thr Pro Ser Leu Gly Ser
        1200                1205                1210

ggg ttc gac ccc tac ttc gtc tac tcg ggc tcc atc ccc tac cta 20377
Gly Phe Asp Pro Tyr Phe Val Tyr Ser Gly Ser Ile Pro Tyr Leu
        1215                1220                1225

gac ggc acc ttc tac ctc aac cac acc ttc aag aag gtc tcc atc 20422
Asp Gly Thr Phe Tyr Leu Asn His Thr Phe Lys Lys Val Ser Ile
        1230                1235                1240

acc ttc gac tcc tcc gtc agc tgg ccc ggc aac gac cgc ctc ctg 20467
Thr Phe Asp Ser Ser Val Ser Trp Pro Gly Asn Asp Arg Leu Leu
        1245                1250                1255

acg ccc aac gag ttc gaa atc aag cgc acc gtc gac gga gag gga 20512
Thr Pro Asn Glu Phe Glu Ile Lys Arg Thr Val Asp Gly Glu Gly
        1260                1265                1270

tac aac gtg gcc cag tgc aac atg acc aag gac tgg ttc ctg gtc 20557
Tyr Asn Val Ala Gln Cys Asn Met Thr Lys Asp Trp Phe Leu Val
        1275                1280                1285

cag atg ctg gcc cac tac aac atc ggc tac cag ggc ttc tac gtg 20602
Gln Met Leu Ala His Tyr Asn Ile Gly Tyr Gln Gly Phe Tyr Val
        1290                1295                1300

ccc gag ggc tac aag gac cgc atg tac tcc ttc ttc cgc aac ttc 20647
Pro Glu Gly Tyr Lys Asp Arg Met Tyr Ser Phe Phe Arg Asn Phe
        1305                1310                1315

cag ccc atg agc cgc cag gtc gtg gac gag gtc aac tac aag gac 20692
Gln Pro Met Ser Arg Gln Val Val Asp Glu Val Asn Tyr Lys Asp
        1320                1325                1330

tac cag gcc gtc acc ctg gcc tac cag cac aac aac tcg ggc ttc 20737
Tyr Gln Ala Val Thr Leu Ala Tyr Gln His Asn Asn Ser Gly Phe
        1335                1340                1345

gtc ggc tac ctc gcg ccc acc atg cgc cag ggc cag ccc tac ccc 20782
Val Gly Tyr Leu Ala Pro Thr Met Arg Gln Gly Gln Pro Tyr Pro
        1350                1355                1360

gcc aac tac ccc tac ccg ctc atc ggc aag agc gcc gtc gcc agc 20827
Ala Asn Tyr Pro Tyr Pro Leu Ile Gly Lys Ser Ala Val Ala Ser
        1365                1370                1375

gtc acc cag aaa aag ttc ctc tgc gac cgg gtc atg tgg cgc atc 20872
Val Thr Gln Lys Lys Phe Leu Cys Asp Arg Val Met Trp Arg Ile
        1380                1385                1390

ccc ttc tcc agc aac ttc atg tcc atg ggc gcg ctc acc gac ctc 20917
Pro Phe Ser Ser Asn Phe Met Ser Met Gly Ala Leu Thr Asp Leu
        1395                1400                1405

ggc cag aac atg ctc tac gcc aac tcc gcc cac gcg cta gac atg 20962
Gly Gln Asn Met Leu Tyr Ala Asn Ser Ala His Ala Leu Asp Met
        1410                1415                1420

aat ttc gaa gtc gac ccc atg gat gag tcc acc ctt ctc tat gtt 21007
Asn Phe Glu Val Asp Pro Met Asp Glu Ser Thr Leu Leu Tyr Val
        1425                1430                1435

gtc ttc gaa gtc ttc gac gtc gtc cga gtg cac cag ccc cac cgc 21052
Val Phe Glu Val Phe Asp Val Val Arg Val His Gln Pro His Arg
        1440                1445                1450

ggc gtc atc gaa gcc gtc tac ctg cgc acg ccc ttc tcg gcc ggc 21097
Gly Val Ile Glu Ala Val Tyr Leu Arg Thr Pro Phe Ser Ala Gly
        1455                1460                1465

aac gcc acc acc taa gccgctcttg cttcttgcaa gatgacggcg ggctccggcg 21152
Asn Ala Thr Thr
        1470

agcaggagct cagggccatc ctccgcgacc tgggctgcgg gccctgcttc ctgggcacct 21212

tcgacaagcg cttccctgga ttcatggccc cgcacaagct ggcctgcgcc atcgtgaaca 21272

cggccggccg cgagaccggg ggcgagcact ggctggcctt cgcctggaac ccgcgctccc 21332

acacatgcta cctcttcgac cccttcgggt tctcggacga gcgcctcaag cagatctacc 21392

agttcgagta cgagggcctg ctgcgtcgca gcgccctggc caccgaggac cgctgcgtca 21452

ccctggaaaa gtccacccag accgtgcagg gtccgcgctc ggccgcctgc gggctcttct 21512

gctgcatgtt cctgcacgcc ttcgtgcact ggcccgaccg ccccatggac aagaacccca 21572

ccatgaactt actgacgggg gtgcccaacg gcatgctcca gtcgccccag gtggaaccca 21632

ccctgcgccg caaccaggaa gcgctctacc gcttcctcaa tgcccactcc gcctactttc 21692

gctcccaccg cgcgcgcatc gagaaggcca ccgccttcga ccgcatgaat caagacatgt 21752

aaaaaaccgg tgtgtgtatg tgaatgcttt attcataata aacagcacat gtttatgcca 21812

ccttctctga ggctctgact ttatttagaa atcgaagggg ttctgccggc tctcggcatg 21872

gcccgcgggc agggatacgt tgcggaactg gtacttgggc agccacttga actcggggat 21932

cagcagcttg ggcacgggga ggtcggggaa cgagtcgctc cacagcttgc gcgtgagttg 21992

cagggcgccc agcaggtcgg gcgcggagat cttgaaatcg cagttgggac ccgcgttctg 22052

cgcgcgagag ttgcggtaca cggggttgca gcactggaac accatcaggg ccgggtgctt 22112
cacgcttgcc agcaccgtcg cgtcggtgat gccctccacg tccagatcct cggcgttggc 22172
catcccgaag ggggtcatct tgcaggtctg ccgccccatg ctgggcacgc agccgggctt 22232
gtggttgcaa tcgcagtgca gggggatcag catcatctgg gcctgctcgg agctcatgcc 22292
cgggtacatg gccttcatga aagcctccag ctggcggaag gcctgctgcg ccttgccgcc 22352
ctcggtgaag aagaccccgc aggacttgct agagaactgg ttggtggcgc agccggcgtc 22412
gtgcacgcag cagcgcgcgt cgttgttggc cagctgcacc acgctgcgcc cccagcggtt 22472
ctgggtgatc ttggcccggt tggggttctc cttcagcgcg cgctgcccgt tctcgctcgc 22532
cacatccatc tcgatagtgt gctccttctg gatcatcacg gtcccgtgca ggcaccgcag 22592
cttgccctcg gcttcggtgc agccgtgcag ccacagcgcg cagccggtgc actcccagtt 22652
cttgtgggcg atctgggagt gcgagtgcac gaagccctgc aggaagcggc ccatcatcgc 22712
ggtcagggtc ttgttgctgg tgaaggtcag cgggatgccg cggtgctcct cgttcacata 22772
caggtggcag atgcggcggt acacctcgcc ctgctcgggc atcagctgga aggcggactt 22832
caggtcgctc tccacgcggt accggtccat cagcagcgtc atcacttcca tgcccttctc 22892
ccaggccgaa acgatcggca ggctcagggg gttcttcacc gccattgtca tcttagtcgc 22952
cgccgccgag gtcagggggt cgttctcgtc cagggtctca aacactcgct tgccgtcctt 23012
ctcgatgatg cgcacggggg gaaagctgaa gcccacggcc gccagctcct cctcggcctg 23072
cctttcgtcc tcgctgtcct ggctgatgtc ttgcaaaggc acatgcttgg tcttgcgggg 23132
tttctttttg ggcggcagag gcggcggcga tgtgctggga gagcgcgagt tctcgttcac 23192
cacgactatt tcttcttctt ggccgtcgtc cgagaccacg cggcggtagg catgcctctt 23252
ctggggcaga ggcggaggcg acgggctctc gcggttcggc gggcggctgg cagagcccct 23312
tccgcgttcg ggggtgcgct cctggcggcg ctgctctgac tgacttcctc cgcggccggc 23372
cattgtgttc tcctagggag caacaacaag catggagact cagccatcgt cgccaacatc 23432
gccatctgcc cccgccgcca ccgccgacga gaaccagcag cagaatgaaa gcttaaccgc 23492
cccgccgccc agccccacct ccgacgccgc ggccccagac atgcaagaga tggaggaatc 23552
catcgagatt gacctgggct acgtgacgcc cgcggagcac gaggaggagc tggcagcgcg 23612
cttttcagcc ccggaagaga accaccaaga gcagccagag caggaagcag agaacgagca 23672
gaaccaggct gggcacgagc atggcgacta cctgagcggg gcagaggacg tgctcatcaa 23732
gcatctggcc cgccaatgca tcatcgtcaa ggacgcgctg ctcgaccgcg ccgaggtgcc 23792
cctcagcgtg gcggagctca gccgcgccta cgagcgcaac ctcttctcgc cgcgcgtgcc 23852
ccccaagcgc cagcccaacg gcacctgtga gcccaacccg cgcctcaact tctacccggt 23912
cttcgcggtg cccgaggccc tggccaccta ccacctcttt ttcaagaacc aaaggatccc 23972

cgtctcctgc cgcgccaacc gcacccgcgc cgacgccctg ctcaacctgg gccccggcgc 24032
ccgcctacct gatatcacct ccttggaaga ggttcccaag atcttcgagg gtctgggcag 24092
cgacgagact cgggccgcga acgctctgca aggaagcgga gaggagcatg agcaccacag 24152
cgccctggtg gagttggaag gcgacaacgc gcgcctggcg gtcctcaagc gcacggtcga 24212
gctgacccac ttcgcctacc cggcgctcaa cctgcccccc aaggtcatga gcgccgtcat 24272
ggaccaggtg ctcatcaagc gcgcctcgcc cctctcggag gaggagatgc aggaccccga 24332
gagttcggac gagggcaagc ccgtggtcag cgacgagcag ctggcgcgct ggctgggagc 24392
gagtagcacc ccccagagcc tggaagagcg gcgcaagctc atgatggccg tggtcctggt 24452
gaccgtggag ctggagtgtc tgcgccgctt ctttgccgac gcggagaccc tgcgcaaggt 24512
cgaggagaac ctgcactacc tcttcaggca cgggttcgtg cgccaggcct gcaagatctc 24572
caacgtggag ctgaccaacc tggtctccta catgggcatc ctgcacgaga accgcctggg 24632
gcaaaacgtg ctgcacacca ccctgcgcgg ggaggcccgc cgcgactaca tccgcgactg 24692
cgtctacctg tacctctgcc acacctggca gacgggcatg ggcgtgtggc agcagtgcct 24752
ggaggagcag aacctgaaag agctctgcaa gctcctgcag aagaacctca aggccctgtg 24812
gaccgggttc gacgagcgta ccaccgcctc ggacctggcc gacctcatct tccccgagcg 24872
cctgcggctg acgctgcgca acgggctgcc cgactttatg agccaaagca tgttgcaaaa 24932
ctttcgctct ttcatcctcg aacgctccgg gatcctgccc gccacctgct ccgcgctgcc 24992
ctcggacttc gtgccgctga ccttccgcga gtgccccccg ccgctctgga gccactgcta 25052
cttgctgcgc ctggccaact acctggccta ccactcggac gtgatcgagg acgtcagcgg 25112
cgagggtctg ctggagtgcc actgccgctg caacctctgc acgccgcacc gctccctggc 25172
ctgcaacccc cagctgctga gcgagaccca gatcatcggc accttcgagt tgcaaggccc 25232
cggcgacggc gagggcaagg ggggtctgaa actcaccccg gggctgtgga cctcggccta 25292
cttgcgcaag ttcgtgcccg aggactacca tcccttcgag atcaggttct acgaggacca 25352
atcccagccg cccaaggccg agctgtcggc ctgcgtcatc acccaggggg ccatcctggc 25412
ccaattgcaa gccatccaga aatcccgcca agaatttctg ctgaaaaagg gccacggggt 25472
ctacttggac ccccagaccg gagaggagct caaccccagc ttcccccagg atgccccgag 25532
gaagcagcaa gaagctgaaa gtggagctgc cgccgccgga ggatttggag gaagactggg 25592
agagcagtca ggcagaggag gaggagatgg aagactggga cagcactcag gcagaggagg 25652
acagcctgca agacagtctg gaggaggaag acgaggtgga ggaggcagag gaagaagcag 25712
ccgccgccag accgtcgtcc tcggcggaga aagcaagcag cacggatacc atctccgctc 25772
cgggtcgggg tcgcggcggc cgggcccaca gtaggtggga cgagaccggg cgcttcccga 25832

accccaccac ccagaccggt aagaaggagc ggcagggata caagtcctgg cgggggcaca 25892
aaaacgccat cgtctcctgc ttgcaagcct gcgggggcaa catctccttc acccggcgct 25952
acctgctctt ccaccgcggg gtgaacttcc cccgcaacat cttgcattac taccgtcacc 26012
tccacagccc ctactactgt ttccaagaag aggcagaaac ccagcagcag cagaaaacca 26072
gcggcagcag cagctagaaa atccacagcg gcggcaggtg gactgaggat cgcggcgaac 26132
gagccggcgc agacccggga gctgaggaac cggatctttc ccaccctcta tgccatcttc 26192
cagcagagtc gggggcagga gcaggaactg aaagtcaaga accgttctct gcgctcgctc 26252
acccgcagtt gtctgtatca caagagcgaa gaccaacttc agcgcactct cgaggacgcc 26312
gaggctctct tcaacaagta ctgcgcgctc actcttaaag agtagcccgc gcccgcccac 26372
acacggaaaa aggcgggaat tacgtcacca cctgcgccct tcgcccgacc atcatgagca 26432
aagagattcc cacgccttac atgtggagct accagcccca gatgggcctg gccgccggcg 26492
ccgcccagga ctactccacc cgcatgaact ggctcagtgc cgggcccgcg atgatctcac 26552
gggtgaatga catccgcgcc caccgaaacc agatactcct agaacagtca gcgatcaccg 26612
ccacgccccg ccatcacctt aatccgcgta attggcccgc cgccctggtg taccaggaaa 26672
ttccccagcc cacgaccgta ctacttccgc gagacgccca ggccgaagtc cagctgacta 26732
actcaggtgt ccagctggcc ggcggcgccg ccctgtgtcg tcaccgcccc gctcagggta 26792
taaagcggct ggtgatccga ggcagaggca cacagctcaa cgacgaggtg gtgagctctt 26852
cgctgggtct gcgacctgac ggagtcttcc aactcgccgg atcggggaga tcttccttca 26912
cgcctcgtca ggccgtcctg actttggaga gttcgtcctc gcagccccgc tcgggcggca 26972
tcggcactct ccagttcgtg gaggagttca ctccctcggt ctacttcaac cccttctccg 27032
gctcccccgg ccactacccg gacgagttca tcccgaactt cgacgccatc agcgagtcgg 27092
tggacggcta cgattgaatg tcccatggtg gcgcagctga cctagctcgg cttcgacacc 27152
tggaccactg ccgccgcttc cgctgcttcg ctcgggatct cgccgagttt gcctactttg 27212
agctgcccga ggagcaccct cagggcccag cccacggagt gcggatcatc gtcgaagggg 27272
gcctcgactc ccacctgctt cggatcttca gccagcgacc gatcctggtc gagcgcgaac 27332
aaggacagac ccttcttact ttgtactgca tctgcaacca ccccggcctg catgaaagtc 27392
tttgttgtct gctgtgtact gagtataata aaagctgaga tcagcgacta ctccggactc 27452
gattgtggtg ttcctgctat caaccggtcc ctgttcttca ccgggaacga gaccgagctc 27512
cagctccagt gtaagcccca caagaagtac ctcacctggc tgttccaggg ctccccgatc 27572
gccgttgtca accactgcga caacgacgga gtcctgctga gcggccctgc caaccttact 27632
ttttccaccc gcagaagcaa gctccagctc ttccaaccct tcctccccgg gacctatcag 27692
tgcgtctcag gaccctgcca tcacaccttc cacctgatcc cgaataccac agcgccgctc 27752

cccgctacta acaaccaaac tacccaccaa cgccaccgtc gcgacctttc ctctgaatct 27812
aataccacta ccggaggtga gctccgaggt cgaccaacct ctgggattta ctacggcccc 27872
tgggaggtgg tggggttaat agcgctaggc ctagttgcgg gtgggctttt ggttctctgc 27932
tacctatacc tcccttgctg ttcgtactta gtggtgctgt gttgctggtt taagaaatgg 27992
ggaagatcac cctagtgagc tgcggtgcgc tggtggcggt gttgctttcg attgtgggac 28052
tgggcggcgc ggctgtagtg aaggagaagg ccgatccctg cttgcatttc aatcccaaca 28112
aatgccagct gagttttcag cccgatggca atcggtgcgc ggtactgatc aagtgcggat 28172
gggaatgcga gaacgtgaga atcgagtaca ataacaagac tcggaacaat actctcgcgt 28232
ccgtgtggca gcccggggac cccgagtggt acaccgtctc tgtccccggt gctgacggct 28292
ccccgcgcac cgtgaataat actttcattt ttgcgcacat gtgcaacacg gtcatgtgga 28352
tgagcaagca gtacgatatg tggcccccca cgaaggagaa catcgtggtc ttctccatcg 28412
cttacagcct gtgcacggcg ctaatcaccg ctatcgtgtg cctgagcatt cacatgctca 28472
tcgctattcg ccccagaaat aatgccgaga aagagaaaca gccataacac gttttttcac 28532
acaccttgtt tttacagaca atgcgtctgt taaatttttt aaacattgtg ctcagtattg 28592
cttatgcctc tggttatgca aacatacaga aaacccttta tgtaggatct gatggtacac 28652
tagagggtac ccaatcacaa gccaaggttg catggtattt ttatagaacc aacactgatc 28712
cagttaaact ttgtaagggt gaattgccgc gtacacataa aactccactt acatttagtt 28772
gcagcaataa taatcttaca cttttttcaa ttacaaaaca atatactggt acttattaca 28832
gtacaaactt tcatacagga caagataaat attatactgt taaggtagaa aatcctacca 28892
ctcctagaac taccaccacc accactactg caaagcccac tgtgaaaact acaactagga 28952
ccaccacaac tacagaaacc accaccagca caacacttgc tgcaactaca cacacacaca 29012
ctaagctaac cttacagacc actaatgatt tgatcgccct gctgcaaaag ggggataaca 29072
gcaccacttc caatgaggag atacccaaat ccatgattgg cattattgtt gctgtagtgg 29132
tgtgcatgtt gatcatcgcc ttgtgcatgg tgtactatgc cttctgctac agaaagcaca 29192
gactgaacga caagctggaa cacttactaa gtgttgaatt ttaatttttt agaaccatga 29252
agatcctagg cctttttagt ttttctatca ttacctctgc tctttgtgaa tcagtggata 29312
gagatgttac tattaccact ggttctaatt atacactgaa agggccaccc tcaggtatgc 29372
tttcgtggta ttgctatttt ggaactgaca ctgatcaaac tgaattatgc aattttcaaa 29432
aaggcaaaac ctcaaactct aaaatctcta attatcaatg caatggcact gatctgatac 29492
tactcaatgt cacgaaagca tatggtggca gttattattg ccctggacaa aacactgaag 29552
aaatgatttt ttacaaagtg gaagtggttg atcccactac accacccacc accacaacta 29612

ttcataccac acacacagaa caaacaccag aggcaacaga agcagagttg gccttccagg 29672
ttcacggaga ttcctttgct gtcaataccc ctacacccga tcagcggtgt ccggggccgc 29732
tagtcagcgg cattgtcggt gtgctttcgg gattagcagt cataatcatc tgcatgttca 29792
tttttgcttg ctgctataga aggctttacc gacaaaaatc agacccactg ctgaacctct 29852
atgtttaatt ttttccagag ccatgaaggc agttagcgct ctagtttttt gttctttgat 29912
tggcattgtt tttaatagta aaattaccag agttagcttt attaaacatg ttaatgtaac 29972
tgaaggagat aacatcacac tagcaggtgt agaaggtgct caaaacacca cctggacaaa 30032
ataccatcta ggatggagag atatttgcac ctggaatgta acttattatt gcataggagt 30092
taatcttacc attgttaacg ctaaccaatc tcagaatggg ttaattaaag gacagagtgt 30152
tagtgtgacc agtgatgggt actataccca gcatagtttt aactacaaca ttactgtcat 30212
accactgcct acgcctagcc cacctagcac taccacacag acaaccacat acagtacatc 30272
aaatcagcct accaccacta cagcagcaga ggttgccagc tcgtctgggg tccgagtggc 30332
atttttgatg ttggccccat ctagcagtcc cactgctagt accaatgagc agactactga 30392
atttttgtcc actgtcgaga gccacaccac agctacctcc agtgccttct ctagcaccgc 30452
caatctctcc tcgctttcct ctacaccaat cagccccgct actactccta gccccgctcc 30512
tcttcccact cccctgaagc aaacagacgg cggcatgcaa tggcagatca ccctgctcat 30572
tgtgatcggg ttggtcatcc tggccgtgtt gctctactac atcttctgcc gccgcattcc 30632
caacgcgcac cgcaagccgg cctacaagcc catcgttatc gggcagccgg agccgcttca 30692
ggtggaaggg ggtctaagga atcttctctt ctcttttaca gtatggtgat tgaactatga 30752
ttcctagaca attcttgatc actattctta tctgcctcct ccaagtctgt gccaccctcg 30812
ctctggtggc caacgccagt ccagactgta ttgggccctt cgcctcctac gtgctctttg 30872
ccttcgtcac ctgcatctgc tgctgtagca tagtctgcct gcttatcacc ttcttccagt 30932
tcattgactg gatctttgtg cgcatcgcct acctgcgcca ccacccccag taccgcgacc 30992
agcgagtggc gcagctgctc aggctcctct gataagcatg cgggctctgc tacttctcgc 31052
gcttctgctg ttagtgctcc cccgtcccgt cgaccccggg tcccccactc agtcccccga 31112
ggaggttcgc aaatgcaaat tccaagaacc ctggaaattc ctcaaatgct accgccaaaa 31172
atcagacatg catcccagct ggatcatgat cattgggatc gtgaacattc tggcctgcac 31232
cctcatctcc tttgtgattt acccctgctt tgactttggt tggaactcgc cagaggcgct 31292
ctatctcccg cctgaacctg acacaccacc acagcagcaa cctcaggcac acgcactacc 31352
accaccacag cctaggccac aatacatgcc catattagac tatgaggccg agccacagcg 31412
acccatgctc cccgctatta gttacttcaa tctaaccggc ggagatgact gacccactgg 31472
ccaataacaa cgtcaacgac cttctcctgg acatggacgg ccgcgcctcg gagcagcgac 31532

tcgcccaact tcgcattcgt cagcagcagg agagagccgt caaggagctg caggacggca 31592

tagccatcca ccagtgcaag agaggcatct tctgcctggt gaaacaggcc aagatctcct 31652

acgaggtcac ccagaccgac catcgcctct cctacgagct cctgcagcag cgccagaagt 31712

tcacctgcct ggtcggagtc aaccccatcg tcatcaccca gcagtcgggc gataccaagg 31772

ggtgcatcca ctgctcctgc gactcccccg actgcgtcca cactctgatc aagaccctct 31832

gcggcctccg cgacctcctc cccatgaact aatcaccccc ttatccagtg aaataaagat 31892

catattgatg atgatttaaa taaaaaaaat aatcatttga tttgaaataa agatacaatc 31952

atattgatga tttgagttta acaaaaataa agaatcactt acttgaaatc tgataccagg 32012

tctctgtcca tgttttctgc caacaccacc tcactcccct cttcccagct ctggtactgc 32072

aggccccggc gggctgcaaa cttcctccac acgctgaagg ggatgtcaaa ttcctcctgt 32132

ccctcaatct tcattttatc ttctatcag atg tcc aaa aag cgc gtc cgg gtg 32185
Met Ser Lys Lys Arg Val Arg Val
            1475

gat gat gac ttc gac ccc gtc tac ccc tac gat gca gac aac gca 32230
Asp Asp Asp Phe Asp Pro Val Tyr Pro Tyr Asp Ala Asp Asn Ala
1480                1485                1490

ccg acc gtg ccc ttc atc aac ccc ccc ttc gtc tct tca gat gga 32275
Pro Thr Val Pro Phe Ile Asn Pro Pro Phe Val Ser Ser Asp Gly
1495                1500                1505

ttc caa gag aag ccc ctg ggg gtg ttg tcc ctg cga ctg gct gac 32320
Phe Gln Glu Lys Pro Leu Gly Val Leu Ser Leu Arg Leu Ala Asp
1510                1515                1520

ccc gtc acc acc aag aac ggg gaa atc acc ctc aag ctg gga gag 32365
Pro Val Thr Thr Lys Asn Gly Glu Ile Thr Leu Lys Leu Gly Glu
1525                1530                1535

ggg gtg gac ctc gac tcg tcg gga aaa ctc atc tcc aac acg gcc 32410
Gly Val Asp Leu Asp Ser Ser Gly Lys Leu Ile Ser Asn Thr Ala
1540                1545                1550

acc aag gcc gcc gcc cct ctc agt att tca aac aac acc att tcc 32455
Thr Lys Ala Ala Ala Pro Leu Ser Ile Ser Asn Asn Thr Ile Ser
1555                1560                1565

ctt aaa act gct gcc cct ttc tac aac aac aat gga act tta agc 32500
Leu Lys Thr Ala Ala Pro Phe Tyr Asn Asn Asn Gly Thr Leu Ser
1570                1575                1580

ctc aat gtc tcc aca cca tta gca gta ttt ccc aca ttt aac act 32545
Leu Asn Val Ser Thr Pro Leu Ala Val Phe Pro Thr Phe Asn Thr
1585                1590                1595

tta ggc ata agt ctt gga aac ggt ctt cag act tca aat aag ttg 32590
Leu Gly Ile Ser Leu Gly Asn Gly Leu Gln Thr Ser Asn Lys Leu
1600                1605                1610

ttg act gta caa cta act cat cct ctt aca ttc agc tca aat agc 32635
Leu Thr Val Gln Leu Thr His Pro Leu Thr Phe Ser Ser Asn Ser

1615                    1620                    1625

atc aca gta aaa aca gac aaa ggg cta tat att aac tcc agt gga      32680
Ile Thr Val Lys Thr Asp Lys Gly Leu Tyr Ile Asn Ser Ser Gly
1630                    1635                    1640

aac aga gga ctt gag gct aat ata agc cta aaa aga gga cta gtt      32725
Asn Arg Gly Leu Glu Ala Asn Ile Ser Leu Lys Arg Gly Leu Val
1645                    1650                    1655

ttt gac ggt aat gct att gca aca tat att gga aat ggc tta gac      32770
Phe Asp Gly Asn Ala Ile Ala Thr Tyr Ile Gly Asn Gly Leu Asp
1660                    1665                    1670

tat gga tct tat gat agt gat gga aaa aca aga ccc gta att acc      32815
Tyr Gly Ser Tyr Asp Ser Asp Gly Lys Thr Arg Pro Val Ile Thr
1675                    1680                    1685

aaa att gga gca gga tta aat ttt gat gct aac aaa gca ata gct      32860
Lys Ile Gly Ala Gly Leu Asn Phe Asp Ala Asn Lys Ala Ile Ala
1690                    1695                    1700

gtc aaa cta ggc aca ggt tta agt ttt gac tcc gct ggt gcc ttg      32905
Val Lys Leu Gly Thr Gly Leu Ser Phe Asp Ser Ala Gly Ala Leu
1705                    1710                    1715

aca gct gga aac aaa cag gat gac aag cta aca ctt tgg act acc      32950
Thr Ala Gly Asn Lys Gln Asp Asp Lys Leu Thr Leu Trp Thr Thr
1720                    1725                    1730

cct gac cca agc cct aat tgt caa tta ctt tca gac aga gat gcc      32995
Pro Asp Pro Ser Pro Asn Cys Gln Leu Leu Ser Asp Arg Asp Ala
1735                    1740                    1745

aaa ttt act ctc tgt ctt aca aaa tgc ggt agt caa ata cta ggc      33040
Lys Phe Thr Leu Cys Leu Thr Lys Cys Gly Ser Gln Ile Leu Gly
1750                    1755                    1760

act gtg gca gtg gcg gct gtt act gta gga tca gca cta aat cca      33085
Thr Val Ala Val Ala Ala Val Thr Val Gly Ser Ala Leu Asn Pro
1765                    1770                    1775

att aat gac aca gtc aaa agc gcc ata gtt ttc ctt aga ttt gat      33130
Ile Asn Asp Thr Val Lys Ser Ala Ile Val Phe Leu Arg Phe Asp
1780                    1785                    1790

tcc gat ggt gta ctc atg tca aac tca tca atg gta ggt gat tac      33175
Ser Asp Gly Val Leu Met Ser Asn Ser Ser Met Val Gly Asp Tyr
1795                    1800                    1805

tgg aac ttt agg gag gga cag acc act caa agt gta gcc tat aca      33220
Trp Asn Phe Arg Glu Gly Gln Thr Thr Gln Ser Val Ala Tyr Thr
1810                    1815                    1820

aat gct gtg gga ttc atg cca aat ata ggt gca tat cca aaa acc      33265
Asn Ala Val Gly Phe Met Pro Asn Ile Gly Ala Tyr Pro Lys Thr
1825                    1830                    1835

caa agt aaa aca cct aaa aat agc ata gtc agt cag gta tat tta      33310
Gln Ser Lys Thr Pro Lys Asn Ser Ile Val Ser Gln Val Tyr Leu
1840                    1845                    1850

act gga gaa act act atg cca atg aca cta acc ata act ttc aat      33355

Thr Gly Glu Thr Thr Met Pro Met Thr Leu Thr Ile Thr Phe Asn
1855                1860                1865

ggc act gat gaa aaa gac aca acc cca gtt agc acc tac tct atg 33400
Gly Thr Asp Glu Lys Asp Thr Thr Pro Val Ser Thr Tyr Ser Met
1870                1875                1880

act ttt aca tgg cag tgg act gga gac tat aag gac aaa aat att 33445
Thr Phe Thr Trp Gln Trp Thr Gly Asp Tyr Lys Asp Lys Asn Ile
1885                1890                1895

acc ttt gct acc aac tca ttc tct ttt tcc tac atc gcc cag gaa 33490
Thr Phe Ala Thr Asn Ser Phe Ser Phe Ser Tyr Ile Ala Gln Glu
1900                1905                1910

taa tcccacccag caagccaacc ccttttccca ccacctttgt ctatatggaa 33543

actctgaaac agaaaaataa agttcaagtg ttttattgaa tcaacagttt tacaggactc 33603

gagcagttat ttttcctcca ccctcccagg acatggaata caccaccctc tccccccgca 33663

cagccttgaa catctgaatg ccattggtga tggacatgct tttggtctcc acgttccaca 33723

cagtttcaga gcgagccagt ctcggatcgg tcagggagat gaaaccctcc gggcactccc 33783

gcatctgcac ctcacagctc aacagctgag gattgtcctc ggtggtcggg atcacggtta 33843

tctggaagaa gcagaagagc ggcggtggga atcatagtcc gcgaacggga tcggccggtg 33903

gtgtcgcatc aggccccgca gcagtcgctg ccgccgccgc tccgtcaagc tgctgctcag 33963

ggggttcggg tccagggact ccctcagcat gatgcccacg gccctcagca tcagtcgtct 34023

ggtgcggcgg gcgcagcagc gcatgcgaat ctcgctcagg tcactgcagt acgtgcaaca 34083

caggaccacc aggttgttca acagtccata gttcaacacg ctccagccga aactcatcgc 34143

gggaaggatg ctacccacgt ggccgtcgta ccagatcctc aggtaaatca agtggcgctc 34203

cctccagaag acgctgccca tgtacatgat ctccttgggc atgtggcggt tcaccacctc 34263

ccggtaccac atcaccctct ggttgaacat gcagccccgg atgatcctgc ggaaccacag 34323

ggccagcacc gccccgcccg ccatgcagcg aagagacccc ggatcccggc aatgacaatg 34383

gaggacccac cgctcgtacc cgtggatcat ctgggagctg aacaagtcta tgttggcaca 34443

gcacaggcat atgctcatgc atctcttcag cactctcagc tcctcggggg tcaaaaccat 34503

atcccagggc acggggaact cttgcaggac agcgaacccc gcagaacagg gcaatcctcg 34563

cacataactt acattgtgca tggacagggt atcgcaatca ggcagcaccg ggtgatcctc 34623

caccagagaa gcgcgggtct cggtctcctc acagcgtggt aagggggccg gccgatacgg 34683

gtgatggcgg gacgcggctg atcgtgttct cgaccgtgtc atgatgcagt tgctttcgga 34743

cattttcgta cttgctgtag cagaacctgg tccgggcgct gcacaccgat cgccggcggc 34803

ggtctcggcg cttggaacgc tcggtgttaa agttgtaaaa cagccactct ctcagaccgt 34863

gcagcagatc tagggcctca ggagtgatga agatcccatc atgcctgata gctctgatca 34923

catcgaccac cgtggaatgg gccaggccca gccagatgat gcaattttgt tgggtttcgg 34983
tgacggcggg ggagggaaga acaggaagaa ccatgattaa cttttaatcc aaacggtctc 35043
ggagcacttc aaaatgaagg tcacggagat ggcacctctc gcccccgctg tgttggtgga 35103
aaataacagc caggtcaaag gtgatacggt tctcgagatg ttccacggtg gcttccagca 35163
aagcctccac gcgcacatcc agaaacaaga caatagcgaa agcgggaggg ttctctaatt 35223
cctcaaccat catgttacac tcctgcacca tccccagata attttcattt ttccagcctt 35283
gaatgattcg aactagttcc tgaggtaaat ccaagccagc catgataaaa agctcgcgca 35343
gagcaccctc caccggcatt cttaagcaca ccctcataat tccaagatat tctgctcctg 35403
gttcacctgc agcagattga caagcggaat atcaaaatct ctgccgcgat ccctgagctc 35463
ctccctcagc aataactgta agtactcttt catatcgtct ccgaaatttt tagccatagg 35523
acccccagga ataagagaag ggcaagccac attacagata aaccgaagtc ccccccagtg 35583
agcattgcca aatgtaagat tgaaataagc atgctggcta gacccggtga tatcttccag 35643
ataactggac agaaaatcgg gtaagcaatt tttaagaaaa tcaacaaaag aaaaatcttc 35703
caggtgcacg tttagggcct cgggaacaac gatggagtaa gtgcaagggg tgcgttccag 35763
catggttagt tagctgatct gtaaaaaaac aaaaaataaa acattaaacc atgctagcct 35823
ggcgaacagg tgggtaaatc gttctctcca gcaccaggca ggccacgggg tctccggcgc 35883
gaccctcgta aaaattgtcg ctatgattga aaaccatcac agagagacgt tcccggtggc 35943
cggcgtgaat gattcgagaa gaagcataca cccccggaac attggagtcc gtgagtgaaa 36003
aaaagcggcc gaggaagcaa tgaggcacta caacgctcac tctcaagtcc agcaaagcga 36063
tgccatgcgg atgaagcaca aaattttcag gtgcgtaaaa aatgtaatta ctcccctcct 36123
gcacaggcag cgaagctccc gatccctcca gatacacata caaagcctca gcgtccatag 36183
cttaccgagc ggcagcagca gcggcacaca acaggcgcaa gagtcagaga aaagactgag 36243
ctctaacctg tccgcccgct ctctgctcaa tatatagccc cagatctaca ctgacgtaaa 36303
ggccaaagtc taaaaatacc cgccaaataa tcacacacgc ccagcacacg cccagaaacc 36363
ggtgacacac tcagaaaaat acgcgcactt cctcaaacgg ccaaactgcc gtcatttccg 36423
ggttcccacg ctacgtcatc aaaacacgac tttcaaattc cgtcgaccgt taaaaacatc 36483
acccgccccg cccctaacgg tcgccgctcc cgcagccaat caccttcctc cctccccaaa 36543
ttcaaacagc tcatttgcat attaacgcgc accaaaagtt tgaggtatat tattgatgat 36603
g 36604

<210> 6
<211> 529

<212>  PRT

<213>  chimpanzee adenovirus serotype Pan6

<400>  6

Met Met Arg Arg Val Tyr Pro Glu Gly Pro Pro Pro Ser Tyr Glu Ser
1               5                   10                  15

Val Met Gln Gln Ala Val Ala Ala Ala Met Gln Pro Pro Leu Glu Ala
            20                  25                  30

Pro Tyr Val Pro Pro Arg Tyr Leu Ala Pro Thr Glu Gly Arg Asn Ser
        35                  40                  45

Ile Arg Tyr Ser Glu Leu Ala Pro Leu Tyr Asp Thr Thr Arg Leu Tyr
    50                  55                  60

Leu Val Asp Asn Lys Ser Ala Asp Ile Ala Ser Leu Asn Tyr Gln Asn
65                  70                  75                  80

Asp His Ser Asn Phe Leu Thr Thr Val Val Gln Asn Asn Asp Phe Thr
                85                  90                  95

Pro Thr Glu Ala Ser Thr Gln Thr Ile Asn Phe Asp Glu Arg Ser Arg
            100                 105                 110

Trp Gly Gly Gln Leu Lys Thr Ile Met His Thr Asn Met Pro Asn Val
        115                 120                 125

Asn Glu Phe Met Tyr Ser Asn Lys Phe Lys Ala Arg Val Met Val Ser
    130                 135                 140

Arg Lys Thr Pro Asn Gly Val Asp Asp Asp Tyr Asp Gly Ser Gln Asp
145                 150                 155                 160

Glu Leu Thr Tyr Glu Trp Val Glu Phe Glu Leu Pro Glu Gly Asn Phe
                165                 170                 175

Ser Val Thr Met Thr Ile Asp Leu Met Asn Asn Ala Ile Ile Asp Asn
            180                 185                 190

Tyr Leu Ala Val Gly Arg Gln Asn Gly Val Leu Glu Ser Asp Ile Gly
        195                 200                 205

Val Lys Phe Asp Thr Arg Asn Phe Arg Leu Gly Trp Asp Pro Val Thr
    210                 215                 220

Glu Leu Val Met Pro Gly Val Tyr Thr Asn Glu Ala Phe His Pro Asp
225                 230                 235                 240

Ile Val Leu Leu Pro Gly Cys Gly Val Asp Phe Thr Glu Ser Arg Leu
                245                 250                 255

Ser Asn Leu Leu Gly Ile Arg Lys Arg Gln Pro Phe Gln Glu Gly Phe
            260                 265                 270

Gln Ile Leu Tyr Glu Asp Leu Glu Gly Gly Asn Ile Pro Ala Leu Leu
        275                 280                 285

Asp Val Glu Ala Tyr Glu Lys Ser Lys Glu Asp Ser Thr Ala Ala Ala
    290                 295                 300

Thr Ala Ala Val Ala Thr Ala Ser Thr Glu Val Arg Gly Asp Asn Phe
305                 310                 315                 320

Ala Ser Ala Ala Ala Ala Ala Glu Ala Ala Glu Thr Glu Ser Lys Ile
                325                 330                 335

Val Ile Gln Pro Val Glu Lys Asp Ser Lys Asp Arg Ser Tyr Asn Val
            340                 345                 350

Leu Ala Asp Lys Lys Asn Thr Ala Tyr Arg Ser Trp Tyr Leu Ala Tyr
        355                 360                 365

Asn Tyr Gly Asp Pro Glu Lys Gly Val Arg Ser Trp Thr Leu Leu Thr
    370                 375                 380

Thr Ser Asp Val Thr Cys Gly Val Glu Gln Val Tyr Trp Ser Leu Pro
385                 390                 395                 400

Asp Met Met Gln Asp Pro Val Thr Phe Arg Ser Thr Arg Gln Val Ser
                405                 410                 415

Asn Tyr Pro Val Val Gly Ala Glu Leu Leu Pro Val Tyr Ser Lys Ser
            420                 425                 430

Phe Phe Asn Glu Gln Ala Val Tyr Ser Gln Gln Leu Arg Ala Phe Thr
        435                 440                 445

Ser Leu Thr His Val Phe Asn Arg Phe Pro Glu Asn Gln Ile Leu Val
    450                 455                 460

Arg Pro Pro Ala Pro Thr Ile Thr Thr Val Ser Glu Asn Val Pro Ala
465                 470                 475                 480

Leu Thr Asp His Gly Thr Leu Pro Leu Arg Ser Ser Ile Arg Gly Val
                485                 490                 495

Gln Arg Val Thr Val Thr Asp Ala Arg Arg Arg Thr Cys Pro Tyr Val
            500                 505                 510

Tyr Lys Ala Leu Gly Val Val Ala Pro Arg Val Leu Ser Ser Arg Thr
        515                 520                 525

Phe

<210>  7

<211>  942

<212>  PRT

<213>  chimpanzee adenovirus serotype Pan6

<400>  7

Met Ala Thr Pro Ser Met Leu Pro Gln Trp Ala Tyr Met His Ile Ala
1               5                   10                  15

Gly Gln Asp Ala Ser Glu Tyr Leu Ser Pro Gly Leu Val Gln Phe Ala
            20                  25                  30

Arg Ala Thr Asp Thr Tyr Phe Ser Leu Gly Asn Lys Phe Arg Asn Pro
        35                  40                  45

Thr Val Ala Pro Thr His Asp Val Thr Thr Asp Arg Ser Gln Arg Leu
    50                  55                  60

Thr Leu Arg Phe Val Pro Val Asp Arg Glu Asp Asn Thr Tyr Ser Tyr
65                  70                  75                  80

Lys Val Arg Tyr Thr Leu Ala Val Gly Asp Asn Arg Val Leu Asp Met
                85                  90                  95

Ala Ser Thr Tyr Phe Asp Ile Arg Gly Val Leu Asp Arg Gly Pro Ser
            100                 105                 110

Phe Lys Pro Tyr Ser Gly Thr Ala Tyr Asn Ser Leu Ala Pro Lys Gly
        115                 120                 125

Ala Pro Asn Ser Ser Gln Trp Glu Gln Ala Lys Thr Gly Asn Gly Gly

130                 135                 140

Thr Met Glu Thr His Thr Tyr Gly Val Ala Pro Met Gly Gly Glu Asn
145                 150                 155                 160

Ile Thr Lys Asp Gly Leu Gln Ile Gly Thr Asp Val Thr Ala Asn Gln
                165                 170                 175

Asn Lys Pro Ile Tyr Ala Asp Lys Thr Phe Gln Pro Glu Pro Gln Val
            180                 185                 190

Gly Glu Glu Asn Trp Gln Glu Thr Glu Asn Phe Tyr Gly Gly Arg Ala
        195                 200                 205

Leu Lys Lys Asp Thr Asn Met Lys Pro Cys Tyr Gly Ser Tyr Ala Arg
    210                 215                 220

Pro Thr Asn Glu Lys Gly Gly Gln Ala Lys Leu Lys Val Gly Asp Asp
225                 230                 235                 240

Gly Val Pro Thr Lys Glu Phe Asp Ile Asp Leu Ala Phe Phe Asp Thr
                245                 250                 255

Pro Gly Gly Thr Val Asn Gly Gln Asp Glu Tyr Lys Ala Asp Ile Val
            260                 265                 270

Met Tyr Thr Glu Asn Thr Tyr Leu Glu Thr Pro Asp Thr His Val Val
        275                 280                 285

Tyr Lys Pro Gly Lys Asp Asp Ala Ser Ser Glu Ile Asn Leu Val Gln
    290                 295                 300

Gln Ser Met Pro Asn Arg Pro Asn Tyr Ile Gly Phe Arg Asp Asn Phe
305                 310                 315                 320

Ile Gly Leu Met Tyr Tyr Asn Ser Thr Gly Asn Met Gly Val Leu Ala
                325                 330                 335

Gly Gln Ala Ser Gln Leu Asn Ala Val Val Asp Leu Gln Asp Arg Asn
            340                 345                 350

Thr Glu Leu Ser Tyr Gln Leu Leu Leu Asp Ser Leu Gly Asp Arg Thr
        355                 360                 365

Arg Tyr Phe Ser Met Trp Asn Gln Ala Val Asp Ser Tyr Asp Pro Asp
    370                 375                 380

Val Arg Ile Ile Glu Asn His Gly Val Glu Asp Glu Leu Pro Asn Tyr
385                 390                 395                 400

Cys Phe Pro Leu Asp Gly Ser Gly Thr Asn Ala Ala Tyr Gln Gly Val
                405                 410                 415

Lys Val Lys Asp Gly Gln Asp Gly Asp Val Glu Ser Glu Trp Glu Asn
            420                 425                 430

Asp Asp Thr Val Ala Ala Arg Asn Gln Leu Cys Lys Gly Asn Ile Phe
        435                 440                 445

Ala Met Glu Ile Asn Leu Gln Ala Asn Leu Trp Arg Ser Phe Leu Tyr
    450                 455                 460

Ser Asn Val Ala Leu Tyr Leu Pro Asp Ser Tyr Lys Tyr Thr Pro Thr
465                 470                 475                 480

Asn Val Thr Leu Pro Thr Asn Thr Asn Thr Tyr Asp Tyr Met Asn Gly
                485                 490                 495

Arg Val Thr Pro Pro Ser Leu Val Asp Ala Tyr Leu Asn Ile Gly Ala
            500                 505                 510

Arg Trp Ser Leu Asp Pro Met Asp Asn Val Asn Pro Phe Asn His His
        515                 520                 525

Arg Asn Ala Gly Leu Arg Tyr Arg Ser Met Leu Leu Gly Asn Gly Arg
    530                 535                 540

Tyr Val Pro Phe His Ile Gln Val Pro Gln Lys Phe Phe Ala Ile Lys
545                 550                 555                 560

Ser Leu Leu Leu Leu Pro Gly Ser Tyr Thr Tyr Glu Trp Asn Phe Arg
                565                 570                 575

Lys Asp Val Asn Met Ile Leu Gln Ser Ser Leu Gly Asn Asp Leu Arg
            580                 585                 590

Thr Asp Gly Ala Ser Ile Ala Phe Thr Ser Ile Asn Leu Tyr Ala Thr
        595                 600                 605

Phe Phe Pro Met Ala His Asn Thr Ala Ser Thr Leu Glu Ala Met Leu
    610                 615                 620

Arg Asn Asp Thr Asn Asp Gln Ser Phe Asn Asp Tyr Leu Ser Ala Ala
625                 630                 635                 640

Asn Met Leu Tyr Pro Ile Pro Ala Asn Ala Thr Asn Val Pro Ile Ser
                645                 650                 655

Ile Pro Ser Arg Asn Trp Ala Ala Phe Arg Gly Trp Ser Phe Thr Arg
            660                 665                 670

Leu Lys Thr Arg Glu Thr Pro Ser Leu Gly Ser Gly Phe Asp Pro Tyr
        675                 680                 685

Phe Val Tyr Ser Gly Ser Ile Pro Tyr Leu Asp Gly Thr Phe Tyr Leu
    690                 695                 700

Asn His Thr Phe Lys Lys Val Ser Ile Thr Phe Asp Ser Ser Val Ser
705                 710                 715                 720

Trp Pro Gly Asn Asp Arg Leu Leu Thr Pro Asn Glu Phe Glu Ile Lys
                725                 730                 735

Arg Thr Val Asp Gly Glu Gly Tyr Asn Val Ala Gln Cys Asn Met Thr
            740                 745                 750

Lys Asp Trp Phe Leu Val Gln Met Leu Ala His Tyr Asn Ile Gly Tyr
        755                 760                 765

Gln Gly Phe Tyr Val Pro Glu Gly Tyr Lys Asp Arg Met Tyr Ser Phe
    770                 775                 780

Phe Arg Asn Phe Gln Pro Met Ser Arg Gln Val Val Asp Glu Val Asn
785                 790                 795                 800

Tyr Lys Asp Tyr Gln Ala Val Thr Leu Ala Tyr Gln His Asn Asn Ser
                805                 810                 815

Gly Phe Val Gly Tyr Leu Ala Pro Thr Met Arg Gln Gly Gln Pro Tyr
            820                 825                 830

Pro Ala Asn Tyr Pro Tyr Pro Leu Ile Gly Lys Ser Ala Val Ala Ser
        835                 840                 845

Val Thr Gln Lys Lys Phe Leu Cys Asp Arg Val Met Trp Arg Ile Pro
    850                 855                 860

Phe Ser Ser Asn Phe Met Ser Met Gly Ala Leu Thr Asp Leu Gly Gln
865                 870                 875                 880

Asn Met Leu Tyr Ala Asn Ser Ala His Ala Leu Asp Met Asn Phe Glu
                885                 890                 895

Val Asp Pro Met Asp Glu Ser Thr Leu Leu Tyr Val Val Phe Glu Val
            900                 905                 910

Phe Asp Val Val Arg Val His Gln Pro His Arg Gly Val Ile Glu Ala
        915                 920                 925

Val Tyr Leu Arg Thr Pro Phe Ser Ala Gly Asn Ala Thr Thr
    930                 935                 940

<210>  8

<211>  443

<212>  PRT

<213>  chimpanzee adenovirus serotype Pan6

<400>  8

Met Ser Lys Lys Arg Val Arg Val Asp Asp Asp Phe Asp Pro Val Tyr
1               5                   10                  15

Pro Tyr Asp Ala Asp Asn Ala Pro Thr Val Pro Phe Ile Asn Pro Pro
            20                  25                  30

Phe Val Ser Ser Asp Gly Phe Gln Glu Lys Pro Leu Gly Val Leu Ser
        35                  40                  45

Leu Arg Leu Ala Asp Pro Val Thr Thr Lys Asn Gly Glu Ile Thr Leu
    50                  55                  60

Lys Leu Gly Glu Gly Val Asp Leu Asp Ser Ser Gly Lys Leu Ile Ser
65                  70                  75                  80

Asn Thr Ala Thr Lys Ala Ala Ala Pro Leu Ser Ile Ser Asn Asn Thr
                85                  90                  95

Ile Ser Leu Lys Thr Ala Ala Pro Phe Tyr Asn Asn Asn Gly Thr Leu
            100                 105                 110

Ser Leu Asn Val Ser Thr Pro Leu Ala Val Phe Pro Thr Phe Asn Thr
        115                 120                 125

Leu Gly Ile Ser Leu Gly Asn Gly Leu Gln Thr Ser Asn Lys Leu Leu
    130                 135                 140

Thr Val Gln Leu Thr His Pro Leu Thr Phe Ser Ser Asn Ser Ile Thr

145                 150                 155                 160

Val Lys Thr Asp Lys Gly Leu Tyr Ile Asn Ser Ser Gly Asn Arg Gly
                165                 170                 175

Leu Glu Ala Asn Ile Ser Leu Lys Arg Gly Leu Val Phe Asp Gly Asn
            180                 185                 190

Ala Ile Ala Thr Tyr Ile Gly Asn Gly Leu Asp Tyr Gly Ser Tyr Asp
        195                 200                 205

Ser Asp Gly Lys Thr Arg Pro Val Ile Thr Lys Ile Gly Ala Gly Leu
    210                 215                 220

Asn Phe Asp Ala Asn Lys Ala Ile Ala Val Lys Leu Gly Thr Gly Leu
225                 230                 235                 240

Ser Phe Asp Ser Ala Gly Ala Leu Thr Ala Gly Asn Lys Gln Asp Asp
                245                 250                 255

Lys Leu Thr Leu Trp Thr Thr Pro Asp Pro Ser Pro Asn Cys Gln Leu
            260                 265                 270

Leu Ser Asp Arg Asp Ala Lys Phe Thr Leu Cys Leu Thr Lys Cys Gly
        275                 280                 285

Ser Gln Ile Leu Gly Thr Val Ala Val Ala Ala Val Thr Val Gly Ser
    290                 295                 300

Ala Leu Asn Pro Ile Asn Asp Thr Val Lys Ser Ala Ile Val Phe Leu
305                 310                 315                 320

Arg Phe Asp Ser Asp Gly Val Leu Met Ser Asn Ser Ser Met Val Gly
                325                 330                 335

Asp Tyr Trp Asn Phe Arg Glu Gly Gln Thr Thr Gln Ser Val Ala Tyr
            340                 345                 350

Thr Asn Ala Val Gly Phe Met Pro Asn Ile Gly Ala Tyr Pro Lys Thr
        355                 360                 365

Gln Ser Lys Thr Pro Lys Asn Ser Ile Val Ser Gln Val Tyr Leu Thr
    370                 375                 380

Gly Glu Thr Thr Met Pro Met Thr Leu Thr Ile Thr Phe Asn Gly Thr
385                 390                 395                 400

Asp Glu Lys Asp Thr Thr Pro Val Ser Thr Tyr Ser Met Thr Phe Thr
                405                 410                 415

Trp Gln Trp Thr Gly Asp Tyr Lys Asp Lys Asn Ile Thr Phe Ala Thr
            420                 425                 430

Asn Ser Phe Ser Phe Ser Tyr Ile Ala Gln Glu
        435                 440

<210> 9

<211> 36535

<212> DNA

<213> chimpanzee adenovirus serotype Pan7

<220>

<221> CDS

<222> (13874)..(15469)

<223> L2 Penton

<220>

<221> CDS

<222> (18288)..(21086)

<223> L3 Hexon

<220>

<221> CDS

<222> (32094)..(33425)

<223> L5 Fiber

<400> 9
catcatcaat aatatacctc aaacttttgg tgcgcgttaa tatgcaaatg agctgtttga 60

atttggggag ggaggaaggt gattggccga gagacgggcg accgttaggg gcggggcggg 120

tgacgttttt aatacgtggc cgtgaggcgg agccggtttg caagttctcg tgggaaaagt 180

gacgtcaaac gaggtgtggt ttgaacacgg aaatactcaa ttttcccgcg ctctctgaca 240

ggaaatgagg tgtttctggg cggatgcaag tgaaaacggg ccattttcgc gcgaaaactg 300

aatgaggaag tgaaaatctg agtaatttcg cgtttatggc agggaggagt atttgccgag 360
ggccgagtag actttgaccg attacgtggg ggtttcgatt accgtatttt tcacctaaat 420
ttccgcgtac ggtgtcaaag tccggtgttt ttacgtaggc gtcagctgat cgccagggta 480
tttaaacctg cgctctctag tcaagaggcc actcttgagt gccagcgagt agagttttct 540
cctccgcgcc gcgagtcaga tctacacttt gaaagatgag gcacctgaga gacctgcccg 600
gtaatgtttt cctggctact gggaacgaga ttctggaatt ggtggtggac gccatgatgg 660
gtggcgaccc tcctgagccc cctaccccat ttgaggcgcc ttcgctgtac gatttgtatg 720
atctggaggt ggatgtgccc gagaacgacc ccaacgagga ggcggtgaat gatttgttta 780
gcgatgccgc gctgctggct gccgagcagg ctaatacgga ctctggctca gacagcgatt 840
cctctctcca taccccgaga cccggcagag gtgagaaaaa gatccccgag cttaaagggg 900
aagagctcga cctgcgctgc tatgaggaat gcttgcctcc gagcgatgat gaggaggacg 960
aggaggcgat tcgagctgca tcgaaccagg gagtgaaagc tgcgggcgaa agctttagcc 1020
tggactgtcc tactctgccc ggacacggct gtaagtcttg tgaatttcat cgcatgaata 1080
ctggagataa gaatgtgatg tgtgccctgt gctatatgag agcttacaac cattgtgttt 1140
acagtaagtg tgattaactt tagttgggaa ggcagagggt gactgggtgc tgactggttt 1200
atttatgtat atgttttttt atgtgtaggt cccgtctctg acgtagatga gacccccact 1260
tcagagtgca tttcatcacc cccagaaatt ggcgaggaac cgcccgaaga tattattcat 1320
agaccagttg cagtgagagt caccgggcgg agagcagctg tggagagttt ggatgacttg 1380
ctacagggtg gggatgaacc tttggacttg tgtacccgga aacgccccag gcactaagtg 1440
ccacacatgt gtgtttactt aaggtgatgt cagtatttat agggtgtgga gtgcaataaa 1500
atccgtgttg actttaagtg cgtggtttat gactcagggg tggggactgt gggtatataa 1560
gcaggtgcag acctgtgtgg tcagttcaga gcaggactca tggagatctg gacggtcttg 1620
gaagactttc accagactag acagctgcta gagaactcat cggagggggt ctcttacctg 1680
tggagattct gcttcggtgg gcctctagct aagctagtct atagggccaa acaggattat 1740
aaggatcaat ttgaggatat tttgagagag tgtcctggta tttttgactc tctcaacttg 1800
ggccatcagt ctcactttaa ccagagtatt ctgagagccc ttgacttttc tactcctggc 1860
agaactaccg ccgcggtagc cttttttgcc tttatccttg acaaatggag tcaagaaacc 1920
catttcagca gggattaccg tctggactgc ttagcagtag ctttgtggag aacatggagg 1980
tgccagcgcc tgaatgcaat ctccggctac ttgccagtac agccggtaga cacgctgagg 2040
atcctgagtc tccagtcacc ccaggaacac caacgccgcc agcagccgca gcaggagcag 2100
cagcaagagg aggaggagga tcgagaagag aacccgagag ccggtctgga ccctccggtg 2160
gcggaggagg aggagtagct gacttgtttc ccgagctgcg ccgggtgctg actaggtctt 2220

ccagtggacg ggagaggggg attaagcggg agaggcatga ggagactagc cacagaactg 2280
aactgactgt cagtctgatg agccgcaggc gcccagaatc ggtgtggtgg catgaggttc 2340
agtcgcaggg gatagatgag gtctcggtga tgcatgagaa atattccctg gaacaagtca 2400
agacttgttg gttggagcct gaggatgatt gggaggtagc catcaggaat tatgccaagc 2460
tggctctgaa gccagacaag aagtacaaga ttaccaaact gattaatatc agaaattcct 2520
gctacatttc agggaatggg gccgaggtgg agatcagtac ccaggagagg gtggccttca 2580
gatgttgtat gatgaatatg tacccggggg tggtgggcat ggagggagtc acctttatga 2640
acgcgaggtt caggggtgat gggtataatg gggtggtctt tatggccaac accaagctga 2700
cagtgcacgg atgctccttc tttgggttca ataacatgtg catcgaggcc tggggcagtg 2760
tttcagtgag gggatgcagc ttttcagcca actggatggg ggtcgtgggc agaaccaaga 2820
gcaaggtgtc agtgaagaaa tgcctgttcg agaggtgcca cctgggggtg atgagcgagg 2880
gcgaagccaa agtcaaacac tgcgcctcta ctgagacggg ctgctttgtg ctgatcaagg 2940
gcaatgccca agtcaagcat aacatgatct gtggggcctc ggatgagcgc ggctaccaga 3000
tgctgacctg cgccggtggg aacagccata tgctggccac cgtgcatgtg acctcgcacc 3060
cccgcaagac atggcccgag ttcgagcaca acgtcatgac ccgatgcaat gtgcacctgg 3120
ggtcccgccg aggcatgttc atgccctacc agtgcaacat gcaatttgtg aaggtgctgc 3180
tggagcccga tgccatgtcc agagtgagcc tgacgggggt gtttgacatg aatgtggagc 3240
tgtggaaaat tctgagatat gatgaatcca agaccaggtg ccgggcctgc gaatgcggag 3300
gcaagcacgc caggcttcag cccgtgtgtg tggaggtgac ggaggacctg cgacccgatc 3360
atttggtgtt gtcctgcaac gggacggagt tcggctccag cggggaagaa tctgactaga 3420
gtgagtagtg tttgggggag gtggagggct tgtatgaggg gcagaatgac taaaatctgt 3480
gtttttctgt gtgttgcagc agcatgagcg gaagcgcctc ctttgaggga ggggtattca 3540
gcccttatct gacggggcgt ctcccctcct gggcgggagt gcgtcagaat gtgatgggat 3600
ccacggtgga cggccggccc gtgcagcccg cgaactcttc aaccctgacc tacgcgaccc 3660
tgagctcctc gtccgtggac gcagctgccg ccgcagctgc tgcttccgcc gccagcgccg 3720
tgcgcggaat ggccctgggc gccggctact acagctctct ggtggccaac tcgacttcca 3780
ccaataatcc cgccagcctg aacgaggaga agctgctgct gctgatggcc cagctcgagg 3840
ccctgaccca gcgcctgggc gagctgaccc agcaggtggc tcagctgcag gcggagacgc 3900
gggccgcggt tgccacggtg aaaaccaaat aaaaaatgaa tcaataaata aacggagacg 3960
gttgttgatt ttaacacaga gtcttgaatc tttatttgat ttttcgcgcg cggtaggccc 4020
tggaccaccg gtctcgatca ttgagcaccc ggtggatttt ttccaggacc cggtagaggt 4080

gggcttggat gttgaggtac atgggcatga gcccgtcccg ggggtggagg tagctccatt 4140
gcagggcctc gtgctcgggg gtggtgttgt aaatcaccca gtcatagcag gggcgcaggg 4200
cgtggtgctg cacgatgtcc ttgaggagga gactgatggc cacgggcagc cccttggtgt 4260
aggtgttgac gaacctgttg agctgggagg gatgcatgcg gggggagatg agatgcatct 4320
tggcctggat cttgagattg gcgatgttcc cgcccagatc ccgccggggg ttcatgttgt 4380
gcaggaccac cagcacggtg tatccggtgc acttggggaa tttgtcatgc aacttggaag 4440
ggaaggcgtg aaagaatttg gagacgccct tgtgaccgcc caggttttcc atgcactcat 4500
ccatgatgat ggcgatgggc ccgtgggcgg cggcctgggc aaagacgttt cgggggtcgg 4560
acacatcgta gttgtggtcc tgggtgagct cgtcataggc cattttaatg aatttggggc 4620
ggagggtgcc cgactggggg acgaaggtgc cctcgatccc gggggcgtag ttgccctcgc 4680
agatctgcat ctcccaggcc ttgagctcgg agggggggat catgtccacc tgcggggcga 4740
tgaaaaaaac ggtttccggg gcgggggaga tgagctgggc cgaaagcagg ttccggagca 4800
gctgggactt gccgcagccg gtggggccgt agatgacccc gatgaccggc tgcaggtggt 4860
agttgaggga gagacagctg ccgtcctcgc ggaggagggg ggccacctcg ttcatcatct 4920
cgcgcacatg catgttctcg cgcacgagtt ccgccaggag gcgctcgccc cccagcgaga 4980
ggagctcttg cagcgaggcg aagtttttca gcggcttgag yccgtcggcc atgggcattt 5040
tggagagggt ctgttgcaag agttccagac ggtcccagag ctcggtgatg tgctctaggg 5100
catctcgatc cagcagacct cctcgtttcg cgggttgggg cgactgcggg agtagggcac 5160
caggcgatgg gcgtccagcg aggccagggt ccggtccttc cagggtcgca gggtccgcgt 5220
cagcgtggtc tccgtcacgg tgaaggggtg cgcgccgggc tgggcgcttg cgagggtgcg 5280
cttcaggctc atccggctgg tcgagaaccg ctcccggtcg gcgccctgcg cgtcggccag 5340
gtagcaattg agcatgagtt cgtagttgag cgcctcggcc gcgtggccct tggcgcggag 5400
cttacctttg gaagtgtgtc cgcagacggg acagaggagg gacttgaggg cgtagagctt 5460
gggggcgagg aagacggact cgggggcgta ggcgtccgcg ccgcagctgg cgcagacggt 5520
ctcgcactcc acgagccagg tgaggtcggg ccggttgggg tcaaaaacga ggtttcctcc 5580
gtgctttttg atgcgtttct tacctctggt ctccatgagc tcgtgtcccc gctgggtgac 5640
aaagaggctg tccgtgtccc cgtagaccga ctttatgggc cggtcctcga gcggggtgcc 5700
gcggtcctcg tcgtagagga accccgccca ctccgagacg aaggcccggg tccaggccag 5760
cacgaaggag gccacgtggg aggggtagcg gtcgttgtcc accagcgggt ccaccttctc 5820
cagggtatgc aagcacatgt ccccctcgtc cacatccagg aaggtgattg gcttgtaagt 5880
gtaggccacg tgaccggggg tcccggccgg gggggtataa aagggggcgg gcccctgctc 5940
gtcctcactg tcttccggat cgctgtccag gagcgccagc tgttggggta ggtattccct 6000

ctcgaaggct ggcataacct cggcactcag gttgtcagtt tctagaaacg aggaggattt 6060
gatattgacg gtgccgttgg agacgccttt catgagcccc tcgtccatct ggtcagaaaa 6120
gacgatcttt ttgttgtcga gcttggtggc gaaggagccg tagagggcgt tggagaggag 6180
cttggcgatg gagcgcatgg tctggttctt ttccttgtcg gcgcgctcct tggcggcgat 6240
gttgagctgc acgtactcgc gcgccacgca cttccattcg gggaagacgg tggtgagctc 6300
gtcgggcacg attctgaccc gccagccgcg gttgtgcagg gtgatgaggt ccacgctggt 6360
ggccacctcg ccgcgcaggg gctcgttggt ccagcagagg cgcccgccct tgcgcgagca 6420
gaaggggggc agcgggtcca gcatgagctc gtcggggggg tcggcgtcca cggtgaagat 6480
gccgggcaga agctcggggt cgaagtagct gatgcaggtg tccagatcgt ccagcgccgc 6540
ttgccagtcg cgcacggcca gcgcgcgctc gtaggggctg aggggcgtgc cccagggcat 6600
ggggtgcgtg agcgcggagg cgtacatgcc gcagatgtcg tagacgtaga ggggctcctc 6660
gaggacgccg atgtaggtgg ggtagcagcg ccccccgcgg atgctggcgc gcacgtagtc 6720
gtacagctcg tgcgagggcg cgaggagccc cgtgccgagg ttggagcgtt gcggcttttc 6780
ggcgcggtag acgatctggc ggaagatggc gtgggagttg gaggagatgg tgggcctctg 6840
gaagatgttg aagtgggcgt ggggcaggcc gaccgagtcc ctgatgaagt gggcgtagga 6900
gtcctgcagc ttggcgacga gctcggcggt gacgaggacg tccagggcgc agtagtcgag 6960
ggtctcttgg atgatgtcgt acttgagctg gcccttctgc ttccacagct cgcggttgag 7020
aaggaactct tcgcggtcct tccagtactc ttcgaggggg aacccgtcct gatcggcacg 7080
gtaagagccc accatgtaga actggttgac ggccttgtag gcgcagcagc ccttctccac 7140
ggggagggcg taagcttgtg cggccttgcg cagggaggtg tgggtgaggg cgaaggtgtc 7200
gcgcaccatg accttgagga actggtgctt gaagtcgagg tcgtcgcagc cgccctgctc 7260
ccagagctgg aagtccgtgc gcttcttgta ggcggggttg ggcaaagcga aagtaacatc 7320
gttgaagagg atcttgcccg cgcggggcat gaagttgcga gtgatgcgga aaggctgggg 7380
cacctcggcc cggttgttga tgacctgggc ggcgaggacg atctcgtcga agccgttgat 7440
gttgtgcccg acgatgtaga gttccacgaa tcgcgggcgg cccttaacgt ggggcagctt 7500
cttgagctcg tcgtaggtga gctcggcggg gtcgctgagc ccgtgctgct cgagggccca 7560
gtcggcgacg tgggggttgg cgctgaggaa ggaagtccag agatccacgg ccagggcggt 7620
ctgcaagcgg tcccggtact gacggaactg ctggcccacg gccatttttt cgggggtgac 7680
gcagtagaag gtgcgggggt cgccgtgcca gcggtcccac ttgagctgga gggcgaggtc 7740
gtgggcgagc tcgacgagcg gcgggtcccc ggagagtttc atgaccagca tgaaggggac 7800
gagctgcttg ccgaaggacc ccatccaggt gtaggtttcc acatcgtagg tgaggaagag 7860

cctttcggtg cgaggatgcg agccgatggg gaagaactgg atctcctgcc accagttgga 7920
ggaatggctg ttgatgtgat ggaagtagaa atgccgacgg cgcgccgagc actcgtgctt 7980
gtgtttatac aagcgtccgc agtgctcgca acgctgcacg ggatgcacgt gctgcacgag 8040
ctgtacctgg gttcctttga cgaggaattt cagtgggcag tggagcgctg gcggctgcat 8100
ctggtgctgt actacgtcct ggccatcggc gtggccatcg tctgcctcga tggtggtcat 8160
gctgacgagc ccgcgcggga ggcaggtcca gacttcggct cggacgggtc ggagagcgag 8220
gacgagggcg cgcaggccgg agctgtccag ggtcctgaga cgctgcggag tcaggtcagt 8280
gggcagcggc ggcgcgcggt tgacttgcag gagcttttcc agggcgcgcg ggaggtccag 8340
atggtacttg atctccacgg cgccgttggt ggcgacgtcc acggcttgca gggtcccgtg 8400
cccctggggc gccaccaccg tgccccgttt cttcttgggc gctgcttcca tgccggtcag 8460
aagcggcggc gaggacgcgc gccgggcggc aggggcggct cgggacccgg aggcaggggc 8520
ggcaggggca cgtcggcgcc gcgcgcgggc aggttctggt actgcgcccg gagaagactg 8580
gcgtgagcga cgacgcgacg gttgacgtcc tggatctgac gcctctgggt gaaggccacg 8640
ggacccgtga gtttgaacct gaaagagagt tcgacagaat caatctcggt atcgttgacg 8700
gcggcctgcc gcaggatctc ttgcacgtcg cccgagttgt cctggtaggc gatctcggtc 8760
atgaactgct cgatctcctc ctcctgaagg tctccgcggc cggcgcgctc gacggtggcc 8820
gcgaggtcgt tggagatgcg gcccatgagc tgcgagaagg cgttcatgcc ggcctcgttc 8880
cagacgcggc tgtagaccac ggctccgtcg gggtcgcgcg cgcgcatgac cacctgggcg 8940
aggttgagct cgacgtggcg cgtgaagacc gcgtagttgc agaggcgctg gtagaggtag 9000
ttgagcgtgg tggcgatgtg ctcggtgacg aagaagtaca tgatccagcg gcggagcggc 9060
atctcgctga cgtcgcccag ggcttccaag cgctccatgg cctcgtagaa gtccacggcg 9120
aagttgaaaa actgggagtt gcgcgccgag acggtcaact cctcctccag aagacggatg 9180
agctcagcga tggtggcgcg cacctcgcgc tcgaaggccc cggggggctc ctcttcttcc 9240
atctcttcct cctccactaa catctcttct acttcctcct caggaggcgg cggcggggga 9300
ggggccctgc gtcgccggcg gcgcacgggc agacggtcga tgaagcgctc gatggtctcc 9360
ccgcgccggc gacgcatggt ctcggtgacg gcgcgcccgt cctcgcgggg ccgcagcgtg 9420
aagacgccgc cgcgcatctc caggtggccg ccgggggggt ctccgttggg cagggagagg 9480
gcgctgacga tgcatcttat caattggccc gtagggactc cgcgcaagga cctgagcgtc 9540
tcgagatcca cgggatccga aaaccgctga acgaaggctt cgagccagtc gcagtcgcaa 9600
ggtaggctga gcccggtttc ttgttcttcg gggatttcgg gaggcgggcg ggcgatgctg 9660
ctggtgatga agttgaagta ggcggtcctg agacggcgga tggtggcgag gagcaccagg 9720
tccttgggcc cggcttgctg gatgcgcaga cggtcggcca tgccccaggc gtggtcctga 9780

cacctggcga ggtccttgta gtagtcctgc atgagccgct ccacgggcac ctcctcctcg 9840
cccgcgcggc cgtgcatgcg cgtgagcccg aacccgcgct ggggctggac gagcgccagg 9900
tcggcgacga cgcgctcggc gaggatggcc tgctgtatct gggtgagggt ggtctggaag 9960
tcgtcgaagt cgacgaagcg gtggtaggct ccggtgttga tggtatagga gcagttggcc 10020
atgacggacc agttgacggt ctggtggccg ggtcgcacga gctcgtggta cttgaggcgc 10080
gagtaggcgc gcgtgtcgaa gatgtagtcg ttgcaggtgc gcacgaggta ctggtatccg 10140
acgaggaagt gcggcggcgg ctggcggtag agcggccatc gctcggtggc gggggcgccg 10200
ggcgcgaggt cctcgagcat gaggcggtgg tagccgtaga tgtacctgga catccaggtg 10260
atgccggcgg cggtggtgga ggcgcgcggg aactcgcgga cgcggttcca gatgttgcgc 10320
agcggcagga agtagttcat ggtggccgcg gtctggcccg tgaggcgcgc gcagtcgtgg 10380
atgctctaga catacgggca aaaacgaaag cggtcagcgg ctcgactccg tggcctggag 10440
gctaagcgaa cgggttgggc tgcgcgtgta ccccggttcg aatctcgaat caggctggag 10500
ccgcagctaa cgtggtactg gcactcccgt ctcgacccaa gcctgctaac gaaacctcca 10560
ggatacggag gcgggtcgtt ttttggcctt ggtcgctggt catgaaaaac tagtaagcgc 10620
ggaaagcgac cgcccgcgat ggctcgctgc cgtagtctgg agaaagaatc gccagggttg 10680
cgttgcggtg tgccccggtt cgagcctcag cgctcggcgc cggccggatt ccgcggctaa 10740
cgtgggcgtg gctgccccgt cgtttccaag accccttagc cagccgactt ctccagttac 10800
ggagcgagcc cctctttttc ttgtgttttt gccagatgca tcccgtactg cggcagatgc 10860
gcccccaccc tccacctcaa ccgcccctac cgccgcagca gcagcaacag ccggcgcttc 10920
tgcccccgcc ccagcagcag ccagccacta ccgcggcggc cgccgtgagc ggagccggcg 10980
ttcagtatga cctggccttg gaagagggcg aggggctggc gcggctgggg gcgtcgtcgc 11040
cggagcggca cccgcgcgtg cagatgaaaa gggacgctcg cgaggcctac gtgcccaagc 11100
agaacctgtt cagagacagg agcggcgagg agcccgagga gatgcgcgcc tcccgcttcc 11160
acgcggggcg ggagctgcgg cgcggcctgg accgaaagcg ggtgctgagg gacgaggatt 11220
tcgaggcgga cgagctgacg gggatcagcc ccgcgcgcgc gcacgtggcc gcggccaacc 11280
tggtcacggc gtacgagcag accgtgaagg aggagagcaa cttccaaaaa tccttcaaca 11340
accacgtgcg cacgctgatc gcgcgcgagg aggtgaccct gggcctgatg cacctgtggg 11400
acctgctgga ggccatcgtg cagaacccca cgagcaagcc gctgacggcg cagctgtttc 11460
tggtggtgca gcacagtcgg gacaacgaga cgttcaggga ggcgctgctg aatatcaccg 11520
agcccgaggg ccgctggctc ctggacctgg tgaacattct gcagagcatc gtggtgcagg 11580
agcgcgggct gccgctgtcc gagaagctgg cggctatcaa cttctcggtg ctgagcctgg 11640

gcaagtacta cgctaggaag atctacaaga ccccgtacgt gcccatagac aaggaggtga 11700
agatcgacgg gttttacatg cgcatgaccc tgaaagtgct gaccctgagc gacgatctgg 11760
gggtgtaccg caacgacagg atgcaccgcg cggtgagcgc cagccgccgg cgcgagctga 11820
gcgaccagga gctgatgcac agcctgcagc gggccctgac cggggccggg accgaggggg 11880
agagctactt tgacatgggc gcggacctgc gctggcagcc cagccgccgg gccttggaag 11940
ctgccggcgg ttccccctac gtggaggagg tggacgatga ggaggaggag ggcgagtacc 12000
tggaagactg atggcgcgac cgtatttttg ctagatgcag caacagccac cgcctcctga 12060
tcccgcgatg cgggcggcgc tgcagagcca gccgtccggc attaactcct cggacgattg 12120
gacccaggcc atgcaacgca tcatggcgct gacgacccgc aatcccgaag cctttagaca 12180
gcagcctcag gccaaccggc tctcggccat cctggaggcc gtggtgccct cgcgctcgaa 12240
ccccacgcac gagaaggtgc tggccatcgt gaacgcgctg gtggagaaca aggccatccg 12300
cggcgacgag gccgggctgg tgtacaacgc gctgctggag cgcgtggccc gctacaacag 12360
caccaacgtg cagacgaacc tggaccgcat ggtgaccgac gtgcgcgagg cggtgtcgca 12420
gcgcgagcgg ttccaccgcg agtcgaacct gggctccatg gtggcgctga acgccttcct 12480
gagcacgcag cccgccaacg tgccccgggg ccaggaggac tacaccaact tcatcagcgc 12540
gctgcggctg atggtggccg aggtgcccca gagcgaggtg taccagtcgg ggccggacta 12600
cttcttccag accagtcgcc agggcttgca gaccgtgaac ctgagccagg ctttcaagaa 12660
cttgcaggga ctgtggggcg tgcaggcccc ggtcggggac cgcgcgacgg tgtcgagcct 12720
gctgacgccg aactcgcgcc tgctgctgct gctggtggcg cccttcacgg acagcggcag 12780
cgtgagccgc gactcgtacc tgggctacct gcttaacctg taccgcgagg ccatcgggca 12840
ggcgcacgtg gacgagcaga cctaccagga gatcacccac gtgagccgcg cgctgggcca 12900
ggaggacccg ggcaacctgg aggccaccct gaacttcctg ctgaccaacc ggtcgcagaa 12960
gatcccgccc cagtacgcgc tgagcaccga ggaggagcgc atcctgcgct acgtgcagca 13020
gagcgtgggg ctgttcctga tgcaggaggg ggccacgccc agcgccgcgc tcgacatgac 13080
cgcgcgcaac atggagccca gcatgtacgc tcgcaaccgc ccgttcatca ataagctgat 13140
ggactacttg catcgggcgg ccgccatgaa ctcggactac tttaccaacg ccatcttgaa 13200
cccgcactgg ctcccgccgc ccgggttcta cacgggcgag tacgacatgc ccgaccccaa 13260
cgacgggttc ctgtgggacg acgtggacag cagcgtgttc tcgccgcgcc ccgccaccac 13320
cgtgtggaag aaagagggcg gggaccggcg gccgtcctcg gcgctgtccg gtcgcgcggg 13380
tgctgccgcg gcggtgcctg aggccgccag ccccttcccg agcctgccct tttcgctgaa 13440
cagcgtgcgc agcagcgagc tgggtcggct gacgcggccg cgcctgctgg gcgaggagga 13500
gtacctgaac gactccttgt tgaggcccga gcgcgagaag aacttcccca ataacgggat 13560

agagagcctg gtggacaaga tgagccgctg gaagacgtac gcgcacgagc acagggacga 13620

gccccgagct agcagcagcg caggcacccg tagacgccag cgacacgaca ggcagcgggg 13680

tctggtgtgg gacgatgagg attccgccga cgacagcagc gtgttggact tgggtgggag 13740

tggtggtggt aacccgttcg ctcacttgcg cccccgtatc gggcgcctga tgtaagaatc 13800

tgaaaaaata aaaaacggta ctcaccaagg ccatggcgac cagcgtgcgt tcttctctgt 13860

tgtttgtagt agt atg atg agg cgc gtg tac ccg gag ggt cct cct ccc 13909
Met Met Arg Arg Val Tyr Pro Glu Gly Pro Pro Pro
1               5                   10

tcg tac gag agc gtg atg cag cag gcg gtg gcg gcg gcg atg cag ccc 13957
Ser Tyr Glu Ser Val Met Gln Gln Ala Val Ala Ala Ala Met Gln Pro
        15                  20                  25

ccg ctg gag gcg cct tac gtg ccc ccg cgg tac ctg gcg cct acg gag 14005
Pro Leu Glu Ala Pro Tyr Val Pro Pro Arg Tyr Leu Ala Pro Thr Glu
    30                  35                  40

ggg cgg aac agc att cgt tac tcg gag ctg gca ccc ttg tac gat acc 14053
Gly Arg Asn Ser Ile Arg Tyr Ser Glu Leu Ala Pro Leu Tyr Asp Thr
45                  50                  55                  60

acc cgg ttg tac ctg gtg gac aac aag tcg gcg gac atc gcc tcg ctg 14101
Thr Arg Leu Tyr Leu Val Asp Asn Lys Ser Ala Asp Ile Ala Ser Leu
                65                  70                  75

aac tac cag aac gac cac agc aac ttc ctg acc acc gtg gtg cag aac 14149
Asn Tyr Gln Asn Asp His Ser Asn Phe Leu Thr Thr Val Val Gln Asn
            80                  85                  90

aac gat ttc acc ccc acg gag gcc agc acc cag acc atc aac ttt gac 14197
Asn Asp Phe Thr Pro Thr Glu Ala Ser Thr Gln Thr Ile Asn Phe Asp
        95                  100                 105

gag cgc tcg cgg tgg ggc ggc cag ctg aaa acc atc atg cac acc aac 14245
Glu Arg Ser Arg Trp Gly Gly Gln Leu Lys Thr Ile Met His Thr Asn
    110                 115                 120

atg ccc aac gtg aac gag ttc atg tac agc aac aag ttc aag gcg cgg 14293
Met Pro Asn Val Asn Glu Phe Met Tyr Ser Asn Lys Phe Lys Ala Arg
125                 130                 135                 140

gtg atg gtc tcg cgc aag acc ccc aat ggg gtc gcg gtg gat gag aat 14341
Val Met Val Ser Arg Lys Thr Pro Asn Gly Val Ala Val Asp Glu Asn
                145                 150                 155

tat gat ggt agt cag gac gag ctg act tac gag tgg gtg gag ttt gag 14389
Tyr Asp Gly Ser Gln Asp Glu Leu Thr Tyr Glu Trp Val Glu Phe Glu
            160                 165                 170

ctg ccc gag ggc aac ttc tcg gtg acc atg acc atc gat ctg atg aac 14437
Leu Pro Glu Gly Asn Phe Ser Val Thr Met Thr Ile Asp Leu Met Asn
        175                 180                 185

aac gcc atc atc gac aac tac ttg gcg gtg ggg cgt cag aac ggg gtg 14485
Asn Ala Ile Ile Asp Asn Tyr Leu Ala Val Gly Arg Gln Asn Gly Val
    190                 195                 200

ctg gag agc gac atc ggc gtg aag ttc gac acg cgc aac ttc cgg ctg     14533
Leu Glu Ser Asp Ile Gly Val Lys Phe Asp Thr Arg Asn Phe Arg Leu
205                 210                 215                 220

ggc tgg gac ccc gtg acc gag ctg gtg atg ccg ggc gtg tac acc aac     14581
Gly Trp Asp Pro Val Thr Glu Leu Val Met Pro Gly Val Tyr Thr Asn
                225                 230                 235

gag gcc ttc cac ccc gac atc gtc ctg ctg ccc ggc tgc ggc gtg gac     14629
Glu Ala Phe His Pro Asp Ile Val Leu Leu Pro Gly Cys Gly Val Asp
            240                 245                 250

ttc acc gag agc cgc ctc agc aac ctg ctg ggc atc cgc aag cgg cag     14677
Phe Thr Glu Ser Arg Leu Ser Asn Leu Leu Gly Ile Arg Lys Arg Gln
        255                 260                 265

ccc ttc cag gag ggc ttc cag atc ctg tac gag gac ctg gag ggg ggc     14725
Pro Phe Gln Glu Gly Phe Gln Ile Leu Tyr Glu Asp Leu Glu Gly Gly
    270                 275                 280

aac atc ccc gcg ctc ttg gat gtc gaa gcc tat gag aaa agc aag gag     14773
Asn Ile Pro Ala Leu Leu Asp Val Glu Ala Tyr Glu Lys Ser Lys Glu
285                 290                 295                 300

gag gcc gcc gca gcg gcg acc gca gcc gtg gcc acc gcc tct acc gag     14821
Glu Ala Ala Ala Ala Ala Thr Ala Ala Val Ala Thr Ala Ser Thr Glu
                305                 310                 315

gtg cgg ggc gat aat ttt gct agc gcc gcg gca gtg gcc gag gcg gct     14869
Val Arg Gly Asp Asn Phe Ala Ser Ala Ala Ala Val Ala Glu Ala Ala
            320                 325                 330

gaa acc gaa agt aag ata gtc atc cag ccg gtg gag aag gac agc aag     14917
Glu Thr Glu Ser Lys Ile Val Ile Gln Pro Val Glu Lys Asp Ser Lys
        335                 340                 345

gac agg agc tac aac gtg ctc gcg gac aag aaa aac acc gcc tac cgc     14965
Asp Arg Ser Tyr Asn Val Leu Ala Asp Lys Lys Asn Thr Ala Tyr Arg
    350                 355                 360

agc tgg tac ctg gcc tac aac tac ggc gac ccc gag aag ggc gtg cgc     15013
Ser Trp Tyr Leu Ala Tyr Asn Tyr Gly Asp Pro Glu Lys Gly Val Arg
365                 370                 375                 380

tcc tgg acg ctg ctc acc acc tcg gac gtc acc tgc ggc gtg gag caa     15061
Ser Trp Thr Leu Leu Thr Thr Ser Asp Val Thr Cys Gly Val Glu Gln
                385                 390                 395

gtc tac tgg tcg ctg ccc gac atg atg caa gac ccg gtc acc ttc cgc     15109
Val Tyr Trp Ser Leu Pro Asp Met Met Gln Asp Pro Val Thr Phe Arg
            400                 405                 410

tcc acg cgt caa gtt agc aac tac ccg gtg gtg ggc gcc gag ctc ctg     15157
Ser Thr Arg Gln Val Ser Asn Tyr Pro Val Val Gly Ala Glu Leu Leu
        415                 420                 425

ccc gtc tac tcc aag agc ttc ttc aac gag cag gcc gtc tac tcg cag     15205
Pro Val Tyr Ser Lys Ser Phe Phe Asn Glu Gln Ala Val Tyr Ser Gln
    430                 435                 440

cag ctg cgc gcc ttc acc tcg ctc acg cac gtc ttc aac cgc ttc ccc     15253
Gln Leu Arg Ala Phe Thr Ser Leu Thr His Val Phe Asn Arg Phe Pro
445                 450                 455                 460

gag aac cag atc ctc gtc cgc ccg ccc gcg ccc acc att acc acc gtc 15301
Glu Asn Gln Ile Leu Val Arg Pro Pro Ala Pro Thr Ile Thr Thr Val
                465                 470                 475

agt gaa aac gtt cct gct ctc aca gat cac ggg acc ctg ccg ctg cgc 15349
Ser Glu Asn Val Pro Ala Leu Thr Asp His Gly Thr Leu Pro Leu Arg
            480                 485                 490

agc agt atc cgg gga gtc cag cgc gtg acc gtc act gac gcc aga cgc 15397
Ser Ser Ile Arg Gly Val Gln Arg Val Thr Val Thr Asp Ala Arg Arg
        495                 500                 505

cgc acc tgc ccc tac gtc tac aag gcc ctg ggc gta gtc gcg ccg cgc 15445
Arg Thr Cys Pro Tyr Val Tyr Lys Ala Leu Gly Val Val Ala Pro Arg
    510                 515                 520

gtc ctc tcg agc cgc acc ttc taa aaaatgtcca ttctcatctc gcccagtaat 15499
Val Leu Ser Ser Arg Thr Phe
525                 530

aacaccggtt ggggcctgcg cgcgcccagc aagatgtacg gaggcgctcg ccaacgctcc 15559

acgcaacacc ccgtgcgcgt gcgcgggcac ttccgcgctc cctggggcgc cctcaagggc 15619

cgcgtgcgct cgcgcaccac cgtcgacgac gtgatcgacc aggtggtggc cgacgcgcgc 15679

aactacacgc ccgccgccgc gcccgcctcc accgtggacg ccgtcatcga cagcgtggtg 15739

gccgatgcgc gccggtacgc ccgcgccaag agccggcggc ggcgcatcgc ccggcggcac 15799

cggagcaccc ccgccatgcg cgcggcgcga gccttgctgc gcagggccag gcgcacggga 15859

cgcagggcca tgctcagggc ggccagacgc gcggcctccg gcagcagcag cgccggcagg 15919

acccgcagac gcgcggccac ggcggcggcg gcggccatcg ccagcatgtc ccgcccgcgg 15979

cgcggcaacg tgtactgggt gcgcgacgcc gccaccggtg tgcgcgtgcc cgtgcgcacc 16039

cgcccccctc gcacttgaag atgctgactt cgcgatgttg atgtgtccca gcggcgagga 16099

ggatgtccaa gcgcaaatac aaggaagaga tgctccaggt catcgcgcct gagatctacg 16159

gccccgcggt gaaggaggaa agaaagcccc gcaaactgaa gcgggtcaaa aaggacaaaa 16219

aggaggagga agatgtggac ggactggtgg agtttgtgcg cgagttcgcc ccccggcggc 16279

gcgtgcagtg gcgcgggcgg aaagtgaaac cggtgctgcg gcccggcacc acggtggtct 16339

tcacgcccgg cgagcgttcc ggctccgcct ccaagcgctc ctacgacgag gtgtacgggg 16399

acgaggacat cctcgagcag gcggtcgagc gtctgggcga gtttgcttac ggcaagcgca 16459

gccgccccgc gcccttgaaa gaggaggcgg tgtccatccc gctggaccac ggcaacccca 16519

cgccgagcct gaagccggtg accctgcagc aggtgctgcc gagcgcggcg ccgcgccggg 16579

gcttcaagcg cgagggcggc gaggatctgt acccgaccat gcagctgatg gtgcccaagc 16639

gccagaagct ggaggacgtg ctggagcaca tgaaggtgga ccccgaggtg cagcccgagg 16699

tcaaggtgcg gcccatcaag caggtggccc cgggcctggg cgtgcagacc gtggacatca 16759

agatccccac ggagcccatg gaaacgcaga ccgagcccgt gaagcccagc accagcacca 16819

tggaggtgca gacggatccc tggatgccgg cgccggcttc caccactcgc cgaagacgca 16879

agtacggcgc ggccagcctg ctgatgccca actacgcgct gcatccttcc atcatcccca 16939

cgccgggcta ccgcggcacg cgcttctacc gcggctacac cagcagccgc cgcaagacca 16999

ccacccgccg ccgccgtcgt cgcacccgcc gcagcagcac cgcgacttcc gccgccgccc 17059

tggtgcggag agtgtaccgc agcgggcgcg agcctctgac cctgccgcgc gcgcgctacc 17119

acccgagcat cgccatttaa ctctgccgtc gcctcctact tgcagatatg gccctcacat 17179

gccgcctccg cgtccccatt acgggctacc gaggaagaaa gccgcgccgt agaaggctga 17239

cggggaacgg gctgcgtcgc catcaccacc ggcggcggcg cgccatcagc aagcggttgg 17299

ggggaggctt cctgcccgcg ctgatcccca tcatcgccgc ggcgatcggg gcgatccccg 17359

gcatagcttc cgtggcggtg caggcctctc agcgccactg agacacagct tggaaaattt 17419

gtaataaaaa aatggactga cgctcctggt cctgtgatgt gtgtttttag atggaagaca 17479

tcaatttttc gtccctggca ccgcgacacg gcacgcggcc gtttatgggc acctggagcg 17539

acatcggcaa cagccaactg aacgggggcg ccttcaattg gagcagtctc tggagcgggc 17599

ttaagaattt cgggtccacg ctcaaaacct atggcaacaa ggcgtggaac agcagcacag 17659

ggcaggcgct gagggaaaag ctgaaagagc agaacttcca gcagaaggtg gtcgatggcc 17719

tggcctcggg catcaacggg gtggtggacc tggccaacca ggccgtgcag aaacagatca 17779

acagccgcct ggacgcggtc ccgcccgcgg ggtccgtgga gatgccccag gtggaggagg 17839

agctgcctcc cctggacaag cgcggcgaca agcgaccgcg tcccgacgcg gaggagacgc 17899

tgctgacgca cacggacgag ccgcccccgt acgaggaggc ggtgaaactg ggtctgccca 17959

ccacgcggcc cgtggcgcct ctggccaccg gggtgctgaa acccagcagc agcagccagc 18019

ccgcgaccct ggacttgcct ccgcctgctt cccgcccctc cacagtggct aagcccctgc 18079

cgccggtggc cgtcgcgtcg cgcgcccccc gaggccgccc ccaggcgaac tggcagagca 18139

ctctgaacag catcgtgggt ctgggagtgc agagtgtgaa gcgccgccgc tgctattaaa 18199

agacactgta gcgcttaact tgcttgtctg tgtgtatatg tatgtccgcc gaccagaagg 18259

aggaagaggc gcgtcgccga gttgcaag atg gcc acc cca tcg atg ctg ccc 18311
Met Ala Thr Pro Ser Met Leu Pro
            535

cag tgg gcg tac atg cac atc gcc gga cag gac gct tcg gag tac ctg 18359
Gln Trp Ala Tyr Met His Ile Ala Gly Gln Asp Ala Ser Glu Tyr Leu
540                 545                 550                 555

agt ccg ggt ctg gtg cag ttc gcc cgc gcc aca gac acc tac ttc agt 18407
Ser Pro Gly Leu Val Gln Phe Ala Arg Ala Thr Asp Thr Tyr Phe Ser
                560                 565                 570

ctg ggg aac aag ttt agg aac ccc acg gtg gcg ccc acg cac gat gtg 18455

Leu Gly Asn Lys Phe Arg Asn Pro Thr Val Ala Pro Thr His Asp Val
            575                 580                 585

acc acc gac cgc agc cag cgg ctg acg ctg cgc ttc gtg ccc gtg gac      18503
Thr Thr Asp Arg Ser Gln Arg Leu Thr Leu Arg Phe Val Pro Val Asp
        590                 595                 600

cgc gag gac aac acc tac tcg tac aaa gtg cgc tac acg ctg gcc gtg      18551
Arg Glu Asp Asn Thr Tyr Ser Tyr Lys Val Arg Tyr Thr Leu Ala Val
    605                 610                 615

ggc gac aac cgc gtg ctg gac atg gcc agc acc tac ttt gac atc cgc      18599
Gly Asp Asn Arg Val Leu Asp Met Ala Ser Thr Tyr Phe Asp Ile Arg
620                 625                 630                 635

ggc gtg ctg gat cgg ggg ccc agc ttc aaa ccc tac tcc ggc acc gcc      18647
Gly Val Leu Asp Arg Gly Pro Ser Phe Lys Pro Tyr Ser Gly Thr Ala
                640                 645                 650

tac aac agc ctg gct ccc aag gga gcg ccc aac act tgc cag tgg aca      18695
Tyr Asn Ser Leu Ala Pro Lys Gly Ala Pro Asn Thr Cys Gln Trp Thr
            655                 660                 665

tat aaa gct ggt gat act gat aca gaa aaa acc tat aca tat gga aat      18743
Tyr Lys Ala Gly Asp Thr Asp Thr Glu Lys Thr Tyr Thr Tyr Gly Asn
        670                 675                 680

gca cct gtg caa ggc att agc att aca aag gat ggt att caa ctt gga      18791
Ala Pro Val Gln Gly Ile Ser Ile Thr Lys Asp Gly Ile Gln Leu Gly
    685                 690                 695

act gac agc gat ggt cag gca atc tat gca gac gaa act tat caa cca      18839
Thr Asp Ser Asp Gly Gln Ala Ile Tyr Ala Asp Glu Thr Tyr Gln Pro
700                 705                 710                 715

gag cct caa gtg ggt gat gct gaa tgg cat gac atc act ggt act gat      18887
Glu Pro Gln Val Gly Asp Ala Glu Trp His Asp Ile Thr Gly Thr Asp
                720                 725                 730

gaa aaa tat gga ggc aga gct ctt aag cct gac acc aaa atg aag cct      18935
Glu Lys Tyr Gly Gly Arg Ala Leu Lys Pro Asp Thr Lys Met Lys Pro
            735                 740                 745

tgc tat ggt tct ttt gcc aag cct acc aat aaa gaa gga ggc cag gca      18983
Cys Tyr Gly Ser Phe Ala Lys Pro Thr Asn Lys Glu Gly Gly Gln Ala
        750                 755                 760

aat gtg aaa acc gaa aca ggc ggt acc aaa gaa tat gac att gac atg      19031
Asn Val Lys Thr Glu Thr Gly Gly Thr Lys Glu Tyr Asp Ile Asp Met
    765                 770                 775

gca ttc ttc gat aat cga agt gca gct gcc gcc ggc cta gcc cca gaa      19079
Ala Phe Phe Asp Asn Arg Ser Ala Ala Ala Ala Gly Leu Ala Pro Glu
780                 785                 790                 795

att gtt ttg tat act gag aat gtg gat ctg gaa act cca gat acc cat      19127
Ile Val Leu Tyr Thr Glu Asn Val Asp Leu Glu Thr Pro Asp Thr His
                800                 805                 810

att gta tac aag gca ggt aca gat gac agt agc tct tct atc aat ttg      19175
Ile Val Tyr Lys Ala Gly Thr Asp Asp Ser Ser Ser Ser Ile Asn Leu
            815                 820                 825

ggt cag cag tcc atg ccc aac aga ccc aac tac att ggc ttc aga gac 19223
Gly Gln Gln Ser Met Pro Asn Arg Pro Asn Tyr Ile Gly Phe Arg Asp
        830                 835                 840

aac ttt atc ggt ctg atg tac tac aac agc act ggc aat atg ggt gta 19271
Asn Phe Ile Gly Leu Met Tyr Tyr Asn Ser Thr Gly Asn Met Gly Val
    845                 850                 855

ctg gct gga cag gcc tcc cag ctg aat gct gtg gtg gac ttg cag gac 19319
Leu Ala Gly Gln Ala Ser Gln Leu Asn Ala Val Val Asp Leu Gln Asp
860                 865                 870                 875

aga aac acc gaa ctg tcc tac cag ctc ttg ctt gac tct ctg ggt gac 19367
Arg Asn Thr Glu Leu Ser Tyr Gln Leu Leu Leu Asp Ser Leu Gly Asp
                880                 885                 890

aga acc agg tat ttc agt atg tgg aat cag gcg gtg gac agt tat gac 19415
Arg Thr Arg Tyr Phe Ser Met Trp Asn Gln Ala Val Asp Ser Tyr Asp
            895                 900                 905

ccc gat gtg cgc att att gaa aat cac ggt gtg gag gat gaa ctt cct 19463
Pro Asp Val Arg Ile Ile Glu Asn His Gly Val Glu Asp Glu Leu Pro
        910                 915                 920

aac tat tgc ttc ccc ctg gat gct gtg ggt aga act gat act tac cag 19511
Asn Tyr Cys Phe Pro Leu Asp Ala Val Gly Arg Thr Asp Thr Tyr Gln
    925                 930                 935

gga att aag gcc aat ggt gat aat caa acc acc tgg acc aaa gat gat 19559
Gly Ile Lys Ala Asn Gly Asp Asn Gln Thr Thr Trp Thr Lys Asp Asp
940                 945                 950                 955

act gtt aat gat gct aat gaa ttg ggc aag ggc aat cct ttc gcc atg 19607
Thr Val Asn Asp Ala Asn Glu Leu Gly Lys Gly Asn Pro Phe Ala Met
                960                 965                 970

gag atc aac atc cag gcc aac ctg tgg cgg aac ttc ctc tac gcg aac 19655
Glu Ile Asn Ile Gln Ala Asn Leu Trp Arg Asn Phe Leu Tyr Ala Asn
            975                 980                 985

gtg gcg ctg tac ctg ccc gac tcc tac aag tac acg ccg gcc aac atc 19703
Val Ala Leu Tyr Leu Pro Asp Ser Tyr Lys Tyr Thr Pro Ala Asn Ile
        990                 995                 1000

acg ctg ccc acc aac acc aac acc tac gat tac atg aac ggc cgc 19748
Thr Leu Pro Thr Asn Thr Asn Thr Tyr Asp Tyr Met Asn Gly Arg
    1005                1010                1015

gtg gtg gcg ccc tcg ctg gtg gac gcc tac atc aac atc ggg gcg 19793
Val Val Ala Pro Ser Leu Val Asp Ala Tyr Ile Asn Ile Gly Ala
    1020                1025                1030

cgc tgg tcg ctg gac ccc atg gac aac gtc aac ccc ttc aac cac 19838
Arg Trp Ser Leu Asp Pro Met Asp Asn Val Asn Pro Phe Asn His
    1035                1040                1045

cac cgc aac gcg ggc ctg cga tac cgc tcc atg ctc ctg ggc aac 19883
His Arg Asn Ala Gly Leu Arg Tyr Arg Ser Met Leu Leu Gly Asn
    1050                1055                1060

ggg cgc tac gtg ccc ttc cac atc cag gtg ccc caa aag ttt ttc 19928
Gly Arg Tyr Val Pro Phe His Ile Gln Val Pro Gln Lys Phe Phe
    1065                1070                1075

gcc atc aag agc ctc ctg ctc ctg ccc ggg tcc tac acc tac gag 19973
Ala Ile Lys Ser Leu Leu Leu Leu Pro Gly Ser Tyr Thr Tyr Glu
    1080                1085                1090

tgg aac ttc cgc aag gac gtc aac atg atc ctg cag agc tcc ctc 20018
Trp Asn Phe Arg Lys Asp Val Asn Met Ile Leu Gln Ser Ser Leu
    1095                1100                1105

ggc aac gac ctg cgc acg gac ggg gcc tcc atc gcc ttc acc agc 20063
Gly Asn Asp Leu Arg Thr Asp Gly Ala Ser Ile Ala Phe Thr Ser
    1110                1115                1120

atc aac ctc tac gcc acc ttc ttc ccc atg gcg cac aac acc gcc 20108
Ile Asn Leu Tyr Ala Thr Phe Phe Pro Met Ala His Asn Thr Ala
    1125                1130                1135

tcc acg ctc gag gcc atg ctg cgc aac gac acc aac gac cag tcc 20153
Ser Thr Leu Glu Ala Met Leu Arg Asn Asp Thr Asn Asp Gln Ser
    1140                1145                1150

ttc aac gac tac ctc tcg gcg gcc aac atg ctc tac ccc atc ccg 20198
Phe Asn Asp Tyr Leu Ser Ala Ala Asn Met Leu Tyr Pro Ile Pro
    1155                1160                1165

gcc aac gcc acc aac gtg ccc atc tcc atc ccc tcg cgc aac tgg 20243
Ala Asn Ala Thr Asn Val Pro Ile Ser Ile Pro Ser Arg Asn Trp
    1170                1175                1180

gcc gcc ttc cgc ggc tgg tcc ttc acg cgc ctc aag acc cgc gag 20288
Ala Ala Phe Arg Gly Trp Ser Phe Thr Arg Leu Lys Thr Arg Glu
    1185                1190                1195

acg ccc tcg ctc ggc tcc ggg ttc gac ccc tac ttc gtc tac tcg 20333
Thr Pro Ser Leu Gly Ser Gly Phe Asp Pro Tyr Phe Val Tyr Ser
    1200                1205                1210

ggc tcc atc ccc tac ctc gac ggc acc ttc tac ctc aac cac acc 20378
Gly Ser Ile Pro Tyr Leu Asp Gly Thr Phe Tyr Leu Asn His Thr
    1215                1220                1225

ttc aag aag gtc tcc atc acc ttc gac tcc tcc gtc agc tgg ccc 20423
Phe Lys Lys Val Ser Ile Thr Phe Asp Ser Ser Val Ser Trp Pro
    1230                1235                1240

ggc aac gac cgc ctc ctg acg ccc aac gag ttc gaa atc aag cgc 20468
Gly Asn Asp Arg Leu Leu Thr Pro Asn Glu Phe Glu Ile Lys Arg
    1245                1250                1255

acc gtc gac gga gag ggg tac aac gtg gcc cag tgc aac atg acc 20513
Thr Val Asp Gly Glu Gly Tyr Asn Val Ala Gln Cys Asn Met Thr
    1260                1265                1270

aag gac tgg ttc ctg gtc cag atg ctg gcc cac tac aac atc ggc 20558
Lys Asp Trp Phe Leu Val Gln Met Leu Ala His Tyr Asn Ile Gly
    1275                1280                1285

tac cag ggc ttc tac gtg ccc gag ggc tac aag gac cgc atg tac 20603
Tyr Gln Gly Phe Tyr Val Pro Glu Gly Tyr Lys Asp Arg Met Tyr
    1290                1295                1300

tcc ttc ttc cgc aac ttc cag ccc atg agc cgc cag gtc gtg gac 20648
Ser Phe Phe Arg Asn Phe Gln Pro Met Ser Arg Gln Val Val Asp

1305                    1310                    1315

gag gtc aac tac aag gac tac cag gcc gtc acc ctg gcc tac cag      20693
Glu Val Asn Tyr Lys Asp Tyr Gln Ala Val Thr Leu Ala Tyr Gln
    1320                    1325                    1330

cac aac aac tcg ggc ttc gtc ggc tac ctc gcg ccc acc atg cgc      20738
His Asn Asn Ser Gly Phe Val Gly Tyr Leu Ala Pro Thr Met Arg
    1335                    1340                    1345

cag ggc cag ccc tac ccc gcc aac tac ccc tac ccg ctc atc ggc      20783
Gln Gly Gln Pro Tyr Pro Ala Asn Tyr Pro Tyr Pro Leu Ile Gly
    1350                    1355                    1360

aag agc gcc gtc gcc agc gtc acc cag aaa aag ttc ctc tgc gac      20828
Lys Ser Ala Val Ala Ser Val Thr Gln Lys Lys Phe Leu Cys Asp
    1365                    1370                    1375

cgg gtc atg tgg cgc atc ccc ttc tcc agc aac ttc atg tcc atg      20873
Arg Val Met Trp Arg Ile Pro Phe Ser Ser Asn Phe Met Ser Met
    1380                    1385                    1390

ggc gcg ctc acc gac ctc ggc cag aac atg ctc tac gcc aac tcc      20918
Gly Ala Leu Thr Asp Leu Gly Gln Asn Met Leu Tyr Ala Asn Ser
    1395                    1400                    1405

gcc cac gcg cta gac atg aat ttc gaa gtc gac ccc atg gat gag      20963
Ala His Ala Leu Asp Met Asn Phe Glu Val Asp Pro Met Asp Glu
    1410                    1415                    1420

tcc acc ctt ctc tat gtt gtc ttc gaa gtc ttc gac gtc gtc cga      21008
Ser Thr Leu Leu Tyr Val Val Phe Glu Val Phe Asp Val Val Arg
    1425                    1430                    1435

gtg cac cag ccc cac cgc ggc gtc atc gag gcc gtc tac ctg cgc      21053
Val His Gln Pro His Arg Gly Val Ile Glu Ala Val Tyr Leu Arg
    1440                    1445                    1450

acg ccc ttc tcg gcc ggc aac gcc acc acc taa gcctcttgct           21096
Thr Pro Phe Ser Ala Gly Asn Ala Thr Thr
    1455                    1460

tcttgcaaga tgacggcctg cgcgggctcc ggcgagcagg agctcagggc catcctccgc   21156

gacctgggct gcgggccctg cttcctgggc accttcgaca agcgcttccc gggattcatg   21216

gccccgcaca agctggcctg cgccatcgtc aacacggccg gccgcgagac cgggggcgag   21276

cactggctgg ccttcgcctg gaacccgcgc tcccacacct gctacctctt cgaccccttc   21336

gggttctcgg acgagcgcct caagcagatc taccagttcg agtacgaggg cctgctgcgt   21396

cgcagcgccc tggccaccga ggaccgctgc gtcaccctgg aaaagtccac ccagaccgtg   21456

cagggtccgc gctcggccgc ctgcgggctc ttctgctgca tgttcctgca cgccttcgtg   21516

cactggcccg accgccccat ggacaagaac cccaccatga acttgctgac gggggtgccc   21576

aacggcatgc tccagtcgcc ccaggtggaa cccaccctgc gccgcaacca ggaggcgctc   21636

taccgcttcc tcaacgccca ctccgcctac tttcgctccc accgcgcgcg catcgagaag   21696

gccaccgcct tcgaccgcat gaatcaagac atgtaatccg gtgtgtgtat gtgaatgctt   21756

tattcatcat aataaacagc acatgtttat gccaccttct ctgaggctct gactttattt 21816
agaaatcgaa ggggttctgc cggctctcgg catggcccgc gggcagggat acgttgcgga 21876
actggtactt gggcagccac ttgaactcgg ggatcagcag cttcggcacg gggaggtcgg 21936
ggaacgagtc gctccacagc ttgcgcgtga gttgcagggc gcccagcagg tcgggcgcgg 21996
agatcttgaa atcgcagttg ggacccgcgt tctgcgcgcg agagttacgg tacacggggt 22056
tgcagcactg gaacaccatc agggccgggt gcttcacgct cgccagcacc gtcgcgtcgg 22116
tgatgccctc cacgtccaga tcctcggcgt tggccatccc gaagggggtc atcttgcagg 22176
tctgccgccc catgctgggc acgcagccgg gcttgtggtt gcaatcgcag tgcaggggga 22236
tcagcatcat ctgggcctgc tcggagctca tgcccgggta catggccttc atgaaagcct 22296
ccagctggcg gaaggcctgc tgcgccttgc cgccctcggt gaagaagacc ccgcaggact 22356
tgctagagaa ctggttggtg gcgcagccag cgtcgtgcac gcagcagcgc gcgtcgttgt 22416
tggccagctg caccacgctg cgcccccagc ggttctgggt gatcttggcc cggtcggggt 22476
tctccttcag cgcgcgctgc ccgttctcgc tcgccacatc catctcgatc gtgtgctcct 22536
tctggatcat cacggtcccg tgcaggcacc gcagcttgcc ctcggcctcg gtgcacccgt 22596
gcagccacag cgcgcagccg gtgctctccc agttcttgtg ggcgatctgg gagtgcgagt 22656
gcacgaagcc ctgcaggaag cggcccatca tcgtggtcag ggtcttgttg ctggtgaagg 22716
tcagcggaat gccgcggtgc tcctcgttca catacaggtg gcagatacgg cggtacacct 22776
cgccctgctc gggcatcagc tggaaggcgg acttcaggtc gctctccacg cggtaccggt 22836
ccatcagcag cgtcatcact tccatgccct tctcccaggc cgaaacgatc ggcaggctca 22896
gggggttctt caccgttgtc atcttagtcg ccgccgccga agtcaggggg tcgttctcgt 22956
ccagggtctc aaacactcgc ttgccgtcct tctcggtgat gcgcacgggg ggaaagctga 23016
agcccacggc cgccagctcc tcctcggcct gcctttcgtc ctcgctgtcc tggctgatgt 23076
cttgcaaagg cacatgcttg gtcttgcggg gtttcttttt gggcggcaga ggcggcggcg 23136
gagacgtgct gggcgagcgc gagttctcgc tcaccacgac tatttcttct ccttggccgt 23196
cgtccgagac cacgcggcgg taggcatgcc tcttctgggg cagaggcgga ggcgacgggc 23256
tctcgcggtt cggcgggcgg ctggcagagc cccttccgcg ttcgggggtg cgctcctggc 23316
ggcgctgctc tgactgactt cctccgcggc cggccattgt gttctcctag ggagcaagca 23376
tggagactca gccatcgtcg ccaacatcgc catctgcccc cgccgccgcc gacgagaacc 23436
agcagcagca gaatgaaagc ttaaccgccc cgccgcccag ccccacctcc gacgccgcag 23496
ccccagacat gcaagagatg gaggaatcca tcgagattga cctgggctac gtgacgcccg 23556
cggagcacga ggaggagctg gcagcgcgct tttcagcccc ggaagagaac caccaagagc 23616

agccagagca ggaagcagag agcgagcaga accaggctgg gctcgagcat ggcgactacc 23676
tgagcggggc agaggacgtg ctcatcaagc atctggcccg ccaatgcatc atcgtcaagg 23736
acgcgctgct cgaccgcgcc gaggtgcccc tcagcgtggc ggagctcagc cgcgcctacg 23796
agcgcaacct cttctcgccg cgcgtgcccc ccaagcgcca gcccaacggc acctgcgagc 23856
ccaacccgcg cctcaacttc tacccggtct tcgcggtgcc cgaggccctg gccacctacc 23916
acctcttttt caagaaccaa aggatccccg tctcctgccg cgccaaccgc acccgcgccg 23976
acgccctgct caacctgggc cccggcgccc gcctacctga tatcgcctcc ttggaagagg 24036
ttcccaagat cttcgagggt ctgggcagcg acgagactcg ggccgcgaac gctctgcaag 24096
gaagcggaga ggagcatgag caccacagcg ccctggtgga gttggaaggc gacaacgcgc 24156
gcctggcggt cctcaagcgc acggtcgagc tgacccactt cgcctacccg gcgctcaacc 24216
tgccccccaa ggtcatgagc gccgtcatgg accaggtgct catcaagcgc gcctcgcccc 24276
tctcggagga ggagatgcag gaccccgaga gctcggacga gggcaagccc gtggtcagcg 24336
acgagcagct ggcgcgctgg ctgggagcga gtagcacccc ccagagcctg gaagagcggc 24396
gcaagctcat gatggccgtg gtcctggtga ccgtggagct ggagtgtctg cgccgcttct 24456
tcgccgacgc ggagaccctg cgcaaggtcg aggagaacct gcactacctc ttcagacacg 24516
ggttcgtgcg ccaggcctgc aagatctcca acgtggagct gaccaacctg gtctcctaca 24576
tgggcatcct gcacgagaac cgcctggggc agaacgtgct gcacaccacc ctgcgcgggg 24636
aggcccgccg cgactacatc cgcgactgcg tctacctgta cctctgccac acctggcaga 24696
cgggcatggg cgtgtggcag cagtgcctgg aggagcagaa cctgaaagag ctctgcaagc 24756
tcctgcagaa gaacctcaag gccctgtgga ccgggttcga cgagcgcacc accgccgcgg 24816
acctggccga cctcatcttc cccgagcgcc tgcggctgac gctgcgcaac gggctgcccg 24876
actttatgag ccaaagcatg ttgcaaaact ttcgctcttt catcctcgaa cgctccggga 24936
tcctgcccgc cacctgctcc gcgctgccct cggacttcgt gccgctgacc ttccgcgagt 24996
gcccccgccc gctctggagc cactgctacc tgctgcgcct ggccaactac ctggcctacc 25056
actcggacgt gatcgaggac gtcagcggcg agggcctgct cgagtgccac tgccgctgca 25116
acctctgcac gccgcaccgc tccctggcct gcaaccccca gctgctgagc gagacccaga 25176
tcatcggcac cttcgagttg caaggccccg gcgagggcaa ggggggtctg aaactcaccc 25236
cggggctgtg gacctcggcc tacttgcgca agttcgtgcc cgaggactac catcccttcg 25296
agatcaggtt ctacgaggac caatcccagc cgcccaaggc cgagctgtcg gcctgcgtca 25356
tcacccaggg ggccatcctg gcccaattgc aagccatcca gaaatcccgc caagaatttc 25416
tgctgaaaaa gggccacggg gtctacttgg acccccagac cggagaggag ctcaacccca 25476
gcttccccca ggatgccccg aggaagcagc aagaagctga aagtggagct gccgccgccg 25536

ccggaggatt tggaggaaga ctgggagagc agtcaggcag aggaggagga gatggaagac 25596
tgggacagca ctcaggcaga ggaggacagc ctgcaagaca gtctggagga ggaagacgag 25656
gtggaggagg cagaggaaga agcagccgcc gccagaccgt cgtcctcggc ggaggaggag 25716
aaagcaagca gcacggatac catctccgct ccgggtcggg gtcgcggcgg ccgggcccac 25776
agtagatggg acgagaccgg gcgcttcccg aaccccacca cccagaccgg taagaaggag 25836
cggcagggat acaagtcctg gcgggggcac aaaaacgcca tcgtctcctg cttgcaagcc 25896
tgcgggggca acatctcctt cacccggcgc tacctgctct tccaccgcgg ggtgaacttc 25956
ccccgcaaca tcttgcatta ctaccgtcac ctccacagcc cctactactg tttccaagaa 26016
gaggcagaaa cccagcagca gcagcagcag cagaaaacca gcggcagcag ctagaaaatc 26076
cacagcggcg gcaggtggac tgaggatcgc ggcgaacgag ccggcgcaga cccgggagct 26136
gaggaaccgg atctttccca ccctctatgc catcttccag cagagtcggg ggcaagagca 26196
ggaactgaaa gtcaagaacc gttctctgcg ctcgctcacc cgcagttgtc tgtatcacaa 26256
gagcgaagac caacttcagc gcactctcga ggacgccgag gctctcttca acaagtactg 26316
cgcgctcact cttaaagagt agcccgcgcc cgcccacaca cggaaaaagg cgggaattac 26376
gtcaccacct gcgcccttcg cccgaccatc atcatgagca aagagattcc cacgccttac 26436
atgtggagct accagcccca gatgggcctg gccgccggcg ccgcccagga ctactccacc 26496
cgcatgaact ggctcagtgc cgggcccgcg atgatctcac gggtgaatga catccgcgcc 26556
caccgaaacc agatactcct agaacagtca gcgatcaccg ccacgccccg ccatcacctt 26616
aatccgcgta attggcccgc cgccctggtg taccaggaaa ttccccagcc cacgaccgta 26676
ctacttccgc gagacgccca ggccgaagtc cagctgacta actcaggtgt ccagctggcc 26736
ggcggcgccg ccctgtgtcg tcaccgcccc gctcagggta taaagcggct ggtgatccga 26796
ggcagaggca cacagctcaa cgacgaggtg gtgagctctt cgctgggtct gcgacctgac 26856
ggagtcttcc aactcgccgg atcggggaga tcttccttca cgcctcgtca ggccgtcctg 26916
actttggaga gttcgtcctc gcagccccgc tcgggtggca tcggcactct ccagttcgtg 26976
gaggagttca ctccctcggt ctacttcaac cccttctccg gctcccccgg ccactacccg 27036
gacgagttca tcccgaactt cgacgccatc agcgagtcgg tggacggcta cgattgaatg 27096
tcccatggtg gcgcggctga cctagctcgg cttcgacacc tggaccactg ccgccgcttc 27156
cgctgcttcg ctcgggatct cgccgagttt gcctactttg agctgcccga ggagcaccct 27216
cagggcccgg cccacggagt gcggatcgtc gtcgaagggg gtctcgactc ccacctgctt 27276
cggatcttca gccagcgtcc gatcctggcc gagcgcgagc aaggacagac ccttctgacc 27336
ctgtactgca tctgcaacca ccccggcctg catgaaagtc tttgttgtct gctgtgtact 27396

gagtataata aaagctgaga tcagcgacta ctccggactt ccgtgtgttc ctgctatcaa 27456
ccagtccctg ttcttcaccg ggaacgagac cgagctccag ctccagtgta agccccacaa 27516
gaagtacctc acctggctgt tccagggctc tccgatcgcc gttgtcaacc actgcgacaa 27576
cgacggagtc ctgctgagcg gccctgccaa ccttactttt tccacccgca gaagcaagct 27636
ccagctcttc caacccttcc tccccgggac ctatcagtgc gtctcgggac cctgccatca 27696
caccttccac ctgatcccga ataccacagc gtcgctcccc gctactaaca accaaactac 27756
ccaccaacgc caccgtcgcg acctttcctc tgggtctaat accactaccg gaggtgagct 27816
ccgaggtcga ccaacctctg ggatttacta cggcccctgg gaggtggtag ggttaatagc 27876
gctaggccta gttgcgggtg ggcttttggc tctctgctac ctatacctcc cttgctgttc 27936
gtacttagtg gtgctgtgtt gctggtttaa gaaatgggga agatcaccct agtgagctgc 27996
ggtgtgctgg tggcggtggt gctttcgatt gtgggactgg gcggcgcggc tgtagtgaag 28056
gagaaggccg atccctgctt gcatttcaat cccgacaaat gccagctgag ttttcagccc 28116
gatggcaatc ggtgcgcggt gctgatcaag tgcggatggg aatgcgagaa cgtgagaatc 28176
gagtacaata acaagactcg gaacaatact ctcgcgtccg tgtggcagcc cggggacccc 28236
gagtggtaca ccgtctctgt ccccggtgct gacggctccc cgcgcaccgt gaataatact 28296
ttcatttttg cgcacatgtg cgacacggtc atgtggatga gcaagcagta cgatatgtgg 28356
ccccccacga aggagaacat cgtggtcttc tccatcgctt acagcgtgtg cacggcgcta 28416
atcaccgcta tcgtgtgcct gagcattcac atgctcatcg ctattcgccc cagaaataat 28476
gccgaaaaag aaaaacagcc ataacacgtt ttttcacaca cctttttcag accatggcct 28536
ctgttaaatt tttgctttta tttgccagtc tcattgccgt cattcatgga atgagtaatg 28596
agaaaattac tatttacact ggcactaatc acacattgaa aggtccagaa aaagccacag 28656
aagtttcatg gtattgttat tttaatgaat cagatgtatc tactgaactc tgtggaaaca 28716
ataacaaaaa aaatgagagc attactctca tcaagtttca atgtggatct gacttaaccc 28776
taattaacat cactagagac tatgtaggta tgtattatgg aactacagca ggcatttcgg 28836
acatggaatt ttatcaagtt tctgtgtctg aacccaccac gcctagaatg accacaacca 28896
caaaaactac acctgttacc actatacagc tcactaccaa tggctttctt gccatgcttc 28956
aagtggctga aaatagcacc agcattcaac ccaccccacc cagtgaggaa attcccagat 29016
ccatgattgg cattattgtt gctgtagtgg tgtgcatgtt gatcatcgcc ttgtgcatgg 29076
tgtactatgc cttctgctac agaaagcaca gactgaacga caagctggaa cacttactaa 29136
gtgttgaatt ttaatttttt agaaccatga agatcctagg ccttttagtt ttttctatca 29196
ttacctctgc tctatgcaat tctgacaatg aggacgttac tgtcgttgtc ggatcaaatt 29256
atacactaaa aggtccagca aaaggtatgc tttcgtggta ttgttggttc ggaactgacg 29316

agcaacagac agaactttgc aatgctcaaa aaggcaaaac ctcaaattct aaaatctcta 29376
attatcaatg caatggcact gacttagtat tgctcaatgt cacgaaagca tatgctggca 29436
gttacacctg ccctggagat gatgccgaca atatgatttt ttacaaagtg gaagtggttg 29496
atcccactac tccaccgccc accaccacaa ctactcatac cacacacaca gaacaaacac 29556
cagaggcagc agaagcagag ttggccttcc aggttcacgg agattccttt gctgtcaata 29616
cccctacacc cgatcagcgg tgtccggggc tgctcgtcag cggcattgtc ggtgtgcttt 29676
cgggattagc agtcataatc atctgcatgt tcatttttgc ttgctgctat agaaggcttt 29736
accgacaaaa atcagaccca ctgctgaacc tctatgttta attttttcca gagccatgaa 29796
ggcagttagc gctctagttt tttgttcttt gattggcatt gtttttagtg ctgggttttt 29856
gaaaaatctt accatttatg aaggtgagaa tgccactcta gtgggcatca gtggtcaaaa 29916
tgtcagctgg ctaaaatacc atctagatgg gtggaaagac atttgcgatt ggaatgtcac 29976
tgtgtataca tgtaatggag ttaacctcac cattactaat gccacccaag atcagaatgg 30036
taggtttaag ggccagagtt tcactagaaa taatgggtat gaatcccata acatgtttat 30096
ctatgacgtc actgtcatca gaaatgagac tgccaccacc acacagatgc ccactacaca 30156
cagttctacc actactacca tgcaaaccac acagacaacc actacatcaa ctcagcatat 30216
gaccaccact acagcagcaa agccaagtag tgcagcgcct cagccccagg ctttggcttt 30276
gaaagctgca caacctagta caactactag gaccaatgag cagactactg aatttttgtc 30336
cactgtcgag agccacacca cagctacctc cagtgccttc tctagcaccg ccaatctctc 30396
ctcgctttcc tctacaccaa tcagtcccgc tactactccc accccagctc ttctccccac 30456
tcccctgaag caaactgagg acagcggcat gcaatggcag atcaccctgc tcattgtgat 30516
cgggttggtc atcctggccg tgttgctcta ctacatcttc tgccgccgca ttcccaacgc 30576
gcaccgcaaa ccggcctaca agcccatcgt tatcgggcag ccggagccgc ttcaggtgga 30636
agggggtcta aggaatcttc tcttctcttt tacagtatgg tgattgaact atgattccta 30696
gacaattctt gatcactatt cttatctgcc tcctccaagt ctgtgccacc ctcgctctgg 30756
tggccaacgc cagtccagac tgtattgggc ccttcgcctc ctacgtgctc tttgccttca 30816
tcacctgcat ctgctgctgt agcatagtct gcctgcttat caccttcttc cagttcattg 30876
actggatctt tgtgcgcatc gcctacctgc gccaccaccc ccagtaccgc gaccagcgag 30936
tggcgcggct gctcaggctc ctctgataag catgcgggct ctgctacttc tcgcgcttct 30996
gctgttagtg ctcccccgcc ccgtcgaccc ccggtccccc actcagtccc ccgaagaggt 31056
ccgcaaatgc aaattccaag aaccctggaa attcctcaaa tgctaccgcc aaaaatcaga 31116
catgcttccc agctggatca tgatcattgg gatcgtgaac attctggcct gcaccctcat 31176

ctcctttgtg atttacccct gctttgactt tggttggaac tcgccagagg cgctctatct 31236

cccgcctgaa cctgacacac caccacagca acctcaggca cacgcactac caccaccaca 31296

gcctaggcca caatacatgc ccatattaga ctatgaggcc gagccacagc gacccatgct 31356

ccccgctatt agttacttca atctaaccgg cggagatgac tgacccactg gccaacaaca 31416

acgtcaacga ccttctcctg gacatggacg gccgcgcctc ggagcagcga ctcgcccaac 31476

ttcgcattcg ccagcagcag gagagagccg tcaaggagct gcaggacggc atagccatcc 31536

accagtgcaa gaaaggcatc ttctgcctgg tgaaacaggc caagatctcc tacgaggtca 31596

ccccgaccga ccatcgcctc tcctacgagc tcctgcagca gcgccagaag ttcacctgcc 31656

tggtcggagt caaccccatc gtcatcaccc agcagtcggg cgataccaag gggtgcatcc 31716

actgctcctg cgactccccc gactgcgtcc acactctgat caagaccctc tgcggcctcc 31776

gcgacctcct ccccatgaac taatcacccc cttatccagt gaaataaata tcatattgat 31836

gatgatttaa ataaaaaata atcatttgat ttgaaataaa gatacaatca tattgatgat 31896

ttgagtttta aaaaataaag aatcacttac ttgaaatctg ataccaggtc tctgtccatg 31956

ttttctgcca acaccacctc actcccctct tcccagctct ggtactgcag accccggcgg 32016

gctgcaaact tcctccacac gctgaagggg atgtcaaatt cctcctgtcc ctcaatcttc 32076

attttatctt ctatcag atg tcc aaa aag cgc gtc cgg gtg gat gat gac 32126
Met Ser Lys Lys Arg Val Arg Val Asp Asp Asp
    1465                1470

ttc gac ccc gtc tac ccc tac gat gca gac aac gca ccg acc gtg 32171
Phe Asp Pro Val Tyr Pro Tyr Asp Ala Asp Asn Ala Pro Thr Val
1475                1480                1485

ccc ttc atc aac ccc ccc ttc gtc tct tca gat gga ttc caa gag 32216
Pro Phe Ile Asn Pro Pro Phe Val Ser Ser Asp Gly Phe Gln Glu
1490                1495                1500

aag ccc ctg ggg gtg ctg tcc ctg cga ctg gct gac ccc gtc acc 32261
Lys Pro Leu Gly Val Leu Ser Leu Arg Leu Ala Asp Pro Val Thr
1505                1510                1515

acc aag aac ggg gaa atc acc ctc aag ctg gga gag ggg gtg gac 32306
Thr Lys Asn Gly Glu Ile Thr Leu Lys Leu Gly Glu Gly Val Asp
1520                1525                1530

ctc gac tcc tcg gga aaa ctc atc tcc aac acg gcc acc aag gcc 32351
Leu Asp Ser Ser Gly Lys Leu Ile Ser Asn Thr Ala Thr Lys Ala
1535                1540                1545

gcc gcc cct ctc agt ttt tcc aac aac acc att tcc ctt aac atg 32396
Ala Ala Pro Leu Ser Phe Ser Asn Asn Thr Ile Ser Leu Asn Met
1550                1555                1560

gat acc cct ctt tat acc aaa gat gga aaa tta tcc tta caa gtt 32441
Asp Thr Pro Leu Tyr Thr Lys Asp Gly Lys Leu Ser Leu Gln Val
1565                1570                1575

tct cca ccg tta aac ata tta aaa tca acc att ctg aac aca tta 32486

Ser Pro Pro Leu Asn Ile Leu Lys Ser Thr Ile Leu Asn Thr Leu
1580                1585                1590

gct gta gct tat gga tca ggt tta gga ctg agt ggt ggc act gct 32531
Ala Val Ala Tyr Gly Ser Gly Leu Gly Leu Ser Gly Gly Thr Ala
1595                1600                1605

ctt gca gta cag ttg gcc tct cca ctc act ttt gat gaa aaa gga 32576
Leu Ala Val Gln Leu Ala Ser Pro Leu Thr Phe Asp Glu Lys Gly
1610                1615                1620

aat att aaa att aac cta gcc agt ggt cca tta aca gtt gat gca 32621
Asn Ile Lys Ile Asn Leu Ala Ser Gly Pro Leu Thr Val Asp Ala
1625                1630                1635

agt cga ctt agt atc aac tgc aaa aga ggg gtc act gtc act acc 32666
Ser Arg Leu Ser Ile Asn Cys Lys Arg Gly Val Thr Val Thr Thr
1640                1645                1650

tca gga gat gca att gaa agc aac ata agc tgg cct aaa ggt ata 32711
Ser Gly Asp Ala Ile Glu Ser Asn Ile Ser Trp Pro Lys Gly Ile
1655                1660                1665

aga ttt gaa ggt aat ggc ata gct gca aac att ggc aga gga ttg 32756
Arg Phe Glu Gly Asn Gly Ile Ala Ala Asn Ile Gly Arg Gly Leu
1670                1675                1680

gaa ttt gga acc act agt aca gag act gat gtc aca gat gca tac 32801
Glu Phe Gly Thr Thr Ser Thr Glu Thr Asp Val Thr Asp Ala Tyr
1685                1690                1695

cca att caa gtt aaa ttg ggt act ggc ctt acc ttt gac agt aca 32846
Pro Ile Gln Val Lys Leu Gly Thr Gly Leu Thr Phe Asp Ser Thr
1700                1705                1710

ggc gcc att gtt gct tgg aac aaa gag gat gat aaa ctt aca tta 32891
Gly Ala Ile Val Ala Trp Asn Lys Glu Asp Asp Lys Leu Thr Leu
1715                1720                1725

tgg acc aca gcc gac ccc tcg cca aat tgc aaa ata tac tct gaa 32936
Trp Thr Thr Ala Asp Pro Ser Pro Asn Cys Lys Ile Tyr Ser Glu
1730                1735                1740

aaa gat gcc aaa ctc aca ctt tgc ttg aca aag tgt gga agt caa 32981
Lys Asp Ala Lys Leu Thr Leu Cys Leu Thr Lys Cys Gly Ser Gln
1745                1750                1755

att ctg ggt act gtg act gta ttg gca gtg aat aat gga agt ctc 33026
Ile Leu Gly Thr Val Thr Val Leu Ala Val Asn Asn Gly Ser Leu
1760                1765                1770

aac cca atc aca aac aca gta agc act gca ctc gtc tcc ctc aag 33071
Asn Pro Ile Thr Asn Thr Val Ser Thr Ala Leu Val Ser Leu Lys
1775                1780                1785

ttt gat gca agt gga gtt ttg cta agc agc tcc aca tta gac aaa 33116
Phe Asp Ala Ser Gly Val Leu Leu Ser Ser Ser Thr Leu Asp Lys
1790                1795                1800

gaa tat tgg aac ttc aga aag gga gat gtt aca cct gct gag ccc 33161
Glu Tyr Trp Asn Phe Arg Lys Gly Asp Val Thr Pro Ala Glu Pro
1805                1810                1815

tat act aat gct ata ggt ttt atg cct aac ata aag gcc tat cct 33206
Tyr Thr Asn Ala Ile Gly Phe Met Pro Asn Ile Lys Ala Tyr Pro
1820                1825                1830

aaa aac aca tct gca gct tca aaa agc cat att gtc agt caa gtt 33251
Lys Asn Thr Ser Ala Ala Ser Lys Ser His Ile Val Ser Gln Val
1835                1840                1845

tat ctc aat ggg gat gag gcc aaa cca ctg atg ctg att att act 33296
Tyr Leu Asn Gly Asp Glu Ala Lys Pro Leu Met Leu Ile Ile Thr
1850                1855                1860

ttt aat gaa act gag gat gca act tgc acc tac agt atc act ttt 33341
Phe Asn Glu Thr Glu Asp Ala Thr Cys Thr Tyr Ser Ile Thr Phe
1865                1870                1875

caa tgg aaa tgg gat agt act aag tac aca ggt gaa aca ctt gct 33386
Gln Trp Lys Trp Asp Ser Thr Lys Tyr Thr Gly Glu Thr Leu Ala
1880                1885                1890

acc agc tcc ttc acc ttc tcc tac atc gcc caa gaa tga acactgtatc 33435
Thr Ser Ser Phe Thr Phe Ser Tyr Ile Ala Gln Glu
1895                1900                1905

ccaccctgca tgccaaccct tcccacccca ctctgtctat ggaaaaaact ctgaagcaca 33495

aaataaaata aagttcaagt gttttattga ttcaacagtt ttacaggatt cgagcagtta 33555

tttttcctcc accctcccag gacatggaat acaccaccct ctccccccgc acagccttga 33615

acatctgaat gccattggtg atggacatgc ttttggtctc cacgttccac acagtttcag 33675

agcgagccag tctcgggtcg gtcagggaga tgaaaccctc cgggcactcc cgcatctgca 33735

cctcacagct caacagctga ggattgtcct cggtggtcgg gatcacggtt atctggaaga 33795

agcagaagag cggcggtggg aatcatagtc cgcgaacggg atcggccggt ggtgtcgcat 33855

caggccccgc agcagtcgct gccgccgccg ctccgtcaag ctgctgctca gggggtccgg 33915

gtccagggac tccctcagca tgatgcccac ggccctcagc atcagtcgtc tggtgcggcg 33975

ggcgcagcag cgcatgcgga tctcgctcag gtcgctgcag tacgtgcaac acaggaccac 34035

caggttgttc aacagtccat agttcaacac gctccagccg aaactcatcg cgggaaggat 34095

gctacccacg tggccgtcgt accagatcct caggtaaatc aagtggcgct ccctccagaa 34155

cacgctgccc acgtacatga tctccttggg catgtggcgg ttcaccacct cccggtacca 34215

catcaccctc tggttgaaca tgcagccccg gatgatcctg cggaaccaca gggccagcac 34275

cgccccgccc gccatgcagc gaagagaccc cgggtcccgg caatggcaat ggaggaccca 34335

ccgctcgtac ccgtggatca tctgggagct gaacaagtct atgttggcac agcacaggca 34395

tatgctcatg catctcttca gcactctcag ctcctcgggg gtcaaaacca tatcccaggg 34455

cacggggaac tcttgcagga cagcgaaccc cgcagaacag ggcaatcctc gcacataact 34515

tacattgtgc atggacaggg tatcgcaatc aggcagcacc gggtgatcct ccaccagaga 34575

agcgcgggtc tcggtctcct cacagcgtgg taagggggcc ggccgatacg ggtgatggcg 34635

ggacgcggct gatcgtgttc gcgaccgtgt catgatgcag ttgctttcgg acattttcgt 34695
acttgctgta gcagaacctg gtccgggcgc tgcacaccga tcgccggcgg cggtcccggc 34755
gcttggaacg ctcggtgttg aaattgtaaa acagccactc tctcagaccg tgcagcagat 34815
ctagggcctc aggagtgatg aagatcccat catgcctgat agctctgatc acatcgacca 34875
ccgtggaatg ggccagaccc agccagatga tgcaattttg ttgggtttcg gtgacggcgg 34935
gggagggaag aacaggaaga accatgatta acttttaatc caaacggtct cggagcactt 34995
caaaatgaag gtcgcggaga tggcacctct cgcccccgct gtgttggtgg aaaataacag 35055
ccaggtcaaa ggtgatacgg ttctcgagat gttccacggt ggcttccagc aaagcctcca 35115
cgcgcacatc cagaaacaag acaatagcga aagcgggagg gttctctaat tcctcaatca 35175
tcatgttaca ctcctgcacc atccccagat aattttcatt tttccagcct tgaatgattc 35235
gaactagttc ctgaggtaaa tccaagccag ccatgataaa gagctcgcgc agagcgccct 35295
ccaccggcat tcttaagcac accctcataa ttccaagata ttctgctcct ggttcacctg 35355
cagcagattg acaagcggaa tatcaaaatc tctgccgcga tccctaagct cctccctcag 35415
caataactgt aagtactctt tcatatcctc tccgaaattt ttagccatag gaccaccagg 35475
aataagatta gggcaagcca cagtacagat aaaccgaagt cctccccagt gagcattgcc 35535
aaatgcaaga ctgctataag catgctggct agacccggtg atatcttcca gataactgga 35595
cagaaaatca cccaggcaat ttttaagaaa atcaacaaaa gaaaaatcct ccaggtgcac 35655
gtttagagcc tcgggaacaa cgatgaagta aatgcaagcg gtgcgttcca gcatggttag 35715
ttagctgatc tgtaaaaaac aaaaaataaa acattaaacc atgctagcct ggcgaacagg 35775
tgggtaaatc gttctctcca gcaccaggca ggccacgggg tctccggcgc gaccctcgta 35835
aaaattgtcg ctatgattga aaaccatcac agagagacgt tcccggtggc cggcgtgaat 35895
gattcgacaa gatgaataca cccccggaac attggcgtcc gcgagtgaaa aaaagcgccc 35955
gaggaagcaa taaggcacta caatgctcag tctcaagtcc agcaaagcga tgccatgcgg 36015
atgaagcaca aaatcctcag gtgcgtacaa aatgtaatta ctcccctcct gcacaggcag 36075
cgaagccccc gatccctcca gatacacata caaagcctca gcgtccatag cttaccgagc 36135
agcagcacac aacaggcgca agagtcagag aaaggctgag ctctaacctg tccacccgct 36195
ctctgctcaa tatatagccc agatctacac tgacgtaaag gccaaagtct aaaaataccc 36255
gccaaataat cacacacgcc cagcacacgc ccagaaaccg gtgacacact caaaaaaata 36315
cgcgcacttc ctcaaacgcc caaactgccg tcatttccgg gttcccacgc tacgtcatcg 36375
gaattcgact ttcaaattcc gtcgaccgtt aaaaacgtca cccgccccgc ccctaacggt 36435
cgcccgtctc tcggccaatc accttcctcc ctccccaaat tcaaacagct catttgcata 36495

ttaacgcgca ccaaaagttt gaggtatatt attgatgatg 36535

<210> 10

<211> 531

<212> PRT

<213> chimpanzee adenovirus serotype Pan7

<400> 10

Met Met Arg Arg Val Tyr Pro Glu Gly Pro Pro Pro Ser Tyr Glu Ser
1               5                   10                  15

Val Met Gln Gln Ala Val Ala Ala Ala Met Gln Pro Pro Leu Glu Ala
            20                  25                  30

Pro Tyr Val Pro Pro Arg Tyr Leu Ala Pro Thr Glu Gly Arg Asn Ser
        35                  40                  45

Ile Arg Tyr Ser Glu Leu Ala Pro Leu Tyr Asp Thr Thr Arg Leu Tyr
    50                  55                  60

Leu Val Asp Asn Lys Ser Ala Asp Ile Ala Ser Leu Asn Tyr Gln Asn
65                  70                  75                  80

Asp His Ser Asn Phe Leu Thr Thr Val Val Gln Asn Asn Asp Phe Thr
                85                  90                  95

Pro Thr Glu Ala Ser Thr Gln Thr Ile Asn Phe Asp Glu Arg Ser Arg
            100                 105                 110

Trp Gly Gly Gln Leu Lys Thr Ile Met His Thr Asn Met Pro Asn Val
        115                 120                 125

Asn Glu Phe Met Tyr Ser Asn Lys Phe Lys Ala Arg Val Met Val Ser
    130                 135                 140

Arg Lys Thr Pro Asn Gly Val Ala Val Asp Glu Asn Tyr Asp Gly Ser
145                 150                 155                 160

Gln Asp Glu Leu Thr Tyr Glu Trp Val Glu Phe Glu Leu Pro Glu Gly
                165                 170                 175

Asn Phe Ser Val Thr Met Thr Ile Asp Leu Met Asn Asn Ala Ile Ile
            180                 185                 190

Asp Asn Tyr Leu Ala Val Gly Arg Gln Asn Gly Val Leu Glu Ser Asp
        195                 200                 205

Ile Gly Val Lys Phe Asp Thr Arg Asn Phe Arg Leu Gly Trp Asp Pro
    210                 215                 220

Val Thr Glu Leu Val Met Pro Gly Val Tyr Thr Asn Glu Ala Phe His
225                 230                 235                 240

Pro Asp Ile Val Leu Leu Pro Gly Cys Gly Val Asp Phe Thr Glu Ser
                245                 250                 255

Arg Leu Ser Asn Leu Leu Gly Ile Arg Lys Arg Gln Pro Phe Gln Glu
            260                 265                 270

Gly Phe Gln Ile Leu Tyr Glu Asp Leu Glu Gly Gly Asn Ile Pro Ala
        275                 280                 285

Leu Leu Asp Val Glu Ala Tyr Glu Lys Ser Lys Glu Glu Ala Ala Ala
    290                 295                 300

Ala Ala Thr Ala Ala Val Ala Thr Ala Ser Thr Glu Val Arg Gly Asp
305                 310                 315                 320

Asn Phe Ala Ser Ala Ala Ala Val Ala Glu Ala Ala Glu Thr Glu Ser
                325                 330                 335

Lys Ile Val Ile Gln Pro Val Glu Lys Asp Ser Lys Asp Arg Ser Tyr
            340                 345                 350

Asn Val Leu Ala Asp Lys Lys Asn Thr Ala Tyr Arg Ser Trp Tyr Leu
        355                 360                 365

Ala Tyr Asn Tyr Gly Asp Pro Glu Lys Gly Val Arg Ser Trp Thr Leu
    370                 375                 380

Leu Thr Thr Ser Asp Val Thr Cys Gly Val Glu Gln Val Tyr Trp Ser
385                 390                 395                 400

Leu Pro Asp Met Met Gln Asp Pro Val Thr Phe Arg Ser Thr Arg Gln
                405                 410                 415

Val Ser Asn Tyr Pro Val Val Gly Ala Glu Leu Leu Pro Val Tyr Ser
            420                 425                 430

Lys Ser Phe Phe Asn Glu Gln Ala Val Tyr Ser Gln Gln Leu Arg Ala
        435                 440                 445

Phe Thr Ser Leu Thr His Val Phe Asn Arg Phe Pro Glu Asn Gln Ile
    450                 455                 460

Leu Val Arg Pro Pro Ala Pro Thr Ile Thr Thr Val Ser Glu Asn Val
465                 470                 475                 480

Pro Ala Leu Thr Asp His Gly Thr Leu Pro Leu Arg Ser Ser Ile Arg
                485                 490                 495

Gly Val Gln Arg Val Thr Val Thr Asp Ala Arg Arg Arg Thr Cys Pro
            500                 505                 510

Tyr Val Tyr Lys Ala Leu Gly Val Val Ala Pro Arg Val Leu Ser Ser
        515                 520                 525

Arg Thr Phe
    530

<210>  11

<211>  932

<212>  PRT

<213>  chimpanzee adenovirus serotype Pan7

<400>  11

Met Ala Thr Pro Ser Met Leu Pro Gln Trp Ala Tyr Met His Ile Ala
1               5                   10                  15

Gly Gln Asp Ala Ser Glu Tyr Leu Ser Pro Gly Leu Val Gln Phe Ala
            20                  25                  30

Arg Ala Thr Asp Thr Tyr Phe Ser Leu Gly Asn Lys Phe Arg Asn Pro
        35                  40                  45

Thr Val Ala Pro Thr His Asp Val Thr Thr Asp Arg Ser Gln Arg Leu
    50                  55                  60

Thr Leu Arg Phe Val Pro Val Asp Arg Glu Asp Asn Thr Tyr Ser Tyr
65                  70                  75                  80

Lys Val Arg Tyr Thr Leu Ala Val Gly Asp Asn Arg Val Leu Asp Met
                85                  90                  95

Ala Ser Thr Tyr Phe Asp Ile Arg Gly Val Leu Asp Arg Gly Pro Ser
            100                 105                 110

Phe Lys Pro Tyr Ser Gly Thr Ala Tyr Asn Ser Leu Ala Pro Lys Gly
        115                 120                 125

Ala Pro Asn Thr Cys Gln Trp Thr Tyr Lys Ala Gly Asp Thr Asp Thr
    130                 135                 140

Glu Lys Thr Tyr Thr Tyr Gly Asn Ala Pro Val Gln Gly Ile Ser Ile
145                 150                 155                 160

Thr Lys Asp Gly Ile Gln Leu Gly Thr Asp Ser Asp Gly Gln Ala Ile
                165                 170                 175

Tyr Ala Asp Glu Thr Tyr Gln Pro Glu Pro Gln Val Gly Asp Ala Glu
            180                 185                 190

Trp His Asp Ile Thr Gly Thr Asp Glu Lys Tyr Gly Gly Arg Ala Leu
        195                 200                 205

Lys Pro Asp Thr Lys Met Lys Pro Cys Tyr Gly Ser Phe Ala Lys Pro
    210                 215                 220

Thr Asn Lys Glu Gly Gly Gln Ala Asn Val Lys Thr Glu Thr Gly Gly
225                 230                 235                 240

Thr Lys Glu Tyr Asp Ile Asp Met Ala Phe Phe Asp Asn Arg Ser Ala
                245                 250                 255

Ala Ala Ala Gly Leu Ala Pro Glu Ile Val Leu Tyr Thr Glu Asn Val
            260                 265                 270

Asp Leu Glu Thr Pro Asp Thr His Ile Val Tyr Lys Ala Gly Thr Asp
        275                 280                 285

Asp Ser Ser Ser Ser Ile Asn Leu Gly Gln Gln Ser Met Pro Asn Arg
    290                 295                 300

Pro Asn Tyr Ile Gly Phe Arg Asp Asn Phe Ile Gly Leu Met Tyr Tyr
305                 310                 315                 320

Asn Ser Thr Gly Asn Met Gly Val Leu Ala Gly Gln Ala Ser Gln Leu
                325                 330                 335

Asn Ala Val Val Asp Leu Gln Asp Arg Asn Thr Glu Leu Ser Tyr Gln
            340                 345                 350

Leu Leu Leu Asp Ser Leu Gly Asp Arg Thr Arg Tyr Phe Ser Met Trp

        355                 360                 365

Asn Gln Ala Val Asp Ser Tyr Asp Pro Asp Val Arg Ile Ile Glu Asn
    370                 375                 380

His Gly Val Glu Asp Glu Leu Pro Asn Tyr Cys Phe Pro Leu Asp Ala
385                 390                 395                 400

Val Gly Arg Thr Asp Thr Tyr Gln Gly Ile Lys Ala Asn Gly Asp Asn
                405                 410                 415

Gln Thr Thr Trp Thr Lys Asp Asp Thr Val Asn Asp Ala Asn Glu Leu
            420                 425                 430

Gly Lys Gly Asn Pro Phe Ala Met Glu Ile Asn Ile Gln Ala Asn Leu
        435                 440                 445

Trp Arg Asn Phe Leu Tyr Ala Asn Val Ala Leu Tyr Leu Pro Asp Ser
    450                 455                 460

Tyr Lys Tyr Thr Pro Ala Asn Ile Thr Leu Pro Thr Asn Thr Asn Thr
465                 470                 475                 480

Tyr Asp Tyr Met Asn Gly Arg Val Val Ala Pro Ser Leu Val Asp Ala
                485                 490                 495

Tyr Ile Asn Ile Gly Ala Arg Trp Ser Leu Asp Pro Met Asp Asn Val
            500                 505                 510

Asn Pro Phe Asn His His Arg Asn Ala Gly Leu Arg Tyr Arg Ser Met
        515                 520                 525

Leu Leu Gly Asn Gly Arg Tyr Val Pro Phe His Ile Gln Val Pro Gln
    530                 535                 540

Lys Phe Phe Ala Ile Lys Ser Leu Leu Leu Leu Pro Gly Ser Tyr Thr
545                 550                 555                 560

Tyr Glu Trp Asn Phe Arg Lys Asp Val Asn Met Ile Leu Gln Ser Ser
                565                 570                 575

Leu Gly Asn Asp Leu Arg Thr Asp Gly Ala Ser Ile Ala Phe Thr Ser
            580                 585                 590

Ile Asn Leu Tyr Ala Thr Phe Phe Pro Met Ala His Asn Thr Ala Ser
        595                 600                 605

Thr Leu Glu Ala Met Leu Arg Asn Asp Thr Asn Asp Gln Ser Phe Asn
    610                 615                 620

Asp Tyr Leu Ser Ala Ala Asn Met Leu Tyr Pro Ile Pro Ala Asn Ala
625                 630                 635                 640

Thr Asn Val Pro Ile Ser Ile Pro Ser Arg Asn Trp Ala Ala Phe Arg
                645                 650                 655

Gly Trp Ser Phe Thr Arg Leu Lys Thr Arg Glu Thr Pro Ser Leu Gly
            660                 665                 670

Ser Gly Phe Asp Pro Tyr Phe Val Tyr Ser Gly Ser Ile Pro Tyr Leu
        675                 680                 685

Asp Gly Thr Phe Tyr Leu Asn His Thr Phe Lys Lys Val Ser Ile Thr
    690                 695                 700

Phe Asp Ser Ser Val Ser Trp Pro Gly Asn Asp Arg Leu Leu Thr Pro
705                 710                 715                 720

Asn Glu Phe Glu Ile Lys Arg Thr Val Asp Gly Glu Gly Tyr Asn Val
                725                 730                 735

Ala Gln Cys Asn Met Thr Lys Asp Trp Phe Leu Val Gln Met Leu Ala
            740                 745                 750

His Tyr Asn Ile Gly Tyr Gln Gly Phe Tyr Val Pro Glu Gly Tyr Lys
        755                 760                 765

Asp Arg Met Tyr Ser Phe Phe Arg Asn Phe Gln Pro Met Ser Arg Gln
    770                 775                 780

Val Val Asp Glu Val Asn Tyr Lys Asp Tyr Gln Ala Val Thr Leu Ala
785                 790                 795                 800

Tyr Gln His Asn Asn Ser Gly Phe Val Gly Tyr Leu Ala Pro Thr Met
                805                 810                 815

Arg Gln Gly Gln Pro Tyr Pro Ala Asn Tyr Pro Tyr Pro Leu Ile Gly
            820                 825                 830

Lys Ser Ala Val Ala Ser Val Thr Gln Lys Lys Phe Leu Cys Asp Arg
        835                 840                 845

Val Met Trp Arg Ile Pro Phe Ser Ser Asn Phe Met Ser Met Gly Ala
    850                 855                 860

Leu Thr Asp Leu Gly Gln Asn Met Leu Tyr Ala Asn Ser Ala His Ala
865                 870                 875                 880

Leu Asp Met Asn Phe Glu Val Asp Pro Met Asp Glu Ser Thr Leu Leu
                885                 890                 895

Tyr Val Val Phe Glu Val Phe Asp Val Val Arg Val His Gln Pro His
            900                 905                 910

Arg Gly Val Ile Glu Ala Val Tyr Leu Arg Thr Pro Phe Ser Ala Gly
        915                 920                 925

Asn Ala Thr Thr
    930

<210>  12

<211>  443

<212>  PRT

<213>  chimpanzee adenovirus serotype Pan7

<400>  12

Met Ser Lys Lys Arg Val Arg Val Asp Asp Asp Phe Asp Pro Val Tyr
1               5                   10                  15

Pro Tyr Asp Ala Asp Asn Ala Pro Thr Val Pro Phe Ile Asn Pro Pro
            20                  25                  30

Phe Val Ser Ser Asp Gly Phe Gln Glu Lys Pro Leu Gly Val Leu Ser
        35                  40                  45

Leu Arg Leu Ala Asp Pro Val Thr Thr Lys Asn Gly Glu Ile Thr Leu
    50                  55                  60

Lys Leu Gly Glu Gly Val Asp Leu Asp Ser Ser Gly Lys Leu Ile Ser
65                  70                  75                  80

Asn Thr Ala Thr Lys Ala Ala Ala Pro Leu Ser Phe Ser Asn Asn Thr
                85                  90                  95

Ile Ser Leu Asn Met Asp Thr Pro Leu Tyr Thr Lys Asp Gly Lys Leu
            100                 105                 110

Ser Leu Gln Val Ser Pro Pro Leu Asn Ile Leu Lys Ser Thr Ile Leu
        115                 120                 125

Asn Thr Leu Ala Val Ala Tyr Gly Ser Gly Leu Gly Leu Ser Gly Gly
    130                 135                 140

Thr Ala Leu Ala Val Gln Leu Ala Ser Pro Leu Thr Phe Asp Glu Lys
145                 150                 155                 160

Gly Asn Ile Lys Ile Asn Leu Ala Ser Gly Pro Leu Thr Val Asp Ala
                165                 170                 175

Ser Arg Leu Ser Ile Asn Cys Lys Arg Gly Val Thr Val Thr Thr Ser
            180                 185                 190

Gly Asp Ala Ile Glu Ser Asn Ile Ser Trp Pro Lys Gly Ile Arg Phe
        195                 200                 205

Glu Gly Asn Gly Ile Ala Ala Asn Ile Gly Arg Gly Leu Glu Phe Gly
    210                 215                 220

Thr Thr Ser Thr Glu Thr Asp Val Thr Asp Ala Tyr Pro Ile Gln Val
225                 230                 235                 240

Lys Leu Gly Thr Gly Leu Thr Phe Asp Ser Thr Gly Ala Ile Val Ala
                245                 250                 255

Trp Asn Lys Glu Asp Asp Lys Leu Thr Leu Trp Thr Thr Ala Asp Pro
            260                 265                 270

Ser Pro Asn Cys Lys Ile Tyr Ser Glu Lys Asp Ala Lys Leu Thr Leu
        275                 280                 285

Cys Leu Thr Lys Cys Gly Ser Gln Ile Leu Gly Thr Val Thr Val Leu
    290                 295                 300

Ala Val Asn Asn Gly Ser Leu Asn Pro Ile Thr Asn Thr Val Ser Thr
305                 310                 315                 320

Ala Leu Val Ser Leu Lys Phe Asp Ala Ser Gly Val Leu Leu Ser Ser
                325                 330                 335

Ser Thr Leu Asp Lys Glu Tyr Trp Asn Phe Arg Lys Gly Asp Val Thr
            340                 345                 350

Pro Ala Glu Pro Tyr Thr Asn Ala Ile Gly Phe Met Pro Asn Ile Lys
        355                 360                 365

Ala Tyr Pro Lys Asn Thr Ser Ala Ala Ser Lys Ser His Ile Val Ser

370                 375                 380

Gln Val Tyr Leu Asn Gly Asp Glu Ala Lys Pro Leu Met Leu Ile Ile
385                 390                 395                 400

Thr Phe Asn Glu Thr Glu Asp Ala Thr Cys Thr Tyr Ser Ile Thr Phe
                405                 410                 415

Gln Trp Lys Trp Asp Ser Thr Lys Tyr Thr Gly Glu Thr Leu Ala Thr
            420                 425                 430

Ser Ser Phe Thr Phe Ser Tyr Ile Ala Gln Glu
        435                 440

<210>  13

<211>  338

<212>  PRT

<213>  simian serotype C1

<400>  13

Ala Pro Lys Gly Ala Pro Asn Thr Ser Gln Trp Leu Asp Lys Gly Val
1               5                   10                  15

Thr Thr Thr Asp Asn Asn Thr Glu Asn Gly Asp Glu Glu Asp Glu Val
            20                  25                  30

Ala Glu Glu Gly Glu Glu Glu Lys Gln Ala Thr Tyr Thr Phe Gly Asn
        35                  40                  45

Ala Pro Val Lys Ala Glu Ala Glu Ile Thr Lys Glu Gly Leu Pro Ile
    50                  55                  60

Gly Leu Glu Val Pro Ser Glu Gly Asp Pro Lys Pro Ile Tyr Ala Asp
65                  70                  75                  80

Lys Leu Tyr Gln Pro Glu Pro Gln Val Gly Glu Glu Ser Trp Thr Asp
                85                  90                  95

Thr Asp Gly Thr Asp Glu Lys Tyr Gly Gly Arg Ala Leu Lys Pro Glu
            100                 105                 110

Thr Lys Met Lys Pro Cys Tyr Gly Ser Phe Ala Lys Pro Thr Asn Val
        115                 120                 125

Lys Gly Gly Gln Ala Lys Val Lys Lys Val Glu Glu Gly Lys Val Glu
    130                 135                 140

Tyr Asp Ile Asp Met Asn Phe Phe Asp Leu Arg Ser Gln Lys Thr Gly
145                 150                 155                 160

Leu Lys Pro Lys Ile Val Met Tyr Ala Glu Asn Val Asp Leu Glu Thr
                165                 170                 175

Pro Asp Thr His Val Val Tyr Lys Pro Gly Ala Ser Asp Ala Ser Ser
            180                 185                 190

His Ala Asn Leu Gly Gln Gln Ser Met Pro Asn Arg Pro Asn Tyr Ile
        195                 200                 205

Gly Phe Arg Asp Asn Phe Ile Gly Leu Met Tyr Tyr Asn Ser Thr Gly
    210                 215                 220

Asn Met Gly Val Leu Ala Gly Gln Ala Ser Gln Leu Asn Ala Val Val
225                 230                 235                 240

Asp Leu Gln Asp Arg Asn Thr Glu Leu Ser Tyr Gln Leu Leu Leu Asp
                245                 250                 255

Ser Leu Gly Asp Arg Thr Arg Tyr Phe Ser Met Trp Asn Gln Ala Val
            260                 265                 270

Asp Ser Tyr Asp Pro Asp Val Arg Val Ile Glu Asn His Gly Val Glu
        275                 280                 285

Asp Glu Leu Pro Asn Tyr Cys Phe Pro Leu Asp Gly Val Gly Pro Arg
    290                 295                 300

Thr Asp Ser Tyr Lys Gly Ile Glu Thr Asn Gly Asp Glu Asn Thr Thr
305                 310                 315                 320

Trp Lys Asp Leu Asp Pro Asn Gly Ile Ser Glu Leu Ala Lys Gly Asn
                325                 330                 335

Pro Phe

<210>  14

<211>  315

<212>  PRT

<213>  chimpanzee adenovirus Pan-9

<400> 14

Ala Pro Lys Gly Ala Pro Asn Thr Cys Gln Trp Thr Tyr Lys Ala Asp
1               5                   10                  15

Gly Glu Thr Ala Thr Glu Lys Thr Tyr Thr Tyr Gly Asn Ala Pro Val
            20                  25                  30

Gln Gly Ile Asn Ile Thr Lys Asp Gly Ile Gln Leu Gly Thr Asp Thr
        35                  40                  45

Asp Asp Gln Pro Ile Tyr Ala Asp Lys Thr Tyr Gln Pro Glu Pro Gln
    50                  55                  60

Val Gly Asp Ala Glu Trp His Asp Ile Thr Gly Thr Asp Glu Lys Tyr
65                  70                  75                  80

Gly Gly Arg Ala Leu Lys Pro Asp Thr Lys Met Lys Pro Cys Tyr Gly
                85                  90                  95

Ser Phe Ala Lys Pro Thr Asn Lys Glu Gly Gly Gln Ala Asn Val Lys
            100                 105                 110

Thr Gly Thr Gly Thr Thr Lys Glu Tyr Asp Ile Asp Met Ala Phe Phe
        115                 120                 125

Asp Asn Arg Ser Ala Ala Ala Ala Gly Leu Ala Pro Glu Ile Val Leu
    130                 135                 140

Tyr Thr Glu Asn Val Asp Leu Glu Thr Pro Asp Thr His Ile Val Tyr
145                 150                 155                 160

Lys Ala Gly Thr Asp Asp Ser Ser Ser Ser Ile Asn Leu Gly Gln Gln
                165                 170                 175

Ala Met Pro Asn Arg Pro Asn Tyr Ile Gly Phe Arg Asp Asn Phe Ile
            180                 185                 190

Gly Leu Met Tyr Tyr Asn Ser Thr Gly Asn Met Gly Val Leu Ala Gly
        195                 200                 205

Gln Ala Ser Gln Leu Asn Ala Val Val Asp Leu Gln Asp Arg Asn Thr
    210                 215                 220

Glu Leu Ser Tyr Gln Leu Leu Leu Asp Ser Leu Gly Asp Arg Thr Arg
225                 230                 235                 240

Tyr Phe Ser Met Trp Asn Gln Ala Val Asp Ser Tyr Asp Pro Asp Val
                245                 250                 255

Arg Ile Ile Glu Asn His Gly Val Glu Asp Glu Leu Pro Asn Tyr Cys
            260                 265                 270

Phe Pro Leu Asp Ala Val Gly Arg Thr Asp Thr Tyr Gln Gly Ile Lys
        275                 280                 285

Ala Asn Gly Thr Asp Gln Thr Thr Trp Thr Lys Asp Asp Ser Val Asn
    290                 295                 300

Asp Ala Asn Glu Ile Gly Lys Gly Asn Pro Phe
305                 310                 315

<210>  15

<211>  315

<212>  PRT

<213>  chimpanzee adenovirus Pan-5


<400>  15

Ala Pro Lys Gly Ala Pro Asn Thr Cys Gln Trp Thr Tyr Lys Ala Asp
1               5                   10                  15

Gly Asp Thr Gly Thr Glu Lys Thr Tyr Thr Tyr Gly Asn Ala Pro Val
            20                  25                  30

Gln Gly Ile Ser Ile Thr Lys Asp Gly Ile Gln Leu Gly Thr Asp Thr
        35                  40                  45

Asp Asp Gln Pro Ile Tyr Ala Asp Lys Thr Tyr Gln Pro Glu Pro Gln
    50                  55                  60

Val Gly Asp Ala Glu Trp His Asp Ile Thr Gly Thr Asp Glu Lys Tyr
65                  70                  75                  80

Gly Gly Arg Ala Leu Lys Pro Asp Thr Lys Met Lys Pro Cys Tyr Gly
                85                  90                  95

Ser Phe Ala Lys Pro Thr Asn Lys Glu Gly Gly Gln Ala Asn Val Lys
            100                 105                 110

Thr Glu Thr Gly Gly Thr Lys Glu Tyr Asp Ile Asp Met Ala Phe Phe

        115                 120                 125

Asp Asn Arg Ser Ala Ala Ala Ala Gly Leu Ala Pro Glu Ile Val Leu
    130                 135                 140

Tyr Thr Glu Asn Val Asp Leu Glu Thr Pro Asp Thr His Ile Val Tyr
145                 150                 155                 160

Lys Ala Gly Thr Asp Asp Ser Ser Ser Ser Ile Asn Leu Gly Gln Gln
                165                 170                 175

Ser Met Pro Asn Arg Pro Asn Tyr Ile Gly Phe Arg Asp Asn Phe Ile
            180                 185                 190

Gly Leu Met Tyr Tyr Asn Ser Thr Gly Asn Met Gly Val Leu Ala Gly
        195                 200                 205

Gln Ala Ser Gln Leu Asn Ala Val Val Asp Leu Gln Asp Arg Asn Thr
    210                 215                 220

Glu Leu Ser Tyr Gln Leu Leu Leu Asp Ser Leu Gly Asp Arg Thr Arg
225                 230                 235                 240

Tyr Phe Ser Met Trp Asn Gln Ala Val Asp Ser Tyr Asp Pro Asp Val
                245                 250                 255

Arg Ile Ile Glu Asn His Gly Val Glu Asp Glu Leu Pro Asn Tyr Cys
            260                 265                 270

Phe Pro Leu Asp Ala Val Gly Arg Thr Asp Thr Tyr Gln Gly Ile Lys
        275                 280                 285

Ala Asn Gly Ala Asp Gln Thr Thr Trp Thr Lys Asp Asp Thr Val Asn
    290                 295                 300

Asp Ala Asn Glu Leu Gly Lys Gly Asn Pro Phe
305                 310                 315

<210> 16

<211> 324

<212> PRT

<213> chimpanzee adenovirus Pan-6

<400> 16

Ala Pro Lys Gly Ala Pro Asn Ser Ser Gln Trp Glu Gln Ala Lys Thr
1               5                   10                  15

Gly Asn Gly Gly Thr Met Glu Thr His Thr Tyr Gly Val Ala Pro Met
            20                  25                  30

Gly Gly Glu Asn Ile Thr Lys Asp Gly Leu Gln Ile Gly Thr Asp Val
        35                  40                  45

Thr Ala Asn Gln Asn Lys Pro Ile Tyr Ala Asp Lys Thr Phe Gln Pro
    50                  55                  60

Glu Pro Gln Val Gly Glu Glu Asn Trp Gln Glu Thr Glu Asn Phe Tyr
65                  70                  75                  80

Gly Gly Arg Ala Leu Lys Lys Asp Thr Lys Met Lys Pro Cys Tyr Gly
                85                  90                  95

Ser Tyr Ala Arg Pro Thr Asn Glu Lys Gly Gly Gln Ala Lys Leu Lys
            100                 105                 110

Val Gly Asp Asp Gly Val Pro Thr Lys Glu Phe Asp Ile Asp Leu Ala
        115                 120                 125

Phe Phe Asp Thr Pro Gly Gly Thr Val Asn Gly Gln Asp Glu Tyr Lys
    130                 135                 140

Ala Asp Ile Val Met Tyr Thr Glu Asn Thr Tyr Leu Glu Thr Pro Asp
145                 150                 155                 160

Thr His Val Val Tyr Lys Pro Gly Lys Asp Asp Ala Ser Ser Glu Ile
                165                 170                 175

Asn Leu Val Gln Gln Ser Met Pro Asn Arg Pro Asn Tyr Ile Gly Phe
            180                 185                 190

Arg Asp Asn Phe Ile Gly Leu Met Tyr Tyr Asn Ser Thr Gly Asn Met
        195                 200                 205

Gly Val Leu Ala Gly Gln Ala Ser Gln Leu Asn Ala Val Val Asp Leu
    210                 215                 220

Gln Asp Arg Asn Thr Glu Leu Ser Tyr Gln Leu Leu Leu Asp Ser Leu
225                 230                 235                 240

Gly Asp Arg Thr Arg Tyr Phe Ser Met Trp Asn Gln Ala Val Asp Ser
                245                 250                 255

Tyr Asp Pro Asp Val Arg Ile Ile Glu Asn His Gly Val Glu Asp Glu
            260                 265                 270

Leu Pro Asn Tyr Cys Phe Pro Leu Asp Gly Ser Gly Thr Asn Ala Ala
        275                 280                 285

Tyr Gln Gly Val Lys Val Lys Asp Gly Gln Asp Gly Asp Val Glu Ser
    290                 295                 300

Glu Trp Glu Asn Asp Asp Thr Val Ala Ala Arg Asn Gln Leu Cys Lys
305                 310                 315                 320

Gly Asn Ile Phe

<210> 17

<211> 314

<212> PRT

<213> chimpanzee adenovirus Pan-7

<400> 17

Ala Pro Lys Gly Ala Pro Asn Thr Cys Gln Trp Thr Tyr Lys Ala Gly
1               5                   10                  15

Asp Thr Asp Thr Glu Lys Thr Tyr Thr Tyr Gly Asn Ala Pro Val Gln
            20                  25                  30

Gly Ile Ser Ile Thr Lys Asp Gly Ile Gln Leu Gly Thr Asp Ser Asp
        35                  40                  45

Gly Gln Ala Ile Tyr Ala Asp Glu Thr Tyr Gln Pro Glu Pro Gln Val
    50                  55                  60

Gly Asp Ala Glu Trp His Asp Ile Thr Gly Thr Asp Glu Lys Tyr Gly
65                  70                  75                  80

Gly Arg Ala Leu Lys Pro Asp Thr Lys Met Lys Pro Cys Tyr Gly Ser
                85                  90                  95

Phe Ala Lys Pro Thr Asn Lys Glu Gly Gly Gln Ala Asn Val Lys Thr
            100                 105                 110

Glu Thr Gly Gly Thr Lys Glu Tyr Asp Ile Asp Met Ala Phe Phe Asp
        115                 120                 125

Asn Arg Ser Ala Ala Ala Ala Gly Leu Ala Pro Glu Ile Val Leu Tyr
    130                 135                 140

Thr Glu Asn Val Asp Leu Glu Thr Pro Asp Thr His Ile Val Tyr Lys
145                 150                 155                 160

Ala Gly Thr Asp Asp Ser Ser Ser Ser Ile Asn Leu Gly Gln Gln Ser
                165                 170                 175

Met Pro Asn Arg Pro Asn Tyr Ile Gly Phe Arg Asp Asn Phe Ile Gly
            180                 185                 190

Leu Met Tyr Tyr Asn Ser Thr Gly Asn Met Gly Val Leu Ala Gly Gln
        195                 200                 205

Ala Ser Gln Leu Asn Ala Val Val Asp Leu Gln Asp Arg Asn Thr Glu
    210                 215                 220

Leu Ser Tyr Gln Leu Leu Leu Asp Ser Leu Gly Asp Arg Thr Arg Tyr
225                 230                 235                 240

Phe Ser Met Trp Asn Gln Ala Val Asp Ser Tyr Asp Pro Asp Val Arg
                245                 250                 255

Ile Ile Glu Asn His Gly Val Glu Asp Glu Leu Pro Asn Tyr Cys Phe
            260                 265                 270

Pro Leu Asp Ala Val Gly Arg Thr Asp Thr Tyr Gln Gly Ile Lys Ala
        275                 280                 285

Asn Gly Asp Asn Gln Thr Thr Trp Thr Lys Asp Asp Thr Val Asn Asp
    290                 295                 300

Ala Asn Glu Leu Gly Lys Gly Asn Pro Phe
305                 310

<210>  18

<211>  179

<212>  PRT

<213>  chimpanzee adenovirus Pan9


<400>  18

Thr Leu Trp Thr Thr Pro Asp Pro Ser Pro Asn Cys Gln Ile Leu Ala

1                   5                   10                  15

Glu Asn Asp Ala Lys Leu Thr Leu Cys Leu Thr Lys Cys Gly Ser Gln
            20                  25                  30

Ile Leu Ala Thr Val Ser Val Leu Val Val Gly Ser Gly Asn Leu Asn
        35                  40                  45

Pro Ile Thr Gly Thr Val Ser Ser Ala Gln Val Phe Leu Arg Phe Asp
    50                  55                  60

Ala Asn Gly Val Leu Leu Thr Glu His Ser Thr Leu Lys Lys Tyr Trp
65                  70                  75                  80

Gly Tyr Arg Gln Gly Asp Ser Ile Asp Gly Thr Pro Tyr Thr Asn Ala
                85                  90                  95

Val Gly Phe Met Pro Asn Leu Lys Ala Tyr Pro Lys Ser Gln Ser Ser
            100                 105                 110

Thr Thr Lys Asn Asn Ile Val Gly Gln Val Tyr Met Asn Gly Asp Val
        115                 120                 125

Ser Lys Pro Met Leu Leu Thr Ile Thr Leu Asn Gly Thr Asp Asp Ser
    130                 135                 140

Asn Ser Thr Tyr Ser Met Ser Phe Ser Tyr Thr Trp Thr Asn Gly Ser
145                 150                 155                 160

Tyr Val Gly Ala Thr Phe Gly Ala Asn Ser Tyr Thr Phe Ser Tyr Ile
                165                 170                 175

Ala Gln Glu

<210>  19

<211>  185

<212>  PRT

<213>  chimpanzee adenovirus Pan6

<400>  19

Thr Leu Trp Thr Thr Pro Asp Pro Ser Pro Asn Cys Gln Leu Leu Ser
1               5                   10                  15

Asp Arg Asp Ala Lys Phe Thr Leu Cys Leu Thr Lys Cys Gly Ser Gln
            20                  25                  30

Ile Leu Gly Thr Val Ala Val Ala Ala Val Thr Val Gly Ser Ala Leu
        35                  40                  45

Asn Pro Ile Asn Asp Thr Val Lys Ser Ala Ile Val Phe Leu Arg Phe
    50                  55                  60

Asp Ser Asp Gly Val Leu Met Ser Asn Ser Ser Met Val Gly Asp Tyr
65                  70                  75                  80

Trp Asn Phe Arg Glu Gly Gln Thr Thr Gln Ser Val Ala Tyr Thr Asn
                85                  90                  95

Ala Val Gly Phe Met Pro Asn Ile Gly Ala Tyr Pro Lys Thr Gln Ser
            100                 105                 110

Lys Thr Pro Lys Asn Ser Ile Val Ser Gln Val Tyr Leu Thr Gly Glu
        115                 120                 125

Thr Thr Met Pro Met Thr Leu Thr Ile Thr Phe Asn Gly Thr Asp Glu
    130                 135                 140

Lys Asp Thr Thr Pro Val Ser Thr Tyr Ser Met Thr Phe Thr Trp Gln
145                 150                 155                 160

Trp Thr Gly Asp Tyr Lys Asp Lys Asn Ile Thr Phe Ala Thr Asn Ser
                165                 170                 175

Phe Ser Phe Ser Tyr Ile Ala Gln Glu
            180                 185

<210>  20

<211>  179

<212>  PRT

<213>  chimpanzee adenovirus Pan7


<400>  20

Thr Leu Trp Thr Thr Ala Asp Pro Ser Pro Asn Cys Lys Ile Tyr Ser
1               5                   10                  15

Glu Lys Asp Ala Lys Leu Thr Leu Cys Leu Thr Lys Cys Gly Ser Gln
            20                  25                  30

Ile Leu Gly Thr Val Thr Val Leu Ala Val Asn Asn Gly Ser Leu Asn
        35                  40                  45

Pro Ile Thr Asn Thr Val Ser Thr Ala Leu Val Ser Leu Lys Phe Asp
    50                  55                  60

Ala Ser Gly Val Leu Leu Ser Ser Ser Thr Leu Asp Lys Glu Tyr Trp
65                  70                  75                  80

Asn Phe Arg Lys Gly Asp Val Thr Pro Ala Glu Pro Tyr Thr Asn Ala
                85                  90                  95

Ile Gly Phe Met Pro Asn Ile Lys Ala Tyr Pro Lys Asn Thr Ser Ala
            100                 105                 110

Ala Ser Lys Ser His Ile Val Ser Gln Val Tyr Leu Asn Gly Asp Glu
        115                 120                 125

Ala Lys Pro Leu Met Leu Ile Ile Thr Phe Asn Glu Thr Glu Asp Ala
    130                 135                 140

Thr Cys Thr Tyr Ser Ile Thr Phe Gln Trp Lys Trp Asp Ser Thr Lys
145                 150                 155                 160

Tyr Thr Gly Glu Thr Leu Ala Thr Ser Ser Phe Thr Phe Ser Tyr Ile
                165                 170                 175

Ala Gln Glu

<210>  21

<211>  179

<212>  PRT

<213>  chimpanzee adenovirus Pan5

<400>  21

Thr Leu Trp Thr Thr Ala Asp Pro Ser Pro Asn Cys His Ile Tyr Ser
1               5                   10                  15

Glu Lys Asp Ala Lys Leu Thr Leu Cys Leu Thr Lys Cys Gly Ser Gln
            20                  25                  30

Ile Leu Gly Thr Val Ser Leu Ile Ala Val Asp Thr Gly Ser Leu Asn
        35                  40                  45

Pro Ile Thr Gly Thr Val Thr Thr Ala Leu Val Ser Leu Lys Phe Asp
    50                  55                  60

Ala Asn Gly Val Leu Gln Ser Ser Ser Thr Leu Asp Ser Asp Tyr Trp
65                  70                  75                  80

Asn Phe Arg Gln Gly Asp Val Thr Pro Ala Glu Ala Tyr Thr Asn Ala
                85                  90                  95

Ile Gly Phe Met Pro Asn Leu Lys Ala Tyr Pro Lys Asn Thr Ser Gly
            100                 105                 110

Ala Ala Lys Ser His Ile Val Gly Lys Val Tyr Leu His Gly Asp Thr
        115                 120                 125

Gly Lys Pro Leu Asp Leu Ile Ile Thr Phe Asn Glu Thr Ser Asp Glu
    130                 135                 140

Ser Cys Thr Tyr Cys Ile Asn Phe Gln Trp Gln Trp Gly Ala Asp Gln
145                 150                 155                 160

Tyr Lys Asn Glu Thr Leu Ala Val Ser Ser Phe Thr Phe Ser Tyr Ile
                165                 170                 175

Ala Lys Glu

<210>  22

<211>  183

<212>  PRT

<213>  human adenovirus Ad 2

<400>  22

Thr Leu Trp Thr Thr Pro Asp Pro Ser Pro Asn Cys Arg Ile His Ser
1               5                   10                  15

Asp Asn Asp Cys Lys Phe Thr Leu Val Leu Thr Lys Cys Gly Ser Gln
            20                  25                  30

Val Leu Ala Thr Val Ala Ala Leu Ala Val Ser Gly Asp Leu Ser Ser
        35                  40                  45

Met Thr Gly Thr Val Ala Ser Val Ser Ile Phe Leu Arg Phe Asp Gln

50                  55                  60

Asn Gly Val Leu Met Glu Asn Ser Ser Leu Lys Lys His Tyr Trp Asn
65                  70                  75                  80

Phe Arg Asn Gly Asn Ser Thr Asn Ala Asn Pro Tyr Thr Asn Ala Val
                85                  90                  95

Gly Phe Met Pro Asn Leu Leu Ala Tyr Pro Lys Thr Gln Ser Gln Thr
            100                 105                 110

Ala Lys Asn Asn Ile Val Ser Gln Val Tyr Leu His Gly Asp Lys Thr
        115                 120                 125

Lys Pro Met Ile Leu Thr Ile Thr Leu Asn Gly Thr Ser Glu Ser Thr
    130                 135                 140

Glu Thr Ser Glu Val Ser Thr Tyr Ser Met Ser Phe Thr Trp Ser Trp
145                 150                 155                 160

Glu Ser Gly Lys Tyr Thr Thr Glu Thr Phe Ala Thr Asn Ser Tyr Thr
                165                 170                 175

Phe Ser Tyr Ile Ala Gln Glu
            180

<210>  23

<211>  182

<212>  PRT

<213>  human adenovirus Ad 5


<400>  23

Thr Leu Trp Thr Thr Pro Ala Pro Ser Pro Asn Cys Arg Leu Asn Ala
1               5                   10                  15

Glu Lys Asp Ala Lys Leu Thr Leu Val Leu Thr Lys Cys Gly Ser Gln
            20                  25                  30

Ile Leu Ala Thr Val Ser Val Leu Ala Val Lys Gly Ser Leu Ala Pro
        35                  40                  45

Ile Ser Gly Thr Val Gln Ser Ala His Leu Ile Ile Arg Phe Asp Glu
    50                  55                  60

Asn Gly Val Leu Ile Asn Asn Ser Phe Leu Asp Pro Glu Tyr Trp Asn
65                  70                  75                  80

Phe Arg Asn Gly Asp Leu Thr Glu Gly Thr Ala Tyr Thr Asn Ala Val
                85                  90                  95

Gly Phe Met Pro Asn Leu Ser Ala Tyr Pro Lys Ser His Gly Lys Thr
            100                 105                 110

Ala Lys Ser Asn Ile Val Ser Gln Val Tyr Leu Asn Gly Asp Lys Thr
        115                 120                 125

Lys Pro Val Thr Leu Thr Ile Thr Leu Asn Gly Thr Gln Glu Thr Gly
    130                 135                 140

Asp Thr Thr Pro Ser Ala Tyr Ser Met Ser Phe Ser Trp Asp Trp Ser
145                 150                 155                 160

Gly His Asn Tyr Ile Asn Glu Ile Phe Ala Thr Ser Ser Tyr Thr Glu
                165                 170                 175

Ser Tyr Ile Ala Gln Glu
            180

<210>  24

<211>  34264

<212>  DNA

<213>  simian adenovirus SV-1


<220>

<221>  CDS

<222>  (12454)..(13965)

<223>  L2 Penton


<220>

<221>  CDS

<222>  (16841)..(19636)

<223>  L3 Hexon


<220>

<221>  CDS
<222>  (28059)..(29150)
<223>  L5 Fiber #2

<220>
<221>  CDS
<222>  (29183)..(30865)
<223>  L5 Fiber #1

<400>  24
   tccttattct ggaaacgtgc caatatgata atgagcgggg aggagcgagg cggggccggg      60
   gtgacgtgcg gtgacgtggg gtgacgcggg gtggcgcgag ggcggggcgg gagtggggag     120
   gcgcttagtt tttacgtatg cggaaggagg ttttataccg gaagttgggt aatttgggcg     180
   tatacttgta agttttgtgt aatttggcgc gaaaaccggg taatgaggaa gttgaggtta     240
   atatgtactt tttatgactg ggcggaattt ctgctgatca gcagtgaact ttgggcgctg     300
   acggggaggt ttcgctacgt ggcagtacca cgagaaggct caaaggtccc atttattgta     360
   ctcctcagcg ttttcgctgg gtatttaaac gctgtcagat catcaagagg ccactcttga     420
   gtgccggcga gtagagtttt ctcctccgcg ctgccgcgat gaggctggtt cccgagatgt     480
   acggtgtttt ctgcagcgag acggcccgga actcagatga gctgcttaat acagatctgc     540
   tggatgttcc caactcgcct gtggcttcgc ctccgtcgct tcatgatctt ttcgatgtgg     600
   aagtggatcc accgcaagat cccaacgagg acgcggtaaa cagtatgttc cctgaatgtc     660
   tgtttgaggc ggctgaggag ggttctcaca gcagtgaaga gagcagacgg ggagaggaac     720
   tggacttgaa atgctacgag gaatgtctgc cttctagcga ttctgaaacg gaacagacag     780
   ggggagacgg ctgtgagtcg gcaatgaaaa atgaacttgt attagactgt ccagaacatc     840
   ctggtcatgg ctgccgtgcc tgtgcttttc atagaaatgc cagcggaaat cctgagactc     900
   tatgtgctct gtgttatctg cgccttacca gcgattttgt atacagtaag taaagtgttt     960
   tcattggcgt acggtagggg attcgttgaa gtgctttgtg acttattatg tgtcattatt    1020
   tctaggtgac gtgtccgacg tggaagggga aggagataga tcaggggctg ctaattctcc    1080
   ttgcactttg ggggctgtgg ttccagttgg catttttaaa ccgagtggtg gaggagaacg    1140
   agccggagga gaccgagaat ctgagagccg gcctggaccc tccagtggaa gactaggtgc    1200
   tgaggatgat cctgaagagg ggactagtgg gggtgctagg aaaaagcaaa aaactgagcc    1260
   tgaacctaga aactttttga atgagttgac tgtaagccta atgaatcggc agcgtcctga    1320
   gacggtgttt tggactgagt tggaggatga gttcaagaag ggggaattaa acctcttgta 1380
   caagtatggg tttgagcagt tgaaaactca ctggttggag ccgtgggagg atatggaaat 1440
   ggctctagac acctttgcta aagtggctct gcggccggat aaagtttaca ctattcgccg 1500
   cactgttaat ataaaaaaga gtgtttatgt tatcggccat ggagctctgg tgcaggtgca 1560
   gaccccagac cgggtggctt tcaattgcgg catgcagagt ttgggccccg gggtgatagg 1620
   tttgaatgga gttacatttc aaaatgtcag gtttactggt gatgatttta atggctctgt 1680
   gtttgtgact agcacccagc taaccctcca cggtgtttac ttttttaact ttaacaatac 1740
   atgtgtggag tcatggggta gggtgtctct gaggggctgc agttttcatg gttgctggaa 1800
   ggcggtggtg ggaagaatta aaagtgtcat gtctgtgaag aaatgcatat ttgaacgctg 1860
   tgtgatagct ctagcagtag aggggtacgg acggatcagg aataacgccg catctgagaa 1920
   tggatgtttt cttttgctga aaggtacggc cagcgttaag cataatatga tttgcggcag 1980
   cggcctgtgc ccctcgcagc tcttaacttg cgcagatgga aactgtcaca ccttgcgcac 2040
   cgtgcacata gtgtcccact cgcgccgcac ctggccaaca tttgagcaca atatgctcat 2100
   gcgttgcgcc gttcacctag gtgctagacg cggcgtgttt atgccttatc aatgtaactt 2160
   tagtcatact aagattttgc tggaaactga ttccttccct cgagtatgtt tcaatggggt 2220
   gtttgacatg tcaatggaac tttttaaagt gataagatat gatgaaacca agtctcgttg 2280
   tcgctcatgt gaatgcggag ctaatcattt gaggttgtat cctgtaaccc tgaacgttac 2340
   cgaggagctg aggacggacc accacatgct gtcttgcctg cgtaccgact atgaatccag 2400
   cgatgaggag tgaggtgagg ggcggagcca caaagggtat aaaggggcat gaggggtggg 2460
   cgcggtgttt caaaatgagc gggacgacgg acggcaatgc gtttgagggg ggagtgttca 2520
   gcccatatct gacatctcgt cttccttcct gggcaggagt tcgtcagaat gtagtgggct 2580
   ccaccgtgga cggacggccg gtcgcccctg caaattccgc caccctcacc tatgccaccg 2640
   tgggatcatc gttggacact gccgcggcag ctgccgcttc tgctgccgct tctactgctc 2700
   gcggcatggc ggctgatttt ggactatata accaactggc cactgcagct gtggcgtctc 2760
   ggtctctggt tcaagaagat gccctgaatg tgatcttgac tcgcctggag atcatgtcac 2820
   gtcgcctgga cgaactggct gcgcagatat cccaagctaa ccccgatacc gcttcagaat 2880
   cttaaaataa agacaaacaa atttgttgaa aagtaaaatg gctttatttg ttttttttgg 2940
   ctcggtaggc tcgggtccac ctgtctcggt cgttaaggac tttgtgtatg ttttccaaaa 3000
   cacggtacag atgggcttgg atgttcaagt acatgggcat gaggccatct ttggggtgga 3060
   gataggacca ctgaagagcg tcatgttccg gggtggtatt gtaaatcacc cagtcgtagc 3120
   agggtttttg agcgtggaac tggaatatgt ccttcaggag caggctaatg gccaagggta 3180
   gacccttagt gtaggtgttt acaaagcggt tgagctggga gggatgcatg cggggggaga 3240
   tgatatgcat cttggcttgg attttgaggt tagctatgtt accacccagg tctctgcggg 3300
   ggttcatgtt atgaaggacc accagcacgg tatagccagt gcatttgggg aacttgtcat 3360
   gcagtttgga ggggaaggcg tggaagaatt tagatacccc cttgtgcccc cctaggtttt 3420
   ccatgcactc atccataata atggcaatgg gacccctggc ggccgcttta gcaaacacgt 3480
   tttgggggtt ggaaacatca tagttttgct ctagagtgag ctcatcatag gccatcttta 3540
   caaagcgggg taggagggtg cccgactggg ggatgatagt tccatctggg cctggagcgt 3600
   agttgccctc acagatctgc atctcccagg ccttaatttc cgaggggggg atcatgtcca 3660
   cctggggggc gataaaaaac acggtttctg gcggggggtt aatgagctgg gtggaaagca 3720
   agttacgcaa cagctgggat ttgccgcaac cggtgggacc gtagatgacc ccgatgacgg 3780
   gttgcagctg gtagttcaga gaggaacagc tgccgtcggg gcgcaggagg ggagctacct 3840
   cattcatcat gcttctgaca tgtttatttt cactcactaa gttttgcaag agcctctccc 3900
   cacccaggga taagagttct tccaggctgt tgaagtgttt cagcggtttc aggccgtcgg 3960
   ccatgggcat cttttcaagc gactgacgaa gcaagtacag tcggtcccag agctcggtga 4020
   cgtgctctat ggaatctcga tccagcagac ttcttggttt cgggggttgg gccgactttc 4080
   gctgtagggc accagccggt gggcgtccag ggccgcgagg gttctgtcct tccagggtct 4140
   cagcgttcgg gtgagggtgg tctcggtgac ggtgaaggga tgagccccgg gctgggcgct 4200
   tgcgagggtg cgcttcaggc tcatcctgct ggtgctgaag cgggcgtcgt ctccctgtga 4260
   gtcggccaga tagcaacgaa gcatgaggtc gtagctgagg gactcggccg cgtgtccctt 4320
   ggcgcgcagc tttcccttgg aaacgtgctg acatttggtg cagtgcagac acttgagggc 4380
   gtagagtttt ggggccagga agaccgactc gggcgagtag gcgtcggctc cgcactgagc 4440
   gcagacggtc tcgcactcca ccagccacgt gagctcgggt ttagcgggat caaaaaccaa 4500
   gttgcctcca ttttttttga tgcgtttctt accttgcgtc tccatgagtc tgtgtcccgc 4560
   ttccgtgaca aaaaggctgt cggtatcccc gtagaccgac ttgagggggc gatcttccaa 4620
   aggtgttccg aggtcttccg cgtacaggaa ctgggaccac tccgagacaa aggctcgggt 4680
   ccaggctaac acgaaggagg cgatctgcga ggggtatctg tcgttttcaa tgagggggtc 4740
   caccttttcc agggtgtgca gacacaggtc gtcctcctcc gcgtccacga aggtgattgg 4800
   cttgtaagtg taggtcacgt gacccgcacc cccccaaggg gtataaaagg gggcgtgccc 4860
   actctccccg tcactttctt ccgcatcgct gtggaccaga gccagctgtt cgggtgagta 4920
   ggccctctca aaagccggca tgatttcggc gctcaagttg tcagtttcta caaacgaggt 4980
   ggatttgata ttcacgtgcc ccgcggcgat gcttttgatg gtggaggggt ccatctgatc 5040
   agaaaacacg atctttttat tgtcaagttt ggtggcgaaa gacccgtaga gggcgttgga 5100
   aagcaacttg gcgatggagc gcagggtctg atttttctcc cgatcggccc tctccttggc 5160
   ggcgatgttg agttgcacgt actcgcgggc cacgcaccgc cactcgggga acacggcggt 5220
   gcgctcgtcg ggcaggatgc gcacgcgcca gccgcggttg tgcagggtga tgaggtccac 5280
   gctggtggcc acctccccgc ggaggggctc gttggtccaa cacaatcgcc ccccttttct 5340
   ggagcagaac ggaggcaggg gatctagcaa gttggcgggc ggggggtcgg cgtcgatggt 5400
   aaatatgccg ggtagcagaa ttttattaaa ataatcgatt tcggtgtccg tgtcttgcaa 5460
   cgcgtcttcc cacttcttca ccgccagggc cctttcgtag ggattcaggg gcggtcccca 5520
   gggcatgggg tgggtcaggg ccgaggcgta catgccgcag atgtcgtaca cgtacagggg 5580
   ctccctcaac accccgatgt aagtggggta acagcgcccc ccgcggatgc tggctcgcac 5640
   gtagtcgtac atctcgtgag agggagccat gagcccgtct cccaagtggg tcttgtgggg 5700
   tttttcggcc cggtagagga tctgcctgaa gatggcgtgg gagttggaag agatagtggg 5760
   gcgttggaag acgttaaagt tggctccggg cagtcccacg gagtcttgga tgaactgggc 5820
   gtaggattcc cggagcttgt ccaccagggc tgcggttacc agcacgtcga gagcgcagta 5880
   gtccaacgtc tcgcggacca ggttgtaggc cgtctcttgt tttttctccc acagttcgcg 5940
   attgaggagg tattcctcgc ggtctttcca gtactcttcg gcgggaaatc ctttttcgtc 6000
   cgctcggtaa gaacctaaca tgtaaaattc gttcacggct ttgtatggac aacagccttt 6060
   ttctaccggc agggcgtacg cttgagcggc ctttctgaga gaggtgtggg tgagggcgaa 6120
   ggtgtcccgc accatcactt tcaggtactg atgtttgaag tccgtgtcgt cgcaggcgcc 6180
   ctgttcccac agcgtgaagt cggtgcgctt tttctgcctg ggattgggga gggcgaatgt 6240
   gacgtcgtta aagaggattt tcccggcgcg gggcatgaag ttgcgagaga tcctgaaggg 6300
   tccgggcacg tccgagcggt tgttgatgac ttgcgccgcc aggacgatct cgtcgaagcc 6360
   gttgatgttg tggcccacga tgtaaagttc gataaagcgc ggctgtccct tgagggccgg 6420
   cgcttttttc aactcctcgt aggtgagaca gtccggcgag gagagaccca gctccgcccg 6480
   ggcccagtcg gagagctgag ggttagccgc gaggaaagag ctccacaggt caagggctag 6540
   cagagtttgc aagcggtcgc ggaactcgcg aaactttttc cccacggcca ttttctccgg 6600
   cgtcaccacg tagaaagtgc aggggcggtc gttccagacg tcccatcgga gctctagggc 6660
   cagctcgcag gcttgacgaa cgagggtctc ctcgcccgag acgtgcatga ccagcatgaa 6720
   gggtaccaac tgtttcccga acgagcccat ccatgtgtag gtttctacgt cgtaggtgac 6780
   aaagagccgc tgggtgcgcg cgtgggagcc gatcgggaag aagctgatct cctgccacca 6840
   gttggaggaa tgggtgttga tgtggtgaaa gtagaagtcc cgccggcgca cagagcattc 6900
   gtgctgatgt ttgtaaaagc gaccgcagta gtcgcagcgc tgcacgctct gtatctcctg 6960
   aatgagatgc gcttttcgcc cgcgcaccag aaaccggagg gggaagttga gacgggggct 7020
   tggtggggcg gcatcccctt cgccttggcg gtgggagtct gcgtctgcgc cctccttctc 7080
   tgggtggacg acggtgggga cgacgacgcc ccgggtgccg caagtccaga tctccgccac 7140
   ggaggggcgc aggcgttgca ggaggggacg cagctgcccg ctgtccaggg agtcgagggc 7200
   ggccgcgctg aggtcggcgg gaagcgtttg caagttcact ttcagaagac cggtaagagc 7260
   gtgagccagg tgcacatggt acttgatttc caggggggtg ttggaagagg cgtccacggc 7320
   gtagaggagg ccgtgtccgc gcggggccac caccgtgccc cgaggaggtt ttatctcact 7380
   cgtcgagggc gagcgccggg gggtagaggc ggctctgcgc cggggggcag cggaggcagt 7440
   ggcacgtttt cgtgaggatt cggcagcggt tgatgacgag cccggagact gctggcgtgg 7500
   gcgacgacgc ggcggttgag gtcctggatg tgccgtctct gcgtgaagac caccggcccc 7560
   cgggtcctga acctgaaaga gagttccaca gaatcaatgt ctgcatcgtt aacggcggcc 7620
   tgcctgagga tctcctgtac gtcgcccgag ttgtcttgat aggcgatctc ggccatgaac 7680
   tgctccactt cttcctcgcg gaggtcgccg tggcccgctc gctccacggt ggcggccagg 7740
   tcgttggaga tgcgacgcat gagttgagag aaggcgttga ggccgttctc gttccacacg 7800
   cggctgtaca ccacgtttcc gaaggagtcg cgcgctcgca tgaccacctg ggccacgttg 7860
   agttccacgt ggcgggcgaa gacggcgtag tttctgaggc gctggaagag gtagttgagc 7920
   gtggtggcga tgtgctcgca gacgaagaag tacatgatcc agcgccgcag ggtcatctcg 7980
   ttgatgtctc cgatggcttc gagacgctcc atggcctcgt agaagtcgac ggcgaagttg 8040
   aaaaattggg agttgcgggc ggccaccgtg agttcttctt gcaggaggcg gatgagatcg 8100
   gcgaccgtgt cgcgcacctc ctgctcgaaa gcgccccgag gcgcctctgc ttcttcctcc 8160
   ggctcctcct cttccagggg cacgggttcc tccggcagct ctgcgacggg gacggggcgg 8220
   cgacgtcgtc gtctgaccgg caggcggtcc acgaagcgct cgatcatttc gccgcgccgg 8280
   cgacgcatgg tctcggtgac ggcgcgtccg ttttcgcgag gtcgcagttc gaagacgccg 8340
   ccgcgcagag cgcccccgtg cagggagggt aagtggttag ggccgtcggg cagggacacg 8400
   gcgctgacga tgcattttat caattgctgc gtaggcactc cgtgcaggga tctgagaacg 8460
   tcgaggtcga cgggatccga gaacttctct aggaaagcgt ctatccaatc gcagtcgcaa 8520
   ggtaagctga ggacggtggg ccgctggggg gcgtccgcgg gcagttggga ggtgatgctg 8580
   ctgatgatgt aattaaagta ggcggtcttc aggcggcgga tggtggcgag gaggaccacg 8640
   tctttgggcc cggcctgttg aatgcgcagg cgctcggcca tgccccaggc ctcgctctga 8700
   cagcgacgca ggtctttgta gtagtcttgc atcagtctct ccaccggaac ctctgcttct 8760
   cccctgtctg ccatgcgagt cgagccgaac cccgcagggg gctgcagcaa cgctaggtcg 8820
   gccacgaccc tctcggccag cacggcctgt tggatctgcg tgagggtggt ctggaagtcg 8880
   tccaggtcca cgaagcggtg ataggccccc gtgttgatgg tgtaggtgca gttggccatg 8940
   acggaccagt tgacgacttg catgccgggt tgggtgatct ccgtgtactt gaggcgcgag 9000
   taggcgcggg actcgaacac gtagtcgttg catgtgcgta ccagatactg gtagccaacc 9060
   aggaagtggg gaggcggttc tcggtacagg ggccagccga ctgtggcggg ggcgccgggg 9120
   gacaggtcgt ccagcatgag gcgatggtag tggtagatgt agcgggagag ccaggtgatg 9180
   ccggccgagg tggtcgcggc cctggtgaat tcgcggacgc ggttccagat gttgcgcagg 9240
   gggcgaaagc gctccatggt gggcacgctc tgccccgtga ggcgggcgca atcttgtacg 9300
   ctctagatgg aaaaaagaca gggcggtcat cgactccctt ccgtagctcg gggggtaaag 9360
   tcgcaagggt gcggcggcgg ggaaccccgg ttcgagaccg gccggatccg ccgctcccga 9420
   tgcgcctggc cccgcatcca cgacgtccgc gtcgagaccc agccgcgacg ctccgcccca 9480
   atacggaggg gagtcttttg gtgtttttcg tagatgcatc cggtgctgcg gcagatgcga 9540
   acctcagacg cccaccacca ccgccgcggc ggcagtaaac ctgagcggag gcggtgacag 9600
   ggaggaggag gagctggctt tagacctgga agagggagag gggctggccc ggctgggagc 9660
   gccgtcccca gagagacacc ctagggttca gctcgtgagg gacgccaggc aggcttttgt 9720
   gccgaagcag aacctgttta gggaccgcag cggtcaggag gcggaggaga tgcgcgattg 9780
   caggtttcgg gcgggtagag agctgagggc gggcttcgat cgggagcggc tcctgagggc 9840
   ggaggatttc gagcccgacg agcgttctgg ggtgagcccg gcccgcgctc acgtctcggc 9900
   ggccaacctg gtgagcgcgt acgagcagac ggtgaacgag gagcgcaact tccaaaagag 9960
   ctttaacaat cacgtgagga ccctgatcgc gagggaggag gtgaccatcg ggctgatgca 10020
   tctgtgggac ttcgtggagg cctacgtgca gaacccggcc agcaaacctc tgacggccca 10080
   gctgttcctg atcgtgcagc acagccgcga caacgagacg ttccgcgacg ccatgttgaa 10140
   catcgcggag cccgagggtc gctggctctt ggatctgatt aacatcctgc agagcatcgt 10200
   ggtgcaggag aggggcctca gcttagcgga caaggtggcg gccattaact attcgatgca 10260
   gagcctgggg aagttctacg ctcgcaagat ctacaagagc ccttacgtgc ccatagacaa 10320
   ggaggtgaag atagacagct tttacatgcg catggcgctg aaggtgctga cgctgagcga 10380
   cgacctcggc gtgtaccgta acgacaagat ccacaaggcg gtgagcgcca gccgccggcg 10440
   ggagctgagc gacagggagc tgatgcacag cctgcagagg gcgctggcgg gcgccgggga 10500
   cgaggagcgc gaggcttact tcgacatggg agccgatctg cagtggcgtc ccagcgcgcg 10560
   cgccttggag gcggcgggct accccgacga ggaggatcgg gacgatttgg aggaggcagg 10620
   cgagtacgag gacgaagcct gaccgggcag gtgttgtttt agatgcagcg gccggcggac 10680
   ggggccaccg cggatcccgc acttttggca tccatgcaga gtcaaccttc gggcgtgacc 10740
   gcctccgatg actgggcggc ggccatggac cgcattatgg cgctgactac ccgcaacccc 10800
   gaggctttta gacagcaacc ccaggccaac cgtttttcgg ccatcttgga agcggtggtg 10860
   ccctcccgca ccaaccccac acacgagaaa gtcctgacta tcgtgaacgc cctggtagac 10920
   agcaaggcca tccgccgcga cgaggcgggc ttgatttaca acgctctgct ggaacgggtg 10980
   gcgcgctaca acagcactaa cgttcagacc aatctggatc gcctcaccac cgacgtgaag 11040
   gaggcgctgg ctcagaagga gcggtttctg agggacagca atctgggctc tctggtggca 11100
   ctcaacgcct tcctgagcac gcagccggcc aacgtgcccc gcgggcagga ggactacgtg 11160
   agcttcatca gcgctctgag gctgctggtg tccgaggtgc cccagagcga ggtgtatcag 11220
   tctgggccgg attacttctt ccagacgtcc cgacagggct tgcaaacggt gaacctgact 11280
   caggccttta aaaacttgca aggcatgtgg ggcgttaagg ccccggtggg cgatcgagcc 11340
   accatctcca gtctgctgac ccccaacact cgcctgctgc tgctcttgat cgcgccgttc 11400
   accaacagta gcactatcag ccgtgactcg tacctgggtc atctcatcac tttgtaccgc 11460
   gaggccatcg gtcaggctca gatcgacgag cacacatatc aggagatcac taacgtgagc 11520
   cgggccctgg gtcaggaaga taccggcagc ctggaagcca cgttgaactt tttgctaacc 11580
   aaccggaggc aaaaaatacc ctcccagttt acgttaagcg ccgaggagga gaggattctg 11640
   cgatacgtgc agcagtccgt gagtctgtac ttgatgcggg agggcgccac cgcttccacg 11700
   gctttagaca tgacggctcg gaacatggaa ccgtcctttt actccgccca ccggccgttc 11760
   attaaccgtc tgatggacta cttccatcgc gcggccgcca tgaacgggga gtacttcacc 11820
   aatgccatcc tgaatccgca ttggatgccc ccgtccggct tctacaccgg cgagtttgac 11880
   ctgcccgaag ccgacgacgg ctttctttgg gacgacgtgt ccgacagcat tttcacgccg 11940
   ggcaatcgcc gattccagaa gaaggagggc ggagacgagc tccccctctc cagcgtggag 12000
   gcggcctcta ggggagagag tccctttccc agtctgtctt ccgccagcag tggtcgggta 12060
   acgcgcccgc ggttgccggg ggagagcgac tacctgaacg accccttgct gcggccggct 12120
   aggaagaaaa atttccccaa caacggggtg gaaagcttgg tggataaaat gaatcgttgg 12180
   aagacctacg cccaggagca gcgggagtgg gaggacagtc agccgcgacc gctggttccg 12240
   ccgcactggc gtcgtcagag agaagacccg gacgactccg cagacgatag tagcgtgttg 12300
   gacctgggag ggagcggagc caaccccttt gctcacttgc aacccaaggg gcgttccagt 12360
   cgcctctact aataaaaaag acgcggaaac ttaccagagc catggccaca gcgtgtgtcc 12420
   tttcttcctc tctttcttcc tcggcgcggc aga atg aga aga gcg gtg aga gtc 12474
   Met Arg Arg Ala Val Arg Val
   1 5
   acg ccg gcg gcg tat gag ggt ccg ccc cct tct tac gaa agc gtg atg 12522
   Thr Pro Ala Ala Tyr Glu Gly Pro Pro Pro Ser Tyr Glu Ser Val Met
   10 15 20
   gga tca gcg aac gtg ccg gcc acg ctg gag gcg cct tac gtt cct ccc     12570
   Gly Ser Ala Asn Val Pro Ala Thr Leu Glu Ala Pro Tyr Val Pro Pro
       25                  30                  35
   aga tac ctg gga cct acg gag ggc aga aac agc atc cgt tac tcc gag     12618
   Arg Tyr Leu Gly Pro Thr Glu Gly Arg Asn Ser Ile Arg Tyr Ser Glu
   40                  45                  50                  55
   ctg gca ccc ctg tac gat acc acc aag gtg tac ctg gtg gac aac aag     12666
   Leu Ala Pro Leu Tyr Asp Thr Thr Lys Val Tyr Leu Val Asp Asn Lys
                   60                  65                  70
   tcg gcg gac atc gcc tcc ctg aat tat caa aac gat cac agc aat ttt     12714
   Ser Ala Asp Ile Ala Ser Leu Asn Tyr Gln Asn Asp His Ser Asn Phe
               75                  80                  85
   ctg act acc gtg gtg cag aac aat gac ttc acc ccg acg gag gcg ggc     12762
   Leu Thr Thr Val Val Gln Asn Asn Asp Phe Thr Pro Thr Glu Ala Gly
           90                  95                  100
   acg cag acc att aac ttt gac gag cgt tcc cgc tgg ggc ggt cag ctg     12810
   Thr Gln Thr Ile Asn Phe Asp Glu Arg Ser Arg Trp Gly Gly Gln Leu
       105                 110                 115
   aaa acc atc ctg cac acc aac atg ccc aac atc aac gag ttc atg tcc     12858
   Lys Thr Ile Leu His Thr Asn Met Pro Asn Ile Asn Glu Phe Met Ser
   120                 125                 130                 135
   acc aac aag ttc agg gcc agg ctg atg gtt aaa aag gct gaa aac cag     12906
   Thr Asn Lys Phe Arg Ala Arg Leu Met Val Lys Lys Ala Glu Asn Gln
                   140                 145                 150
   cct ccc gag tac gaa tgg ttt gag ttc acc att ccc gag ggc aac tat     12954
   Pro Pro Glu Tyr Glu Trp Phe Glu Phe Thr Ile Pro Glu Gly Asn Tyr
               155                 160                 165
   tcc gag acc atg act atc gat ctg atg aac aat gcg atc gtg gac aat     13002
   Ser Glu Thr Met Thr Ile Asp Leu Met Asn Asn Ala Ile Val Asp Asn
           170                 175                 180
   tac ctg caa gtg ggg agg cag aac ggg gta ttg gaa agc gat atc ggc     13050
   Tyr Leu Gln Val Gly Arg Gln Asn Gly Val Leu Glu Ser Asp Ile Gly
       185                 190                 195
   gta aaa ttt gat acc aga aac ttc cga ctg ggg tgg gat ccc gtg acc     13098
   Val Lys Phe Asp Thr Arg Asn Phe Arg Leu Gly Trp Asp Pro Val Thr
   200                 205                 210                 215
   aag ctg gtg atg cca ggc gtg tac acc aac gag gct ttt cac ccc gac     13146
   Lys Leu Val Met Pro Gly Val Tyr Thr Asn Glu Ala Phe His Pro Asp
                   220                 225                 230
   atc gtg ctg ctg ccg ggg tgc ggt gtg gac ttc act cag agc cgt ttg     13194
   Ile Val Leu Leu Pro Gly Cys Gly Val Asp Phe Thr Gln Ser Arg Leu
               235                 240                 245
   agt aac ctg tta ggg atc aga aag cgc cgc ccc ttc caa gag ggc ttt     13242
   Ser Asn Leu Leu Gly Ile Arg Lys Arg Arg Pro Phe Gln Glu Gly Phe
           250                 255                 260
   cag atc atg tat gag gac ctg gaa gga ggt aac att cca ggt ttg cta     13290
   Gln Ile Met Tyr Glu Asp Leu Glu Gly Gly Asn Ile Pro Gly Leu Leu
   265                 270                 275
   gac gtg ccg gcg tat gaa gag agt gtt aaa cag gcg gag gcg cag gga     13338
   Asp Val Pro Ala Tyr Glu Glu Ser Val Lys Gln Ala Glu Ala Gln Gly
   280                 285                 290                 295
   cga gag att cga ggc gac acc ttt gcc acg gaa cct cac gaa ctg gta     13386
   Arg Glu Ile Arg Gly Asp Thr Phe Ala Thr Glu Pro His Glu Leu Val
                   300                 305                 310
   ata aaa cct ctg gaa caa gac agt aaa aaa cgg agt tac aac att ata     13434
   Ile Lys Pro Leu Glu Gln Asp Ser Lys Lys Arg Ser Tyr Asn Ile Ile
               315                 320                 325
   tcc ggc act atg aat acc ttg tac cgg agc tgg ttt ctg gct tac aac     13482
   Ser Gly Thr Met Asn Thr Leu Tyr Arg Ser Trp Phe Leu Ala Tyr Asn
           330                 335                 340
   tac ggg gat ccc gaa aag gga gtg aga tca tgg acc ata ctc acc acc     13530
   Tyr Gly Asp Pro Glu Lys Gly Val Arg Ser Trp Thr Ile Leu Thr Thr
       345                 350                 355
   acg gac gtg acc tgc ggc tcg cag caa gtg tac tgg tcc ctg ccg gat     13578
   Thr Asp Val Thr Cys Gly Ser Gln Gln Val Tyr Trp Ser Leu Pro Asp
   360                 365                 370                 375
   atg atg caa gac ccg gtc acc ttc cgc ccc tcc acc caa gtc agc aac     13626
   Met Met Gln Asp Pro Val Thr Phe Arg Pro Ser Thr Gln Val Ser Asn
                   380                 385                 390
   ttc ccg gtg gtg ggc acc gag ctg ctg ccc gtc cat gcc aag agc ttc     13674
   Phe Pro Val Val Gly Thr Glu Leu Leu Pro Val His Ala Lys Ser Phe
               395                 400                 405
   tac aac gaa cag gcc gtc tac tcg caa ctc att cgc cag tcc acc gcg     13722
   Tyr Asn Glu Gln Ala Val Tyr Ser Gln Leu Ile Arg Gln Ser Thr Ala
           410                 415                 420
   ctt acc cac gtg ttc aat cgc ttt ccc gag aac cag att ctg gtg cgc     13770
   Leu Thr His Val Phe Asn Arg Phe Pro Glu Asn Gln Ile Leu Val Arg
       425                 430                 435
   cct ccc gct cct acc att acc acc gtc agt gaa aac gtt ccc gcc ctc     13818
   Pro Pro Ala Pro Thr Ile Thr Thr Val Ser Glu Asn Val Pro Ala Leu
   440                 445                 450                 455
   aca gat cac gga acc ctg ccg ctg cgc agc agt atc agt gga gtt cag     13866
   Thr Asp His Gly Thr Leu Pro Leu Arg Ser Ser Ile Ser Gly Val Gln
                   460                 465                 470
   cgc gtg acc atc acc gac gcc aga cgt cga acc tgt ccc tac gtt tac     13914
   Arg Val Thr Ile Thr Asp Ala Arg Arg Arg Thr Cys Pro Tyr Val Tyr
               475                 480                 485
   aaa gct ctt ggc gta gtg gct cct aaa gtg ctc tct agt cgc acc ttc     13962
   Lys Ala Leu Gly Val Val Ala Pro Lys Val Leu Ser Ser Arg Thr Phe
           490                 495                 500
   taa acatgtccat cctcatctct cccgataaca acaccggctg gggactgggc          14015
   tccggcaaga tgtacggcgg agccaaaagg cgctccagtc agcacccagt tcgagttcgg   14075
   ggccacttcc gtgctccctg gggagcttac aagcgaggac tctcgggccg aacggcggta   14135
   gacgatacca tagatgccgt gattgccgac gcccgccggt acaaccccgg accggtcgct 14195
   agcgccgcct ccaccgtgga ttccgtgatc gacagcgtgg tagctggcgc tcgggcctat 14255
   gctcgccgca agaggcggct gcatcggaga cgtcgcccca ccgccgccat gctggcagcc 14315
   agggccgtgc tgaggcgggc ccggagggta ggcagaaggg ctatgcgccg cgctgccgcc 14375
   aacgccgccg ccgggagggc ccgccgacag gctgcccgcc aggctgctgc cgccatcgct 14435
   agcatggcca gacccaggag agggaacgtg tactgggtgc gcgattctgt gacgggagtc 14495
   cgagtgccgg tgcgcagccg acctccccga agttagaaga tccaagctgc gaagacggcg 14555
   gtactgagtc tccctgttgt tatcagccca acatgagcaa gcgcaagttt aaagaagaac 14615
   tgctgcagac gctggtgcct gagatctatg gccctccgga cgtgaagcct gacattaagc 14675
   cccgcgatat caagcgtgtt aaaaagcggg aaaagaaaga ggaactcgcg gtggtagacg 14735
   atggcggagt ggaatttatt aggagtttcg ccccgcgacg cagggttcaa tggaaagggc 14795
   ggcgggtaca acgcgttttg aggccgggca ccgcggtagt ttttaccccg ggagagcggt 14855
   cggccgttag ggtttcaaaa ggcagtacga acgaggtgta cggcgacgag gacatattgg 14915
   aacaggcggc tcaacagatc ggagaatttg cctacggaaa gcgttcgcgt cgcgaagacc 14975
   tggccatcgc tttagacagc ggcaacccca cgcccagcct caaacctgtg acgctgcagc 15035
   aggtgctccc cgtgagcgcc agcacggaca gcaagagggg aataaaaaga gaaatggaag 15095
   atctgcagcc caccatccag ctcatggtcc ctaaacggca gaggctggaa gaggtcctgg 15155
   agaaaatgaa agtggaccca agcatagagc cggacgtcaa agtcaggccg atcaaagaag 15215
   tggcccctgg tctcggggtg cagacggtgg atatccagat ccccgtcacg tcagcttcga 15275
   ccgccgtgga agccatggaa acgcaaacgg aaacccctgc cgcgatcggt accagggaag 15335
   tggcgttgca aaccgacccc tggtacgaat acgccgcccc tcggcgtcag aggcgacccg 15395
   ctcgttacgg ccccgccaac gccatcatgc cagaatatgc gctgcatccg tctatcctgc 15455
   ccacccccgg ctaccgggga gtgacgtatc gcccgtcagg aacccgccgc cgaacccgtc 15515
   gccgccgccg ctcccgtcgt gctctggccc ccgtgtcggt gcgccgcgta acacgccggg 15575
   gaaagacagt taccattccc aacccgcgct accaccctag catcctttaa tgactctgcc 15635
   gttttgcaga tggctctgac ttgccgcgtg cgccttcccg ttccgcacta tcgaggaaga 15695
   tctcgtcgta ggagaggcat ggcgggtagt ggtcgccggc gggctttgcg caggcgcatg 15755
   aaaggcggaa ttttacccgc tctgataccc ataatcgccg ccgccatcgg tgccataccc 15815
   ggcgtcgctt cagtggcctt gcaagcagct cgtaataaat aaacgaaggc ttttgcactt 15875
   atgtcctggt cctgactatt ttatgcagaa agagcatgga agacatcaat tttacgtcgc 15935
   tggctccgcg gcacggctcg cggccgctca tgggcacctg gaacgacatc ggcaccagtc 15995
   agctcaacgg gggcgctttc aattggggga gcctttggag cggcattaaa aactttggct 16055
   ccacgattaa atcctacggc agcaaagcct ggaacagtag tgctggtcag atgctccgag 16115
   ataaactgaa ggacaccaac ttccaagaaa aagtggtcaa tggggtggtg accggcatcc 16175
   acggcgcggt agatctcgcc aaccaagcgg tgcagaaaga gattgacagg cgtttggaaa 16235
   gctcgcgggt gccgccgcag agaggggatg aggtggaggt cgaggaagta gaagtagagg 16295
   aaaagctgcc cccgctggag aaagttcccg gtgcgcctcc gagaccgcag aagcgaccca 16355
   ggccagaact agaagaaact ctggtgacgg agagcaagga gcctccctcg tacgagcaag 16415
   ccttgaaaga gggcgcctct ccaccctacc caatgacaaa accgatcgcg cctatggctc 16475
   ggccggtgta cgggaaggac tacaagcctg tcacgctaga gctccccccg ccgccaccgc 16535
   cgccccccac gcgcccgacc gttccccccc ccctgccggc tccgtcggcg ggacccgtgt 16595
   ccgcacccgt cgccgtgcct ctgccagccg cccgcccagt ggccgtggcc actgccagaa 16655
   accccagagg ccagagagga gccaactggc aaagcacgct gaacagcatc gtgggcctgg 16715
   gagtgaaaag cctgaaacgc cgccgttgct attattaaaa gtgtagctaa aaaatttccc 16775
   gttgtatacg cctcctatgt taccgccaga gacgcgtgac tgtcgccgcg agcgccgctt 16835
   tcaag atg gcc acc cca tcg atg atg ccg cag tgg tct tac atg cac atc 16885
   Met Ala Thr Pro Ser Met Met Pro Gln Trp Ser Tyr Met His Ile
   505 510 515
   gcc ggg cag gac gcc tcg gag tac ctg agc ccc ggt ctc gtg cag ttc 16933
   Ala Gly Gln Asp Ala Ser Glu Tyr Leu Ser Pro Gly Leu Val Gln Phe
   520 525 530
   gcc cgc gcc acc gac acc tac ttc agc ttg gga aac aag ttt aga aac 16981
   Ala Arg Ala Thr Asp Thr Tyr Phe Ser Leu Gly Asn Lys Phe Arg Asn
   535 540 545 550
   ccc acc gtg gcc ccc acc cac gat gta acc acg gac cgc tcg caa agg 17029
   Pro Thr Val Ala Pro Thr His Asp Val Thr Thr Asp Arg Ser Gln Arg
   555 560 565
   ctg acc ctg cgt ttt gtg ccc gta gac cgg gag gac acc gcg tac tct 17077
   Leu Thr Leu Arg Phe Val Pro Val Asp Arg Glu Asp Thr Ala Tyr Ser
   570 575 580
   tac aaa gtg cgc tac acg ctg gcc gta ggg gac aac cga gtg ctg gac 17125
   Tyr Lys Val Arg Tyr Thr Leu Ala Val Gly Asp Asn Arg Val Leu Asp
   585 590 595
   atg gcc agc acc tac ttt gac atc cgg gga gtg ctg gat cgc ggt ccc 17173
   Met Ala Ser Thr Tyr Phe Asp Ile Arg Gly Val Leu Asp Arg Gly Pro
   600 605 610
   agt ttt aag ccc tac tcg ggt acc gcg tac aat tcc ctg gct ccc aag 17221
   Ser Phe Lys Pro Tyr Ser Gly Thr Ala Tyr Asn Ser Leu Ala Pro Lys
   615 620 625 630
   ggc gct ccc aac cct gca gaa tgg acg aat tca gac agc aaa gtt aaa 17269
   Gly Ala Pro Asn Pro Ala Glu Trp Thr Asn Ser Asp Ser Lys Val Lys
   635 640 645
   gtg agg gca cag gcg cct ttt gtt agc tcg tat ggt gct aca gcg att     17317
   Val Arg Ala Gln Ala Pro Phe Val Ser Ser Tyr Gly Ala Thr Ala Ile
               650                 655                 660
   aca aaa gag ggt att cag gtg gga gta acc tta aca gac tcc gga tca     17365
   Thr Lys Glu Gly Ile Gln Val Gly Val Thr Leu Thr Asp Ser Gly Ser
           665                 670                 675
   aca cca cag tat gca gat aaa acg tat cag cct gag ccg caa att gga     17413
   Thr Pro Gln Tyr Ala Asp Lys Thr Tyr Gln Pro Glu Pro Gln Ile Gly
       680                 685                 690
   gaa cta cag tgg aac agc gat gtt gga acc gat gac aaa ata gca gga     17461
   Glu Leu Gln Trp Asn Ser Asp Val Gly Thr Asp Asp Lys Ile Ala Gly
   695                 700                 705                 710
   aga gtg cta aag aaa aca acg ccc atg ttc cct tgt tac ggc tca tat     17509
   Arg Val Leu Lys Lys Thr Thr Pro Met Phe Pro Cys Tyr Gly Ser Tyr
                   715                 720                 725
   gcc agg ccc act aat gaa aaa gga gga cag gca aca ccg tcc gct agt     17557
   Ala Arg Pro Thr Asn Glu Lys Gly Gly Gln Ala Thr Pro Ser Ala Ser
               730                 735                 740
   caa gac gtg caa aat ccc gaa tta caa ttt ttt gcc tct act aat gtc     17605
   Gln Asp Val Gln Asn Pro Glu Leu Gln Phe Phe Ala Ser Thr Asn Val
           745                 750                 755
   gcc aat aca cca aaa gca gtt cta tat gcg gag gac gtg tca att gaa     17653
   Ala Asn Thr Pro Lys Ala Val Leu Tyr Ala Glu Asp Val Ser Ile Glu
       760                 765                 770
   gcg cca gac act cac ttg gtg ttc aaa cca aca gtc act gaa ggc att     17701
   Ala Pro Asp Thr His Leu Val Phe Lys Pro Thr Val Thr Glu Gly Ile
   775                 780                 785                 790
   aca agt tca gag gct cta ctg acc caa caa gct gct ccc aac cgt cca     17749
   Thr Ser Ser Glu Ala Leu Leu Thr Gln Gln Ala Ala Pro Asn Arg Pro
                   795                 800                 805
   aac tac ata gcc ttt aga gat aat ttt att ggt ctc atg tac tac aat     17797
   Asn Tyr Ile Ala Phe Arg Asp Asn Phe Ile Gly Leu Met Tyr Tyr Asn
               810                 815                 820
   agc aca ggt aac atg gga gta ctg gca ggc cag gct tct cag cta aat     17845
   Ser Thr Gly Asn Met Gly Val Leu Ala Gly Gln Ala Ser Gln Leu Asn
           825                 830                 835
   gca gtt gtt gac ctg caa gac aga aat act gag ctg tcc tac caa ctc     17893
   Ala Val Val Asp Leu Gln Asp Arg Asn Thr Glu Leu Ser Tyr Gln Leu
       840                 845                 850
   atg ttg gac gcc ctc gga gac cgc agt cgg tac ttt tct atg tgg aac     17941
   Met Leu Asp Ala Leu Gly Asp Arg Ser Arg Tyr Phe Ser Met Trp Asn
   855                 860                 865                 870
   caa gct gtg gat agt tac gat cct gat gta aga atc ata gaa aac cat     17989
   Gln Ala Val Asp Ser Tyr Asp Pro Asp Val Arg Ile Ile Glu Asn His
                   875                 880                 885
   ggc gta gaa gat gaa ttg cct aat tat tgc ttt cct ttg gga ggc atg     18037
   Gly Val Glu Asp Glu Leu Pro Asn Tyr Cys Phe Pro Leu Gly Gly Met
   890 895 900
   gca gta acc gac acc tac tcg cct ata aag gtt aat gga gga ggc aat 18085
   Ala Val Thr Asp Thr Tyr Ser Pro Ile Lys Val Asn Gly Gly Gly Asn
   905 910 915
   gga tgg gaa gcc aat aac ggc gtt ttc acc gaa aga gga gtg gaa ata 18133
   Gly Trp Glu Ala Asn Asn Gly Val Phe Thr Glu Arg Gly Val Glu Ile
   920 925 930
   ggt tca ggg aac atg ttt gcc atg gag att aac ctg caa gcc aac cta 18181
   Gly Ser Gly Asn Met Phe Ala Met Glu Ile Asn Leu Gln Ala Asn Leu
   935 940 945 950
   tgg cgt agc ttt ctg tac tcc aat att ggg ctg tac ctg cca gac tct 18229
   Trp Arg Ser Phe Leu Tyr Ser Asn Ile Gly Leu Tyr Leu Pro Asp Ser
   955 960 965
   ctc aaa atc act cct gac aac atc aca ctc cca gag aac aaa aac acc 18277
   Leu Lys Ile Thr Pro Asp Asn Ile Thr Leu Pro Glu Asn Lys Asn Thr
   970 975 980
   tat cag tat atg aac ggt cgc gtg acg cca ccc ggg ctg gtt gac acc 18325
   Tyr Gln Tyr Met Asn Gly Arg Val Thr Pro Pro Gly Leu Val Asp Thr
   985 990 995
   tac gtt aac gtg ggc gcg cgc tgg tcc ccc gat gtc atg gac agt 18370
   Tyr Val Asn Val Gly Ala Arg Trp Ser Pro Asp Val Met Asp Ser
   1000 1005 1010
   att aac cct ttt aat cac cac cgc aac gcc gga ctc cgc tac cgt 18415
   Ile Asn Pro Phe Asn His His Arg Asn Ala Gly Leu Arg Tyr Arg
   1015 1020 1025
   tcc atg ctc ctg gga aac gga cgc tac gtg ccc ttc cac atc cag 18460
   Ser Met Leu Leu Gly Asn Gly Arg Tyr Val Pro Phe His Ile Gln
   1030 1035 1040
   gtg ccc cag aaa ttc ttt gca att aaa aac ctg ctg ctg ctc ccc 18505
   Val Pro Gln Lys Phe Phe Ala Ile Lys Asn Leu Leu Leu Leu Pro
   1045 1050 1055
   ggt tcc tac acc tac gag tgg aac ttc cgc aag gac gtg aac atg 18550
   Gly Ser Tyr Thr Tyr Glu Trp Asn Phe Arg Lys Asp Val Asn Met
   1060 1065 1070
   atc ttg cag agc tcg ctg ggc aat gac ctg cga gtg gac ggg gcc 18595
   Ile Leu Gln Ser Ser Leu Gly Asn Asp Leu Arg Val Asp Gly Ala
   1075 1080 1085
   agc atc cgc ttc gac agc atc aac ctg tac gcc aac ttt ttc ccc 18640
   Ser Ile Arg Phe Asp Ser Ile Asn Leu Tyr Ala Asn Phe Phe Pro
   1090 1095 1100
   atg gcc cac aac acg gcc tcc acc ctg gaa gcc atg ctg cgc aac 18685
   Met Ala His Asn Thr Ala Ser Thr Leu Glu Ala Met Leu Arg Asn
   1105 1110 1115
   gac acc aac gac caa tct ttc aac gac tac ctg tgc gcg gcc aac 18730
   Asp Thr Asn Asp Gln Ser Phe Asn Asp Tyr Leu Cys Ala Ala Asn
   1120 1125 1130
   atg ctg tac ccc atc ccc gcc aac gcc acc agc gtg ccc atc tcc 18775
   Met Leu Tyr Pro Ile Pro Ala Asn Ala Thr Ser Val Pro Ile Ser
       1135                1140                1145
   att ccc tct cgc aac tgg gca gcc ttc agg ggc tgg agt ttc acc    18820
   Ile Pro Ser Arg Asn Trp Ala Ala Phe Arg Gly Trp Ser Phe Thr
       1150                1155                1160
   cgc ctc aaa acc aag gag acc ccc tcg ctg ggc tcc ggg ttc gac    18865
   Arg Leu Lys Thr Lys Glu Thr Pro Ser Leu Gly Ser Gly Phe Asp
       1165                1170                1175
   ccc tac ttc gtc tac tcc ggc tcc atc ccc tac ctg gac ggc acc    18910
   Pro Tyr Phe Val Tyr Ser Gly Ser Ile Pro Tyr Leu Asp Gly Thr
       1180                1185                1190
   ttc tac ctc aac cat act ttc aaa aag gtg tca atc atg ttc gac    18955
   Phe Tyr Leu Asn His Thr Phe Lys Lys Val Ser Ile Met Phe Asp
       1195                1200                1205
   tcc tcc gtc agc tgg ccc ggc aac gac cgt ctg ctg acg ccc aac    19000
   Ser Ser Val Ser Trp Pro Gly Asn Asp Arg Leu Leu Thr Pro Asn
       1210                1215                1220
   gag ttc gaa atc aag cgt tcg gtg gac ggt gaa ggg tac aac gtg    19045
   Glu Phe Glu Ile Lys Arg Ser Val Asp Gly Glu Gly Tyr Asn Val
       1225                1230                1235
   gct cag agc aac atg acc aag gac tgg ttc ctg att cag atg ctc    19090
   Ala Gln Ser Asn Met Thr Lys Asp Trp Phe Leu Ile Gln Met Leu
       1240                1245                1250
   agc cac tac aac atc ggc tac cag ggc ttc tac gtg ccc gaa aat    19135
   Ser His Tyr Asn Ile Gly Tyr Gln Gly Phe Tyr Val Pro Glu Asn
       1255                1260                1265
   tac aag gac cgc atg tac tct ttc ttc aga aac ttc caa ccc atg    19180
   Tyr Lys Asp Arg Met Tyr Ser Phe Phe Arg Asn Phe Gln Pro Met
       1270                1275                1280
   agc cgc caa att gta gat tca acg gct tac act aat tat cag gat    19225
   Ser Arg Gln Ile Val Asp Ser Thr Ala Tyr Thr Asn Tyr Gln Asp
       1285                1290                1295
   gtg aaa ctg cca tac cag cat aac aac tca ggg ttc gtg ggc tac    19270
   Val Lys Leu Pro Tyr Gln His Asn Asn Ser Gly Phe Val Gly Tyr
       1300                1305                1310
   atg gga ccc acc atg cga gag ggg cag gcc tac ccg gcc aac tat    19315
   Met Gly Pro Thr Met Arg Glu Gly Gln Ala Tyr Pro Ala Asn Tyr
       1315                1320                1325
   ccc tat ccc ctg att ggg gcc acc gcc gtg ccc agc ctc acg cag    19360
   Pro Tyr Pro Leu Ile Gly Ala Thr Ala Val Pro Ser Leu Thr Gln
       1330                1335                1340
   aaa aag ttc ctc tgc gac cgg gtg atg tgg agg atc ccc ttc tct    19405
   Lys Lys Phe Leu Cys Asp Arg Val Met Trp Arg Ile Pro Phe Ser
       1345                1350                1355
   agc aac ttc atg tct atg ggc tcc ctc acc gac ctg ggg cag aac    19450
   Ser Asn Phe Met Ser Met Gly Ser Leu Thr Asp Leu Gly Gln Asn
       1360                1365                1370
   atg ctg tac gcc aac tcc gct cac gcc ttg gat atg acc ttt gag 19495
   Met Leu Tyr Ala Asn Ser Ala His Ala Leu Asp Met Thr Phe Glu
   1375 1380 1385
   gtg gat ccc atg gat gag ccc acg ctt ctc tat gtt ctg ttt gaa 19540
   Val Asp Pro Met Asp Glu Pro Thr Leu Leu Tyr Val Leu Phe Glu
   1390 1395 1400
   gtc ttc gac gtg gtg cgc atc cac cag ccg cac cgc ggc gtc atc 19585
   Val Phe Asp Val Val Arg Ile His Gln Pro His Arg Gly Val Ile
   1405 1410 1415
   gag gcc gtc tac ctg cgc aca cct ttc tct gcc ggt aac gcc acc 19630
   Glu Ala Val Tyr Leu Arg Thr Pro Phe Ser Ala Gly Asn Ala Thr
   1420 1425 1430
   acc taa agaagccgat gggctccagc gaacaggagc tgcaggccat tgttcgcgac 19686
   Thr
   ctgggctgcg ggccctactt tttgggcacc ttcgacaagc gttttcccgg cttcatgtcc 19746
   ccccacaagc cggcctgtgc catcgttaac acggccggac gggagaccgg gggggtccac 19806
   tggctcgcct tcgcctggaa cccgcgtaac cgcacctgct acctgttcga cccttttggt 19866
   ttctccgacg aaaggctgaa gcagatctac cagttcgagt acgaggggct cctcaagcgc 19926
   agcgctctgg cctccacgcc cgaccactgc gtcaccctgg aaaagtccac ccaaacggtc 19986
   caggggcccc tctcggccgc ctgcgggctc ttctgttgca tgtttttgca cgccttcgtg 20046
   cactggcctc acacccccat ggatcacaac cccaccatgg atctgctcac cggagtgccc 20106
   aacagcatgc ttcacagccc ccaggtcgcc cccaccctgc gccgtaacca ggaacacctg 20166
   tatcgctttc tggggaaaca ctctgcctat tttcgccgcc accggcagcg catcgaacgg 20226
   gccacggcct tcgaaagcat gagccaaaga gtgtaatcaa taaaaaacat ttttatttga 20286
   catgatacgc gcttctggcg ttttattaaa aatcgaaggg ttcgagggag gggtcctcgt 20346
   gcccgctggg gagggacacg ttgcgatact ggaaacgggc gctccaacga aactcgggga 20406
   tcaccagccg cggcaggggc acgtcttcta ggttctgctt ccaaaactgc cgcaccagct 20466
   gcagggctcc catgacgtcg ggcgccgata tcttgaagtc gcagttaggg ccggagctcc 20526
   cgcggctgtt gcggaacacg gggttggcac actggaacac cagcacgccg gggttgtgga 20586
   tactggccag ggccgtcggg tcggtcacct ccgacgcatc cagatcctcg gcgttgctca 20646
   gggcaaacgg ggtcagcttg cacatctgcc gcccaatctg ggtactaggg tcgcgcttgt 20706
   tgaggcagtc gcagcgcaga gggatcagga tgcgtcgctg cccgcgttgc atgatagggt 20766
   aactcgccgc caggaactcc tccatttgac ggaaggccat ctgggctttg ccgccctcgg 20826
   tgtagaatag cccgcaggac ttgctagaga atacgttatg accgcagttg acgtcctccg 20886
   cgcagcagcg ggcgtcttcg ttcttcagct gaaccacgtt gcggccccaa cggttctgga 20946
   ccaccttggc tctagtgggg tgctccttca gcgcccgctg tccgttctcg ctggttacat 21006
   ccatttccaa cacgtgctcc ttgcagacca tctccactcc gtggaagcaa aacaggacgc 21066
   cctcctgctg ggtactgcga tgctcccata cggcgcatcc ggtgggctcc cagctcttgt 21126
   gttttacccc cgcgtaggct tccatgtaag ccataaggaa tctgcccatc agctcggtga 21186
   aggtcttctg gttggtgaag gttagcggca ggccgcggtg ctcctcgttc aaccaagttt 21246
   gacagatctt gcggtacacc gctccctggt cgggcagaaa cttaaaagcc gctctgctgt 21306
   cgttgtctac gtggaacttc tccattaaca tcatcatggt ttccataccc ttctcccacg 21366
   ctgtcaccag tggtttgctg tcggggttct tcaccaacac ggcggtagag gggccctcgc 21426
   cggccccgac gtccttcatg gtcattcttt gaaactccac ggagccgtcc gcgcgacgta 21486
   ctctgcgcac cggagggtag ctgaagccca cctccaccac ggtgccttcg ccctcgctgt 21546
   cggagacaat ctccggggat ggcggcggcg cgggtgtcgc cttgcgagcc ttcttcttgg 21606
   gagggagctg aggcgcctcc tgctcgcgct cggggctcat ctcccgcaag tagggggtaa 21666
   tggagctgcc tgcttggttc tgacggttgg ccattgtatc ctaggcagaa agacatggag 21726
   cttatgcgcg aggaaacttt aaccgccccg tcccccgtca gcgacgaaga tgtcatcgtc 21786
   gaacaggacc cgggctacgt tacgccgccc gaggatctgg aggggcctga ccggcgcgac 21846
   gctagtgagc ggcaggaaaa tgagaaagag gaggcctgct acctcctgga aggcgacgtt 21906
   ttgctaaagc atttcgccag gcagagcacc atagttaagg aggccttgca agaccgctcc 21966
   gaggtgccct tggacgtcgc cgcgctctcc caggcctacg aggcgaacct tttctcgcct 22026
   cgagtgcctc cgaagagaca gcccaacggc acctgcgagc ccaacccgcg actcaacttc 22086
   taccccgtgt tcgccgtacc agaggcgctg gccacctatc acattttttt caaaaaccaa 22146
   cgcatccccc tatcgtgccg ggccaaccgc accgcggccg ataggaatct caggcttaaa 22206
   aacggagcca acatacctga tatcacgtcg ctggaggaag tgcccaagat tttcgagggt 22266
   ctgggtcgag atgagaagcg ggcggcgaac gctctgcaga aagaacagaa agagagtcag 22326
   aacgtgctgg tggagctgga gggggacaac gcgcgtctgg ccgtcctcaa acgctgcata 22386
   gaagtctccc acttcgccta ccccgccctc aacttgccac ccaaagttat gaaatcggtc 22446
   atggatcagc tgctcatcaa gagagctgag cccctggatc ccgaccaccc cgaggcggaa 22506
   aactcagagg acggaaagcc cgtcgtcagc gacgaggagc tcgagcggtg gctggaaacc 22566
   agggaccccc aacagttgca agagaggcgc aagatgatga tggcggccgt gctggtcacc 22626
   gtggagctgg aatgcctgca acggtttttc agcgacgtgg agacgctacg caaaatcggg 22686
   gaatccctgc actacacctt ccgccagggc tacgtccgcc aggcctgcaa gatctccaac 22746
   gtggagctca gcaacctggt ctcctacatg ggcatcctcc acgagaaccg gctggggcag 22806
   agcgtgctgc actgcacctt gcaaggcgag gcgcggcggg actacgtgcg agactgcatc 22866
   tacctcttcc tcaccctcac ctggcagacc gccatgggcg tctggcagca gtgcttggaa 22926
   gagagaaacc tcaaagagct agacaaactc ctctgccgcc agcggcgcgc cctgtggtcc 22986
   ggtttcagcg agcgcacggt cgccagcgct ctggcggaca tcatcttccc ggagcgcctg 23046
   atgaaaacct tgcaaaacgg cctgccggat ttcatcagtc aaagcatttt gcaaaacttc 23106
   cgctcttttg tcctggaacg ctccgggatc ttgcccgcca tgagctgcgc gctaccttct 23166
   gactttgtcc ccctctccta ccgcgagtgc cctcccccac tgtggagcca ctgctacctc 23226
   ttccaactgg ccaactttct ggcctaccac tccgacctca tggaagacgt aagcggagag 23286
   ggtttactgg agtgccactg ccgctgcaac ctgtgcaccc cccacagatc gctggcctgc 23346
   aacaccgagc tactcagcga aacccaggtc ataggtacct tcgagatcca ggggccccag 23406
   cagcaagagg gtgcttccgg cttgaagctc actccggcgc tgtggacctc ggcttactta 23466
   cgcaaatttg tagccgagga ctaccacgcc cacaaaattc agttttacga agaccaatct 23526
   cgaccaccga aagcccccct cacggcctgc gtcatcaccc agagcaagat cctggcccaa 23586
   ttgcaatcca tcaaccaagc gcgccgcgat ttccttttga aaaagggtcg gggggtgtac 23646
   ctggaccccc agaccggcga ggaactcaac ccgtccacac tctccgtcga agcagccccc 23706
   ccgagacatg ccgcccaagg gaaccgccaa gcagctgatc gctcggcaga gagcgaagaa 23766
   gcaagagctg ctccagcagc aggtggagga cgaggaagag atgtgggaca gccaggcaga 23826
   ggaggtgtca gaggacgagg aggagatgga aagctgggac agcctagacg aggaggagga 23886
   cgagctttca gaggaagagg cgaccgaaga aaaaccacct gcatccagcg cgccttctct 23946
   gagccgacag ccgaagcccc ggcccccgac gcccccggcc ggctcactca aagccagccg 24006
   taggtgggac gccaccgaat ctccagcggc agcggcaacg gcagcgggta aggccaaacg 24066
   cgagcggcgg gggtattgct cctggcgggc ccacaaaagc agtattgtga actgcttgca 24126
   acactgcggg ggaaacatct cctttgcccg acgctacctc ctcttccatc acggtgtggc 24186
   cttccctcgc aacgttctct attattaccg tcatctctac agcccctacg aaacgctcgg 24246
   agaaaaaagc taaggcctcc tccgccgcga ggaaaaactc cgccgccgct gccgccgcca 24306
   aggatccacc ggccaccgaa gagctgagaa agcgcatctt tcccactctg tatgctatct 24366
   ttcagcaaag ccgcgggcag caccctcagc gcgaactgaa aataaaaaac cgctccttcc 24426
   gctcgctcac ccgcagctgt ctgtaccaca agagagaaga ccagctgcag cgcaccctgg 24486
   acgacgccga agcactgttc agcaaatact gctcagcgtc tcttaaagac taaaagaccc 24546
   gcgctttttc cccctcggcc gccaaaaccc acgtcatcgc cagcatgagc aaggagattc 24606
   ccacccccta catgtggagc tatcagcccc agatgggcct ggccgcgggg gccgcccagg 24666
   actactccag caagatgaac tggctcagcg ccggccccca catgatctca cgagttaacg 24726
   gcatccgagc ccaccgaaac cagattctct tagaacaggc ggcaatcacc gccacacccc 24786
   ggcgccaact caacccgcct agttggcccg ccgcccaggt gtatcaggaa aatccccgcc 24846
   cgaccacagt cctcctgcca cgcgacgcgg aggccgaagt cctcatgact aactctgggg 24906
   tacaattagc gggcgggtcc aggtacgcca ggtacagagg tcgggccgct ccttactctc 24966
   ccgggagtat aaagagggtg atcattcgag gccgaggtat ccagctcaac gacgagacgg 25026
   tgagctcctc aaccggtctc agacctgacg gagtcttcca gctcggagga gcgggccgct 25086
   cttccttcac cactcgccag gcctacctga ccctgcagag ctcttcctcg cagccgcgct 25146
   ccgggggaat cggcactctc cagttcgtgg aagagttcgt tccctccgtc tacttcaacc 25206
   ccttctccgg ctcgcctgga cgctacccgg acgccttcat tcccaacttt gacgcagtga 25266
   gtgaatccgt ggacggctac gactgatgac agatggtgcg gccgtgagag ctcggctgcg 25326
   acatctgcat cactgccgtc agcctcgctg ctacgctcgg gaggcgatcg tcttcagcta 25386
   ctttgagctg ccggacgagc accctcaggg tccggctcac gggttgaaac tcgagatcga 25446
   gaacgcgctc gagtctcgcc tcatcgacac cttcaccgcc cgacctctcc tggtagaaat 25506
   ccaacggggg atcactacca tcaccctgtt ctgcatctgc cccacgcccg gattacatga 25566
   agatctgtgt tgtcatcttt gcgctcagtt taataaaaac tgaacttttt gccgcacctt 25626
   caacgccatc tgtgatttct acaacaaaaa gttcttctgg caaaggtaca caaactgtat 25686
   tttattctaa ttctacctca tctatcgtgc tgaactgcgc ctgcactaac gaacttatcc 25746
   agtggattgc aaacggtagt gtgtgcaagt acttttgggg gaacgatata gttagtagaa 25806
   ataacagcct ttgcgagcac tgcaactcct ccacactaat cctttatccc ccatttgtta 25866
   ctggatggta tatgtgcgtt ggctccggtt taaatcctag ttgctttcat aagtggtttc 25926
   tacaaaaaga gacccttccc aacaattctg tttctttttt cgccctatcc tactgctgtt 25986
   ctccctctgg ttactctttc aaacctctaa ttggtatttt agctttgata ctcataatct 26046
   ttattaactt tataataatt aacaacttac agtaaacatg cttgttctac tgctcgccac 26106
   atctttcgct ctctctcacg ccagaacaag tattgttggc gcaggttaca atgcaactct 26166
   tcaatctgct tacatgccag attccgacca gataccccat attacgtggt acttacaaac 26226
   ctccaaacct aattcttcat tttatgaagg aaacaaactc tgcgatgact ccgacaacag 26286
   aacgcacaca tttccccacc cttcactaca attcgaatgc gtaaacaaaa gcttgaagct 26346
   ttacaactta aagccttcag attctggctt gtaccatgct gtagttgaaa aaagtaattt 26406
   agaagtccac agtgattaca ttgaattgac ggttgtggac ctgccacctc caaaatgtga 26466
   ggtttcctcc tcttaccttg aagttcaagg cgtggatgcc tactgcctca tacacattaa 26526
   ctgcagcaac tctaaatatc cagctagaat ttactataat ggacaggaaa gtaatctttt 26586
   ttattattta acaacaagcg ctggtaacgg taaacagtta cctgactatt ttactgctgt 26646
   tgttgaattt tccacctaca gagaaacgta tgccaagcgg ccttacaatt tctcataccc 26706
   gtttaacgac ctttgcaatg aaatacaagc gctcgaaact ggaactgatt ttactccaat 26766
   tttcattgct gccattgttg taagcttaat taccattatt gtcagcctag cattttactg 26826
   cttttacaag cccaaaaacc ctaagtttga aaaacttaaa ctaaaacctg tcattcaaca 26886
   agtgtgattt tgttttccag catggtagct gcatttctac ttctcctctg tctacccatc 26946
   attttcgtct cttcaacttt cgccgcagtt tcccacctgg aaccagagtg cctaccgcct 27006
   tttgacgtgt atctgattct cacctttgtt tgttgtatat ccatttgcag tatagcctgc 27066
   ttttttataa caatctttca agccgccgac tatttttacg tgcgaattgc ttactttaga 27126
   caccatcctg aatacagaaa tcaaaacgtt gcctccttac tttgtttggc atgattaagt 27186
   tattgctgat acttaattat ttacccctaa tcaactgtaa ttgtccattc accaaaccct 27246
   ggtcattcta cacctgttat gataaaatcc ccgacactcc tgttgcttgg ctttacgcag 27306
   ccaccgccgc tttggtattt atatctactt gccttggagt aaaattgtat tttattttac 27366
   acactgggtg gctacatccc agagaagatt tacctagata tcctcttgta aacgcttttc 27426
   aattacagcc tctgcctcct cctgatcttc ttcctcgagc tccctctatt gtgagctact 27486
   ttcaactcac cggtggagat gactgactct caggacatta atattagtgt ggaaagaata 27546
   gctgctcagc gtcagcgaga aacgcgagtg ttggaatacc tggaactaca gcaacttaaa 27606
   gagtcccact ggtgtgagaa aggagtgctg tgccatgtta agcaggcagc cctttcctac 27666
   gatgtcagcg ttcagggaca tgaactgtct tacactttgc ctttgcagaa acaaaccttc 27726
   tgcaccatga tgggctctac ctccatcaca atcacccaac aagccgggcc tgtagagggg 27786
   gctatcctct gtcactgtca cgcacctgat tgcatgtcca aactaatcaa aactctctgt 27846
   gctttaggtg atatttttaa ggtgtaaatc aataataaac ttaccttaaa tttgacaaca 27906
   aatttctggt gacatcattc agcagcacca ctttaccctc ttcccagctc tcgtatggga 27966
   tgcgatagtg ggtggcaaac ttcctccaaa ccctaaaaga aatattggta tccacttcct 28026
   tgtcctcacc cacaattttc atcttttcat ag atg aaa aga acc aga gtt gat 28079
   Met Lys Arg Thr Arg Val Asp
   1435 1440
   gaa gac ttc aac ccc gtc tac ccc tat gac acc aca acc act cct 28124
   Glu Asp Phe Asn Pro Val Tyr Pro Tyr Asp Thr Thr Thr Thr Pro
   1445 1450 1455
   gca gtt ccc ttt ata tca ccc ccc ttt gta aac agc gat ggt ctt 28169
   Ala Val Pro Phe Ile Ser Pro Pro Phe Val Asn Ser Asp Gly Leu
   1460 1465 1470
   cag gaa aac ccc cca ggt gtt tta agt ctg cga ata gct aaa ccc 28214
   Gln Glu Asn Pro Pro Gly Val Leu Ser Leu Arg Ile Ala Lys Pro
   1475 1480 1485
   cta tat ttc gac atg gag aga aaa cta gcc ctt tca ctt gga aga 28259
   Leu Tyr Phe Asp Met Glu Arg Lys Leu Ala Leu Ser Leu Gly Arg
   1490 1495 1500
   ggg ttg aca att acc gcc gcc gga caa tta gaa agt acg cag agc 28304
   Gly Leu Thr Ile Thr Ala Ala Gly Gln Leu Glu Ser Thr Gln Ser
   1505 1510 1515
   gta caa acc aac cca ccg ttg ata att acc aac aac aac aca ctg 28349
   Val Gln Thr Asn Pro Pro Leu Ile Ile Thr Asn Asn Asn Thr Leu
   1520 1525 1530
   acc cta cgt cat tct ccc ccc tta aac cta act gac aat agc tta 28394
   Thr Leu Arg His Ser Pro Pro Leu Asn Leu Thr Asp Asn Ser Leu
   1535 1540 1545
   gtg cta ggc tac tcg agt cct ctc cgc gtc aca gac aac aaa ctt 28439
   Val Leu Gly Tyr Ser Ser Pro Leu Arg Val Thr Asp Asn Lys Leu
   1550 1555 1560
   aca ttt aac ttc aca tca cca ctc cgt tat gaa aat gaa aac ctt 28484
   Thr Phe Asn Phe Thr Ser Pro Leu Arg Tyr Glu Asn Glu Asn Leu
   1565 1570 1575
   act ttt aac tat aca gag cct ctt aaa ctt ata aat aac agc ctt 28529
   Thr Phe Asn Tyr Thr Glu Pro Leu Lys Leu Ile Asn Asn Ser Leu
   1580 1585 1590
   gcc att gac atc aat tcc tca aaa ggc ctt agt agc gtc gga ggc 28574
   Ala Ile Asp Ile Asn Ser Ser Lys Gly Leu Ser Ser Val Gly Gly
   1595 1600 1605
   tca cta gct gta aac ctg agt tca gac tta aag ttt gac agc aac 28619
   Ser Leu Ala Val Asn Leu Ser Ser Asp Leu Lys Phe Asp Ser Asn
   1610 1615 1620
   gga tcc ata gct ttt ggc ata caa acc ctg tgg acc gct ccg acc 28664
   Gly Ser Ile Ala Phe Gly Ile Gln Thr Leu Trp Thr Ala Pro Thr
   1625 1630 1635
   tcg act ggc aac tgc acc gtc tac agc gag ggc gat tcc cta ctt 28709
   Ser Thr Gly Asn Cys Thr Val Tyr Ser Glu Gly Asp Ser Leu Leu
   1640 1645 1650
   agt ctc tgt tta acc aaa tgc gga gct cac gtc tta gga agt gta 28754
   Ser Leu Cys Leu Thr Lys Cys Gly Ala His Val Leu Gly Ser Val
   1655 1660 1665
   agt tta acc ggt tta aca gga acc ata acc caa atg act gat att 28799
   Ser Leu Thr Gly Leu Thr Gly Thr Ile Thr Gln Met Thr Asp Ile
   1670 1675 1680
   tct gtc acc att caa ttt aca ttt gac aac aat ggt aag cta cta 28844
   Ser Val Thr Ile Gln Phe Thr Phe Asp Asn Asn Gly Lys Leu Leu
   1685 1690 1695
   agc tct cca ctt ata aac aac gcc ttt agt att cga cag aat gac 28889
   Ser Ser Pro Leu Ile Asn Asn Ala Phe Ser Ile Arg Gln Asn Asp
   1700 1705 1710
   agt acg gcc tca aac cct acc tac aac gcc ctg gcg ttt atg cct 28934
   Ser Thr Ala Ser Asn Pro Thr Tyr Asn Ala Leu Ala Phe Met Pro
   1715 1720 1725
   aac agt acc ata tat gca aga ggg gga ggt ggt gaa cca cga aac 28979
   Asn Ser Thr Ile Tyr Ala Arg Gly Gly Gly Gly Glu Pro Arg Asn
   1730 1735 1740
   aac tac tac gtc caa acg tat ctt agg gga aat gtt caa aaa cca 29024
   Asn Tyr Tyr Val Gln Thr Tyr Leu Arg Gly Asn Val Gln Lys Pro
   1745 1750 1755
   atc att ctt act gta acc tac aac tca gtc gcc aca gga tat tcc 29069
   Ile Ile Leu Thr Val Thr Tyr Asn Ser Val Ala Thr Gly Tyr Ser
   1760 1765 1770
   tta tct ttt aag tgg act gct ctt gca cgt gaa aag ttt gca acc 29114
   Leu Ser Phe Lys Trp Thr Ala Leu Ala Arg Glu Lys Phe Ala Thr
   1775 1780 1785
   cca aca acc tcg ttt tgc tac att aca gaa caa taa aaccgtgtac 29160
   Pro Thr Thr Ser Phe Cys Tyr Ile Thr Glu Gln
   1790 1795
   cccaccgttt cgtttttttc ag atg aaa cgg gcg aga gtt gat gaa gac 29209
   Met Lys Arg Ala Arg Val Asp Glu Asp
   1800 1805
   ttc aac cca gtg tac cct tat gac ccc cca cat gct cct gtt atg 29254
   Phe Asn Pro Val Tyr Pro Tyr Asp Pro Pro His Ala Pro Val Met
   1810 1815 1820
   ccc ttc att act cca cct ttt acc tcc tcg gat ggg ttg cag gaa 29299
   Pro Phe Ile Thr Pro Pro Phe Thr Ser Ser Asp Gly Leu Gln Glu
   1825 1830 1835
   aaa cca ctt gga gtg tta agt tta aac tac aga gat ccc att act 29344
   Lys Pro Leu Gly Val Leu Ser Leu Asn Tyr Arg Asp Pro Ile Thr
   1840 1845 1850
   acg caa aat gag tct ctt aca att aaa cta gga aac ggc ctc act 29389
   Thr Gln Asn Glu Ser Leu Thr Ile Lys Leu Gly Asn Gly Leu Thr
   1855 1860 1865
   cta gac aac cag gga caa cta aca tca acc gct ggc gaa gta gaa 29434
   Leu Asp Asn Gln Gly Gln Leu Thr Ser Thr Ala Gly Glu Val Glu
   1870 1875 1880
   cct cca ctc act aac gct aac aac aaa ctt gca ctg gtc tat agc 29479
   Pro Pro Leu Thr Asn Ala Asn Asn Lys Leu Ala Leu Val Tyr Ser
   1885 1890 1895
   gat cct tta gca gta aag cgc aac agc cta acc tta tcg cac acc 29524
   Asp Pro Leu Ala Val Lys Arg Asn Ser Leu Thr Leu Ser His Thr
   1900 1905 1910
   gct ccc ctt gtt att gct gat aac tct tta gca ttg caa gtt tca 29569
   Ala Pro Leu Val Ile Ala Asp Asn Ser Leu Ala Leu Gln Val Ser
   1915 1920 1925
   gag cct att ttt ata aat gac aag gac aaa cta gcc ctg caa aca 29614
   Glu Pro Ile Phe Ile Asn Asp Lys Asp Lys Leu Ala Leu Gln Thr
   1930 1935 1940
   gcc gcg ccc ctt gta act aac gct ggc acc ctt cgc tta caa agc 29659
   Ala Ala Pro Leu Val Thr Asn Ala Gly Thr Leu Arg Leu Gln Ser
   1945 1950 1955
   gcc gcc cct tta ggc att gca gac caa acc cta aaa ctc ctg ttt 29704
   Ala Ala Pro Leu Gly Ile Ala Asp Gln Thr Leu Lys Leu Leu Phe
   1960 1965 1970
   acc aac cct ttg tac ttg cag aat aac ttt ctc acg tta gcc att 29749
   Thr Asn Pro Leu Tyr Leu Gln Asn Asn Phe Leu Thr Leu Ala Ile
   1975 1980 1985
   gaa cga ccc ctt gcc att acc aat act gga aag ctg gct cta cag 29794
   Glu Arg Pro Leu Ala Ile Thr Asn Thr Gly Lys Leu Ala Leu Gln
   1990 1995 2000
   ctc tcc cca ccg cta caa aca gca gac aca ggc ttg act ttg caa 29839
   Leu Ser Pro Pro Leu Gln Thr Ala Asp Thr Gly Leu Thr Leu Gln
   2005 2010 2015
   acc aac gtg cca tta act gta agc aac ggg acc cta ggc tta gcc 29884
   Thr Asn Val Pro Leu Thr Val Ser Asn Gly Thr Leu Gly Leu Ala
   2020 2025 2030
   ata aag cgc cca ctt att att cag gac aac aac ttg ttt ttg gac 29929
   Ile Lys Arg Pro Leu Ile Ile Gln Asp Asn Asn Leu Phe Leu Asp
   2035 2040 2045
   ttc aga gct ccc ctg cgt ctt ttc aac agc gac cca gta cta ggg 29974
   Phe Arg Ala Pro Leu Arg Leu Phe Asn Ser Asp Pro Val Leu Gly
   2050 2055 2060
   ctt aac ttt tac acc cct ctt gcg gta cgc gat gag gcg ctc act 30019
   Leu Asn Phe Tyr Thr Pro Leu Ala Val Arg Asp Glu Ala Leu Thr
   2065 2070 2075
   gtt aac aca ggc cgc ggc ctc aca gtg agt tac gat ggt tta att 30064
   Val Asn Thr Gly Arg Gly Leu Thr Val Ser Tyr Asp Gly Leu Ile
   2080 2085 2090
   tta aat ctt ggt aag gat ctt cgc ttt gac aac aac acc gtt tct 30109
   Leu Asn Leu Gly Lys Asp Leu Arg Phe Asp Asn Asn Thr Val Ser
   2095 2100 2105
   gtc gct ctt agt gct gct ttg cct tta caa tac act gat cag ctt 30154
   Val Ala Leu Ser Ala Ala Leu Pro Leu Gln Tyr Thr Asp Gln Leu
   2110 2115 2120
   cgc ctt aac gtg ggc gct ggg ctg cgt tac aat cca gtg agt aag 30199
   Arg Leu Asn Val Gly Ala Gly Leu Arg Tyr Asn Pro Val Ser Lys
   2125 2130 2135
   aaa ttg gac gtg aac ccc aat caa aac aag ggt tta acc tgg gaa 30244
   Lys Leu Asp Val Asn Pro Asn Gln Asn Lys Gly Leu Thr Trp Glu
   2140 2145 2150
   aat gac tac ctc att gta aag cta gga aat gga tta ggt ttt gat 30289
   Asn Asp Tyr Leu Ile Val Lys Leu Gly Asn Gly Leu Gly Phe Asp
   2155 2160 2165
   ggc gat gga aac ata gct gtt tct cct caa gtt aca tcg cct gac 30334
   Gly Asp Gly Asn Ile Ala Val Ser Pro Gln Val Thr Ser Pro Asp
   2170 2175 2180
   acc tta tgg acc act gcc gac cca tcc ccc aat tgt tcc atc tac 30379
   Thr Leu Trp Thr Thr Ala Asp Pro Ser Pro Asn Cys Ser Ile Tyr
   2185                2190                2195
   act gat tta gat gcc aaa atg tgg ctc tcg ttg gta aaa caa ggg     30424
   Thr Asp Leu Asp Ala Lys Met Trp Leu Ser Leu Val Lys Gln Gly
               2200                2205                2210
   ggt gtg gtt cac ggt tct gtt gct tta aaa gca ttg aaa gga acc     30469
   Gly Val Val His Gly Ser Val Ala Leu Lys Ala Leu Lys Gly Thr
               2215                2220                2225
   cta ttg agt cct acg gaa agc gcc att gtt att ata cta cat ttt     30514
   Leu Leu Ser Pro Thr Glu Ser Ala Ile Val Ile Ile Leu His Phe
               2230                2235                2240
   gac aat tat gga gtg cga att ctc aat tat ccc act ttg ggc act     30559
   Asp Asn Tyr Gly Val Arg Ile Leu Asn Tyr Pro Thr Leu Gly Thr
               2245                2250                2255
   caa ggc acg ttg gga aat aat gca act tgg ggt tat agg cag gga     30604
   Gln Gly Thr Leu Gly Asn Asn Ala Thr Trp Gly Tyr Arg Gln Gly
               2260                2265                2270
   gaa tct gca gac act aat gta ctc aat gca cta gca ttt atg ccc     30649
   Glu Ser Ala Asp Thr Asn Val Leu Asn Ala Leu Ala Phe Met Pro
               2275                2280                2285
   agt tca aaa agg tac cca aga ggg cgt gga agc gaa gtt cag aat     30694
   Ser Ser Lys Arg Tyr Pro Arg Gly Arg Gly Ser Glu Val Gln Asn
               2290                2295                2300
   caa act gtg ggc tac act tgt ata cag ggt gac ttt tct atg ccc     30739
   Gln Thr Val Gly Tyr Thr Cys Ile Gln Gly Asp Phe Ser Met Pro
               2305                2310                2315
   gta ccg tac caa ata cag tac aac tat gga cca act ggc tac tcc     30784
   Val Pro Tyr Gln Ile Gln Tyr Asn Tyr Gly Pro Thr Gly Tyr Ser
               2320                2325                2330
   ttt aaa ttt att tgg aga act gtt tca aga caa cca ttt gac atc     30829
   Phe Lys Phe Ile Trp Arg Thr Val Ser Arg Gln Pro Phe Asp Ile
               2335                2340                2345
   cca tgc tgt ttt ttc tct tac att acg gaa gaa taa aacaactttt     30875
   Pro Cys Cys Phe Phe Ser Tyr Ile Thr Glu Glu
               2350                2355
   tctttttatt ttctttttat tttacacgca cagtaaggct tcctccaccc ttccatctca     30935
   cagcatacac cagcctctcc cccttcatgg cagtaaactg ttgtgagtca gtccggtatt     30995
   tgggagttaa gatccaaaca gtctctttgg tgatgaaaca tggatccgtg atggacacaa     31055
   atccctggga caggttctcc aacgtttcgg taaaaaactg catgccgccc tacaaaacaa     31115
   acaggttcag gctctccacg ggttatctcc ccgatcaaac tcagacagag taaaggtgcg     31175
   atgatgttcc actaaaccac gcaggtggcg ctgtctgaac ctctcggtgc gactcctgtg     31235
   aggctggtaa gaagttagat tgtccagcag cctcacagca tggatcatca gtctacgagt     31295
   gcgtctggcg cagcagcgca tctgaatctc actgagattc cggcaagaat cgcacaccat     31355
   cacaatcagg ttgttcatga tcccatagct gaacacgctc cagccaaagc tcattcgctc     31415
   caacagcgcc accgcgtgtc cgtccaacct tactttaaca taaatcaggt gtctgccgcg 31475
   tacaaacatg ctacccgcat acagaacctc ccggggcaaa cccctgttca ccacctgcct 31535
   gtaccaggga aacctcacat ttatcaggga gccatagata gccattttaa accaattagc 31595
   taacaccgcc ccaccagctc tacactgaag agaaccggga gagttacaat gacagtgaat 31655
   aatccatctc tcataacccc taatggtctg atggaaatcc agatctaacg tggcacagca 31715
   gatacacact ttcatataca ttttcatcac atgtttttcc caggccgtta aaatacaatc 31775
   ccaatacacg ggccactcct gcagtacaat aaagctaata caagatggta tactcctcac 31835
   ctcactaaca ttgtgcatgt tcatattttc acattctaag taccgagagt tctcctctac 31895
   aacagcactg ccgcggtcct cacaaggtgg tagctggtga cgattgtaag gagccagtct 31955
   gcagcgatac cgtctgtcgc gttgcatcgt agaccaggga ccgacgcact tcctcgtact 32015
   tgtagtagca gaaccacgtc cgctgccagc acgtctccaa gtaacgccgg tccctgcgtc 32075
   gctcacgctc cctcctcaac gcaaagtgca accactcttg taatccacac agatccctct 32135
   cggcctccgg ggcgatgcac acctcaaacc tacagatgtc tcggtacagt tccaaacacg 32195
   tagtgagggc gagttccaac caagacagac agcctgatct atcccgacac actggaggtg 32255
   gaggaagaca cggaagaggc atgttattcc aagcgattca ccaacgggtc gaaatgaaga 32315
   tcccgaagat gacaacggtc gcctccggag ccctgatgga atttaacagc cagatcaaac 32375
   attatgcgat tttccaggct atcaatcgcg gcctccaaaa gagcctggac ccgcacttcc 32435
   acaaacacca gcaaagcaaa agcgttatta tcaaactctt cgatcatcaa gctgcaggac 32495
   tgtacaatgc ccaagtaatt ttcatttctc cactcgcgaa tgatgtcgcg gcaaatagtc 32555
   tgaaggttca tgccgtgcat attaaaaagc tccgaaaggg cgccctctat agccatgcgt 32615
   agacacacca tcatgactgc aagatatcgg gctcctgaga cacctgcagc agatttaaca 32675
   gacccaggtc aggttgctct ccgcgatcgc gaatctccat ccgcaaagtc atttgcaaat 32735
   aattaaatag atctgcgccg actaaatctg ttaactccgc gctaggaact aaatcaggtg 32795
   tggctacgca gcacaaaagt tccagggatg gcgccaaact cactagaacc gctcccgagt 32855
   agcaaaactg atgaatggga gtaacacagt gtaaaatgtt cagccaaaaa tcactaagct 32915
   gctcctttaa aaagtccagt acttctatat tcagttcgtg caagtactga agcaactgtg 32975
   cgggaatatg cacagcaaaa aaaatagggc ggctcagata catgttgacc taaaataaaa 33035
   agaatcatta aactaaagaa gcctggcgaa cggtgggata tatgacacgc tccagcagca 33095
   ggcaagcaac cggctgtccc cgggaaccgc ggtaaaattc atccgaatga ttaaaaagaa 33155
   caacagagac ttcccaccat gtactcggtt ggatctcctg agcacagagc aatacccccc 33215
   tcacattcat atccgctaca gaaaaaaaac gtcccagata cccagcggga atatccaacg 33275
   acagctgcaa agacagcaaa acaatccctc tgggagcaat cacaaaatcc tccggtgaaa 33335
   aaagcacata catattagaa taaccctgtt gctggggcaa aaaggcccgt cgtcccagca 33395
   aatgcacata aatatgttca tcagccattg ccccgtctta ccgcgtaaac agccacgaaa 33455
   aaatcgagct aaaatccacc caacagccta tagctatata tacactccac ccaatgacgc 33515
   taataccgca ccacccacga ccaaagttca cccacaccca caaaacccgc gaaaatccag 33575
   cgccgtcagc acttccgcaa tttcagtctc acaacgtcac ttccgcgcgc cttttcactt 33635
   tcccacacac gcccttcgcc cgcccgccct cgcgccaccc cgcgtcaccc cacgtcaccg 33695
   cacgtcaccc cggccccgcc tcgctcctcc ccgctcatta tcatattggc acgtttccag 33755
   aataaggtat attattgatg cagcaaaaca atccctctgg gagcaatcac aaaatcctcc 33815
   ggtgaaaaaa gcacatacat attagaataa ccctgttgct ggggcaaaaa ggcccgtcgt 33875
   cccagcaaat gcacataaat atgttcatca gccattgccc cgtcttaccg cgtaaacagc 33935
   cacgaaaaaa tcgagctaaa atccacccaa cagcctatag ctatatatac actccaccca 33995
   atgacgctaa taccgcacca cccacgacca aagttcaccc acacccacaa aacccgcgaa 34055
   aatccagcgc cgtcagcact tccgcaattt cagtctcaca acgtcacttc cgcgcgcctt 34115
   ttcactttcc cacacacgcc cttcgcccgc ccgccctcgc gccaccccgc gtcaccccac 34175
   gtcaccgcac gtcaccccgg ccccgcctcg ctcctccccg ctcattatca tattggcacg 34235
   tttccagaat aaggtatatt attgatgca 34264

<210> 25

<211> 503

<212> PRT

<213> simian adenovirus SV-1

<400> 25

Met Arg Arg Ala Val Arg Val Thr Pro Ala Ala Tyr Glu Gly Pro Pro
1               5                   10                  15

Pro Ser Tyr Glu Ser Val Met Gly Ser Ala Asn Val Pro Ala Thr Leu
            20                  25                  30

Glu Ala Pro Tyr Val Pro Pro Arg Tyr Leu Gly Pro Thr Glu Gly Arg
        35                  40                  45

Asn Ser Ile Arg Tyr Ser Glu Leu Ala Pro Leu Tyr Asp Thr Thr Lys
    50                  55                  60

Val Tyr Leu Val Asp Asn Lys Ser Ala Asp Ile Ala Ser Leu Asn Tyr
65                  70                  75                  80

Gln Asn Asp His Ser Asn Phe Leu Thr Thr Val Val Gln Asn Asn Asp
                85                  90                  95

Phe Thr Pro Thr Glu Ala Gly Thr Gln Thr Ile Asn Phe Asp Glu Arg
            100                 105                 110

Ser Arg Trp Gly Gly Gln Leu Lys Thr Ile Leu His Thr Asn Met Pro
        115                 120                 125

Asn Ile Asn Glu Phe Met Ser Thr Asn Lys Phe Arg Ala Arg Leu Met
    130                 135                 140

Val Lys Lys Ala Glu Asn Gln Pro Pro Glu Tyr Glu Trp Phe Glu Phe
145                 150                 155                 160

Thr Ile Pro Glu Gly Asn Tyr Ser Glu Thr Met Thr Ile Asp Leu Met
                165                 170                 175

Asn Asn Ala Ile Val Asp Asn Tyr Leu Gln Val Gly Arg Gln Asn Gly
            180                 185                 190

Val Leu Glu Ser Asp Ile Gly Val Lys Phe Asp Thr Arg Asn Phe Arg
        195                 200                 205

Leu Gly Trp Asp Pro Val Thr Lys Leu Val Met Pro Gly Val Tyr Thr
    210                 215                 220

Asn Glu Ala Phe His Pro Asp Ile Val Leu Leu Pro Gly Cys Gly Val
225                 230                 235                 240

Asp Phe Thr Gln Ser Arg Leu Ser Asn Leu Leu Gly Ile Arg Lys Arg
                245                 250                 255

Arg Pro Phe Gln Glu Gly Phe Gln Ile Met Tyr Glu Asp Leu Glu Gly
            260                 265                 270

Gly Asn Ile Pro Gly Leu Leu Asp Val Pro Ala Tyr Glu Glu Ser Val
        275                 280                 285

Lys Gln Ala Glu Ala Gln Gly Arg Glu Ile Arg Gly Asp Thr Phe Ala
    290                 295                 300

Thr Glu Pro His Glu Leu Val Ile Lys Pro Leu Glu Gln Asp Ser Lys
305                 310                 315                 320

Lys Arg Ser Tyr Asn Ile Ile Ser Gly Thr Met Asn Thr Leu Tyr Arg
                325                 330                 335

Ser Trp Phe Leu Ala Tyr Asn Tyr Gly Asp Pro Glu Lys Gly Val Arg
            340                 345                 350

Ser Trp Thr Ile Leu Thr Thr Thr Asp Val Thr Cys Gly Ser Gln Gln
        355                 360                 365

Val Tyr Trp Ser Leu Pro Asp Met Met Gln Asp Pro Val Thr Phe Arg
    370                 375                 380

Pro Ser Thr Gln Val Ser Asn Phe Pro Val Val Gly Thr Glu Leu Leu
385                 390                 395                 400

Pro Val His Ala Lys Ser Phe Tyr Asn Glu Gln Ala Val Tyr Ser Gln
                405                 410                 415

Leu Ile Arg Gln Ser Thr Ala Leu Thr His Val Phe Asn Arg Phe Pro
            420                 425                 430

Glu Asn Gln Ile Leu Val Arg Pro Pro Ala Pro Thr Ile Thr Thr Val
        435                 440                 445

Ser Glu Asn Val Pro Ala Leu Thr Asp His Gly Thr Leu Pro Leu Arg
    450                 455                 460

Ser Ser Ile Ser Gly Val Gln Arg Val Thr Ile Thr Asp Ala Arg Arg
465                 470                 475                 480

Arg Thr Cys Pro Tyr Val Tyr Lys Ala Leu Gly Val Val Ala Pro Lys
                485                 490                 495

Val Leu Ser Ser Arg Thr Phe
            500

<210> 26

<211> 931

<212> PRT

<213> simian adenovirus SV-1


<400> 26

Met Ala Thr Pro Ser Met Met Pro Gln Trp Ser Tyr Met His Ile Ala
1               5                   10                  15

Gly Gln Asp Ala Ser Glu Tyr Leu Ser Pro Gly Leu Val Gln Phe Ala
            20                  25                  30

Arg Ala Thr Asp Thr Tyr Phe Ser Leu Gly Asn Lys Phe Arg Asn Pro
        35                  40                  45

Thr Val Ala Pro Thr His Asp Val Thr Thr Asp Arg Ser Gln Arg Leu
    50                  55                  60

Thr Leu Arg Phe Val Pro Val Asp Arg Glu Asp Thr Ala Tyr Ser Tyr
65                  70                  75                  80

Lys Val Arg Tyr Thr Leu Ala Val Gly Asp Asn Arg Val Leu Asp Met
                85                  90                  95

Ala Ser Thr Tyr Phe Asp Ile Arg Gly Val Leu Asp Arg Gly Pro Ser
            100                 105                 110

Phe Lys Pro Tyr Ser Gly Thr Ala Tyr Asn Ser Leu Ala Pro Lys Gly
        115                 120                 125

Ala Pro Asn Pro Ala Glu Trp Thr Asn Ser Asp Ser Lys Val Lys Val
    130                 135                 140

Arg Ala Gln Ala Pro Phe Val Ser Ser Tyr Gly Ala Thr Ala Ile Thr
145                 150                 155                 160

Lys Glu Gly Ile Gln Val Gly Val Thr Leu Thr Asp Ser Gly Ser Thr
                165                 170                 175

Pro Gln Tyr Ala Asp Lys Thr Tyr Gln Pro Glu Pro Gln Ile Gly Glu
            180                 185                 190

Leu Gln Trp Asn Ser Asp Val Gly Thr Asp Asp Lys Ile Ala Gly Arg
        195                 200                 205

Val Leu Lys Lys Thr Thr Pro Met Phe Pro Cys Tyr Gly Ser Tyr Ala
    210                 215                 220

Arg Pro Thr Asn Glu Lys Gly Gly Gln Ala Thr Pro Ser Ala Ser Gln
225                 230                 235                 240

Asp Val Gln Asn Pro Glu Leu Gln Phe Phe Ala Ser Thr Asn Val Ala
                245                 250                 255

Asn Thr Pro Lys Ala Val Leu Tyr Ala Glu Asp Val Ser Ile Glu Ala

            260                 265                 270

Pro Asp Thr His Leu Val Phe Lys Pro Thr Val Thr Glu Gly Ile Thr
        275                 280                 285

Ser Ser Glu Ala Leu Leu Thr Gln Gln Ala Ala Pro Asn Arg Pro Asn
    290                 295                 300

Tyr Ile Ala Phe Arg Asp Asn Phe Ile Gly Leu Met Tyr Tyr Asn Ser
305                 310                 315                 320

Thr Gly Asn Met Gly Val Leu Ala Gly Gln Ala Ser Gln Leu Asn Ala
                325                 330                 335

Val Val Asp Leu Gln Asp Arg Asn Thr Glu Leu Ser Tyr Gln Leu Met
            340                 345                 350

Leu Asp Ala Leu Gly Asp Arg Ser Arg Tyr Phe Ser Met Trp Asn Gln
        355                 360                 365

Ala Val Asp Ser Tyr Asp Pro Asp Val Arg Ile Ile Glu Asn His Gly
    370                 375                 380

Val Glu Asp Glu Leu Pro Asn Tyr Cys Phe Pro Leu Gly Gly Met Ala
385                 390                 395                 400

Val Thr Asp Thr Tyr Ser Pro Ile Lys Val Asn Gly Gly Gly Asn Gly
                405                 410                 415

Trp Glu Ala Asn Asn Gly Val Phe Thr Glu Arg Gly Val Glu Ile Gly
            420                 425                 430

Ser Gly Asn Met Phe Ala Met Glu Ile Asn Leu Gln Ala Asn Leu Trp
        435                 440                 445

Arg Ser Phe Leu Tyr Ser Asn Ile Gly Leu Tyr Leu Pro Asp Ser Leu
    450                 455                 460

Lys Ile Thr Pro Asp Asn Ile Thr Leu Pro Glu Asn Lys Asn Thr Tyr
465                 470                 475                 480

Gln Tyr Met Asn Gly Arg Val Thr Pro Pro Gly Leu Val Asp Thr Tyr
                485                 490                 495

Val Asn Val Gly Ala Arg Trp Ser Pro Asp Val Met Asp Ser Ile Asn
            500                 505                 510

Pro Phe Asn His His Arg Asn Ala Gly Leu Arg Tyr Arg Ser Met Leu
        515                 520                 525

Leu Gly Asn Gly Arg Tyr Val Pro Phe His Ile Gln Val Pro Gln Lys
    530                 535                 540

Phe Phe Ala Ile Lys Asn Leu Leu Leu Leu Pro Gly Ser Tyr Thr Tyr
545                 550                 555                 560

Glu Trp Asn Phe Arg Lys Asp Val Asn Met Ile Leu Gln Ser Ser Leu
                565                 570                 575

Gly Asn Asp Leu Arg Val Asp Gly Ala Ser Ile Arg Phe Asp Ser Ile
            580                 585                 590

Asn Leu Tyr Ala Asn Phe Phe Pro Met Ala His Asn Thr Ala Ser Thr
        595                 600                 605

Leu Glu Ala Met Leu Arg Asn Asp Thr Asn Asp Gln Ser Phe Asn Asp
    610                 615                 620

Tyr Leu Cys Ala Ala Asn Met Leu Tyr Pro Ile Pro Ala Asn Ala Thr
625                 630                 635                 640

Ser Val Pro Ile Ser Ile Pro Ser Arg Asn Trp Ala Ala Phe Arg Gly
                645                 650                 655

Trp Ser Phe Thr Arg Leu Lys Thr Lys Glu Thr Pro Ser Leu Gly Ser
            660                 665                 670

Gly Phe Asp Pro Tyr Phe Val Tyr Ser Gly Ser Ile Pro Tyr Leu Asp
        675                 680                 685

Gly Thr Phe Tyr Leu Asn His Thr Phe Lys Lys Val Ser Ile Met Phe
    690                 695                 700

Asp Ser Ser Val Ser Trp Pro Gly Asn Asp Arg Leu Leu Thr Pro Asn
705                 710                 715                 720

Glu Phe Glu Ile Lys Arg Ser Val Asp Gly Glu Gly Tyr Asn Val Ala
                725                 730                 735

Gln Ser Asn Met Thr Lys Asp Trp Phe Leu Ile Gln Met Leu Ser His
            740                 745                 750

Tyr Asn Ile Gly Tyr Gln Gly Phe Tyr Val Pro Glu Asn Tyr Lys Asp
        755                 760                 765

Arg Met Tyr Ser Phe Phe Arg Asn Phe Gln Pro Met Ser Arg Gln Ile
    770                 775                 780

Val Asp Ser Thr Ala Tyr Thr Asn Tyr Gln Asp Val Lys Leu Pro Tyr
785                 790                 795                 800

Gln His Asn Asn Ser Gly Phe Val Gly Tyr Met Gly Pro Thr Met Arg
                805                 810                 815

Glu Gly Gln Ala Tyr Pro Ala Asn Tyr Pro Tyr Pro Leu Ile Gly Ala
            820                 825                 830

Thr Ala Val Pro Ser Leu Thr Gln Lys Lys Phe Leu Cys Asp Arg Val
        835                 840                 845

Met Trp Arg Ile Pro Phe Ser Ser Asn Phe Met Ser Met Gly Ser Leu
    850                 855                 860

Thr Asp Leu Gly Gln Asn Met Leu Tyr Ala Asn Ser Ala His Ala Leu
865                 870                 875                 880

Asp Met Thr Phe Glu Val Asp Pro Met Asp Glu Pro Thr Leu Leu Tyr
                885                 890                 895

Val Leu Phe Glu Val Phe Asp Val Val Arg Ile His Gln Pro His Arg
            900                 905                 910

Gly Val Ile Glu Ala Val Tyr Leu Arg Thr Pro Phe Ser Ala Gly Asn
        915                 920                 925

Ala Thr Thr
    930

<210>  27

<211>  363

<212>  PRT

<213>  simian adenovirus SV-1


<400>  27
   Met Lys Arg Thr Arg Val Asp Glu Asp Phe Asn Pro Val Tyr Pro Tyr
   1               5                   10                  15
   Asp Thr Thr Thr Thr Pro Ala Val Pro Phe Ile Ser Pro Pro Phe Val
               20                  25                  30
   Asn Ser Asp Gly Leu Gln Glu Asn Pro Pro Gly Val Leu Ser Leu Arg
           35                  40                  45
   Ile Ala Lys Pro Leu Tyr Phe Asp Met Glu Arg Lys Leu Ala Leu Ser
       50                  55                  60
   Leu Gly Arg Gly Leu Thr Ile Thr Ala Ala Gly Gln Leu Glu Ser Thr
   65                  70                  75                  80
   Gln Ser Val Gln Thr Asn Pro Pro Leu Ile Ile Thr Asn Asn Asn Thr
                   85                  90                  95
   Leu Thr Leu Arg His Ser Pro Pro Leu Asn Leu Thr Asp Asn Ser Leu
               100                 105                 110
   Val Leu Gly Tyr Ser Ser Pro Leu Arg Val Thr Asp Asn Lys Leu Thr
           115                 120                 125
   Phe Asn Phe Thr Ser Pro Leu Arg Tyr Glu Asn Glu Asn Leu Thr Phe
       130                 135                 140
   Asn Tyr Thr Glu Pro Leu Lys Leu Ile Asn Asn Ser Leu Ala Ile Asp
   145                 150                 155                 160
   Ile Asn Ser Ser Lys Gly Leu Ser Ser Val Gly Gly Ser Leu Ala Val
                   165                 170                 175
   Asn Leu Ser Ser Asp Leu Lys Phe Asp Ser Asn Gly Ser Ile Ala Phe
               180                 185                 190
   Gly Ile Gln Thr Leu Trp Thr Ala Pro Thr Ser Thr Gly Asn Cys Thr
           195                 200                 205
   Val Tyr Ser Glu Gly Asp Ser Leu Leu Ser Leu Cys Leu Thr Lys Cys
       210                 215                 220
   Gly Ala His Val Leu Gly Ser Val Ser Leu Thr Gly Leu Thr Gly Thr
   225                 230                 235                 240
   Ile Thr Gln Met Thr Asp Ile Ser Val Thr Ile Gln Phe Thr Phe Asp
                   245                 250                 255
   Asn Asn Gly Lys Leu Leu Ser Ser Pro Leu Ile Asn Asn Ala Phe Ser
               260                 265                 270
   Ile Arg Gln Asn Asp Ser Thr Ala Ser Asn Pro Thr Tyr Asn Ala Leu
   275                 280                 285
   Ala Phe Met Pro Asn Ser Thr Ile Tyr Ala Arg Gly Gly Gly Gly Glu
       290                 295                 300
   Pro Arg Asn Asn Tyr Tyr Val Gln Thr Tyr Leu Arg Gly Asn Val Gln
   305                 310                 315                 320
   Lys Pro Ile Ile Leu Thr Val Thr Tyr Asn Ser Val Ala Thr Gly Tyr
                   325                 330                 335
   Ser Leu Ser Phe Lys Trp Thr Ala Leu Ala Arg Glu Lys Phe Ala Thr
               340                 345                 350
   Pro Thr Thr Ser Phe Cys Tyr Ile Thr Glu Gln
           355                 360

<210>  28

<211>  560

<212>  PRT

<213>  simian adenovirus SV-1

<400>  28

Met Lys Arg Ala Arg Val Asp Glu Asp Phe Asn Pro Val Tyr Pro Tyr
1               5                   10                  15

Asp Pro Pro His Ala Pro Val Met Pro Phe Ile Thr Pro Pro Phe Thr
            20                  25                  30

Ser Ser Asp Gly Leu Gln Glu Lys Pro Leu Gly Val Leu Ser Leu Asn
        35                  40                  45

Tyr Arg Asp Pro Ile Thr Thr Gln Asn Glu Ser Leu Thr Ile Lys Leu
    50                  55                  60

Gly Asn Gly Leu Thr Leu Asp Asn Gln Gly Gln Leu Thr Ser Thr Ala
65                  70                  75                  80

Gly Glu Val Glu Pro Pro Leu Thr Asn Ala Asn Asn Lys Leu Ala Leu
                85                  90                  95

Val Tyr Ser Asp Pro Leu Ala Val Lys Arg Asn Ser Leu Thr Leu Ser
            100                 105                 110

His Thr Ala Pro Leu Val Ile Ala Asp Asn Ser Leu Ala Leu Gln Val
        115                 120                 125

Ser Glu Pro Ile Phe Ile Asn Asp Lys Asp Lys Leu Ala Leu Gln Thr
    130                 135                 140

Ala Ala Pro Leu Val Thr Asn Ala Gly Thr Leu Arg Leu Gln Ser Ala
145                 150                 155                 160

Ala Pro Leu Gly Ile Ala Asp Gln Thr Leu Lys Leu Leu Phe Thr Asn
                165                 170                 175

Pro Leu Tyr Leu Gln Asn Asn Phe Leu Thr Leu Ala Ile Glu Arg Pro
            180                 185                 190

Leu Ala Ile Thr Asn Thr Gly Lys Leu Ala Leu Gln Leu Ser Pro Pro
        195                 200                 205

Leu Gln Thr Ala Asp Thr Gly Leu Thr Leu Gln Thr Asn Val Pro Leu
    210                 215                 220

Thr Val Ser Asn Gly Thr Leu Gly Leu Ala Ile Lys Arg Pro Leu Ile
225                 230                 235                 240

Ile Gln Asp Asn Asn Leu Phe Leu Asp Phe Arg Ala Pro Leu Arg Leu
                245                 250                 255

Phe Asn Ser Asp Pro Val Leu Gly Leu Asn Phe Tyr Thr Pro Leu Ala
            260                 265                 270

Val Arg Asp Glu Ala Leu Thr Val Asn Thr Gly Arg Gly Leu Thr Val
        275                 280                 285

Ser Tyr Asp Gly Leu Ile Leu Asn Leu Gly Lys Asp Leu Arg Phe Asp
    290                 295                 300

Asn Asn Thr Val Ser Val Ala Leu Ser Ala Ala Leu Pro Leu Gln Tyr
305                 310                 315                 320

Thr Asp Gln Leu Arg Leu Asn Val Gly Ala Gly Leu Arg Tyr Asn Pro
                325                 330                 335

Val Ser Lys Lys Leu Asp Val Asn Pro Asn Gln Asn Lys Gly Leu Thr
            340                 345                 350

Trp Glu Asn Asp Tyr Leu Ile Val Lys Leu Gly Asn Gly Leu Gly Phe
        355                 360                 365

Asp Gly Asp Gly Asn Ile Ala Val Ser Pro Gln Val Thr Ser Pro Asp
    370                 375                 380

Thr Leu Trp Thr Thr Ala Asp Pro Ser Pro Asn Cys Ser Ile Tyr Thr
385                 390                 395                 400

Asp Leu Asp Ala Lys Met Trp Leu Ser Leu Val Lys Gln Gly Gly Val
                405                 410                 415

Val His Gly Ser Val Ala Leu Lys Ala Leu Lys Gly Thr Leu Leu Ser
            420                 425                 430

Pro Thr Glu Ser Ala Ile Val Ile Ile Leu His Phe Asp Asn Tyr Gly
        435                 440                 445

Val Arg Ile Leu Asn Tyr Pro Thr Leu Gly Thr Gln Gly Thr Leu Gly
    450                 455                 460

Asn Asn Ala Thr Trp Gly Tyr Arg Gln Gly Glu Ser Ala Asp Thr Asn
465                 470                 475                 480

Val Leu Asn Ala Leu Ala Phe Met Pro Ser Ser Lys Arg Tyr Pro Arg
                485                 490                 495

Gly Arg Gly Ser Glu Val Gln Asn Gln Thr Val Gly Tyr Thr Cys Ile
            500                 505                 510

Gln Gly Asp Phe Ser Met Pro Val Pro Tyr Gln Ile Gln Tyr Asn Tyr
        515                 520                 525

Gly Pro Thr Gly Tyr Ser Phe Lys Phe Ile Trp Arg Thr Val Ser Arg
    530                 535                 540

Gln Pro Phe Asp Ile Pro Cys Cys Phe Phe Ser Tyr Ile Thr Glu Glu
545                 550                 555                 560

<210>  29

<211>  31044

<212>  DNA

<213>  simian adenovirus SV-25


<220>

<221>  CDS

<222>  (12284)..(13801)
<223>  Penton

<220>
<221>  CDS
<222>  (16681)..(19446)
<223>  Hexon

<220>
<221>  CDS
<222>  (25380)..(26423)
<223>  Fiber #2

<220>
<221>  CDS
<222>  (26457)..(28136)
<223>  Fiber #1

<400>  29
catcatcaat aatatacctt attctggaaa cgtgccaata tgataatgag cggggaggag      60
cgaggcgggg ccggggtgac gtgcggtgac gcggggtggc gcgagggcgg ggcgaagggc     120
gcgggtgtgt gtgtgggagg cgcttagttt ttacgtatgc ggaaggaggt tttataccgg     180
aagatgggta atttgggcgt atacttgtaa gttttgtgta atttggcgcg aaaactgggt     240
aatgaggaag ttgaggttaa tatgtacttt ttatgactgg gcggaatttc tgctgatcag     300
cagtgaactt tgggcgctga cggggaggtt tcgctacgtg acagtaccac gagaaggctc     360
aaaggtccca tttattgtac tcttcagcgt tttcgctggg tatttaaacg ctgtcagatc     420
atcaagaggc cactcttgag tgctggcgag aagagttttc tcctccgtgc tgccacgatg     480
aggctggtcc ccgagatgta cggtgttttt agcgacgaga cggtgcgtaa ctcagatgac     540
ctgctgaatt cagacgcgct ggaaatttcc aattcgcctg tgctttcgcc gccgtcactt     600
cacgacctgt ttgtgttttg gctcaacgct tagcaacgtg ttatataggg tcaagaagga     660
gcaggagacg cagtttgcta ggctgttggc cgatactcct ggagtttttg tggctctgga     720
tctaggccat cactctcttt tccaagagaa aattatcaaa aacttaactt ttacgtctcc     780

tggtcgcacg gttgcttccg ctgcctttat tacctatatt ttggatcaat ggagcaacag 840
cgacagccac ctgtcgtggg agtacatgct ggattacatg tcgatggcgc tgtggagggc 900
catgctgcgg aggagggttt gcatttactt gcgggcgcag cctccgcggc tggaccgagt 960
ggaggaggag gacgagccgg gggagaccga gaacctgagg gccgggctgg accctccaac 1020
ggaggactag gtgctgagga tgatcccgaa gaggggacta gtggggctag gaagaagcaa 1080
aagactgagt ctgaacctcg aaactttttg aatgagttga ctgtgagttt gatgaatcgt 1140
cagcgtccgg agacaatttt ctggtctgaa ttggaggagg aattcaggag gggggaactg 1200
aacctgctat acaagtatgg gtttgaacag ttaaaaactc actggttgga gccgtgggag 1260
gattttgaaa ccgccttgga cacttttgct aaagtggctc tgcggccgga taaggtttac 1320
actatccgcc gcactgttaa cataaagaag agtgtttatg ttataggcca tggagctctg 1380
gtgcaggtgc aaaccgtcga ccgggtggcc tttagttgcg gtatgcaaaa tctgggcccc 1440
ggggtgatag gcttaaatgg tgtaacattt cacaatgtaa ggtttactgg tgaaagtttt 1500
aacggctctg tgtttgcaaa taacacacag ctgacgctcc acggcgttta cttttttaac 1560
tttaataaca catgtgtgga gtcgtggggc agggtgtctt tgaggggctg ctgttttcac 1620
ggctgctgga aggcggtggt gggaagactt aaaagtgtaa catctgtaaa aaaatgcgtg 1680
tttgagcggt gtgtgttggc tttaactgtg gagggctgtg gacgcattag gaataatgcg 1740
gcgtctgaga atggatgttt tcttttgcta aaaggcacgg ctagtattaa gcataacatg 1800
atatgcggca gcggtctgta cccttcacag ctgttaactt gcgcggatgg aaactgtcag 1860
accttgcgca ccgtgcacat agcgtcccac cagcgccgcg cctggccaac attcgagcac 1920
aatatgctta tgcgttgtgc cgtccacttg ggccctaggc gaggcgtgtt tgtgccttac 1980
cagtgtaact ttagccatac caagatttta ctagaacctg ataccttctc tcgagtgtgt 2040
ttcaatgggg tgtttgacat gtcaatggaa ctgtttaaag tgataagata tgatgaatcc 2100
aagtctcgtt gtcgcccatg tgaatgcgga gctaatcatc tgaggttgta tcctgtaacc 2160
ctaaacgtta ccgaggagct gaggacggat caccacatgt tgtcctgcct gcgcaccgac 2220
tatgaatcca gcgacgagga gtgaggtgag gggcggagcc acaaagggta taaaggggcg 2280
tgaggggtgg gtgtgatgat tcaaaatgag cgggacgacg gacggcaacg cgtttgaggg 2340
tggagtgttc agcccttatc tgacatctcg tcttccttcc tgggcaggag tgcgtcagaa 2400
tgtagtgggc tccaccgtgg acggacgacc ggtcgcccct gcaaattccg ccaccctcac 2460
ctatgccacc gtgggatcat cgttggacac tgccgcggca gctgccgctt ctgctgccgc 2520
ttctactgct cgcggcatgg cggctgattt tggactgtat aaccaactgg ccactgcagc 2580
tgtggcgtct cggtctctgg ttcaagaaga tgccctgaat gtgatcctga ctcgcctgga 2640
gatcatgtca cgtcgcttgg acgaactggc tgcgcagata tcccaagcta accccgatac 2700

cacttcagaa tcctaaaata aagacaaaca aatatgttga aaagtaaaat ggctttattt 2760
gttttttttg gctcggtagg ctcgggtcca cctgtctcgg tcgttaagaa ctttgtgtat 2820
gttttccaaa acacggtaca gatgggcttg gatgttcaag tacatgggca tgaggccatc 2880
tttggggtga agataggacc attgaagagc gtcatgctcc ggggtggtgt tgtaaattac 2940
ccagtcgtag cagggtttct gggcgtggaa ctggaagatg tcctttagga gtaggctgat 3000
ggccaagggc aggcccttag tgtaggtgtt tacaaagcgg ttaagctggg agggatgcat 3060
gcggggggag atgatatgca tcttggcttg gatcttgagg ttagctatgt taccacccag 3120
gtctctgcgg gggttcatgt tatgaaggac caccagcacg gtgtagccgg tgcatttggg 3180
gaacttgtca tgcagtttgg aggggaaggc gtggaagaat ttagagaccc ccttgtggcc 3240
ccctaggttt tccatgcact catccataat gatggcaatg ggacccctgg cggccgcttt 3300
ggcaaacacg ttttgggggt tggaaacatc atagttttgc tctagagtga gctcatcata 3360
ggccatctta acaaagcggg gtaggagggt gcccgactgg gggatgatag ttccatctgg 3420
gcctggggcg tagttaccct cacagatctg catctcccag gccttaattt ccgagggggg 3480
tatcatgtcc acctgggggg caataaagaa cacggtttct ggcgggggat tgatgagctg 3540
ggtggaaagc aagttacgca gcagttgaga tttgccacag ccggtggggc cgtagatgac 3600
cccgatgacg ggttgcagct ggtagttgag agaggaacag ctgccgtcgg ggcgcaggag 3660
gggggctacc tcattcatca tgcttctaac atgtttattt tcactcacta agttttgcaa 3720
gagcctctcc ccacccaggg ataagagttc ttccaggctg ttgaagtgtt tcagcggttt 3780
taggccgtcg gccatgggca tcttttcgag cgactgacga agcaagtaca gtcggtccca 3840
gagctcggtg acgtgctcta tggaatctcg atccagcaga cttcttggtt gcgggggttg 3900
ggtcgacttt cgctgtaggg caccagccgg tgggcgtcca gggccgcgag ggttctgtcc 3960
ttccagggtc tcagcgtccg ggtgagggtg gtctcggtga cggtgaaggg atgagccccg 4020
ggctgggcgc ttgcgagggt gcgcttcagg ctcatcctgc tggtgctgaa gcggacgtcg 4080
tctccctgtg agtcggccag atagcaacga agcatgaggt cgtagctgag ggactcggcc 4140
gcgtgtccct tggcgcgcag ctttcccttg gaaacgtgct gacatttggt gcagtgcaga 4200
cattggaggg cgtagagttt gggggccagg aagaccgact cgggcgagta ggcgtcggct 4260
ccgcactgag cgcagacggt ctcgcactcc actagccacg tgagctcggg tttagcggga 4320
tcaaaaacca agttgcctcc attttttttg atgcgtttct taccttgcgt ttccatgagt 4380
ttgtggcccg cttccgtgac aaaaaggctg tcggtgtctc cgtagacaga cttgaggggg 4440
cgatcttcca aaggtgttcc gaggtcttcc gcgtacagga actgggacca ctccgagacg 4500
aaggctctgg tccaggctaa cacgaaggag gcaatctgcg aggggtatct gtcgttttca 4560

atgagggggt ccaccttttc cagggtgtgc agacacaggt cgtcctcctc cgcgtccacg 4620
aaggtgattg gcttgtaagt gtaggtcacg tgatctgcac cccccaaagg ggtataaaag 4680
ggggcgtgcc caccctctcc gtcactttct tccgcatcgc tgtggaccag agccagctgt 4740
tcgggtgagt aggccctctc aaaagccggc atgatctcgg cgctcaagtt gtcagtttct 4800
acaaacgagg tggatttgat attcacgtgc cccgcggcga tgcttttgat ggtggagggg 4860
tccatctgat cagaaaacac gatctttttg ttgtcaagtt tggtggcgaa agacccgtag 4920
agggcgttgg aaagcaactt ggcgatggag cgcagggtct gatttttctc ccgatcggcc 4980
ctctccttgg cggcgatgtt gagttgcacg tactcccggg ccgcgcaccg ccactcgggg 5040
aacacggcgg tgcgctcgtc gggcaggatg cgcacgcgcc agccgcgatt gtgcagggtg 5100
atgaggtcca cgctggtagc cacctccccg cggaggggct cgttggtcca acacaatcgc 5160
cccccttttc tggagcagaa cggaggcagg ggatctagca agttggcggg cggggggtcg 5220
gcgtcgatgg tgaagatacc gggtagcagg atcttattaa aataatcgat ttcggtgtcc 5280
gtgtcttgca acgcgtcttc ccacttcttc accgccaggg ccctttcgta gggattcagg 5340
ggcggtcccc agggcatggg gtgggtcagg gccgaggcgt acatgccgca gatgtcatac 5400
acgtacaggg gttccctcaa caccccgatg taagtggggt aacagcgccc cccgcggatg 5460
ctggctcgca cgtagtcgta catctcgcgc gagggagcca tgaggccgtc tcccaagtgg 5520
gtcttgtggg gtttttcggc ccggtagagg atctgtctga agatggcgtg ggagttggaa 5580
gagatggtgg ggcgttggaa gacgttaaag ttggccccgg gtagtcccac ggagtcttgg 5640
atgaactggg cgtaggattc ccggagtttg tccaccaggg cggcggtcac cagcacgtcg 5700
agagcgcagt agtccaacgt ctcgcggacc aggttgtagg ccgtctcttg ttttttctcc 5760
cacagttcgc ggttgaggag gtattcctcg cggtctttcc agtactcttc ggcgggaaat 5820
cctttttcgt ccgctcggta agaacctaac atgtaaaatt cgttcaccgc tttgtatgga 5880
caacagcctt tttctaccgg cagggcgtac gcttgagcgg cctttctgag agaggtgtgg 5940
gtgagggcga aggtgtcccg caccatcact ttcaggtact gatgtttgaa gtccgtgtcg 6000
tcgcaggcgc cctgttccca cagcgtgaag tcggtgcgct ttttctgcct gggattgggg 6060
agggcgaagg tgacatcgtt aaagagtatt ttcccggcgc ggggcatgaa gttgcgagag 6120
atcctgaagg gcccgggcac gtccgagcgg ttgttgatga cctgcgccgc caggacgatc 6180
tcgtcgaagc cgttgatgtt gtgacccacg atgtaaagtt cgatgaagcg cggctgtccc 6240
ttgagggccg gcgctttttt caactcctcg taggtgagac agtccggcga ggagagaccc 6300
agctcagccc gggcccagtc ggagagttga ggattagccg caaggaagga gctccataga 6360
tccaaggcca ggagagtttg caagcggtcg cggaactcgc ggaacttttt ccccacggcc 6420
attttctccg gtgtcactac gtaaaaggtg ttggggcggt tgttccacac gtcccatcgg 6480

agctctaggg ccagctcgca ggcttggcga acgagggtct cctcgccaga gacgtgcatg 6540
accagcataa agggtaccaa ctgtttcccg aacgagccca tccatgtgta ggtttctacg 6600
tcgtaggtga caaagagccg ctgggtgcgc gcgtgggagc cgatcggaaa gaagctgatc 6660
tcctgccacc agctggagga atgggtgtta atgtggtgga agtagaagtc ccgccggcgc 6720
acagagcatt cgtgctgatg tttgtaaaag cgaccgcagt agtcgcagcg ctgcacgctc 6780
tgtatctcct gaacgagatg cgcttttcgc ccgcgcacca gaaaccggag ggggaagttg 6840
agacgggggg ctggtggggc gacatcccct tcgccttggc ggtgggagtc tgcgtctgcg 6900
tcctccttct ctgggtggac gacggtgggg acgacgacgc cccgggtgcc gcaagtccag 6960
atctccgcca cggaggggtg caggcgctgc aggaggggac gcagctgccc gctgtccagg 7020
gagtcgaggg aagtcgcgct gaggtcggcg ggaagcgttt gcaagttcac tttcagaaga 7080
ccggtaagag cgtgagccag gtgcagatgg tacttgattt ccaggggggt gttggatgaa 7140
gcgtccacgg cgtagaggag tccgtgtccg cgcggggcca ccaccgtgcc ccgaggaggt 7200
tttatctcac tcgtcgaggg cgagcgccgg ggggtagagg cggctctgcg ccggggggca 7260
gcggaggcag aggcacgttt tcgtgaggat tcggcagcgg ttgatgacga gcccggagac 7320
tgctggcgtg ggcgacgacg cggcggttga ggtcctggat gtgccgtctc tgcgtgaaga 7380
ccaccggccc ccgggtcctg aacctaaaga gagttccaca gaatcaatgt ctgcatcgtt 7440
aacggcggcc tgcctgagga tctcctgcac gtcgcccgag ttgtcctgat aggcgatctc 7500
ggccatgaac tgttccactt cttcctcgcg gaggtcaccg tggcccgctc gctccacggt 7560
ggcggccagg tcgttggaga tgcggcgcat gagttgagag aaggcgttga ggccgttctc 7620
gttccacacg cggctgtaca ccacgtttcc gaaggagtcg cgcgctcgca tgaccacctg 7680
ggccacgttg agttccacgt ggcgggcgaa gacggcgtag tttctgaggc gctggaagag 7740
gtagttgagc gtggtggcga tgtgctcgca gacgaagaag tacataatcc agcgccgcag 7800
ggtcatctcg ttgatgtctc cgatggcttc gagacgctcc atggcctcgt agaagtcgac 7860
ggcgaagttg aaaaattggg agttgcgggc ggccaccgtg agttcttctt gcaggaggcg 7920
gatgagatcg gcgaccgtgt cgcgcacctc ctgttcgaaa gcgccccgag gcgcctctgc 7980
ttcttcctcc ggctcctcct cttccagggg ctcgggttcc tccggcagct ctgcgacggg 8040
gacggggcgg cgacgtcgtc gtctgaccgg caggcggtcc acgaagcgct cgatcatttc 8100
gccgcgccgg cgacgcatgg tctcggtgac ggcgcgtccg ttttcgcgag gtcgcagttc 8160
gaagacgccg ccgcgcagag cgcccccgtg cagggagggt aagtggttag ggccgtcggg 8220
cagggacacg gcgctgacga tgcattttat caattgctgc gtaggcactc cgtgcaggga 8280
tctgagaacg tcgaggtcga cgggatccga gaacttctct aggaaagcgt ctatccaatc 8340

gcaatcgcaa ggtaagctga gaacggtggg tcgctggggg gcgttcgcgg gcagttggga 8400
ggtgatgctg ctgatgatgt aattaaagta ggcggtcttc aggcggcgga tggtggcgag 8460
gaggaccacg tctttgggcc cggcctgttg aatgcgcagg cgctcggcca tgccccaggc 8520
ctcgctctga cagcgacgca ggtctttgta gaagtcttgc atcagtctct ccaccggaac 8580
ctctgcttct cccctgtctg ccatgcgagt cgagccgaac ccccgcaggg gctgcagcaa 8640
cgctaggtcg gccacgaccc tttcggccag cacggcctgt tgaatctgcg tgagggtggc 8700
ctggaagtcg tccaggtcca cgaagcggtg ataggccccc gtgttgatgg tgtaggtgca 8760
gttggccatg acggaccagt tgacgacttg catgccgggt tgggtgatct ccgtgtactt 8820
gaggcgcgag taggccctgg actcgaacac gtagtcgttg catgtgcgca ccagatactg 8880
gtagccgacc aggaagtgag gaggcggctc tcggtacagg ggccagccaa cggtggcggg 8940
ggcgccgggg gacaggtcgt ccagcatgag gcggtggtag tggtagatgt agcgggagag 9000
ccaggtgatg ccggccgagg tggttgcggc cctggtgaat tcgcggacgc ggttccagat 9060
gttgcgcagg ggaccaaagc gctccatggt gggcacgctc tgccccgtga ggcgggcgca 9120
atcttgtacg ctctagatgg aaaaaagaca gggcggtcat cgactccttt ccgtagcttg 9180
gggggtaaag tcgcaagggt gcggcggcgg ggaaccccgg ttcgagaccg gccggatccg 9240
ccgctcccga tgcgcctggc cccgcatcca cgacgtccgc gccgagaccc agccgcgacg 9300
ctccgcccca atacggaggg gagtcttttg gtgttttttc gtagatgcat ccggtgctgc 9360
ggcagatgcg accccagacg cccactacca ccgccgtggc ggcagtaaac ctgagcggag 9420
gcggtgacag ggaggaggaa gagctggctt tagacctgga agagggagag gggctggccc 9480
ggctgggagc gccatcccca gagagacacc ctagggttca gctcgtgagg gacgccaggc 9540
aggcttttgt gccgaagcag aacctgttta gggaccgcag cggtcaggag gcggaggaga 9600
tgcgcgattg caggtttcgg gcgggcagag agctcagggc gggcttcgat cgggagcggc 9660
tcctgagggc ggaggatttc gagcccgacg agcgttctgg ggtgagcccg gcccgcgctc 9720
acgtatcggc ggccaacctg gtgagcgcgt acgagcagac ggtgaacgag gagcgcaact 9780
tccaaaagag ctttaacaat cacgtgagga ccctgatcgc gagggaggag gtgaccatcg 9840
ggctgatgca tctgtgggac ttcgtggagg cctacgtgca gaacccggct agcaaacccc 9900
tgacggccca gctgttcctg atcgtgcagc acagccgcga caacgagacg ttccgcgacg 9960
ccatgttgaa catcgcggag cccgagggtc gctggctctt ggatctgatt aacatcctgc 10020
agagcatcgt ggtgcaggag aggggcctga gtttagcgga caaggtggcg gccattaact 10080
attcgatgca gagcctgggg aagttctacg ctcgcaagat ctacaagagc ccttacgtgc 10140
ccatagacaa ggaggtgaag atagacagct tttacatgcg catggcgctg aaggtgctga 10200
cgctgagcga cgacctcggc gtgtaccgta acgacaagat ccacaaggcg gtgagcgcca 10260

gccgccggcg ggagctgagc gacagggagc tgatgcacag cctgcagagg gcgctggcgg 10320
gcgccgggga cgaggagcgc gaggcttact tcgacatggg agccgatctg cagtggcgtc 10380
ccagcgcgcg cgccttggag gcggcgggtt atcccgacga ggaggatcgg gacgatttgg 10440
aggaggcagg cgagtacgag gacgaagcct gaccgggcag gtgttgtttt agatgcagcg 10500
gccggcggac gggaccaccg cggatcccgc acttttggca tccatgcaga gtcaaccttc 10560
gggcgtgacc gcctccgatg actgggcggc ggccatggac cgcatcatgg cgctgaccac 10620
ccgcaacccc gaggctttta ggcagcaacc ccaggccaac cgtttttcgg ccatcttgga 10680
agcggtggtg ccgtcgcgca ccaacccgac gcacgagaaa gtcctgacta tcgtgaacgc 10740
cctggtagac agcaaggcca tccgccgtga cgaggcgggc ttgatttaca acgctctttt 10800
ggaacgcgtg gcgcgctaca acagcactaa cgtgcagacc aatctggacc gcctcaccac 10860
cgacgtgaag gaggcgctgg cgcagaagga gcggtttctg agggacagta atctgggctc 10920
tctggtggca ctgaacgcct tcctgagctc acagccggcc aacgtgcccc gcgggcagga 10980
ggattacgtg agcttcatca gcgctctgag actgctggtg tccgaggtgc cccagagcga 11040
ggtgtaccag tctgggccgg attacttttt ccagacgtcc cgacagggct tgcaaacggt 11100
gaacctgact caggccttta aaaacttgca aggcatgtgg ggggtcaagg ccccggtggg 11160
cgatcgcgcc actatctcca gtctgctgac ccccaacact cgcctgctgc tgctcttgat 11220
cgcaccgttt accaacagta gcactatcag ccgtgactcg tacctgggtc atctcatcac 11280
tctgtaccgc gaggccatcg gccaggctca gatcgacgag catacgtatc aggagattac 11340
taacgtgagc cgtgccctgg gtcaggaaga taccggcagc ctggaagcca cgttgaactt 11400
tttgctaacc aaccggaggc aaaaaatacc ctcccagttc acgttaagcg ccgaggagga 11460
gaggattctg cgatacgtgc agcagtccgt gagcctgtac ttgatgcgcg agggcgccac 11520
cgcttccacg gctttagaca tgacggctcg gaacatggaa ccgtcctttt actccgccca 11580
ccggccgttc attaaccgtc tgatggacta cttccatcgc gcggccgcca tgaacgggga 11640
gtacttcacc aatgccatcc tgaatccgca ttggatgccc ccgtccggct tctacaccgg 11700
ggagtttgac ctgcccgaag ccgacgacgg ctttctgtgg gacgacgtgt ccgatagcat 11760
tttcacgccg gctaatcgcc gattccagaa gaaggagggc ggagacgagc tccccctctc 11820
cagcgtggaa gcggcctcaa ggggagagag tccctttcca agtctgtctt ccgccagtag 11880
cggtcgggta acgcgtccac ggttgccggg ggagagcgac tacctgaacg accccttgct 11940
gcgaccggct agaaagaaaa attttcccaa taacggggtg gaaagcttgg tggataaaat 12000
gaatcgttgg aagacgtacg cccaggagca gcgggagtgg gaggacagtc agccgcggcc 12060
gctggtaccg ccgcattggc gtcgccagag agaagacccg gacgactccg cagacgatag 12120

tagcgtgttg gacctgggag ggagcggagc caaccccttt gctcacttgc aacccaaggg 12180

gcgctcgagt cgcctgtatt aataaaaaag acgcggaaac ttaccagagc catggccaca 12240

gcgtgtgtgc tttcttcctc tctttcttcc tcggcgcggc aga atg aga aga gcg 12295
Met Arg Arg Ala
1

gtg aga gtc acg ccg gcg gcg tat gag ggc ccg ccc cct tct tac gaa 12343
Val Arg Val Thr Pro Ala Ala Tyr Glu Gly Pro Pro Pro Ser Tyr Glu
5                   10                  15                  20

agc gtg atg gga tca gcg aac gtg ccg gcc acg ctg gag gcg cct tac 12391
Ser Val Met Gly Ser Ala Asn Val Pro Ala Thr Leu Glu Ala Pro Tyr
                25                  30                  35

gtt cct ccc aga tac ctg gga cct acg gag ggc aga aac agc atc cgt 12439
Val Pro Pro Arg Tyr Leu Gly Pro Thr Glu Gly Arg Asn Ser Ile Arg
            40                  45                  50

tac tcc gag ctg gcg ccc ctg tac gat acc acc aag gtg tac ctg gtg 12487
Tyr Ser Glu Leu Ala Pro Leu Tyr Asp Thr Thr Lys Val Tyr Leu Val
        55                  60                  65

gac aac aag tcg gcg gac atc gcc tcc ctg aat tac caa aac gat cac 12535
Asp Asn Lys Ser Ala Asp Ile Ala Ser Leu Asn Tyr Gln Asn Asp His
    70                  75                  80

agt aac ttt ctg act acc gtg gtg cag aac aat gac ttc acc ccg acg 12583
Ser Asn Phe Leu Thr Thr Val Val Gln Asn Asn Asp Phe Thr Pro Thr
85                  90                  95                  100

gag gcg ggc acg cag acc att aac ttt gac gag cgt tcc cgc tgg ggc 12631
Glu Ala Gly Thr Gln Thr Ile Asn Phe Asp Glu Arg Ser Arg Trp Gly
                105                 110                 115

ggt cag ctg aaa acc atc ctg cac acc aac atg ccc aac atc aac gag 12679
Gly Gln Leu Lys Thr Ile Leu His Thr Asn Met Pro Asn Ile Asn Glu
            120                 125                 130

ttc atg tcc acc aac aag ttc agg gct aag ctg atg gta gaa aaa agt 12727
Phe Met Ser Thr Asn Lys Phe Arg Ala Lys Leu Met Val Glu Lys Ser
        135                 140                 145

aat gcg gaa act cgg cag ccc cga tac gag tgg ttc gag ttt acc att 12775
Asn Ala Glu Thr Arg Gln Pro Arg Tyr Glu Trp Phe Glu Phe Thr Ile
    150                 155                 160

cca gag ggc aac tat tcc gaa act atg act atc gat ctc atg aat aac 12823
Pro Glu Gly Asn Tyr Ser Glu Thr Met Thr Ile Asp Leu Met Asn Asn
165                 170                 175                 180

gcg atc gtg gac aat tac ctg caa gtg ggg aga cag aac ggg gtg ctg 12871
Ala Ile Val Asp Asn Tyr Leu Gln Val Gly Arg Gln Asn Gly Val Leu
                185                 190                 195

gaa agc gat atc ggc gtg aaa ttc gat acc aga aac ttc cga ctg ggg 12919
Glu Ser Asp Ile Gly Val Lys Phe Asp Thr Arg Asn Phe Arg Leu Gly
            200                 205                 210

tgg gat ccc gtg acc aag ctg gtg atg cca ggc gtg tac acc aac gag 12967
Trp Asp Pro Val Thr Lys Leu Val Met Pro Gly Val Tyr Thr Asn Glu
        215                 220                 225

gct ttt cac ccg gac atc gtg ctg ctg ccg ggg tgc ggt gtg gac ttc 13015
Ala Phe His Pro Asp Ile Val Leu Leu Pro Gly Cys Gly Val Asp Phe
    230                 235                 240

act cag agc cgt ttg agt aac ctg tta gga att aga aag cgc cgc ccc 13063
Thr Gln Ser Arg Leu Ser Asn Leu Leu Gly Ile Arg Lys Arg Arg Pro
245                 250                 255                 260

ttc caa gag ggc ttt caa atc atg tat gag gac ctg gag gga ggt aat 13111
Phe Gln Glu Gly Phe Gln Ile Met Tyr Glu Asp Leu Glu Gly Gly Asn
                265                 270                 275

ata ccc gcc tta ctg gac gtg tcg aag tac gaa gct agc ata caa cgc 13159
Ile Pro Ala Leu Leu Asp Val Ser Lys Tyr Glu Ala Ser Ile Gln Arg
            280                 285                 290

gcc aaa gcg gag ggt aga gag att cgg gga gac acc ttt gcg gta gct 13207
Ala Lys Ala Glu Gly Arg Glu Ile Arg Gly Asp Thr Phe Ala Val Ala
        295                 300                 305

ccc cag gac ctg gaa ata gtg cct tta act aaa gac agc aaa gac aga 13255
Pro Gln Asp Leu Glu Ile Val Pro Leu Thr Lys Asp Ser Lys Asp Arg
    310                 315                 320

agc tac aat att ata aac aac acg acg gac acc ctg tat cgg agc tgg 13303
Ser Tyr Asn Ile Ile Asn Asn Thr Thr Asp Thr Leu Tyr Arg Ser Trp
325                 330                 335                 340

ttt ctg gct tac aac tac gga gac ccc gag aaa gga gtg aga tca tgg 13351
Phe Leu Ala Tyr Asn Tyr Gly Asp Pro Glu Lys Gly Val Arg Ser Trp
                345                 350                 355

acc ata ctc acc acc acg gac gtg acc tgt ggc tcg cag caa gtg tac 13399
Thr Ile Leu Thr Thr Thr Asp Val Thr Cys Gly Ser Gln Gln Val Tyr
            360                 365                 370

tgg tcc ctg ccg gat atg atg caa gac ccg gtc acc ttc cgc ccc tcc 13447
Trp Ser Leu Pro Asp Met Met Gln Asp Pro Val Thr Phe Arg Pro Ser
        375                 380                 385

acc caa gtc agc aac ttc ccg gtg gtg ggc acc gag ctg ctg ccc gtc 13495
Thr Gln Val Ser Asn Phe Pro Val Val Gly Thr Glu Leu Leu Pro Val
    390                 395                 400

cat gcc aag agc ttc tac aac gag cag gcc gtc tac tcg caa ctt att 13543
His Ala Lys Ser Phe Tyr Asn Glu Gln Ala Val Tyr Ser Gln Leu Ile
405                 410                 415                 420

cgc cag tcc acc gcg ctt acc cac gtg ttc aat cgc ttt ccc gag aac 13591
Arg Gln Ser Thr Ala Leu Thr His Val Phe Asn Arg Phe Pro Glu Asn
                425                 430                 435

cag att ctg gtg cgc cct ccc gct cct acc att acc acc gtc agt gaa 13639
Gln Ile Leu Val Arg Pro Pro Ala Pro Thr Ile Thr Thr Val Ser Glu
            440                 445                 450

aac gtt ccc gcc ctc aca gat cac gga acc ctg ccg ctg cgc agc agt 13687
Asn Val Pro Ala Leu Thr Asp His Gly Thr Leu Pro Leu Arg Ser Ser
        455                 460                 465

atc agt gga gtt cag cgc gtg acc atc acc gac gcc aga cgt cga acc 13735
Ile Ser Gly Val Gln Arg Val Thr Ile Thr Asp Ala Arg Arg Arg Thr

470                 475                 480

tgc ccc tac gtt tac aaa gcg ctt ggc gtg gtg gct cct aaa gtt ctt     13783
Cys Pro Tyr Val Tyr Lys Ala Leu Gly Val Val Ala Pro Lys Val Leu
485                 490                 495                 500

tct agt cgc acc ttc taa aaacatgtcc atcctcatct ctcccgataa            13831
Ser Ser Arg Thr Phe
                505

caacaccggc tggggactgg gctccggcaa gatgtacggc ggagccaaaa ggcgctccag  13891

tcagcaccca gttcgagttc ggggccactt ccgcgctcct tggggagctt acaagcgagg  13951

actctcgggt cgaacggctg tagacgatac catagatgcc gtgattgccg acgcccgccg  14011

gtacaacccc ggaccggtcg ctagcgccgc ctccaccgtg gattccgtga tcgacagcgt  14071

ggtagccggc gctcgggcct atgctcgccg caagaggcgg ctgcatcgga gacgtcgccc  14131

caccgccgcc atgctggcag ccagggccgt gctgaggcgg gcccggaggg caggcagaag  14191

ggctatgcgc cgcgctgccg ccaacgccgc cgccgggagg gcccgccgac aggctgcccg  14251

ccaggctgcc gctgccatcg ctagcatggc cagacccagg agagggaacg tgtactgggt  14311

gcgtgattct gtgacgggag tccgagtgcc ggtgcgcagc cgacctcccc gaagttagaa  14371

gatccaagct gcgaagacgg cggtactgag tctccctgtt gttatcagcc caacatgagc  14431

aagcgcaagt ttaaagaaga actgctgcag acgctggtgc ctgagatcta tggccctccg  14491

gacgtgaagc cagacattaa gccccgcgat atcaagcgtg ttaaaaagcg ggaaaagaaa  14551

gaggaactcg cggtggtaga cgatggcgga gtggaattta ttaggagttt cgccccgcga  14611

cgcagggttc aatggaaagg gcggcgggta caacgcgttt tgaggccggg caccgcggta  14671

gtttttaccc cgggagagcg gtcggccgtt aggggtttca aaaggcagta cgacgaggtg  14731

tacggcgacg aggacatatt ggaacaggcg gctcaacaga tcggagaatt tgcctacgga  14791

aagcgttcgc gtcgcgaaga cctggccatc gccttagaca gcggcaaccc cacgcccagc  14851

ctcaaacccg tgacgctgca gcaggtgctt cccgtgagcg ccagcacgga cagcaagagg  14911

gggattaaga gagaaatgga agatctgcat cccaccatcc aactcatggt ccctaaacgg  14971

cagaggctgg aagaggtcct ggagaagatg aaagtggacc ccagcataga gccggatgta  15031

aaagtcagac ctattaagga agtggccccc ggtcttgggg tgcaaacggt ggacattcaa  15091

atccccgtca ccaccgcttc aaccgccgtg gaagctatgg aaacgcaaac ggagacccct  15151

gccgcgatcg gtaccaggga agtggcgttg caaacggagc cttggtacga atacgcagcc  15211

cctcggcgtc agaggcgttc cgctcgttac ggccccgcca acgccatcat gccagaatat  15271

gcgctgcatc cgtctattct gcccactccc ggataccggg gtgtgacgta tcgcccgtct  15331

ggaacccgcc gccgaacccg tcgccgccgc cgctcccgtc gcgctctggc ccccgtgtcg  15391

gtgcggcgtg tgacccgccg gggaaagaca gtcgtcattc ccaacccgcg ttaccaccct  15451

agcatccttt aataactctg ccgttttgca gatggctctg acttgccgcg tgcgccttcc 15511

cgttccgcac tatcgaggaa gatctcgtcg taggagaggc atgacgggca gtggtcgccg 15571

gcgggctttg cgcaggcgca tgaaaggcgg aattttaccc gccctgatac ccataattgc 15631

cgccgccatc ggtgccatac ccggcgttgc ttcagtggcg ttgcaagcag ctcgtaataa 15691

ataaacaaag gcttttgcac ttatgacctg gtcctgacta ttttatgcag aaagagcatg 15751

gaagacatca attttacgtc gctggctccg cggcacggct cgcggccgct catgggcacc 15811

tggaacgaca tcggcaccag tcagctcaac gggggcgctt tcaattgggg gagcctttgg 15871

agcggcatta aaaactttgg ctccacgatt aaatcctacg gcagcaaagc ctggaacagt 15931

agtgctggtc agatgctccg agataaactg aaggacacca acttccaaga aaaagtggtc 15991

aatggggtgg tgaccggcat ccacggcgcg gtagatctcg ccaaccaagc ggtgcagaaa 16051

gagattgaca ggcgtttgga aagctcgcgg gtgccgccgc agagagggga tgaggtggag 16111

gtcgaggaag tagaagtaga ggaaaagctg cccccgctgg agaaagttcc cggtgcgcct 16171

ccgagaccgc agaagcggcc caggccagaa ctagaagaga ctctggtgac ggagagcaag 16231

gagcctccct cgtacgagca agccttgaaa gagggcgcct ctccaccctc ctacccgatg 16291

actaagccga tcgcacccat ggctcgaccg gtgtacggca aggattacaa gcccgtcacg 16351

ctagagctgc ccccaccgcc ccccacgcgc ccgaccgtcc cccccctgcc gactccgtcg 16411

gcggccgcgg cgggacccgt gtccgcacca tccgctgtgc ctctgccagc cgcccgtcca 16471

gtggccgtgg ccactgccag aaaccccaga ggccagagag gagccaactg gcaaagcacg 16531

ctgaacagca tcgtgggcct gggagtgaaa agcctgaaac gccgccgttg ctattattaa 16591

aaaagtgtag ctaaaaagtc tcccgttgta tacgcctcct atgttaccgc cagagacgag 16651

tgactgtcgc cgcgagcgcc gctttcaag atg gcc acc cca tcg atg atg ccg 16704
Met Ala Thr Pro Ser Met Met Pro
                510

cag tgg tct tac atg cac atc gcc ggc cag gac gcc tcg gag tac ctg 16752
Gln Trp Ser Tyr Met His Ile Ala Gly Gln Asp Ala Ser Glu Tyr Leu
    515                 520                 525

agt ccc ggc ctc gtg cag ttt gcc cgc gcc acc gac acc tac ttc agc 16800
Ser Pro Gly Leu Val Gln Phe Ala Arg Ala Thr Asp Thr Tyr Phe Ser
530                 535                 540                 545

ttg gga aac aag ttt aga aac ccc acc gtg gcc ccc acc cac gat gtg 16848
Leu Gly Asn Lys Phe Arg Asn Pro Thr Val Ala Pro Thr His Asp Val
                550                 555                 560

acc acg gac cgc tcg cag agg ctg acc ctg cgc ttt gtg ccc gta gac 16896
Thr Thr Asp Arg Ser Gln Arg Leu Thr Leu Arg Phe Val Pro Val Asp
            565                 570                 575

cgg gag gac acc gcg tac tct tac aaa gtg cgc tac acg ttg gcc gta 16944
Arg Glu Asp Thr Ala Tyr Ser Tyr Lys Val Arg Tyr Thr Leu Ala Val

580                 585                 590

ggg gac aac cga gtg ctg gac atg gcc agc acc tac ttt gac atc cgg      16992
Gly Asp Asn Arg Val Leu Asp Met Ala Ser Thr Tyr Phe Asp Ile Arg
    595                 600                 605

ggg gtg ctg gat cgg ggt ccc agc ttc aag ccc tat tcc ggc acc gct      17040
Gly Val Leu Asp Arg Gly Pro Ser Phe Lys Pro Tyr Ser Gly Thr Ala
610                 615                 620                 625

tac aac tcc ctg gcc ccc aag gga gct ccc aac ccc tcg gaa tgg acg      17088
Tyr Asn Ser Leu Ala Pro Lys Gly Ala Pro Asn Pro Ser Glu Trp Thr
                630                 635                 640

gac act tcc gac aac aaa ctt aaa gca tat gct cag gct ccc tac cag      17136
Asp Thr Ser Asp Asn Lys Leu Lys Ala Tyr Ala Gln Ala Pro Tyr Gln
            645                 650                 655

agt caa gga ctt aca aag gat ggt att cag gtt ggg cta gtt gtg aca      17184
Ser Gln Gly Leu Thr Lys Asp Gly Ile Gln Val Gly Leu Val Val Thr
        660                 665                 670

gag tca gga caa aca ccc caa tat gca aac aaa gtg tac caa ccc gag      17232
Glu Ser Gly Gln Thr Pro Gln Tyr Ala Asn Lys Val Tyr Gln Pro Glu
    675                 680                 685

cca caa att ggg gaa aac caa tgg aat tta gaa caa gaa gat aaa gcg      17280
Pro Gln Ile Gly Glu Asn Gln Trp Asn Leu Glu Gln Glu Asp Lys Ala
690                 695                 700                 705

gcg gga aga gtc cta aag aaa gat acc cct atg ttt ccc tgc tat ggg      17328
Ala Gly Arg Val Leu Lys Lys Asp Thr Pro Met Phe Pro Cys Tyr Gly
                710                 715                 720

tca tat gcc agg ccc aca aac gaa caa gga ggg cag gca aaa aac caa      17376
Ser Tyr Ala Arg Pro Thr Asn Glu Gln Gly Gly Gln Ala Lys Asn Gln
            725                 730                 735

gaa gta gat tta cag ttt ttt gcc act ccg ggc gac acc cag aac acg      17424
Glu Val Asp Leu Gln Phe Phe Ala Thr Pro Gly Asp Thr Gln Asn Thr
        740                 745                 750

gct aaa gtg gta ctt tat gct gaa aat gtc aac ctg gaa act cca gat      17472
Ala Lys Val Val Leu Tyr Ala Glu Asn Val Asn Leu Glu Thr Pro Asp
    755                 760                 765

act cac tta gtg ttt aaa ccc gat gac gac agc acc agt tca aaa ctt      17520
Thr His Leu Val Phe Lys Pro Asp Asp Asp Ser Thr Ser Ser Lys Leu
770                 775                 780                 785

ctt ctt ggg cag cag gct gca cct aac aga ccc aac tac ata ggt ttt      17568
Leu Leu Gly Gln Gln Ala Ala Pro Asn Arg Pro Asn Tyr Ile Gly Phe
                790                 795                 800

aga gat aat ttt att ggt tta atg tac tac aat agc act gga aac atg      17616
Arg Asp Asn Phe Ile Gly Leu Met Tyr Tyr Asn Ser Thr Gly Asn Met
            805                 810                 815

ggc gtg ctg gcc gga cag gct tct caa ttg aat gcc gta gtc gac ttg      17664
Gly Val Leu Ala Gly Gln Ala Ser Gln Leu Asn Ala Val Val Asp Leu
        820                 825                 830

cag gac aga aac acc gag ttg tcc tac cag ctg atg ctg gac gca ctg      17712

Gln Asp Arg Asn Thr Glu Leu Ser Tyr Gln Leu Met Leu Asp Ala Leu
    835                 840                 845

ggg gat cgc agc cga tat ttt tca atg tgg aat cag gca gta gac agc     17760
Gly Asp Arg Ser Arg Tyr Phe Ser Met Trp Asn Gln Ala Val Asp Ser
850                 855                 860                 865

tat gac cca gac gtt aga att ata gaa aac cac gga gtg gaa gac gaa     17808
Tyr Asp Pro Asp Val Arg Ile Ile Glu Asn His Gly Val Glu Asp Glu
                870                 875                 880

ctg cca aac tat tgt ttt cct ctg gga gga atg gtg gtg act gac aat     17856
Leu Pro Asn Tyr Cys Phe Pro Leu Gly Gly Met Val Val Thr Asp Asn
            885                 890                 895

tac aac tct gtg acg cct caa aat gga ggc agt gga aat aca tgg cag     17904
Tyr Asn Ser Val Thr Pro Gln Asn Gly Gly Ser Gly Asn Thr Trp Gln
        900                 905                 910

gca gac aat act aca ttt agt caa aga gga gcg cag att ggc tcc gga     17952
Ala Asp Asn Thr Thr Phe Ser Gln Arg Gly Ala Gln Ile Gly Ser Gly
    915                 920                 925

aac atg ttt gcc ctg gaa att aac cta cag gcc aac ctc tgg cgc ggc     18000
Asn Met Phe Ala Leu Glu Ile Asn Leu Gln Ala Asn Leu Trp Arg Gly
930                 935                 940                 945

ttc ttg tat tcc aat att ggg ttg tat ctt cca gac tct ctg aaa atc     18048
Phe Leu Tyr Ser Asn Ile Gly Leu Tyr Leu Pro Asp Ser Leu Lys Ile
                950                 955                 960

acc ccc gac aac atc acg ctg cca gaa aac aaa aac act tat cag tac     18096
Thr Pro Asp Asn Ile Thr Leu Pro Glu Asn Lys Asn Thr Tyr Gln Tyr
            965                 970                 975

atg aac ggt cgc gta acg cca ccc ggg ctc ata gac acc tat gta aac     18144
Met Asn Gly Arg Val Thr Pro Pro Gly Leu Ile Asp Thr Tyr Val Asn
        980                 985                 990

gtg ggc gcg cgc tgg tcc ccc  gat gtc atg gac agc  att aac ccc ttc   18192
Val Gly Ala Arg Trp Ser Pro  Asp Val Met Asp Ser  Ile Asn Pro Phe
    995                 1000                1005

aac  cac cac cgt aac gcg  ggc ttg cgc tac cgc  tcc atg ctc ttg     18237
Asn  His His Arg Asn Ala  Gly Leu Arg Tyr Arg  Ser Met Leu Leu
1010                1015                1020

ggc  aac ggc cgt tat gtg  cct ttt cac att cag  gtg ccc caa aaa     18282
Gly  Asn Gly Arg Tyr Val  Pro Phe His Ile Gln  Val Pro Gln Lys
1025                1030                1035

ttc  ttt gcc att aaa aac  ctg ctg ctt ctc ccc  ggt tcc tat acc     18327
Phe  Phe Ala Ile Lys Asn  Leu Leu Leu Leu Pro  Gly Ser Tyr Thr
1040                1045                1050

tat  gag tgg aac ttc cgc  aag gat gtc aac atg  atc ctg cag agc     18372
Tyr  Glu Trp Asn Phe Arg  Lys Asp Val Asn Met  Ile Leu Gln Ser
1055                1060                1065

tcg  ctg ggt aat gac ctg  cga gtg gac ggg gcc  agc ata cgc ttt     18417
Ser  Leu Gly Asn Asp Leu  Arg Val Asp Gly Ala  Ser Ile Arg Phe
1070                1075                1080

gac agc att aac ctg tat gcc aac ttt ttt ccc atg gcc cac aac 18462
Asp Ser Ile Asn Leu Tyr Ala Asn Phe Phe Pro Met Ala His Asn
1085                1090                1095

acg gcc tct acc ctg gaa gcc atg ctg cgc aac gac acc aat gac 18507
Thr Ala Ser Thr Leu Glu Ala Met Leu Arg Asn Asp Thr Asn Asp
1100                1105                1110

cag tcc ttc aac gac tac ctg tgc gcg gct aac atg ctg tac ccc 18552
Gln Ser Phe Asn Asp Tyr Leu Cys Ala Ala Asn Met Leu Tyr Pro
1115                1120                1125

atc ccc gcc aac gcc acc agc gtg ccc att tct att cct tct cgg 18597
Ile Pro Ala Asn Ala Thr Ser Val Pro Ile Ser Ile Pro Ser Arg
1130                1135                1140

aac tgg gct gcc ttc agg ggc tgg agt ttt act cgc ctc aaa acc 18642
Asn Trp Ala Ala Phe Arg Gly Trp Ser Phe Thr Arg Leu Lys Thr
1145                1150                1155

aag gag act ccc tcg ctg ggc tcc ggt ttt gac ccc tac ttt gtt 18687
Lys Glu Thr Pro Ser Leu Gly Ser Gly Phe Asp Pro Tyr Phe Val
1160                1165                1170

tac tcc ggc tcc att ccc tac cta gat ggc acc ttt tac ctc aac 18732
Tyr Ser Gly Ser Ile Pro Tyr Leu Asp Gly Thr Phe Tyr Leu Asn
1175                1180                1185

cac act ttc aaa aag gtg tct att atg ttt gac tcc tcg gtt agc 18777
His Thr Phe Lys Lys Val Ser Ile Met Phe Asp Ser Ser Val Ser
1190                1195                1200

tgg ccc ggc aac gac cgc ctg cta acg ccc aac gag ttc gaa att 18822
Trp Pro Gly Asn Asp Arg Leu Leu Thr Pro Asn Glu Phe Glu Ile
1205                1210                1215

aag cgt tcc gtg gac ggt gaa ggg tac aac gtg gcc cag agc aac 18867
Lys Arg Ser Val Asp Gly Glu Gly Tyr Asn Val Ala Gln Ser Asn
1220                1225                1230

atg acc aag gac tgg ttt cta att caa atg ctc agt cac tat aat 18912
Met Thr Lys Asp Trp Phe Leu Ile Gln Met Leu Ser His Tyr Asn
1235                1240                1245

ata ggt tac cag ggc ttc tat gtg ccc gag aac tac aag gac cgc 18957
Ile Gly Tyr Gln Gly Phe Tyr Val Pro Glu Asn Tyr Lys Asp Arg
1250                1255                1260

atg tac tcc ttc ttc cgc aac ttc caa cca atg agc cgg cag gtg 19002
Met Tyr Ser Phe Phe Arg Asn Phe Gln Pro Met Ser Arg Gln Val
1265                1270                1275

gta gat acc gtg act tat aca gac tac aaa gat gtc aag ctc ccc 19047
Val Asp Thr Val Thr Tyr Thr Asp Tyr Lys Asp Val Lys Leu Pro
1280                1285                1290

tac caa cac aac aac tca ggg ttc gtg ggc tac atg gga ccc acc 19092
Tyr Gln His Asn Asn Ser Gly Phe Val Gly Tyr Met Gly Pro Thr
1295                1300                1305

atg cga gag gga cag gcc tac ccg gcc aac tat ccc tac ccc ctg 19137
Met Arg Glu Gly Gln Ala Tyr Pro Ala Asn Tyr Pro Tyr Pro Leu
1310                1315                1320

atc gga gag act gcc gta ccc agc ctc acg cag aaa aag ttc ctc 19182
Ile Gly Glu Thr Ala Val Pro Ser Leu Thr Gln Lys Lys Phe Leu
1325                1330                1335

tgc gac cgg gtg atg tgg agg ata ccc ttc tct agc aac ttt atg 19227
Cys Asp Arg Val Met Trp Arg Ile Pro Phe Ser Ser Asn Phe Met
1340                1345                1350

tcg atg ggc tcc ctc acc gac ctg ggg cag aac atg ctg tac gcc 19272
Ser Met Gly Ser Leu Thr Asp Leu Gly Gln Asn Met Leu Tyr Ala
1355                1360                1365

aac tcc gct cac gcc ttg gac atg act ttt gag gtg gat ccc atg 19317
Asn Ser Ala His Ala Leu Asp Met Thr Phe Glu Val Asp Pro Met
1370                1375                1380

gat gag ccc acg ctt ctc tat gtt ctg ttt gaa gtc ttc gac gtg 19362
Asp Glu Pro Thr Leu Leu Tyr Val Leu Phe Glu Val Phe Asp Val
1385                1390                1395

gtg cgc atc cac cag ccg cac cgc ggc gtc atc gag gcc gtc tac 19407
Val Arg Ile His Gln Pro His Arg Gly Val Ile Glu Ala Val Tyr
1400                1405                1410

ctg cgc aca cct ttc tct gcc ggt aac gcc acc acc taa agaagctgat 19456
Leu Arg Thr Pro Phe Ser Ala Gly Asn Ala Thr Thr
1415                1420                1425

gggttccagc gaacaggagt tgcaggccat tgttcgcgac ctgggctgcg ggccctgctt 19516

tttgggcacc ttcgacaagc gttttcccgg attcatgtcc ccccacaagc cggcctgcgc 19576

catcgttaac acggccggac gggagacagg gggggtgcac tggctcgcct tcgcctggaa 19636

cccgcgcaac cgcacctgct acctgttcga cccttttggt ttctccgacg aaaggctgaa 19696

gcagatctac caattcgagt acgaggggct cctcaagcgc agcgctctgg cctccacgcc 19756

cgaccactgc gtcaccctgg aaaagtccac ccagacggtc caggggcccc tctcggccgc 19816

ctgcgggctt ttctgttgca tgtttttgca cgccttcgtg cactggcctc acacccccat 19876

ggagcgcaac cccaccatgg atctgctcac cggagtgccc aacagcatgc ttcacagtcc 19936

ccaggtcgcc cccaccctgc gtcgcaatca ggaccacctg tatcgctttc tggggaaaca 19996

ctctgcctat ttccgccgcc accggcagcg catcgaacag gccacggcct tcgaaagcat 20056

gagccaaaga gtgtaatcaa taaaaaccgt ttttatttga catgatacgc gcttctggcg 20116

tttttattaa aaatcgaagg gttcgaggga ggggtcctcg tgcccgctgg ggagggacac 20176

gttgcggtac tggaatcggg cgctccaacg aaactcgggg atcaccagcc gcggcagggc 20236

cacgtcttcc atgttctgct tccaaaactg tcgcaccagc tgcagggctc ccatcacgtc 20296

gggcgctgag atcttgaagt cgcagttagg gccggagccc ccgcggctgt tgcggaacac 20356

ggggttggca cactggaaca ccaacacgct ggggttgtgg atactagcca gggccgtcgg 20416

gtcggtcacc tccgatgcat ccagatcctc ggcattgctc agggcgaacg gggtcagctt 20476

gcacatctgc cgcccgatct ggggtaccag gtcgcgcttg ttgaggcagt cgcagcgcag 20536
agggatgagg atgcgacgct gcccgcgttg catgatgggg taactcgccg ccaggaactc 20596
ctctatctga cggaaggcca tctgggcctt gacgccctcg gtgaaaaata gcccacagga 20656
cttgctggaa aacacgttat tgccacagtt gatgtcttcc gcgcagcagc gcgcatcttc 20716
gttcttcagc tgaaccacgt tgcgacccca gcggttctga accaccttgg ctttcgtggg 20776
atgctccttc agcgcccgct gtccgttctc gctggtcaca tccatttcca ccacgtgctc 20836
cttgcagacc atctccactc cgtggaaaca gaacagaatg ccctcctgtt gggtattgcg 20896
atgctcccac acggcgcacc cggtggactc ccagctcttg tgtttcaccc ccgcgtaggc 20956
ttccatgtaa gccattagaa atctgcccat cagctcagtg aaggtcttct ggttggtgaa 21016
ggttagcggc aggccgcggt gttcctcgtt caaccaagtt tgacagatct tgcggtacac 21076
ggctccctgg tcgggcagaa acttaaaagt cgttctgctc tcgttgtcca cgtggaactt 21136
ctccatcaac atcgtcatga cttccatgcc cttctcccag gcagtcacca gcggcgcgct 21196
ctcggggttc ttcaccaaca cggcggtgga ggggccctcg ccggccccga cgtccttcat 21256
ggacattttt tgaaactcca cggtgccgtc cgcgcggcgt actctgcgca tcggagggta 21316
gctgaagccc acctccatga cggtgctttc gccctcgctg tcggagacga tctccgggga 21376
gggcggcgga acgggggcag acttgcgagc cttcttcttg ggagggagcg gaggcacctc 21436
ctgctcgcgc tcgggactca tctcccgcaa gtagggggtg atggagcttc ctggttggtt 21496
ctgacggttg gccattgtat cctaggcaga aagacatgga gcttatgcgc gaggaaactt 21556
taaccgcccc gtcccccgtc agcgacgaag aggtcatcgt cgaacaggac ccgggctacg 21616
ttacgccgcc cgaggatctg gaggggccct tagacgaccg gcgcgacgct agtgagcggc 21676
aggaaaatga gaaagaggag gaggagggct gctacctcct ggaaggcgac gttttgctaa 21736
agcatttcgc caggcagagc accatactca aggaggcctt gcaagaccgc tccgaggtgc 21796
ccttggacgt cgccgcgctc tcccaggcct acgaggcgaa cctttctcgc ccccgagtgc 21856
ctccgaagag acagcccaac ggcacctgcg agcccaaccc gcgactcaac ttctaccccg 21916
tgttcgccgt gcccgaggcg ctggccacct accacatctt tttcaaaaac cagcgcattc 21976
ccctttcctg ccgggccaac cgcaccgcgg ccgataggaa gctaacactc agaaacggag 22036
tcagcatacc tgatatcacg tcactggagg aagtgcctaa gatcttcgag ggtctgggtc 22096
gagatgagaa gcgggcggcg aacgctctgc agaaagaaca gaaagagagt cagaacgtgc 22156
tggtggagct ggagggggac aacgcgcgtc tgaccgtcct caaacgttgc atagaagttt 22216
cccacttcgc ctacccggcc ctcaacctgc cgcccaaagt tatgaaatcg gtcatggacc 22276
agctactcat caagagagct gagcccctga atcccgacca ccctgaggcg gaaaactcag 22336
aggacggaaa gcccgtcgtc agcgacgagg agctcgagcg gtggctggaa accagggacc 22396

cccagcagtt gcaagagagg cgcaagatga tgatggcggc cgtgctggtc acggtggagc 22456
tagaatgcct gcaacggttt ttcagcgacg tggagacgct acgcaaaatc ggggagtccc 22516
tgcactacac cttccgccag ggctacgttc gccaggcctg caaaatctcc aacgtagagc 22576
tcagcaacct ggtttcctac atgggcatcc tccacgagaa ccggctgggg cagagcgtgc 22636
tgcactgcac cttgcaaggc gaggcgcgaa gggactacgt ccgagactgc gtctacctct 22696
tcctcaccct cacctggcag accgccatgg gcgtgtggca gcagtgcttg gaagagagaa 22756
acctcaaaga gctggacaaa ctcctctgcc gccagcggcg ggccctctgg accggcttca 22816
gcgagcgcac ggtcgcctgc gccctggcag acatcatttt cccagaacgc ctgatgaaaa 22876
ccttgcagaa cggcctgccg gatttcatca gtcagagcat cttgcaaaac ttccgctcct 22936
tcgtcctgga gcgctccggg atcttgcccg ccatgagctg cgcgctgcct tctgactttg 22996
tccccctttc ctaccgcgag tgccctcccc cactgtggag ccactgctac ctcttccaac 23056
tggccaactt tctggcctac cactccgacc tcatggaaga cgtgagcgga gaggggctgc 23116
tcgagtgcca ctgccgctgc aacctctgca ccccccacag atcgctggcc tgcaacaccg 23176
agctgctcag cgaaacccag gtcataggta ccttcgagat ccaggggccc cagcagcaag 23236
agggtgcttc cggcttgaag ctcactccgg cgctgtggac ctcggcttac ttacgcaaat 23296
ttgtagccga ggactaccac gcccacaaaa ttcagtttta cgaagaccaa tctcgaccac 23356
cgaaagcccc cctcacggcc tgcgtcatca cccagagcaa aatcctggcc caattgcaat 23416
ccatcaacca agcgcgccga gatttccttt tgaaaaaggg tcggggggtg tacctggacc 23476
cccagaccgg cgaggaactc aacccgtcca cactttccgt cgaagcagcc cccccgagac 23536
atgccaccca agggaaccgc caagcagctg atcgctcggc agagagcgaa gaagcaagag 23596
ctgctccagc agcaggtgga ggacgaggaa gagctgtggg acagccaggc agaggaggtg 23656
tcagaggacg aggaggagat ggaaagctgg gacagcctag acgaggagga cgagctttca 23716
gaggaagagg cgaccgaaga aaaaccacct gcatccagcg cgccttctct gagccgacag 23776
ccgaagcccc ggcccccgac gcccccggcc ggctcactca aagccagccg taggtgggac 23836
gccaccggat ctccagcggc agcggcaacg gcagcgggta aggccaaacg cgagcggcgg 23896
gggtattgct cctggcggac ccacaaaagc agtatcgtga actgcttgca acactgcggg 23956
ggaaacatct cctttgcccg acgctacctc ctcttccatc acggtgtggc cttccctcgc 24016
aacgttctct attattaccg tcatctctac agcccctacg aaacgctcgg agaaaaaagc 24076
taaggcctcc tctgccgcga ggaaaaactc cgccgccgct gccgccaagg atccgccggc 24136
caccgaggag ctgagaaagc gcatctttcc cactctgtat gctatctttc agcaaagccg 24196
cgggcagcac cctcagcgcg aactgaaaat aaaaaaccgc tccttccgct cactcacccg 24256

cagctgtctg taccacaaga gagaagacca gctgcagcgc accctggacg acgccgaagc 24316

actgttcagc aaatactgct cagcgtctct taaagactaa aagacccgcg ctttttcccc 24376

ctcgggcgcc aaaacccacg tcatcgccag catgagcaag gagattccca ccccttacat 24436

gtggagctat cagccccaga tgggcctggc cgcgggggcc gcccaggact actccagcaa 24496

aatgaactgg ctcagcgccg gcccccacat gatctcacga gttaacggca tccgagccca 24556

ccgaaaccag atcctcttag aacaggcggc aatcaccgcc acaccccggc gccaactcaa 24616

cccgcccagt tggcccgccg cccaggtgta tcaggaaact ccccgcccga ccacagtcct 24676

cctgccacgc gacgcggagg ccgaagtcct catgactaac tctggggtac aattagcggg 24736

cgggtccagg tacgccaggt acagaggtcg ggccgctcct tactctcccg ggagtataaa 24796

gagggtgatc attcgaggcc gaggtatcca gctcaacgac gaggcggtga gctcctcaac 24856

cggtctcaga cctgacggag tcttccagct cggaggagcg ggccgctctt ccttcaccac 24916

tcgccaggcc tacctgaccc tgcagagctc ttcctcgcag ccgcgctccg ggggaatcgg 24976

cactctccag ttcgtggaag agttcgtccc ctccgtctac ttcaacccgt tttccggctc 25036

acctggacgc tacccggacg ccttcattcc caactttgac gcagtgagtg aatccgtgga 25096

cggctacgac tgatgacaga tggtgcggcc gtgagagctc ggctgcgaca tctgcatcac 25156

tgccgccagc ctcgctgcta cgctcgggag gcgatcgtgt tcagctactt tgagctgccg 25216

gacgagcacc ctcagggacc ggctcacggg ttgaaactcg agattgagaa cgcgcttgag 25276

tctcacctca tcgacgcctt caccgcccgg cctctcctgg tagaaaccga acgcgggatc 25336

actaccatca ccctgttctg catctgcccc acgcccggat tac atg aag atc tgt 25391
Met Lys Ile Cys
            1430

gtt gtc atc ttt gcg ctc agt tta ata aaa act gaa ctt ttt gcc 25436
Val Val Ile Phe Ala Leu Ser Leu Ile Lys Thr Glu Leu Phe Ala
                1435                1440                1445

gta cct tca acg cca cgc gtt gtt tct cct tgt gaa aaa acc cca 25481
Val Pro Ser Thr Pro Arg Val Val Ser Pro Cys Glu Lys Thr Pro
                1450                1455                1460

gga gtc ctt aac tta cac ata gca aaa ccc ttg tat ttt acc ata 25526
Gly Val Leu Asn Leu His Ile Ala Lys Pro Leu Tyr Phe Thr Ile
                1465                1470                1475

gaa aaa caa cta gcc ctt tca att gga aaa ggg tta aca att tct 25571
Glu Lys Gln Leu Ala Leu Ser Ile Gly Lys Gly Leu Thr Ile Ser
                1480                1485                1490

gct aca gga cag ttg gaa agc aca gca agc gta cag gac agc gct 25616
Ala Thr Gly Gln Leu Glu Ser Thr Ala Ser Val Gln Asp Ser Ala
                1495                1500                1505

aca cca ccc cta cgt ggt att tcc cct tta aag ctg aca gac aac 25661
Thr Pro Pro Leu Arg Gly Ile Ser Pro Leu Lys Leu Thr Asp Asn
                1510                1515                1520

ggt tta aca tta agc tat tca gat ccc ctg cgt gtg gta ggt gac 25706
Gly Leu Thr Leu Ser Tyr Ser Asp Pro Leu Arg Val Val Gly Asp
                1525                1530                1535

caa ctt acg ttt aat ttt act tct cca cta cgt tac gaa aat ggc 25751
Gln Leu Thr Phe Asn Phe Thr Ser Pro Leu Arg Tyr Glu Asn Gly
                1540                1545                1550

agt ctt aca ttc aac tac act tct ccc atg aca cta ata aac aac 25796
Ser Leu Thr Phe Asn Tyr Thr Ser Pro Met Thr Leu Ile Asn Asn
                1555                1560                1565

agt ctt gct att aac gtc aat acc tcc aaa ggc ctc agt agt gac 25841
Ser Leu Ala Ile Asn Val Asn Thr Ser Lys Gly Leu Ser Ser Asp
                1570                1575                1580

aac ggc aca ctc gct gta aat gtt act cca gat ttt aga ttt aac 25886
Asn Gly Thr Leu Ala Val Asn Val Thr Pro Asp Phe Arg Phe Asn
                1585                1590                1595

agc tct ggt gcc tta act ttt ggc ata caa agt cta tgg act ttt 25931
Ser Ser Gly Ala Leu Thr Phe Gly Ile Gln Ser Leu Trp Thr Phe
                1600                1605                1610

cca acc aaa act cct aac tgt acc gtg ttt acc gaa agt gac tcc 25976
Pro Thr Lys Thr Pro Asn Cys Thr Val Phe Thr Glu Ser Asp Ser
                1615                1620                1625

ctg ctg agt ctt tgc ttg act aaa tgc gga gct cac gta ctt gga 26021
Leu Leu Ser Leu Cys Leu Thr Lys Cys Gly Ala His Val Leu Gly
                1630                1635                1640

agc gtg agt tta agc gga gtg gca gga acc atg cta aaa atg acc 26066
Ser Val Ser Leu Ser Gly Val Ala Gly Thr Met Leu Lys Met Thr
                1645                1650                1655

cac act tct gtt acc gtt cag ttt tcg ttt gat gac agt ggt aaa 26111
His Thr Ser Val Thr Val Gln Phe Ser Phe Asp Asp Ser Gly Lys
                1660                1665                1670

cta ata ttc tct cca ctt gcg aac aac act tgg ggt gtt cga caa 26156
Leu Ile Phe Ser Pro Leu Ala Asn Asn Thr Trp Gly Val Arg Gln
                1675                1680                1685

agc gag agt ccg ttg ccc aac cca tcc ttc aac gct ctc acg ttt 26201
Ser Glu Ser Pro Leu Pro Asn Pro Ser Phe Asn Ala Leu Thr Phe
                1690                1695                1700

atg cca aac agt acc att tat tct aga gga gca agt aac gaa cct 26246
Met Pro Asn Ser Thr Ile Tyr Ser Arg Gly Ala Ser Asn Glu Pro
                1705                1710                1715

caa aac aat tat tat gtc cag acg tat ctt aga ggc aac gtg cga 26291
Gln Asn Asn Tyr Tyr Val Gln Thr Tyr Leu Arg Gly Asn Val Arg
                1720                1725                1730

aag cca att cta cta act gtt acc tac aac tca gtt aat tca gga 26336
Lys Pro Ile Leu Leu Thr Val Thr Tyr Asn Ser Val Asn Ser Gly
                1735                1740                1745

tat tcc tta act ttt aaa tgg gat gct gtc gcc aat gaa aaa ttt 26381
Tyr Ser Leu Thr Phe Lys Trp Asp Ala Val Ala Asn Glu Lys Phe

1750                1755                1760

gcc act cct aca tct  tcg ttt tgc tat gtt  gca gag caa taa          26423
Ala Thr Pro Thr Ser  Ser Phe Cys Tyr Val  Ala Glu Gln
                1765                 1770

aaccctgtta ccccaccgtc tcgttttttt cag atg aaa  cga gcg aga gtt      26474
                                     Met Lys  Arg Ala Arg Val
                                          1775

gat  gaa gac ttc aac cca  gtg tac cct tat gac  ccc cca tac gct     26519
Asp  Glu Asp Phe Asn Pro  Val Tyr Pro Tyr Asp  Pro Pro Tyr Ala
1780                 1785                 1790

ccc  gtc atg ccc ttc att  act ccg cct ttt acc  tcc tcg gat ggg     26564
Pro  Val Met Pro Phe Ile  Thr Pro Pro Phe Thr  Ser Ser Asp Gly
1795                 1800                 1805

ttg  cag gaa aaa cca ctt  gga gtg tta agt tta  aac tac agg gat     26609
Leu  Gln Glu Lys Pro Leu  Gly Val Leu Ser Leu  Asn Tyr Arg Asp
1810                 1815                 1820

ccc  att act aca caa aat  ggg tct ctc acg tta  aaa cta gga aac     26654
Pro  Ile Thr Thr Gln Asn  Gly Ser Leu Thr Leu  Lys Leu Gly Asn
1825                 1830                 1835

ggc  ctc act cta aac aac  cag gga cag tta aca  tca act gct ggc     26699
Gly  Leu Thr Leu Asn Asn  Gln Gly Gln Leu Thr  Ser Thr Ala Gly
1840                 1845                 1850

gaa  gtg gag cct ccg ctc  act aat gct aac aac  aaa ctt gca cta     26744
Glu  Val Glu Pro Pro Leu  Thr Asn Ala Asn Asn  Lys Leu Ala Leu
1855                 1860                 1865

gcc  tat agc gaa cca tta  gca gta aaa agc aac  cgc cta act cta     26789
Ala  Tyr Ser Glu Pro Leu  Ala Val Lys Ser Asn  Arg Leu Thr Leu
1870                 1875                 1880

tca  cac acc gct ccc ctt  gtc atc gct aat aat  tct tta gcg ttg     26834
Ser  His Thr Ala Pro Leu  Val Ile Ala Asn Asn  Ser Leu Ala Leu
1885                 1890                 1895

caa  gtt tca gag cct att  ttt gta aat gac gat  gac aag cta gcc     26879
Gln  Val Ser Glu Pro Ile  Phe Val Asn Asp Asp  Asp Lys Leu Ala
1900                 1905                 1910

ctg  cag aca gcc gcc ccc  ctt gta acc aac gct  ggc acc ctt cgc     26924
Leu  Gln Thr Ala Ala Pro  Leu Val Thr Asn Ala  Gly Thr Leu Arg
1915                 1920                 1925

tta  cag agc gct gcc cct  tta gga ttg gtt gaa  aat act ctt aaa     26969
Leu  Gln Ser Ala Ala Pro  Leu Gly Leu Val Glu  Asn Thr Leu Lys
1930                 1935                 1940

ctg  ctg ttt tct aaa ccc  ttg tat ttg caa aat  gat ttt ctt gca     27014
Leu  Leu Phe Ser Lys Pro  Leu Tyr Leu Gln Asn  Asp Phe Leu Ala
1945                 1950                 1955

tta  gcc att gaa cgc ccc  ctg gct gta gca gcc  gca ggt act ctg     27059
Leu  Ala Ile Glu Arg Pro  Leu Ala Val Ala Ala  Ala Gly Thr Leu
1960                 1965                 1970

acc  cta caa ctt act cct  cca tta aag act aac  gat gac ggg cta     27104

Thr Leu Gln Leu Thr Pro Pro Leu Lys Thr Asn Asp Asp Gly Leu
1975                1980                1985

aca cta tcc aca gtc gag cca tta act gta aaa aac gga aac cta 27149
Thr Leu Ser Thr Val Glu Pro Leu Thr Val Lys Asn Gly Asn Leu
1990                1995                2000

ggc ttg caa ata tcg cgc cct tta gtt gtt caa aac aac ggc ctt 27194
Gly Leu Gln Ile Ser Arg Pro Leu Val Val Gln Asn Asn Gly Leu
2005                2010                2015

tcg ctt gct att acc ccc ccg ctg cgt ttg ttt aac agc gac ccc 27239
Ser Leu Ala Ile Thr Pro Pro Leu Arg Leu Phe Asn Ser Asp Pro
2020                2025                2030

gtt ctt ggt ttg ggc ttc act ttt ccc cta gct gtc aca aac aac 27284
Val Leu Gly Leu Gly Phe Thr Phe Pro Leu Ala Val Thr Asn Asn
2035                2040                2045

ctc ctc tcc tta aac atg gga gac gga gtt aaa ctt acc tat aat 27329
Leu Leu Ser Leu Asn Met Gly Asp Gly Val Lys Leu Thr Tyr Asn
2050                2055                2060

aaa cta aca gcc aat ttg ggt agg gat tta caa ttt gaa aac ggt 27374
Lys Leu Thr Ala Asn Leu Gly Arg Asp Leu Gln Phe Glu Asn Gly
2065                2070                2075

gcg att gcc gta acg ctt act gcc gaa tta cct ttg caa tac act 27419
Ala Ile Ala Val Thr Leu Thr Ala Glu Leu Pro Leu Gln Tyr Thr
2080                2085                2090

aac aaa ctt caa ctg aat att gga gct ggc ctt cgt tac aat gga 27464
Asn Lys Leu Gln Leu Asn Ile Gly Ala Gly Leu Arg Tyr Asn Gly
2095                2100                2105

gcc agc aga aaa cta gat gta aac att aac caa aat aaa ggc tta 27509
Ala Ser Arg Lys Leu Asp Val Asn Ile Asn Gln Asn Lys Gly Leu
2110                2115                2120

act tgg gac aac gat gca gtt att ccc aaa cta gga tcg ggc tta 27554
Thr Trp Asp Asn Asp Ala Val Ile Pro Lys Leu Gly Ser Gly Leu
2125                2130                2135

caa ttt gac cct aat ggc aac atc gct gtt atc cct gaa acc gtg 27599
Gln Phe Asp Pro Asn Gly Asn Ile Ala Val Ile Pro Glu Thr Val
2140                2145                2150

aag ccg caa acg tta tgg acg act gca gat ccc tcg cct aac tgc 27644
Lys Pro Gln Thr Leu Trp Thr Thr Ala Asp Pro Ser Pro Asn Cys
2155                2160                2165

tca gtg tac cag gac ttg gat gcc agg ctg tgg ctc gct ctt gtt 27689
Ser Val Tyr Gln Asp Leu Asp Ala Arg Leu Trp Leu Ala Leu Val
2170                2175                2180

aaa agt ggc gac atg gtg cat gga agc att gcc cta aaa gcc cta 27734
Lys Ser Gly Asp Met Val His Gly Ser Ile Ala Leu Lys Ala Leu
2185                2190                2195

aaa ggg acg ttg cta aat cct aca gcc agc tac att tcc att gtg 27779
Lys Gly Thr Leu Leu Asn Pro Thr Ala Ser Tyr Ile Ser Ile Val
2200                2205                2210

ata tat ttt tac agc aac gga gtc agg cgt acc aac tat cca acg 27824
Ile Tyr Phe Tyr Ser Asn Gly Val Arg Arg Thr Asn Tyr Pro Thr
2215                2220                2225

ttt gac aac gaa ggc acc tta gct aac agc gcc act tgg gga tac 27869
Phe Asp Asn Glu Gly Thr Leu Ala Asn Ser Ala Thr Trp Gly Tyr
2230                2235                2240

cga cag ggg caa tct gct aac act aat gtg acc aat gcc act gaa 27914
Arg Gln Gly Gln Ser Ala Asn Thr Asn Val Thr Asn Ala Thr Glu
2245                2250                2255

ttt atg ccc agc tca agc agg tac ccc gtg aat aaa gga gac aac 27959
Phe Met Pro Ser Ser Ser Arg Tyr Pro Val Asn Lys Gly Asp Asn
2260                2265                2270

att caa aat caa tct ttt tca tac acc tgt att aaa gga gat ttt 28004
Ile Gln Asn Gln Ser Phe Ser Tyr Thr Cys Ile Lys Gly Asp Phe
2275                2280                2285

gct atg cct gtc ccg ttc cgt gta aca tat aat cac gcc ctg gaa 28049
Ala Met Pro Val Pro Phe Arg Val Thr Tyr Asn His Ala Leu Glu
2290                2295                2300

ggg tat tcc ctt aag ttc acc tgg cgc gtt gta gcc aat cag gcc 28094
Gly Tyr Ser Leu Lys Phe Thr Trp Arg Val Val Ala Asn Gln Ala
2305                2310                2315

ttt gat att cct tgc tgt tca ttt tca tac atc aca gaa taa 28136
Phe Asp Ile Pro Cys Cys Ser Phe Ser Tyr Ile Thr Glu
2320                2325                2330

aaaaccactt tttcatttta atttcttttt attttacacg aacagtgaga cttcctccac 28196

ccttccattt gacagcatac accagcctct cccccttcat agcagtaaac tgttgtgaat 28256

cagtccggta tttgggagtt aaaatccaaa cagtctcttt ggtgatgaaa cgtcgatcag 28316

taatggacac aaatccctgg gacaggtttt ccaacgtttc ggtgaaaaac tgcacaccgc 28376

cctacaaaac aaacaggttc aggctctcca cgggttatct ccccgatcaa actcagacag 28436

ggtaaaggtg cggtggtgtt ccactaaacc acgcaggtgg cgctgtctga acctctcggt 28496

gcgactcctg tgaggctggt aagaagttag attgtccagt agcctcacag catgtatcat 28556

cagtctacga gtgcgtctgg cgcagcagcg catctgaatc tcactgagat tccggcaaga 28616

atcgcacacc atcacaatca ggttgttcat gatcccatag ctgaacacgc tccagccaaa 28676

gctcattcgc tccaacagcg ccaccgcgtg tccgtccaac cttactttaa cataaatcag 28736

gtgtctgccg cgtacaaaca tgctacccac atacagaact tcccggggca ggcccctgtt 28796

caccacctgt ctgtaccagg gaaacctcac atttatcagg gagccataga tggccatttt 28856

aaaccaatta gctaataccg ccccaccagc tctacactga agagaaccgg gagagttaca 28916

atgacagtga ataatccatc tctcataacc cctgatggtc tgatgaaaat ctagatctaa 28976

cgtggcacaa caaatacaca ctttcatata cattttcata acatgttttt cccaggccgt 29036

taaaatacaa tcccaataca cgggccactc ctgcagtaca ataaagctaa tacaagatgg 29096

tatactcctc acctcactga cactgtgcat gttcatattt tcacattcta agtaccgaga 29156
gttctcctct acagcagcac tgctgcggtc ctcacaaggt ggtagctggt gatgattgta 29216
gggggccagt ctgcagcgat accgtctgtc gcgttgcatc gtagaccagg aaccgacgca 29276
cctcctcgta cttgtggtag cagaaccacg tccgctgcca gcacgtctcc acgtaacgcc 29336
ggtccctgcg tcgctcacgc tccctcctca atgcaaagtg caaccactct tgtaatccac 29396
acagatccct ctcggcctcc ggggtgatgc acacctcaaa cctacagatg tctcggtaca 29456
gttccaaaca cgtagtgagg gcgagttcca accaagacag acagcctgat ctatcccgac 29516
acactggagg tggaggaaga cacggaagag gcatgttatt ccaagcgatt caccaacggg 29576
tcgaaatgaa gatcccgaag atgacaacgg tcgcctccgg agccctgatg gaatttaaca 29636
gccagatcaa acgttatgcg attctccaag ctatcgatcg ccgcttccaa aagagcctgg 29696
acccgcactt ccacaaacac cagcaaagca aaagcactat tatcaaactc ttcaatcatc 29756
aagctgcagg actgtacaat gcctaagtaa ttttcgtttc tccactcgcg aatgatgtcg 29816
cggcagatag tctgaaggtt catcccgtgc agggtaaaaa gctccgaaag ggcgccctct 29876
acagccatgc gtagacacac catcatgact gcaagatatc gggctcctga gacacctgca 29936
gcagatttaa cagatcaagg tcaggttgct ctccgcgatc acgaatctcc atccgcaagg 29996
tcatttgcaa aaaattaaat aaatctatgc cgactagatc tgtcaactcc gcattaggaa 30056
ccaaatcagg tgtggctacg cagcacaaaa gttccaggga tggtgccaaa ctcactagaa 30116
ccgctcccga gtaacaaaac tgatgaatgg gagtaacaca gtgtaaaatg tgcaaccaaa 30176
aatcactaag gtgctccttt aaaaagtcca gtacttctat attcagtccg tgcaagtact 30236
gaagcaactg tgcgggaata tgcacaacaa aaaaaatagg gcggctcaga tacatgttga 30296
cctaaaataa aaagaatcat taaactaaag aagcttggcg aacggtggga taaatgacac 30356
gctccagcag cagacaggca accggctgtc cccgggaacc gcggtaaaat tcatccgaat 30416
gattaaaaag aacaacagaa acttcccacc atgtactcgg ttggatctcc tgagcacaca 30476
gcaatacccc cctcacattc atgtccgcca cagaaaaaaa acgtcccaga tacccagcgg 30536
ggatatccaa cgacagctgc aaagacagca aaacaatccc tctgggagcg atcacaaaat 30596
cctccggtga aaaaagcaca tacatattag aataaccctg ttgctggggc aaaaaggccc 30656
ggcgtcccag caaatgcaca taaatatgtt catcagccat tgccccgtct taccgcgtaa 30716
tcagccacga aaaaatcgag ctaaaattca cccaacagcc tatagctata tatacactcc 30776
gcccaatgac gctaataccg caccacccac gaccaaagtt cacccacacc cacaaaaccc 30836
gcgaaaatcc agcgccgtca gcacttccgc aatttcagtc tcacaacgtc acttccgcgc 30896
gccttttcac attcccacac acacccgcgc ccttcgcccc gccctcgcgc caccccgcgt 30956

caccgcacgt caccccggcc ccgcctcgct cctccccgct cattatcata ttggcacgtt 31016
tccagaataa ggtatattat tgatgatg 31044

<210> 30
<211> 505
<212> PRT
<213> simian adenovirus SV-25

<400> 30
Met Arg Arg Ala Val Arg Val Thr Pro Ala Ala Tyr Glu Gly Pro Pro
1               5                   10                  15

Pro Ser Tyr Glu Ser Val Met Gly Ser Ala Asn Val Pro Ala Thr Leu
            20                  25                  30

Glu Ala Pro Tyr Val Pro Pro Arg Tyr Leu Gly Pro Thr Glu Gly Arg
        35                  40                  45

Asn Ser Ile Arg Tyr Ser Glu Leu Ala Pro Leu Tyr Asp Thr Thr Lys
    50                  55                  60

Val Tyr Leu Val Asp Asn Lys Ser Ala Asp Ile Ala Ser Leu Asn Tyr
65                  70                  75                  80

Gln Asn Asp His Ser Asn Phe Leu Thr Thr Val Val Gln Asn Asn Asp
                85                  90                  95

Phe Thr Pro Thr Glu Ala Gly Thr Gln Thr Ile Asn Phe Asp Glu Arg
            100                 105                 110

Ser Arg Trp Gly Gly Gln Leu Lys Thr Ile Leu His Thr Asn Met Pro
        115                 120                 125

Asn Ile Asn Glu Phe Met Ser Thr Asn Lys Phe Arg Ala Lys Leu Met
    130                 135                 140

Val Glu Lys Ser Asn Ala Glu Thr Arg Gln Pro Arg Tyr Glu Trp Phe
145                 150                 155                 160

Glu Phe Thr Ile Pro Glu Gly Asn Tyr Ser Glu Thr Met Thr Ile Asp
                165                 170                 175

Leu Met Asn Asn Ala Ile Val Asp Asn Tyr Leu Gln Val Gly Arg Gln
            180                 185                 190

Asn Gly Val Leu Glu Ser Asp Ile Gly Val Lys Phe Asp Thr Arg Asn
        195                 200                 205

Phe Arg Leu Gly Trp Asp Pro Val Thr Lys Leu Val Met Pro Gly Val
    210                 215                 220

Tyr Thr Asn Glu Ala Phe His Pro Asp Ile Val Leu Leu Pro Gly Cys
225                 230                 235                 240

Gly Val Asp Phe Thr Gln Ser Arg Leu Ser Asn Leu Leu Gly Ile Arg
                245                 250                 255

Lys Arg Arg Pro Phe Gln Glu Gly Phe Gln Ile Met Tyr Glu Asp Leu
            260                 265                 270

Glu Gly Gly Asn Ile Pro Ala Leu Leu Asp Val Ser Lys Tyr Glu Ala
        275                 280                 285

Ser Ile Gln Arg Ala Lys Ala Glu Gly Arg Glu Ile Arg Gly Asp Thr
    290                 295                 300

Phe Ala Val Ala Pro Gln Asp Leu Glu Ile Val Pro Leu Thr Lys Asp
305                 310                 315                 320

Ser Lys Asp Arg Ser Tyr Asn Ile Ile Asn Asn Thr Thr Asp Thr Leu
                325                 330                 335

Tyr Arg Ser Trp Phe Leu Ala Tyr Asn Tyr Gly Asp Pro Glu Lys Gly
            340                 345                 350

Val Arg Ser Trp Thr Ile Leu Thr Thr Thr Asp Val Thr Cys Gly Ser
        355                 360                 365

Gln Gln Val Tyr Trp Ser Leu Pro Asp Met Met Gln Asp Pro Val Thr
    370                 375                 380

Phe Arg Pro Ser Thr Gln Val Ser Asn Phe Pro Val Val Gly Thr Glu
385                 390                 395                 400

Leu Leu Pro Val His Ala Lys Ser Phe Tyr Asn Glu Gln Ala Val Tyr
                405                 410                 415

Ser Gln Leu Ile Arg Gln Ser Thr Ala Leu Thr His Val Phe Asn Arg
            420                 425                 430

Phe Pro Glu Asn Gln Ile Leu Val Arg Pro Pro Ala Pro Thr Ile Thr

435                 440                 445

Thr Val Ser Glu Asn Val Pro Ala Leu Thr Asp His Gly Thr Leu Pro
    450                 455                 460

Leu Arg Ser Ser Ile Ser Gly Val Gln Arg Val Thr Ile Thr Asp Ala
465                 470                 475                 480

Arg Arg Arg Thr Cys Pro Tyr Val Tyr Lys Ala Leu Gly Val Val Ala
                485                 490                 495

Pro Lys Val Leu Ser Ser Arg Thr Phe
            500                 505

<210>  31

<211>  921

<212>  PRT

<213>  simian adenovirus SV-25

<400>  31

Met Ala Thr Pro Ser Met Met Pro Gln Trp Ser Tyr Met His Ile Ala
1               5                   10                  15

Gly Gln Asp Ala Ser Glu Tyr Leu Ser Pro Gly Leu Val Gln Phe Ala
            20                  25                  30

Arg Ala Thr Asp Thr Tyr Phe Ser Leu Gly Asn Lys Phe Arg Asn Pro
        35                  40                  45

Thr Val Ala Pro Thr His Asp Val Thr Thr Asp Arg Ser Gln Arg Leu
    50                  55                  60

Thr Leu Arg Phe Val Pro Val Asp Arg Glu Asp Thr Ala Tyr Ser Tyr
65                  70                  75                  80

Lys Val Arg Tyr Thr Leu Ala Val Gly Asp Asn Arg Val Leu Asp Met
                85                  90                  95

Ala Ser Thr Tyr Phe Asp Ile Arg Gly Val Leu Asp Arg Gly Pro Ser
            100                 105                 110

Phe Lys Pro Tyr Ser Gly Thr Ala Tyr Asn Ser Leu Ala Pro Lys Gly
        115                 120                 125

Ala Pro Asn Pro Ser Glu Trp Thr Asp Thr Ser Asp Asn Lys Leu Lys
    130                 135                 140

Ala Tyr Ala Gln Ala Pro Tyr Gln Ser Gln Gly Leu Thr Lys Asp Gly
145                 150                 155                 160

Ile Gln Val Gly Leu Val Val Thr Glu Ser Gly Gln Thr Pro Gln Tyr
                165                 170                 175

Ala Asn Lys Val Tyr Gln Pro Glu Pro Gln Ile Gly Glu Asn Gln Trp
            180                 185                 190

Asn Leu Glu Gln Glu Asp Lys Ala Ala Gly Arg Val Leu Lys Lys Asp
        195                 200                 205

Thr Pro Met Phe Pro Cys Tyr Gly Ser Tyr Ala Arg Pro Thr Asn Glu
    210                 215                 220

Gln Gly Gly Gln Ala Lys Asn Gln Glu Val Asp Leu Gln Phe Phe Ala
225                 230                 235                 240

Thr Pro Gly Asp Thr Gln Asn Thr Ala Lys Val Val Leu Tyr Ala Glu
                245                 250                 255

Asn Val Asn Leu Glu Thr Pro Asp Thr His Leu Val Phe Lys Pro Asp
            260                 265                 270

Asp Asp Ser Thr Ser Ser Lys Leu Leu Leu Gly Gln Gln Ala Ala Pro
        275                 280                 285

Asn Arg Pro Asn Tyr Ile Gly Phe Arg Asp Asn Phe Ile Gly Leu Met
    290                 295                 300

Tyr Tyr Asn Ser Thr Gly Asn Met Gly Val Leu Ala Gly Gln Ala Ser
305                 310                 315                 320

Gln Leu Asn Ala Val Val Asp Leu Gln Asp Arg Asn Thr Glu Leu Ser
                325                 330                 335

Tyr Gln Leu Met Leu Asp Ala Leu Gly Asp Arg Ser Arg Tyr Phe Ser
            340                 345                 350

Met Trp Asn Gln Ala Val Asp Ser Tyr Asp Pro Asp Val Arg Ile Ile
        355                 360                 365

Glu Asn His Gly Val Glu Asp Glu Leu Pro Asn Tyr Cys Phe Pro Leu
    370                 375                 380

Gly Gly Met Val Val Thr Asp Asn Tyr Asn Ser Val Thr Pro Gln Asn
385                 390                 395                 400

Gly Gly Ser Gly Asn Thr Trp Gln Ala Asp Asn Thr Thr Phe Ser Gln
                405                 410                 415

Arg Gly Ala Gln Ile Gly Ser Gly Asn Met Phe Ala Leu Glu Ile Asn
            420                 425                 430

Leu Gln Ala Asn Leu Trp Arg Gly Phe Leu Tyr Ser Asn Ile Gly Leu
        435                 440                 445

Tyr Leu Pro Asp Ser Leu Lys Ile Thr Pro Asp Asn Ile Thr Leu Pro
    450                 455                 460

Glu Asn Lys Asn Thr Tyr Gln Tyr Met Asn Gly Arg Val Thr Pro Pro
465                 470                 475                 480

Gly Leu Ile Asp Thr Tyr Val Asn Val Gly Ala Arg Trp Ser Pro Asp
                485                 490                 495

Val Met Asp Ser Ile Asn Pro Phe Asn His His Arg Asn Ala Gly Leu
            500                 505                 510

Arg Tyr Arg Ser Met Leu Leu Gly Asn Gly Arg Tyr Val Pro Phe His
        515                 520                 525

Ile Gln Val Pro Gln Lys Phe Phe Ala Ile Lys Asn Leu Leu Leu Leu
    530                 535                 540

Pro Gly Ser Tyr Thr Tyr Glu Trp Asn Phe Arg Lys Asp Val Asn Met
545                 550                 555                 560

Ile Leu Gln Ser Ser Leu Gly Asn Asp Leu Arg Val Asp Gly Ala Ser
                565                 570                 575

Ile Arg Phe Asp Ser Ile Asn Leu Tyr Ala Asn Phe Phe Pro Met Ala
            580                 585                 590

His Asn Thr Ala Ser Thr Leu Glu Ala Met Leu Arg Asn Asp Thr Asn
        595                 600                 605

Asp Gln Ser Phe Asn Asp Tyr Leu Cys Ala Ala Asn Met Leu Tyr Pro
    610                 615                 620

Ile Pro Ala Asn Ala Thr Ser Val Pro Ile Ser Ile Pro Ser Arg Asn
625                 630                 635                 640

Trp Ala Ala Phe Arg Gly Trp Ser Phe Thr Arg Leu Lys Thr Lys Glu
                645                 650                 655

Thr Pro Ser Leu Gly Ser Gly Phe Asp Pro Tyr Phe Val Tyr Ser Gly
            660                 665                 670

Ser Ile Pro Tyr Leu Asp Gly Thr Phe Tyr Leu Asn His Thr Phe Lys
        675                 680                 685

Lys Val Ser Ile Met Phe Asp Ser Ser Val Ser Trp Pro Gly Asn Asp
    690                 695                 700

Arg Leu Leu Thr Pro Asn Glu Phe Glu Ile Lys Arg Ser Val Asp Gly
705                 710                 715                 720

Glu Gly Tyr Asn Val Ala Gln Ser Asn Met Thr Lys Asp Trp Phe Leu
                725                 730                 735

Ile Gln Met Leu Ser His Tyr Asn Ile Gly Tyr Gln Gly Phe Tyr Val
            740                 745                 750

Pro Glu Asn Tyr Lys Asp Arg Met Tyr Ser Phe Phe Arg Asn Phe Gln
        755                 760                 765

Pro Met Ser Arg Gln Val Val Asp Thr Val Thr Tyr Thr Asp Tyr Lys
    770                 775                 780

Asp Val Lys Leu Pro Tyr Gln His Asn Asn Ser Gly Phe Val Gly Tyr
785                 790                 795                 800

Met Gly Pro Thr Met Arg Glu Gly Gln Ala Tyr Pro Ala Asn Tyr Pro
                805                 810                 815

Tyr Pro Leu Ile Gly Glu Thr Ala Val Pro Ser Leu Thr Gln Lys Lys
            820                 825                 830

Phe Leu Cys Asp Arg Val Met Trp Arg Ile Pro Phe Ser Ser Asn Phe
        835                 840                 845

Met Ser Met Gly Ser Leu Thr Asp Leu Gly Gln Asn Met Leu Tyr Ala
    850                 855                 860

Asn Ser Ala His Ala Leu Asp Met Thr Phe Glu Val Asp Pro Met Asp
865                 870                 875                 880

Glu Pro Thr Leu Leu Tyr Val Leu Phe Glu Val Phe Asp Val Val Arg

885                 890                 895

Ile His Gln Pro His Arg Gly Val Ile Glu Ala Val Tyr Leu Arg Thr
            900                 905                 910

Pro Phe Ser Ala Gly Asn Ala Thr Thr
        915                 920

<210>  32

<211>  347

<212>  PRT

<213>  simian adenovirus SV-25

<400>  32

Met Lys Ile Cys Val Val Ile Phe Ala Leu Ser Leu Ile Lys Thr Glu
1               5                   10                  15

Leu Phe Ala Val Pro Ser Thr Pro Arg Val Val Ser Pro Cys Glu Lys
            20                  25                  30

Thr Pro Gly Val Leu Asn Leu His Ile Ala Lys Pro Leu Tyr Phe Thr
        35                  40                  45

Ile Glu Lys Gln Leu Ala Leu Ser Ile Gly Lys Gly Leu Thr Ile Ser
    50                  55                  60

Ala Thr Gly Gln Leu Glu Ser Thr Ala Ser Val Gln Asp Ser Ala Thr
65                  70                  75                  80

Pro Pro Leu Arg Gly Ile Ser Pro Leu Lys Leu Thr Asp Asn Gly Leu
                85                  90                  95

Thr Leu Ser Tyr Ser Asp Pro Leu Arg Val Val Gly Asp Gln Leu Thr
            100                 105                 110

Phe Asn Phe Thr Ser Pro Leu Arg Tyr Glu Asn Gly Ser Leu Thr Phe
        115                 120                 125

Asn Tyr Thr Ser Pro Met Thr Leu Ile Asn Asn Ser Leu Ala Ile Asn
    130                 135                 140

Val Asn Thr Ser Lys Gly Leu Ser Ser Asp Asn Gly Thr Leu Ala Val
145                 150                 155                 160

Asn Val Thr Pro Asp Phe Arg Phe Asn Ser Ser Gly Ala Leu Thr Phe
                165                 170                 175

Gly Ile Gln Ser Leu Trp Thr Phe Pro Thr Lys Thr Pro Asn Cys Thr
            180                 185                 190

Val Phe Thr Glu Ser Asp Ser Leu Leu Ser Leu Cys Leu Thr Lys Cys
        195                 200                 205

Gly Ala His Val Leu Gly Ser Val Ser Leu Ser Gly Val Ala Gly Thr
    210                 215                 220

Met Leu Lys Met Thr His Thr Ser Val Thr Val Gln Phe Ser Phe Asp
225                 230                 235                 240

Asp Ser Gly Lys Leu Ile Phe Ser Pro Leu Ala Asn Asn Thr Trp Gly
                245                 250                 255

Val Arg Gln Ser Glu Ser Pro Leu Pro Asn Pro Ser Phe Asn Ala Leu
            260                 265                 270

Thr Phe Met Pro Asn Ser Thr Ile Tyr Ser Arg Gly Ala Ser Asn Glu
        275                 280                 285

Pro Gln Asn Asn Tyr Tyr Val Gln Thr Tyr Leu Arg Gly Asn Val Arg
    290                 295                 300

Lys Pro Ile Leu Leu Thr Val Thr Tyr Asn Ser Val Asn Ser Gly Tyr
305                 310                 315                 320

Ser Leu Thr Phe Lys Trp Asp Ala Val Ala Asn Glu Lys Phe Ala Thr
                325                 330                 335

Pro Thr Ser Ser Phe Cys Tyr Val Ala Glu Gln
            340                 345

<210>  33

<211>  559

<212>  PRT

<213>  simian adenovirus SV-25


<400>  33

Met Lys Arg Ala Arg Val Asp Glu Asp Phe Asn Pro Val Tyr Pro Tyr
1               5                   10                  15

Asp Pro Pro Tyr Ala Pro Val Met Pro Phe Ile Thr Pro Pro Phe Thr
            20                  25                  30

Ser Ser Asp Gly Leu Gln Glu Lys Pro Leu Gly Val Leu Ser Leu Asn
        35                  40                  45

Tyr Arg Asp Pro Ile Thr Thr Gln Asn Gly Ser Leu Thr Leu Lys Leu
    50                  55                  60

Gly Asn Gly Leu Thr Leu Asn Asn Gln Gly Gln Leu Thr Ser Thr Ala
65                  70                  75                  80

Gly Glu Val Glu Pro Pro Leu Thr Asn Ala Asn Asn Lys Leu Ala Leu
                85                  90                  95

Ala Tyr Ser Glu Pro Leu Ala Val Lys Ser Asn Arg Leu Thr Leu Ser
            100                 105                 110

His Thr Ala Pro Leu Val Ile Ala Asn Asn Ser Leu Ala Leu Gln Val
        115                 120                 125

Ser Glu Pro Ile Phe Val Asn Asp Asp Asp Lys Leu Ala Leu Gln Thr
    130                 135                 140

Ala Ala Pro Leu Val Thr Asn Ala Gly Thr Leu Arg Leu Gln Ser Ala
145                 150                 155                 160

Ala Pro Leu Gly Leu Val Glu Asn Thr Leu Lys Leu Leu Phe Ser Lys
                165                 170                 175

Pro Leu Tyr Leu Gln Asn Asp Phe Leu Ala Leu Ala Ile Glu Arg Pro
            180                 185                 190

Leu Ala Val Ala Ala Ala Gly Thr Leu Thr Leu Gln Leu Thr Pro Pro
        195                 200                 205

Leu Lys Thr Asn Asp Asp Gly Leu Thr Leu Ser Thr Val Glu Pro Leu
    210                 215                 220

Thr Val Lys Asn Gly Asn Leu Gly Leu Gln Ile Ser Arg Pro Leu Val
225                 230                 235                 240

Val Gln Asn Asn Gly Leu Ser Leu Ala Ile Thr Pro Pro Leu Arg Leu
                245                 250                 255

Phe Asn Ser Asp Pro Val Leu Gly Leu Gly Phe Thr Phe Pro Leu Ala
            260                 265                 270

Val Thr Asn Asn Leu Leu Ser Leu Asn Met Gly Asp Gly Val Lys Leu
        275                 280                 285

Thr Tyr Asn Lys Leu Thr Ala Asn Leu Gly Arg Asp Leu Gln Phe Glu
    290                 295                 300

Asn Gly Ala Ile Ala Val Thr Leu Thr Ala Glu Leu Pro Leu Gln Tyr
305                 310                 315                 320

Thr Asn Lys Leu Gln Leu Asn Ile Gly Ala Gly Leu Arg Tyr Asn Gly
                325                 330                 335

Ala Ser Arg Lys Leu Asp Val Asn Ile Asn Gln Asn Lys Gly Leu Thr
            340                 345                 350

Trp Asp Asn Asp Ala Val Ile Pro Lys Leu Gly Ser Gly Leu Gln Phe
        355                 360                 365

Asp Pro Asn Gly Asn Ile Ala Val Ile Pro Glu Thr Val Lys Pro Gln
    370                 375                 380

Thr Leu Trp Thr Thr Ala Asp Pro Ser Pro Asn Cys Ser Val Tyr Gln
385                 390                 395                 400

Asp Leu Asp Ala Arg Leu Trp Leu Ala Leu Val Lys Ser Gly Asp Met
                405                 410                 415

Val His Gly Ser Ile Ala Leu Lys Ala Leu Lys Gly Thr Leu Leu Asn
            420                 425                 430

Pro Thr Ala Ser Tyr Ile Ser Ile Val Ile Tyr Phe Tyr Ser Asn Gly
        435                 440                 445

Val Arg Arg Thr Asn Tyr Pro Thr Phe Asp Asn Glu Gly Thr Leu Ala
    450                 455                 460

Asn Ser Ala Thr Trp Gly Tyr Arg Gln Gly Gln Ser Ala Asn Thr Asn
465                 470                 475                 480

Val Thr Asn Ala Thr Glu Phe Met Pro Ser Ser Ser Arg Tyr Pro Val
                485                 490                 495

Asn Lys Gly Asp Asn Ile Gln Asn Gln Ser Phe Ser Tyr Thr Cys Ile
            500                 505                 510

Lys Gly Asp Phe Ala Met Pro Val Pro Phe Arg Val Thr Tyr Asn His

515                 520                 525

Ala Leu Glu Gly Tyr Ser Leu Lys Phe Thr Trp Arg Val Val Ala Asn
    530                 535                 540

Gln Ala Phe Asp Ile Pro Cys Cys Ser Phe Ser Tyr Ile Thr Glu
545                 550                 555

<210>  34

<211>  34115

<212>  DNA

<213>  simian adenovirus SV-39


<220>

<221>  CDS

<222>  (13448)..(14959)

<223>  L2 Penton


<220>

<221>  CDS

<222>  (17785)..(20538)

<223>  L3 Hexon


<220>

<221>  CDS

<222>  (29515)..(31116)

<223>  L5 Fiber #1


<400>  34
catcatcaat ataacaccgc aagatggcga ccgagttaac atgcaaatga ggtgggcgga      60

gttacgcgac ctttgtcttg ggaacgcgga agtgggcgcg gcgggtttcg gggaggagcg     120

cggggcgggg cgggcgtgtc gcgcggcggt gacgcgccgg ggacccggaa attgagtagt     180

ttttattcat tttgcaagtt tttctgtaca ttttggcgcg aaaactgaaa cgaggaagtg     240

aaaagtgaaa aatgccgagg tagtcaccgg gtggagatct gacctttgcc gtgtggagtt     300

tacccgctga cgtgtgggtt tcggtctcta ttttttcact gtggttttcc gggtacggtc     360

aaaggtcccc attttatgac tccacgtcag ctgatcgcta gggtatttaa tgcgcctcag 420
accgtcaaga ggccactctt gagtgccggc gagaagagtt ttctcctccg cgttccgcca 480
actgtgaaaa aatgaggaac ttcttgctat ctccggggct gccagcgacc gtagccgccg 540
agctgttgga ggacattgtt accggagctc tgggagacga tcctcaggtg atttctcact 600
tttgtgaaga ttttagtctt catgatctct atgatattga tccgggtgtt gaggggcaag 660
aggatgaatg gctggagtct gtggatgggt tttttccgga cgctatgctg ctagaggctg 720
atttgccacc acctcacaac tctcacactg agcccgagtc agctgctatt cctgaattgt 780
catcaggtga acttgacttg gcttgttacg agactatgcc tccggagtcg gatgaggagg 840
acagcgggat cagcgatccc acggctttta tggtctctaa ggcgattgct atactaaaag 900
aagatgatga tggcgatgat ggatttcgac tggacgctcc ggcggtgccg gggagagact 960
gtaagtcctg tgaataccac cgggatcgta ccggagaccc gtctatgttg tgttctctgt 1020
gttatctccg tcttaacgct gcttttgtct acagtaagtg ttttgtgctt ttttaccctg 1080
tggctttgtt gagtttattt ttttctgtgt ctcatagggt gttgtttatt ataggtcctg 1140
tttcagatgt ggaggaacct gatagtacta ctggaaatga ggaggaaaag ccctccccgc 1200
cgaaactaac tcagcgctgc agacctaata ttttgagacc ctcggcccag cgtgtgtcat 1260
cccggaaacg tgctgctgtt aattgcatag aagatttatt ggaagagccc actgaacctt 1320
tggacttgtc cttaaagcga ccccgcccgc agtagggcgc ggtgccagtt ttttctctct 1380
agcttccggg tgactcagtg caataaaaat tttcttggca acaggtgtat gtgtttactt 1440
tacgggcggg aagggattag gggagtataa agctggaggg gaaaaatctg aggctgtcag 1500
atcgagtgag aagttccatg gacttgtacg agagcctaga gaatctaagt tctttgcgac 1560
gtttgctgga ggaggcctcc gacagaacct cttacatttg gaggtttctg ttcggttccc 1620
ctctgagtcg ctttttgcac cgggtgaagc gagagcacct gacggaattt gatgggcttt 1680
tagagcagct gcctggactg tttgattctt tgaatctcgg ccaccggacg ctgctagagg 1740
agaggctttt tccacaattg gacttttcct ctccaggccg tctgtgttca gcgcttgctt 1800
ttgctgtaca tctgttggac agatggaacg agcagacgca gctcagcccg ggttacactc 1860
tggacttcct gacgctatgc ctatggaagt tcggaatcag gagggggagg aagctgtacg 1920
ggcgcttggt ggagaggcat ccgtctctgc gccagcagcg tctgcaagct caagtgctgc 1980
tgaggcggga ggatctggaa gccatttcgg aggaggagag cggcatggaa gagaagaatc 2040
cgagagcggg gctggaccct ccggcggagg agtagggggg ataccggacc cttttcctga 2100
gttggctttg ggggcggtgg ggggcgcttc tgtggtacgt gaggatgaag aggggcgcca 2160
acgcggtcag aagagggagc attttgagtc ctcgactttc ttggctgatg taaccgtggc 2220

cctgatggcg aaaaacaggc tggaggtggt gtggtacccg gaagtatggg aggactttga 2280
gaagggggac ttgcacctgc tggaaaaata taactttgag caggtgaaaa catactggat 2340
gaacccggat gaggactggg aggtggtttt gaaccgatac ggcaaggtag ctctgcgtcc 2400
cgactgtcgc taccaggttc gcgacaaggt ggtcctgcga cgcaacgtgt acctgttggg 2460
caacggcgcc accgtggaga tggtggaccc cagaaggggt ggttttgtgg ccaatatgca 2520
agaaatgtgc cctggggtgg tgggcttgtc tggggtgact tttcatagtg tgaggtttag 2580
cggtagcaat tttgggggtg tggttattac cgcgaacact cctgtggtcc tgcataattg 2640
ctactttttt ggcttcagca acacctgtgt ggaaatgagg gtgggaggca aagtgcgcgg 2700
gtgttccttt tacgcttgct ggaagggggt ggtgagccag ggtaaggcta aagtgtctgt 2760
tcacaagtgt atgttggaga gatgcacctt gggcatttcc agtgagggct tcctccacgc 2820
cagcgacaac gtggcttctg acaacggctg cgcctttctt atcaagggag ggggtcgcat 2880
ctgtcacaac atgatatgcg gccctgggga tgtcccccca aagccttacc agatggttac 2940
ctgcacagat ggcaaggtgc gcatgctcaa gcctgtgcac attgtgggcc accggcgcca 3000
ccgctggcca gagtttgaac acaatgtgat gacccgctgt agcttgtacc tgggaggcag 3060
gcgaggagtt ttcttgccca gacagtgtaa cctggcccac tgcaacgtga tcatggaaca 3120
atccgccgct acccaggttt gctttggagg aatatttgat ataagcatgg tggtgtataa 3180
gatcctgcgc tacgacgact gtcgggctcg tactcgaacc tgcgactgcg gagcctctca 3240
cctgtgtaac ctgactgtga tggggatggt gactgaggag gtgcgactgg accactgtca 3300
gcactcttgc ctgcgggagg agttttcttc ctcggacgag gaggactagg taggtggttg 3360
gggcgtggcc agcgagaggg tgggctataa aggggaggtg tcggctgacg ctgtcttctg 3420
tttttcaggt accatgagcg gatcaagcag ccagaccgcg ctgagcttcg acggggccgt 3480
gtacagcccc tttctgacgg ggcgcttgcc tgcctgggcc ggagtgcgtc agaatgttac 3540
cggttcgacc gtggacggac gtcccgtgga tccatctaac gctgcttcta tgcgctacgc 3600
tactatcagc acatctactc tggacagcgc cgctgccgcc gcagccgcca cctcagccgc 3660
tctctccgcc gccaagatca tggctattaa cccaagcctt tacagccctg tatccgtgga 3720
cacctcagcc ctggagcttt accggcgaga tctagctcaa gtggtggacc aactcgcagc 3780
cgtgagccaa cagttgcagc tggtgtcgac ccgagtggag caactttccc gccctcccca 3840
gtaaccgcaa aaattcaata aacagaattt aataaacagc acttgagaaa agtttaaact 3900
tgtggttgac tttattcctg gatagctggg gggagggaac ggcgggaacg gtaagacctg 3960
gtccatcgtt cccggtcgtt gagaacacgg tggatttttt ccaagacccg atagaggtgg 4020
gtctgaacgt tgagatacat gggcatgagc ccgtctcggg ggtggaggta ggcccactgc 4080
agggcctcgt tttcaggggt ggtgttgtaa atgatccagt cgtaggcccc ccgctgggcg 4140

tggtgctgga agatgtcctt cagcagcaag ctgatggcaa cgggaagacc cttggtgtag 4200
gtgttgacaa agcggttgag ttgggagggg tgcatgcggg gactgatgag gtgcattttg 4260
gcctggatct tgaggttggc tatgttgccg cccagatcgc gcctgggatt catgttatgc 4320
aagaccacca gcaccgagta accggtgcag cgggggaatt tgtcgtgcag cttggaaggg 4380
aaagcgtgga agaatttgga gacccctcgg tgcccgccta ggttttccat gcactcatcc 4440
atgatgatgg cgatgggccc ccgggaggca gcctgggcaa aaacgttgcg ggggtccgtg 4500
acatcgtagt tgtggtcctg ggtgagttca tcataggaca ttttgacaaa gcgcgggcag 4560
agggtcccag actggggaat gatggttcca tccggtccgg gggcgtagtt gccctcgcag 4620
atttgcattt cccaggcttt gatttcagag ggagggatca tgtcaacctg gggggcgatg 4680
aaaaaaatgg tctctggggc gggggtgatg agctgggtgg aaagcaggtt gcgcaagagc 4740
tgtgacttgc cgcagccggt gggcccgtag atgacagcta tgacgggttg cagggtgtag 4800
tttagagagc tacaactgcc atcatccttc aaaagcgggg ccacactgtt taaaagttct 4860
ctaacatgta agttttcccg cactaagtcc tgcaggagac gtgaccctcc tagggagaga 4920
agttcaggaa gcgaagcaaa gtttttaagt ggcttgaggc catcggccaa gggcaagttc 4980
ctgagagttt gactgagcag ttccagccgg tcccagagct cggttacgtg ctctacggca 5040
tctcgatcca gcagacctcc tcgtttcggg ggttggggcg gctctggctg tagggaatga 5100
ggcggtgggc gtccagctgg gccatggtgc ggtccctcca tgggcgcagg gttctcttca 5160
gggtggtctc ggtcacggtg aatgggtggg ccccgggctg ggcgctggcc agggtgcgct 5220
tgaggctgag gcggctggtg gcgaaccgtt gcttttcgtc tccctgcaag tcagccaaat 5280
agcaacggac catgagctca tagtccaggc tctctgcggc atgtcctttg gcgcgaagct 5340
tgcctttgga aacgtgcccg cagtttgagc agagcaagca ttttagcgcg tagagttttg 5400
gcgccaagaa cacggattcc ggggaataag catccccacc gcagttggag caaacggttt 5460
cgcattccac cagccaggtc agctgaggat cttttgggtc aaaaaccaag cgcccgccgt 5520
tttttttgat gcgcttccta cctcgggtct ccatgaggcg gtgcccgcgt tcggtgacga 5580
agaggctgtc ggtgtctccg tagacggagg tcagggcgcg ctcctccagg ggggtcccgc 5640
ggtcctcggc gtagagaaac tcgcaccact ctgacataaa cgcccgggtc caggctagga 5700
cgaatgaggc gatgtgggaa gggtaccggt cgttatcgat gagggggtcg gttttttcca 5760
aggtgtgcag gcacatgtcc ccctcgtccg cttccaaaaa tgtgattggc ttgtaggtgt 5820
aagtcacgtg atcctgtcct tccgcggggg tataaaaggg ggcgtttccc ccctcctcgt 5880
cactctcttc cggttcgctg tcgccaaagg ccagctgttg gggtacgtaa acgcgggtga 5940
aggcgggcat gacctgtgcg ctgaggttgt cagtttctat atacgaggaa gatttgatgg 6000

cgagcgcccc cgtggagatg cccttgaggt gctcggggcc catttggtca gaaaacacaa 6060
tctgtcggtt atcaagcttg gtggcaaaag acccgtagag ggcgttggag agcaacttgg 6120
cgatggagcg ctgggtttgg tttttttccc ggtcggcttt ttccttggcc gcgatgttga 6180
gctggacgta ctccctggcc acgcacttcc agccgggaaa aacggccgtg cgctcgtccg 6240
gcaccagcct cacgctccat ccgcggttgt gcagggtgat gacgtcgatg ctggtggcca 6300
cctctccgcg caggggctcg ttggtccagc agaggcgacc gcccttgcga gagcagaagg 6360
ggggcagggg gtcaagcagg cgctcgtccg gggggtcggc gtcgatggta aagatggcgg 6420
gcagcaggtg tttgtcaaag taatcgatct gatgcccggg gcaacgcagg gcggtttccc 6480
agtcccgcac cgccaaggcg cgctcgtatg gactgagggg ggcgccccag ggcatgggat 6540
gcgtcagggc cgaggcgtac atgccgcaga tgtcatagac gtaaaggggc tcctccagga 6600
cgccgaggta ggtggggtag cagcgccccc cgcggatgct ggcccgtacg tagtcgtaga 6660
gctcgtgcga gggggccaga aggtggcggc tgaggtgagc gcgctggggc ttttcatctc 6720
ggaagaggat ctgcctgaag atggcgtggg agttggagga gatggtgggc cgctgaaaaa 6780
tgttgaagcg ggcgtcgggc agacccacgg cctcgccgat aaagtgggcg taggactctt 6840
gcagcttttc caccagggag gcggtgacca gcacgtccag agcgcagtag tccagggttt 6900
cccgcacgat gtcataatgc tcttcctttt tttccttcca gaggtctcgg ttgaagagat 6960
actcttcgcg gtctttccag tactcttgga gaggaaaccc gttttcgtct ccacggtaag 7020
agcccaacat gtaaaactgg ttgacggcct gatagggaca gcatcccttc tccacgggca 7080
gcgagtaggc cagggcggcc ttgcgcaggg aggtgtgagt cagggcaaag gtgtcgcgga 7140
ccataacttt tacaaactgg tacttaaagt cccggtcgtc gcacatgcct cgctcccagt 7200
ctgagtagtc tgtgcgcttt ttgtgcttgg ggttaggcag ggagtaggtg acgtcgttaa 7260
agaggatttt gccacatctg ggcataaagt tgcgagagat tctgaagggg ccgggcacct 7320
ccgagcggtt gttgatgact tgggcagcca ggagaatttc gtcgaagccg ttgatgttgt 7380
gccccacgac gtagaactct atgaaacgcg gagcgccgcg cagcaggggg cacttttcaa 7440
gttgctggaa agtaagttcc cgcggctcga cgccgtgttc cgtgcggctc cagtcctcca 7500
ccgggtttcg ctccacaaaa tcctgccaga tgtggtcgac tagcaagagc tgcagtcggt 7560
cgcgaaattc gcggaatttt ctgccgatgg cttgcttctg ggggttcaag caaaaaaagg 7620
tgtctgcgtg gtcgcgccag gcgtcccagc cgagctcgcg agccagattc agggccagca 7680
gcaccagagc cggctcaccg gtgattttca tgacgaggag aaagggcacc agctgttttc 7740
cgaacgcgcc catccaggtg taggtctcca cgtcgtaggt gagaaacaga cgttcggtcc 7800
gcgggtgcga tcccaggggg aaaaacttga tgggctgcca ccattgggag ctctgggcgt 7860
ggatgtgatg gaagtaaaag tcccggcggc gcgtggaaca ttcgtgctgg tttttgtaaa 7920

agcggccgca gtggtcgcag cgcgagacgg agtgaaggct gtgaatcagg tgaatcttgc 7980
gtcgctgagg gggccccaga gccaaaaagc ggagcgggaa cgaccgcgcg gccacttcgg 8040
cgtccgcagg caagatggat gagggttcca ccgttccccg cccgcggacc gaccagactt 8100
ccgccagctg cggcttcagt tcttgcacca gctctcgcag cgtttcgtcg ctgggcgaat 8160
cgtgaatacg gaagttgtcg ggtagaggcg ggaggcggtg gacttccagg aggtgtgtga 8220
gggccggcag gagatgcagg tggtacttga tttcccacgg atgacggtcg cgggcgtcca 8280
aggcgaagag atgaccgtgg ggccgcggcg ccaccagcgt tccgcggggg gtctttatcg 8340
gcggcgggga cgggctcccg gcggcagcgg cggctcggga cccgcgggca agtcgggcag 8400
cggcacgtcg gcgtggagct cgggcagggg ctggtgctgc gcgcggagct gactggcaaa 8460
ggctatcacc cggcgattga cgtcctggat ccggcggcgc tgcgtgaaga ccaccggacc 8520
cgtggtcttg aacctgaaag agagttcgac agaatcaatc tcggcatcgt taaccgcggc 8580
ctggcgcagg atttcggcca cgtccccgga gttgtcttga tacgcgattt ctgccatgaa 8640
ctggtcgatt tcctcttcct gcaagtctcc gtgaccggcg cgttcgacgg tggccgcgag 8700
atcgttggag atgcggccca tgagctggga aaaggcattg atgccgacct cgttccacac 8760
tcggctgtac accacctctc cgtgaacgtc gcgggcgcgc atcaccacct gggcgagatt 8820
gagttccacg tggcgggcga aaaccggata gtttcggagg cgctgataca gatagttgag 8880
ggtggtggcg gcgtgctcgg ccacaaaaaa atacatgatc cagcggcgga gggtcagctc 8940
gttgatgtcg cccagcgcct ccaggcgttc catggcctcg taaaagtcca cggcaaagtt 9000
gaaaaattgg ctgttcctgg ccgagaccgt gagctcttct tccaagagcc gaatgagatc 9060
cgccacggtg gccctgactt cgcgttcgaa agccccgggt gcctcctcca cctcttcctc 9120
ctcgacttct tcgaccgctt cgggcacctc ctcttcctcg accaccacct caggcggggc 9180
tcggcggcgc cggcggcgga cgggcaggcg gtcgacgaaa cgctcgatca tttcccccct 9240
ccgtcgacgc atggtctcgg tgacggcgcg accctgttcg cgaggacgca gggtgaaggc 9300
gccgccgccg agcggaggta acagggagat cggggggcgg tcgtggggga gactgacggc 9360
gctaactatg catctgatca atgtttgcgt agtgacctcg ggtcggagcg agctcagcgc 9420
ttgaaaatcc acgggatcgg aaaaccgttc caggaacgcg tctagccaat cacagtcgca 9480
aggtaagctg aggaccgtct cgggggcttg tctgttctgt cttcccgcgg tggtgctgct 9540
gatgaggtag ttgaagtagg cgctcttgag gcggcggatg gtggacagga gaaccacgtc 9600
tttgcgccca gcttgctgta tccgcaggcg gtcggccatg ccccacactt ctccttgaca 9660
gcggcggagg tccttgtagt attcttgcat cagcctttcc acgggcacct cgtcttcttc 9720
ttccgctcgg ccggacgaga gccgcgtcag gccgtacccg cgctgcccct gtggttggag 9780

cagggccagg tcggccacga cgcgctcggc cagcacggcc tgctggatgc gggtgagggt 9840
gtcctgaaag tcgtcgagat ccacaaagcg gtggtacgcg ccagtgttga tggtgtaggt 9900
gcagttgctc atgacggacc agtttacggt ctgggtgcca tggcccacgg tttccaggta 9960
gcggagacgc gagtaggccc gcgtctcgaa gatgtagtcg ttgcaggtcc gcagcaggta 10020
ctggtagccc accagcagat gcggcggcgg ctggcggtag aggggccacc gctgggtggc 10080
gggggcgttg ggggcgagat cttccaacat gaggcggtga tagccgtaga tgtagcgcga 10140
catccaagtg atgccgctgg ccgtggtgct ggcgcgggcg tagtcgcgaa cgcggttcca 10200
gatgtttcgc agcggctgga agtactcgat ggtggggcga ctctgccccg tgaggcgggc 10260
gcagtcggcg atgctctacg gggaaaaaga agggccagtg aacaaccgcc ttccgtagcc 10320
ggaggagaac gcaagggggt caaagaccac cgaggctcgg gttcgaaacc cgggtggcgg 10380
cccgaatacg gagggcggtt ttttgctttt ttctcagatg catcccgtgc tgcggcagat 10440
gcgtccgaac gcggggtccc agtccccggc ggtgcctgcg gccgtgacgg cggcttctac 10500
ggccacgtcg cgctccaccc cgcctaccac ggcccaggcg gcggtggctc tgcgcggcgc 10560
aggggaaccc gaagcagagg cggtgttgga cgtggaggag ggccaggggt tggctcggct 10620
gggggccctg agtcccgagc ggcacccgcg cgtggctctg aagcgcgacg cggcggaggc 10680
gtacgtgccg cggagcaatc tgtttcgcga ccgcagcggc gaggaggccg aggagatgcg 10740
agacttgcgt tttcgggcgg ggagggagtt gcgtcacggg ctggaccggc agagggttct 10800
gagagaggag gactttgagg cggacgagcg cacgggggtg agtcccgcgc gggctcacgt 10860
ggcggccgcc aacctggtga gcgcgtacga gcagacggtc aaggaggaga tgaacttcca 10920
gaagagcttc aatcatcacg tgcgcacgct gattgcgcgc gaagaggtgg ccatcggcct 10980
catgcatctg tgggattttg tggaggcgta cgttcagaac cccagcagca agccgctgac 11040
ggctcagctg ttcctcatcg tgcaacatag tcgagacaac gaaacgttca gggaggccat 11100
gctgaacatt gcagagcctg aggggcgctg gctcttggat ctcattaaca tcttgcagag 11160
tatcgtagtg caggagcgct cgctgagcct ggccgacaag gtggctgcca tcaactacag 11220
catgctgtcg ctgggcaaat tttacgcccg caagatctac aagtctccgt tcgtccccat 11280
agacaaggag gtgaagatag acagctttta catgcgcatg gcgctcaagg tgctgactct 11340
aagcgacgac ctgggggtgt accgcaacga ccgcatacac aaggcggtga gcgccagccg 11400
ccggcgcgag ctgagcgacc gcgagctttt gcacagcctg catcgggcgt tgactggtgc 11460
cggcagcgcc gaggcggccg agtactttga cgccggagcg gacttgcgct ggcagccatc 11520
ccgacgcgcg ctggaggcgg ctggcgtcgg ggagtacggg gtcgaggacg acgatgaagc 11580
ggacgacgag ttgggcattg acttgtagcc gtttttcgtt agatatgtcg gcgaacgagc 11640
cgtctgcggc cgccatggtg acggcggcgg gcgcgcccca ggacccggcc acgcgcgcgg 11700

cgctgcagag tcagccttcc ggagtgacgc ccgcggacga ctggtccgag gccatgcgtc 11760

gcatcctggc gctgacggcg cgcaaccccg aggcttttcg gcagcagccg caggcaaacc 11820

ggtttgcggc cattttggaa gcggtggtgc cctccagacc caaccccacc cacgaaaagg 11880

tgctggccat cgtcaacgcc ctggcggaga ccaaggccat ccgcccagac gaggccgggc 11940

aggtttacaa cgcgctgcta gaaagggtgg gacgctacaa cagctccaac gtgcagacca 12000

atctggaccg cttggtgacg gacgtgaagg aggccgtagc ccagcgagag cggtttttca 12060

aggaagccaa tctgggctcg ctggtggccc tcaacgcctt cctgagcacg ctgccggcga 12120

acgtgccccg cggtcaggag gactacgtga actttctgag cgccctccgc ctgatggtgg 12180

ccgaggtgcc gcagagcgag gtgtaccagt ctggccccaa ctactacttc cagacctccc 12240

ggcagggcct gcagacggta aacctgacgc aggcctttca gaacctgcag ggcctttggg 12300

gggtgcgcgc tccgctgggc gaccgcagca cggtgtccag cctgctgacc cccaatgccc 12360

ggctgctctt gcttctcatt gctccgttca ccgacagcgg ttccatcagc cgcgactctt 12420

acctgggaca cctgctcacc ctgtaccggg aggccatcgg gcaggcgcgg gtggacgagc 12480

agacgtacca ggaaatcacc agcgtgagcc gcgcgctggg gcaggaggac acgggcagct 12540

tggaggcgac tctgaacttc ctgctgacca accggcggca gcgcctacct ccccagtacg 12600

cgctgaacgc ggaggaggag cgcatcctgc gtttcgtgca gcagagcacc gcgctgtact 12660

tgatgcggga aggcgcctct cccagcgctt cgctggacat gacggcggcc aacatggagc 12720

catcgttcta cgccgccaac cgtcccttcg tcaaccggct aatggactat ttgcatcggg 12780

cggcggccct gaacccggaa tactttacta acgtcatcct gaacgaccgt tggctgccac 12840

ctcccggctt ctacacgggg gagttcgacc tcccggaggc caacgacggt ttcatgtggg 12900

acgacgtgga cagcgtgttc ctgcccggca agaaggaggc gggtgactct cagagccacc 12960

gcgcgagcct cgcagacctg ggggcgaccg ggcccgcgtc tccgctgcct cgcctgccga 13020

gcgccagcag cgccagcgtg gggcgggtga gccgtccgcg cctcagcggt gaggaggact 13080

ggtggaacga tccgctgctc cgtccggccc gcaacaaaaa cttccccaac aacgggatag 13140

aggatttggt agacaaaatg aaccgttgga agacgtatgc ccaggagcat cgggagtggc 13200

aggcgaggca acccatgggc cctgttctgc cgccctctcg gcgcccgcgc agggacgaag 13260

acgccgacga ttcagccgat gacagcagcg tgttggatct gggcgggagc gggaacccct 13320

ttgcccacct gcaacctcgc ggcgtgggtc ggcggtggcg ctaggaaaaa aaattattaa 13380

aagcacttac cagagccatg gtaagaagag caacaaaggt gtgtcctgct ttcttcccgg 13440

tagcaaa atg cgt cgg gcg gtg gca gtt ccc tcc gcg gca atg gcg tta 13489
        Met Arg Arg Ala Val Ala Val Pro Ser Ala Ala Met Ala Leu
        1               5                   10

ggc ccg ccc cct tct tac gaa agc gtg atg gca gcg gcc acc ctg caa 13537
Gly Pro Pro Pro Ser Tyr Glu Ser Val Met Ala Ala Ala Thr Leu Gln
15                  20                  25                  30

gcg ccg ttg gag aat cct tac gtg ccg ccg cga tac ctg gag cct acg 13585
Ala Pro Leu Glu Asn Pro Tyr Val Pro Pro Arg Tyr Leu Glu Pro Thr
                35                  40                  45

ggc ggg aga aac agc att cgt tac tcg gag ctg acg ccc ctg tac gac 13633
Gly Gly Arg Asn Ser Ile Arg Tyr Ser Glu Leu Thr Pro Leu Tyr Asp
            50                  55                  60

acc acc cgc ctg tac ctg gtg gac aac aag tca gca gat atc gcc acc 13681
Thr Thr Arg Leu Tyr Leu Val Asp Asn Lys Ser Ala Asp Ile Ala Thr
        65                  70                  75

ttg aac tac cag aac gac cac agc aac ttt ctc acg tcc gtg gtg cag 13729
Leu Asn Tyr Gln Asn Asp His Ser Asn Phe Leu Thr Ser Val Val Gln
    80                  85                  90

aac agc gac tac acg ccc gcc gaa gcg agc acg cag acc att aac ttg 13777
Asn Ser Asp Tyr Thr Pro Ala Glu Ala Ser Thr Gln Thr Ile Asn Leu
95                  100                 105                 110

gac gac cgc tcg cgc tgg ggc ggg gac ttg aaa acc att ctg cac act 13825
Asp Asp Arg Ser Arg Trp Gly Gly Asp Leu Lys Thr Ile Leu His Thr
                115                 120                 125

aac atg ccc aac gtg aac gag ttc atg ttt acc aac tcg ttc agg gct 13873
Asn Met Pro Asn Val Asn Glu Phe Met Phe Thr Asn Ser Phe Arg Ala
            130                 135                 140

aaa ctt atg gtg gcg cac gag gcc gac aag gac ccg gtt tat gag tgg 13921
Lys Leu Met Val Ala His Glu Ala Asp Lys Asp Pro Val Tyr Glu Trp
        145                 150                 155

gtg cag ctg acg ctg ccg gag ggg aac ttt tca gag att atg acc ata 13969
Val Gln Leu Thr Leu Pro Glu Gly Asn Phe Ser Glu Ile Met Thr Ile
    160                 165                 170

gac ctg atg aac aac gcc att atc gac cac tac ctg gcg gta gcc aga 14017
Asp Leu Met Asn Asn Ala Ile Ile Asp His Tyr Leu Ala Val Ala Arg
175                 180                 185                 190

cag cag ggg gtg aaa gaa agc gag atc ggc gtc aag ttt gac acg cgc 14065
Gln Gln Gly Val Lys Glu Ser Glu Ile Gly Val Lys Phe Asp Thr Arg
                195                 200                 205

aac ttt cgt ctg ggc tgg gac ccg gag acg ggg ctt gtg atg ccg ggg 14113
Asn Phe Arg Leu Gly Trp Asp Pro Glu Thr Gly Leu Val Met Pro Gly
            210                 215                 220

gtg tac acg aac gaa gct ttc cat ccc gac gtg gtc ctc ttg ccg ggc 14161
Val Tyr Thr Asn Glu Ala Phe His Pro Asp Val Val Leu Leu Pro Gly
        225                 230                 235

tgc ggg gtg gac ttt acc tac agc cgg tta aac aac ctg cta ggc ata 14209
Cys Gly Val Asp Phe Thr Tyr Ser Arg Leu Asn Asn Leu Leu Gly Ile
    240                 245                 250

cgc aag aga atg ccc ttt cag gaa ggg ttt cag atc ctg tac gag gac 14257
Arg Lys Arg Met Pro Phe Gln Glu Gly Phe Gln Ile Leu Tyr Glu Asp
255                 260                 265                 270

ctg gag ggc ggt aac atc ccg gcc ctg ctg gac gtg ccg gcg tac gag 14305
Leu Glu Gly Gly Asn Ile Pro Ala Leu Leu Asp Val Pro Ala Tyr Glu
                275                 280                 285

gag agc atc gcc aac gca agg gag gcg gcg atc agg ggc gat aat ttc 14353
Glu Ser Ile Ala Asn Ala Arg Glu Ala Ala Ile Arg Gly Asp Asn Phe
            290                 295                 300

gcg gcg cag ccc cag gcg gct cca acc ata aaa ccc gtt ttg gaa gac 14401
Ala Ala Gln Pro Gln Ala Ala Pro Thr Ile Lys Pro Val Leu Glu Asp
        305                 310                 315

tcc aaa ggg cgg agc tac aac gta ata gcc aac acc aac aac acg gct 14449
Ser Lys Gly Arg Ser Tyr Asn Val Ile Ala Asn Thr Asn Asn Thr Ala
    320                 325                 330

tac agg agc tgg tat ctg gct tat aac tac ggc gac ccg gag aag ggg 14497
Tyr Arg Ser Trp Tyr Leu Ala Tyr Asn Tyr Gly Asp Pro Glu Lys Gly
335                 340                 345                 350

gtt agg gcc tgg acc ctg ctc acc act ccg gac gtg acg tgc ggt tca 14545
Val Arg Ala Trp Thr Leu Leu Thr Thr Pro Asp Val Thr Cys Gly Ser
                355                 360                 365

gag cag gtc tac tgg tcg ctg cct gac atg tac gtg gac cct gtg acg 14593
Glu Gln Val Tyr Trp Ser Leu Pro Asp Met Tyr Val Asp Pro Val Thr
            370                 375                 380

ttt cgc tcc acg cag caa gtt agc aac tac cca gtg gtg gga gcg gag 14641
Phe Arg Ser Thr Gln Gln Val Ser Asn Tyr Pro Val Val Gly Ala Glu
        385                 390                 395

ctt atg ccg att cac agc aag agc ttt tac aac gag cag gcc gtc tac 14689
Leu Met Pro Ile His Ser Lys Ser Phe Tyr Asn Glu Gln Ala Val Tyr
    400                 405                 410

tca cag ctc att cgt cag acc acc gcc cta acg cac gtt ttc aac cgc 14737
Ser Gln Leu Ile Arg Gln Thr Thr Ala Leu Thr His Val Phe Asn Arg
415                 420                 425                 430

ttc ccc gag aac caa atc cta gtg cga cct cca gcg ccc acc atc acc 14785
Phe Pro Glu Asn Gln Ile Leu Val Arg Pro Pro Ala Pro Thr Ile Thr
                435                 440                 445

acc gtc agc gag aac gtg ccc gct cta acc gat cac ggg acg ctg cct 14833
Thr Val Ser Glu Asn Val Pro Ala Leu Thr Asp His Gly Thr Leu Pro
            450                 455                 460

ttg cag aac agc atc cgc gga gtt cag cga gtt acc atc acg gac gcc 14881
Leu Gln Asn Ser Ile Arg Gly Val Gln Arg Val Thr Ile Thr Asp Ala
        465                 470                 475

cgt cgt cgg acc tgt ccc tac gtc tac aaa gcc ttg gga atc gtg gcc 14929
Arg Arg Arg Thr Cys Pro Tyr Val Tyr Lys Ala Leu Gly Ile Val Ala
    480                 485                 490

ccg cgc gtc ctg tcg agt cgc act ttc tag atgtccatcc tcatctctcc 14979
Pro Arg Val Leu Ser Ser Arg Thr Phe
495                 500

cagcaacaat accggttggg gtctgggcgt gaccaaaatg tacggaggcg ccaaacgacg 15039

gtccccacaa catcccgtgc gagtgcgcgg gcactttaga gccccatggg ggtcgcacac 15099
gcgcgggcgc accggccgaa ccaccgtcga cgacgtgatc gatagcgtgg tggccgacgc 15159
ccgcaactac cagcccgctc gatccacggt ggacgaagtc atcgacggcg tggtggccga 15219
cgccagggcc tacgcccgca gaaagtctcg tctgcgccgc cgccgttcgc taaagcgccc 15279
cacggccgcc atgaaagccg ctcgctctct gctgcgtcgc gcacgtatcg tgggtcgccg 15339
cgccgccaga cgcgcagccg ccaacgccgc cgccggccga gtgcgccgcc gggccgccca 15399
gcaggccgcc gccgccatct ccagtctatc cgccccccga cgcgggaatg tgtactgggt 15459
cagggactcg gccaccggcg tgcgagttcc cgtgagaacc cgtcctcctc gtccctgaat 15519
aaaaagttct aagcccaatc ggtgttccgt tgtgtgttca gctcgtcatg accaaacgca 15579
agtttaaaga ggagctgctg caagcgctgg tccccgaaat ctatgcgccg gcgccggacg 15639
tgaaaccgcg tcgcgtgaaa cgcgtgaaga agcaggaaaa gctagagaca aaagaggagg 15699
cggtggcgtt gggagacggg gaggtggagt ttgtgcgctc gttcgcgccg cgtcggcgag 15759
tgaattggaa ggggcgcaag gtgcaacggg tgctgcgtcc cggcacggtg gtgtctttca 15819
ccccgggtga aaaatccgcc tggaagggca taaagcgcgt gtacgatgag gtgtacgggg 15879
acgaagacat tctggagcag gcgctggata gaagcgggga gtttgcttac ggcaagaggg 15939
cgaggacggg cgagatcgcc atcccgctgg acacttccaa ccccaccccc agtctgaaac 15999
ccgtgacgct gcaacaggtg ttgccggtga gcgccccctc gcgacgcggc ataaaacgcg 16059
agggcggcga gctgcagccc accatgcagc tcctggttcc caagaggcag aaactagagg 16119
acgtactgga catgataaaa atggagcccg acgtgcagcc cgatattaaa atccgtccca 16179
tcaaagaagt ggcgccggga atgggcgtgc agaccgtgga catccagatt cccatgacca 16239
gcgccgcaca ggcggtagag gccatgcaga ccgacgtggg gatgatgacg gacctgcccg 16299
cagctgctgc cgccgtggcc agcgccgcga cgcaaacgga agccggcatg cagaccgacc 16359
cgtggacgga ggcgcccgtg cagccggcca gaagacgcgt cagacggacg tacggccccg 16419
tttctggcat aatgccggag tacgcgctgc atccttccat catccccacc cccggctacc 16479
gggggcgcac ctaccgtccg cgacgcagca ccactcgccg ccgtcgccgc acggcacgag 16539
tcgccaccgc cagagtgaga cgcgtaacga cacgtcgcgg ccgccgcttg accctgcccg 16599
tggtgcgcta ccatcccagc attctttaaa aaaccgctcc tacgttgcag atgggcaagc 16659
ttacttgtcg actccgtatg gccgtgcccg gctaccgagg aagatcccgc cgacgacgga 16719
ctttgggagg cagcggtttg cgccgccgtc gggcggttca ccggcgcctc aagggaggca 16779
ttctgccggc cctgatcccc ataatcgccg cagccatcgg ggccattccc ggaatcgcca 16839
gcgtagcggt gcaggctagc cagcgccact gattttacta accctgtcgg tcgcgccgtc 16899
tctttcggca gactcaacgc ccagcatgga agacatcaat ttctcctctc tggccccgcg 16959

gcacggcacg cggccgtata tggggacgtg gagcgagatc ggcacgaacc agatgaacgg 17019

gggcgctttc aattggagcg gtgtgtggag cggcttgaaa aatttcggtt ccactctgaa 17079

aacttacggc aaccgggtgt ggaactccag cacggggcag atgctgaggg acaagctaaa 17139

ggacacgcag tttcagcaaa aggtggtgga cggcatcgct tcgggcctca acggcgccgt 17199

cgacctggcc aaccaggcca ttcaaaagga aattaacagc cgcctggagc cgcggccgca 17259

ggtggaggag aacctgcccc ctctggaggc gctgcccccc aagggagaga agcgcccgcg 17319

gcccgacatg gaggagacgc tagttactaa gagcgaggag ccgccatcat acgaggaggc 17379

ggtgggtagc tcgcagctgc cgtccctcac gctgaagccc accacctatc ccatgaccaa 17439

gcccatcgcc tccatggcgc gccccgtggg agtcgacccg cccatcgacg cggtggccac 17499

tttggacctg ccgcgccccg aacccggcaa ccgcgtgcct cccgtcccca tcgctccgcc 17559

ggtttctcgc cccgccatcc gccccgtcgc cgtggccact ccccgctatc cgagccgcaa 17619

cgccaactgg cagaccaccc tcaacagtat tgtcggactg ggggtgaagt ctctgaagcg 17679

ccgtcgctgt ttttaaagca caatttatta aacgagtagc cctgtcttaa tccatcgttg 17739

tatgtgtgcc tatatcacgc gttcagagcc tgaccgtccg tcaag atg gcc act ccg 17796
Met Ala Thr Pro
    505

tcg atg atg ccg cag tgg tcg tac atg cac atc gcc ggg cag gac gcc 17844
Ser Met Met Pro Gln Trp Ser Tyr Met His Ile Ala Gly Gln Asp Ala
        510                 515                 520

tcg gag tac ctg agc ccg ggt ctg gtg cag ttt gcc cgt gcg acg gaa 17892
Ser Glu Tyr Leu Ser Pro Gly Leu Val Gln Phe Ala Arg Ala Thr Glu
    525                 530                 535

acc tac ttc tca ctg ggc aac aag ttc agg aac ccc acc gtg gcg ccc 17940
Thr Tyr Phe Ser Leu Gly Asn Lys Phe Arg Asn Pro Thr Val Ala Pro
540                 545                 550                 555

acc cac gac gtc acc acc gat cgg tcc cag cga ctg aca atc cgc ttc 17988
Thr His Asp Val Thr Thr Asp Arg Ser Gln Arg Leu Thr Ile Arg Phe
                560                 565                 570

gtc ccc gtg gac aag gaa gac acc gct tac tcc tac aaa acc cgc ttc 18036
Val Pro Val Asp Lys Glu Asp Thr Ala Tyr Ser Tyr Lys Thr Arg Phe
            575                 580                 585

acg ctg gcc gtg ggc gac aac cgg gtg cta gac atg gcc agt acc tac 18084
Thr Leu Ala Val Gly Asp Asn Arg Val Leu Asp Met Ala Ser Thr Tyr
        590                 595                 600

ttt gac atc cgc ggc gtg atc gac cgc gga cct agc ttc aag cct tac 18132
Phe Asp Ile Arg Gly Val Ile Asp Arg Gly Pro Ser Phe Lys Pro Tyr
    605                 610                 615

tcc ggc acg gct tac aac tca ctg gct ccc aaa ggg gcg ccc aac aac 18180
Ser Gly Thr Ala Tyr Asn Ser Leu Ala Pro Lys Gly Ala Pro Asn Asn
620                 625                 630                 635

agc caa tgg aac gcc aca gat aac ggg aac aag cca gtg tgt ttt gct     18228
Ser Gln Trp Asn Ala Thr Asp Asn Gly Asn Lys Pro Val Cys Phe Ala
                640                 645                 650

cag gca gct ttt ata ggt caa agc att aca aaa gac gga gtg caa ata     18276
Gln Ala Ala Phe Ile Gly Gln Ser Ile Thr Lys Asp Gly Val Gln Ile
            655                 660                 665

cag aac tca gaa aat caa cag gct gct gcc gac aaa act tac caa cca     18324
Gln Asn Ser Glu Asn Gln Gln Ala Ala Ala Asp Lys Thr Tyr Gln Pro
        670                 675                 680

gag cct caa att gga gtt tcc acc tgg gat acc aac gtt acc agt aac     18372
Glu Pro Gln Ile Gly Val Ser Thr Trp Asp Thr Asn Val Thr Ser Asn
    685                 690                 695

gct gcc gga cga gtg tta aaa gcc acc act ccc atg ctg cca tgt tac     18420
Ala Ala Gly Arg Val Leu Lys Ala Thr Thr Pro Met Leu Pro Cys Tyr
700                 705                 710                 715

ggt tca tat gcc aat ccc act aat cca aac ggg ggt cag gca aaa aca     18468
Gly Ser Tyr Ala Asn Pro Thr Asn Pro Asn Gly Gly Gln Ala Lys Thr
                720                 725                 730

gaa gga gac att tcg cta aac ttt ttc aca aca act gcg gca gca gac     18516
Glu Gly Asp Ile Ser Leu Asn Phe Phe Thr Thr Thr Ala Ala Ala Asp
            735                 740                 745

aat aat ccc aaa gtg gtt ctt tac agc gaa gat gta aac ctt caa gcc     18564
Asn Asn Pro Lys Val Val Leu Tyr Ser Glu Asp Val Asn Leu Gln Ala
        750                 755                 760

ccc gat act cac tta gta tat aag cca acg gtg gga gaa aac gtt atc     18612
Pro Asp Thr His Leu Val Tyr Lys Pro Thr Val Gly Glu Asn Val Ile
    765                 770                 775

gcc gca gaa gcc ctg cta acg cag cag gcg tgt ccc aac aga gca aac     18660
Ala Ala Glu Ala Leu Leu Thr Gln Gln Ala Cys Pro Asn Arg Ala Asn
780                 785                 790                 795

tac ata ggt ttc cga gat aac ttt atc ggt tta atg tat tat aac agc     18708
Tyr Ile Gly Phe Arg Asp Asn Phe Ile Gly Leu Met Tyr Tyr Asn Ser
                800                 805                 810

aca ggg aac atg gga gtt ctg gca ggt cag gcc tcg cag tta aac gca     18756
Thr Gly Asn Met Gly Val Leu Ala Gly Gln Ala Ser Gln Leu Asn Ala
            815                 820                 825

gtt gta gac ctg caa gat cga aac acg gaa ctg tcc tat cag cta atg     18804
Val Val Asp Leu Gln Asp Arg Asn Thr Glu Leu Ser Tyr Gln Leu Met
        830                 835                 840

cta gat gct ctg ggt gac aga act cga tat ttc tca atg tgg aat cag     18852
Leu Asp Ala Leu Gly Asp Arg Thr Arg Tyr Phe Ser Met Trp Asn Gln
    845                 850                 855

gcc gtg gac agc tac gat cca gac gtt agg att atc gag aac cat ggg     18900
Ala Val Asp Ser Tyr Asp Pro Asp Val Arg Ile Ile Glu Asn His Gly
860                 865                 870                 875

gtg gaa gac gag ctg ccc aat tac tgt ttt cca ctc cca ggc atg ggt     18948
Val Glu Asp Glu Leu Pro Asn Tyr Cys Phe Pro Leu Pro Gly Met Gly
                880                 885                 890

att ttt aac tcc tac aag ggg gta aaa cca caa aat ggc ggt aat ggt 18996
Ile Phe Asn Ser Tyr Lys Gly Val Lys Pro Gln Asn Gly Gly Asn Gly
            895                 900                 905

aac tgg gaa gca aac ggg gac cta tca aat gcc aat gag atc gct tta 19044
Asn Trp Glu Ala Asn Gly Asp Leu Ser Asn Ala Asn Glu Ile Ala Leu
        910                 915                 920

gga aac att ttt gcc atg gaa att aac ctc cac gca aac ctg tgg cgc 19092
Gly Asn Ile Phe Ala Met Glu Ile Asn Leu His Ala Asn Leu Trp Arg
    925                 930                 935

agc ttc ttg tac agc aat gtg gcg ctg tac ctg cca gac agc tat aaa 19140
Ser Phe Leu Tyr Ser Asn Val Ala Leu Tyr Leu Pro Asp Ser Tyr Lys
940                 945                 950                 955

ttc act ccc gct aac atc act ctg ccc gcc aac caa aac acc tac gag 19188
Phe Thr Pro Ala Asn Ile Thr Leu Pro Ala Asn Gln Asn Thr Tyr Glu
                960                 965                 970

tat atc aac ggg cgc gtc act tct cca acc ctg gtg gac acc ttt gtt 19236
Tyr Ile Asn Gly Arg Val Thr Ser Pro Thr Leu Val Asp Thr Phe Val
            975                 980                 985

aac att gga gcc cga tgg tcg ccg gat ccc atg gac aac gtc aac ccc 19284
Asn Ile Gly Ala Arg Trp Ser Pro Asp Pro Met Asp Asn Val Asn Pro
        990                 995                 1000

ttt aac cat cac cgg aac gcg ggc ctc cgt tac cgc tcc atg ctg 19329
Phe Asn His His Arg Asn Ala Gly Leu Arg Tyr Arg Ser Met Leu
    1005                1010                1015

ctg gga aat gga cgc gtg gtg cct ttc cac ata caa gtg ccg caa 19374
Leu Gly Asn Gly Arg Val Val Pro Phe His Ile Gln Val Pro Gln
    1020                1025                1030

aaa ttt ttc gcg att aag aac ctc ctg ctt ttg ccc ggc tcc tac 19419
Lys Phe Phe Ala Ile Lys Asn Leu Leu Leu Leu Pro Gly Ser Tyr
    1035                1040                1045

act tac gag tgg agc ttc aga aaa gac gtg aac atg att ctg cag 19464
Thr Tyr Glu Trp Ser Phe Arg Lys Asp Val Asn Met Ile Leu Gln
    1050                1055                1060

agc acc ctg ggc aat gat ctt cga gtg gac ggg gcc agc gtc cgc 19509
Ser Thr Leu Gly Asn Asp Leu Arg Val Asp Gly Ala Ser Val Arg
    1065                1070                1075

att gac agc gtc aac ttg tac gcc aac ttt ttc ccc atg gcg cac 19554
Ile Asp Ser Val Asn Leu Tyr Ala Asn Phe Phe Pro Met Ala His
    1080                1085                1090

aac acc gct tct acc ttg gaa gcc atg ctg cga aac gac acc aac 19599
Asn Thr Ala Ser Thr Leu Glu Ala Met Leu Arg Asn Asp Thr Asn
    1095                1100                1105

gac cag tcg ttt aac gac tac ctc agc gcg gcc aac atg ctt tat 19644
Asp Gln Ser Phe Asn Asp Tyr Leu Ser Ala Ala Asn Met Leu Tyr
    1110                1115                1120

ccc att ccg gcc aac gcc acc aac gtt ccc att tcc att ccc tcc 19689
Pro Ile Pro Ala Asn Ala Thr Asn Val Pro Ile Ser Ile Pro Ser

1125                    1130                    1135

cgc aac tgg gcg gcc ttc cgg gga tgg agc ttc acc cgc ctt aaa      19734
Arg Asn Trp Ala Ala Phe Arg Gly Trp Ser Phe Thr Arg Leu Lys
    1140                    1145                    1150

gcc aag gaa acg cct tcc ttg ggc tcc ggc ttt gac ccc tac ttt      19779
Ala Lys Glu Thr Pro Ser Leu Gly Ser Gly Phe Asp Pro Tyr Phe
    1155                    1160                    1165

gtg tac tca ggc acc att cct tac ctg gac ggc agc ttt tac ctc      19824
Val Tyr Ser Gly Thr Ile Pro Tyr Leu Asp Gly Ser Phe Tyr Leu
    1170                    1175                    1180

aac cac act ttc aaa cgt ctg tcc atc atg ttc gat tct tcc gta      19869
Asn His Thr Phe Lys Arg Leu Ser Ile Met Phe Asp Ser Ser Val
    1185                    1190                    1195

agt tgg ccg ggc aac gac cgc ctc ctg acg ccg aac gag ttc gaa      19914
Ser Trp Pro Gly Asn Asp Arg Leu Leu Thr Pro Asn Glu Phe Glu
    1200                    1205                    1210

att aag cgc att gtg gac ggg gaa ggc tac aac gtg gct caa agt      19959
Ile Lys Arg Ile Val Asp Gly Glu Gly Tyr Asn Val Ala Gln Ser
    1215                    1220                    1225

aac atg acc aaa gac tgg ttt tta att caa atg ctc agc cac tac      20004
Asn Met Thr Lys Asp Trp Phe Leu Ile Gln Met Leu Ser His Tyr
    1230                    1235                    1240

aac atc ggc tac caa ggc ttc tat gtt ccc gag ggc tac aag gat      20049
Asn Ile Gly Tyr Gln Gly Phe Tyr Val Pro Glu Gly Tyr Lys Asp
    1245                    1250                    1255

cgg atg tat tct ttc ttc cga aac ttt cag ccc atg agc cgc cag      20094
Arg Met Tyr Ser Phe Phe Arg Asn Phe Gln Pro Met Ser Arg Gln
    1260                    1265                    1270

gtg ccg gat ccc acc gct gcc ggc tat caa gcc gtt ccc ctg ccc      20139
Val Pro Asp Pro Thr Ala Ala Gly Tyr Gln Ala Val Pro Leu Pro
    1275                    1280                    1285

aga caa cac aac aac tcg ggc ttt gtg ggg tac atg ggc ccg acc      20184
Arg Gln His Asn Asn Ser Gly Phe Val Gly Tyr Met Gly Pro Thr
    1290                    1295                    1300

atg cgc gaa gga cag cca tac ccg gcc aac tac ccc tat ccc ctg      20229
Met Arg Glu Gly Gln Pro Tyr Pro Ala Asn Tyr Pro Tyr Pro Leu
    1305                    1310                    1315

atc ggc gct acc gcc gtc ccc gcc att acc cag aaa aag ttt ttg      20274
Ile Gly Ala Thr Ala Val Pro Ala Ile Thr Gln Lys Lys Phe Leu
    1320                    1325                    1330

tgc gac cgc gtc atg tgg cgc ata cct ttt tcc agc aac ttt atg      20319
Cys Asp Arg Val Met Trp Arg Ile Pro Phe Ser Ser Asn Phe Met
    1335                    1340                    1345

tca atg ggg gcc ctg acc gac ctc gga cag aac atg ctt tac gct      20364
Ser Met Gly Ala Leu Thr Asp Leu Gly Gln Asn Met Leu Tyr Ala
    1350                    1355                    1360

aac tcc gcc cat gcc ctg gat atg act ttt gag gtg gac ccc atg      20409

Asn Ser Ala His Ala Leu Asp Met Thr Phe Glu Val Asp Pro Met
    1365                1370                1375

aac gag ccc acg ttg ctg tac atg ctt ttt gag gtg ttc gac gtg    20454
Asn Glu Pro Thr Leu Leu Tyr Met Leu Phe Glu Val Phe Asp Val
    1380                1385                1390

gtc aga gtg cac cag ccg cac cgc ggt att atc gag gcc gtg tac    20499
Val Arg Val His Gln Pro His Arg Gly Ile Ile Glu Ala Val Tyr
    1395                1400                1405

ctg cgc acc ccc ttc tct gcg ggc aat gcc acc aca taa gccgctgaac    20548
Leu Arg Thr Pro Phe Ser Ala Gly Asn Ala Thr Thr
    1410                1415                1420

tagctggttt ttaccccaga tcccatgggc tccacggaag acgaactgcg ggccattgtg    20608

cgagacctgg gctgcggacc ctacttcctg ggcacctttg acaagcggtt tcccgggttc    20668

gtgtctcctc gcaaactcgc gtgcgcgatc gtgaataccg ccggccgaga gaccggagga    20728

gagcattggc tagctctggg ctggaacccc cgctcgtcca cgtttttcct gttcgacccc    20788

tttggctttt cagaccaacg cttgaagcag atctatgcat ttgaatatga gggtctactc    20848

aagcgaagcg cgctggcctc ctccgccgat cactgtctaa ccctggtaaa gagcactcag    20908

acggttcagg gccctcacag cgccgcctgt ggcctttttt gttgcatgtt tttgcacgcc    20968

tttgtgaact ggccggacac ccccatggaa aacaacccca ccatggacct cctgactggc    21028

gttcccaact ccatgctcca aagccccagc gtgcagacca ccctcctcca aaaccagaaa    21088

aatctgtacg cctttctgca caagcactct ccctactttc gccgccatcg ggaacaaata    21148

gaaaatgcaa ccgcgtttaa caaaactctg taacgtttaa taaatgaact ttttattgaa    21208

ctggaaaacg ggtttgtgat ttttaaaaat caaaggggtt gagctggaca tccatgtggg    21268

aggccggaag ggtggtgttc ttgtactggt acttgggcag ccacttaaac tctggaatca    21328

caaacttggg cagcggtatt tctgggaagt tgtcgtgcca cagctggcgg gtcagctgaa    21388

gtgcctgcag aacatcgggg gcggagatct tgaagtcgca gtttatctgg ttcacggcac    21448

gcgcgttgcg gtacatggga ttggcacact gaaacaccag caggctggga ttcttgatgc    21508

tagccagggc cacggcgtcg gtcacgtcac cggtgtcttc tatgttggac agcgaaaaag    21568

gcgtgacttt gcaaagctgg cgtcccgcgc gaggcacgca atctcccagg tagttgcact    21628

cacagcggat gggcagaaga agatgcttgt ggccgcgggt catgtaggga taggccgctg    21688

ccataaaagc ttcgatctgc ctgaaagcct gcttggcctt gtgcccttcg gtataaaaaa    21748

caccgcagga cttgttggaa aaggtattac tggcgcaagc ggcatcgtga aagcaagcgc    21808

gtgcgtcttc gtttcgtaac tgcaccacgc tgcggcccca ccggttctga atcaccttgg    21868

ccctgccggg gttttccttg agagcgcgct ggccggcttc gctgcccaca tccatttcca    21928

cgacatgctc cttgttaatc atggccagac cgtggaggca gcgcagctcc tcgtcatcgt    21988

cggtgcagtg atgctcccac acgacgcagc cagtgggctc ccacttgggc ttggaggcct 22048
cggcaatgcc agaatacagg agaacgtagt ggtgcagaaa acgtcccatc atggtgccaa 22108
aggttttctg gctgctgaag gtcatcgggc agtacctcca gtcctcgtta agccaagtgt 22168
tgcagatctt cctgaagacc gtgtactgat cgggcataaa gtggaactca ttgcgctcgg 22228
tcttgtcgat cttatacttt tccatcagac tatgcataat ctccatgccc ttttcccagg 22288
cgcaaacaat cttggtgcta cacgggttag gtatggccaa agtggttggc ctctgaggcg 22348
gcgcttgttc ttcctcttga gccctctccc gactgacggg ggttgaaaga gggtgcccct 22408
tggggaacgg cttgaacacg gtctggcccg aggcgtcccg aagaatctgc atcgggggat 22468
tgctggccgt catggcgatg atctgacccc ggggctcctc cacttcgtcc tcctcgggac 22528
tttcctcgtg cttttcgggg gacggtacgg gagtaggggg aagagcgcgg cgcgccttct 22588
tcttgggcgg cagttccgga gcctgctctt gacgactggc cattgtcttc tcctaggcaa 22648
gaaaaacaag atggaagact ctttctcctc ctcctcgtca acgtcagaaa gcgagtcttc 22708
caccttaagc gccgagaact cccagcgcat agaatccgat gtgggctacg agactccccc 22768
cgcgaacttt tcgccgcccc ccataaacac taacgggtgg acggactacc tggccctagg 22828
agacgtactg ctgaagcaca tcaggcggca gagcgttatc gtgcaagatg ctctcaccga 22888
gcgactcgcg gttccgctgg aagtggcgga acttagcgcc gcctacgagc gaaccctctt 22948
ctccccaaag actcccccca agaggcaggc taacggcacc tgcgagccta accctcgact 23008
caacttctac cctgcctttg ccgtgccaga ggtactggct acgtaccaca tttttttcca 23068
aaaccacaaa atccctctct cgtgccgcgc caaccgcacc aaagccgatc gcgtgctgcg 23128
actggaggaa ggggctcgca tacctgagat tgcgtgtctg gaggaagtcc caaaaatctt 23188
tgaaggtctg ggccgcgacg aaaagcgagc agcaaacgct ctggaagaga acgcagagag 23248
tcacaacagc gccttggtag aactcgaggg cgacaacgcc agactggccg tcctcaaacg 23308
gtccatagaa gtcacgcact tcgcctaccc cgccgttaac ctccctccaa aagttatgac 23368
agcggtcatg gactcgctgc tcataaagcg cgctcagccc ttagacccag agcacgaaaa 23428
caacagtgac gaaggaaaac cggtggtttc tgatgaggag ttgagcaagt ggctgtcctc 23488
caacgacccc gccacgttgg aggaacgaag aaaaaccatg atggccgtgg tgctagttac 23548
cgtgcaatta gaatgtctgc agaggttctt ttcccaccca gagaccctga gaaaagtgga 23608
ggaaacgctg cactacacat ttaggcacgg ctacgtgaag caagcctgca agatttccaa 23668
cgtagaactt agcaacctca tctcctacct ggggatcttg cacgaaaacc gcctcggaca 23728
aaacgtgctg cacagcacac tgaaaggaga agcccgccga gactatgtgc gagactgcgt 23788
gttcctagcg ctagtgtaca cctggcagag cggaatggga gtctggcagc agtgcctgga 23848
ggacgaaaac ctcaaagagc ttgaaaagct gctggtgcgc tccagaaggg cactgtggac 23908

cagttttgac gagcgcaccg ccgcgcgaga cctagctgat attatttttc ctcccaagct 23968
ggtgcagact ctccgggaag gactgccaga ttttatgagt caaagcatct tgcaaaactt 24028
ccgctctttc atcttggaac gctcgggaat cttgcccgcc actagctgcg ccctacccac 24088
agattttgtg cctctccact accgcgaatg cccaccgccg ctgtggccgt acacttactt 24148
gcttaaactg gccaactttc taatgttcca ctctgacctg gcagaagacg ttagcggcga 24208
ggggctgcta gaatgccact gccgctgcaa cctgtgcacc ccccaccgct ctctagtatg 24268
caacactccc ctgctcaatg agacccagat catcggtacc tttgaaatcc agggaccctc 24328
cgacgcggaa aacggcaagc aggggtctgg gctaaaactc acagccggac tgtggacctc 24388
cgcctacttg cgcaaatttg taccagaaga ctatcacgcc caccaaatta aattttacga 24448
aaaccaatca aaaccaccca aaagcgagtt aacggcttgc gtcattacgc agagcagcat 24508
agttgggcag ttgcaagcca ttaacaaagc gcggcaagag tttctcctaa aaaaaggaaa 24568
aggggtctac ttggaccccc agaccggcga ggaactcaac ggaccctcct cagtcgcagg 24628
ttgtgtgccc catgccgccc aaaaagaaca cctcgcagtg gaacatgcca gagacggagg 24688
aagaggagtg gagcagtgtg agcaacagcg aaacggagga agagccgtgg cccgaggggt 24748
gcaacgggga agaggacacg gagggacggc gaagtcttcg ccgaagaact ctcgccgctg 24808
cccccgaagt cccagccggc cgcctcggcc caagatcccg cacacacccg tagatgggat 24868
agcaagacca aaaagccggg taagagaaac gctcgccccc gccagggcta ccgctcgtgg 24928
agaaagcaca aaaactgcat cttatcgtgc ttgctccagt gcggcggaga cgtttcgttc 24988
acccgtagat acttgctttt taacaaaggg gtggccgtcc cccgtaacgt cctccactac 25048
taccgtcact cttacagctc cgaagcggac ggctaagaaa acgcagcagt tgccggcggg 25108
aggactgcgt ctcagcgccc gagaaccccc agccaccagg gagctccgaa accgcatatt 25168
tcccaccctc tacgctatct ttcagcaaag ccgggggcag cagcaagaac tgaaaataaa 25228
aaaccgcacg ctgaggtcgc ttacccgaag ctgcctctat cacaagagcg aagagcagct 25288
gcagcgaacc ctggaggacg cagaagcgct gttccagaag tactgcgcga ccaccctaaa 25348
taactaaaaa agcccgcgcg cgggacttca aaccgtctga cgtcaccagc cgcgcgccaa 25408
aatgagcaaa gagattccca cgccttacat gtggagttac cagccgcaga tgggattagc 25468
cgccggcgcc gcccaggatt actccacgaa aatgaactgg ctcagcgccg ggccccacat 25528
gatttcccgc gtaaacgaca ttcgcgccca ccgcaatcag ctattgttag aacaggctgc 25588
tctgaccgcc acgccccgta ataacctgaa ccctcccagc tggccagctg ccctggtgta 25648
ccaggaaacg cctccaccca ccagcgtact tttgccccgt gacgcccagg cggaagtcca 25708
gatgactaac gcgggcgcgc aattagcggg cggatcccgg tttcggtaca gagttcacgg 25768

cgccgcaccc tatagcccag gtataaagag gctgatcatt cgaggcagag gtgtccagct 25828
caacgacgag acagtgagct cttcgcttgg tctacgacca gacggagtgt tccagctcgc 25888
gggctcgggc cgctcttcgt tcacgcctcg ccaggcatac ctgactctgc agagctctgc 25948
ctctcagcct cgctcgggag gaatcggacc ccttcagttt gtggaggagt ttgtgccctc 26008
ggtctacttt cagcctttct ccggatcgcc cggccagtac ccggacgagt tcatccccaa 26068
cttcgacgcg gtgagtgact ctgtggacgg ttatgactga tgtcgagccc gcttcagtgc 26128
tagtggaaca agcgcggctc aatcacctgg ttcgttgccg ccgccgctgc tgcgtggctc 26188
gcgacttgag cttagctctc aagtttgtaa aaaacccgtc cgaaaccggg agcgctgtgc 26248
acgggttgga gctagtgggt cctgagaagg ccaccatcca cgttctcaga aactttgtgg 26308
aaaaacccat tttggttaaa cgagatcagg ggccttttgt aatcagctta ctctgcacct 26368
gtaaccatgt tgaccttcac gactatttta tggatcattt gtgcgctgaa ttcaataagt 26428
aaagcgaatt cttaccaaga ttatgatgtc catgactgtt cctcgccact atacgatgtt 26488
gtgccagtaa actctcttgt cgacatctat ctgaactgtt ccttttggtc cgcacagctt 26548
acttggtact acggtgacac cgtcctttct ggctcactgg gcagctcaca cggaataaca 26608
cttcacctct tttcgccgtt tcgatacgga aactacagct gtcgtgccgg tacctgcctc 26668
cacgttttca atcttcagcc ctgtccaccg accaaacttg tatttgtcga ctctaagcac 26728
ttacagctca actgcagcat tctaggcccc agtatcttgt ggacatacaa taaaatcagg 26788
ttggtggaat ttgtctacta cccacccagc gcccgcggtt ttggggaaat tcctttccag 26848
atctactaca actatcttgc cacacattat gcaagtcaac agcaactaaa cttgcaagca 26908
cccttcacgc caggagagta ctcctgtcac gtaggctcct gcacagaaac ttttattctc 26968
ttcaacagat cttctgccat tgaacgcttc actactaact actttagaaa ccaagttgtg 27028
cttttcactg acgaaacccc taacgtcacc ctggactgtg catgtttttc tcatgacacc 27088
gtaacttgga ctcttaacaa tactctctgg ctcgcgttcg ataaccaaag cttgattgtt 27148
aaaaattttg atttaacctt tactaaaccc tctcctcgcg aaatagttat ctttgctcct 27208
tttaatccaa aaactacctt agcctgtcag gttttgttta agccttgcca aacaaacttt 27268
aagtttgttt atttgcctcc gcaatctgtc aaactcatag aaaaatacaa caaagcgccc 27328
gtcttggctc ctaaaacctt ctaccactgg ctaacctaca cggggctgtt tgcactaatt 27388
gtttttttcc taattaacat ttttatatgt ttcttgcctt cctccttctt ttcgcgaaca 27448
ccgttgccgc agaaagacct ctccttatta ctgtagcgct tgctatacaa aaccaagagt 27508
ggtcaaccgt gctctcaatc tattttcaat ttttcatttt gtccttaata ctttctctta 27568
ttgtcgttaa caatgatctg gagcattggt ctcgcctttt tttggctgct tagtgcaaaa 27628
gccactattt ttcacaggta tgtggaagaa ggaactagca ccctctttac gatacctgaa 27688

acaattaagg cggctgatga agtttcttgg tacaaaggct cgctctcaga cggcaaccac 27748
tcattctcag gacagaccct ttgcatccaa gaaacttatt ttaaatcaga actacaatac 27808
agctgcataa aaaacttttt ccatctctac aacatctcaa aaccctatga gggtatttac 27868
aatgccaagg tttcagacaa ctccagcaca cggaactttt actttaatct gacagttatt 27928
aaagcaattt ccattcctat ctgtgagttt agctcccagt ttctttctga aacctactgt 27988
ttaattacta taaactgcac taaaaatcgc cttcacacca ccataatcta caatcacaca 28048
caatcacctt gggttttaaa cctaaaattt tctccacaca tgccttcgca atttctcacg 28108
caagttaccg tctctaacat aagcaagcag tttggctttt actatccttt ccacgaactg 28168
tgcgaaataa ttgaagccga atatgaacca gactacttta cttacattgc cattggtgta 28228
atcgttgttt gcctttgctt tgttattggg gggtgtgttt atttgtacat tcagagaaaa 28288
atattgctct cgctgtgctc ctgcggttac aaagcagaag aaagaattaa aatctctaca 28348
ctttattaat gttttccaga aatggcaaaa ctaacgctcc tacttttgct tctcacgccg 28408
gtgacgcttt ttaccatcac tttttctgcc gccgccacac tcgaacctca atgtttgcca 28468
ccggttgaag tctactttgt ctacgtgttg ctgtgctgcg ttagcgtttg cagtataaca 28528
tgttttacct ttgtttttct tcagtgcatt gactacttct gggtcagact ctactaccgc 28588
agacacgcgc ctcagtatca aaatcaacaa attgccagac tactcggtct gccatgattg 28648
tcttgtattt taccctgatt ttttttcacc ttacttgcgc ttgtgatttt cacttcactc 28708
aattttggaa aacgcaatgc ttcgacccgc gcctctccaa cgactggatg atggctcttg 28768
caattgccac gcttggggcg tttggacttt ttagtggttt tgctttgcat tacaaattta 28828
agactccatg gacacatggc tttctttcag attttccagt tacacctact ccgccgcctc 28888
ccccggccat cgacgtgcct caggttccct caccttctcc atctgtctgc agctactttc 28948
atctgtaatg gccgacctag aatttgacgg agtgcaatct gagcaaaggg ctatacactt 29008
ccaacgccag tcggaccgcg aacgcaaaaa cagagagctg caaaccatac aaaacaccca 29068
ccaatgtaaa cgcgggatat tttgtattgt aaaacaagct aagctccact acgagcttct 29128
atctggcaac gaccacgagc tccaatacgt ggtcgatcag cagcgtcaaa cctgtgtatt 29188
cttaattgga gtttccccca ttaaagttac tcaaaccaag ggtgaaacca agggaaccat 29248
aaggtgctca tgtcacctgt cagaatgcct ttacactcta gttaaaaccc tatgtggctt 29308
acatgattct atccccttta attaaataaa cttactttaa atctgcaatc acttcttcgt 29368
ccttgttttt gtcgccatcc agcagcacca ccttcccctc ttcccaactt tcatagcata 29428
ttttccgaaa agaggcgtac tttcgccaca ccttaaaggg aacgtttact tcgctttcaa 29488
gctctcccac gatttcatt gcagat atg aaa cgc gcc aaa gtg gaa gaa gga 29541
Met Lys Arg Ala Lys Val Glu Glu Gly

                1425

ttt aac ccc gtt tat ccc tat gga tat tct act ccg act gac gtg 29586
Phe Asn Pro Val Tyr Pro Tyr Gly Tyr Ser Thr Pro Thr Asp Val
1430                1435                1440

gct cct ccc ttt gta gcc tct gac ggt ctt caa gaa aac cca cct 29631
Ala Pro Pro Phe Val Ala Ser Asp Gly Leu Gln Glu Asn Pro Pro
1445                1450                1455

ggg gtc ttg tcc cta aaa ata tcc aaa cct tta act ttt aat gcc 29676
Gly Val Leu Ser Leu Lys Ile Ser Lys Pro Leu Thr Phe Asn Ala
1460                1465                1470

tcc aag gct cta agc ctg gct att ggt cca gga tta aaa att caa 29721
Ser Lys Ala Leu Ser Leu Ala Ile Gly Pro Gly Leu Lys Ile Gln
1475                1480                1485

gat ggt aaa cta gtg ggg gag gga caa gca att ctt gca aac ctg 29766
Asp Gly Lys Leu Val Gly Glu Gly Gln Ala Ile Leu Ala Asn Leu
1490                1495                1500

ccg ctt caa atc acc aac aac aca att tca cta cgt ttt ggg aac 29811
Pro Leu Gln Ile Thr Asn Asn Thr Ile Ser Leu Arg Phe Gly Asn
1505                1510                1515

aca ctt gcc ttg aat gac aat aat gaa ctc caa acc aca cta aaa 29856
Thr Leu Ala Leu Asn Asp Asn Asn Glu Leu Gln Thr Thr Leu Lys
1520                1525                1530

tct tca tcg ccc ctt aaa atc aca gac cag act ctg tcc ctt aac 29901
Ser Ser Ser Pro Leu Lys Ile Thr Asp Gln Thr Leu Ser Leu Asn
1535                1540                1545

ata ggg gac agc ctt gca att aaa gat gac aaa cta gaa agc gct 29946
Ile Gly Asp Ser Leu Ala Ile Lys Asp Asp Lys Leu Glu Ser Ala
1550                1555                1560

ctt caa gcg acc ctc cca ctc tcc att agc aac aac acc atc agc 29991
Leu Gln Ala Thr Leu Pro Leu Ser Ile Ser Asn Asn Thr Ile Ser
1565                1570                1575

ctc aac gtg ggc acc gga ctc acc ata aat gga aac gtt tta caa 30036
Leu Asn Val Gly Thr Gly Leu Thr Ile Asn Gly Asn Val Leu Gln
1580                1585                1590

gct gtt ccc tta aat gct cta agt ccc cta act att tcc aac aat 30081
Ala Val Pro Leu Asn Ala Leu Ser Pro Leu Thr Ile Ser Asn Asn
1595                1600                1605

aac atc agc ctg cgc tat ggc agt tcc ctg acg gtg ctt aac aat 30126
Asn Ile Ser Leu Arg Tyr Gly Ser Ser Leu Thr Val Leu Asn Asn
1610                1615                1620

gaa ctg caa agc aac ctc aca gtt cac tcc cct tta aaa ctc aac 30171
Glu Leu Gln Ser Asn Leu Thr Val His Ser Pro Leu Lys Leu Asn
1625                1630                1635

tcc aac aac tca att tct ctc aac act cta tct ccg ttt aga atc 30216
Ser Asn Asn Ser Ile Ser Leu Asn Thr Leu Ser Pro Phe Arg Ile
1640                1645                1650

gag aat ggt ttc ctc acg ctc tat ttg gga aca aaa tct ggc ttg 30261

Glu Asn Gly Phe Leu Thr Leu Tyr Leu Gly Thr Lys Ser Gly Leu
1655                1660                1665

cta gtt caa aac agt ggc tta aaa gtt caa gcg ggc tac ggc ctg 30306
Leu Val Gln Asn Ser Gly Leu Lys Val Gln Ala Gly Tyr Gly Leu
1670                1675                1680

caa gta aca gac acc aat gct ctc aca tta aga tat ctc gct cca 30351
Gln Val Thr Asp Thr Asn Ala Leu Thr Leu Arg Tyr Leu Ala Pro
1685                1690                1695

ctg acc att cca gac tcg ggc tca gaa caa ggc att ctt aaa gta 30396
Leu Thr Ile Pro Asp Ser Gly Ser Glu Gln Gly Ile Leu Lys Val
1700                1705                1710

aac act gga cag ggc cta agt gtg aac caa gct gga gcg ctt gaa 30441
Asn Thr Gly Gln Gly Leu Ser Val Asn Gln Ala Gly Ala Leu Glu
1715                1720                1725

aca tcc cta gga ggt gga tta aaa tat gct gat aac aaa ata acc 30486
Thr Ser Leu Gly Gly Gly Leu Lys Tyr Ala Asp Asn Lys Ile Thr
1730                1735                1740

ttt gat aca gga aac gga ctg aca tta tct gaa aat aaa ctt gca 30531
Phe Asp Thr Gly Asn Gly Leu Thr Leu Ser Glu Asn Lys Leu Ala
1745                1750                1755

gta gct gca ggt agt ggt cta act ttt aga gat ggt gcc ttg gta 30576
Val Ala Ala Gly Ser Gly Leu Thr Phe Arg Asp Gly Ala Leu Val
1760                1765                1770

gcc acg gga acc gca ttt acg caa aca ctg tgg act acg gct gat 30621
Ala Thr Gly Thr Ala Phe Thr Gln Thr Leu Trp Thr Thr Ala Asp
1775                1780                1785

ccg tct ccc aac tgc aca att ata cag gac cgc gac aca aaa ttt 30666
Pro Ser Pro Asn Cys Thr Ile Ile Gln Asp Arg Asp Thr Lys Phe
1790                1795                1800

act ttg gcg ctt acc att agt ggg agc caa gtg ctg ggg acg gtt 30711
Thr Leu Ala Leu Thr Ile Ser Gly Ser Gln Val Leu Gly Thr Val
1805                1810                1815

tcc att att gga gta aaa ggc ccc ctt tca agt agc ata ccg tca 30756
Ser Ile Ile Gly Val Lys Gly Pro Leu Ser Ser Ser Ile Pro Ser
1820                1825                1830

gct acc gtt aca gta caa ctt aac ttt gat tcc aac gga gcc cta 30801
Ala Thr Val Thr Val Gln Leu Asn Phe Asp Ser Asn Gly Ala Leu
1835                1840                1845

ttg agc tcc tct tca ctt aaa ggt tac tgg ggg tat cgc caa ggt 30846
Leu Ser Ser Ser Ser Leu Lys Gly Tyr Trp Gly Tyr Arg Gln Gly
1850                1855                1860

ccc tca att gac cct tac ccc ata att aat gcc tta aac ttt atg 30891
Pro Ser Ile Asp Pro Tyr Pro Ile Ile Asn Ala Leu Asn Phe Met
1865                1870                1875

cca aac tca ctg gct tat ccc ccg gga caa gaa atc caa gca aaa 30936
Pro Asn Ser Leu Ala Tyr Pro Pro Gly Gln Glu Ile Gln Ala Lys
1880                1885                1890

tgt aac atg tac gtt tct act ttt tta cga gga aat cca caa aga 30981
Cys Asn Met Tyr Val Ser Thr Phe Leu Arg Gly Asn Pro Gln Arg
1895                1900                1905

cca ata gtt tta aac atc act ttt aat aat caa acc agc ggg ttt 31026
Pro Ile Val Leu Asn Ile Thr Phe Asn Asn Gln Thr Ser Gly Phe
1910                1915                1920

tcc att aga ttt aca tgg aca aat tta acc aca gga gaa gca ttt 31071
Ser Ile Arg Phe Thr Trp Thr Asn Leu Thr Thr Gly Glu Ala Phe
1925                1930                1935

gca atg ccc cca tgc act ttt tcc tac att gct gaa caa caa taa 31116
Ala Met Pro Pro Cys Thr Phe Ser Tyr Ile Ala Glu Gln Gln
1940                1945                1950

actatgtaac cctcaccgtt aacccgcctc cgcccttcca ttttatttta taaaccaccc 31176

gatccacctt ttcagcagta aacaattgca tgtcagtagg ggcagtaaaa cttttgggag 31236

ttaaaatcca cacaggttct tcacaagcta agcgaaaatc agttacactt ataaaaccat 31296

cgctaacatc ggacaaagac aagcatgagt ccaaagcttc cggttctgga tcagattttt 31356

gttcattaac agcgggagaa acagcttctg gaggattttc catctccatc tccttcatca 31416

gttccaccat gtccaccgtg gtcatctggg acgagaacga cagttgtcat acacctcata 31476

agtcaccggt cgatgacgaa cgtacagatc tcgaagaatg tcctgtcgcc gcctttcggc 31536

agcactgggc cgaaggcgaa agcgcccatg tttaacaatg gccagcaccg cccgcttcat 31596

caggcgccta gttcttttag cgcaacagcg catgcgcagc tcgctaagac tggcgcaaga 31656

aacacagcac agaaccacca gattgttcat gatcccataa gcgtgctgac accagcccat 31716

actaacaaat tgtttcacta ttctagcatg aatgtcatat ctgatgttca agtaaattaa 31776

atggcgcccc cttatgtaaa cacttcccac gtacaacacc tcctttggca tctgataatt 31836

aaccacctcc cgataccaaa tacatctctg attaatagtc gccccgtaca ctacccgatt 31896

aaaccaagtt gccaacataa tcccccctgc catacactgc aaagaacctg gacggctaca 31956

atgacagtgc aaagtccaca cctcgttgcc atggataact gaggaacgcc ttaagtcaat 32016

agtggcacaa ctaatacaaa catgtaaata gtgtttcaac aagtgccact cgtatgaggt 32076

gagtatcatg tcccagggaa cgggccactc cataaacact gcaaaaccaa cacatcctac 32136

catcccccgc acggcactca catcgtgcat ggtgttcata tcacagtccg gaagctgagg 32196

acaaggaaaa gtctcgggag cattttcata gggcggtagt gggtactcct tgtaggggtt 32256

cagtcggcac cggtatctcc tcaccttctg ggccataaca cacaagttga gatctgattt 32316

caaggtactt tctgaatgaa aaccaagtgc tttcccaaca atgtatccga tgtcttcggt 32376

ccccgcgtcg gtagcgctcc ttgcagtaca cacggaacaa ccactcacgc aggcccagaa 32436

gacagttttc cgcggacggt gacaagttaa tccccctcag tctcagagcc aatatagttt 32496

cttccacagt agcataggcc aaacccaacc aggaaacaca agctggcacg tcccgttcaa 32556

cgggaggaca aggaagcaga ggcagaggca taggcaaagc aacagaattt ttattccaac 32616
tggtcacgta gcacttcaaa caccaggtca cgtaaatggc agcgatcttg ggtttcctga 32676
tggaacataa cagcaagatc aaacatgaga cgattctcaa ggtgattaac cacagctgga 32736
attaaatcct ccacgcgcac atttagaaac accagcaata caaaagcccg gttttctccg 32796
ggatctatca tagcagcaca gtcatcaatt agtcccaagt aattttcccg tttccaatct 32856
gttataattt gcagaataat gccctgtaaa tccaagccgg ccatggcgaa aagctcagat 32916
aatgcacttt ccacgtgcat tcgtaaacac accctcatct tgtcaatcca aaaagtcttc 32976
ttcttgagaa acctgtagta aattaagaat cgccaggtta ggctcgatgc ctacatcccg 33036
gagcttcatt ctcagcatgc actgcaaatg atccagcaga tcagaacagc aattagcagc 33096
cagctcatcc ccggtttcca gttccggagt tcccacggca attatcactc gaaacgtggg 33156
acaaatcgaa ataacatgag ctcccacgtg agcaaaagcc gtagggccag tgcaataatc 33216
acagaaccag cggaaaaaag attgcagctc atgtttcaaa aagctctgca gatcaaaatt 33276
cagctcatgc aaataacaca gtaaagtttg cggtatagta accgaaaacc acacgggtcg 33336
acgttcaaac atctcggctt acctaaaaaa gaagcacatt tttaaaccac agtcgcttcc 33396
tgaacaggag gaaatatggt gcggcgtaaa accagacgcg ccaccggatc tccggcagag 33456
ccctgataat acagccagct gtggttaaac agcaaaacct ttaattcggc aacggttgag 33516
gtctccacat aatcagcgcc cacaaaaatc ccatctcgaa cttgctcgcg tagggagcta 33576
aaatggccag tatagcccca tggcacccga acgctaatct gcaagtatat gagagccacc 33636
ccattcggcg ggatcacaaa atcagtcgga gaaaacaacg tatacacccc ggactgcaaa 33696
agctgttcag gcaaacgccc ctgcggtccc tctcggtaca ccagcaaagc ctcgggtaaa 33756
gcagccatgc caagcgctta ccgtgccaag agcgactcag acgaaaaagt gtactgaggc 33816
gctcagagca gcggctatat actctacctg tgacgtcaag aaccgaaagt caaaagttca 33876
cccggcgcgc ccgaaaaaac ccgcgaaaat ccacccaaaa agcccgcgaa aaacacttcc 33936
gtataaaatt tccgggttac cggcgcgtca ccgccgcgcg acacgcccgc cccgccccgc 33996
gctcctcccc gaaacccgcc gcgcccactt ccgcgttccc aagacaaagg tcgcgtaact 34056
ccgcccacct catttgcatg ttaactcggt cgccatcttg cggtgttata ttgatgatg 34115

<210> 35
<211> 503
<212> PRT
<213> simian adenovirus SV-39

<400> 35

Met Arg Arg Ala Val Ala Val Pro Ser Ala Ala Met Ala Leu Gly Pro
1               5                   10                  15

Pro Pro Ser Tyr Glu Ser Val Met Ala Ala Ala Thr Leu Gln Ala Pro
            20                  25                  30

Leu Glu Asn Pro Tyr Val Pro Pro Arg Tyr Leu Glu Pro Thr Gly Gly
        35                  40                  45

Arg Asn Ser Ile Arg Tyr Ser Glu Leu Thr Pro Leu Tyr Asp Thr Thr
    50                  55                  60

Arg Leu Tyr Leu Val Asp Asn Lys Ser Ala Asp Ile Ala Thr Leu Asn
65                  70                  75                  80

Tyr Gln Asn Asp His Ser Asn Phe Leu Thr Ser Val Val Gln Asn Ser
                85                  90                  95

Asp Tyr Thr Pro Ala Glu Ala Ser Thr Gln Thr Ile Asn Leu Asp Asp
            100                 105                 110

Arg Ser Arg Trp Gly Gly Asp Leu Lys Thr Ile Leu His Thr Asn Met
        115                 120                 125

Pro Asn Val Asn Glu Phe Met Phe Thr Asn Ser Phe Arg Ala Lys Leu
    130                 135                 140

Met Val Ala His Glu Ala Asp Lys Asp Pro Val Tyr Glu Trp Val Gln
145                 150                 155                 160

Leu Thr Leu Pro Glu Gly Asn Phe Ser Glu Ile Met Thr Ile Asp Leu
                165                 170                 175

Met Asn Asn Ala Ile Ile Asp His Tyr Leu Ala Val Ala Arg Gln Gln
            180                 185                 190

Gly Val Lys Glu Ser Glu Ile Gly Val Lys Phe Asp Thr Arg Asn Phe
        195                 200                 205

Arg Leu Gly Trp Asp Pro Glu Thr Gly Leu Val Met Pro Gly Val Tyr
    210                 215                 220

Thr Asn Glu Ala Phe His Pro Asp Val Val Leu Leu Pro Gly Cys Gly
225                 230                 235                 240

Val Asp Phe Thr Tyr Ser Arg Leu Asn Asn Leu Leu Gly Ile Arg Lys
                245                 250                 255

Arg Met Pro Phe Gln Glu Gly Phe Gln Ile Leu Tyr Glu Asp Leu Glu
            260                 265                 270

Gly Gly Asn Ile Pro Ala Leu Leu Asp Val Pro Ala Tyr Glu Glu Ser
        275                 280                 285

Ile Ala Asn Ala Arg Glu Ala Ala Ile Arg Gly Asp Asn Phe Ala Ala
    290                 295                 300

Gln Pro Gln Ala Ala Pro Thr Ile Lys Pro Val Leu Glu Asp Ser Lys
305                 310                 315                 320

Gly Arg Ser Tyr Asn Val Ile Ala Asn Thr Asn Asn Thr Ala Tyr Arg
                325                 330                 335

Ser Trp Tyr Leu Ala Tyr Asn Tyr Gly Asp Pro Glu Lys Gly Val Arg
            340                 345                 350

Ala Trp Thr Leu Leu Thr Thr Pro Asp Val Thr Cys Gly Ser Glu Gln
        355                 360                 365

Val Tyr Trp Ser Leu Pro Asp Met Tyr Val Asp Pro Val Thr Phe Arg
    370                 375                 380

Ser Thr Gln Gln Val Ser Asn Tyr Pro Val Val Gly Ala Glu Leu Met
385                 390                 395                 400

Pro Ile His Ser Lys Ser Phe Tyr Asn Glu Gln Ala Val Tyr Ser Gln
                405                 410                 415

Leu Ile Arg Gln Thr Thr Ala Leu Thr His Val Phe Asn Arg Phe Pro
            420                 425                 430

Glu Asn Gln Ile Leu Val Arg Pro Pro Ala Pro Thr Ile Thr Thr Val
        435                 440                 445

Ser Glu Asn Val Pro Ala Leu Thr Asp His Gly Thr Leu Pro Leu Gln
    450                 455                 460

Asn Ser Ile Arg Gly Val Gln Arg Val Thr Ile Thr Asp Ala Arg Arg
465                 470                 475                 480

Arg Thr Cys Pro Tyr Val Tyr Lys Ala Leu Gly Ile Val Ala Pro Arg
                485                 490                 495

Val Leu Ser Ser Arg Thr Phe
                500

<210>  36

<211>  917

<212>  PRT

<213>  simian adenovirus SV-39


<400>  36

Met Ala Thr Pro Ser Met Met Pro Gln Trp Ser Tyr Met His Ile Ala
1               5                   10                  15

Gly Gln Asp Ala Ser Glu Tyr Leu Ser Pro Gly Leu Val Gln Phe Ala
            20                  25                  30

Arg Ala Thr Glu Thr Tyr Phe Ser Leu Gly Asn Lys Phe Arg Asn Pro
        35                  40                  45

Thr Val Ala Pro Thr His Asp Val Thr Thr Asp Arg Ser Gln Arg Leu
    50                  55                  60

Thr Ile Arg Phe Val Pro Val Asp Lys Glu Asp Thr Ala Tyr Ser Tyr
65                  70                  75                  80

Lys Thr Arg Phe Thr Leu Ala Val Gly Asp Asn Arg Val Leu Asp Met
                85                  90                  95

Ala Ser Thr Tyr Phe Asp Ile Arg Gly Val Ile Asp Arg Gly Pro Ser
            100                 105                 110

Phe Lys Pro Tyr Ser Gly Thr Ala Tyr Asn Ser Leu Ala Pro Lys Gly
        115                 120                 125

Ala Pro Asn Asn Ser Gln Trp Asn Ala Thr Asp Asn Gly Asn Lys Pro
    130                 135                 140

Val Cys Phe Ala Gln Ala Ala Phe Ile Gly Gln Ser Ile Thr Lys Asp
145                 150                 155                 160

Gly Val Gln Ile Gln Asn Ser Glu Asn Gln Gln Ala Ala Ala Asp Lys
                165                 170                 175

Thr Tyr Gln Pro Glu Pro Gln Ile Gly Val Ser Thr Trp Asp Thr Asn
            180                 185                 190

Val Thr Ser Asn Ala Ala Gly Arg Val Leu Lys Ala Thr Thr Pro Met
        195                 200                 205

Leu Pro Cys Tyr Gly Ser Tyr Ala Asn Pro Thr Asn Pro Asn Gly Gly
    210                 215                 220

Gln Ala Lys Thr Glu Gly Asp Ile Ser Leu Asn Phe Phe Thr Thr Thr
225                 230                 235                 240

Ala Ala Ala Asp Asn Asn Pro Lys Val Val Leu Tyr Ser Glu Asp Val
                245                 250                 255

Asn Leu Gln Ala Pro Asp Thr His Leu Val Tyr Lys Pro Thr Val Gly
            260                 265                 270

Glu Asn Val Ile Ala Ala Glu Ala Leu Leu Thr Gln Gln Ala Cys Pro
        275                 280                 285

Asn Arg Ala Asn Tyr Ile Gly Phe Arg Asp Asn Phe Ile Gly Leu Met
    290                 295                 300

Tyr Tyr Asn Ser Thr Gly Asn Met Gly Val Leu Ala Gly Gln Ala Ser
305                 310                 315                 320

Gln Leu Asn Ala Val Val Asp Leu Gln Asp Arg Asn Thr Glu Leu Ser
                325                 330                 335

Tyr Gln Leu Met Leu Asp Ala Leu Gly Asp Arg Thr Arg Tyr Phe Ser
            340                 345                 350

Met Trp Asn Gln Ala Val Asp Ser Tyr Asp Pro Asp Val Arg Ile Ile
        355                 360                 365

Glu Asn His Gly Val Glu Asp Glu Leu Pro Asn Tyr Cys Phe Pro Leu
    370                 375                 380

Pro Gly Met Gly Ile Phe Asn Ser Tyr Lys Gly Val Lys Pro Gln Asn
385                 390                 395                 400

Gly Gly Asn Gly Asn Trp Glu Ala Asn Gly Asp Leu Ser Asn Ala Asn
                405                 410                 415

Glu Ile Ala Leu Gly Asn Ile Phe Ala Met Glu Ile Asn Leu His Ala
            420                 425                 430

Asn Leu Trp Arg Ser Phe Leu Tyr Ser Asn Val Ala Leu Tyr Leu Pro

435                 440                 445

Asp Ser Tyr Lys Phe Thr Pro Ala Asn Ile Thr Leu Pro Ala Asn Gln
    450                 455                 460

Asn Thr Tyr Glu Tyr Ile Asn Gly Arg Val Thr Ser Pro Thr Leu Val
465                 470                 475                 480

Asp Thr Phe Val Asn Ile Gly Ala Arg Trp Ser Pro Asp Pro Met Asp
                485                 490                 495

Asn Val Asn Pro Phe Asn His His Arg Asn Ala Gly Leu Arg Tyr Arg
            500                 505                 510

Ser Met Leu Leu Gly Asn Gly Arg Val Val Pro Phe His Ile Gln Val
        515                 520                 525

Pro Gln Lys Phe Phe Ala Ile Lys Asn Leu Leu Leu Leu Pro Gly Ser
    530                 535                 540

Tyr Thr Tyr Glu Trp Ser Phe Arg Lys Asp Val Asn Met Ile Leu Gln
545                 550                 555                 560

Ser Thr Leu Gly Asn Asp Leu Arg Val Asp Gly Ala Ser Val Arg Ile
                565                 570                 575

Asp Ser Val Asn Leu Tyr Ala Asn Phe Phe Pro Met Ala His Asn Thr
            580                 585                 590

Ala Ser Thr Leu Glu Ala Met Leu Arg Asn Asp Thr Asn Asp Gln Ser
        595                 600                 605

Phe Asn Asp Tyr Leu Ser Ala Ala Asn Met Leu Tyr Pro Ile Pro Ala
    610                 615                 620

Asn Ala Thr Asn Val Pro Ile Ser Ile Pro Ser Arg Asn Trp Ala Ala
625                 630                 635                 640

Phe Arg Gly Trp Ser Phe Thr Arg Leu Lys Ala Lys Glu Thr Pro Ser
                645                 650                 655

Leu Gly Ser Gly Phe Asp Pro Tyr Phe Val Tyr Ser Gly Thr Ile Pro
            660                 665                 670

Tyr Leu Asp Gly Ser Phe Tyr Leu Asn His Thr Phe Lys Arg Leu Ser
        675                 680                 685

Ile Met Phe Asp Ser Ser Val Ser Trp Pro Gly Asn Asp Arg Leu Leu
    690                 695                 700

Thr Pro Asn Glu Phe Glu Ile Lys Arg Ile Val Asp Gly Glu Gly Tyr
705                 710                 715                 720

Asn Val Ala Gln Ser Asn Met Thr Lys Asp Trp Phe Leu Ile Gln Met
                725                 730                 735

Leu Ser His Tyr Asn Ile Gly Tyr Gln Gly Phe Tyr Val Pro Glu Gly
            740                 745                 750

Tyr Lys Asp Arg Met Tyr Ser Phe Phe Arg Asn Phe Gln Pro Met Ser
        755                 760                 765

Arg Gln Val Pro Asp Pro Thr Ala Ala Gly Tyr Gln Ala Val Pro Leu
    770                 775                 780

Pro Arg Gln His Asn Asn Ser Gly Phe Val Gly Tyr Met Gly Pro Thr
785                 790                 795                 800

Met Arg Glu Gly Gln Pro Tyr Pro Ala Asn Tyr Pro Tyr Pro Leu Ile
                805                 810                 815

Gly Ala Thr Ala Val Pro Ala Ile Thr Gln Lys Lys Phe Leu Cys Asp
            820                 825                 830

Arg Val Met Trp Arg Ile Pro Phe Ser Ser Asn Phe Met Ser Met Gly
        835                 840                 845

Ala Leu Thr Asp Leu Gly Gln Asn Met Leu Tyr Ala Asn Ser Ala His
    850                 855                 860

Ala Leu Asp Met Thr Phe Glu Val Asp Pro Met Asn Glu Pro Thr Leu
865                 870                 875                 880

Leu Tyr Met Leu Phe Glu Val Phe Asp Val Val Arg Val His Gln Pro
                885                 890                 895

His Arg Gly Ile Ile Glu Ala Val Tyr Leu Arg Thr Pro Phe Ser Ala
            900                 905                 910

Gly Asn Ala Thr Thr
        915

<210>  37

<211>  533

<212>  PRT

<213>  simian adenovirus SV-39

<400>  37

Met Lys Arg Ala Lys Val Glu Glu Gly Phe Asn Pro Val Tyr Pro Tyr
1               5                   10                  15

Gly Tyr Ser Thr Pro Thr Asp Val Ala Pro Pro Phe Val Ala Ser Asp
            20                  25                  30

Gly Leu Gln Glu Asn Pro Pro Gly Val Leu Ser Leu Lys Ile Ser Lys
        35                  40                  45

Pro Leu Thr Phe Asn Ala Ser Lys Ala Leu Ser Leu Ala Ile Gly Pro
    50                  55                  60

Gly Leu Lys Ile Gln Asp Gly Lys Leu Val Gly Glu Gly Gln Ala Ile
65                  70                  75                  80

Leu Ala Asn Leu Pro Leu Gln Ile Thr Asn Asn Thr Ile Ser Leu Arg
                85                  90                  95

Phe Gly Asn Thr Leu Ala Leu Asn Asp Asn Asn Glu Leu Gln Thr Thr
            100                 105                 110

Leu Lys Ser Ser Ser Pro Leu Lys Ile Thr Asp Gln Thr Leu Ser Leu
        115                 120                 125

Asn Ile Gly Asp Ser Leu Ala Ile Lys Asp Asp Lys Leu Glu Ser Ala
    130                 135                 140

Leu Gln Ala Thr Leu Pro Leu Ser Ile Ser Asn Asn Thr Ile Ser Leu
145                 150                 155                 160

Asn Val Gly Thr Gly Leu Thr Ile Asn Gly Asn Val Leu Gln Ala Val
                165                 170                 175

Pro Leu Asn Ala Leu Ser Pro Leu Thr Ile Ser Asn Asn Asn Ile Ser
            180                 185                 190

Leu Arg Tyr Gly Ser Ser Leu Thr Val Leu Asn Asn Glu Leu Gln Ser
        195                 200                 205

Asn Leu Thr Val His Ser Pro Leu Lys Leu Asn Ser Asn Asn Ser Ile
    210                 215                 220

Ser Leu Asn Thr Leu Ser Pro Phe Arg Ile Glu Asn Gly Phe Leu Thr
225                 230                 235                 240

Leu Tyr Leu Gly Thr Lys Ser Gly Leu Leu Val Gln Asn Ser Gly Leu
                245                 250                 255

Lys Val Gln Ala Gly Tyr Gly Leu Gln Val Thr Asp Thr Asn Ala Leu
            260                 265                 270

Thr Leu Arg Tyr Leu Ala Pro Leu Thr Ile Pro Asp Ser Gly Ser Glu
        275                 280                 285

Gln Gly Ile Leu Lys Val Asn Thr Gly Gln Gly Leu Ser Val Asn Gln
    290                 295                 300

Ala Gly Ala Leu Glu Thr Ser Leu Gly Gly Gly Leu Lys Tyr Ala Asp
305                 310                 315                 320

Asn Lys Ile Thr Phe Asp Thr Gly Asn Gly Leu Thr Leu Ser Glu Asn
                325                 330                 335

Lys Leu Ala Val Ala Ala Gly Ser Gly Leu Thr Phe Arg Asp Gly Ala
            340                 345                 350

Leu Val Ala Thr Gly Thr Ala Phe Thr Gln Thr Leu Trp Thr Thr Ala
        355                 360                 365

Asp Pro Ser Pro Asn Cys Thr Ile Ile Gln Asp Arg Asp Thr Lys Phe
    370                 375                 380

Thr Leu Ala Leu Thr Ile Ser Gly Ser Gln Val Leu Gly Thr Val Ser
385                 390                 395                 400

Ile Ile Gly Val Lys Gly Pro Leu Ser Ser Ser Ile Pro Ser Ala Thr
                405                 410                 415

Val Thr Val Gln Leu Asn Phe Asp Ser Asn Gly Ala Leu Leu Ser Ser
            420                 425                 430

Ser Ser Leu Lys Gly Tyr Trp Gly Tyr Arg Gln Gly Pro Ser Ile Asp
        435                 440                 445

Pro Tyr Pro Ile Ile Asn Ala Leu Asn Phe Met Pro Asn Ser Leu Ala
    450                 455                 460

Tyr Pro Pro Gly Gln Glu Ile Gln Ala Lys Cys Asn Met Tyr Val Ser

465                 470                 475                 480

Thr Phe Leu Arg Gly Asn Pro Gln Arg Pro Ile Val Leu Asn Ile Thr
                485                 490                 495

Phe Asn Asn Gln Thr Ser Gly Phe Ser Ile Arg Phe Thr Trp Thr Asn
            500                 505                 510

Leu Thr Thr Gly Glu Ala Phe Ala Met Pro Pro Cys Thr Phe Ser Tyr
        515                 520                 525

Ile Ala Glu Gln Gln
    530

<210>  38

<211>  50

<212>  DNA

<213>  Artificial sequence

<220>

<223>  oligomer SV25T

<400>  38
aatttaaata cgtagcgcac tagtcgcgct aagcgcggat atcatttaaa                50

<210>  39

<211>  49

<212>  DNA

<213>  Artificial sequence

<220>

<223>  oligomer SV25B

<400>  39
tatttaaatg atatccgcgc ttagcgcgac tagtgcgct acgtattta                49

## Description

### BACKGROUND OF THE INVENTION

Adenovirus is a double-stranded DNA virus with a genome size of about 36 kilobases (kb), which has been widely used for gene transfer applications due to its ability to achieve highly efficient gene transfer in a variety of target tissues and large transgene capacity. Conventionally, E1 genes of adenovirus are deleted and replaced with a transgene cassette consisting of the promoter of choice, cDNA sequence of the gene of interest and a poly A signal, resulting in a replication defective recombinant virus.

Adenoviruses have a characteristic morphology with an icosahedral capsid consisting of three major proteins, hexon (II), penton base (III) and a knobbed fibre (IV), along with a number of other minor proteins, VI, VIII, IX, IIIa and IVa2 [W.C. Russell, J. Gen Virol., 81:2573-2604 (Nov 2000)]. The virus genome is a linear, double-stranded DNA with a terminal protein attached covalently to the 5' termini, which have inverted terminal repeats (ITRs). The virus DNA is intimately associated with the highly basic protein VII and a small peptide termed mu. Another protein, V, is packaged with this DNA-protein complex and provides a structural link to the capsid via protein VI. The virus also contains a virus-encoded protease, which is necessary for processing of some of the structural proteins to produce mature infectious virus.

Recombinant adenoviruses have been described for delivery of molecules to host cells. See, US Patent 6,083,716, which describes the genome of two chimpanzee adenoviruses.

What is needed in the art are more effective vectors which avoid the effect of pre-existing immunity to selected adenovirus serotypes in the population and/or which are useful for repeat administration and for titer boosting by second vaccination, if required.

### Summary of the Invention

The present invention provides the isolated nucleic acid sequences and amino acid sequences of six simian adenoviruses, vectors containing these sequences, and cell lines expressing simian adenovirus genes. Also provided are a number of methods for using the vectors and cells of the invention.

The methods of the invention involve delivering one or more selected heterologous gene(s) to a mammalian patient by administering a vector of the invention. Because the various vector constructs are derived from simian rather than from human adenoviruses, the immune system of the non-simian human or veterinary patient is not primed to respond immediately to the vector as a foreign antigen. Use of the compositions of this invention thus permits a more stable expression of the selected transgene when administered to a non-simian patient. Use of the compositions of this invention for vaccination permits presentation of a selected antigen for the elicitation of protective immune responses. Without wishing to be bound by theory, the ability of the adenoviruses of the invention to transduce human dendritic cells is at least partially responsible for the ability of the recombinant constructs of the invention to induce an immune response. The recombinant simian adenoviruses of this invention may also be used for producing heterologous gene products *in vitro.* Such gene products are themselves useful in a variety for a variety of purposes such as are described herein.

These and other embodiments and advantages of the invention are described in more detail below.

### Brief Description of the Drawings

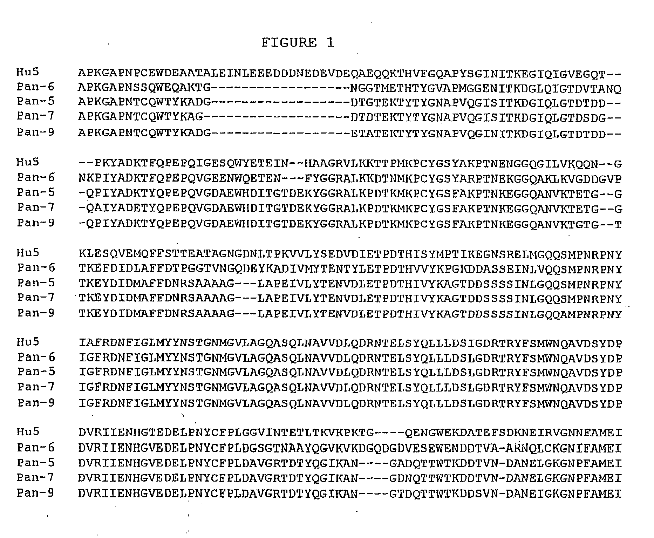

Fig. 1 provides an alignment of the amino acid sequences of the L1 and a portion of the L2 loops of the capsid protein hexon of the chimpanzee adenovirus C1 [SEQ ID NO:13], chimpanzee adenovirus C68 (Pan-9) [SEQ ID NO:14], and the novel Pan5 [SEQ ID NO:15], Pan6 [SEQ ID NO: 16] and Pan7 [SEQ ID NO: 17] chimpanzee adenovirus sequences of the invention. The intervening conserved region is part of the pedestal domain conserved between adenovirus serotypes.

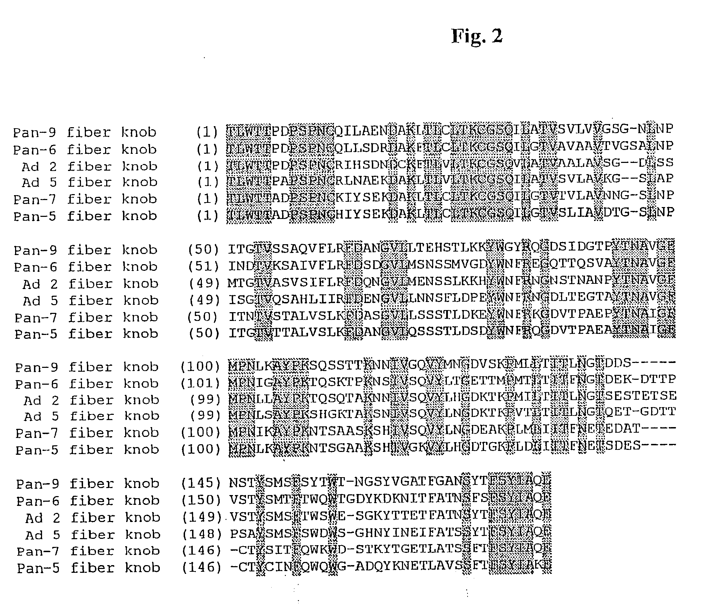

Fig. 2 provides an alignment of the amino acid sequences of the fiber knob domains of chimpanzee C68 (Pan-9) [SEQ ID NO:18], Pan-6 [SEQ ID NO:19], Pan-7 [SEQ ID NO:20], and Pan-5 [SEQ ID NO:21] and the human adenoviruses serotypes 2 [SEQ ID NO:22] and [SEQ ID NO:23].

### DETAILED DESCRIPTION OF THE INVENTION

The invention provides novel nucleic acid and amino acid sequences from Ad Pan5 [SEQ ID NO:1-4, 15 and 21], Ad Pan6 [SEQ ID NO: 5-8, 16, 19], and Ad serotype Pan7 [SEQ ID NO: 9-12, 17,20], which were originally isolated from chimpanzee lymph nodes. In several instances throughout the specification, these adenoviruses are alternatively termed herein C5, C6 and C7, respectively. Also provided are sequences from adenovirus SV1 [SEQ ID NO: 24-28], which was originally isolated from the kidney cells of cynomolgus monkey. The invention also provides sequences of adenoviruses SV-25 [SEQ ID NO:29-33] and SV-39 [SEQ ID NO: 34-37], which were originally isolated from rhesus monkey kidney cells.

The present invention provides novel adenovirus vectors and packaging cell lines to produce those vectors for use in the *in vitro* production of recombinant proteins or fragments or other reagents. The invention further provides compositions for use in delivering a heterologous molecule for therapeutic or vaccine purposes. Such therapeutic or vaccine compositions contain the adenoviral vectors carrying an inserted heterologous molecule. In addition, novel sequences of the invention are useful in providing the essential helper functions required for production of recombinant adeno-associated viral (AAV) vectors. Thus, the invention provides helper constructs, methods and cell lines which use these sequences in such production methods.

The term "substantial homology" or "substantial similarity," when referring to a nucleic acid or fragment thereof, indicates that, when optimally aligned with appropriate nucleotide insertions or deletions with another nucleic acid (or its complementary strand), there is nucleotide sequence identity in at least about 95 to 99% of the aligned sequences.

The term "substantial homology" or "substantial similarity," when referring to amino acids or fragments thereof, indicates that, when optimally aligned with appropriate amino acid insertions or deletions with another amino acid (or its complementary strand), there is amino acid sequence identity in at least about 95 to 99% of the aligned sequences. Preferably, the homology is over full-length sequence, or a protein thereof, or a fragment thereof which is at least 8 amino acids, or more desirably, at least 15 amino acids in length. Examples of suitable fragments are described herein.

The term "percent sequence identity" or "identical" in the context of nucleic acid sequences refers to the residues in the two sequences that are the same when aligned for maximum correspondence. The length of sequence identity comparison may be over the full-length of the genome (e.g., about 36 kbp), the full-length of an open reading frame of a gene, protein, subunit, or enzyme [see, e.g., the tables providing the adenoviral coding regions], or a fragment of at least about 500 to 5000 nucleotides, is desired. However, identity among smaller fragments, e.g. of at least about nine nucleotides, usually at least about 20 to 24 nucleotides, at least about 28 to 32 nucleotides, at least about 36 or more nucleotides, may also be desired. Similarly, "percent sequence identity" may be readily determined for amino acid sequences, over the full-length of a protein, or a fragment thereof. Suitably, a fragment is at least about 8 amino acids in length, and may be up to about 700 amino acids. Examples of suitable fragments are described herein.

Identity is readily determined using such algorithms and computer programs as are defined herein at default settings. Preferably, such identity is over the full length of the protein, enzyme, subunit, or over a fragment of at least about 8 amino acids in length. However, identity may be based upon shorter regions, where suited to the use to which the identical gene product is being put.

As described herein, alignments are performed using any of a variety of publicly or commercially available Multiple Sequence Alignment Programs, such as "Clustal W", accessible through Web Servers on the internet. Alternatively, Vector NTI utilities are also used. There are also a number of algorithms known in the art that can be used to measure nucleotide sequence identity, including those contained in the programs described above. As another example, polynucleotide sequences can be compared using Fasta, a program in GCG Version 6.1. Fasta provides alignments and percent sequence identity of the regions of the best overlap between the query and search sequences. For instance, percent sequence identity between nucleic acid sequences can be determined using Fasta with its default parameters (a word size of 6 and the NOPAM factor for the scoring matrix) as provided in GCG Version 6.1, herein incorporated by reference. Similarly programs are available for performing amino acid alignments. Generally, these programs are used at default settings, although one of skill in the art can alter these settings as needed. Alternatively, one of skill in the art can utilize another algorithm or computer program that provides at least the level of identity or alignment as that provided by the referenced algorithms and programs.

As used throughout this specification and the claims, the term "comprise" and its variants including, "comprises", "comprising", among other variants, is inclusive of other components, elements, integers, steps and the like. The term "consists of" or "consisting of" are exclusive of other components, elements, integers, steps and the like.

### I. The Simian Adenovirus Sequences

The invention provides nucleic acid sequences and amino acid sequences of Pan5, Pan6, Pan7, SV1, SV25 and SV39, which are isolated from the other viral material with which they are associated in nature.

### A. Nucleic Acid Sequences

The Pan5 nucleic acid sequences of the invention include nucleotides 1 to 36462 of SEQ ID NO:1. The Pan6 nucleic acid sequences of the invention include nucleotides 1 to 36604 of SEQ ID NO: 5. The Pan7 nucleic acid sequences of the invention include nucleotides 1 to 36535 of SEQ ID NO: 9. The SV1 nucleic acid sequences of the invention include nucleotides 1 to 34264 of SEQ ID NO: 24. The SV25 nucleic acid sequences of the invention include nucleotides 1 to 31044 of SEQ ID NO: 29. The SV39 nucleic acid sequences of the invention include nucleotides 1 to 34115 of SEQ ID NO: 34. See, Sequence Listing, which is incorporated by reference herein.

The nucleic acid sequences of the invention further encompass the strand which is complementary to the sequences of SEQ ID NO: 5, 9, 24, 29 and 34, as well as the RNA and cDNA sequences corresponding to the sequences of these sequences figures and their complementary strands. Further included in this invention are nucleic acid sequences which are greater than 95 to 98%, and more preferably about 99 to 99.9% homologous or identical to the Sequence Listing. Also included in the nucleic acid sequences of the invention are natural variants and engineered modifications of the sequences provided in SEQ ID NO: 5, 9, 24,29 and 34 and their complementary strands. Such modifications include, for example, labels that are known in the art, methylation, and substitution of one or more of the naturally occurring nucleotides with a degenerate nucleotide.

The invention further encompasses fragments of the sequences of Pan5, Pan6, Pan7, SV1, SV25 and SV39, their complementary strand, cDNA and RNA complementary thereto. Suitable fragments are at least 15 nucleotides in length, and encompass functional fragments, i.e., fragments which are of biological interest. For example, a functional fragment can express a desired adenoviral product or may be useful in production of recombinant viral vectors. Such fragments include the gene sequences and fragments listed in the tables below.

The following tables provide the transcript regions and open reading frames in the simian adenovirus sequences of the invention. For certain genes, the transcripts and open reading frames (ORFs) are located on the strand complementary to that presented in SEQ ID NO: 5, 9, 24, 29 and 34. See, e.g., E2b, E4 and E2a. The calculated molecular weights of the encoded proteins are also shown. Note that the E1a open reading frame Pan5 [nt 576-1436 of SEQ ID NO:1], Pan6 [nt 576 to 1437 of SEQ ID NO: 5] and Pan7 [nt 576 to 1437 of SEQ ID NO: 9] contain internal splice sites. These splice sites are noted in the following tables.

| Ad Pan-5 [SEQ ID NO:1] | | | | |
|---|---|---|---|---|
| Regions | | Start (nt) | End (nt) | *M.W. (Daltons)* |
| ITR | | 1 | 120 | - |
| E1a | Transcript | 478 | | - |
| | 13S | 576-664, 1233-1436 | | *28120* |
| | 12S | 576-1046, 1233-1436 | | *24389* |
| | 9S | 576-644, 1233-1436 | | *9962* |
| | Transcript | | 1516 | - |
| E1b | Transcript | 1552 | | - |
| | Small T | 1599 | 2171 | *22317* |
| | Large T | 1904 | 3412 | *55595* |
| | IX | 3492 | 3920 | *14427* |
| | Transcript | | 3959 | - |
| E2b | Transcript | 10349 | | - |
| | PTP | 10349 | 8451 | *72930* |
| | Polymerase | 8448 | 5083 | *127237* |
| | IVa2 | 5604 | 3980 | *50466* |
| | Transcript | | 3960 | |
| 28.1 kD | | 5155 | 5979 | *28141* |
| Agnoprotein | | 7864 | 8580 | *25755* |
| L1 | Transcript | 10849 | | - |
| | 52/55D | 10851 | 12025 | |
| | IIIa | 12050 | 13819 | *65669* |
| | Transcript | | 13832 | - |
| | Transcript | 13894 | | - |
| L2 | Penton | 13898 | 15490 | *59292* |
| | VII | 15494 | 16078 | *21478* |
| | V | 16123 | 17166 | *39568* |
| | Mu | 17189 | 17422 | *8524* |
| | transcript | | 17442 | - |
| | Transcript | 17488 | | - |
| L3 | VI | 17491 | 18222 | *26192* |
| | Hexon | 18315 | 21116 | *104874* |
| | Endoprotease | 20989 | 21783 | *28304* |
| | transcript | | 21811 | - |
| E2a | Transcript | 26782 | | |
| | DBP | 23386 | 21845 | *57358* |
| | transcript | | 21788 | - |
| L4 | Transcript | 23406 | | - |
| | 100kD | 23412 | 25805 | *88223* |
| | 33 kD homolog | 25525 | 26356 | *24538* |
| | VIII | 26428 | 27111 | *24768* |
| | transcript | | 27421 | - |
| E3 | Transcript | 26788 | | - |
| | Orf #1 | 27112 | 27432 | *12098* |
| | Orf #2 | 27386 | 28012 | *23040* |
| | Orf #3 | 27994 | 28527 | *19525* |
| | Orf #4 | 28557 | 29156 | *22507* |
| | Orf #5 | 29169 | 29783 | *22267* |
| | Orf #6 | 29798 | 30673 | *31458* |
| | Orf #7 | 30681 | 30956 | *10477* |
| | Orf #8 | 30962 | 31396 | *16523* |
| | Orf #9 | 31389 | 31796 | *15236* |
| | transcript | | 31837 | - |
| L5 | Transcript | 32032 | | - |
| | Fiber | 32035 | 33372 | *47670* |
| | transcript | | 33443 | - |
| E4 | Transcript | 36135 | | - |
| | Orf 7 | 33710 | 33462 | *9191* |
| | Orf 6 | 34615 | 33710 | *35005* |
| | Orf 4 | 34886 | 34521 | *13878* |
| | Orf 3 | 35249 | 34896 | *13641* |
| | Orf 2 | 35635 | 35246 | *14584* |
| | Orf 1 | 36050 | 35676 | *13772* |
| | Transcript | | 33437 | - |
| ITR | | 36343 | 36462 | - |

| Ad Pan-6 [SEQ ID NO: 5] | | | | |
|---|---|---|---|---|
| Regions | | Start (nt) | End (nt) | *M. W. (Daltons)* |
| ITR | | 1 | 123 | - |
| E1a | transcript | 478 | | - |
| | 13S | 576-1143,1229-1437 | | *28291* |
| | 12S | 576-1050, 1229-1437 | | *24634* |
| | 9S | 576-645, 1229-1437 | | *10102* |
| | transcript | | 1516 | - |
| E1b | transcript | 1553 | | - |
| | Small T | 1600 | 2172 | *22315* |
| | LargeT | 1905 | 3413 | *55594* |
| | IX | 3498 | 3926 | *14427* |
| | transcript | | 3965 | - |
| E2b | transcript | 10341 | | - |
| | PTP | 10340 | 8451 | *72570* |
| | Polymerase | 8445 | 5089 | *126907* |
| | IVa2 | 5610 | 3986 | *50452* |
| | transcript | | 3966 | - |
| L1 | transcript | 10838 | | - |
| | 52/55 kD | 10840 | 12012 | *44205* |
| | IIIa | 12036 | 13799 | *65460* |
| | Transcript | | 13812 | - |
| 28.1 kd | | 5161 | 5985 | *28012* |
| Agnoprotein | | 7870 | 8580 | *25382* |
| L2 | transcript | 13874 | | |
| | Penton | 13878 | 15467 | *59314* |
| | VII | 15471 | 16055 | *21508* |
| | V | 16100 | 17137 | *39388* |
| | Mu | 17160 | 17393 | *8506* |
| | transcript | | 17415 | - |
| L3 | transcript | 17466 | | - |
| | VI | 17469 | 18188 | *25860* |
| | Hexon | 18284 | 21112 | *106132* |
| | Endoprotease | 21134 | 21754 | *23445* |
| | transcript | | 21803 | - |
| E2a | transcript | 26780 | | - |
| | DBP | 23375 | 21837 | *57299* |
| | transcript | | 21780 | - |
| L4 | Transcript | 23398 | | - |
| | 100kD | 23404 | 25806 | 88577 |
| | 33 kD homolog | 25523 | 26357 | *24609* |
| | VIII | 26426 | 27109 | *24749* |
| | transcript | | 27419 | - |
| E3 | transcript | 26786 | | |
| | Orf #1 | 27110 | 27430 | *12098* |
| | Orf #2 | 27384 | 28007 | *22880* |
| | Orf #3 | 27989 | 28519 | *19460* |
| | Orf #4 | 28553 | 29236 | *25403* |
| | Orf #5 | 29249 | 29860 | *22350* |
| | Orf #6 | 29875 | 30741 | *31028* |
| | Orf #7 | 30749 | 31024 | *10469* |
| | Orf #8 | 31030 | 31464 | *16540* |
| | Orf #9 | 31457 | 31864 | *15264* |
| | transcript | | 31907 | - |
| L5 | transcript | 32159 | | |
| | Fiber | 32162 | 33493 | *4736*4 |
| | transcript | | 33574 | - |
| E4 | transcript | 36276 | | - |
| | Orf 7 | 33841 | 33593 | *9177* |
| | Orf 6 | 34746 | 33841 | *35094* |
| | Orf 4 | 35017 | 34652 | *13937* |
| | Orf 3 | 35380 | 35027 | *13627* |
| | Orf 2 | 35766 | 35377 | *14727* |
| | Orf 1 | 36181 | 35807 | *13739* |
| | transcript | | 33558 | - |
| ITR | | 36482 | 36604 | - |

| Ad Pan-7 [SEQ ID NO:9] | | | | |
|---|---|---|---|---|
| Regions | | Start (nt) | End (nt) | *M.W. (Daltons)* |
| ITR | | 1 | 132 | - |
| E1a | transcript | 478 | | - |
| | 13S | 576 - 1143, 1229 - 1437 | | *28218* |
| | 12S | 576 - 1050, 1229 - 1437 | | *24561* |
| | 9S | 576 - 645, 1229 - 1437 | | *10102* |
| | transcript | | 1516 | - |
| E1b | transcript | 1553 | | - |
| | Small T | 1600 | 2178 | *22559* |
| | LargeT | 1905 | 3419 | *55698* |
| | IVa2 | 3992 | 5616 | *50210* |
| | transcript | | 3971 | - |
| E2b | transcript | 10341 | | - |
| | PTP | 10340 | 8457 | *72297* |
| | Polymerase | 8451 | 5095 | *126994* |
| | IX | 3504 | 3932 | *14441* |
| | transcript | | 3972 | - |
| 28.1kD | | 5167 | 5991 | *28028* |
| Agnoprotein | | 7876 | 8586 | *25424* |
| L1 | transcript | 10834 | | |
| | 52/55 kD | 10836 | 12011 | *44302* |
| | IIIa | 12035 | 13795 | *65339* |
| | transcript | | 13808 | - |
| L2 | transcript | 13870 | | - |
| | Penton | 13874 | 15469 | 59494 |
| | VII | 15473 | 16057 | *21339* |
| | V | 16102 | 17139 | *39414* |
| | Mu | 17167 | 17400 | *8506* |
| | transcript | | 17420 | - |
| L3 | transcript | 17467 | | - |
| | VI | 17470 | 18198 | *26105* |
| | Hexon | 18288 | 21086 | *104763* |
| | Endoprotease | 21106 | 21732 | *23620* |
| | transcript | | 21781 | *-* |
| E2a | transcript | 26764 | | *-* |
| | DBP | 23353 | 21815 | *57199* |
| | transcript | | 21755 | - |
| L4 | transcript | 23370 | | - |
| | 100kD | 23376 | 25781 | *88520* |
| | 33 kD homolog | 25489 | 26338 | *25155* |
| | VIII | 26410 | 27093 | *24749* |
| | transcript | | 27403 | - |
| E3 | transcript | 26770 | | - |
| | Or f#1 | 27094 | 27414 | *12056* |
| | Orf #2 | 27368 | 27988 | *22667* |
| | Orf #3 | 27970 | 28500 | *19462* |
| | Orf #4 | 28530 | 29150 | *22999* |
| | Orf #5 | 29163 | 29777 | *22224* |
| | Orf #6 | 29792 | 30679 | *32153* |
| | Orf #7 | 30687 | 30962 | *10511* |
| | Orf #8 | 30968 | 31399 | *16388* |
| | Orf #9 | 31392 | 31799 | *15205* |
| | transcript | | 31842 | - |
| L5 | transcript | 32091 | | - |
| | Fiber | 32094 | 33425 | *47344* |
| | transcript | | 33517 | - |
| E4 | transcript | 36208 | | - |
| | Orf 7 | 33784 | 33536 | *9191* |
| | Orf 6 | 34689 | 33784 | *35063* |
| | Orf 4 | 34960 | 34595 | *13879* |
| | Orf 3 | 35323 | 34970 | *13641* |
| | Orf 2 | 35709 | 35320 | *14644* |
| | Orf 1 | 36123 | 35749 | *13746* |
| | transcript | | 33501 | - |
| ITR | | 36404 | 36535 | - |

| | Ad SV-1 | | Ad SV-25 | | Ad SV-39 | |
|---|---|---|---|---|---|---|
| | [SEQ ID NO:24] | | [SEQ ID NO:29] | | [SEQ ID NO: 34] | |
| Region | Start | End | Start | End | Start | End |
| ITR | 1 | 106 | 1 | 133 | 1 | 150 |
| E1a | 352 | 1120 | - | - | 404 | 1409 |
| E1b | 1301 | 2891 | 359 | 2273 | 1518 | 3877 |
| E2b | 9257 | 2882 | 9087 | 2754 | 10143 | 3868 |
| E2a | 24415 | 20281 | 24034 | 20086 | 25381 | 21228 |
| E3 | 24974 | 27886 | 24791 | 25792 | 25790 | 29335 |
| E4 | 33498 | 30881 | 30696 | 28163 | 33896 | 31157 |
| ITR | 34145 | 34264 | 30912 | 31044 | 33960 | 34115 |

| | Ad SV-1 | | Ad SV-25 | | Ad SV-39 | |
|---|---|---|---|---|---|---|
| | [SEQ ID NO:24] | | [SEQ ID NO:29] | | [SEQ ID NO: 34] | |
| Region | Start | End | Start | End | Start | End |
| ITR | 1 | 106 | 1 | 133 | 1 | 150 |
| L1 | 9513 | 12376 | 9343 | 12206 | 10416 | 13383 |
| L2 | 12453 | 15858 | 12283 | 15696 | 13444 | 16877 |
| L3 | 15910 | 20270 | 15748 | 20080 | 17783 | 21192 |
| L4 | 21715 | 25603 | 21526 | 25420 | 22659 | 26427 |
| L5 | 28059 | 30899 | 25320 | 28172 | 29513 | 31170 |
| ITR | 34145 | 34264 | 30912 | 31044 | 33966 | 34115 |

| | protein | Ad SV-1, SEQ ID NO: 24 | | |
|---|---|---|---|---|
| | | Start | End | *M.W.* |
| ITR | | 1 | 106 | - |
| E1a | 13S | 459 | 953 | *18039* |
| | 12S | | | |
| E1b | Small T | | | |
| | LargeT | 1301 | 2413 | *42293* |
| | IX | 2391 | 2885 | *16882* |
| E2b | IVa2 | 4354 | 2924 | *54087* |
| | Polymerase | 6750 | 4027 | *102883* |
| | PTP | 9257 | 7371 | *72413* |
| | Agno-protein | 6850 | 7455 | *20984* |
| L1 | 52/55 kD | 9515 | 10642 | *42675* |
| | IIIa | 10663 | 12372 | *636568* |
| L2 | Penton | 12454 | 13965 | *56725* |
| | VII | 13968 | 14531 | *20397* |
| | V | 14588 | 15625 | *39374* |
| | Mu | 15645 | 15857 | *7568* |
| L3 | VI | 15911 | 16753 | *30418* |
| | Hexon | 16841 | 19636 | *104494* |
| | Endoprotease | 19645 | 20262 | *23407* |
| 2a | DBP | 21700 | 20312 | *52107* |
| L4 | 100kD | 21721 | 24009 | *85508* |
| | VIII | 24591 | 25292 | *25390* |
| E3 | Orf #1 | 25292 | 25609 | *11950* |
| | Orf #2 | 25563 | 26081 | *18940* |
| | Orf #3 | 26084 | 26893 | *30452* |
| | Orf #4 | 26908 | 27180 | *10232* |
| | Orf #5 | 27177 | 17512 | *12640* |
| | Orf #6 | 27505 | 27873 | *13639* |
| L5 | Fiber #2 | 28059 | 29150 | *39472* |
| | Fiber #1 | 29183 | 30867 | *61128* |
| E4 | Grf 7 | 31098 | 30892 | *7837* |
| | Orf 6 | 31982 | 31122 | *33921* |
| | Orf 4 | 32277 | 31915 | *14338* |
| | Orf 3 | 32629 | 32279 | *13386* |
| | Orf 2 | 33018 | 32626 | *14753* |
| | Orf 1 | 33423 | 33043 | *14301* |
| ITR | | 34145 | 34264 | |

| | protein | Ad SV-25, SEQ ID NO:29 | | | Ad SV-39, SEQ ID NO:34 | | |
|---|---|---|---|---|---|---|---|
| | | Start | End | *M.W.* | Start | End | *M. W.* |
| ITR | | 1 | 133 | - | 1 | 150 | - |
| E1a | 13S | | | | 492 | 1355 | *28585* |
| | 12S | | | | 492 | 1355 | *25003* |
| E1b | Small T | 478 | 1030 | *20274* | 1518 | 2075 | *21652* |
| | Large T | 829 | 2244 | *52310* | 1823 | 3349 | *55534* |
| | IX | 2306 | 2716 | *13854* | 3434 | 3844 | *14075* |
| E2b | IVa2 | 4208 | 2755 | *54675* | 3912 | 5141 | *46164* |
| | Polymerase | 6581 | 3858 | *102839* | 7753 | 5033 | *103988* |
| | PTP | 9087 | 7207 | *71326* | 10143 | 8335 | *69274* |
| | Agno-protein | 6681 | 7139 | *16025* | - | - | - |
| L1 | 52/55 kD | 9345 | 10472 | *42703* | 10418 | 11608 | *44232* |
| | IIIa | 10493 | 12202 | *63598* | 11574 | 13364 | *66078* |
| L2 | Penton | 12284 | 13801 | *56949* | 13448 | 14959 | *56292* |
| | VII | 13806 | 14369 | *20369* | 14960 | 15517 | *20374* |
| | V | 14426 | 15463 | *39289* | 15567 | 16628 | *39676* |
| | Mu | 15483 | 15695 | *7598* | 16650 | 16871 | *7497* |
| L3 | VI | 15749 | 16591 | *30347* | 16925 | 17695 | *28043* |
| | Hexon | 16681 | 19446 | *104035* | 17785 | 20538 | *102579* |
| | Endo-protease | 19455 | 20072 | *23338* | 20573 | 21181 | *22716* |
| 2a | DBP | 21511 | 20123 | *52189* | 22631 | 21231 | *53160* |
| L4 | 100kD | 21532 | 23829 | *85970* | 22659 | 25355 | *100362* |
| | VIII | 24408 | 25109 | *25347* | 25410 | 26108 | *25229* |
| E3 | Orf #1 | 25109 | 25426 | *11890* | 26375 | 27484 | *42257* |
| | Orf #2 | | | | 27580 | 28357 | *29785* |
| | Orf #3 | | | | 28370 | 28645 | *10514* |
| | Orf #4 | | | | 28863 | 29333 | *18835* |
| | Orf #5 | | | | | | |
| | Orf #6 | | | | | | |
| L5 | Fiber #2 | 25380 | 26423 | *37529* | | | |
| | Fiber #1 | 26457 | 28136 | *60707* | 29515 | 31116 | *56382* |
| E4 | Orf 7 | | | | 31441 | 31118 | *11856* |
| | Orf 6 | 29255 | 28395 | *33905* | 32292 | 31438 | *33437* |
| | Orf 4 | 29550 | 29188 | *14399* | 32587 | 32222 | *13997* |
| | Orf 3 | 29902 | 29552 | *13284* | 32954 | 32607 | *13353* |
| | Orf 2 | 30291 | 29899 | *14853* | 33348 | 32959 | *14821* |
| | Orf 1 | 30316 | 30696 | *14301* | 33764 | 33378 | *14235* |
| ITR | | 30912 | 31044 | | 33966 | 34115 | |

The Pan5, Pan6, Pan7, SV1, SV25 and SV39 adenoviral nucleic acid sequences are useful as therapeutic agents and in construction of a variety of vector systems and host cells. As used herein, a vector includes any suitable nucleic acid molecule including, naked DNA, a plasmid, a virus, a cosmid, or an episome. These sequences and products may be used alone or in combination with other adenoviral sequences or fragments, or in combination with elements from other adenoviral or non-adenoviral sequences. The adenoviral sequences of the invention are also useful as antisense delivery vectors, gene therapy vectors, or vaccine vectors. Thus, the invention further provides nucleic acid molecules, gene delivery vectors, and host cells which contain the Ad sequences of the invention.

For example, the invention encompasses a nucleic acid molecule containing simian Ad ITR sequences of the invention. In another example, the invention provides a nucleic acid molecule containing simian Ad sequences of the invention encoding a desired Ad gene product. Still other nucleic acid molecule constructed using the sequences of the invention will be readily apparent to one of skill in the art, in view of the information provided herein.

In one embodiment, the simian Ad gene regions identified herein may be used in a variety of vectors for delivery of a heterologous molecule to a cell. For example, vectors are generated for expression of an adenoviral capsid protein (or fragment thereof) for purposes of generating a viral vector in a packaging host cell. Such vectors may be designed for expression in trans. Alternatively, such vectors are designed to provide cells which stably contain sequences which express desired adenoviral functions, e.g., one or more of E1a, E1b, the terminal repeat sequences, E2a, E2b, E4, E4ORF6 region.

In addition, the adenoviral gene sequences and fragments thereof are useful for providing the helper-functions necessary for production of helper-dependent viruses (e.g., adenoviral vectors deleted of essential functions or adeno-associated viruses (AAV)). For such production methods, the simian adenoviral sequences of the invention are utilized in such a method in a manner similar to those described for the human Ad. However, due to the differences in sequences between the simian adenoviral sequences of the invention and those of human Ad, the use of the sequences of the invention essentially eliminate the possibility of homologous recombination with helper functions in a host cell carrying human Ad E1 functions, e.g., 293 cells, which may produce infectious adenoviral contaminants during rAAV production.

Methods of producing rAAV using adenoviral helper functions have been described at length in the literature with human adenoviral serotypes. See, e.g., US Patent 6,258,595 and the references cited therein. See, also, US Patent 5,871,982; WO 99/14354; WO 99/15685; WO 99/47691. These methods may also be used in production of non-human serotype AAV, including non-human primate AAV serotypes. The simian adenoviral gene sequences of the invention which provide the necessary helper functions (e.g., E1a, E1b, E2a and/or E4 ORF6) can be particularly useful in providing the necessary adenoviral function while minimizing or eliminating the possibility of recombination with any other adenoviruses present in the rAAV-packaging cell which are typically of human origin. Thus, selected genes or open reading frames of the adenoviral sequences of the invention may be utilized in these rAAV production methods.

Alternatively, recombinant adenoviral simian vectors of the invention may be utilized in these methods. Such recombinant adenoviral simian vectors may include, e.g., a hybrid chimp Ad/AA V in which chimp Ad sequences flank a rAAV expression cassette composed of, e.g., AAV 3' and/or 5' ITRs and a transgene under the control of regulatory sequences which control its expression. One of skill in the art will recognize that still other simian adenoviral vectors and/or gene sequences of the invention will be useful for production of rAAV and other viruses dependent upon adenoviral helper.

In still another embodiment, nucleic acid molecules are designed for delivery and expression of selected adenoviral gene products in a host cell to achieve a desired physiologic effect. For example, a nucleic acid molecule containing sequences encoding an adenovirus E1a protein of the invention may be delivered to a subject for use as a cancer therapeutic. Optionally, such a molecule is formulated in a lipid-based carrier and preferentially targets cancer cells. Such a formulation may be combined with other cancer therapeutics (e.g., cisplatin, taxol, or the like). Still other uses for the adenoviral sequences provided herein will be readily apparent to one of skill in the art.

In addition, one of skill in the an will readily understand that the Ad sequences of the invention can be readily adapted for use for a variety of viral and non-viral vector systems for in vitro, ex vivo or in vivo delivery of therapeutic and immunogenic molecules. For example, the Pan5, Pan6, Pan7, SV1, SV25 and/or SV39 simian Ad genomes of the invention can be utilized in a variety of rAd and non-rAd vector systems. Such vectors systems may include, e.g., plasmids, lentiviruses, retroviruses, poxviruses, vaccinia viruses, and adeno-associated viral systems, among others. Selection of these vector systems is not a limitation of the present invention.

The invention further provides molecules useful for production of the simian and simian-derived proteins of the invention. Such molecules which carry polynucleotides including the simian Ad DNA sequences of the invention can be in the form of naked DNA, a plasmid, a virus or any other genetic element.

### B. Simian Adenoviral Proteins of the Invention

The invention further provides gene products of the above adenoviruses, such as proteins, enzymes, and fragments thereof, which are encoded by the adenoviral nucleic acids of the invention. The invention further encompasses Pan5, Pan6 and Pan7, SV1, SV25 and SV39 proteins, enzymes, and fragments thereof, having the amino acid sequences encoded by these nucleic acid sequences which are generated by other methods. Such proteins include those encoded by the open reading frames identified in the tables above, in Figs. 1 and 2, and fragments thereof.

Thus, in one aspect, the invention provides unique simian adenoviral proteins which are substantially pure, i.e., are free of other viral and proteinaceous proteins. Preferably, these proteins are at least 10% homogeneous, more preferably 60% homogeneous, and most preferably 95% homogeneous.

In one embodiment, the invention provides unique simian-derived capsid proteins. As used herein, a simian-derived capsid protein includes any adenoviral capsid protein that contains a Pan5, Pan6, Pan7, SV1, SV25 or SV39 capsid protein or a fragment thereof, as defined above, including, without limitation, chimeric capsid proteins, fusion proteins, artificial capsid proteins, synthetic capsid proteins, and recombinantly capsid proteins, without limitation to means of generating these proteins.

Suitably, these simian-derived capsid proteins contain one or more Pan5, Pan6, Pan7, SV1, SV25 or SV39 regions or fragments thereof (e.g., a hexon, penton, fiber or fragment thereof) in combination with capsid regions or fragments thereof of different adenoviral serotypes, or modified simian capsid proteins or fragments, as described herein. A "modification of a capsid protein associated with altered tropism" as used herein includes an altered capsid protein, i.e, a penton, hexon or fiber protein region, or fragment thereof, such as the knob domain of the fiber region, or a polynucleotide encoding same, such that specificity is altered. The simian-derived capsid may be constructed with one or more of the simian Ad of the invention or another Ad serotypes which may be of human or non-human origin. Such Ad may be obtained from a variety of sources including the ATCC, commercial and academic sources, or the sequences of the Ad may be obtained from GenBank or other suitable sources.

The amino acid sequences of the simian adenoviruses penton proteins of the invention are provided herein. The AdPan5 penton protein is provided in SEQ ID NO:2. The AdPan7 penton is provided in SEQ ID NO:6. The AdPan6 penton is provided in SEQ ID NO:10. The SV1 penton is provided in SEQ ID NO:25. The SV25 penton protein is provided in SEQ ID NO:30. The SV39 penton is provided in SEQ ID NO:35. Suitably, any of these penton proteins, or unique fragments thereof, may be utilized for a variety of purposes. Examples of suitable fragments include the penton having N-terminal and/or C-terminal truncations of about 50, 100, 150, or 200 amino acids, based upon the amino acid numbering provided above and in SEQ ID NO:2; SEQ ID NO:6; SEQ ID NO:25; SEQ ID NO:30, or SEQ ID NO:35. Other suitable fragments include shorter internal, C-terminal, or N-terminal fragments. Further, the penton protein may be modified for a variety of purposes known to those of skill in the art.

The invention further provides the amino acid sequences of the hexon protein of Pan5 [SEQ ID NO:3], Pan6 [SEQ ID NO:7], Pan 7 [SEQ ID NO:11], SV1 [SEQ ID NO:26], SV25 [SEQ ID NO:31], and/or SV39 [SEQ ID NO:36]. Suitably, this hexon protein, or unique fragments thereof, may be utilized for a variety of purposes. Examples of suitable fragments include the hexon having N-terminal and/or C-terminal truncations of about 50, 100, 150, 200, 300, 400, or 500 amino acids, based upon the amino acid numbering provided above and in SEQ ID NO: 3, 7, 11, 26, 31 and 36. Other suitable fragments include shorter internal, C-terminal, or N-terminal fragments. For example, one suitable fragment the loop region (domain) of the hexon protein, designated DE1 and FG1, or a hypervariable region thereof. Such fragments include the regions spanning amino acid residues about 125 to 443; about 138 to 441, or smaller fragments, such as those spanning about residue 138 to residue 163; about 170 to about 176; about 195 to about 203; about 233 to about 246; about 253 to about 264; about 287 to about 297; and about 404 to about 430 of the simian hexon proteins, with reference to SEQ ID NO: 3, 7, 11, 26, 31 or 36. Other suitable fragments may be readily identified by one of skill in the art. Further, the hexon protein may be modified for a variety of purposes known to those of skill in the art. Because the hexon protein is the determinant for serotype of an adenovirus, such artificial hexon proteins would result in adenoviruses having artificial serotypes. Other artificial capsid proteins can also be constructed using the chimp Ad penton sequences and/or fiber sequences of the invention and/or fragments thereof.

In one example, it may be desirable to generate an adenovirus having an altered hexon protein utilizing the sequences of a hexon protein of the invention. One suitable method for altering hexon proteins is described in US Patent 5,922,315, which is incorporated by reference. In this method, at least one loop region of the adenovirus hexon is changed with at least one loop region of another adenovirus serotype. Thus, at least one loop region of such an altered adenovirus hexon protein is a simian Ad hexon loop region of the invention (e.g. Pan7). In one embodiment, a loop region ofthe Pan7 hexon protein is replaced by a loop region from another adenovirus serotype. In another embodiment, the loop region of the Pan7 hexon is used to replace a loop region from another adenovirus serotype. Suitable adenovirus serotypes may be readily selected from among human and non-human serotypes, as described herein. Pan7 is selected for purposes of illustration only; the other simian Ad hexon proteins of the invention may be similarly altered, or used to alter another Ad hexon. The selection of a suitable serotype is not a limitation of the present invention. Still other uses for the hexon protein sequences of the invention will be readily apparent to those of skill in the art.

The invention further encompasses the fiber proteins of the simian adenoviruses of the invention. The fiber protein of AdPan 5 has the amino acid sequence of SEQ ID NO:4. The fiber protein AdPan6 has the amino acid sequence of SEQ ID NO: 8. The fiber protein of AdPan7 has the amino acid sequence of SEQ ID NO: 12. SV-1 has two fiber proteins; fiber 2 has the amino acid sequence of SEQ ID NO:27 and fiber 1 has the amino acid sequence of SEQ ID NO:28. SV-25 also has two fiber proteins; fiber 2 has the amino acid sequence of SEQ ID NO:32 and fiber 1 has the amino acid sequence of SEQ ID NO:33. The fiber protein of SV-39 has the amino acid sequence of SEQ ID NO:37.

Suitably, this fiber protein, or unique fragments thereof, may be utilized for a variety of purposes. One suitable fragment is the fiber knob, which spans about amino acids 247 to 425 of SEQ ID NO: 4, 8, 12, 28, 32, 33 and 37. See Fig. 2. Examples of other suitable fragments include the fiber having N-terminal and/or C-terminal truncations of about 50, 100, 150, or 200 amino acids, based upon the amino acid numbering provided above and in SEQ ID NO: 4, 8, 12, 28, 32, 33 and 37. Still other suitable fragments include internal fragments. Further, the fiber protein may be modified using a variety of techniques known to those of skill in the art.

The invention further encompasses unique fragments of the proteins of the invention which are at least 8 amino acids in length. However, fragments of other desired lengths can be readily utilized. In addition, the invention encompasses such modifications as may be introduced to enhance yield and/or expression of a Pan5, Pan6, Pan7, SV1, SV25 or SV39 gene product, e.g., construction of a fusion molecule in which all or a fragment of the Pan5, Pan6, Pan7, SV1, SV25 or SV39 gene product is fused (either directly or via a linker) with a fusion partner to enhance. Other suitable modifications include, without limitation, truncation of a coding region (e.g., a protein or enzyme) to eliminate a pre- or pro-protein ordinarily cleaved and to provide the mature protein or enzyme and/or mutation of a coding region to provide a secretable gene product. Still other modifications will be readily apparent to one of skill in the art. The invention further encompasses proteins having at least about 95% to 99% identity to the Pan5, Pan6, Pan7, SV1, SV25 or SV39 proteins provided herein.

As described herein, vectors of the invention containing the adenoviral capsid proteins of the invention are particularly well suited for use in applications in which the neutralizing antibodies diminish the effectiveness of other Ad serotype based vectors, as well as other viral vectors. The rAd vectors of the invention are particularly advantageous in readministration for repeat gene therapy or for boosting immune response (vaccine titers).

Under certain circumstances, it may be desirable to use one or more of the Pan5, Pan6, Pan7, SV1, SV25 and/or SV39 gene products (e.g., a capsid protein or a fragment thereof) to generate an antibody. The term "an antibody," as used herein, refers to an immunoglobulin molecule which is able to specifically bind to an epitope. Thus, the antibodies of the invention bind, preferably specifically and without cross-reactivity, to a Pan5, Pan6, Pan7, SV1, SV25 or SV39 epitope. The antibodies in the present invention exist in a variety of forms including, for example, high affinity polyclonal antibodies, monoclonal antibodies, synthetic antibodies, chimeric antibodies, recombinant antibodies and humanized antibodies. Such antibodies originate from immunoglobulin classes IgG, IgM, IgA, IgD and IgE.

Such antibodies may be generated using any of a number of methods know in the art. Suitable antibodies may be generated by well-known conventional techniques, e.g. Kohler and Milstein and the many known modifications thereof. Similarly desirable high titer antibodies are generated by applying known recombinant techniques to the monoclonal or polyclonal antibodies developed to these antigens [see, e.g., PCT Patent Application No. PCT/GB85/00392; British Patent Application Publication No. GB2188638A; Amit et al., 1986 Science, 233:747-753; Queen et al., 1989 Proc. Nat'l. Acad. Sci. USA, 86:10029-10033; PCT Patent Application No. PCT/WO9007861; and Riechmann et al., Nature, 332:323-327 (1988); Huse et al, 1988a Science, 246:1275-1281]. Alternatively, antibodies can be produced by manipulating the complementarity determining regions of animal or human antibodies to the antigen of this invention. See, e.g., E. Mark and Padlin, "Humanization of Monoclonal Antibodies", Chapter 4, The Handbook of Experimental Pharmacology, Vol. 113, The Pharmacology of Monoclonal Antibodies, Springer-Verlag (June, 1994); Harlow et al., 1999, Using Antibodies: A Laboratory Manual, Cold Spring Harbor Laboratory Press, NY; Harlow et al., 1989, Antibodies: A Laboratory Manual, Cold Spring Harbor, New York; Houston et al., 1988, Proc. Natl. Acad. Sci. USA 85:5879-5883; and Bird et al., 1988, Science 242:423-426. Further provided by the present invention are anti-idiotype antibodies (Ab2) and anti-anti-idiotype antibodies (Ab3). See, e.g., M. Wettendorff et al., "Modulation of anti-tumor immunity by anti-idiotypic antibodies." In Idiotypic Network and Diseases, ed. by J. Cerny and J. Hiernaux, 1990 J. Am. Soc. Microbiol., Washington DC: pp. 203-229]. These anti-idiotype and anti-anti-idiotype antibodies are produced using techniques well known to those of skill in the art. These antibodies may be used for a variety of purposes, including diagnostic and clinical methods and kits.

Under certain circumstances, it may be desirable to introduce a detectable label or a tag onto a Pan5, Pan6, Pan7, SV1, SV25 or SV39 gene product, antibody or other construct of the invention. As used herein, a detectable label is a molecule which is capable, alone or upon interaction with another molecule, of providing a detectable signal. Most desirably, the label is detectable visually, e.g. by fluorescence, for ready use in immunohistochemical analyses or immunofluorescent microscopy. For example, suitable labels include fluorescein isothiocyanate (FITC), phycoerythrin (PE), allophycocyanin (APC), coriphosphine-O (CPO) or tandem dyes, PE-cyanin-5 (PC5), and PE-Texas Red (ECD). All of these fluorescent dyes are commercially available, and their uses known to the art. Other useful labels include a colloidal gold label. Still other useful labels include radioactive compounds or elements. Additionally, labels include a variety of enzyme systems that operate to reveal a colorimetric signal in an assay, e.g., glucose oxidase (which uses glucose as a substrate) releases peroxide as a product which in the presence of peroxidase and a hydrogen donor such as tetramethyl benzidine (TMB) produces an oxidized TMB that is seen as a blue color. Other examples include horseradish peroxidase (HRP) or alkaline phosphatase (AP), and hexokinase in conjunction with glucose-6-phosphate dehydrogenase which reacts with ATP, glucose, and NAD+to yield, among other products, NADH that is detected as increased absorbance at 340 nm wavelength.

Other label systems that are utilized in the methods of this invention are detectable by other means, e.g., colored latex microparticles [Bangs Laboratories, Indiana] in which a dye is embedded are used in place of enzymes to form conjugates with the target sequences provide a visual signal indicative of the presence of the resulting complex in applicable assays.

Methods for coupling or associating the label with a desired molecule are similarly conventional and known to those of skill in the art. Known methods of label attachment are described [see, for example, Handbook of Fluorescent probes and Research Chemicals, 6th Ed., R. P. M. Haugland, Molecular Probes, Inc., Eugene, OR, 1996; Pierce Catalog and Handbook, Life Science and Analytical Research Products, Pierce Chemical Company, Rockford, IL, 1994/1995]. Thus, selection of the label and coupling methods do not limit this invention.

The sequences, proteins, and fragments of the invention may be produced by any suitable means, including recombinant production, chemical synthesis, or other synthetic means. Suitable production techniques are well known to those of skill in the art. See, e.g., Sambrook et al, Molecular Cloning: A Laboratory Manual, Cold Spring Harbor Press (Cold Spring Harbor, NY). Alternatively, peptides can also be synthesized by the well known solid phase peptide synthesis methods (Merrifield, J. Am. Chem. Soc., 85:2149 (1962); Stewart and Young, Solid Phase Peptide Synthesis (Freeman, San Francisco, 1969) pp. 27-62). These and other suitable production methods are within the knowledge of those of skill in the art and are not a limitation of the present invention.

In addition, one of skill in the art will readily understand that the Ad sequences of the invention can be readily adapted for use for a variety of viral and non-viral vector systems for *in vitro, ex vivo or in vivo* delivery of therapeutic and immunogenic molecules. For example, in one embodiment, the simian Ad capsid proteins and other simian adenovirus proteins described herein are used for non-viral, protein-based delivery of genes, proteins, and other desirable diagnostic, therapeutic and immunogenic molecules. In one such embodiment, a protein of the invention is linked, directly or indirectly, to a molecule for targeting to cells with a receptor for adenoviruses. Preferably, a capsid protein such as a hexon, penton, fiber or a fragment thereof having a ligand for a cell surface receptor is selected for such targeting. Suitable molecules for delivery are selected from among the therapeutic molecules described herein and their gene products. A variety of linkers including, lipids, polyLys, and the like may be utilized as linkers. For example, the simian penton protein may be readily utilized for such a purpose by production of a fusion protein using the simian penton sequences in a manner analogous to that described in Medina-Kauwe LK, et al, Gene Ther. 2001 May; 8(10):795-803 and Medina-Kauwe LK, et al, Gene Ther. 2001 Dec; 8(23): 1753-1761. Alternatively, the amino acid sequences of simian Ad protein IX may be utilized for targeting vectors to a cell surface receptor, as described in US Patent Appln 20010047081. Suitable ligands include a CD40 antigen, an RGD-containing or polylysine-containing sequence, and the like. Still other simian Ad proteins, including, e.g., the hexon protein and/or the fiber protein, may be used for used for these and similar purposes.

Still other adenoviral proteins of the invention may be used as alone, or in combination with other adenoviral protein, for a variety of purposes which will be readily apparent to one of skill in the art. In addition, still other uses for the adenoviral proteins of the invention will be readily apparent to one of skill in the art.

### II. Recombinant Adenoviral Vectors

The compositions of this invention include vectors that deliver a heterologous molecule to cells, either for therapeutic or vaccine purposes. As used herein, a vector may include any genetic element including, without limitation, naked DNA, a phage, transposon, cosmid, episome, plasmid, or a virus. Such vectors contain simian adenovirus DNA of Pan5, Pan6, Pan7, SV1, SV25 and/or SV39 and a minigene. By "minigene" is meant the combination of a selected heterologous gene and the other regulatory elements necessary to drive translation, transcription and/or expression of the gene product in a host cell.

Typically, an adenoviral vector of the invention is designed such that the minigene is located in a nucleic acid molecule which contains other adenoviral sequences in the region native to a selected adenoviral gene. The minigene may be inserted into an existing gene region to disrupt the function of that region, if desired. Alternatively, the minigene may be inserted into the site of a partially or fully deleted adenoviral gene. For example, the minigene may be located in the site of such as the site of a functional E1 deletion or functional E3 deletion, among others that may be selected. The term "functionally deleted" or "functional deletion" means that a sufficient amount of the gene region is removed or otherwise damaged, e.g., by mutation or modification, so that the gene region is no longer capable of producing functional products of gene expression. If desired, the entire gene region may be removed. Other suitable sites for gene disruption or deletion are discussed elsewhere in the application.

For example, for a production vector useful for generation of a recombinant virus, the vector may contain the minigene and either the 5' end of the adenoviral genome or the 3' end of the adenoviral genome, or both the 5' and 3' ends of the adenoviral genome. The 5' end of the adenoviral genome contains the 5' cis-elements necessary for packaging and replication; i.e., the 5' inverted terminal repeat (ITR) sequences (which functions as origins of replication) and the native 5' packaging enhancer domains (that contain sequences necessary for packaging linear Ad genomes and enhancer elements for the E1 promoter). The 3' end of the adenoviral genome includes the 3' cis-elements (including the ITRs) necessary for packaging and encapsidation. Suitably, a recombinant adenovirus contains both 5' and 3' adenoviral cis-elements and the minigene is located between the 5' and 3' adenoviral sequences. Any adenoviral vector ofthe invention may also contain additional adenoviral sequences.

Suitably, these adenoviral vectors of the invention contain one or more adenoviral elements derived from an adenoviral genome of the invention. In one embodiment, the vectors contain adenoviral ITRs from Pan5, Pan6, Pan7, SV1, SV25 or SV39 and additional adenoviral sequences from the same adenoviral serotype. In another embodiment, the vectors contain adenoviral sequences that are derived from a different adenoviral serotype than that which provides the ITRs. As defined herein, a pseudotyped adenovirus refers to an adenovirus in which the capsid protein of the adenovirus is from a different serotype than the serotype which provides the ITRs. The selection of the serotype of the ITRs and the serotype of any other adenoviral sequences present in vector is not a limitation of the present invention. A variety of adenovirus strains are available from the American Type Culture Collection, Manassas, Virginia, or available by request from a variety of commercial and institutional sources. Further, the sequences of many such strains are available from a variety of databases including, e.g., PubMed and GenBank. Homologous adenovirus vectors prepared from other simian or from human adenoviruses are described in the published literature [see, for example, US Patent No. 5,240,846]. The DNA sequences of a number of adenovirus types are available from GenBank, including type Ad5 [GenBank Accession No. M73260]. The adenovirus sequences may be obtained from any known adenovirus serotype, such as serotypes 2, 3, 4, 7, 12 and 40, and further including any of the presently identified human types. Similarly adenoviruses known to infect non-human animals (e.g., simians) may also be employed in the vector constructs of this invention. See, e.g., US Patent No. 6,083,716.

The viral sequences, helper viruses; if needed, and recombinant viral particles, and other vector components and sequences employed in the construction of the vectors described herein are obtained as described above. The DNA sequences of the Pan5, Pan6, Pan7, SV1, SV25 and/or SV39 simian adenovirus sequences of the invention are employed to construct vectors and cell lines useful in the preparation of such vectors.

Modifications of the nucleic acid sequences forming the vectors ofthis invention, including sequence deletions, insertions, and other mutations may be generated using standard molecular biological techniques and are within the scope of this invention.

### A. The "Minigene"

The methods employed for the selection of the transgene, the cloning and construction ofthe "minigene" and its insertion into the viral vector are within the skill in the art given the teachings provided herein.

### 1. The transgene

The transgene is a nucleic acid sequence, heterologous to the vector sequences flanking the transgene, which encodes a polypeptide, protein, or other product, of interest. The nucleic acid coding sequence is operatively linked to regulatory components in a manner which permits transgene transcription, translation, and/or expression in a host cell.

The composition of the transgene sequence will depend upon the use to which the resulting vector will be put. For example, one type of transgene sequence includes a reporter sequence, which upon expression produces a detectable signal. Such reporter sequences include, without limitation, DNA sequences encoding β-lactamase, β-galactosidase (LacZ), alkaline phosphatase, thymidine kinase, green fluorescent protein (GFP)_{,} chloramphenicol aceyltransferase (CAT), luciferase, membrane bound proteins including, for example, CD2, CD4, CD8, the influenza hemagglutinin protein, and others well known in the an, to which high affinity antibodies directed thereto exist or can be produced by conventional means, and fusion proteins comprising a membrane bound protein appropriately fused to an antigen tag domain from, among others, hemagglutinin or Myc. These coding sequences, when associated with regulatory elements which drive their expression, provide signals detectable by conventional means, including enzymatic, radiographic, colorimetric, fluorescence or other spectrographic assays, fluorescent activating cell sorting assays and immunological assays, including enzyme linked immunosorbent assay (ELISA), radioimmunoassay (RIA) and immunohistochemistry. For example, where the marker sequence is the LacZ gene, the presence of the vector carrying the signal is detected by assays for beta-galactosidase activity. Where the transgene is GFP or luciferase, the vector carrying the signal may be measured visually by color or light production in a luminometer.

However, desirably, the transgene is a non-marker sequence encoding a product which is useful in biology and medicine, such as proteins, peptides, RNA, enzymes, or catalytic RNAs. Desirable RNA molecules include tRNA, dsRNA, ribosomal RNA, catalytic RNAs, and antisense RNAs. One example of a useful RNA sequence is a sequence which extinguishes expression of a targeted nucleic-acid sequence in the treated animal.

The transgene may be used for treatment, e.g., of genetic deficiencies, as a cancer therapeutic or vaccine, for induction of an immune response, and/or for prophylactic vaccine purposes. As used herein, induction of an immune response refers to the ability of a molecule (e.g., a gene product) to induce a T cell and/or a humoral immune response to the molecule. The invention further includes using multiple transgenes, e.g., to correct or ameliorate a condition caused by a multi-subunit protein. In certain situations, a different transgene may be used to encode each subunit of a protein, or to encode different peptides or proteins. This is desirable when the size of the DNA encoding the protein subunit is large, e.g., for an immunoglobulin, the platelet-derived growth factor, or a dystrophin protein. In order for the cell to produce the multi-subunit protein, a cell is infected with the recombinant virus containing each of the different subunits. Alternatively, different subunits of a protein may be encoded by the same transgene. In this case, a single transgene includes the DNA encoding each of the subunits, with the DNA for each subunit separated by an internal ribozyme entry site (IRES). This is desirable when the size of the DNA encoding each of the subunits is small, e.g., the total size of the DNA encoding the subunits and the IRES is less than five kilobases. As an alternative to an IRES, the DNA may be separated by sequences encoding a 2A peptide, which self-cleaves in a post-translational event. See, e.g., M.L. Donnelly, et al, J. Gen. Virol., 78(Pt 1):13-21 (Jan 1997); Furler, S., et al, Gene Ther., 8(11):864-873 (June 2001); Klump H., et al., Gene Ther., 8(10):811-817 (May 2001). This 2A peptide is significantly smaller than an IRES, making it well suited for use when space is a limiting factor. However, the selected transgene may encode any biologically active product or other product, e.g., a product desirable for study.

Suitable transgenes may be readily selected by one of skill in the art. The selection of the transgene is not considered to be a limitation of this invention.

### 2. Regulatory Elements

In addition to the major elements identified above for the minigene, the vector also includes conventional control elements necessary which are operably linked to the transgene in a manner that permits its transcription, translation and/or expression in a cell transfected with the plasmid vector or infected with the virus produced by the invention. As used herein, "operably linked" sequences include both expression control sequences that are contiguous with the gene of interest and expression control sequences that act in *trans* or at a distance to control the gene of interest.

Expression control sequences include appropriate transcription initiation, termination, promoter and enhancer sequences; efficient RNA processing signals such as splicing and polyadenylation (polyA) signals; sequences that stabilize cytoplasmic mRNA; sequences that enhance translation efficiency (i.e., Kozak consensus sequence); sequences that enhance protein stability; and when desired, sequences that enhance secretion of the encoded product. A great number of expression control sequences, including promoters which are native, constitutive, inducible and/or tissue-specific, are known in the art and may be utilized.

Examples of constitutive promoters include, without limitation, the retroviral Rous sarcoma virus (RSV) LTR promoter (optionally with the RSV enhancer), the cytomegalovirus (CMV) promoter (optionally with the CMV enhancer) [see, e.g., Boshart et al, Cell, 41:521-530 (1985)], the SV40 promoter, the dihydrofolate reductase promoter, the β-actin promoter, the phosphoglycerol kinase (PGK) promoter, and the EF1α promoter [Invitrogen].

Inducible promoters allow regulation of gene expression and can be regulated by exogenously supplied compounds, environmental factors such as temperature, or the presence of a specific physiological state, e.g., acute phase, a particular differentiation state of the cell, or in replicating cells only. Inducible promoters and inducible systems are available from a variety of commercial sources, including, without limitation, Invitrogen, Clontech and Ariad. Many other systems have been described and can be readily selected by one of skill in the art. For example, inducible promoters include the zinc-inducible sheep metallothionine (MT) promoter and the dexamethasone (Dex)-inducible mouse mammary tumor virus (MMTV) promoter. Other inducible systems include the T7 polymerase promoter system [WO 98/10088]; the ecdysone insect promoter [No et al, Proc. Natl. Acad. Sci. USA, 93:3346-3351 (1996)], the tetracycline-repressible system [Gossen et al, Proc. Natl. Acad. Sci. USA, 89:5547-5551 (1992)], the tetracycline-inducible system [Gossen et al, Science, 268:1766-1769 (1995), see also Harvey et al, Curr. Opin. Chem. Biol., 2:512-518 (1998)]. Other systems include the FK506 dimer, VP16 or p65 using castradiol, diphenol murislerone, the RU486-inducible system [Wang et al, Nat. Biotech., 15:239-243 (1997) and Wang et al, Gene Ther., 4:432-441 (1997)] and the rapamycin-inducible system [Magari et al, J. Clin. Invest., 100:2865-2872 (1997)]. The effectiveness of some inducible promoters increases over time. In such cases one can enhance the effectiveness of such systems by inserting multiple repressors in tandem, e.g., TetR linked to a TetR by an IRES. Alternatively, one can wait at least 3 days before screening for the desired function. One can enhance expression of desired proteins by known means to enhance the effectiveness of this system. For example, using the Woodchuck Hepatitis Virus Posttranscriptional Regulatory Element (WPRE).

In another embodiment, the native promoter for the transgene will be used. The native promoter may be preferred when it is desired that expression of the transgene should mimic the native expression. The native promoter may be used when expression of the transgene must be regulated temporally or developmentally, or in a tissue-specific manner, or in response to specific transcriptional stimuli. In a further embodiment, other native expression control elements, such as enhancer elements, polyadenylation sites or Kozak consensus sequences may also be used to mimic the native expression.

Another embodiment of the transgene includes a transgene operably linked to a tissue-specific promoter. For instance, if expression in skeletal muscle is desired, a promoter active in muscle should be used. These include the promoters from genes encoding skeletal β-actin, myosin light chain 2A, dystrophin, muscle creatine kinase, as well as synthetic muscle promoters with activities higher than naturally occurring promoters (see Li et al., Nat. Biotech., 17:241-245 (1999)). Examples of promoters that are tissue-specific are known for liver (albumin, Miyatake et al., J. Virol., 71:5124-32 (1997); hepatitis B virus core promoter, Sandig et al., Gene Ther., 3:1002-9 (1996); alpha-fetoprotein (AFP), Arbuthnot el al., Hum. Gene Ther., 7:1503-14 (1996)), bone osteocalcin (Stein et al., Mol. Biol. Rep., 24:185-96 (1997)); bone sialoprotein (Chen et al., J. Bone Miner. Res., 11:654-64 (1996)), lymphocytes (CD2, Hansal et al., J. Immunol., 161:1063-8 (1998); immunoglobulin heavy chain; T cell receptor chain), neuronal such as neuron-specific enolase (NSE) promoter (Andersen et al., Cell. Mol. Neurobiol., 13:503-15 (1993)), neurofilament light-chain gene (Piccioli et al., Proc. Natl. Acacl. Sci. USA, 88:5611-5 (1991)), and the neuron-specific vgf gene (Piccioli et al., Neuron, 15:373-84 (1995)), among others.

Optionally, vectors carrying transgenes encoding therapeutically useful or immunogenic products may also include selectable markers or reporter genes may include sequences encoding geneticin, hygromicin or purimycin resistance, among others. Such selectable reporters or marker genes (preferably located outside the viral genome to be packaged into a viral particle) can be used to signal the presence of the plasmids in bacterial cells, such as ampicillin resistance. Other components of the vector may include an origin of replication. Selection of these and other promoters and vector elements are conventional and many such sequences are available [see, e.g., Sambrook et al, and references cited therein].

These vectors are generated using the techniques and sequences provided herein, in conjunction with techniques known to those of skill in the art. Such techniques include conventional cloning techniques of cDNA such as those described in texts [Sambrook et al, Molecular Cloning: A Laboratory Manual, Cold Spring Harbor Press, Cold Spring Harbor, NY], use of overlapping oligonucleotide sequences of the adenovirus genomes, polymerase chain reaction, and any suitable method which provides the desired nucleotide sequence.

### III. Production of the Recombinant Viral Particle

In one embodiment, the simian adenoviral plasmids (or other vectors) are used to produce recombinant adenoviral particles. In one embodiment, the recombinant adenoviruses are functionally deleted in the E1a or E1b genes, and optionally bearing other mutations, e.g., temperature-sensitive mutations or deletions in other genes. In other embodiments, it is desirable to retain an intact E1a and/or E1b region in the recombinant adenoviruses. Such an intact E1 region may be located in its native location in the adenoviral genome or placed in the site of a deletion in the native adenoviral genome (e.g., in the E3 region).

In the construction of useful simian adenovirus vectors for delivery of a gene to the human (or other mammalian) cell, a range of adenovirus nucleic acid sequences can be employed in the vectors. For example, all or a portion of the adenovirus delayed early gene E3 may be eliminated from the simian adenovirus sequence which forms a part of the recombinant virus. The function of simian E3 is believed to be irrelevant to the function and production of the recombinant virus particle. Simian adenovirus vectors may also be constructed having a deletion of at least the ORF6 region of the E4 gene, and more desirably because of the redundancy in the function of this region, the entire E4 region. Still another vector of this invention contains a deletion in the delayed early gene E2a. Deletions may also be made in any of the late genes L1 through L5 of the simian adenovirus genome. Similarly, deletions in the intermediate genes IX and IVa₂ may be useful for some purposes. Other deletions may be made in the other structural or non-structural adenovirus genes. The above discussed deletions may be used individually, i.e., an adenovirus sequence for use in the present invention may contain deletions in only a single region. Alternatively, deletions of entire genes or portions thereof effective to destroy their biological activity may be used in any combination. For example, in one exemplary vector, the adenovirus sequence may have deletions of the E1 genes and the E4 gene, or ofthe E1, E2a and E3 genes, or of the E1 and E3 genes, or of E1, E2a and E4 genes, with or without deletion of E3, and so on. As discussed above, such deletions may be used in combination with other mutations, such as temperature-sensitive mutations, to achieve a desired result.

An adenoviral vector lacking any essential adenoviral sequences (e.g., E1a, E1b, E2a, E2b, E4 ORF6, L1, L2, L3, L4 and L5) may be cultured in the presence of the missing adenoviral gene products which are required for viral infectivity and propagation of an adenoviral particle. These helper functions may be provided by culturing the adenoviral vector in the presence of one or more helper constructs (e.g., a plasmid or virus) or a packaging host cell. See, for example, the techniques described for preparation of a "minimal" human Ad vector in International Patent Application WO96/13597, published May 9, 1996, and incorporated herein by reference.

### 1. Helper Viruses

Thus, depending upon the simian adenovirus gene content ofthe viral vectors employed to carry the minigene, a helper adenovirus or non-replicating virus fragment may be necessary to provide sufficient simian adenovirus gene sequences necessary to produce an infective recombinant viral particle containing the minigene. Useful helper viruses contain selected adenovirus gene sequences not present in the adenovirus vector construct and/or not expressed by the packaging cell line in which the vector is transfected. In one embodiment, the helper virus is replication-defective and contains a variety of adenovirus genes in addition to the sequences described above. Such a helper virus is desirably used in combination with an E1-expressing cell line.

Helper viruses may also be formed into poly-cation conjugates as described in Wu et al, J. Biol. Chem., 264:16985-16987 (1989); K. J. Fisher and J. M. Wilson, Biochem. J., 299:49 (April 1, 1994). Helper virus may optionally contain a second reporter minigene. A number of such reporter genes are known to the art. The presence of a reporter gene on the helper virus which is different from the transgene on the adenovirus vector allows both the Ad vector and the helper virus to be independently monitored. This second reporter is used to enable separation between the resulting recombinant virus and the helper virus upon purification.

### 2. Complementation Cell Lines

To generate recombinant simian adenoviruses (Ad) deleted in any of the genes described above, the function of the deleted gene region, if essential to the replication and infectivity of the virus, must be supplied to the recombinant virus by a helper virus or cell line, i.e., a complementation or packaging cell line. In many circumstances, a cell line expressing the human E1 can be used to transcomplement the chimp Ad vector. This is particularly advantageous because, due to the diversity between the chimp Ad sequences of the invention and the human AdE1 sequences found in currently available packaging cells, the use of the current human E1-containing cells prevents the generation of replication-competent adenoviruses during the replication and production process. However, in certain circumstances, it will be desirable to utilize a cell line which expresses the E1 gene products can be utilized for production of an E1-deleted simian adenovirus. Such cell lines have been described. See, e.g., US Patent 6,083,716.

If desired, one may utilize the sequences provided herein to generate a packaging cell or cell line that expresses, at a minimum, the adenovirus E1 gene from Pan5, Pan6, Pan7, SV1, SV25 or SV39 under the transcriptional control of a promoter for expression in a selected parent cell line. Inducible or constitutive promoters may be employed for this purpose. Examples of such promoters are described in detail elsewhere in this specification. A parent cell is selected for the generation of a novel cell line expressing any desired AdPan5, Pan6, Pan7, SV1, SV25 or SV39 gene. Without limitation, such a parent cell line may be HeLa [ATCC Accession No. CCL 2], A549 [ATCC Accession No. CCL 185], HEK 293, KB [CCL 17], Detroit [e.g., Detroit 510, CCL 72] and WI-38 [CCL 75] cells, among others. These cell lines are all available from the American Type Culture Collection, 10801 University Boulevard, Manassas, Virginia 20110-2209. Other suitable parent cell lines may be obtained from other sources.

Such E1-expressing cell lines are useful in the generation of recombinant simian adenovirus E1 deleted vectors. Additionally, or alternatively, the invention provides cell lines that express one or more simian adenoviral gene products, e.g., E1a, E1b, E2a, and/or E4 ORF6, can be constructed using essentially the same procedures for use in the generation of recombinant simian viral vectors. Such cell lines can be utilized to transcomplement adenovirus vectors deleted in the essential genes that encode those products, or to provide helper functions necessary for packaging of a helper-dependent virus (e.g., adeno-associated virus). The preparation of a host cell according to this invention involves techniques such as assembly of selected DNA sequences. This assembly may be accomplished utilizing conventional techniques. Such techniques include cDNA.and genomic cloning, which are well known and are described in Sambrook et al., cited above, use of overlapping oligonucleotide sequences of the adenovirus genomes, combined with polymerase chain reaction, synthetic methods, and any other suitable methods which provide the desired nucleotide sequence.

In still another alternative, the essential adenoviral gene products are provided in *trans* by the adenoviral vector and/or helper virus. In such an instance, a suitable host cell can be selected from any biological organism, including prokaryotic (e.g., bacterial) cells, and eukaryotic cells, including, insect cells, yeast cells and mammalian cells. Particularly desirable host cells are selected from among any mammalian species, including, without limitation, cells such as A549, WEHI, 3T3, 10T1/2, HEK 293 cells or PERC6 (both of which express functional adenoviral E1) [Fallaux, FJ et al, (1998), Hum Gene Ther, 9:1909-1917], Saos, C2Cl2, L cells, HT1080, HepG2 and primary fibroblast, hepatocyte and myoblast cells derived from mammals including human, monkey, mouse, rat, rabbit, and hamster. The selection of the mammalian species providing the cells is not a limitation of this invention; nor is the type of mammalian cell, i.e., fibroblast, hepatocyte, tumor cell, etc.

### 3. Assembly of Viral Particle and Transfection of a Cell Line

Generally, when delivering the vector comprising the minigene by transfection, the vector is delivered in an amount from about 5 µg to about 100 µg DNA, and preferably about 10 to about 50 µg DNA to about 1 x 10⁴ cells to about 1 x 10¹³ cells, and preferably about 10⁵ cells. However, the relative amounts of vector DNA to host cells may be adjusted, taking into consideration such factors as the selected vector, the delivery method and the host cells selected.

The vector may be any vector known in the art or disclosed above, including naked DNA, a plasmid, phage, transposon, cosmids, episomes, viruses, etc. Introduction into the host cell of the vector may be achieved by any means known in the art or as disclosed above, including transfection, and infection. One or more of the adenoviral genes may be stably integrated into the genome of the host cell, stably expressed as episomes, or expressed transiently. The gene products may all be expressed transiently, on an episome or stably integrated, or some of the gene products may be expressed stably while others are expressed transiently. Furthermore, the promoters for each of the adenoviral genes may be selected independently from a constitutive promoter, an inducible promoter or a native adenoviral promoter. The promoters may be regulated by a specific physiological state of the organism or cell (i.e., by the differentiation state or in replicating or quiescent cells) or by exogenously-added factors, for example.

Introduction of the molecules (as plasmids or viruses) into the host cell may also be accomplished using techniques known to the skilled artisan and as discussed throughout the specification. In preferred embodiment, standard transfection techniques are used, e.g., CaPO₄ transfection or electroporation.

Assembly of the selected DNA sequences of the adenovirus (as well as the transgene and other vector elements into various intermediate plasmids, and the use of the plasmids and vectors to produce a recombinant viral particle are all achieved using conventional techniques. Such techniques include conventional cloning techniques of cDNA such as those described in texts [Sambrook et al, cited above], use of overlapping oligonucleotide sequences of the adenovirus genomes, polymerase chain reaction, and any suitable method which provides the desired nucleotide sequence. Standard transfection and co-transfection techniques are employed, e.g., CaPO₄ precipitation techniques. Other conventional methods employed include homologous recombination of the viral genomes, plaquing of viruses in agar overlay, methods of measuring signal generation, and the like.

For example, following the construction and assembly of the desired minigene-containing viral vector, the vector is transfected *in vitro* in the presence of a helper virus into the packaging cell line. Homologous recombination occurs between the helper and the vector sequences, which permits the adenovirus-transgene sequences in the vector to be replicated and packaged into virion capsids, resulting in the recombinant viral vector particles. The current method for producing such virus particles is transfection-based. However, the invention is not limited to such methods.

The resulting recombinant simian adenoviruses are useful in transferring a selected transgene to a selected cell. In *in vivo* experiments with the recombinant virus grown in the packaging cell lines, the E1-deleted recombinant simian adenoviral vectors of the invention demonstrate utility in transferring a transgene to a non-simian, preferably a human, cell.

### IV. Use of the Recombinant Adenovirus Vectors

The recombinant simian adenovirus vectors of the invention are useful for gene transfer to a human or non-simian veterinary patient *in vitro, ex vivo,* and *in vivo.*

The recombinant adenovirus vectors described herein can be used as expression vectors for the production of the products encoded by the heterologous genes *in vitro.* For example, the recombinant adenoviruses containing a gene inserted into the location of an E1 deletion may be transfected into an E1-expressing cell line as described above. Alternatively, replication-competent adenoviruses may be used in another selected cell line. The transfected cells are then cultured in the conventional manner, allowing the recombinant adenovirus to express the gene product from the promoter. The gene product may then be recovered from the culture medium by known conventional methods of protein isolation and recovery from culture.

A Pan5, Pan6, Pan7, SV1, SV25 or SV39-derived recombinant simian adenoviral vector of the invention provides an efficient gene transfer vehicle that can deliver a selected transgene to a selected host cell *in vivo or ex vivo* even where the organism has neutralizing antibodies to one or more AAV serotypes. In one embodiment, the rAAV and the cells are mixed *ex vivo*; the infected cells are cultured using conventional methodologies; and the transduced cells are re-infused into the patient. These compositions are particularly well suited to gene delivery for therapeutic purposes and for immunization, including inducing protective immunity.

More commonly, the Pan 5, Pan6, Pan7, SV1, SV25, or SV39 recombinant adenoviral vectors of the invention will be utilized for delivery of therapeutic or immunogenic molecules, as described below. It will be readily understood for both applications, that the recombinant adenoviral vectors of the invention are particularly well suited for use in regimens involving repeat delivery of recombinant adenoviral vectors. Such regimens typically involve delivery of a series of viral vectors in which the viral capsids are alternated. The viral capsids may be changed for each subsequent administration, or after a pre-selected number of administrations of a particular serotype capsid (e.g., one, two, three, four or more). Thus, a regimen may involve delivery of a rAd with a first simian capsid, delivery with a rAd with a second simian capsid, and delivery with a third simian capsid. A variety of other regimens which use the Ad capsids of the invention alone, in combination with one another, or in combination with other Ad serotypes will be apparent to those of skill in the art. Optionally, such a regimen may involve administration of rAd with capsids of other non-human primate adenoviruses, human adenoviruses, or artificial serotypes such as are described herein. Each phase of the regimen may involve administration of a series of injections (or other delivery routes) with a single Ad serotype capsid followed by a series with another Ad serotype capsid. Alternatively, the recombinant Ad vectors of the invention may be utilized in regimens involving other non-adenoviral-mediated delivery systems, including other viral systems, non-viral delivery systems, protein, peptides, and other biologically active molecules.

The following sections will focus on exemplary molecules which may be delivered via the adenoviral vectors of the invention.

### A. Ad-Mediated Delivery of Therapeutic Molecules

In one embodiment, the above-described recombinant vectors are administered to humans according to published methods for gene therapy. A simian viral vector bearing the selected transgene may be administered to a patient, preferably suspended in a biologically compatible solution or pharmaceutically acceptable delivery vehicle. A suitable vehicle includes sterile saline. Other aqueous and non-aqueous isotonic sterile injection solutions and aqueous and non-aqueous sterile suspensions known to be pharmaceutically acceptable carriers and well known to those of skill in the art may be employed for this purpose.

The simian adenoviral vectors are administered in sufficient amounts to transduce the target cells and to provide sufficient levels of gene transfer and expression to provide a therapeutic benefit without undue adverse or with medically acceptable physiological effects, which can be determined by those skilled in the medical arts. Conventional and pharmaceutically acceptable routes of administration include, but are not limited to, direct delivery to the retina and other intraocular delivery methods, direct delivery to the liver, inhalation, intranasal, intravenous, intramuscular, intratracheal, subcutaneous, intradermal, rectal, oral and other parenteral routes of administration. Routes of administration may be combined, if desired, or adjusted depending upon the transgene or the condition. The route of administration primarily will depend on the nature of the condition being treated.

Dosages of the viral vector will depend primarily on factors such as the condition being treated, the age, weight and health of the patient, and may thus vary among patients. For example, a therapeutically effective adult human or veterinary dosage of the viral vector is generally in the range of from about 100 µL to about 100 mL of a carrier containing concentrations of from about 1 x 10⁶ to about 1 x 10¹⁵ particles, about 1 x 10¹¹ to 1 x 10¹³ particles, or about 1 x 10⁹ to 1x 10¹² particles virus. Dosages will range depending upon the size of the animal and the route of administration. For example, a suitable human or veterinary dosage (for about an 80 kg animal) for intramuscular injection is in the range of about 1 x 10⁹ to about 5 x 10¹² particles per mL, for a single site. Optionally, multiple sites of administration may be delivered. In another example, a suitable human or veterinary dosage may be in the range of about 1 x 10¹¹ to about 1 x 10¹⁵ particles for an oral formulation. One of skill in the art may adjust these doses, depending the route of administration, and the therapeutic or vaccinal application for which the recombinant vector is employed. The levels of expression of the transgene, or for an immunogen, the level of circulating antibody, can be monitored to determine the frequency of dosage administration. Yet other methods for determining the timing of frequency of administration will be readily apparent to one of skill in the art.

An optional method step involves the co-administration to the patient, either concurrently with, or before or after administration of the viral vector, of a suitable amount of a short acting immune modulator. The selected immune modulator is defined herein as an agent capable of inhibiting the formation of neutralizing antibodies directed against the recombinant vector of this invention or capable of inhibiting cytolytic T lymphocyte (CTL) elimination of the vector. The immune modulator may interfere with the interactions between the T helper subsets (T_{H1} or T_{H2}) and B cells to inhibit neutralizing antibody formation. Alternatively, the immune modulator may inhibit the interaction between T_{H1} cells and CTLs to reduce the occurrence of CTL elimination of the vector. A variety of useful immune modulators and dosages for use of same are disclosed, for example, in Yang et al., J. Virol., 70(9) (Sept., 1996); International Patent Application No. WO96/12406, published May 2, 1996; and International Patent Application No.PCT/US96/03035, all incorporated herein by reference.

### 1. Therapeutic Transgenes

Useful therapeutic products encoded by the transgene include hormones and growth and differentiation factors including, without limitation, insulin, glucagon, growth hormone (GH), parathyroid hormone (PTH), growth hormone releasing factor (GRF), follicle stimulating hormone (FSH), luteinizing hormone (LH), human chorionic gonadotropin (hCG), vascular endothelial growth factor (VEGF), angiopoietins, angiostatin, granulocyte colony stimulating factor (GCSF), erythropoietin (EPO), connective tissue growth factor (CTGF), basic fibroblast growth factor (bFGF), acidic fibroblast growth factor (aFGF), epidermal growth factor (EGF), transforming growth factor (TGF), platelet-derived growth factor (PDGF), insulin growth factors I and II (IGF-I and IGF-II), any one of the transforming growth factor superfamily, including TGF, activins, inhibins, or any of the bone morphogenic proteins (BMP) BMPs 1-15, any one of the heregluin/neuregulin/ARIA/neu differentiation factor (NDF) family of growth factors, nerve growth factor (NGF), brain-derived neurotrophic factor (BDNF), neurotrophins NT-3 and NT-4/5, ciliary neurotrophic factor (CNTF), glial cell line derived neurotrophic factor (GDNF), neurturin, agrin, any one of the family of semaphorins/collapsins, netrin-1 and netrin-2, hepatocyte growth factor (HGF), ephrins, noggin, sonic hedgehog and tyrosine hydroxylase.

Other useful transgene products include proteins that regulate the immune system including, without limitation, cytokines and lymphokines such as thrombopoietin (TPO), interleukins (IL) IL-1 through IL-25 (including, e.g., IL-2, IL-4, IL-12 and IL-18), monocyte chemoattractant protein, leukemia inhibitory factor, granulocyte-macrophage colony stimulating factor, Fas ligand, tumor necrosis factors and, interferons, and, stem cell factor, flk-2/flt3 ligand. Gene products produced by the immune system are also useful in the invention. These include, without limitation, immunoglobulins IgG, IgM, IgA, IgD and IgE, chimeric immunoglobulins, humanized antibodies, single chain antibodies, T cell receptors, chimeric T cell receptors, single chain T cell receptors, class I and class II MHC molecules, as well as engineered immunoglobulins and MHC molecules. Useful gene products also include complement regulatory proteins such as complement regulatory proteins, membrane cofactor protein (MCP), decay accelerating factor (DAF), CR1, CF2 and CD59.

Still other useful gene products include any one of the receptors for the hormones, growth factors, cytokines, lymphokines, regulatory proteins and immune system proteins. The invention encompasses receptors for cholesterol regulation, including the low density lipoprotein (LDL) receptor, high density lipoprotein (HDL) receptor, the very low density lipoprotein (VLDL) receptor, and the scavenger receptor. The invention also encompasses gene products such as members of the steroid hormone receptor superfamily including glucocorticoid receptors and estrogen receptors, Vitamin D receptors and other nuclear receptors. In addition, useful gene products include transcription factors such as *jun, fos*, max, mad, serum response factor (SRF), AP-1, AP2, *myb*, MyoD and myogenin, ETS-box containing proteins, TFE3, E2F, ATF1, ATF2, ATF3, ATF4, ZF5, NFAT, CREB, HNF-4, C/EBP, SP1, CCAAT-box binding proteins, interferon regulation factor (IRF-1), Wilms tumor protein, ETS-binding protein, STAT, GATA-box binding proteins, e.g., GATA-3, and the forkhead family of winged helix proteins.

Other useful gene products include, carbamoyl synthetase I, ornithine transcarbamylase, arginosuccinate synthetase, arginosuccinate lyase, arginase, fumarylacetacetate hydrolase, phenylalanine hydroxylase, alpha-1 antitrypsin, glucose-6-phosphatase, porphobilinogen deaminase, factor VIII, factor IX, cystathione beta-synthase, branched chain ketoacid decarboxylase, albumin, isovaleryl-coA dehydrogenase, propionyl CoA carboxylase, methyl malonyl CoA mutase, glutaryl CoA dehydrogenase, insulin, beta-glucosidase, pyruvate carboxylate, hepatic phosphorylase, phosphorylase kinase, glycine decarboxylase, H-protein, T-protein, a cystic fibrosis transmembrane regulator(CFTR) sequence, and a dystrophin cDNA sequence.

Other useful gene products include non-naturally occurring polypeptides, such as chimeric or hybrid polypeptides having a non-naturally occurring amino acid sequence containing insertions, deletions or amino acid substitutions. For example, single-chain engineered immunoglobulins could be useful in certain immunocompromised patients. Other types of non-naturally occurring gene sequences include antisense molecules and catalytic nucleic acids, such as ribozymes, which could be used to reduce overexpression of a target.

Reduction and/or modulation of expression of a gene are particularly desirable for treatment of hyperproliferative conditions characterized by hyperproliferating cells, as are cancers and psoriasis. Target polypeptides include those polypeptides which are produced exclusively or at higher levels in hyperproliferative cells as compared to normal cells. Target antigens include polypeptides encoded by oncogenes such as myb, myc, fyn, and the translocation gene bcr/abl, ras, src, P53, neu, trk and EGRF. In addition to oncogene products as target antigens, target polypeptides for anti-cancer treatments and protective regimens include variable regions of antibodies made by B cell lymphomas and variable regions of T cell receptors of T cell lymphomas which, in some embodiments, are also used as target antigens for autoimmune disease. Other tumor-associated polypeptides can be used as target polypeptides such as polypeptides which are found at higher levels in tumor cells including the polypeptide recognized by monoclonal antibody 17-1A and folate binding polypeptides.

Other suitable therapeutic polypeptides and proteins include those which may be useful for treating individuals suffering from autoimmune diseases and disorders by conferring a broad based protective immune response against targets that are associated with autoimmunity including cell receptors and cells which produce self-directed antibodies. T cell mediated autoimmune diseases include Rheumatoid arthritis (RA), multiple sclerosis (MS), Sjögren's syndrome, sarcoidosis, insulin dependent diabetes mellitus (IDDM), autoimmune thyroiditis, reactive arthritis, ankylosing spondylitis, scleroderma, polymyositis, dermatomyositis, psoriasis, vasculitis, Wegener's granulomatosis, Crohn's disease and ulcerative colitis. Each of these diseases is characterized by T cell receptors (TCRs) that bind to endogenous antigens and initiate the inflammatory cascade associated with autoimmune diseases.

The simian adenoviral vectors of the invention are particularly well suited for therapeutic regimens in which multiple adenoviral-mediated deliveries of transgenes is desired, e.g., in regimens involving redelivery of the same transgene or in combination regimens involving delivery of other transgenes. Such regimens may involve administration of a Pan5, Pan6, Pan7, SV1, SV25 or SV39 simian adenoviral vector, followed by re-administration with a vector from the same serotype adenovirus. Particularly desirable regimens involve administration of a Pan5, Pan6, Pan7, SV1, SV25 or SV39 simian adenoviral vector of the invention, in which the serotype ofthe viral vector delivered in the first administration differs from the serotype of the viral vector utilized in one or more of the subsequent administrations. For example, a therapeutic regimen involves administration of a Pan5, Pan6, Pan7, SV1, SV25 or SV39 vector and repeat administration with one or,more adenoviral vectors of the same or different serotypes. In another example, a therapeutic regimen involves administration of an adenoviral vector followed by repeat administration with a Pan5, Pan6, Pan7, SV1, SV25 or SV39 vector of the invention which differs from the serotype of the first delivered adenoviral vector, and optionally further administration with another vector which is the same or, preferably, differs from the serotype of the vector in the prior administration steps. These regimens are not limited to delivery of adenoviral vectors constructed using the Pan5, Pan6, Pan7, SV1, SV25 or SV39 simian serotypes of the invention. Rather, these regimens can readily utilize vectors other adenoviral serotypes, including, without limitation, other simian adenoviral serotypes (e.g., Pan9 or C68, C1, etc), other non-human primate adenoviral serotypes, or human adenoviral serotypes, in combination with one or more of the Pan5, Pan6, Pan7, SV1, SV25 or SV39 vectors of the invention. Examples of such simian, other non-human primate and human adenoviral serotypes are discussed elsewhere in this document. Further, these therapeutic regimens may involve either simultaneous or sequential delivery of Pan 5, Pan6, Pan7, SV1, SV25, and/or SV39 adenoviral vectors of the invention in combination with non-adenoviral vectors, non-viral vectors, and/or a variety of other therapeutically useful compounds or molecules. The present invention is not limited to these therapeutic regimens, a variety of which will be readily apparent to one of skill in the art.

### B. Ad-Mediated Delivery of Immunogenic Transgenes

The recombinant simian adenoviruses may also be employed as immunogenic compositions. As used herein, an immunogenic composition is a composition to which a humoral (e.g., antibody) or cellular (e.g., a cytotoxic T cell) response is mounted to a transgene product delivered by the immunogenic composition following delivery to a mammal, and preferably a primate. The present invention provides a recombinant simian Ad that can contain in any of its adenovirus sequence deletions a gene encoding a desired immunogen. The simian adenovirus is likely to be better suited for use as a live recombinant virus vaccine in different animal species compared to an adenovirus of human origin, but is not limited to such a use. The recombinant adenoviruses can be used as prophylactic or therapeutic vaccines against any pathogen for which the antigen(s) crucial for induction of an immune response and able to limit the spread of the pathogen has been identified and for which the cDNA is available.

Such vaccinal (or other immunogenic) compositions are formulated in a suitable delivery vehicle, as described above. Generally, doses for the immunogenic compositions are in the range defined above for therapeutic compositions. The levels of immunity of the selected gene can be monitored to determine the need, if any, for boosters. Following an assessment of antibody titers in the serum, optional booster immunizations may be desired.

Optionally, a vaccinal composition of the invention may be formulated to contain other components, including, e.g. adjuvants, stabilizers, pH adjusters, preservatives and the like. Such components are well known to those of skill in the vaccine art. Examples of suitable adjuvants include, without limitation, liposomes, alum, monophosphoryl lipid A, and any biologically active factor, such as cytokine, an interleukin, a chemokine, a ligands, and optimally combinations thereof. Certain of these biologically active factors can be expressed *in vivo*, e.g., via a plasmid or viral vector. For example, such an adjuvant can be administered with a priming DNA vaccine encoding an antigen to enhance the antigen-specific immune response compared with the immune response generated upon priming with a DNA vaccine encoding the antigen only.

The recombinant adenoviruses are administered in a "an immunogenic amount", that is, an amount of recombinant adenovirus that is effective in a route of administration to transfect the desired cells and provide sufficient levels of expression of the selected gene to induce an immune response. Where protective immunity is provided, the recombinant adenoviruses are considered to be vaccine compositions useful in preventing infection and/or recurrent disease.

Alternatively, or in addition, the vectors of the invention may contain a transgene encoding a peptide, polypeptide or protein which induces an immune response to a selected immunogen. The recombinant adenoviruses of this invention are expected to be highly efficacious at inducing cytolytic T cells and antibodies to the inserted heterologous antigenic protein expressed by the vector.

For example, immunogens may be selected from a variety of viral families. Example of desirable viral families against which an immune response would be desirable include, the picornavirus family, which includes the genera rhinoviruses, which are responsible for about 50% of cases of the common cold; the genera enteroviruses, which include polioviruses, coxsackieviruses, echoviruses, and human enteroviruses such as hepatitis A virus; and the genera apthoviruses, which are responsible for foot and mouth diseases, primarily in non-human animals. Within the picornavirus family of viruses, target antigens include the VP1, VP2, VP3, VP4, and VPG. Another viral family includes the calcivirus family, which encompasses the Norwalk group of viruses, which are an important causative agent of epidemic gastroenteritis. Still another viral family desirable for use in targeting antigens for inducing immune responses in humans and non-human animals is the togavirus family, which includes the genera alphavirus, which include Sindbis viruses, RossRiver virus, and Venezuelan, Eastern & Western Equine encephalitis, and rubivirus, including Rubella virus. The flaviviridae family includes dengue, yellow fever, Japanese encephalitis, St. Louis encephalitis and tick borne encephalitis viruses. Other target antigens may be generated from the Hepatitis C or the coronavirus family, which includes a number of non-human viruses such as infectious bronchitis virus (poultry), porcine transmissible gastroenteric virus (pig), porcine hemagglutinating encephalomyelitis virus (pig), feline infectious peritonitis virus (cats), feline enteric coronavirus (cat), canine coronavirus (dog), and human respiratory coronaviruses, which may cause the common cold and/or non-A, B or C hepatitis. Within the coronavirus family, target antigens include the E1 (also called M or matrix protein), E2 (also called S or Spike protein), E3 (also called HE or hemagglutin-elterose) glycoprotein (not present in all coronaviruses), or N (nucleocapsid). Still other antigens may be targeted against the rhabdovirus family, which includes the genera vesiculovirus (e.g., Vesicular Stomatitis Virus), and the general lyssavirus (e.g., rabies). Within the rhabdovirus family, suitable antigens may be derived from the G protein or the N protein. The family filoviridae, which includes hemorrhagic fever viruses such as Marburg and Ebola virus, may be a suitable source of antigens. The paramyxovirus family includes parainfluenza Virus Type 1, parainfluenza Virus Type 3, bovine parainfluenza Virus Type 3, rubulavirus (mumps virus), parainfluenza Virus Type 2, parainfluenza virus Type 4, Newcastle disease virus (chickens), rinderpest, morbillivirus, which includes measles and canine distemper, and pneumovirus, which includes respiratory syncytial virus. The influenza virus is classified within the family orthomyxovirus and is a suitable source of antigen (e.g., the HA protein, the N1 protein). The bunyavirus family includes the genera bunyavirus (California encephalitis, La Crosse), phlebovirus (Rift Valley Fever), hantavirus (puremala is a hemahagin fever virus), nairovirus (Nairobi sheep disease) and various unassigned bungaviruses. The arenavirus family provides a source of antigens against LCM and Lassa fever virus. The reovirus family includes the genera reovirus, rotavirus (which causes acute gastroenteritis in children), orbiviruses, and cultivirus (Colorado Tick fever, Lebombo (humans), equine encephalosis, blue tongue).

The retrovirus family includes the sub-family oncorivirinal which encompasses such human and veterinary diseases as feline leukemia virus, HTLVI and HTLVII, lentivirinal (which includes human immunodeficiency virus (HIV), simian immunodeficiency virus (SIV), feline immunodeficiency virus (FIV), equine infectious anemia virus, and spumavirinal). Among the lentiviruses, many suitable antigens have been described and can readily be selected. Examples of suitable HIV and SIV antigens include, without limitation the gag, pol, Vif, Vpx, VPR, Env, Tat, Nef, and Rev proteins, as well as various fragments thereof. For example, suitable fragments of the Env protein may include any of its subunits such as the gp120, gp160, gp41, or smaller fragments thereof, e.g., of at least about 8 amino acids in length. Similarly, fragments of the tat protein may be selected. [See, US Patent 5,891,994 and US Patent 6,193,981.] See, also, the HIV and SIV proteins described in D.H. Barouch et al, J. Virol., 75(5):2462-2467 (March 2001), and R.R. Amara, et al, Science, 292:69-74 (6 April 2001). In another example, the HIV and/or SIV immunogenic proteins or peptides may be used to form fusion proteins or other immunogenic molecules. See, e.g., the HIV-1 Tat and/or Nef fusion proteins and immunization regimens described in WO 01/54719, published August 2,2001, and WO 99/16884, published April 8, 1999. The invention is not limited to the HIV and/or SIV immunogenic proteins or peptides described herein. In addition, a variety of modifications to these proteins have been described or could readily be made by one of skill in the art. See, e.g., the modified gag protein that is described in US Patent 5,972,596. Further, any desired HIV and/or SIV immunogens may be delivered alone or in combination. Such combinations may include expression from a single vector or from multiple vectors. Optionally, another combination may involve delivery of one or more expressed immunogens with delivery of one or more of the immunogens in protein form. Such combinations are discussed in more detail below.

The papovavirus family includes the sub-family polyomaviruses (BKU and JCU viruses) and the sub-family papillomavirus (associated with cancers or malignant progression of papilloma). The adenovirus family includes viruses (EX, AD7, ARD, O.B.) which cause respiratory disease and/or enteritis. The parvovirus family feline parvovirus (feline enteritis), feline panleucopeniavirus, canine parvovirus, and porcine parvovirus. The herpesvirus family includes the sub-family alphaherpesvirinae, which encompasses the genera simplexvirus (HSVI, HSVII), varicellovirus (pseudorabies, varicella zoster) and the sub-family betaherpesvirinae, which includes the genera cytomegalovirus (HCMV, muromegalovirus) and the sub-family gammaherpesvirinae, which includes the genera lymphocryptovirus, EBV (Burkitts lymphoma), infectious rhinotracheitis, Marek's disease virus, and rhadinovirus. The poxvirus family includes the sub-family chordopoxvirinae, which encompasses the genera orthopoxvirus (Variola (Smallpox) and Vaccinia (Cowpox)), parapoxvirus, avipoxvirus, capripoxvirus, leporipoxvirus, suipoxvirus, and the sub-family entomopoxvirinae. The hepadnavirus family includes the Hepatitis B virus. One unclassified virus which may be suitable source of antigens is the Hepatitis delta virus. Still other viral sources may include avian infectious bursal disease virus and porcine respiratory and reproductive syndrome virus. The alphavirus family includes equine arteritis virus and various Encephalitis viruses.

The present invention may also encompass immunogens which are useful to immunize a human or non-human animal against other pathogens including bacteria, fungi, parasitic microorganisms or multicellular parasites which infect human and non-human vertebrates, or from a cancer cell or tumor cell. Examples of bacterial pathogens include pathogenic gram-positive cocci include pneumococci; staphylococci; and streptococci. Pathogenic gram-negative cocci include meningococcus; gonococcus. Pathogenic enteric gram-negative bacilli include enterobacteriaceae; pseudomonas, acinetobacteria and eikenella; melioidosis; salmonella; shigella; haemophilus; moraxella; *H. ducreyi* (which causes chancroid); brucella; *Franisella tularensis* (which causes tularemia); yersinia (pasteurella); streptobacillus moniliformis and spirillum; Gram-positive bacilli include listeria monocytogenes; erysipelothrix rhusiopathiae; *Corynebacterium diphtheria* (diphtheria); cholera; *B. anthracis* (anthrax); donovanosis (granuloma inguinale); and bartonellosis. Diseases caused by pathogenic anaerobic bacteria include tetanus; botulism; other clostridia; tuberculosis; leprosy; and other mycobacteria. Pathogenic spirochetal diseases include syphilis; treponematoses: yaws, pinta and endemic syphilis; and leptospirosis. Other infections caused by higher pathogen bacteria and pathogenic fungi include actinomycosis; nocardiosis; cryptococcosis, blastomycosis, histoplasmosis and coccidioidomycosis; candidiasis, aspergillosis, and mucormycosis; sporotrichosis; paracoccidiodomycosis, petriellidiosis, torulopsosis, mycetoma and chromomycosis; and dermatophytosis. Rickettsial infections include Typhus fever, Rocky Mountain spotted fever, Q fever, and Rickettsialpox. Examples of mycoplasma and chlamydial infections include: mycoplasma pneumoniae; lymphogranuloma venereum; psittacosis; and perinatal chlamydial infections. Pathogenic eukaryotes encompass pathogenic protozoans and helminths and infections produced thereby include: amebiasis; malaria; leishmaniasis; trypanosomiasis; toxoplasmosis; *Pneumocystis carinii; Trichans*; *Toxoplasma gondii*; babesiosis; giardiasis; trichinosis; filariasis; schistosomiasis; nematodes; trematodes or flukes; and cestode (tapeworm) infections.

Many of these organisms and/or toxins produced thereby have been identified by the Centers for Disease Control [(CDC), Department of Heath and Human Services, USA], as agents which have potential for use in biological attacks. For example, some of these biological agents, include, *Bacillus anthracis* (anthrax), *Clostridium botulinum* and its toxin (botulism), *Yersinia pestis* (plague), variola major (smallpox), *Francisella tularensis* (tularemia), and viral hemorrhagic fevers [filoviruses (e.g., Ebola, Marburg], and arenaviruses [e.g., Lassa, Machupo]), all of which are currently classified as Category A agents; *Coxiella burnetti* (Q fever); Brucella species (brucellosis), *Burkholderia mallei* (glanders), *Burkholderia pseudomallei* (meloidosis), *Ricinus communis* and its toxin (ricin toxin), *Clostridium perfringens* and its toxin (epsilon toxin), *Staphylococcus* species and their toxins (enterotoxin B), *Chlamydia psittaci* (psittacosis), water safety threats (e.g., *Vibrio cholerae, Crytosporidium parvum*), Typhus fever (*Richettsia powazekii*), and viral encephalitis (alphaviruses, e.g., Venezuelan equine encephalitis; eastern equine encephalitis; western equine encephalitis); all of which are currently classified as Category B agents; and Nipan virus and hantaviruses, which are currently classified as Category C agents. In addition, other organisms, which are so classified or differently classified, may be identified and/or used for such a purpose in the future. It will be readily understood that the viral vectors and other constructs described herein are useful to deliver antigens from these organisms, viruses, their toxins or other by-products, which will prevent and/or treat infection or other adverse reactions with these biological agents.

Administration of the vectors of the invention to deliver immunogens against the variable region of the T cells elicit an immune response including CTLs to eliminate those T cells. In RA, several specific variable regions of TCRs which are involved in the disease have been characterized. These TCRs include V-3, V-14, V-17 and Vα-17. Thus, delivery of a nucleic acid sequence that encodes at least one of these polypeptides will elicit an immune response that will target T cells involved in RA. In MS, several specific variable regions of TCRs which are involved in the disease have been characterized. These TCRs include V-7 and Vα-10. Thus, delivery of a nucleic acid sequence that encodes at least one of these polypeptides will elicit an immune response that will target T cells involved in MS. In scleroderma, several specific variable regions of TCRs which are involved in the disease have been characterized. These TCRs include V-6, V-8, V-14 and Vα-16, Vα-3C, Vα-7, Vα-14, Vα-15, Vα-16, Vα-28 and Vα-12. Thus, delivery of a recombinant simian adenovirus that encodes at least one of these polypeptides will elicit an immune response that will target T cells involved in scleroderma.

### C. Ad-Mediated Delivery Methods

The therapeutic levels, or levels of immunity, of the selected gene can be monitored to determine the need, if any, for boosters. Following an assessment of CD8+ T cell response, or optionally, antibody titers, in the serum, optional booster immunizations may be desired. Optionally, the recombinant simian-adenoviral vectors of the invention may be delivered in a single administration or in various combination regimens, e.g., in combination with a regimen or course of treatment involving other active ingredients or in a prime-boost regimen. A variety of such regimens have been described in the art and may be readily selected.

For example, prime-boost regimens may involve the administration of a DNA (e.g., plasmid) based vector to prime the immune system to second, booster, administration with a traditional antigen, such as a protein or a recombinant virus carrying the sequences encoding such an antigen. See, e.g., WO 00/11140, published March 2, 2000, incorporated by reference. Alternatively, an immunization regimen may involve the administration of a recombinant simian adenoviral vector of the invention to boost the immune response to a vector (either viral or DNA-based) carrying an antigen, or a protein. In still another alternative, an immunization regimen involves administration of a protein followed by booster with a vector encoding the antigen.

In one embodiment, the invention provides a method of priming and boosting an immune response to a selected antigen by delivering a plasmid DNA vector carrying said antigen, followed by boosting with a recombinant simian adenoviral vector of the invention. In one embodiment, the prime-boost regimen involves the expression of multiproteins from the prime and/or the boost vehicle. See, e.g., R.R. Amara, Science, 292:69-74 (6 April 2001) which describes a multiprotein regimen for expression of protein subunits useful for generating an immune response against HIV and SIV. For example, a DNA prime may deliver the Gag, Pol, Vif, VPX and Vpr and Env, Tat, and Rev from a single transcript. Alternatively, the SIV Gag, Pol and HIV-1 Env is delivered in a recombinant adenovirus construct of the invention. Still other regimens are described in WO 99/16884 and WO 01/54719.

However, the prime-boost regimens are not limited to immunization for HIV or to delivery of these antigens. For example, priming may involve delivering with a first chimp vector of the invention followed by boosting with a second chimp vector, or with a composition containing the antigen itself in protein form. In one example, the prime-boost regimen can provide a protective immune response to the virus, bacteria or other organism from which the antigen is derived. In another desired embodiment, the prime-boost regimen provides a therapeutic effect that can be measured using convention assays for detection of the presence of the condition for which therapy is being administered.

The priming composition may be administered at various sites in the body in a dose dependent manner, which depends on the antigen to which the desired immune response is being targeted. The invention is not limited to the amount or situs of injection(s) or to the pharmaceutical carrier. Rather, the regimen may involve a priming and/or boosting step, each of which may include a single dose or dosage that is administered hourly, daily, weekly or monthly, or yearly. As an example, the mammals may receive one or two doses containing between about 10 µg to about 50 µg of plasmid in carrier. A desirable amount of a DNA composition ranges between about 1 µg to about 10,000 µg of the DNA vector. Dosages may vary from about 1 µg to 1000 µg DNA per kg of subject body weight. The amount or site of delivery is desirably selected based upon the identity and condition of the mammal.

The dosage unit of the vector suitable for delivery of the antigen to the mammal is described herein. The vector is prepared for administration by being suspended or dissolved in a pharmaceutically or physiologically acceptable carrier such as isotonic saline; isotonic salts solution or other formulations that will be apparent to those skilled in such administration. The appropriate carrier will be evident to those skilled in the art and will depend in large part upon the route of administration. The compositions of the invention may be administered to a mammal according to the routes described above, in a sustained release formulation using a biodegradable biocompatible polymer, or by on-site delivery using micelles, gels and liposomes. Optionally, the priming step of this invention also includes administering with the priming composition, a suitable amount of an adjuvant, such as are defined herein.

Preferably, a boosting composition is administered about 2 to about 27 weeks after administering the priming composition to the mammalian subject. The administration of the boosting composition is accomplished using an effective amount of a boosting composition containing or capable of delivering the same antigen as administered by the priming DNA vaccine. The boosting composition may be composed of a recombinant viral vector derived from the same viral source (e.g., adenoviral sequences of the invention) or from another source. Alternatively, the "boosting composition" can be a composition containing the same antigen as encoded in the priming DNA vaccine, but in the form of a protein or peptide, which composition induces an immune response in the host. In another embodiment, the boosting composition contains a DNA sequence encoding the antigen under the control of a regulatory sequence directing its expression in a mammalian cell, e.g., vectors such as well-known bacterial or viral vectors. The primary requirements of the boosting composition are that the antigen of the composition is the same antigen, or a cross-reactive antigen, as that encoded by the priming composition.

In another embodiment, the simian adenoviral vectors of the invention are also well suited for use in a variety of other immunization and therapeutic regimens. Such regimens may involve delivery of simian adenoviral vectors of the invention simultaneously or sequentially with Ad vectors of different serotype capsids, regimens in which adenoviral vectors of the invention are delivered simultaneously or sequentially with non-Ad vectors, regimens in which the adenoviral vectors of the invention are delivered simultaneously or sequentially with proteins, peptides, and/or other biologically useful therapeutic or immunogenic compounds. Such uses will be readily apparent to one of skill in the art.

The following examples illustrate the cloning of the simian adenoviruses and the construction of exemplary recombinant adenovirus vectors of the present invention. These examples are illustrative only, and do not limit the scope of the present invention.

### Example 1 - Viral Propagation

The Pan5 [ATCC Accession No. VR-591], Pan6 [ATCC Accession No. VR-592], and Pan7 [ATCC Accession No. VR-593] viruses, originally isolated from lymph nodes from chimpanzees, were propagated in 293 cells [ATCC CRL1573]. Typically, these cells are cultured in Dulbecco's Modified Eagles Medium (DMEM; Sigma, St. Louis, MO.) supplemented with 10% fetal calf serum (FCS) [Sigma or Hyclone, Logan, UT] and 1 % Penicillin-Streptomycin (Sigma). Infection of 293 cells is carried out in DMEM supplemented with 2% FCS for the first 24 hours, after which FCS is added to bring the final concentration to 10%. Infected cells are harvested when 100% of the cells exhibit virus-induced cytopathic effect (CPE), and are then collected, and concentrated by centrifugation. Cell pellets are resuspended in 10 mM Tris (pH 8.0), and lysed by 3 cycles of freezing and thawing. Virus preparations are obtained following two ultra centrifugation steps on cesium chloride density gradients and stocks of virus are diluted to 1 to 5 x 10¹² particles/ml in 10 mM Tris/100 mM NaCl/50% glycerol and stored at -70°C.

The ability of 293 cells to propagate these adenoviruses exceeded expectations which were based on knowledge of other non-human adenovirus serotypes.

| Virus | Yield (virus particles produced in 8x10⁸ cells) |
|---|---|
| Pan5 | 8.8 x 10¹³ |
| Pan6 | 1.6 x 10¹⁴ |
| Pan7 | 8.8 x 10¹³ |

### Example 2 - Characterization of Viral Genomic DNA

Genomic DNA was isolated from the purified virus preparations of Example 1 and digested with HindIII or BamHI restriction enzymes following the manufacturers' recommendations. The results (not shown) revealed that that the Pan5, Pan6, Pan7 genomes ofthe invention and the published Pan 9 (C68) genome show different restriction patterns, and thus, are distinct from each other.

The nucleotide sequences of Pan5, Pan6 and Pan7 were determined. The nucleotide sequence ofthe top strand of Pan5 DNA is reported in SEQ ID NO: 1. The nucleotide sequence of the top strand of Pan6 DNA is reported in SEQ ID NO: 5. The nucleotide sequence of the top strand of Pan7 DNA is reported in SEQ ID NO: 9.

Regulatory and coding regions in the viral DNA sequences were identified by homology to known adenoviral sequences using the "Clustal W" program described above at conventional settings. See the tables above providing the adenoviral sequences. Open reading frames were translated and the predicted amino acid sequences examined for homology to previously described adenoviral protein sequences, Ad4, Ad5, Ad7, Ad12, and Ad40.

Analysis of the sequence revealed a genome organization that is similar to that present in human adenoviruses, with the greatest similarity to human Ad4. However, substantial differences in the hexon hypervariable regions were noted between the chimpanzee adenoviruses and other known adenoviruses, including AdHu4. These differences fit well with the serological cross-reactivity data that has been obtained (see below).

An alignment of a portion of the hexon sequences is shown in Fig. 1. The portion shown is from the region of the hexon that corresponds to the outwardly disposed extended loops DE1 and FG1 where the most variability between serotypes is observed. An intervening portion that contributes to the base of the hexon (corresponding to residues 308-368 of the published AdC68 sequence; US Patent 6,083,716), and is highly conserved between serotypes, is also present. The following table summarizes the pair-wise comparisons of the amino acids in the hexon proteins.

| Comparison | | Hexon amino-acid |
|---|---|---|
| | | Similarity (%) |
| #1 | #2 | |
| AdC5 | AdC7 | 99.0 |
| AdC5 | AdC68 | 98.3 |
| AdC5 | AdC6 | 88.0 |
| AdC5 | AdC1 | 84.9 |
| AdC6 | AdC7 | 87.7 |
| AdC6 | AdC68 | 87.3 |
| AdC6 | AdC1 | 84.9 |
| AdC7 | AdC68 | 97.5 |
| AdC7 | AdC1 | 84.8 |
| AdC68 | AdC1 | 84.9 |

Analysis of the fiber knob domain (which is responsible for receptor binding) of the chimpanzee adenoviruses shows an overall similarity in structure (Fig. 2).

The degree of sequence similarity between the E1 proteins of huAd5 and C68 (see Tables below) is similar to that between huAd5 and Pan-5, Pan-6, and Pan-7.

| Comparison | | E1a (13S) amino-acid identity (%) |
|---|---|---|
| #1 | #2 | |
| AdHu5 | AdC5 | 36.6 |
| AdHu5 | AdC6 | 28.5 |
| AdHu5 | AdC7 | 34.9 |
| AdHu5 | AdC68 | 35.6 |
| AdHu5 | AdC1 | 35.6 |
| AdC5 | AdC6 | 68.3 |
| AdC5 | AdC7 | 96.9 |
| AdC5 | AdC68 | 80.4 |
| AdC5 | AdC1 | 51.3 |
| AdC6 | AdC7 | 69.3 |
| AdC6 | AdC68 | 59.4 |
| AdC6 | Add | 37.7 |
| AdC7 | AdC68 | 81.5 |
| AdC7 | AdC1 | 51.0 |
| AdC68 | AdC1 | 54.9 |

| | Sequence Identity with human Ad5 | |
|---|---|---|
| | E1b Small T Protein | E1b Large T Protein |
| C68 | 47.3% | 55.8% |
| Pan-5 | 43.2% | 54.5% |
| Pan-6 | 45.3% | 54.5% |
| Pan-7 | 46.4% | 53.8% |

Replication-defective versions of AdC5, AdC6 and AdC7 were created by molecular cloning methods described in the following examples in which minigene cassettes were inserted into the place of the E1a and E1b genes. The molecular clones of the recombinant viruses were rescued and grown up in 293 cells for large-scale purification using the published CsCl sedimentation method [K. Fisher et al., J. Virol., 70:520 (1996)]. Vector yields were based on 50 plate (150 mm) preps in which approximately 1 x 10⁹ 293 cells were infected with the corresponding viruses. Yields were determined by measuring viral particle concentrations spectrophotometrically. After having constructed E1-deleted vectors, it was determined that HEK 293 cells (which express human adenovirus serotype 5 E1 functions) trans-complement the E1 deletions of the novel viral vectors and allow for the production of high titer stocks. Examples of virus yields for a few of these recombinant viruses are shown in the table below.

The transgenes for these vectors, β-galactosidase (LacZ), green fluorescent protein (GFP), alpha-1-anti-trypsin (A1AT), ebola glycoprotein (ebo), a soluble ebola glycoprotein variant lacking the transmembrane and cytoplasmic domains (sEbo), and three deletion mutants of the ebola glycoprotein (EboΔ2, EboΔ3, and EboΔ4), were expressed by the cytomegalovirus promoter (CMV). In the following table, ND indicates that the study has not yet been done.

| Transgene | Viral backbone/Vector yield (Viral particles x 10¹³) | | | | | | |
|---|---|---|---|---|---|---|---|
| | AdHu5 | | AdC7 | | AdC68 | | AdC6 |
| | | | | | | | |
| CMVLacZ | 1.5 | | 1.4 | | 3.3 | | 6.1 |
| CMVGFP | 2.5 | | 3.6 | | 8 | | 10 |
| CMVA1AT | 3.7 | | 6 | | 10 | | ND |
| CMVEbo | 1.1 | | 4.3 | | ND | | ND |
| CMVsEbo | 4.9 | | 5.4 | | ND | | ND |
| CMVEboΔ2 | 1 | | 9.3 | | ND | | ND |
| CMVEboΔ3 | 0.8 | | 9.5 | | ND | | ND |
| CMVEboΔ4 | 1.4 | | 6.2 | | ND | | ND |

The ability of human adenovirus E1 to trans-complement the E1-deleted chimpanzee viruses of the invention is highly advantageous, as it permits the production of E1-deleted chimpanzee adenoviral vectors of the invention, while reducing or eliminating the risk of homologous recombination due to the differences in sequences between human Ad and the chimpanzee adenoviruses described herein.

### Example 3 - Serological Studies of Pan 5, 6, and 7 Viruses

Because of the differences in the hexon hypervariable region, it was anticipated that the C5, C6, and C7 viruses would be serologically distinct from human adenoviruses, including AdHu4.

### 1. Cross-Reactivity of Wild-type Viruses

For screening of wild-type viruses in order to make a determination of antibody cross-reactivity, the replication competent viruses were used and inhibition of cytopathic effects (CPE) was measured. Briefly, preparations of adenoviruses (Adhu5, Pan-5, Pan-6, Pan-7 and AdC68) stored at 5x10¹² particles/ml were diluted 1/600 for the assays. This concentration of virus was selected since it results in 100% CPE within 48 hours in the absence of neutralization. Prior to adding the virus to 293 cells (4x10⁴ cells/well in a 96 well dish), 1:20 dilutions of sera were added. The assay is read as the presence or absence of CPE; full neutralization would read as no cytopathic effect. The results are summarized in the Table below. The fact that 9/36 human sera neutralized Adhu5 induced CPE is consistent with previous estimates of neutralizing antibodies in the human population. The numbers indicate the total individuals who showed neutralization (numerator) versus the total number screened (denominator). ND = not determined.

| | Neutralization by 1/20 diln of serum | | |
|---|---|---|---|
| | Human | Rhesus | Chimpanzee |
| | (N=36) | (N=52) | (N=20) |
| Adhu5 | 9/36 | ND | ND |
| AdC68 | 1/36 | 0/52 | 12/20 |
| Pan 5 | 0/36' | 0/52 | 10/20 |
| Pan 6 | 0/36 | 0/52 | 9/20 |
| Pan 7 | 0/36 | 0/52 | 12/20 |

Of all human sera screened, 35/36 were negative for neutralization to AdC68 while 36/36 were negative for neutralization to Pan-5, Pan-6 and Pan-7. Of 52 rhesus monkeys screened, none showed neutralization to any chimpanzee adenovirus; rhesus monkey is the preferred pre-clinical model for evaluating HIV vaccines. Between 9 to 12 out of 20 chimpanzees had substantial neutralization to one or another of the chimpanzee adenoviruses consistent with the fact these are indeed endemic chimpanzee-specific pathogens. Interestingly, there are chimpanzees with neutralizing antibodies only to Pan-5, Pan-6 or AdC68 supporting the hypothesis that several of these chimpanzee adenoviral vectors will not cross neutralize each other and are distinct serotypes.

The same assay was carried out for 20 chimpanzee serum samples. Fifty percent (50%) of the samples reacted serologically, in different degrees to Pan5; 40% to Pan6; 55% to Pan7 and 60% to C68. Among the positive serum samples, one of them had strong neutralizing activity to all four chimp viruses.

### 2. Cross-neutralization with Recombinant Viruses

High-titer polyclonal antibodies were obtained to each of the simian adenoviruses in order to more precisely gauge the degree of cross-neutralization among the different serotypes. This was done by intramuscular immunization of rabbits using a recombinant virus containing GFP based on previously the described C68 chimpanzee adenovirus as an adjuvant. The serum was then used to assay for neutralizing activity against each of the three chimpanzee adenoviruses of the invention, AdC5, AdC6 and AdC7. A rabbit was injected with 5x10¹² viral particle per kg of C68CMVGFP vector intramuscularly and boosted 5 weeks later using the same dose. A bleed collected at the 9 week time point revealed extremely potent neutralizing activity against C68 as well as Pan-5 and Pan-7 but not against Pan-6 (see Table below), indicating that the administration of a C68 (or Pan-5 and Pan-7) based vaccine could be effectively followed by a boost using a vector based on Pan-6. However, it has been found that this level of inter-relatedness does not necessarily prevent with re-administration in a setting where antiviral antibody titers were not as high as was achieved in this rabbit. In the following table, + indicates 33% CPE; ++ indicates 66% CPE; +++ indicates 100% CPE.

| Infection on 293 cells with virus: | | | | | |
|---|---|---|---|---|---|
| Pan5 | Pan6 | Pan7 | Pan9(C68) | C68 GFP | Serum Dilution |
| - | +++ | - | - | - | 1/20 |
| - | +++ | - | - | - | 1/40 |
| - | +++ | - | - | - | 1/80 |
| - | +++ | - | - | - | 1/160 |
| - | +++ | - | - | - | 1/320 |
| - | +++ | - | - | - | 1/640 |
| - | +++ | - | - | - | 1/1,280 |
| - | +++ | - | - | - | 1/2,560 |
| - | +++ | - | - | - | 1/5,120 |
| + | +++ | - | - | - | 1/10,240 |
| + | +++ | ++ | - | - | 1/20,480 |
| ++ | +++ | +++ | - | - | 1/40,960 |
| ++ | +++ | +++ | + | + | 1/81,920 |
| +++ | +++ | +++ | ++ | ++ | 1/163,840 |
| +++ | +++ | +++ | +++ | +++ | 1/327,680 |
| +++ | +++ | +++ | +++ | +++ | 1/665,360 |
| +++ | +++ | +++ | +++ | +++ | 1/1,310,720 |
| +++ | +++ | +++ | +++ | +++ | 1/2,621,440 |

### 3. Quantitative Assay for Detection of Neutralizing Antibody

The result was validated by a more quantitative-based assay for detecting neutralizing antibody, which is based on transduction of a GFP vector. Briefly, groups of C57BL/6 mice were immunized intramuscularly or intravenously with 5.0 x 10¹⁰ particles/ml Pan5, Pan6, Pan7 or C68. Sera from day 28 bleeds were tested for cross-neutralizing activity against C68CMVEGFP at dilutions of 1/20 and 1/80. In summary, when a pharmaceutical preparation of human immunoglobulin was tested for serological reactions to Pan 5, 6, and 7, and C68, some low levels of neutralizing activities against Pan 7 and C68 were detected. No neutralizing activity against Pan5 or Pan6 was detected. Serum samples from 36 human subjects were run for the same assay. Serum samples were tested at a 1/20 dilution. The results indicated that only one individual has clear neutralizing activity to C68. No neutralizing activity to Pan5, Pan6 or Pan7 was detected.

### 4. In Vitro Cross-Neutralization

Cross-neutralization of the simian adenoviruses by high-titer rabbit polyclonal antibodies raised against each of the adenoviruses Pan-5, Pan-6, Pan-7, and C68 was tested.

Rabbits were immunized with intra-muscular injections of 10¹³ particles of each of the chimpanzee adenoviruses and boosted 40 days later with the same dose with incomplete Freund's adjuvant. Sera were analyzed or the presence of neutralizing antibodies by incubating serial two-fold dilutions with 10⁹ genome copies of each of the appropriate chimpanzee adenovirus vector expressing GFP and testing for the attenuation of GFP expression when applied to 293 cells. The serum dilution which produced a 50% reduction of GFP expression was scored as the neutralizing antibody titer against that particular virus.

The results are shown in the Table. The data are consistent with the expectation from sequence analysis ofthe hexon amino-acid sequences, which indicated that Ad Pan-6 was likely to be the most serologically distinct compared to the other chimpanzee adenoviruses.

| | Infection of 293 cells with 10⁹ genome copies of | | | |
|---|---|---|---|---|
| Serum from rabbit immunized with: | Ad Pan-5 | Ad Pan-6 | Ad Pan-7 | Ad·C68 |
| Ad Pan-5 | 1/5120 | <1/20 | 1/2560 | 1/2560 |
| Ad Pan-6 | No neutralization | 1/20,480 | <1/20 | <1/20 |
| Ad Pan-7 | 1/2560 | 1/160 | 1/163,840 | 1/2560 |
| Ad C68 | No neutralization | <1/20 | <1/20 | 1/5120 |

In order to determine whether antibodies cross-reacting with the simian adenoviruses were likely to be of low prevalence in humans, simian adenoviruses SV1, SV39, and SV25 were tested for their ability to withstand neutralization when incubated with commercially available pooled human immunoglobulins (Ig). The same assay was also performed with Adhu5 and the chimpanzee adenoviruses Pan-5, Pan-6, Pan-7, and C68. In a further study, sera from mice has been immunized with one of the chimpanzee adenoviruses C5, C6, C7, and C68 and their ability to cross-neutralize the simian adenoviruses SV-15, SV-23, SA-17, and Baboon Adenovirus has been tested. No cross-reactivity was observed in any case.

### Example 4 - Generation of Recombinant E1-Deleted Pan5 Vector

A modified pX plasmid was prepared by destroying the FspI site in the bla gene region of pX (Clontech) by site-directed mutagenesis. The resulting modified plasmid, termed pX', is a circular plasmid of 3000 bp which contains an f1 ori and an ampicillin resistance gene (AmpR-cds).

### A. Production of Pan-5 Adenovirus Plasmid

A polylinker for sequential cloning of the Pan5 DNA fragments into pX' is created. The polylinker is substituted for the existing pX' polylinker following digestion with *MluI* and *EcoRI.* The blunt-*FseI* fragment of the Pan 5 is inserted into the *SmaI* and *FseI* sites of the polylinker. This fragment contains the 5' end of the adenoviral genome (bp 1 to 3606, SEQ ID NO:1). The *SnaBI-FspI* fragment of Pan 5 (bp 455 to 3484, SEQ ID NO:1) is replaced with a short sequence flanked by *I-Ceu* and *PI-Sce* sites from pShuttle (Clontech), to eliminate the E1 region of the adenoviral genome. The *EcoRI*-blunt fragment of Pan5 (bp 28658 to 36462, SEQ ID NO:1) is inserted into the *EcoRI* and *EcoRV* sites of the polylinker (to provide the 3' end ofthe adenoviral genome); the *FseI-MluI* fragment (bp 3606 to 15135, SEQ ID NO:1) is inserted into the polylinker; and the *MluI-EcoRI* fragment is inserted into the polylinker (bp 15135 to 28658, SEQ ID NO:1). Optionally, a desired transgene is inserted into *I-CeuI* and *PI-SceI* sites of the newly created pX'Pan5ΔE1 vector.

### B. Alternative Method of Generating pX'Pan5ΔE1.

The initial plasmid pX is derived from pAdX adenovirus plasmid available from Clontech, as described above. Thereafter, a *PacI-XhoI* region of pX' was deleted and the blunt-ended Pan5 polylinker was inserted into the *FspI* site to generate pX'PLNK (2994 bp). The 5'end-*FseI* region of Pan 5 (bp 1-3607, SEQ ID NO:1) was inserted into *SmaI* and *FseI* sites of pX'LNK to generate the pX'Pan5-5' plasmid (6591 bp). The *SnaBi-NdeI* region of pX'Pan5-5' was excised and replaced with the *Ceu*/*Sce* cassette, which had been PCR amplified from pRCS to create pX'Pan5-5'ΔE1 (4374 bp). Briefly, a sequence containing *I*-*CeuI* and *PI*-*SceI* rare cutter sites was PCR amplified from pRCS (3113bp). The 3' PCR primer was introduced an *NdeI* site into the PCR product.

To extend the Pan5 DNA in pX'Pan5-5' ΔE1 (4374 bp), the *FseI-MluI* region of Pan 5 (bp 3607-15135, SEQ ID NO:1) is added, to create pX'Pan5-5'Mlu (15900 bp). The remaining MluI-3' end of the Pan5 sequence (bp 15135-36462, SEQ ID NO:1) is added to the vector between the MluI and EcoRV sites of the vector polylinker to form pX'Pan5ΔE1 which contains the full-length Pan5 sequence containing a deletion in the E1 region.

### C. Generation of Recombinant Viruses

To generate the recombinant adenoviruses from pX'Pan5ΔE1, the plasmid is co-transfected with a helper expressing E1, or from an E1-expressing packaging cell line, such as 293 cell line or a cell line prepared as described herein. The expression of E1 in the packaging cell permits the replication and packaging of the Pan5ΔE1 into a virion capsid. In another embodiment, the packaging cell transfected with pX'Pan5ΔE1 is transfected with an adenovirus vector as described above bearing the transgene of interest. Homologous recombination occurs between the helper and the plasmid, which permits the adenovirus-transgene sequences in the vector to be replicated and packaged into virion capsids, resulting in the recombinant adenovirus.

Transfection is followed by an agar overlay for 2 weeks, after which the viruses are plaqued, expanded and screened for expression of the transgene. Several additional rounds of plaque purification are followed by another expansion of the cultures. Finally the cells are harvested, a virus extract prepared and the recombinant chimpanzee adenovirus containing the desired transgene is purified by buoyant density ultracentrifugation in a CsCl gradient or by alternative means known to those of skill in the art.

### Example 5 - Generation of Recombinant E1-Deleted Pan6 Vector

### A. Strategy for Construction of Pan-6 Adenoviral Plasmid

### 1. Cloning of terminal fragments

Pan 6 virus is deproteinated by pronase and proteanase K treatment and phenol extraction. Synthetic 12 bp Pme I linkers are ligated onto the viral DNA as described by Berkner and Sharp, Nucleic Acids Research, 11: 6003 (1983). The viral DNA is then digested with Xba I to isolate a 5' terminal fragment (6043 bp). The Ad6 Xbal 5' fragment is then ligated into pX link at Sma I and Xba I sites to form pX-AdPan6-0-16.5. The viral DNA with Pme I linkers is also digested with Pac I to isolate the 6475 bp 3' terminal fragment and cloned into pX link at Pac I and Sma I sites, resulting in pXAdPan6-82-100.

### 2. Deletion of E1 from the 5' clone

To delete E1 (m.u.1.2-9), the BsiWi-Xba I fragment in pX-AdPan6-0-16.5 is replaced with a PCR fragment spanning m.u.9-16.7 fragment treated with BsiWi and Xba I, leading to pX-Ad-Pan6 m.u.0-1, 9-16.5 .

### 3. Fusion of 5' and 3' clones and to create an anchor site to accept the middle Hind III fragment

First, the 5' clone, pX-Ad-Pan6 m.u.0-1, 9-16.5, is further expanded by inserting the 2^{nd} Xba I fragment (4350 bp, m.u.16.5 - 28) from Pan 6 genome into the Xba I site in the pX-Ad-Pan6 m.u.0-1, 9-16.5. This construct is named pXAd-Pan6-mu 0-1, 9-28.

Second, the 3' clone is also expanded by inserting the 15026 bp Mlu I/Pac I fragment covering m.u.41-82 from Pan 6 genome into the Mlu I/Pac I sites of pXAdPan6-82-100, generating pXAdPan6-m u.41-100.

Then, a 8167 bp Hind in/Eco 47III Pan 6 fragment is isolated from pXAd-Pan6-mu 0-1, 9-28 and subcloned into pXAdPan6-m.u.41-100 at Hind III and Xba I blunt sites. This 5' and 3' fusion clone is called pXAdPan6mu0-1, 9-19.5, 64-100.

### 4. Drop of the middle fragment of the genome into the fusion clone

A 16335 bp Hind III fragment (m.u.19.5 - 64) from Pan 6 is inserted into Hind III site of pXAdPan6mu0-1, 9-19.5, 64-100 to form pXAdPan6-0-1, 9-100.

### 5. Introduction of a PKGFP selective marker into the final construct for direct cloning the gene of interest and green/white selection of recombinant transformants.

A minigene cassette that expresses GFP under a lac promoter and is flanked with recognition sites of rare intron encoding restriction enzymes, PI-Sce I and I-Ceu I, was isolated from pShuttle-pkGFP (bare) by Sap I and Dra III digestions followed by filling-in reaction. The pShuttle-pkGFP (bare) plasmid is 4126 bp in length, and contains a ColE1-Ori, a kanamycin resistance gene, plac, a LacZ promoter-GFPmut3-1 cds (Clontech), and a GFPmut3-1 cds (Clontech). This cassette is subcloned into SrfI cut and blunted pXAdPan6-0-1, 9-100. This final construct is called pX-Pan6-pkGFP mu.0-1, 9-100, which is useful for generating recombinant E1-deleted Pan 6 molecular clones carrying genes of interest by direct ligation and green/white selection in combination with the generic pShuttlepkGFP vectors.

### B. Alternative Strategy for Generation of Pan-6 Plasmid

### 1. Cloning of 5' terminal fragment

The Pan 6 virus is deproteinated by pronase and proteanase K treatment and phenol extraction as described above and synthetic 12 bp Pme I linkers are ligated onto the viral DNA as described. The AdPan6 5' XbaI fragment is isolated and ligated into pX to form pX-AdPan6-0-16.5 (9022 bp) as described in Part A above.

### 2. Deletion of E1 from the 5' clone

To delete E1 (m.u. 1.2-9), pX-AdPan6-0-16.5 is digested with SnaBI and NdeI to remove the regions encoding the E1a and E1b proteins (3442-6310 bp). This vector is subsequently digested with BsiWI in preparation for blunting with the minigene cassette carrying a selective marker.

### 3. Introduction of a selective marker

A minigene cassette that expressed GFP under a lac promoter and which is flanked with recognition sites of rare intron encoding restriction enzymes, PI-XceI and I-Ceul, was isolated from pShuttle-pkGFP as described above. The DraIII-SapI fragment is then ligated with the digested pX-AdPan6-0-16.5 to form pX-AdPan6 MU 0-16.5ΔE1 (7749 bp).

### 4. Extension of Pan-6 Adenoviral Sequences

pX-AdPan6 MU 0-16.5ΔE1 was subjected to XbaI digestion to permit insertion of an XbaI-RsrII linker. An XbaI/RsrII digestion fragment from the AdPan6 genome was isolated (mu 28-100, 26240 bp) and ligated into the Xba/RsrII-digested pX-AdPan6 MU 0-16.5ΔE1 to provide pX-AdPan6 MU 0-1, 9-16.5, 28-100. A second XbaI fragment from the Pan6 genome (mu 16.5-28, 4350 bp) is then ligated into this plasmid to form pX-AdPan6 MU 0-1, 9-100 (38551 bp).

### C. Generation of Recombinant Adenoviruses

To generate the recombinant adenoviruses from a E1-deleted Pan6 plasmid prepared as described in Parts A or b, the plasmid is co-transfected with a helper expressing E1, or from an E1-expressing packaging cell line, such as 293 cell line or a cell line prepared as described herein. The expression of E1 in the packaging cell permits the replication and packaging of the Pan6-pkGFP mu.0-1, 9-100 into a virion capsid. Alternatively, the packaging cell transfected with pX-Pan6-pkGFP mu.0-1,9-100 is transfected with an adenovirus vector as described above bearing another transgene of interest.

### Example 6 - Generation of Recombinant E1-Deleted Pan7 Vector

### A. Generation of Pan7 Plasmids

A synthetic linker containing the restriction sites PacI-SmaI-FseI-MluI-EcoRV-PacI was cloned into pBR322 that was cut with EcoPI and NdeI. The left end (bp1 to 3618) of Ad Pan7 was cloned into the linker between the SmaI and FseI sites. The adenovirus E1 was then excised from the cloned left end by cutting with SnaBI and NdeI and inserting an I-CeuI-GFP-PI-SceI cassette from pShuttle (Clontech) in its place. The resulting plasmid was cut with FseI and MluI and Ad Pan7 fragment FseI (bp 3618) to MluI (bp 155114 was inserted to extend the left end. The construct (pPan7pGFP) was completed by inserting the 21421 bp Ad Pan7 right end fragment from the MluI site (bp 15114) into the above plasmid between MluI and EcoRV to generate a complete molecular clone of E1 deleted adenovirus Pan7 suitable for the generation of recombinant adenoviruses. Optionally, a desired transgene is inserted into the I-CeuI and PI-SceI sites of the newly created pPan7 vector plasmid.

### B. Construction of E1-Deleted Pan7 Viral Vectors

To generate the recombinant adenoviruses from pPan7ΔE1, the plasmid is co-transfected with a helper expressing E1, or from an E1-expressing packaging cell line, such as 293 cell line or a cell line prepared as described herein. The expression of E1 in the packaging cell permits the replication and packaging of the Pan7ΔE1 into a virion capsid. In another embodiment, the packaging cell transfected with pX'Pan7 ΔE1 is transfected with an adenovirus vector as described above bearing the transgene of interest. Homologous recombination occurs between the helper and the plasmid, which permits the adenovirus-transgene sequences in the vector to be replicated and packaged into virion capsids, resulting in the recombinant adenovirus. Transfection and purification is as described above.

### Example 7 - Generation of Plasmid Vectors Expressing the E1 Genes

Plasmid vectors are constructed which encode the Pan5 E1 region gene, and these plasmids are used to generate stable cell lines expressing viral E1 proteins.

The E1 region of Pan5 is cloned into pX', essentially.as described in Example 4 above, prior to replacement of this region with the fragment from pShuttle (Clontech). The expression plasmid contains the Pan5 adenoviral genome sequence spanning at least bp 1 to 3959 in the Pan5 genomic sequence. Thus, the expression plasmid contains the sequence encoding E1a and E1b of chimpanzee Ad Pan5 under the control of a heterologous promoter. Similar expression plasmids can be generated using the Ad Pan6 and AdPan 7 E1 regions, identified in the tables above.

### Example 8 - Generation of Cell Lines Expressing Chimpanzee Adenovirus E1 Proteins

Cell lines expressing viral E1 proteins are generated by transfecting HeLa (ATCC Acc. No. CCL2) with the plasmid of Example 6. These cell lines are useful for the production of E1-deleted recombinant chimpanzee adenoviruses by co-transfection of genomic viral DNA and the expression plasmids described above. Transfection of these cell lines, as well as isolation and purification of recombinant chimpanzee adenoviruses therefrom are performed by methods conventional for other adenoviruses, i.e., human adenoviruses [see, e.g., Horwitz, cited above and other standard texts].

### A. Cell lines expressing Pan5 E1 proteins

HeLa cells in 10cm dishes are transfected with 10 µg of pX-Pan51-E1 DNA using a Cellphect™ kit (Pharmacia, Uppsala, Sweden) and following the manufacturer's protocol. 22 hours post-transfection, the cells are subjected to a three minute glycerol shock (15% glycerol in Hepes Buffered Saline, pH 7.5) washed once in DMEM (HeLa) or F12K (A549; Life Technologies, Inc., Grand Island, NY) media supplemented with 10% FCS, 1% Pen-Strep, then incubated for six hours at 37°C in the above described media. The transfected cells are then split into duplicate 15cm plates at ratios of 1:20, 1:40, 1:80, 1:160, and 1:320. Following incubation at 37°C overnight, the media is supplemented with G418 (Life Technologies, Inc.) at a concentration of 1 µg/ml. The media is replaced every 5 days and clones are isolated 20 days post-transfection.

HeLa E1 cell clones are isolated and assayed for their ability to augment adeno-associated virus (AAV) infection and expression of recombinant LacZ protein as described below.

### B. AAV Augmentation Assay for Screening E1 Expressing Cell Lines

AAV requires adenovirus-encoded proteins in order to complete its life cycle. The adenoviral E1 proteins as well as the E4 region-encoded ORF6 protein are necessary for the augmentation of AAV infection. An assay for E1 expression based on AAV augmentation is used. Briefly, the method for identifying adenoviral E1-expressing cells comprises the steps of infecting in separate cultures a putative adenovirus E1-expressing cell and a cell containing no adenovirus sequence, with both an adeno-associated virus (AAV) expressing a marker gene and an AAV expressing the ORF6 of the E4 gene of human adenovirus, for a suitable time. The marker gene activity in the resulting cells is measured and those cells with significantly greater measurable marker activity than the control cells are selected as confirmed E1-expressing cells. In the following experiment, the marker gene is a lacZ gene and the marker activity is the appearance of blue stain.

For example, the cell lines described above, as well as untransfected control cells (HeLa) are infected with 100 genomes per cell of an AAV vector bearing a marker gene, e.g., AV.LacZ [K. Fisher *et al*., J. Virol., **70**:520 (1996)] and an AAV vector expressing the ORF6 region of human 5 (AV.orf6). The DNA sequence of the plasmid generates a novel recombinant adeno-associated virus (rAAV) containing the *LacZ* transgene and the Ad E4 ORF 6, which is an open reading frame whose expression product facilitates single-stranded (ss) to double-stranded (ds) conversion of rAAV genomic DNA. These vectors are incubated in medium containing 2% FCS and 1% Pen-Strep at 37°C for 4 hours, at which point an equal volume of medium containing 10% FCS is added. It should be understood by one of skill in the art that any marker gene (or reporter gene) may be employed in the first AAV vector of this assay, e.g., alkaline phosphatase, luciferase, and others. An antibody-enzyme assay can also be used to quantitate levels of antigen, where the marker expresses an antigen. The assay is not limited by the identity of the marker gene. Twenty to twenty-four hours post-infection, the cells are stained for LacZ activity using standard methods. After 4 hours the cells are observed microscopically and cell lines with significantly more blue cells than the A549 or HeLa cell controls are scored as positive.

### Example 9 - Delivery of Transgene to Host Cell

The resulting recombinant chimpanzee adenovirus described in Example 4, 5 or 6 above is then employed to deliver the transgene to a mammalian, preferably human, cell. For example, following purification of the recombinant virus, human embryonic kidney 293 cells are infected at an MOI of 50 particles per cell. GFP expression was documented 24 hours post-infection.

### A. Gene Transfer in Mouse Models via Pan-6, Pan-7, and Pan-9 vectors

Gene transfer efficiencies and toxicological profile of recombinant chimpanzee adenoviruses were compared in mouse liver directed gene transfer, mouse lung directed gene transfer, and mouse muscle directed gene transfer.

E1-deleted adenoviral vectors containing LacZ under the control of the CMV promoter were constructed using the techniques herein for human Ad5, chimpanzee Pan 6, chimpanzee Pan 7 and chimpanzee Pan 9 (C68). The vectors were delivered to immune-deficient NCR nude mice (80 for each study) as follows. For the liver study, 100 µl (1 x10¹¹ particles) were injected into the tail vein. For the lung study, 50 µl (5 x10¹⁰ particles) were delivered intratracheally. For the muscle study, 25 µl (5 x10¹⁰particles) were injected into tibialis anterior. The mice were sacrificed on days 3, 7, 14 and 28 post-vector injection (5 animals per group at each time point). At each necropsy, the liver/lung/muscle tissue was harvested and prepared for cryoblocks and paraffin embedding. The cryoblocks were sectioned for X-gal staining and the paraffin sections are H&E stained for histopathic analysis. At each time point, terminal bleeding was performed. Serum samples were subjected to liver function tests.

It was observed in this experiment the chimpanzee adenoviruses Pan-6, Pan-7, and Pan-9 were less efficient than huAd5 in gene transfer to the liver and to the lung. However, this may be desirable in certain circumstances, to reduce liver toxicity observed for huAd5. The gene transfer efficiency in muscle varied less between serotypes.

### B. Mouse study to feasibility of re-administration of adenovirus vectors by serotype switching between Adhu5, Pan-6, Pan-7, and Pan-9 vectors

Mice were administered (C57/B16; 4/group) LacZ vectors based on huAd5, Pan-6, Pan-7, and Pan-9 (H5.040CMVLacZ, Pan6.000CMVLacZ, Pan7.000CMVLacZ, Pan9.000CMVLacZ; 10¹¹particles/injection) by tail vein. Thirty days later the mice were re-administered adenovirus vectors expressing α1-antitrypsin (H5.040CMVhA1AT, Pan6.000CMVhA1AT, 1x10¹¹ particles, Pan7.000CMVhA1AT, Pan9.000CMVhAlAT, 10¹¹particles/injection). Successful transduction by the re-administered vector is monitored by measuring serum α1-antitrypsin on days 3 and 7, following re-administration.

The ability of adenovirus vectors based on huAd5, Pan-6, Pan-7, and Pan-9 respectively to transduce the livers of mice in the presence of neutralizing antibodies to the other serotypes was determined. The results are tabulated here.

| 1^{st} injection | 2^{nd} injection | Cross-neutralization |
|---|---|---|
| Adhu5 | Adhu5 | Yes (+ve control) |
| | Pan-6 | No |
| | Pan-7 | No |
| | Pan-9 (C68) | No |
| Pan-6 | Adhu5 | No |
| | Pan-6 | Yes (+ve control) |
| | Pan-7 | Yes |
| | Pan-9 (C68) | No |
| Pan-7 | Adhu5 | No |
| | Pan-6 | Yes |
| | Pan-7 | Yes (+ve control) |
| | Pan-9 (C68) | Yes |
| Pan-9 (C68) | Adhu5 | No |
| | Pan-6 | No |
| | Pan-7 | Yes |
| | Pan-9 (C68) | Yes (+ve control) |

Ability of vectors to transduce murine liver in the presence of neutralizing antibodies to other serotypes.

Thus, immunization with huAd5 does not prevent re-administration with either of the chimpanzee adenovirus vectors Pan-6, Pan-7, or Pan-9 (C68). This experiment also appears to indicate that Pan-7 is between Pan-6 and Pan-9 in the spectrum of antigenic relatedness and cross-reacts with both; however Pan-6 and Pan-9 do not neutralize each other. This is a surprising result based on homology comparisons, which indicates that Pan-6 is quite distinct from Pan-7 and Pan-9. Evaluation of antisera generated against Pan-9 indicated no cross-neutralization against Pan-6 but some neutralization against Pan-7, arguing that Pan-6 is distinct from the others.

### Example 10 - Generation of Recombinant E1-Deleted SV-25 Vector

A plasmid was constructed containing the complete SV-25 genome except for an engineered E1 deletion. At the site of the E1 deletion recognition sites for the restriction enzymes I-CcuI and PI-SceI which would allow insertion oftransgene from a shuttle plasmid where the transgene expression cassette is flanked by these two enzyme recognition sites were inserted.

A synthetic linker containing the restriction sites SwaI-SnaBI-SpeI-AfIII-EcoRV-SwaI was cloned into pBR322 that was cut with EcoRI and Ndel. This was done by annealing together two synthetic oligomers SV25T (5'-AAT TTA AAT ACG TAG CGC ACT AGT CGC GCT AAG CGC GGA TAT CAT TTA AA-3', SEQ ID NO: 38) and SV25B (5'-TAT TTA AAT GAT ATC CGC GCT TAA GCG CGA CTA GTG CGC TAC GTA TTT A-3', SEQ ID NO:39) and inserting it into pBR322 digested with EcoRI and NdeI. The left end (bp1 to 1057, SEQ ID NO:29) of Ad SV25 was cloned into the above linker between the SnaBI and SpeI sites. The right end (bp28059 to 31042, SEQ ID NO: 29) of Ad SV25 was cloned into the linker between the AfIII and EcoRV sites. The adenovirus E1 was then excised between the EcoRI site (bp 547) to XhoI (bp 2031) from the cloned left end as follows. A PCR generated I-CeuI-PI-SceI cassette from pShuttle (Clontech) was inserted between the EcoRI and SpeI sites. The 10154 bp XhoI fragment of Ad SV-25 (bp2031 to 12185, SEQ ID NO:29) was then inserted into the SpeI site. The resulting plasmid was digested with HindIII and the construct (pSV25) was completed by inserting the 18344 bp Ad SV-25 HindIII fragment (bp11984 to 30328. SEQ ID NO:29) to generate a complete molecular clone of E1 deleted adenovirus SV25 suitable for the generation of recombinant adenoviruses. Optionally, a desired transgene is inserted into the I-CeuI and PI-SceI sites of the newly created pSV25 vector plasmid.

To generate an AdSV25 carrying a marker gene, a GFP (green fluorescent protein) expression cassette previously cloned in the plasmid pShuttle (Clontech) was excised with the restriction enzymes I-CeuI and PI-Sce1 and ligated into pSV25 (or another of the Ad chimp plasmids described herein) digested with the same enzymes. The resulting plasmid (pSV25GFP) was digested with SwaI to separate the bacterial plasmid backbone and transfected into the E1 complementing cell line HEK 293. About 10 days later, a cytopathic effect was observed indicating the presence of replicative virus. The successful generation of an Ad SV25 based adenoviral vector expressing GFP was confirmed by applying the supernatant from the transfected culture on to fresh cell cultures. The presence of secondarily infected cells was determined by observation of green fluorescence in a population of the cells.

### Example 11 - Construction of E3 deleted Pan-5, Pan-6, Pan-7 and C68 vectors

In order to enhance the cloning capacity of the adenoviral vectors, the E3 region can be deleted because this region encodes genes that are not required for the propagation of the virus in culture. Towards this end, E3-deleted versions of Pan-5, Pan-6, Pan-7, and C68 have been made (a 3.5 kb Nru-AvrII fragment containing E31-9 is deleted).

### A. E3 deleted Pan5 based vector

E1-deleted pPan5-pkGFP plasmid was treated with Avr II endonuclease to isolate a 5.8 kb fragment containing the E3 region and re-circulate pPan5-pkGFP with Avr II deletion to form construct pPan5-pkGFP-E3-Avr II. Subsequently, the 5.8 kb Avr II fragment was subcloned into pSL-Pan5-E3-Avr II for a further deletion of E3 region by Nru I digestion. This led to a plasmid pSL-Pan5-E3-deletion. The final construct pPan5-E3-pkGFP was produced by removing a 4.3 kb Avr II/Spe I fragment from pSL-Pan5-E3-deletion plasmid and inserting into pPan5-pkGFP-E3-Avr II at Avr II site. In this final construct, a 3.1 kb deletion in E3 region was accomplished.

### B. E3 deletion in Pan6 based vector

E1-deleted pPan6- pkGFP molecular clone was digested with Sbf I and Not I to isolate 19.3 kb fragment and ligated back at Sbf I site. The resulting construct pPan6-Sbf I-E3 was treated with Eco 47 III and Swa I, generating pPan6-E3. Finally, 21 kb Sbf I fragment from Sbf I digestion of pPan6- pkGFP was subcloned into pPan6-E3 to create pPan6-E3-pkGFP with a 4 kb deletion in E3.

### C. E3 deleted Pan7 and Pan9 vectors

The same strategy was used to achieve E3 deletions in both vectors. First, a 5.8 kb Avr II fragment spanning the E3 region was subcloned pSL-1180, followed by deletion of E3 by Nru I digestion. The resulting plasmids were treated with Spe I and Avr II to obtain 4.4 kb fragments and clone into pPan7- pkGFP and pPan9-pkGFP at Avr II sites to replace the original E3 containing Avr II fragments, respectively. The final pPan7-E3- pkGFP and pPan9-E3- pkGFP constructs have 3.5 kb E3-deletions.

### Example 12 - Construction of E3- and E4-deleted Pan-7 vector

Although the deletion of the E1 region of adenoviruses (first generation adenovirus vectors) renders them replication-incompetent, expression of the adenoviral vector backbone genes is not fully abolished. Deletion of the E4 region considerably attenuates this residual gene expression and may confer a safety advantage. An E4-deleted Pan-7 vector containing a 2.5 kb deletion (a PvuII-AgeI fragment containing E40RF1-ORF7 is deleted) has been constructed. High titer stocks of this virus were generated using a HEK 293-based cell line, which in addition to E1, expresses an essential E4 gene (orf 6).

### 1. E4 deletion in the molecular clone of Pan7

A 19 kb Xba I fragment was deleted from pPan7- pkGFP to create pPan7-Xba I from which a 2.5 kb E4 fragment was deleted by Age I and Pvu II partial digestion, resulting in pPan7-Xba I-E4. pPan7-E4- pkGFP plasmid was generated from pPan7-Xba I-E4 in two sequential cloning steps, adding 19 kb Xba I and 15 kb I-Ceu I/Mlu I fragments, both of which came from pPan7- pkGFP construct.

### 2. Introduction of E3 and E4 deletions in Pan9 vector

A 11 kb plasmid, pPan9-EcoRI, containing E4 region was created by retrieving 11 kb EcoRI fragment from pPan9 pkGFP after EcoRI digestion and self-ligation. E4 region was deleted from this construct by Age I digestion/filled in and Pvu II partial digestion and slef-ligation to generate pPan9-EcoR I-E4. A 23 kb EcoR I fragment was isolated from pPan9-pkGFP and inserted into pPan9-EcoR I-E4 at EcoR I site, followed by adding 5.8 kb Avr II fragment from pPan9-pkGFP, to form the final product pPan9-E3-E4- pkGF. Compared to the genome size of wild type Pan9, this E1-E3-E4-deleted vector could have a transgene capacity up to 8 kb.

### 3. Introduction of minigene cassettes with genes of interest including reporter genes, glyco- and nuclear proteins of Ebo into molecular clones of Pan vectors

A highly efficient direct cloning and green/white selection procedure was employed for creating molecular clones of recombinant viruses. Briefly, genes of interest were cloned into pShuttlepkGFP by screening white colonies for recombinants. Subsequently, the minigene cassettes were transferred into chimpanzee adenovirus backbone plasmids, pPanX-pkGFP with various deletions, easily by swapping with pkGFP cassette at I-Ceu I and PI-Sce I sites and screening a few white colonies for correct recombinants.

### 4. Rescue of molecular clones of Pan vectors with multiple deletions in early regions and virus propagation

For rescue of E1-E3-deleted molecular clones of chimpanzee adenovirus vectors, the clones were linearized with appropriate restriction enzymes and transfected into regular 293 cells. Once a full cytopathic effect (CPE) observed in the transfected cells, crude lysate was harvested and expanded in 293 cells to large-scale infections. The viruses were purified by CsCl sedimentation method.

For E1-E4 and E1-E3-E4-deleted Pan vectors, 10-3 cells, a 293-based E1-E4-complementing cell line, were used for rescue and propagation of vectors. E4 ORF6 gene expression in 10-3 cells was induced by addition of 150 µM ZnSO⁴ to the culture medium.

### Example 13 - Vaccination with adenovirus vectors expressing wild type and variant EboZ GP.

AdHu5 or AdC7 vectors expressing Ebola envelope chimeras were produced for *in vivo* immunization experiments in C57BL/6 mice. Recombinant viruses with different viral backbones were created by molecular cloning method in which the minigene cassettes were inserted into the place of E1-deletions. The molecular clones of all recombinant viruses were rescued and grown up in 293 cells for large-scale purification using CsCl sedimentation method. Five EboZ variants encoded by AdHu5 or AdPan7 (C7) were selected and produced to evaluate their relative immunogenicity following an intramuscular Ad injection. The wt Ebo, a soluble Ebo variant, EboΔ1, EboΔ2, EboΔ3, EboΔ4, EboΔ5S, EboΔ6S, EboΔ7S and EboΔ8S were evaluated in the initial vaccine studies. For the data summarized in the following table, the number of viral particles (per ml or total) produced and amplified from infected 293 cells was established by spectrophotometry reading.

**Table: Production of Adhu5 or AdC7 Adenoviral vector encoding EboZ variant.**

| | HuAd5 | | AdC7 | |
|---|---|---|---|---|
| Gene | Titer (VP x 10¹²/ml) | Total yield (VP x 10¹²) | Titer (VP x 10¹²/ml) | Total yield (VP x 10¹²) |
| Ebo wt | 2.6 | 12 | 4.3 | 43 |
| EboS | 4.9 | 49 | 4.6 | 55 |
| EboΔ2 | 2.1 | 9 | 5.8 | 93 |
| EboΔ3 | 1.7 | 8 | 5.3 | 95 |
| EboΔ4 | 3 | 12 | 4.1 | 62 |

Vector was administered intramuscularly (10¹¹ genome copies/cell) in C57BL/6 mice and the presence of virus neutralizing antibody (VNA0 was evaluated 28 days later as a first measure of an immune response generated against the Ebola envelope glycoprotein. VNA is defined here as serum antibody able to inhibit transduction of HeLa cells mediated by HIV-based vector pseudotyped with the wild-type Ebola envelope.

VNA to the EboZ pseudotypes was detected with AdPan7 (C7) yielding higher titers than AdHu5. The EboZΔ3 elicited the highest VNA in terms of the transgene targets. For the data summarized in the following table, neutralizing antibody titers to HIV-EboZ-GFP pseudotypes (reciprocal dilution) are provided (N=5 animals/group).

| | VNA Titers | | |
|---|---|---|---|
| | EboZ wildtype | EboZs | EboZA3 |
| AdHu5 | 12 | 16 | 12 |
| AdC7 | 44 | 12 | 140 |

### Example 14 - Pan7-mediated Expression of Ebola Proteins

Mouse studies to evaluate Pan-7 vectors expressing Ebola envelope proteins and the Ebola nuclear antigen have been initiated. These are directed towards evaluation of neutralizing antibodies in C57B1/6 mice injected intramuscularly (IM) with Adhu5 or Pan-7 expressing each of 4 Ebola env constructs.

### A. Evaluation of CTL from C57B1/6 mice injected IM with Adhu5 or Pan-7 expressing the Ebola env constructs.

### 1. Challenge experiment in mice with Ebola virus.

Neutralizing antibody (NAB) responses to the Ebola envelope were analyzed by looking at immunized mouse sera mediated neutralization of a lentiviral (HIV) vector pseudotyped with the several constructs (eEbo, NTD2, NTD3, NTD4) of the Ebola envelope glycoprotein. C57BL/6 or BALB/c mice received a single intramuscular injection of 5 x 10¹⁰ particles per mouse of C7 (Ad Pan-7) encoding Ebola envelope variant. Neutralizing antibody was evaluated 30 days post-vaccination. Briefly, Ebola Zaire pseudotyped HIV vector encoding for β-galactosidase (EboZ-HIV-LacZ) was incubated for 2 hr at 37°C with different dilution of heat inactivated mouse serum. Following the incubation with serum, EboZ-HIV-LacZ was then used to infect HeLa cells for 16 hr at 37°C. Infectivity was revealed by X-gal staining of transduced HeLa cells positive for β-galactosidase. Neutralizing titer represent the serum reciprocal dilution where a 50% decrease in the number of β-galactosidase positive blue cells was observed. Sera were collected 30 days post-immunization, which consisted in a single intramuscular (I.M.) administration of 5 x 10¹⁰ particles/animal. Neutralizing antibody to Ebola pseudotyped HIV could be detected from all groups with antibody titers ranging from 20 for Ad-EboZ (Adhu5 expressing EboZ), Ad-NTD3 (Adhu5 expressing NTD3) and C7-sEbo (Ad Pan-7 expressing soluble EboZ) to over 130 for C7-NTD3 (Ad Pan-7 expressing soluble NTD3) and C7-NTD4 (Ad Pan-7 expressing soluble NTD3). The same immunization strategy in BALB/c mice resulted in lower neutralizing antibody titers for Ad- and C7-NTD2, and NTD4.

### B. Cellular Immune Response

The cellular immune response to the Ebola envelope in C57BL/6 mice was evaluated 8 days after a single I.M. administration of 5 x 10¹⁰ particles of C7-LacZ or C7-Ebola envelope variant per animal. Mice were vaccinated I.M. with 5 x 10¹⁰ particles of C7 encoding LacZ or Ebola envelope variant. Splenic lymphocytes from immunized mice were collected 8 days post vaccination and stimulated in vitro with feeder cells (splenic lymphocytes from untreated mice infected with human Adenovirus serotype 5 encoding for the wild-type Eboia envelope and irradiated). Standard 5-hr CTL assays were performed using ⁵¹Cr-labeled syngenic C57 cells transfected with an expressor of EboZ.

A positive MHC-restricted cytotoxic T lymphocyte (CTL) response was observed from all AdPan-7 encoding for Ebola envelope variants with a higher response from NTD2, NTD3 or NTD4 immunized mice. Indeed, effector cells from C7 encoding Ebola envelope variant immunized mice recognized EboZ transfected target cells and gave recall CTL responses up to 30% specific lysis. Less than 5% lysis was seen with effector cells from naïve or LacZ immunized control mice confirming that lysis was specific for Ebola envelope antigens.

### C. Protection Studies

The most direct means of evaluating C7 (Ad Pan-7) encoding for the EboZ variants as a successful vaccine in mice was to assess protection against weight loss and death following lethal challenge with mouse adapted Ebola Zaire virus. BALB/c mice were immunized with a single dose of 5 x 10¹⁰ particles per animal as performed previously and vaccinated animals were challenged with 200 LD₅₀ of mouse adapted Ebola Zaire 21 days later. All control mice (vehicle and C7-LacZ) died between day 5 to day 9 post-challenge. In contrast, all vaccinated mice but one, (from the C7-sEbo group), survived the challenge with Ebola Zaire.

Weight loss was observed from mice vaccinated with C7-sEbo from day 4 to day 7. Signs of illness such as pilo-erection and from light to severe lethargy were also noted from mice vaccinated with C7-sEbo, NTD2 and NTD3 between day 4 to day 7. Mice immunized with C7-EboZ and C7-NTD4 did not show sign of illness. Overall, a single dose of C7-EboZ and C7-NTD4 completely protected immunized mice from illness and death possibly due to a significant T cell mediated immunity.

All documents recited above are incorporated herein by reference. Numerous modifications and variations of the present invention are included in the scope of the above-identified specification and are expected to be obvious to one of skill in the art. Such modifications and alterations to the compositions and processes of the present invention, such as selections of different minigenes or selection or dosage of the vectors or immune modulators are believed to be within the scope of the claims appended hereto.

The following clauses define particular aspects of the present invention:
1. An isolated simian adenovirus nucleic acid sequence selected from the group consisting of: (a) Pan5 having the sequence of nucleic acids 1 to 36462 of SEQ ID NO :1 ; (b) Pan6 having the sequence of nucleic acids 1 to 36604 of SEQ ID NO : 5;(c) Pan7 having the sequence of nucleic acids 1 to 36535 of SEQ ID NO : 9; (d)SV1 having the sequence of nucleic acids 1 to 34264 of SEQ ID NO:24 ; (e) SV25 having the sequence of nucleic acids 1 to 31044 of SEQ ID NO: 29;(f) SV39 having the sequence of to nucleic acids 1 to34115 of SEQ ID NO: 34, and (g) a nucleic acid sequence complementary to the sequence of any of (a) to (f).
2. An isolated simian adenovirus serotype nucleic acid sequence selected from one or more of the group consisting of : (a) 5' inverted terminal repeat (ITR) sequences ; (b) the adenovirus E1a region, or a fragment thereof selected from among the 13S, 12S and 9S regions; (c) the adenovirus E1b region, or a fragment thereof selected from among the group consisting of the small T, large T, IX, and IVa2 regions ; (d) the E2b region ; (e) the L1 region, or a fragment thereof selected from among the group consisting of the 28.1 kD protein, polymerase, agnoprotein, 52/55 kD protein, and IIIa protein; (f) the L2 region, or a fragment thereof selected from the group consisting of the penton, VII, VI, and Mu proteins; (g) the L3 region, or a fragment thereof selected from the group consisting of the VI, hexon, or endoprotease; (h) the 2a protein; (i) the L4 region, or a fragment thereof selected from the group consisting of the100 kD protein, the 33 kD homolog, and VIII;(j) the E3 region, or a fragment thereof selected from the group consisting of E3 ORF1, E3 ORF2, E3 ORF3, E3 ORF4, E3 ORF5, E3ORF6, E3 ORF7, E3 ORF8, and E3 ORF9;(k) the L5 region, or a fragment thereof selected from a fiber protein;(1) the E4 region, or a fragment thereof selected from the group consisting of E4 ORF7, E4 ORF6, E4 ORF4, E4ORF3, E4 ORF2, and E4 ORF1 ; and (m) the 3' ITR, of any of Pan5 SEQ ID NO : 1; Pan6 SEQ ID NO :5 ; Pan7 SEQ ID NO :9 :SV1 SEQ ID NO: 24; SV25 SEQ ID NO:29 ; and SV39 SEQ ID NO: 34, or sequence complementary to any of (a) to (m).
3. A simian adenovirus protein encoded by the nucleic acid sequence according to clause 2.
4. A nucleic acid molecule comprising a heterologous simian adenoviral sequence according to clause 2.
5. The nucleic acid molecule according to clause 4, wherein said simian adenoviral sequence encodes an adenoviral gene product and is operatively linked to regulatory control sequences which direct expression of the adenoviral gene product in a host cells.
6. The nucleic acid molecule according to clause 4 or 5, wherein said simian adenoviral sequence comprises the E1a region of Pan5 SEQ ID NO : 1; Pan6 SEQ ID NO :5 ; Pan7 SEQ ID NO : 9; SV1 SEQ ID NO: 24; SV25 SEQ ID NO: 29; and SV39 SEQ ID NO: 34.
7. A pharmaceutical composition comprising the nucleic acid molecule according to clause 6 and a physiologically compatible carrier.
8. An isolated simian adenoviral capsid protein selected from the group consisting of : (a) a hexon protein of Pan5 SEQID NO : 3, Pan6 SEQID NO : 7, Pan7 SEQ ID NO : 11,SV1 SEQ ID NO : 26, SV25 SEQ ID NO : 31 or SV39 SEQ ID NO : 36, or fragment thereof; (b) a penton protein of Pan5 SEQ ID NO : 2, Pan6 SEQ ID NO : 6, Pan7 SEQ ID NO : 10, SV1 SEQ ID NO : 25, SV25 SEQ ID NO: 30 or SV39 SEQ ID NO : 35 ; (c) a fiber protein of Pan5 SEQ ID NO : 4, Pan6 SEQ ID NO : 8, Pan7 SEQ ID NO : 12, SV1 SEQ ID NO: 27 and SEQ ID NO : 28, SV25 SEQ ID NO: 32 and SEQ ID NO :33 or SV39 SEQ ID NO: 37, or a fragment thereof.
9. An artificial adenovirus serotype comprising a capsid protein according to clause 8 or a fragment thereof.
10. The artificial adenovirus serotype according to clause 9, wherein said capsid comprises a fragment of the hexon protein selected from the group consisting of Pan5 SEQ ID NO:15, Pan6SEQ ID NO : 16 and Pan7 SEQ ID NO : 17.
11. The artificial adenovirus serotype according to clause 9 or 10, wherein said capsid comprises a fragment of the fiber protein selected from the group consisting of Pan6 SEQ ID NO: 19, Pan7 SEQ ID NO : 20 and Pan5 SEQ ID NO : 21.
12. A nucleic acid molecule comprising a heterologous sequence encoding a protein according to clauses 3 or 8 or an artificial adenovirus serotype according to any of clauses 9 to 11.
13. A recombinant vector comprising a simian adenovirus sequence according to clause 2 or a nucleic acid molecule according to clause 4 or 12 and a heterologous gene operatively linked to sequences which direct expression of said gene in a host cell.
14. The recombinant vector according to clause 13, further comprising 5' and 3' adenovirus cis-elements necessary for replication and encapsidation.
15. The recombinant vector according to clause 13 or clause 14, wherein said vector is a virus.
16. The recombinant vector according to any of clauses 13 to 15 wherein said vector lacks all or a part of the E1 gene.
17. A recombinant virus comprising a simian capsid protein according to clause 3 or an artificial adenovirus serotype according to any of clauses 9 to 11.
18. A host cell comprising a nucleic acid molecule according to any of clauses 4 to 6 or 12, a recombinant vector according to any of clauses 13 to 16, or a recombinant virus according to clause 17.
19. The host cell according to clause 18, wherein said host cell is stably transformed with the nucleic acid molecule or the recombinant vector.
20. The host cell according to clause 18 or clause 19, wherein said host cell expresses one or more adenoviral gene products from said nucleic acid molecule or recombinant vector, said adenoviral gene products selected from the group consisting of E1a, E1b, E2a, and E4 ORF6.
21. The host cell according to any of clauses 18 to 20, wherein said host cell is stably transformed with a nucleic acid molecule comprising the simian adenovirus inverted terminal repeats.
22. A composition comprising a recombinant vector in a pharmaceutically acceptable carrier, said vector comprising a simian adenovirus sequence according to any of clauses 1 or 2 and a selected heterologous gene operatively linked to regulatory sequences which direct expression of said gene in a host cell.
23. A method for delivering a heterologous gene to a mammalian cell comprising introducing into said cell an effective amount of the vector of any of clauses 13 to 16 or a virus according to clause 17.
24. A method for repeat administration of a heterologous gene to a mammal comprising the steps of : (a) introducing into said mammal a first vector which comprises the heterologous gene and (b) introducing into said mammal a second vector which comprises the heterologous gene; wherein at least the first virus or the second vector is a virus according to clause 17 and wherein the first and second recombinant vector are different.
25. A method for producing a selected gene product comprising infecting a mammalian cell with the vector of any of clauses 13 to 16 or a virus according to clause 17, culturing said cell under suitable conditions and recovering from said cell culture the expressed gene product.
26. A method for eliciting an immune response in a mammalian host against an infective agent comprising administering to said host an effective amount of the recombinant adenovirus of clause 17, wherein said heterologous gene encodes an antigen of the infective agent.
27. The method according to clause 26, comprising the step of priming the host with a DNA vaccine comprising the heterologous gene prior to administering the recombinant adenovirus.
28. A composition comprising a simian adenovirus capsid protein according to clause 8 linked to a heterologous molecule for delivery to a selected host cell.
29. A method for targeting a cell having an adenoviral receptor comprising delivering to a subject a composition according to clause 28.

## Claims

1. A recombinant adenovirus having a capsid comprising an AdPan6 hexon protein having an amino acid sequence of SEQ ID NO:7, the adenovirus further comprising a transgene operatively linked to sequences which direct expression of said transgene in a host cell.

2. The recombinant adenovirus according to claim 1, further comprising 5' and 3' adenovirus cis-elements necessary for replication and encapsidation.

3. The recombinant adenovirus according to claim 1 or 2 wherein said virus comprises adenovirus sequences lacking a part or all of the E1 gene.

4. The recombinant adenovirus according to any one of claims 1 to 3, wherein the capsid further comprises an AdPan6 fiber protein fragment of SEQ ID NO: 19.

5. The recombinant adenovirus according to claim 4, wherein the capsid comprises an AdPan6 fiber protein of SEQ ID NO: 8.

6. The recombinant adenovirus according to any one of claims 1 to 5, wherein the capsid further comprises an AdPan6 penton protein.

7. The recombinant adenovirus according to any one of claims 1 to 6, wherein the capsid comprises an AdPan6 capsid.

8. The recombinant adenovirus according to claim 7, wherein said recombinant adenovirus is a pseudotyped adenovirus comprising 5' and 3' adenovirus cis-elements necessary for replication and encapsidation, said cis-elements comprising an adenovirus 5' inverted terminal repeat and an adenovirus 3' inverted terminal repeat.

9. The recombinant adenovirus according to any one of claims 1 to 8, wherein the recombinant adenovirus comprises one or more adenovirus genes.

10. The recombinant adenovirus according to any one of claims 1 to 9, wherein the recombinant adenovirus is replication-defective.

11. A recombinant adenovirus having a capsid comprising a hexon containing a fragment of the AdPan6 hexon protein and a nucleic acid sequence heterologous to the AdPan6, wherein the fragment of the AdPan7 hexon protein is selected from the group consisting of:
an N-terminal or C-terminal truncation of about 50 amino acids in length of the AdPan6 hexon protein sequence of SEQ ID NO:7;
amino acid residues 125 to 443;
amino acid residues 138 to 441;
amino acid residues 138 to 163;
amino acid residues 170 to 176; and
amino acid residues 404 to 430, with reference to SEQ ID NO:7.

12. The recombinant adenovirus according to any one of claims 6 to 11, wherein the adenovirus comprises adenoviral nucleic acid sequences selected from one or more of the group consisting of:
5' inverted terminal repeat (ITR) sequences;
the E3 region, or a fragment thereof selected from the group consisting of E3 ORF1, E3 ORF2, E3 ORF3, E3 ORF4, E3 ORF5, E3 ORF6, E3 ORF7, E3 ORF8, and E3 ORF9;
the E4 region, or a fragment thereof selected from the group consisting of E4 ORF7, E4 ORF6, E4 ORF4, E4 ORF3, E4 ORF2, and E4 ORF1; and
the 3' ITR,
of Pan6 SEQ ID NO:5 or sequence complementary thereto.

13. The recombinant adenovirus according to claim 12 comprising at least one simian capsid protein selected from:
a penton protein of Pan6 SEQ ID NO:6; and
a fiber protein of Pan6 SEQ ID NO:8.

14. An isolated host cell comprising a recombinant adenovirus according to any one of claims 6 to 13.

15. A composition comprising a recombinant adenovirus according to any one of claims 6 to 13 and a pharmaceutically acceptable carrier.

16. Use of a recombinant adenovirus of any one of claims 6 to 13 for the manufacture of a medicament formulated for eliciting an immune response against an infective agent in a mammalian host, wherein said heterologous gene encodes an antigen of the infective agent.
